# FASCICULE DE BREVET EUROPEEN

(11) **EP 2 164 521 B1**
(45) Date de publication et mention de la délivrance du brevet: **12.09.2018**
(21) Numéro de dépôt: 08805583.5
(22) Date de dépôt: 16.05.2008
(51) Int. Cl.: A61K 47/64, A61P 31/04, C07H 3/06, C07H 1/00

(54) **GLYCOCONJUGUES ET LEUR UTILISATION COMME VACCIN CONTRE SHIGELLA FLEXNERI DE SEROTYPE 3A ET X**
GLYCOKONJUGATE UND IHRE VERWENDUNG ALS IMPFSTOFF GEGEN DIE SHIGELLA FLEXNERI-SEROTYPEN 3A UND X
GLYCOCONJUGATES AND USE THEREOF AS VACCINE AGAINST SHIGELLA FLEXNERI SEROTYPE 3A AND X

(30) Priorité: 05.06.2007 CA 2591253
(43) Date de publication de la demande: 24.03.2010
(73) Titulaire: Institut Pasteur, 75015 Paris (FR); Centre National de la Recherche Scientifique, 75016 Paris (FR); Université Paris Descartes, 75270 Paris Cedex 06 (FR)
(72) Inventeur: MULARD, Laurence, F-94270 Le Kremlin Bicetre (FR); BOUTET, Julien, F-44770 La Plaine sur Mer (FR); GUERREIRO, Catherine, F-77380 Combes La Ville (FR); NATO, Farida, F-92160 Antony (FR); SANSONETTI, Philippe, F-75014 Paris (FR); PHALIPON, Armelle, F-75015 Paris (FR)
(74) Mandataire: Gevers & Orès
(86) Numéro de dépôt international: PCT/FR2008/000687
(87) Numéro de publication internationale: WO 2008/155487

(56) Documents cités:
- WO-A-2005/003775
- POZSGAY V: "Synthesis of glycoconjugate vaccines against shegella dysenteriae type 1" JOURNAL OF ORGANIC CHEMISTRY, vol. 63, 1 janvier 1998 (1998-01-01), pages 5983-5999, XP002305374 ISSN: 0022-3263
- WRIGHT K ET AL: "Preparation of synthetic glycoconjugates as potential vaccines against Shigella flexneri serotype 2a disease" ORGANIC AND BIOMOLECULAR CHEMISTRY, vol. 2, no. 10, 21 mai 2004 (2004-05-21), pages 1518-1527, XP002305375 ISSN: 1477-0520
- POZSGAY V ET AL: "Towards an oligosaccharide-based glycoconjugate vaccine against Shigella dysenteriae type 1" SYNLETT, no. 6, 2003, pages 743-767, XP002535551 ISSN: 0936-5214
- NORIEGA F R ET AL: "Strategy for cross-protection among Shigella flexneri serotypes" INFECTION AND IMMUNITY, vol. 67, no. 2, 1999, pages 782-788, XP002535552 ISSN: 0019-9567
- EKWALL E ET AL: "Shigella flexneri O-antigen-specific enzyme immunoassay: class-specific antibody titres against lipopolysaccharide antigens in healthy Vietnamese and Swedish populations" SERODIAGNOSIS AND IMMUNOTHERAPY IN INFECTIOUS DISEASE, vol. 2, no. 1, 1 février 1988 (1988-02-01), pages 47-61, XP023250134 ISSN: 0888-0786 [extrait le 1988-02-01]
- WITKOWSKA DANUTA ET AL: "Enterobacterial 38-kDa outer membrane protein is an age-dependent molecular marker of innate immunity and immunoglobulin deficiency as results from its reactivity with IgG and IgA antibody" FEMS IMMUNOLOGY AND MEDICAL MICROBIOLOGY, vol. 48, no. 2, 1 novembre 2006 (2006-11-01), pages 205-214, XP002478615 ISSN: 0928-8244
- CARLIN N I A ET AL: "MONOCLONAL ANTIBODIES SPECIFIC FOR SHIGELLA-FLEXNERI LIPOPOLYSACCHARIDES CLONES BINDING TO TYPE I AND TYPE III 6 7 8 ANTIGENS GROUP 6 ANTIGEN AND A CORE EPITOPE" INFECTION AND IMMUNITY, vol. 53, no. 1, 1986, pages 103-109, XP002535553 ISSN: 0019-9567
- CARLIN N I A ET AL: "MONOCLONAL ANTIBODIES SPECIFIC FOR SHIGELLA-FLEXNERI LIPOPOLYSACCHARIDES CLONES BINDING TO TYPE IV V AND VI ANTIGENS GROUP 3 4 ANTIGEN AND AN EPITOPE COMMON TO ALL SHIGELLA-FLEXNERI AND SHIGELLA-DYSENTERIAE TYPE 1 STRAINS" INFECTION AND IMMUNITY, vol. 55, no. 6, 1987, pages 1412-1420, XP002535554 ISSN: 0019-9567

## Description

La présente invention se rapporte à des composés dérivés de sucres reproduisant les épitopes des sérotypes 3a et X de *Shigella flexneri* et à leur utilisation pour la préparation de compositions vaccinales.

Plus spécifiquement, l'objet de la présente invention se rapporte à de nouveaux composés glycoconjugués comprenant des oligo- ou polysaccharides décrits ci-après, au procédé de synthèse de ces oligo- ou polysaccharides et glycoconjugués, à des dérivés de ces oligo- ou polysaccharides, à des compositions les contenant, ainsi qu'à l'utilisation des glycoconjugués à des fins de vaccination.

La présente invention se rapporte enfin à des méthodes de diagnostic d'une infection de *Shigella flexneri* utilisant un ou plusieurs oligo- ou polysaccharides ou leur conjugués.

Les vaccins conjugués polysaccharide-protéine représentent un progrès majeur dans le développement de vaccins antibactériens compatibles avec une utilisation chez les jeunes enfants. Il s'avère donc utile de mettre au point un vaccin de type glycoconjugué chimiquement défini contre les infections associées aux sérotypes prévalents de *Shigella flexneri,* une bactérie à Gram négatif responsable de la forme endémique de la dysenterie bacillaire. De manière à éviter certaines limitations liées à l'utilisation de polysaccharides extraits de cultures bactériennes, l'emploi d'antigènes saccharidiques synthétiques mimant la partie polysaccharidique (PS) du lipopolysaccharide (LPS) de S. *flexneri*, cible majeure de la réponse protectrice humorale de l'hôte à l'infection, a été privilégié.

La shigellose ou dysenterie bacillaire est responsable d'environ 1 million de décès par an, correspondant à 165 millions de cas, survenant principalement dans les pays en voie de développement (seulement 1,5 million de cas dans les pays développés). Les enfants de moins de 5 ans sont les plus touchés.

Cette maladie diarrhéique, transmise par voie féco-orale, est causée par une entérobactérie à Gram négatif appelée *Shigella.* Parmi les différents types de *Shigella* connus, *Shigella dysenteria* type 1 est à l'origine d'épidémies dévastatrices alors que *Shigella flexneri* et *Shigella sonnei* sont responsables de la forme endémique de la maladie. Dans le cas de *Shigella flexneri,* plusieurs sérotypes sont prévalents, en particulier les sérotypes 2a, 1b, 3a et 6. Améliorer les conditions d'hygiène dans les zones touchées permettrait de réduire le nombre de cas de shigellose, mais cela reste difficile. Ainsi la seule approche réaliste en terme de prévention reste le développement d'un vaccin à large couverture sérotypique. A ce jour, il n'existe aucun vaccin contre la shigellose.

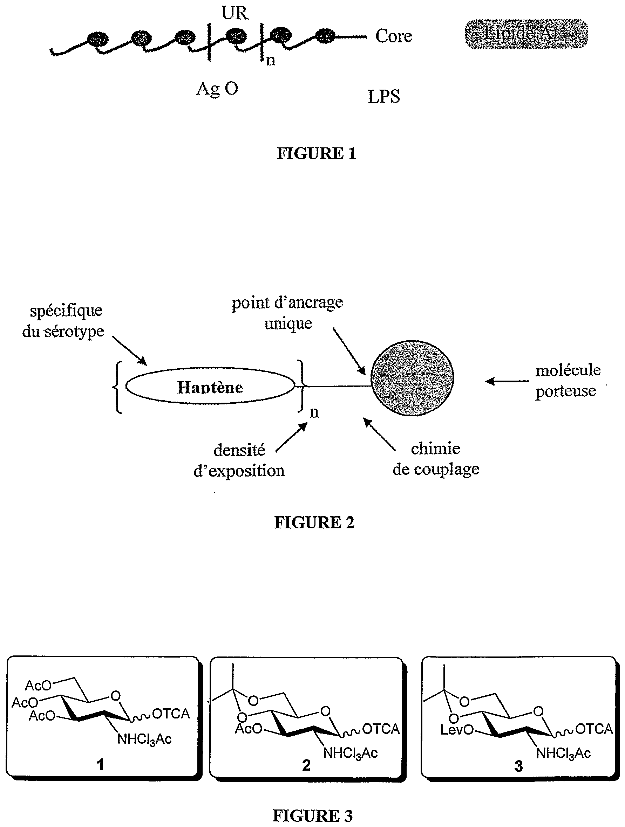

La réponse immunitaire à l'infection est essentiellement humorale. La réponse immunitaire protectrice contre la ré-infection est dirigée contre l'antigène majeur de surface, le lipopolysaccharide ou LPS. Celui-ci est composé de trois entités : une partie lipidique, le lipide A ancré dans la membrane ; un polysaccharide de bas poids moléculaire, appelé antigène O, exposé au milieu extérieur et dont l'unité répétitive caractérise le sérotype de la bactérie ; et un oligosaccharide court appelé le core qui réalise la jonction entre les deux (cf. Figure 1).

Les données disponibles sur le terrain montrent que la réponse immunitaire protectrice est spécifique du sérotype infectieux, ce qui indique l'antigène O comme cible privilégiée. Par analogie avec les vaccins à base de polysaccharides capsulaires déjà commercialisés, on peut donc espérer le développement de vaccins dérivés du LPS.

Or du fait de la présence du lipide A, le LPS est toxique, et ne peut être utilisé tel quel dans les préparations vaccinales de type glycoconjugués. Une étape de détoxification préliminaire est indispensable. Le LPS détoxifié est un polymère de bas poids moléculaire qui doit être conjugué à une molécule porteuse pour être utilisé dans une préparation vaccinale.

Cette approche présente certaines limitations :
- les rendements faibles
- le coût élevé
- le maintien de l'intégrité de l'antigène O et donc des épitopes impliqués dans la protection, pendant les différentes étapes de purification et les différentes étapes de détoxification et activation chimiques.

Aucun vaccin glycoconjugué à base de LPS n'est encore commercialisé.

WO 2005/003775, Ekwall et al. (Serodiagnosis and Immunotherapy in Infections Disease 1988, 2(1), 47), Witkowska et al. (FEMS Immunology and Medical Microbiology 2006, 48(2), 205), Carlin et al. (Infection and Immunity 1986, 53(1), 103 ; Infection and Immunity 1987, 55(6), 1412) décrivent des anticorps contre les sérotypes X et/ou 3a de Shigella flexneri.

Noriega et al. (Infection and Immunity 1999, 67(2), 782) décrit une combinaisaon de sérotypes 2a et 3a pour vacciner contre tout type de S. flexneri.

Pozsgay et al. (Journal of Organic Chemistry 1998, 63, 5983) décrit la préparation d'oligosaccharides pour les conjuguer à une protéine.

Pozsgay et al. (Synlett 2003, 6, 743) décrit la préparation d'oligosaccharides de S. *flexneri* pour les conjuguer à une protéine comme immunogène.

Wright et al. (Organic and Biomolecular Chemistry 2004, 2(10), 1518) décrit des vaccins contre S. flexneri sérotype 2a basés sur des néoglycopeptides contenant les haptènes (E)CD, B(E)CD et AB(E)CD sur PADRE comme peptide.

Cependant, ces conjugués sont différents de ceux de la présente invention.

L'alternative développée consiste à concevoir des vaccins glycoconjugués chimiquement définis reposant sur l'utilisation d'oligosaccharides synthétiques représentatifs des épitopes osidiques impliqués dans la protection.

La stratégie des constructions vaccinales ciblées est de coupler, par liaison covalente et *via* un point d'ancrage unique, un haptène spécifique du sérotype infectieux sur une molécule porteuse lui conférant l'immunogénicité requise (cf. Figure 2). Outre la nature de l'haptène et celle de la molécule porteuse, divers paramètres doivent être pris en considération tels que la densité d'exposition de l'haptène sur la molécule porteuse ou la chimie de couplage.

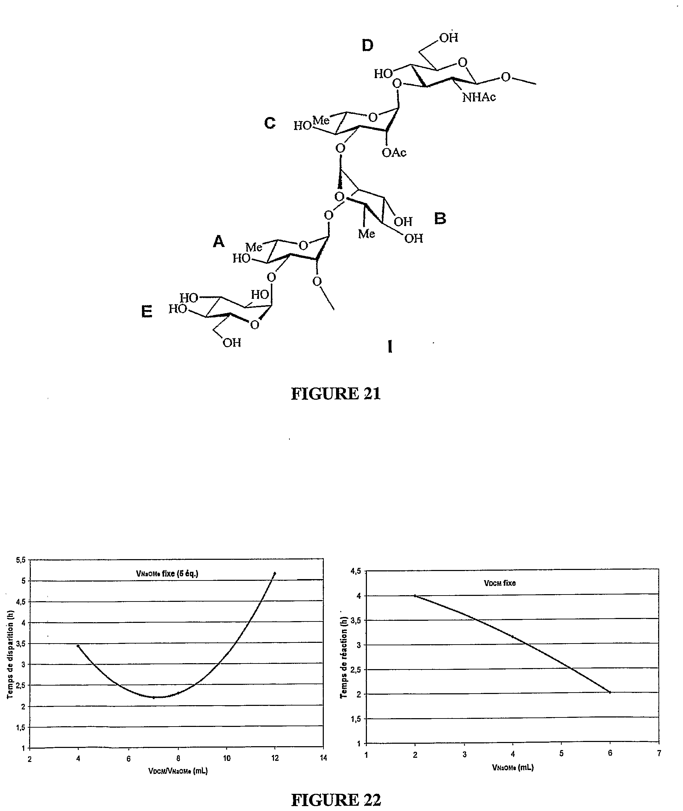

C'est cette approche que les inventeurs ont développée pour les sérotypes prévalents 3a et X ; ainsi, en premier lieu, ils ont été identifiés le(s) épitope(s) saccharidique(s) «protecteurs » contre l'infection par S. *flexneri* 3a. Pour ce faire, divers oligosaccharides représentatifs de l'antigène polymérique naturel dont l'unité répétitive biologique est le pentasaccharide ramifié (illustré à la Figure 21) ont été synthétisés, notamment en série de tri-, tétra- et pentasaccharides.

L'antigène **O** de la bactérie *S. flexneri* sérotype 3a a pour unité répétitive un pentasaccharide ramifié (E)AB_{Ac}CD. La partie linéaire est un tétrasaccharide composé de trois résidus L-rhamnose **A**, **B** et **C** et d'un résidu *N-*acétyl-D-glucosamine **D.** La spécificité sérotypique est associée à la présence d'un résidu α-D-glucose **E** en position 3 du rhamnose **A** ainsi qu'à la présence d'une fonction acétyle en position 2 du rhamnose **C** (Figure 21).

L'antigène O de la bactérie *S. flexneri* sérotype X, est le pentasaccharide ramifié (E)ABCD.

Une fois les épitopes protecteurs identifiés, les inventeurs ont ensuite synthétisé des glycoconjugués, protéiques ou peptidiques, induisant des titres élevés en anticorps anti-PS chez la souris. Ces glycoconjugués incorporent des haptènes saccharidiques mimant de façon optimale le PS naturel. Les haptènes sont sélectionnés sur la base des données d'antigénicité des oligosaccharides synthétisés (évaluées par ELISA).

### Légende des figures :

Figure 1 : Composition du LPS
Figure 2 : Constructions vaccinales
Figure 3 : Donneurs **1, 2** et **3**
Figure 4 : Structure du produit secondaire **11**
Figure 5 : Structure du produit secondaire **13**
Figure 6 : RMN du proton du céto-aldéhyde **16**
Figure 7 : RMN du carbone du céto-aldéhyde **16**
Figure 8 : Structure du diol **26**
Figure 9: Structure du donneur thiophényle **34**
Figure 10: Structure de **33'** issu du réarrangement de Chapman de **33**
Figure 11 : Structure de l'oxazoline **2'**
Figure 12 : Structure du donneur **39**
Figure 13 : Comparaison des spectres du proton de **40** et **41**
Figure 14 : HMBC du produit de couplage **41**
Figure 15 : Superposition des spectres C¹³GD de **40, 41** et **42**
Figure 16 : Structure du produit silylé observé lors du couplage entre **38** et **47**
Figure 17 : HSQC du mélange **77** et **78**
Figure 18 : HMBC du mélange **77** et **78**
Figure 19 : Structure du donneur **80**
Figure 20 : Structure de l'oxazoline **1'**
Figure 21 - Antigène O de la bactérie S. flexneri 3a
Figure 22 : Étude de la réduction de la fonction trichloroacétamide

### Exposé détaillé de l'invention

### L'objet de la présente invention est défini par les revendications 1 à 11.

Ainsi, la présente description a pour objet des conjugués spécifiques de *Shigella flexneri* de sérotype 3a et/ou X, comprenant un saccharide (oligosaccharide ou polysaccharide) sélectionné dans le groupe constitué par la liste L1 :

(X)ₓ-{D(E)A}ₙ-(Y)_{y}

(X)ₓ-{(E)AB}ₙ-(Y)_{y}

(X)ₓ-{CD(E)A}ₙ-(Y)_{y}

(X)ₓ-{D(E)AB}ₙ-(Y)_{y}

(X)ₓ-{(E)ABC}ₙ-(Y)_{y}

(X)ₓ-{BCD(E)A}ₙ-(Y)_{y}

(X)ₓ-{CD(E)AB}ₙ-(Y)_{y}

(X)ₓ-{D(E)ABC}ₙ-(Y)_{y}

(X)ₓ-{(E)ABCD}ₙ-(Y)_{y},

et dans lesquels :
A représente un résidu α-L-R*hap*-(1,2),
B représente un résidu α-L-Rha*p*-(1,3),
C représente un résidu α-L-Rha*p*-(1,3) ou un résidu [2-O-acétyle] α-L-Rha*p*-(1,3),
D représente un résidu β-D-GlcNAc*p*-(1,2) et
E représente un résidu α-D-Glc*p*-(1,3),
x et y représentent indépendamment 0 ou 1,
X et Y représentent un mono-, di- ou oligosaccharide et sont indépendamment sélectionnés dans le groupe constitué par A, B, C, D, E, AB, BC, CD, DA, (E)A, ABC, BCD, CDA, DAB, (E)AB, D(E)A, ABCD, BCDA, CDAB, DABC, (E)ABC, D(E)AB, CD(E)A, (E)ABCD, D(E)ABC, CD(E)AB et BCD(E)A,
X et Y sont tels que la séquence de l'enchaînement -ABCD- est toujours conservée, et n est un nombre entier compris entre 1 et 10, de préférence entre 2 et 6
lesquels saccharides (oligosaccharides et polysaccharides) étant liés à une molécule M de support de préférence.
X et Y sont tels que le saccharide selon la description présente toujours une séquence ou un enchainement de séquence partiel ou complet du saccharide naturel -ABCD-, voir à titre d'exemple, les composés I à XXVIII.
Les abréviations utilisées sont : Rha*p* : rhamnopyranosyl ; Glc*p* : glucopyranosyl ; GlcNAc*p* : 2-acétamido 2-désoxy-glucopyranosyl.

### Définitions

Au sens de la description, un oligosaccharide est un carbohydrate contenant de 2 à 20 unités monosaccharidiques liées ensemble ; dans la présente description le terme oligosaccharidique est utilisé de manière large et inclus tous les saccharides définis dans la présente description ; cet usage diffère de la définition standard des oligosaccharides qui en général ne contiennent pas plus de 10 unités monosaccharidiques (Joint Commission on Biological Nomenclature, Eur. J.Biochem., 1982, 126, 433-437).

Par polysaccharide, on entend dans la présente description un carbohydrate contenant plus de 20 unités monosaccharidiques liées entre elles.

Par la suite, on utilisera le terme saccharide pour désigner indifféremment les oligosaccharides et les polysaccharides.

Le terme « groupe fonctionnel » se réfère à des groupes d'atomes caractérisés par leurs compositions élémentaires ; de tels groupes fonctionnels confèrent une réactivité à la molécule qui les contient. Des groupes fonctionnels usuels incluent : des amines primaires (R-NH₂) ; des imines primaires (-C(=NH)-R') ; azo : [azo, -N=N-R'] ; nitrile : -C=N ; acide carboxylique ; carboxyle : -C(=O)OH ; acide carboxylique et ses dérivés tels que des esters : [-C(=O)O-R'] ou des esters activés ; carbonyle : [aldéhyde : -C(=O)H ; cétone, -C(=O)-R'], ou des dérivés de ces carbonyles tels que des carbonyles masqués (acétal ou thioacétal ; alkènes : [-CH=CH-R'] ; alkynes : [-C≡C-R'] ; isocyanates : [-N=C=O] ; isothiocyanate : [-N=C=S] ; thioacyle : [-SCO-R'], [thiol - SH, dithiol : -S-S-R'] ; azide -N₃ : hydrazide :-CONHNH₂, hydrazine, maléimide, O-alkyle hydroxylamine, halogène. Les groupes fonctionnels selon la description comprennent aussi les donneurs thioglycosides et les donneurs de type phosphates ou encore le trichloroacétimidate ou le trifluoroacétimidate.

Les « oligo- ou polysaccharides structurellement proches » au sens de la présente description correspondent à un oligo- ou un polysaccharide modifié à partir de la liste L1, caractérisé par sa capacité à mimer immunologiquement le déterminant antigénique de l'O-SP de *S. flexneri,* en particulier *S. flexneri* type 3a ou X. De tels oligo- ou polysaccharides modifiés peuvent être obtenus par modification de structure qui rendent les polysaccharides modifiés antigéniquement similaires au déterminant antigénique de l'O-SP de *S. flexneri* 3a ou X. De tels oligo- ou polysaccharides modifiés peuvent être obtenus par exemple à l'aide d'un espaceur spécifique qui permet de mettre ces oligosaccharides dans la même conformation que l'O-SP natif.

Le terme « immunoréactif » signifie une liaison spécifique entre une molécule contenant un déterminant antigénique et une molécule contenant un anticorps.

Un anticorps correspond aux immunoglobulines ou à leurs fragments comprenant un site de reconnaissance et un site de liaison à l'antigène. Des anticorps sont soit des immunoglobulines entières soit des fragments actifs tels que les fragments ab, ab', (ab')₂ et scv, ainsi que les anticorps chimériques.

L'expression « immunologiquement similaire » se réfère à la capacité de l'oligo- ou du polysaccharide selon la description d'immuno-réagir ou de se lier à un anticorps tel que défini dans la présente description qui reconnaît et se lie à un déterminant antigénique natif de l'O-SP de *S. flexneri* de type 3a ou X.

Le terme «molécule M de support » se réfère à n'importe quelle molécule qui peut être liée de manière covalente à un oligo ou à un polysaccharide de la description, pour former les glycoconjugués de la description. Il inclut différentes molécules de support (carriers) pour leur utilisation comme vaccin ou pour préparer des réactifs de diagnostic.

Le terme « immunocarrier » se réfère à une molécule immunogénique ou à un fragment de celle-ci qui est reconnue par les cellules T et est capables d'induire une réponse immune.

Par « autres molécules M pour la préparation de réactif de diagnostic », on entend, au sens de la présente description, des agents habituellement utilisés pour immobiliser les molécules sur un support solide ou pour marquer les molécules.

Un marqueur se réfère à n'importe quelle substance qui peut produire un signal qui peut être détecté par n'importe quel moyen.

Le terme glycoconjugué se réfère à un oligo- ou un polysaccharide de la liste L1 lié de manière covalente à une molécule M de support (carrier).

Par « prévention et traitement », on entend, au sens de la présente description, la prévention de l'infection ou de la réinfection, la réduction ou l'élimination des symptômes et la réduction ou la complète élimination du pathogène. Le traitement peut être effectué de manière prophylactique ou thérapeutique.

Les oligo- et polysaccharides de la description sont dits conjugués ou glycoconjugués lorsqu'ils sont liés de manière covalente à une molécule M de support.

Dans le cadre des procédés décrits ci-après, par clivage, on entend indifféremment une réaction de déprotection par désacétalation ou hydrolyse acide.

Dans le cadre de la présente description et sans autre précision, C représente un résidu α-L-Rha*p*-(1,3) acétylé ou non.

Selon un mode de réalisation avantageux desdits conjugués, ils sont associés à une molécule M sélectionnée dans le groupe constitué par des peptides et des protéines comprenant au moins un épitope T ou un dérivé de celui-ci.

Selon un autre mode de réalisation desdits conjugués, la molécule M de support est sélectionnée dans le groupe constitué par la protéine IpaD, le peptide PADRE, la toxine tétanique, la protéine KLH ou la protéine CRM₁₉₇.

L'oligo- ou le polysaccharide est de préférence conjugué à une molécule M, par l'intermédiaire d'une molécule de liaison ou d'un agent de réticulation, qui est de préférence une molécule linéaire ayant un poids moléculaire > 500 daltons, laquelle molécule n'est ni pyrogénique ni toxique dans la forme finale du produit.

Pour la conjugaison par l'intermédiaire d'une molécule de liaison, soit l'oligo- ou le polysaccharide, soit la molécule M, soit les deux sont tout d'abord liés de manière covalente à ladite molécule de liaison.

Ladite molécule de liaison est avantageusement une molécule homobifonctionnelle ou hétérobifonctionnelle (Bioconjugatte techniques, G.T. Hermanson, Ed., Academic Press San Diego, 1995), e.g., dihydrazide adipique, éthylènediamine, cystamine, N-succinimidyl-3-(2-pyridyldithio) propionate (SPDP), N-succinimidyl-[N-(2-iodoacétyl)]-β-alanyl propionate-propionate (SIAP), succinimidyl-4-(N-maléimido-méthyl) cyclohexane-1 carboxylate (SMCC), acide 3,3'-dithiodipropionique. Parmi les molécules de liaison hétérobifonctionnelles, on peut citer les acides oméga-hydroxy alcanoïques.

Les méthodes pour lier des oligo et/ou des polysaccharides à des protéines non toxiques sont bien connues dans l'art. Par exemple dans les Brevet US 5,204,098 et US 5,738,855, il est précisé qu'un saccharide contenant au moins un groupe carboxylique à l'aide d'une condensation carbodiimide peut être thiolaté avec de la cystamine ou aminaté avec un dihydrazide adipique, des diaminoesters ou de l'éthylènediamine. Les groupes qui peuvent être introduits par cette méthode ou par d'autres méthodes connues dans l'art incluent les groupes suivants : thiols, hydrazides, aminés et acides carboxyliques. Aussi bien les intermédiaires thiolatés que les intermédiaires aminatés sont stables, peuvent être lyophilisés et stockés au froid. L'intermédiaire thiolaté peut être réduit et lié de manière covalente à une molécule M de support polymérique contenant un groupe disulfide tel qu'un groupe 2-pyridyldithio. L'intermédiaire aminaté peut être lié de manière covalente à une molécule M de support polymérique contenant un groupe carboxylique par une réaction de condensation carbodiimide.

Les saccharides peuvent être liés de manière covalente à la molécule M de support avec ou sans molécule de liaison ou espaceur. Une molécule M de support peut être une molécule naturelle, modifiée, synthétique, semi- synthétique ou recombinante et contient un ou plusieurs groupes fonctionnels, par exemple des groupes amino primaires ou secondaires, des groupes azido ou des groupes carboxyle. La molécule M de support peut être soluble dans l'eau ou non. Les molécules M de support immunogènes sont choisies pour accroître l'immunogénicité du saccharide et/ou pour induire la production d'anticorps contre ladite molécule M de support qui sont intéressants d'un point de vue médical.

Les molécules M de support immunogènes incluent des protéines, des peptides, des polysaccharides, des acides polylactiques, des acides polyglycoliques, des agrégats lipidiques et des particules virales inactivées.

Lesdits conjugués peuvent avantageusement être dérivatisés avec des groupes fonctionnels, de manière à les rendre capables de se lier à des ligands chimiques tels que la biotine ou des supports physiques tels que des plaques, billes ou particules (particules de métal, telles que particules d'or ou autres).

Lesdits conjugués peuvent réagir avec la molécule M via le résidu saccharidique soit directement, soit par l'intermédiaire d'un espaceur, qui peut être monovalent ou polyvalent (i.e. comprenant plusieurs copies dudit résidu saccharidique).

Selon un autre mode de réalisation avantageux dudit conjugué, le rapport saccharide:molécule M est compris entre 1:1 et 200:1, de préférence entre 30:1 et 10:1.

La présente description se rapporte également à des conjugués, caractérisé en ce que le saccharide est sélectionné dans le groupe constitué par les tétrasaccharides, les pentasaccharides et les multimères de ces derniers respectant la séquence de l'enchaînement des saccharides naturels des séroptypes 3a et X, à savoir -ABCD-.

Selon un autre mode de réalisation, ledit saccharide est sélectionné dans le groupe constitué par :
[CD(E)A], [D(E)AB], [(E)ABC], [BCD(E)A], [CD(E)AB], [D(E)ABC], [(E)ABCD], [BCD(E)A]ₙ, [CD(E)AB]ₙ, [D(E)ABC]ₙ, [(E)ABCD]ₙ, [BCD(E)AB], [D(E)ABCD], [D(E)ABCD(E)A], [BCD(E)ABCD], [BCD(E)ABCD(E)A], [D(E)ABCD(E)ABC], avec n étant compris entre 2 et 5.

La présente description comprend également les dérivés saccharidiques sélectionnés dans le groupe constitué par :

{D(E)A}ₙ-WQ

{(E)AB}ₙ-WQ

{CD(E)A}ₙ-WQ

{D(E)AB}ₙ-WQ

{(E)ABC}ₙ-WQ

{BCD(E)A}ₙ-WQ

{CD(E)AB}ₙ-WQ

{D(E)ABC}ₙ-WQ

{(E)ABCD}ₙ-WQ

dans lequel A, B, C D, E et n ont la même définition qu'à la revendication 1, W est un atome d'oxygène ou de soufre et Q représente l'un des groupes suivants : alkyle, alkényle, acyle, ou un groupe donneur comprenant de 1 à 12 atomes de carbone, éventuellement fonctionnalisé.

La présente description inclut également les saccharides de la liste L1 comprenant un espaceur du type O-R-Z et de préférence du type O-R-NH₂ (Z=NH₂), dans lesquels R représente un groupe alkyle comprenant de 1 à 12 atomes de carbone et de préférence de 1 à 6 atomes de carbone et Z est un groupe fonctionnel. On obtient ainsi à partir de la liste L1, les saccharides dérivatisés suivants :

{D(E)A}ₘ-O-R-Z

{(E)AB}ₘ-O-R-Z

{CD(E)A}ₘ-O-R-Z

{D(E)AB}ₘ-O-R-Z

{(E)ABC}ₘ-O-R-Z

{BCD(E)A}ₙ-O-R-Z

{CD(E)AB}ₙ-O-R-Z

{D(E)ABC}ₙ-O-R-Z

{(E)ABCD}ₙ-O-R-Z

Le groupe fonctionnel peut être masqué, par exemple, un S-acétyle ou un pont disulfure pour générer -SH qui n'est pas isolé mais qui est l'espèce active. A partir de l'allyle, le passage en aldéhyde est aussi envisageable.

La présente description est également relative à une composition immunogène comprenant un conjugué tel que défini ci-dessus dans un excipient pharmaceutiquement acceptable, c'est-à-dire acceptable d'un point de vue physiologique.

Le conjugué comprend notamment des pentasaccharides tels que ceux compris dans la liste L1 ainsi que les multimère de ces pentasaccharides.

Ladite composition immunogène comprend avantageusement en outre un immunogène capable de protéger contre un autre agent pathogène, tel que *S. flexneri* de sérotype 1b, 2a et 6 ou d'autres espèces de *Shigella,* telles que *S. dysenteriae* et *S. sonnei* ou d'autres pathogènes responsables de diarrhées chez l'homme.

### Vaccins

Les compositions immunogènes selon la description utilisées comme vaccins incluent un ou plusieurs véhicules pharmaceutiques tels que l'eau, le sérum physiologique, le glycérol et l'éthanol. De surcroît, des substances auxiliaires telles que des agents de mouillage ou des agents émulsifiants, des substances tampons et d'autres peuvent être également utilisées comme excipients.

Les compositions immunogènes qui induisent des anticorps protecteurs contre une infection à *S. flexneri,* en particulier *S. flexneri* de sérotype 3a ou X sont administrées à un sujet, de préférence à un humain en quantité suffisante pour prévenir ou atténuer la sévérité ou la durée de l'infection par *S. flexneri,* en particulier *S. flexneri* de sérotype 3a ou X.

Chaque dose vaccinale comprend une quantité effective de conjugué selon la description. Une telle quantité varie selon le sujet à traiter, l'âge et les conditions générales du sujet à traiter, la capacité de la réponse immunitaire du sujet à traiter, le degré de protection désiré, la sévérité de la condition à traiter, le conjugué sélectionné et son mode d'administration, parmi d'autres facteurs. Une quantité efficace appropriée peut être déterminée aisément par l'homme du métier.

Plus particulièrement, le conjugué selon la description peut être administré en quantité thérapeutiquement efficace comprise entre 1 et 1000 µg de saccharide, de préférence entre 1 et 50 µg.

Une quantité optimale pour un vaccin particulier peut être mise au point par des études standard impliquant la mesure du titre en anticorps anti-LPS 3a ou X chez les sujets.

Suite à une première vaccination, les sujets peuvent recevoir une ou deux injections de rappel à environ 4 semaines d'intervalle.

La composition immunogène selon la description peut être administrée avec ou sans adjuvant. Les adjuvants peuvent être ajoutés directement à la composition vaccinale ou bien être administrés de manière séparée soit simultanément soit après l'administration du vaccin. De tels adjuvants inclus mais ne sont pas limités aux sels d'aluminium (hydroxyde d'aluminium) mais incluent également les émulsions huile dans eau avec ou sans agents de stimulation spécifique tels que : muramyl peptides, saponine, cytokines, mutants détoxifiés de toxines bactériennes tels que la toxine cholérique, la toxine de *Pertussis* ou la toxine d'*E*. *Coli* sensible à la chaleur.

La composition immunogène selon la description peut être administrée avec d'autres immunogènes ou agents immuno régulateurs, par exemple des immunoglobulines, des cytokines, des lymphokines et des chemokines.

La description a encore pour objet un procédé de préparation d'un conjugué selon la liste L1, caractérisé en ce qu'il comprend la réaction covalente d'une molécule saccharide-ORZ, telle que définie ci-dessus avec une molécule M de support convenable.

Selon un mode de mise en oeuvre avantageux dudit procédé, la réaction covalente comprend une activation préalable de la molécule M.

Selon un autre mode de mise en oeuvre avantageux dudit procédé, il comprend les étapes suivantes :
a) la conjugaison d'un saccharide-ORZ, tel que défini ci-dessus avec un espaceur pour obtenir une molécule dérivatisée contenant ledit espaceur, et
b) la réaction de la molécule dérivatisée et comprenant l'espaceur avec la molécule M.

En variante, ledit procédé comprend les étapes suivantes :
a) la conjugaison d'une molécule M convenable à un espaceur pour obtenir une molécule M de support dérivatisée comprenant l'espaceur,
b) la réaction de ladite molécule M de support dérivatisée avec un saccharide-ORZ, tel que défini ci-dessus.

Selon un mode de mise en oeuvre avantageux, l'espaceur est une petite molécule ayant un poids moléculaire inférieur à 500 daltons, soit homobifonctionnelle, soit hétéro-bifonctionnelle.

La présente description a également pour objet un anticorps monoclonal IgG immunoréactif avec un le LPS de *S. flexneri* de sérotype 3a ou un anticorps monoclonal IgG immunoréactif avec un le LPS de *S. flexneri* de sérotype X.

La présente description a également pour objet un polynucléotide codant pour la chaîne légère et/ou la chaîne lourde d'un anticorps tel que défini ci-dessus ou un fragment de celui-ci.

La présente description a également pour objet un vecteur d'expression comprenant le polynucléotide tel que défini ci-dessus.

La présente description a également pour objet une cellule hôte modifiée par un polynucléotide ou un vecteur tels que définis ci-dessus.

La présente description a en outre pour objet un animal transgénique non humain ou une plante transgénique, caractérisés en ce que tout ou une partie des cellules sont modifiées par un polynucléotide ou un vecteur tels que définis ci-dessus.

### Protection passive

La description est également relative à une composition pharmaceutique comprenant un anticorps tel que défini ci-dessus ou un fragment fonctionnel de celui-ci et un véhicule ou excipient pharmaceutiquement acceptable.

Les anticorps selon la présente description qui ont un effet protecteur contre une infection à *S. flexneri,* en particulier *S. flexneri* type 3a ou X sont administrés chez un sujet, de préférence un homme, en quantité suffisante pour prévenir ou atténuer la sévérité ou la durée de l'infection par *S. flexneri,* en particulier *S. flexneri* type 3a ou X.

L'administration de l'anticorps peut être soit prophylactique (avant toute exposition à *S. flexneri*) ou thérapeutique (avant l'infection ou tout au début de l'infection).

Les doses d'anticorps varient en fonction de différents facteurs tels que l'âge du sujet et son poids. En général, les doses en anticorps sont comprises entre 1 mg/kg et 10mg/kg.

De préférence, l'anticorps est un anticorps de classe IgG.

La voie d'administration de l'anticorps peut être soit orale soit systémique, par exemple sous cutanée, intra musculaire ou intra veineuse.

### Diagnostic

Les anticorps et les saccharides selon la présente description sont utilisés *in vitro* comme réactifs de diagnostic spécifique de *S. flexneri* type 3a ou X dans des immunoessais standards.

Les anticorps selon la présente description sont utilisés pour tester la présence de *S. flexneri* type 3a ou X dans des échantillons biologiques pour établir le diagnostic de Shigellose chez un individu présentant des symptômes diarrhéique.

Alternativement, les saccharides selon la présente description peuvent être utilisés pour tester la présence d'anticorps anti *S. flexneri* type 3a ou X. Ces saccharides peuvent être utilisés pour des études épidémiologiques, par exemple pour déterminer la distribution géographique ou l'évolution des infections à *S. flexneri* type 3a ou X dans le monde ainsi que pour évaluer la réponse en anticorps induite par un immunogène.

Les anticorps et les saccharides selon la présente description peuvent avantageusement être marqués et/ou immobilisés sur un support solide selon des protocoles standards connus de l'homme du métier. De tels marqueurs incluent mais ne sont pas limités à des enzymes (phosphatase alcaline, peroxydase), molécules luminescentes ou molécules fluorescentes. Par exemple, les saccharides selon la description conjugués à la biotine peuvent être mmobilisés sur un support solide pour détecter la présence d'anticorps anti *S. flexneri* type 3a ou X dans des échantillons biologiques.

De tels immunoessais incluent mais ne sont pas limités à des essais d'agglutination, des radioimmunoessais, des ELISA, des essais de fluorescence, des westerns blots etc....

Ainsi, la description a encore pour objet un kit de diagnostic d'une infection à *Shigella flexneri* de sérotype 3a ou X, caractérisé en ce qu'il comprend au moins un conjugué ou un saccharide tels que définis ci-dessus.

La description a également pour objet un intermédiaire pour la préparation d'un oligo- ou polysaccharide de la liste L1, caractérisé en ce qu'il est sélectionné dans la liste L2 suivante :
- Trichloracétimidate de 3-*O*-acétyl-2-désoxy-4,6-*O*-isopropylidène-2-trichloroacétamido-α/β-D-glucopyranose **2**,
- Trichloracétimidate de 2-désoxy-4,6-*O-*sopropylidène-3-*O-*lévulinoyl-2-trichloroacétamido-α-D-glucopyranose **3**,
- Allyl 2-désoxy-4,6-*O*-isopropylidène-2-trichloroacétamido-β-D-glucopyranoside **20**,
- Allyl (3,4,6-tri-*O*-acétyl-2-désoxy-2-trichloroacétainido-β-D-glucopyranosyl)-(1→2)-[2,3,4,6-tétra-*O*-benzyl-α-D-glucopyranosyl-(1→3)]-4-*O-*benzyl-α-L-rhamnopyranoside **22**,
- Allyl (3-*O*-acétyl-2-désoxy-4,6-*O*-isopropylidène-2-trichloroacétamido-β-D-glucopyranosyl)-(1→2)-[2,3,4,6-tétra-*O*-benzyl-α-D-glucopyranosyl-(1→3)]-4-*O*-benzyl-α-L-rhamnopyranoside **23**,
- Trichloracétimidate de 2,3,4,6-tetra-*O*-benzyl-α/β-D-glucopyranose **33**,
- Allyl (2,3,4,6-tétra-*O*-benzyl-α-D-glucopyranosyl)-(1→3)-2-*O-*acétyl-4-*O*-benzyl-α-L-rhamnopyranoside **35**,
- Allyl (2-désoxy-2-trichloroacétamido-β-D-glucopyranosyl)-(1→2)-[2,3,4,6-tétra-*O*-benzyl-α-D-glucopyranosyl-(1→3)]-4-*O*-benzyl-α-L-rhamnopyranoside **37**,
- Allyl (2-désoxy-4,6-*O*-isopropylidène-2-trichloroacétamido-β-D-glucopyranosyl)-(1→2)-[2,3,4,6-tétra-*O*-benzyl-α-D-glucopyranosyl-(1→3)]-4-*O-*benzyl-α-L-rhamnopyranoside **38**,
- Allyl (2-*O*-acétyl-3,4-di-*O*-benzyl-α-L-rhamnopyranosyl)-(1→3)-(2-désoxy-4,6-*O*-isopropylidène-2-trichloroacétamido-β-D-glucopyranosyl)-(1→2)-[2,3,4,6-tétra-*O*-benzyl-α-D-glucopyranosyl-(1→3)]-4-*O*-benzyl-α-L-rhamnopyranoside **40**,
- Allyl (2-*O*-acétyl-3,4-di-*O*-benzyl-α-L-rhamnopyranosyl)-(1→3)-(2-désoxy-2-trichloroacétamido-β-D-glucopyranosyl)-(1→2)-[2,3,4,6-tétra-*O*-benzyl-α-D-glucopyranosyl-(1→3)]-4-*O*-benzyl-α-L-rhamnopyranoside **41**,
- Allyl (3,4-di-*O*-benzyl-α-L-rhamnopyranosyl)-(1→3)-(2-désoxy-2-trichloroacétamido-β-D-glucopyranosyl)-(1→2)-[2,3,4,6-tétra-*O*-benzyl-α-D-glucopyranosyl-(1→3)]-4-*O*-benzyl-α-L-rhamnopyranoside **69**,
- Tricholroacétimidate de (2,3,4-tri-*O*-acétyl-α-L-rhamnopyranosyl)-(1→3)-2-*O*-acétyl-4-*O*-benzyl-α/β-L-rhamnopyranose **46,**
- Trichloroacétimidate de (3,4-di-*O*-benzyl-2-*O*-lévulinoyl-α-L-rhamnopyranosyl)-(1→3)-2-*O*-acétyl-4-*O*-benzyl-α/β-L-rhamnopyranose **47**,
- Allyl (2,3,4-tri-*O*-acétyl-α-L-rhamnopyranosyl)-(1→3)-(2-*O-*acétyl-4-*O*-benzyl-α-L-rhamnopyranosyl)-(1→3)-(2-désoxy-4,6-*O*-isopropylidène-2-trichloroacétamido-β-D-glucopyranosyl)-(1→2)-[2,3,4,6-tétra-*O*-benzyl-α-D-glucopyranosyl-(1→3)]-4-*O*-benzyl-α-L-rhamnopyranoside **52,**
- Allyl α-L-rhamnopyranosyl-(1→3)-(2-*O*-acétyl-4-*O*-benzyl-α-L-rhamnopyranosyl)-(1→3)-(2-désoxy-4,6-*O*-isopropylidène-2-trichloroacétamido-β-D-glucopyranosyl)-(1→2)-[2,3,4,6-tétra-*O*-benzyl-α-D-glucopyranosyl-(1→3)]-4-*O-*benzyl-α-L-rhamnopyranoside **54**,
- Allyl α-L-rhamnopyranosyl-(1→3)-(4-*O*-benzyl-α-L-rhamnopyranosyl)-(1→3)-(2-désoxy-4,6-*O*-isopropylidène-2-trichloroacétamido-β-D-glucopyranosyl)-(1→2)-[2,3,4,6-tétra-*O*-benzyl-α-D-glucopyranosyl-(1→3)]-4-*O-*benzyl-α-L-rhamnopyranoside **55**,
- Allyl α-L-rhamnopyranosyl-(1→3)-(2-*O*-acétyl-4-*O*-benzyl-α-L-rhamnopyranosyl)-(1→3)-(2-désoxy-2-trichloroacétamido-β-D-glucopyranosyl)-(1→2)-[2,3,4,6-tétra-*O*-benzyl-α-D-glucopyranosyl-(1→3)]1-4-*O*-benzyl-α-L-rhamnopyranoside **56**,
- Allyl α-L-rhamnopyranosyl-(1→3)-(4-*O*-benzyl-α-L-rhamnopyranosyl)-(1→3)-(2-désoxy-2-trichloroacétamido-β-D-glucopyranosyl)-(1→2)-[2,3,4,6-tétra-*O*-benzyl-α-D-glucopyranosyl-(1→3)]-4-*O*-benzyl-α-L-rhamnopyranoside **57**,

Allyl (3,4-di-*O*-benzyl-2-*O*-lévulinoyl-α-L-rhamnopyranosyl)-(1→3)-(2-*O*-acétyl-4-*O*-benzyl-α-rhamnopyranoside **61**,

- Allyl (3,4-di-*O*-benzyl-2-*O*-lévulinoyl-α-L-rhamnopyranosyl)-(1→3)-(2-*O*-acétyl-4-*O*-benzyl-α-L-rhamnopyranosyl)-(1→3)-(2-désoxy-4,6-*O-*isopropylidène-2-trichloroacétamido-β-D-glucopyranosyl)-(1→2)-[2,3,4,6-tétra-*O-*benzyl-α-D-glucopyranosyl-(1→3)]-4-*O*-benzyl-α-L-rhamnopyranoside **64**,
- Allyl (3,4-di-*O*-benzyl-α-L-rhamnopyranosyl)-(1→3)-(2-*O*-acétyl-4-*O*-benzyl-α-L-rhamnopyranosyl)-(1→3)-(2-désoxy-4,6-*O*-isopropylidène-2-trichloroacétamido-β-D-glucopyranosyl)-(1→2)-[2,3,4,6-tétra-*O*-benzyl-α-D-glucopyranosyl-(1→3)]-4-*O*-benzyl-α-L-rhamnopyranoside **66**,
- Allyl (3,4-di-*O*-benzyl-α-L-rhamnopyranosyl)-(1→3)-(2-*O*-acétyl-4-*O*-benzyl-α-L-rhamnopyranosyl)-(1→3)-(2-désoxy-2-trichloroacétamido-β-D-glucopyranosyl)-(1→2)-[2,3,4,6-tétra-*O*-benzyl-α-D-glucopyranosyl-(1→3)]-4-*O-*benzyl-α-L-rhamnopyranoside **67**,
- Allyl (3,4-di-*O*-benzyl-α-L-rhamnopyranosyl)-(1→3)-2-*O*-acétyl-4-*O*-benzyl-α-L-rhamnopyranoside **79**,
- Trichloracétimidate de (2,3,4,6-tétra-*O*-benzyl-α-D-glucopyranosyl)-(1→3)-4-*O*-benzyl-2-*O*-lévulinoyl-α/β-L-rhamnopyranose **80**,
- Trichloracétimidate de (2,3,4,6-tétra-*O*-benzyl-α-D-glucopyranosyl)-(1→3)-2-*O*-benzoyl-4-*O*-benzyl-α/β-L-rhamnopyranose **80'**,
- Allyl (2,3,4,6-tétra-*O*-benzyl-α-D-glucopyranosyl)-(1→3)-(4-*O-*benzyl-α-L-rhamnopyranosyl)-(1→2)-(3,4-di-*O*-beazyl-α-L-rhamnopyranosyl)-(1→3)-2-*O*-acétyl-4-*O*-benzyl-α-L-rhamnopyranoside **85**,
- Trichloroacétimidate (2,3,4,6-tétra-*O*-benzyl-α-D-glucopyranosyl)-(1→3)-(4-*O*-benzyl-2-*O*-lévulinoyl-α-L-rhamnopyranosyl)-(1-2)-(3,4-di-*O*-benzyl-α-L-rhamnopyranosyl)-(1→3)-2-*O*-acétyl-4-*O*-benzyl-α/β-L-rhamnopyranose **86**,
- Allyl (2,3,4,6-tétra-*O*-benzyl-α-D-glucopyranosyl)-(1→3)-(4-*O-*benzyl-2-*O*-lévulinoyl-α-L-rhamnopyranosyl)-(1→2)-(3,4-di-*O*-benzyl-α-L-rhamnopyranosyl)-(1→3)-2-*O*-acétyl-4-*O*-benzyl-α-L-rhamnopyranoside **87**,
- Allyl (2,3,4,6-tétra-*O*-benzyl-α-D-glucopyranosyl)-(1→3)-(4-*O-*benzyl-α-L-rhamnopyranosyl)-(1→2)-(3,4-di-*O*-benzyl-α-L-rhamnopyranosyl)-(1→3)-4-*O*-benzyl-α-L-rhamnopyranoside **89**,
- Allyl (3,4,6-tri-*O*-acetyl-2-désoxy-2-trichloroaeétamido-β-D-glucopyranosyl)-(1→2)-[2,3,4,6-tétra-*O*-benzyl-α-D-glucopyranosyl-(1→3)]-(4-*O-*benzyl-α-L-rhamnopyranosyl)-(1→2)-(3,4-di-*O*-benzyl-α-L-rhamnopyranosyl)-(1→3)-2-*O*-acétyl-4-*O*-benzyl-α-L-rhamnopyranoside **90**,
- Allyl (2-désoxy-2-trichloroacétamido-β-D-glucopyranosyl)-(1→2)-[2,3,4,6-tétra-*O*-benzyl-α-D-glucopyranosyl-(1→3)]-(4-*O*-benzyl-α-L-rhamnopyranosyl)-(1→2)-(3,4-di-*O*-benzyl-α-L-rhamnopyranosyl)-(1→3)-2-*O-*acétyl-4-*O*-benzyl-α-L-rhamnopyranoside **91**,
- Allyl (2-désoxy-2-trichloroacétamido-β-D-glucopyranosyl)-(1→2)-[2,3,4,6-tétra-*O*-benzyl-α-D-glucopyranosyl-(1→3)]-(4-*O*-benzyl-α-L-rhamnopyranosyl)-(1→2)-(3,4-di-*O*-benzyl-α-L-rhamnopyranosyl)-(1→3)-4-*O-*benzyl-α-L-rhamnopyranoside **92**,
- Allyl (2,3,4,6-tétra-*O*-benzyl-α-D-glucopyranosyl)-(1→3)-(4-*O-*benzyl-2*-*O-lévulinoyl-α-L-rhamnopyranosyl)-(1→2)-(3,4-di-*O*-benzyl-α-L-rhamnopyranosyl)-(1→3)-(2-*O*-acétyl-4-*O*-benzyl-α-L-rhamnopyranosyl)-(1→3)-2-acétamido-2-désoxy-4,6-(*O*-isopropylidène-β-D-glucopyranoside **93**,
- Allyl (2,3,4,6-tétra-*O*-benzyl-α-D-glucopyranosyl)-(1→3)-(4-*O-*benzyl-α-L-rhamnopyranosyl)-(1→2)-(3,4-di-*O*-benzyl-α-L-rhamnopyranosyl)-(1→3)-(2-*O*-acétyl-4-*O*-benzyl-α-L-rhamnopyranosyl)-(1→3)-2-acétamido-2-désoxy-4,6-*O*-isopropylidène-β-D-glucopyranoside **94**,
- Allyl (2,3,4,6-tétra-*O*-benzyl-α-D-glucopyranosyl)-(1→3)-(4-*O-*benzyl-α-L-rhamnopyranosyl)-(1→2)-(3,4-di-*O*-benzyl-α-L-rhamnopyranosyl)-(1→3)-(2-*O*-acétyl-4-*O*-benzyl-α-L-rhamnopyranosyl)-(1→3)-2-acétamido-2-désoxy-β-D-glucopyranoside **95**,
- Allyl (2,3,4,6-tétra-*O*-benzyl-α-D-glucopyranosyl)-(1→3)-(4-*O-*benzyl-α-L-rhamnopyranosyl)-(1→2)-(3,4-di-*O*-benzyl-α-L-rhamnopyranosyl)-(1→3)-(4-*O*-benzyl-α-L-rhamnopyranosyl)-(1→3)-2-acétamido-2-désoxy-4,6-*O-*isopropylidène-β-D-glucopyranoside **96**,
- Allyl (2,3,4,6-tétra-*O*-benzyl-α-D-glucopyranosyl)-(1→3)-(4-*O-*benzyl-α-L-rhamnopyranosyl)-(1→2)-(3,4-di-*O*-benzyl-α-L-rhamnopyranosyl)-(1→3)-(4-*O*-benzyl-α-L-rhamnopyranosyl)-(1→3)-2-acétamido-2-désoxy-β-D-glucopyranoside **97**,
- Allyl (2,3,4,6-tétra-*O*-benzyl-α-D-glucopyranosyl)-(1→3)-(4-*O-*benzyl-2-*O*-lévulinoyl-α-L-rhamnopyranosyl)-(1→2)-3,4-di-*O*-benzyl-α-L-rhamnopyranoside **107**,
- Allyl (2,3,4,6-tétra-*O*-benzyl-α-D-glucopyranosyl)-(1→3)-(2-*O-*benzoyl-4-*O*-benzyl-α-L-rhamnopyranosyl)-(1→2)-3,4-di-*O*-benzyl-α-L-rhamnopyranoside **107'**,
- Allyl (2,3,4,6-tétra-*O*-benzyl-α-D-glucopyranosyl)-(1→3)-(4-*O-*benzyl-α-L-rhamnopyranosyl)-(1→2)-3,4-di-*O*-benzyl-α-L-rhamnopyranoside **108**,
- Allyl (2-désoxy-4,6-*O*-isopropylidène-2-trichloroacétamido-β-D-glucopyranosyl)-(1→2)-[2,3,4,6-tétra-*O*-benzyl-α-D-glucopyranosyl-(1→3)]-(4-*O-*benzyl-α-L-rhamnopyranosyl)-(1→2)-3,4-di-*O*-benzyl-α-L-rhamnopyranoside **110**,
- Allyl (3-*O*-acétyl-2-désoxy-4,6-*O*-isopropylidène-2-trichloroacétamido-β-D-glucopyranosyl)-(1→2)-[2,3,4,6-tétra-*O*-benzyl-α-D-glucopyranosyl-(1→3)]-(4-*O*-benzyl-α-L-rhamnopyranosyl)-(1→2)-3,4-di-*O*-benzyl-α-L-rhamnopyranoside **115**,
- Allyl (2-désoxy-2,3,4-tri-*O*-acétyl-2-trichloroacétamido-β-D-glucopyranosyl)-(1→2)-[2,3,4,6-tétra-*O*-benzyl-α-D-glucopyranosyl-(1→3)]-(4-*O-*benzyl-α-L-rhamnopyranosyl)-(1→2)-3,4-di-*O*-benzyl-α-L-rhamnopyranoside **115'**,
- Allyl (2-désoxy-2-trichloroacétamido-β-D-glucopyranosyl)-1→2,3,4,6-tétra-*O-*benzyl-α-D-glucopyranosyl-(1→3)]-(4-*O*-benzyl-α-L-rhamnopyranosyl)-(1→2)-3,4-di-*O*-benzyl-α-L-rhamnopyranoside **115"**
- Allyl (2-désoxy-4,6-*O*-isopropylidène-2-acétamido-β-D-glucopyranosyl)-(1→2)-[2,3,4,6-tétra-*O*-benzyl-α-D-glucopyranosyl-(1→3)]-(4-*O-*benzyl-α-L-rhamnopyranosyl)-(1→2)-3,4-di-*O*-benzyl-α-L-rhamnopyranoside **116**,
- Allyl 2-*O*-acetyl-3,4-di-*O*-benzyl-α-L-rhamnopyranoside-(1→3)-(2-désoxy-4,6-*O*-isopropylidène-2-acétamido-β-D-glucopyranosyl)-(1→2)-[2,3,4,6-tétra-*O*-benzyl-α-D-glucopyranosyl-(1→3)1-(4-*O*-benzyl-α-L-rhamnopyranosyl)-(1→2)-3,4-di-*O*-benzyl-α-L-rhamnopyranoside **116'**,
- Allyl 2-*O*-acetyl-3,4-di-*O*-benzyl-α-L-rhamnopyranoside-(1→3)-(2-désoxy-2-acétamido-β-D-glucopyranosyl)-(1→2)-[2,3,4,6-tétra-*O*-benzyl-α-D-glucopyranosyl-(1→3)]-(4-*O*-benzyl-α-L-rhamnopyranosyl)-(1→2)-3,4-di-*O*-benzyl-α-L-rhamnopyranoside **116"**,
- Allyl (3,4-di-*O-*benzyl-2-*O*-lévulinoyl-α-L-rhamnopyranosyl)-(1→3)-2-*O*-acétyl-4-*O*-benzyl-α-L-rhamnopyranosyl)-(1→3)-(2-désoxy-4,6-*O-*isopropylidène-2-trichloroacétamido-β-D-glucopyranosyl)-(1→2)-[2,3,4,6-tétra-*O-*benzyl-α-D-glucopyranosyl-(1→3)]-(4-*O*-benzyl-α-L-rhamnopyranosyl)-(1→2)-3,4-di-*O*-benzyl-α-L-rhamnopyranoside **122**,
- Allyl (3,4-di-*O*-benzyl-2-*O*-lévulinoyl-α-L-rhamnopyranosyl)-(1→3)-2-*O*-acétyl-4-*O*-benzyl-α-L-rhamnopyranosyl)-(1→3)-(2-désoxy-2-trichloroacétamido-β-D-glucopyranosyl)-(1→2)-[2,3,4,6-tétra-*O*-benzyl-α-D-glucopyranosyl-(1→3)]-(4-*O*-benzyl-α-L-rhamnopyranosyl)-(1→2)-3,4-di-*O*-benzyl-α-L-rhamnopyranoside **123**,
- Allyl (3,4-di-*O*-benzyl-2-α-L-rhamnopyranosyl)-(1→3)-2-*O-*acétyl-4-*O*-benzyl-α-L-rhamnopyranosyl)-(1→3)-(2-désoxy-2-trichloroacétamido-β-D-glucopyranosyl)-(1→2)-[2,3,4,6-tétra-*O*-benzyl-α-D-glucopyranosyl-(1→3)]-(4-*O-*benzyl-α-L-rhamnopyranosyl)-(1→2)-3,4-di-*O*-benzyl-α-L-rhamnopyranoside **124**,
- Allyl (3,4-di-*O*-benzyl-2-α-L-rhamnopyranosyl)-(1→3)-4-*O-*benzyl-α-L-rhamnopyranosyl)-(1→3)-(2-désoxy-2-trichloroacétamido-β-D-glucopyranosyl)-(1→2)-[2,3,4,6-tétra-*O*-benzyl-α-D-glucopyranosyl-(1→3)]-(4-*O-*benzyl-α-L-rhamnopyranosyl)-(1→2)-3,4-di-*O*-benzyl-α-L-rhamnopyranoside **125**,
- Allyl (2,3,4,6-tétra-*O*-benzyl-α-D-glucopyranosyl)-(1→3)-(4-*O-*benzyl-2-*O*-lévulinoyl-α-L-rhamnopyranosyl)-(1→2)-(3,4-di-*O*-benzyl-α-L-rhamnopyranosyl)-(1→3)-(2-*O*-acétyl-4-*O*-benzyl-α-L-rhamnopyranosyl)-(1→3)-(2-désoxy-4,6-*O*-isopropylidène-2-trichloroacétamido-β-D-glucopyranosyl)-(1→2)-[2,3,4,6-tétra-*O*-benzyl-α-D-glucopyranosyl-(1→3)]-4-*O*-benzyl-α-L-rhamnopyranoside **126**,
- Allyl (2,3,4,6-tétra-*O*-benzyl-α-D-glucopyranosyl)-(1→3)-(4-*O-*benzyl-α-L-rhamnopyranosyl)-(1→2)-(3,4-di-*O*-benzyl-α-L-rhamnopyranosyl)-(1→3)-(2-*O*-acétyl-4-*O*-benzyl-α-L-rhamnopyranosyl)-(1→3)-(2-désoxy-4,6-*O-*isopropylidène-2-trichloroacétamido-β-D-glucopyranosyl)-(1→2)-[2,3,4,6-tétra-*O-*benzyl-α-D-glucopyranosyl-(1→3)]-4-*O*-benzyl-α-L-rhamnopyranoside **127**,
- Allyl (2-désoxy-4,6-*O*-isopropylidène-3-*O*-lévulinoyl-2-trichloroacétamido-β-D-glucopyranose)-(1→2)-[2,3,4,6-tétra-*O*-benzyl-α-D-glucopyranosy]-(1→3)]-(4-*O*-benzyl-α-L-rhamnopyranosyl)-(1→2)-(3,4-di-*O*-benzyl-α-L-rhamnopyranosyl)-(1→3)-(2-*O*-acétyl-4-*O*-benzyl-α-L-rhamnopyranosyl)-(1→3)-(2-désoxy-4,6-*O*-isopropylidène-2-trichloroacétamido-β-D-glucopyranosyl)-(1→2)-[2,3,4,6-tétra-*O*-benzyl-α-D-glucopyranosyl-(1→3)]-4-*O*-benzyl-α-L-rhamnopyranoside **128.**

La description se rapporte encore à un intermédiaire pour la préparation d'un saccharide tel que défini dans la liste L1, caractérisé en ce qu'il est sélectionné dans la liste L3 suivante :
- [CD(E)A] (III et IV),
- [D(E)AB] (VIII),
- [(E)ABC] (XI et XII),
- [BCD(E)A] (V et VI),
- [CD(E)AB] (IX et X),
- [D(E)ABC] (XIII et XIV),
- [(E)ABCD] (XV et XVI),
- [BCD(E)AB] (XVII et XVIII),
- [D(E)ABCD] (XXIII et XXIV),
- [D(E)ABCD(E)A] (XIX et XX),
- [BCD(E)ABCD] (XXV et XXVI),
- [BCD(E)ABCD(E)A] (XXI et XXII),
- [D(E)ABCD(E)ABC] (XXVII et XXVIII)

### Description détaillée du mode de préparation des saccharides selon la description

**Tableau 1 : Oligosaccharides représentatifs des fragments de l'antigène O**

| Disaccharides | Trisaccharides | Tétrasaccharides | Pentasaccharides et > |
|---|---|---|---|
| **(E)A *I*** | **D(E)A *II*** | **_{Ac}CD(E)A *III*** | **B_{Ac}CD(E)A *V*** |
| | | **CD(E)A *IV*** | **BCD(E)A *VI*** |
| | **(E)AB *VII*** | **D(E)AB *VIII*** | **_{Ac}CD(E)AB *IX*** |
| | | | **CD(E)AB *X*** |
| | | **(E)AB_{Ac}C *XI*** | **D(E)AB_{Ac}C *XIII*** |
| | | **(E)ABC *XII*** | **D(E)ABC *XIV*** |
| | | | **(E)AB_{Ac}CD *XV*** |
| | | | **(E)ABCD *XVI*** |
| | | | **B_{Ac}CD(E)AB *XVII*** |
| | | | **BCD(E)AB *XVIII*** |

Toutes les synthèses reposent sur l'utilisation de ces synthons clés réprésentés dans le schéma 1.

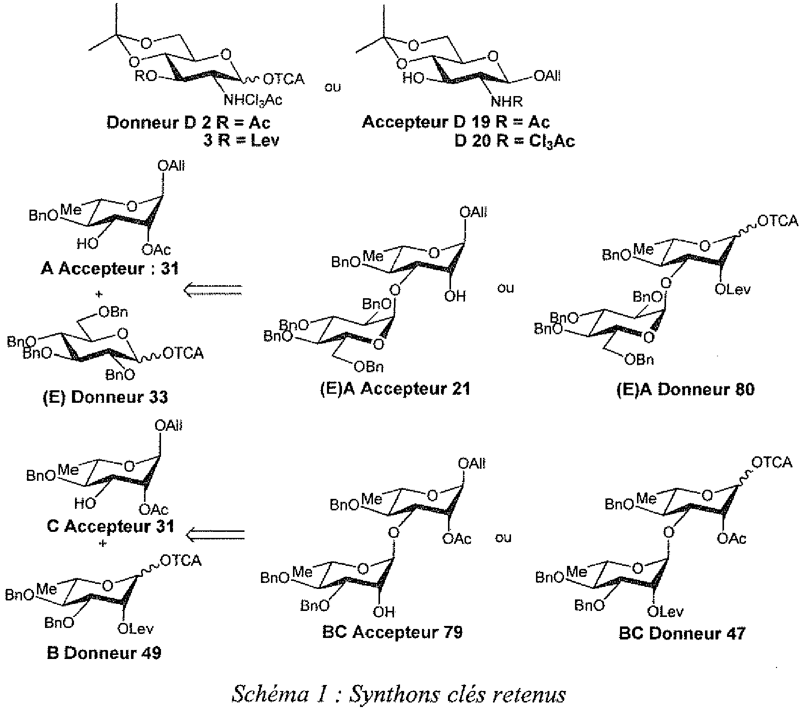

Ainsi les synthons accepteurs peuvent être allongés à leur extrémité non réductrice par incorporation itérative des monosaccharides ou disaccharides donneurs requis fonctionnalisés de façon appropriée. En outre, les glycosides d'allyle peuvent être allongés à leur extrémité réductrice après déallylation sélective et activation, permettant leur couplage sur les monosaccharides ou disaccharides accepteurs requis fonctionnalisés de façon appropriée.

Dans les procédés de synthèse qui suivent, les abréviations utilisées sont :
TA température ambiante
CCM : Chromatographie sur couche mince
Ac: Acétyle
C1]Ac : Trichloroacétyle
TCA : trichloroacétimidate
Bn: Benzyle
Bz : Benzoyle
Lev : Lévulinoyle
Et: Ethyle
Me: Méthyle
Pr: Propyle
iPr : isopropyle
DCM : Dichlorométhane
DCE : 1,2-dichloroéthane
Chex : Cyclohexane
Et₂O : Ether diéthylique
iPr₂O : Ether diisopropylique
EP : Ether de pétrole
ToI: Toluène
THF : Tétrahydrofurane
DMF : N,N-diméthylformamide
APTS : Acide *para-toluènesulfonique*
TfOH : Acide trifluorométhanesulfonique
TMSOTf : trifluorométhanesulfonate de triméthylsilyle
CSA : acide camphor sulfonique
HCI : acide chlorhydrique
DCC : Dicyclohexylcarbodiimide
DCU : dicyclohexylurée
DMAP : N-diméthylaminopyridine
Rf : Rapport frontal
DBU: 1,8-diazo-bicyclo(5.4.0)undec-7-ene
Glc : Glucose
Rha : Rhamnose
Litt. Littérature
éq. : équivalent
aq. : aqueux
quant. : Quantitatif
MS : Spectroscopie de Masse
HRMS : Spectroscopie de Masse Haute Résolution
ESI : Ionisation par Electrospray
HPLC : Chromatographie Liquide Haute Résolution
RMN: Résonance Magnétique Nucléaire
COSY: COrrelation Spectroscopy
DEPT : Distortionless Enhancement by Polarisation Transfer
HSQC : Heteronuclear Single Quantum Corrélation
HMBC : Heteronuclear Multiple Bond Corrélation
TOCSY : TOtal Corrélation Spectroscopy
ROESY : ROtative frame Overhauser Enhancement Spectroscopy
NOESY : Nuclear Overhauser Enhancement SpectroscopY
DOWEX (H+) : DOWEX SOWX8-200
Solution HCI à 10 %
NaHC03 : Hydrogènocarbonate de sodium
K2C03 : Carbonate de Potassium
Na2S04 : Sulfate de sodium
CS2C03 : Carbonate de césium
NaHS03 : Hydrogénosulfite de sodium
HCIlM ou SM
Solution de NaCl sat
Solution d'acide acétique 80% (pour dissach E(A)»
Solution aqueuse de TFA SO %

Les schémas auxquels les procédés font références sont indiqués à titre d'illustration d'un mode de réalisation particulier sans aucunement limiter les conditions de mise en oeuvre de ces procédés à ces conditions particulières.

### Partie I - Etape préalable de synthèse des synthons clés Donneurs de Glucosamine :

L'antigène O de *S. flexneri* 3a comporte une structure de type 3)-β-D-*p*GlcNHAc-(1. Comme indiqué dans la partie rétro synthèse les précurseurs donneurs retenus dérivent de la 4,6-*O*-isopropylidène-2-trichloroacétamido-D-glucosamine. Cette partie présente la synthèse des nouveaux donneurs **2** et **3.** Une voie d'accès alternative à celle publiée pour l'analogue **1**,¹ très utilisé au laboratoire, est également proposée.

### 1. Monosaccharide D donneur 1 et 2

L'objectif a été d'obtenir ces trois donneurs en grande quantité avec de bons rendements et en limitant le nombre d'étapes de purification. Il est a noté que différents accepteurs et quelques donneurs glucosaminyl comportant une fonction 4,6-*O*-isopropylidène² ont déjà été utilisés dans la synthèse d'analogues de lipide A³ ou de peptidoglycane⁴ mais plus rarement dans la synthèse d'oligosaccharides complexes. A notre connaissance, deux donneurs « trichloroacétimidate » dérivés de la glucosamine en série azido^{2,5} et un analogue en série 2-trichloréthoxycarbonyl⁶ ont déjà été décrits. En série trichloroacétamide, seul un donneur thiophényle a déjà été décrit.⁷

### a. Protection de la fonction amine

### Conditions pour introduire la fonction trichloroacétamide sélectionnée comme groupe participant.

Il était connu d'utiliser la méthode de Bergmann et Zervas passant par une imine.⁸⁻¹⁰ Le chlorhydrate de D-glucosamine **4** est mis en réaction avec de la soude et du *para-*anisaldéhyde pour former l'imine **5** (Schéma 2). Après acétylation de toutes les fonctions hydroxyles et clivage de l'imine en condition acide, la fonction amine générée peut être protégée par une fonction trichloroacétyle à l'aide du chlorure de trichloroacétyle ce qui conduit à l'anomère β pur **8** avec un rendement global de 45%. Cette méthode est efficace car chaque intermédiaire est cristallisable. Cependant, elle implique 4 étapes, et l'accès au donneur **2** est rendu impossible puisque la dégradation du milieu réactionnel a été observée lorsque des différentes tentatives de désacétylation du per-acétyle **8**.

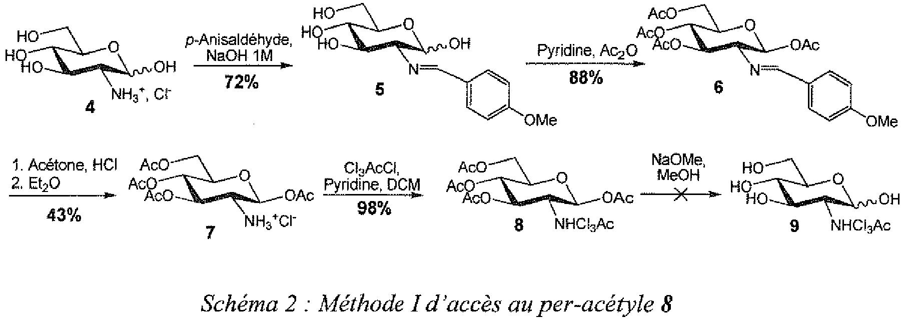

Une alternative est la méthode décrite par Jacquinet *et al.*¹ (Méthode II, Schéma 3). Le chlorhydrate de D-glucosamine **4** mis en réaction avec une base faible, l'hydrogénocarbonate de sodium, puis du chlorure de trichloroacétyle, est converti régiosélectivement en hémiacétal **9** protégé sur la fonction amine par une fonction trichloroacétyle. Cette méthode est efficace sur de grandes quantités si on enchaîne avec l'acétylation directe et la cristallisation du dérivé per-acétylé **8** comme décrit par les auteurs. En revanche, la cristallisation du trichloroacétamide **9** est difficile du fait de la grande solubilité de ce composé dans le milieu de cristallisation (eau froide). Ces deux étapes ont permis d'isoler le mélange d'anomères α et β **8** dans un rapport 90/10 avec un rendement de 45% inférieur à celui décrit (63%).¹

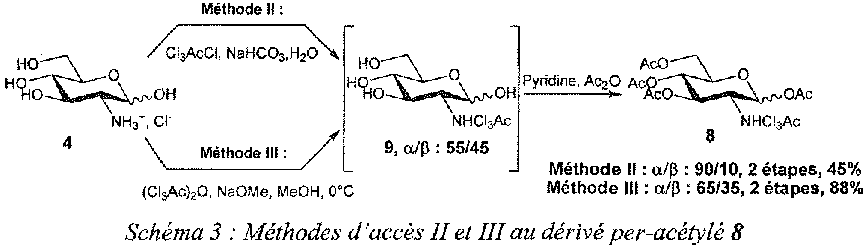

Il a par ailleurs été montré qu'il était possible de convertir une fonction amine en amide avec de bons résultats par introduction de groupements tels que l'acétyle, le palmitoyle ou le butyroyle^{11,12} mais aussi plus récemment l'introduction de butanoyle ou d'un 3,4,5,6-tétra-chlorophtaloyle^{10,13} sur la position 2_{D}. L'introduction de ces groupes acyles repose sur l'utilisation d'une base forte, le methylate de sodium (NaOMe) pour déprotonner la fonction amine du chlorhydrate de glucosamine commerciale. L'amine libre libérée réagit sur l'anhydride correspondant au groupement désiré. Ces conditions ont été testées pour « trichloroacétyler » la fonction amine de la glucosamine commerciale. Après optimisation des conditions, le chlorhydrate de D-glucosamine mis en réaction à 0°C (cette réaction est très sensible) avec du NaOMe et de l'anhydride trichloroacétique, est converti en trichloroacétamide **9** (Méthode III, Schéma 3). La recristallisation à cette étape étant difficile du fait de la présence de sels, l'acétylation de toutes les fonctions hydroxyles est ensuite réalisée sans purification préalable de **9.** Le mélange d'anomères α et β (65/35) **8** est isolé avec un rendement de 88% après chromatographie. Cette méthode est donc très intéressante par rapport aux méthodes précédentes, les rendements obtenus étant en effet nettement supérieurs à ceux décrits antérieurement pour la préparation de **8**. De plus, cette première étape est aisément reproductible à l'échelle de la dizaine de grammes.

### b. Synthèse du trichloroacétimidate de 3,4,6-tri-O-acétyl-2-désoxy-2-trichloroacétamido-α-D-glucopyranose 1

Le dérivé per-acétylé **8**, obtenu sous forme d'un anomère β pur (méthode I) ou d'un mélange d'anomères α/β (méthode II ou méthode III), doit être désacétylé en position anomérique. Parmi toutes les méthodes citées dans la littérature, deux méthodes très utilisées ont été testées :
- l'acétate d'hydrazinium ;^{1,14,15}
- l'éthylènediamine en milieu acide.^{16,17}

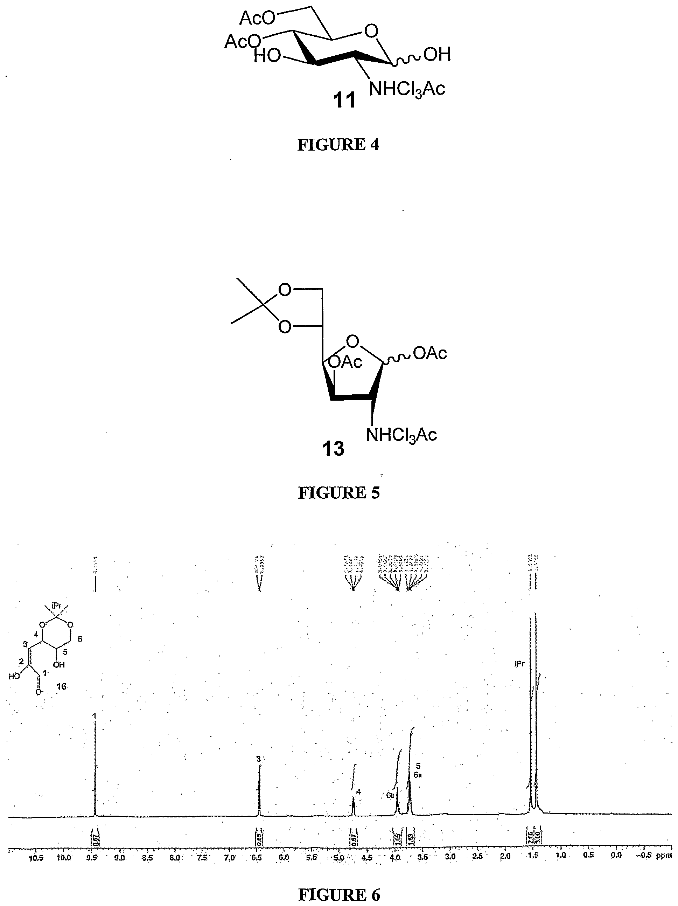

Lorsque la réaction est réalisée en présence d'éthylènediamine et d'acide acétique, elle est plus lente qu'en présence d'acétate d'hydrazinium, mais l'hémiacétal voulu **10** est obtenu avec une meilleure régiosélectivité et donc un rendement de 89% supérieur au 68% obtenu avec la première méthode. Ce meilleur rendement peut s'expliquer par l'absence de la formation du produit secondaire **11,** issu de la désacétylation en position 3, observée lorsque l'acétate d'hydrazinium est utilisé. La structure du composé **11** est confirmée par l'analyse RMN. Un shift du déplacement chimique vers les champs forts du proton 3 de **11** (δ = 4,00 ppm) par rapport à l'hémiacétal **10** (δ = 5,43 ppm) est observé (Figure 4).

L'hémiacétal est ensuite activé en donneur **1.** Ce dernier étant instable dans les conditions de purification sur silice, une base organique facilement éliminable a été sélectionnée sur la base des données de la littérature. ¹⁸⁻²⁰ Le carbonate de césium est apparu comme un choix intéressant car n'impliquant qu'une simple filtration pour éliminer les sels formés. L'alcool **10** est donc mis en réaction dans le dichlorométhane en présence de carbonate de césium et de trichloroacétonitrile pour donner le trichloroacétimidate **1** avec un rendement brut de 95% (Schéma 4). Mais, lors des différents essais de couplage réalisés en utilisant ce donneur, la quantité d'acide (TMSOTf) à additionner a été augmentée, par rapport à des conditions classiques, pour avoir un pH de milieu réactionnel suffisamment acide. Il est vraisemblable que des traces de carbonate de césium subsiste. Pour cette raison, l'emploi d'une base forte, également très utilisée dans la littérature, la DBU²¹ a aussi été testé. L'alcool **10** est mis en réaction dans le 1,2-dichloroéthane en présence de DBU et de trichloroacétonitrile pour donner, après purification rapide sur gel de silice, le trichloroacétimidate **1** avec un rendement de 87%.^{22,23} Une autre optimisation a été apportée. Si les étapes de désacétylation anomérique et d'activation sont enchaînées sans purification intermédiaire, le donneur **1** est obtenu à partir du précurseur **8** avec un rendement de 84% sur 2 étapes, amélioré par rapport aux données publiées¹ (77%).

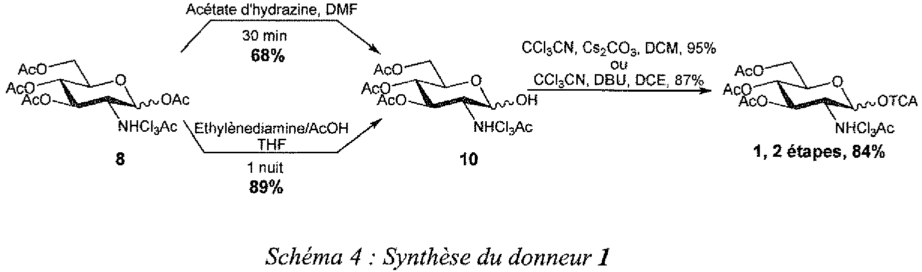

### c. Synthèse du trichloroacétimidate de 3-O-acétyl-2-désoxy-4,6-O-isopropylidène-2-trichloroacétamido-α/β-D-glucopyranose 2

Le trichloroacétamide **9** est préparé selon la méthode III décrite ci-dessus et utilisée telle quelle. Il est protégé régiosélectivement en position 4 et 6 par un groupement isopropylidène par action du 2-méthoxypropène et de CSA pour conduire à l'hémiacétal **12**.²⁴⁻²⁹ L'utilisation du 2-méthoxypropène^{30, 31} a été préférée à l'utilisation plus classique du 1,2-diméthoxypropane³² car celui-ci permet de limiter à 10% la formation du furanose **13** (confirmé par la présence du pic du proton anomérique à 6,26 ppm avec une constante de couplage de 4,9 Hz en RMN ¹H caractéristique d'un furanose α acétylé en position anomérique³³), contre 30% si la réaction implique le 1,2-diméthoxypropane (Figure 5).

Ensuite, les positions hydroxyles 1 et 3 libres du trichloroacétamide **12** sont acétylées pour obtenir le diacétate **14** avec un rendement de 70% sur les 3 étapes (Schéma 5).

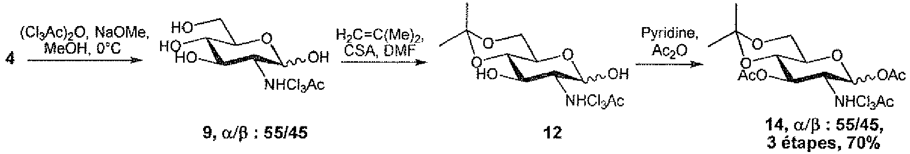

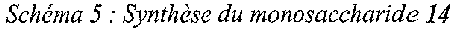

L'avant dernière étape est similaire à celle de la synthèse de l'hémiacétal **10.** Il s'agit d'une désacétylation anomérique. Une fois encore, les meilleurs résultats sont obtenus avec l'éthylènediamine¹⁷ en présence d'acide acétique. L'hémiacétal **15** est isolé avec un rendement de 87%, supérieur au 78% obtenus si l'acétate d'hydrazinium¹⁵ est employé (Schéma 6).

Comme pour le donneur **1,** des bases organiques faibles comme le carbonate de césium²⁰ ou le carbonate de potassium³⁴⁻³⁶ ont été testées pour préparer le donneur **2.** La réaction d'activation n'a jamais été totale, ce qui est probablement dû au fait de l'instabilité du trichloroacétimidate **2** formé. La base a donc été remplacée par une base plus forte, la DBU²¹, ce qui a permis d'isoler le donneur **2,** avec un rendement de 87%.^{22,23}

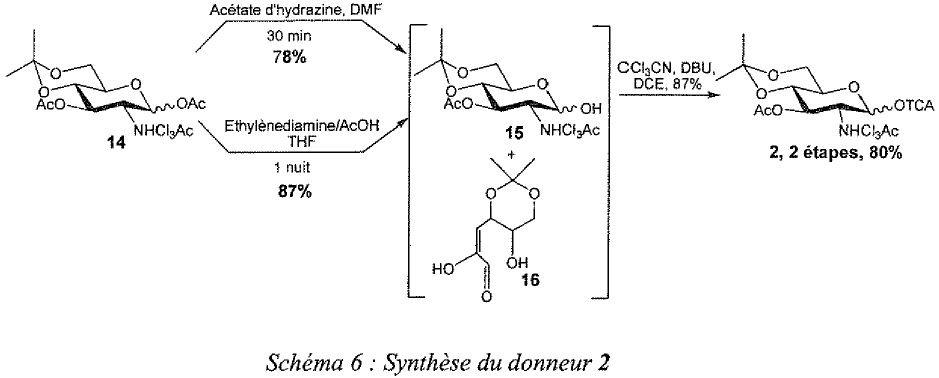

En revanche, lorsque la réaction de désacétylation anomérique a été réalisée à plus grande échelle, la dégradation de l'hémiacétal **15** sur la silice est observée avec la présence, en CCM, d'un composé plus polaire **16,** difficilement isolable en chromatographie. Après avoir forcé la formation de ce composé secondaire **16** en laissant le composé plus longtemps sur la silice, l'analyse par RMN montre la disparition de l'acétyle, de la fonction trichloroacétamide et l'apparition d'un aldéhyde, avec un déplacement chimique caractéristique à 9,43 ppm en proton et 187,0 ppm en carbone. Alors que la perte de l'isopropylidène pouvait être envisagée dû fait de l'acidité de la silice, l'analyse par RMN confirme bien sa présence avec des signaux caractéristiques à 1,54 ppm et 1,43 ppm en proton ainsi que le signal caractéristique du carbone portant les deux méthyles à 99,8 ppm. Le reste de la structure est confirmée par comparaison avec la littérature.³⁷

L'apparition de ce céto-aldéhyde **16** peut s'expliquer par une élimination de l'acétyle, due à l'acidité de la silice, suivie de l'hydrolyse de l'imine formée. Pour empêcher la formation de ce produit secondaire, l'hémiacétal **15** est engagé directement dans la réaction d'activation sans aucune purification à ce stade. Le donneur **2** est ainsi obtenu avec un rendement de 80% sur les deux étapes, désacétylation anomérique et activation (Schéma 6).

### 2. Couplage du donneur fonctionnalisé 2 avec différents accepteurs Un alcool modèle : l'alcool allylique

Après avoir obtenu ce premier donneur **2,** l'objectif a été de comparer ses propriétés en tant que donneur avec le précurseur **1.** L'alcool allylique a été sélectionné comme accepteur, conduisant à un produit de glycosylation potentiellement utile dans la synthèse des oligosaccharides de *S. flexneri* 3a.

L'alcool allylique a donc été couplé, dans des conditions classiques tel que le TMSOTf (0,2 éq.) dans le dichlorométhane en présence de tamis moléculaire. Avec le donneur **2,** la formation du glycoside d'allyle β **17,** isolé avec un rendement de 82% est observée (Schéma 7). En comparaison, le couplage avec le triacétate donneur **1,** permet aussi d'obtenir l'analogue **18** avec un rendement tout à fait comparable de 83%. Ce résultat suggère que l'isopropylidène du donneur **2** est stable dans les conditions de couplage et que la stéréosélectivité observée est tout aussi bonne pour le donneur **2** que pour le donneur **1.**

Pour confirmer que le donneur **2** est utilisable pour une élongation future en position 3_{D} après couplage, le glycoside d'allyle **17** a été transformé en accepteur. Deux voies ont été envisagées, la première est une saponification simultanée de la fonction acétyle en position 3 et de la fonction trichloroacétyle en position 2, suivi d'une acétylation sélective *in situ* de la fonction amine générée pour donner l'accepteur *N*-acétylé **19** avec un rendement de 71%.⁴ L'autre stratégie est la désacétylation sélective de l'acétyle en position 3. Dans ce cas, la fonction trichloroacétamide qui masque la fonction acétamide est conservée. En effet, il a souvent été montré qu'une fonction acétamide de glucosamine pouvait influencer les rendements de couplage.³⁸ Après optimisation des conditions de désacétylation, le carbonate de potassium, qui peut être éliminé par simple filtration est retenu dans la mesure où il conduit au meilleur rendement en accepteur **20** (94%). L'accepteur **20** peut également être obtenu via le donneur **1.** Il est intéressant de constater que les rendements sont comparables pour les deux voies, voire légèrement meilleurs si **1** est impliqué. En résumé, l'accepteur **20** est obtenu avec un rendement de 49% en 7 étapes *via* **1** alors qu'avec le donneur **2** et le même nombre d'étape, il est obtenu avec un rendement de 43% (Schéma 7).

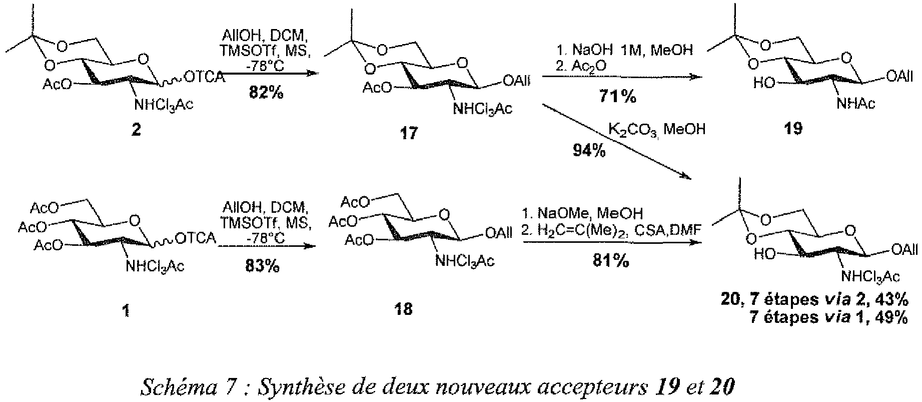

### 3. Prise en compte de la présence potentielle d'un acétyle : Synthèse du donneur 3

Cependant, la présence d'une fonction acétyle isolée peut s'avérer limitante dans le cas d'un allongement ultérieur de séquences comprenant le résidu C. Prenant en compte cette limitation, il s'agit de concevoir un donneur équivalent à **2** mais doté d'un groupement orthogonal à l'acétyle en position 3. Comme vu dans la partie rétrosynthèse, notre choix s'est porté sur le groupement lévulinoyle. La synthèse de l'analogue 3-*O*-lévulinoyle de **2** a donc été envisagée.

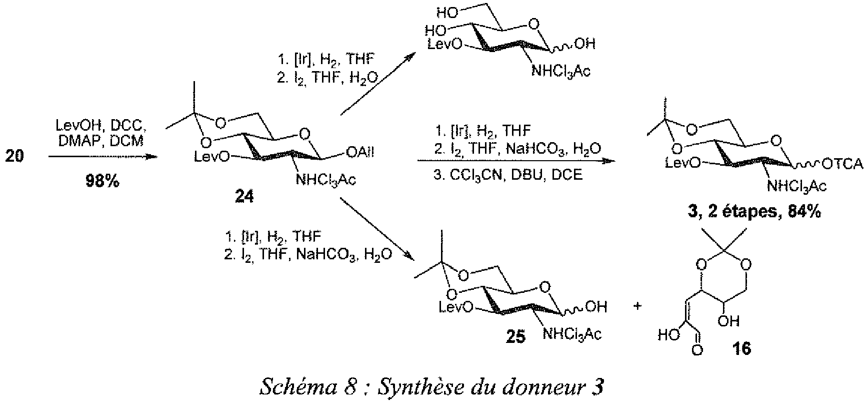

L'intermédiaire clé est le glycoside d'allyle **24,** aisément obtenu à partir de l'accepteur **20** par action d'acide lévulinique activé par la DCC en présence de DMAP (98%). La conversion de ce dernier en donneur **3** implique l'hémiacétal **25** issu d'une étape de déallylation anomérique dans des conditions classiques^{39, 40} (Schéma 8). Mais, en absence de base, la perte de l'isopropylidène a été observée dû à la formation *in situ* de l'acide iodhydrique HI.³⁹ La perte de ce groupement peut être évitée si une base faible, par exemple l'hydrogénocarbonate de sodium,⁴¹ est ajoutée. De plus, l'hémiacétal **25** s'est avéré instable car, lors de la purification par chromatographie sur gel de silice, le céto-aldéhyde **16** a de nouveau été isolé. Dans ces conditions, l'hémiacétal n'a pas pu être isolé sans 10% de contamination. En revanche, si les deux étapes, déallylation et activation, sont enchaînées sans purification intermédiaire, alors le donneur **3** est isolé avec un rendement de 84% sur deux étapes.

### 4. Conclusion

A ce stade, trois donneurs, précurseurs du résidu **D,** sont disponibles. Une synthèse efficace a été mise au point pour chacun d'entre eux, incluant une amélioration de 25% de la synthèse de **1** par rapport aux données de la littérature.¹ Effectivement, le donneur fonctionnalisé **2,** est obtenu avec un rendement de 56% en 5 étapes et le donneur **3** avec un rendement de l'ordre de 40% en 10 étapes (Schéma 9).

De plus, la synthèse des donneurs **1** et **2** n'implique que deux étapes de purification sur gel de silice. Ces précurseurs peuvent ainsi être préparés à grande échelle (de l'ordre de la dizaine de grammes).

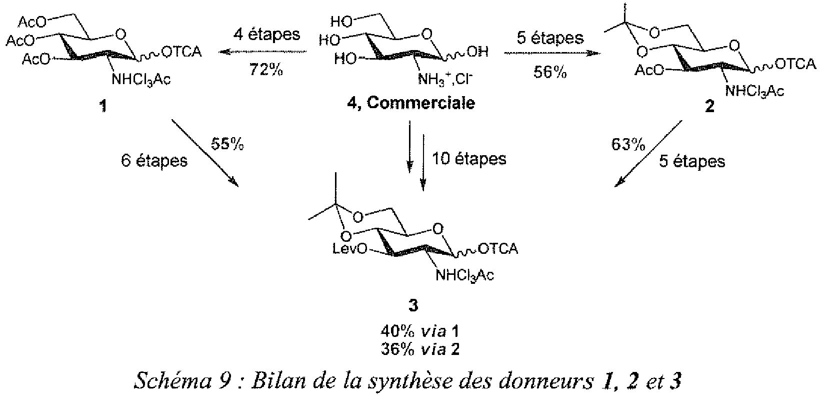

### Conditions expérimentales de synthèse des donneurs 1, 2 et 3 :

### ➢ 2-Désoxy-2-[p-méthoxybenzylidène(amino)]-β-D-glucopyranose¹⁰5:

### ➢ 1,3,4,6-Tétra-O-acétyl-2-désoxy-2-[p-méthoxybenzylidène(amino)]-β-D-glucopyranose¹⁰6:

### ➢ Hydrochlorurede1,3,4,6-tétra-O-acétyl-2-amino-2-désoxy-β-D-glucopyranose¹⁰7:

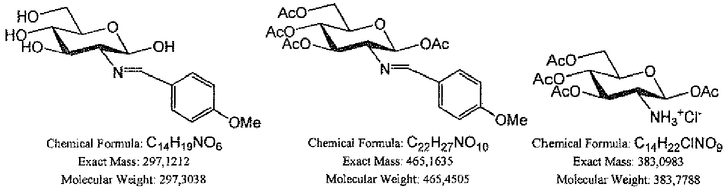

A une solution de chlorhydrate de D-glucosamine **4** (25,0 g, 116 mmol) dans de la soude 1M (120 mL) fraîchement préparée sous agitation vive, est additionné du *para-*anisaldéhyde (17,0 mL, 140 mmol). Après quelques instants, la cristallisation débute. Le milieu réactionnel est placé à 4°C et après 2 h, le précipité est filtré et lavé avec de l'eau froide puis avec un mélange EtOH/H₂O (1/1) pour conduire à l'imine **5** sous forme d'un solide blanc (24,9 g, 72%).
RMN ¹H (DMSO-d6), *δ* 8.11 (s, 1H, CH), 7.68 (d, 2H, CH_{Ph}), 6.97 (d, 2H, CH_{Ph}), 4.69 (d, 1H, *J*_{1,2} = 7.8 Hz, H-1), 3.78 (s, 3H, OMe), 3.71 (m, 1H, H-6a), 3.50 (pt, 1H, H-3), 3.43 (dd, 1H, *J*_{5*,*6a} = 9.0 Hz, H6b), 3.25 (m, 1H, H-5), 3.15 (pt, 1H, *J*_{3,4}= 9.5 Hz, H-4), 2.81 (dd, 1H, *J*_{2,3} = 9.1 Hz, H-2).
RMN ¹³C (DMSO-d6), *δ* 162.7 (CH), 161.9 (C_{Ph}), 130.4 (2C, CH_{Ph}), 129.7 (C_{Ph}), 114.8 (2C, CH_{Ph}), 96.2 (C-1, ¹*J*_{CH}= 153.9 Hz), 78.7 (C-2), 77.5 (C-5), 75.2 (C-3), 71.0 (C-4), 61.9 (C-6), 56.5 (OMe).

Dans un mélange pyridine/anhydride acétique (1/1, 200 mL), placé sous agitation dans un bain de glace, est ajouté par fractions l'imine **5** (24,9 g, 84 mmol). Le milieu réactionnel est agité durant 1 h puis à TA toute une nuit. La solution jaune obtenue est transférée dans 500 mL d'eau glacée. Le précipité blanc résultant est filtré, lavé avec de l'eau froide puis séché. Le solide blanc obtenu est identifié comme étant le tétra-*O-*acétyle **6** (34,3 g, 88%).
RMN ¹H (CDCl₃), *δ* 8.17 (s, 1H, CH), 7.68 (d, 2H, CH_{Ph}), 6.93 (d, 2H, CH_{Ph}), 5.96 (d, 1H, *J*_{1,2} = 7.8 Hz, H-1), 5.44 (pt, 1H, *J*_{2,3} = 9.6 Hz, H-3), 5.15 (pt, 1H, *J*_{3,4} = 9.8 Hz, H-4), 4.38 (dd, 1H, *J*_{5*,*6a} = 4.6 Hz, *J*_{6a,6b} = 12.4 Hz, H-6a), 4.13 (dd, 1H, *J*_{5,6b} = 2.1 Hz, H6b), 4.00 (m, 1H, H-5), 3.85 (s, 3H, OMe), 3.46 (dd, 1H, H-2), 2.13, 2.06, 2.05, 1.89 (4s, 12H, H_{Ac}).
RMN ¹³C (CDCl₃), *δ* 171.0, 170.2, 169.8, 169.1 (4C, C_{Ac}), 164.6 (CH), 162.7 (C_{Ph}), 130.6 (2C, CH_{Ph}), 128.7 (C_{Ph}), 114.7 (2C, CH_{Ph}), 93.5 (C-1, ¹*J*_{CH} = 167.0 Hz), 73.3 (C-3), 73.1 (C-2), 73.0 (C-5), 68.4 (C-4), 62.2 (C-6), 55.9 (OMe), 21.2, 21.1, 21.0, 20.9 (4C, C_{Ac}).

Le tétra-*O*-acétyle **6** (34,3 g, 74,0 mmol) est dissous dans 300 mL d'acétone chaud puis de l'HCl (5M, 15,0 mL) est ajouté goutte à goutte au milieu réactionnel. Immédiatement, un précipité se forme et le ballon est plongé dans un bain glacé. Après addition de 300 mL d'éther diéthylique et 2 h d'agitation, le ballon est placé en chambre froide toute une nuit. Le précipité obtenu est filtré, lavé avec de l'éther diéthylique et séché à la pompe à palette pour donner le chlorhydrate de glucosamine **7** sous forme d'un solide blanc (19,0 g, 43%).
RMN ¹H (D₂O), *δ* 5.90 (d, 1H, *J*_{1,2} = 8.8 Hz, H-1), 5.43 (pt, 1H, *J*_{2,3} = 9.1 Hz, H-3), 5.06 (pt, 1H, *J*_{3,4} = 9.6 Hz, H-4), 4.27 (dd, 1H, *J*_{5*,*6a} = 3.8 Hz, *J*_{6a,6b} = 12.6 Hz, H-6a), 4.16-4.11 (m, 2H, H-5, H-6b), 3.70 (dd, 1H, H-2), 2.13, 2.06, 1.99 (3s, 12H, H_{Ac}).
RMN ¹³C (D₂O), *δ* 173.9, 173.4, 173.0, 171.5 (4C, C_{Ac}), 90.7 (C-1, ¹*J*_{CH} = 161.0 Hz), 72.5 (C-5), 71.2 (C-3), 68.4 (C-4), 61.8 (C-6), 52.7 (C-2), 20.6, 20.5, 20.4, 20.3 (4C, C_{Ac}).

### ➢ 2-Désoxy-2-trichloroacétamido-α/β-D-glucopyranose¹ 9 :

### ➢ 1,3,4,6-Tétra-O-acétyl-2-désoxy-2-trichloroacétamido-α/β-D-glucopyranose¹ 8 :

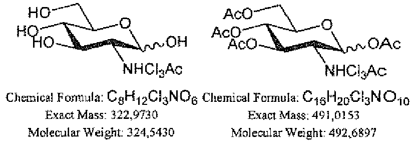

Du chlorhydrate de D-glucosamine **4** commercial (5,0 g, 23,2 mmol) est dissous dans du MeOH (100 mL) et la solution est agitée pendant 2 h à TA. Ensuite, le ballon est placé à 0°C et agité pendant 10 min puis du NaOMe 25% (16,1 mL, 58,1 mmol, 2,5 éq.) est additionné lentement au milieu réactionnel. Après 15 min d'agitation, un précipité est observé puis de l'anhydride trichloroacétique (6,4 mL, 34,9 mmol, 1,5 éq.) est ajouté goutte à goutte au milieu réactionnel maintenu dans un bain de glace. Après 1 h 30 sous agitation, le suivi par CCM (iPrOH/H₂O/NH₃, 4/1/0,5) montre la disparition de **4** (Rf= 0,1) et l'apparition d'un produit moins polaire (Rf= 0,6). Le milieu réactionnel est alors neutralisé par de la résine échangeuse d'ion DOWEX (H⁺), puis filtré sur fritté et concentré à l'évaporateur rotatif. Le résidu est dissous dans un minimum d'H₂O puis lyophilisé. La mousse jaune obtenue, qui est un mélange d'anomère **9**_{α/β} (6/4), est engagée dans l'étape suivante après contrôle RMN pour vérifier l'absence de **4.**
Rf = 0,6 (iPrOH/H₂O/NH₃, 4/1/0,5).
RMN ¹H (DMSO-d6), *δ*: α : 8.13 (bs, 1H, NH), 5.06 (d, 1H, *J*_{1,2} = 3.5 Hz, H-1), 4.99 (bs, 1H, OH-4), 4.90 (bs, 1H, OH-2), 4.82 (bs, 1H, OH-3), 4.43 (bs, 1H, OH-6), 3.73 (m, 1H, H-3), 3.64 (m, 1H, H-6a), 3.61 (m, 1H, H-5), 3.56 (m, 1H, H-2), 3.53 (m, 1H, H-6b), 3.18 (pt, 1H, *J*_{3,4} = 8.6 Hz, H-4).
RMN ¹³C (DMSO-d6), *δ*: α : 162.3 (C_{NTCA}), 93.5 (CCl₃), 90.5 (C-1, ¹*J*_{CH} = 167.4 Hz), 73.1 (C-5), 71.6 (C-4), 70.7 (C-3), 61.8 (C-6), 57.8 (C-2).
RMN ¹H (DMSO-d6), *δ*: β : 8.59 (d, 1H, *J*_{NH,2} = 8.5 Hz, NH), 4.95 (bs, 1H, OH-4), 4.65 (d, 1H, *J*_{1,2} = 7.6 Hz, H-1), 4.50 (bs, 1H, OH-6), 3.68 (m, 1H, H-6a), 3.50 (m, 1H, H-5), 3.46 (m, 1H, H-6b), 3.41 (m, 1H, H-2), 3.18 (m, 2H, H-3, H-4).
RMN ¹³C (DMSO-d6), *δ*: β : 162.1 (C_{NTCA}), 95.4 (C-1, ¹*J*_{CH} = 157.0 Hz), 94.3 (CCl₃), 77.7 (C-4), 74.2 (C-5), 72.0 (C-3), 62.0 (C-6), 59.9 (C-2).
**Voie 1 :** Le brut réactionnel **9** obtenu est dissous dans un mélange pyridine/anhydride acétique (1/1, 50 mL) à 0°C et le milieu réactionnel est laissé sous agitation pendant une nuit à TA. Après avoir vérifié par un suivi CCM (DCM/EtOAc, 9/1) l'apparition de deux nouveaux composés (Rf = 0,45 et 0,2), le milieu réactionnel est concentré à l'évaporateur rotatif puis coévaporé au Tol (3 x 100 mL) et au DCM (3 x 100 mL). Le résidu est séché à la pompe à palettes pour conduire au dérivé attendu sous forme d'un solide correspondant à un mélange d'anomères α/β. Le solide obtenu est purifié par chromatographie sur gel de silice (DCM/EtOAc, 97/3→9/1) pour conduire, par ordre d'élution, au monosaccharide **8_{α}** désiré (6,6 g, 57%) puis au stéréoisomère **8_{β}** (3,5 g, 31%). Les deux anomères **8_{α}** et **8_{β}** (65/35) sont isolés sous forme de solide blanc (10,1 g, 88%).
**Voie 2 :** A une solution de DCM (40 mL) et de pyridine (5,2 mL, 65,3 mmol, 5 éq.) est ajouté le chlorhydrate de glucosamine 7 par fractions (5 g, 13,1 mmol). Le milieu réactionnel est placé dans un bain glacé puis le chlorure de trichoroacétyle (4,1 mL, 36,0 mmol, 2,8 éq.) est ajouté au goutte à goutte. Après 15 min d'agitation à 0°C, le milieu réactionnel est agité pendant 30 min à TA. La réaction est suivie par CCM (DCM/EtOAc, 9/1), l'apparition d'un produit moins polaire (Rf= 0,2) est alors observé. Le milieu réactionnel est repris à l'H₂O (20 mL) et la phase aqueuse est extraite au DCM (3 x 30 mL). Les phases organiques sont regroupées et lavées avec une solution de NaHCO₃ 5% (3 x 50 mL), avec de l'H₂O (3 x 50 mL) puis séchées sur Na₂SO₄ et concentrées à l'évaporateur rotatif pour donner un solide blanc (6,3 g, 98%) correspondant à l'anomère **8β.**
**8_{α} :** Rf = 0,45 (DCM/EtOAc, 9/1).
RMN ¹H (CDCl₃), *δ* 6.83 (d, 1H, *J*_{NH,2} = 8.3 Hz, NH_{α}), 6.29 (d, 1H, *J*_{1,2} = 3.7 Hz, H-1), 5.34 (pt, 1H, *J*_{2,3} = 9.6 Hz, H-3), 5.23 (pt, 1H, *J*_{3,4} = 9.8 Hz, H-4), 4.35-4.25 (m, 2H, H-2, H-6a), 4.07-4.01 (m, 2H, H-5, H-6b), 2.18, 2.08, 2.05 (3s, 12H, H_{Ac}). RMN ¹³C (CDCl₃), *δ* 172.0, 171.0, 169.4, 168.7 (4C, C_{Ac}), 162.3 (C_{NTCA}), 92.1 (CCl₃), 89.9 (C-1, ¹*J*_{CH} = 179.2 Hz), 70.4 (C-5), 70.2 (C-3), 67.4 (C-4), 61.7 (C-6), 53.7 (C-2), 21.1, 21.0, 20.9, 20.8 (4C, C_{Ac}).
**8_{β}** : Rf = 0,2 (DCM/EtOAc, 9/1).
RMN ¹H (CDCl₃), *δ* 7.19 (d, 1H, *J*_{NH,2} = 9.4 Hz, NH), 5.83 (d, 1H, *J*_{1,2} = 8.7 Hz, H-1), 5.37 (pt, 1H, *J*_{2,3} = 9.4 Hz, H-3), 5.18 (pt, 1H, *J*_{3,4} = 9.8 Hz, H-4), 4.32 (m, 2H, H-2, H-6a), 4.18 (m, 1H, *J*_{5,6b} = 2.3 Hz, *J*_{6a,6b} = 12.4 Hz, H-6b), 3.89 (ddd, 1H, *J*_{4,5} = 10.0 Hz, *J*_{5,6a} = 4.7 Hz, H-5), 2.13, 2.06 (2s, 12H, H_{Ac}).
RMN ¹³C (CDCl₃), *δ* 171.6, 171.0, 169.7, 169.6, (4C, C_{Ac}), 162.7 (C_{NTCA}), 92.5 (CCl₃), 92.4 (C-1, ¹*J*_{CH} = 159.6 Hz), 73.5 (C-5), 72.2 (C-3), 68.1 (C-4), 62.0 (C-6), 55.0 (C-2), 21.1, 21.0, 20.9, 20.8 (4C, C_{Ac}).

### ➢ 3,4,6-Tri-O-acétyl-2-désoxy-2-trichloroacéiamido-α/β-D-glucopyranose¹ 10 :

### ➢ 4,6-Tri-O-acétyl-2-désoxy-2-trichloroacétamido-α/β-D-glucopyranose 11 :

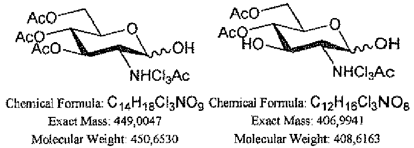

A une solution d'éthylènediamine (1,8 mL, 25 mmol, 1,2 éq.) dans du THF (390 mL) est additionné lentement de l'acide acétique (1,7 mL, 29,1 mmol, 1,4 éq.). Il en résulte immédiatement la formation d'un précipité. Ensuite le tétra-*O*-acétyle **8** (10,2 g, 20,8 mmol) est additionné au milieu réactionnel qui est agité à TA et la réaction est suivie par CCM (DCM/MeOH, 95/5 et Chex/EtOAc, 1/1). Après 24 h, la transformation de **8** (Rf = 0,7 et 0,3, respectivement) en un composé plus polaire est totale (Rf = 0,4 et 0,3, respectivement). Le milieu réactionnel est repris à l'eau froide (100 mL) et à l'acétate d'éthyle (100 mL) et la phase aqueuse est extraite à l'acétate d'éthyle (3 x 100 mL). Les phases organiques sont regroupées et lavées avec une solution de NaHCO₃ 5% (3 x 50 mL), avec une solution de NaClₛₐₜ (3 x 50 mL), avec de l'H₂O (3 x 50 mL) puis séchées sur Na₂SO₄ et concentrées à l'évaporateur rotatif. Le solide blanc obtenu est purifié par chromatographie sur gel de silice (Chex/EtOAc, 8/2→1/1) pour conduire par ordre d'élution à l'hémiacétal **10** sous forme d'un solide blanc (8,3 g, 89%) puis au diiol non voulu **11** (420 mg, 5%). 10 : Rf = 0,3 (Chex/EtOAc, 1/1).
RMN ¹H (CDCl₃), *δ* 7.03 (d, 1H, *J*_{NH,2} = 8.9 Hz, NH), 5.43 (pt, 1H, *J*_{2,3} = 9.6 Hz, H-3), 5.43 (pt, 1H, *J*_{1,2} = 3.6 Hz, H-1), 5.19 (pt, 1H, *J*_{3,4} = 9.6 Hz, H-4), 4.33-4.24 (m, 3H, H-2, H-5, H-6a), 4.19 (m, 1H, H-6b), 3.47 (d, 1H, *J*_{OH,1} = 2.6 Hz, OH), 2.13, 2.09, 2.05 (3s, 9H, H_{Ac}).
RMN ¹³C (CDCl₃), *δ* 170.3, 169.4, 168.5 (3C, C_{Ac}), 161.1 (C_{NTCA}), 91.0 (CCl₃), 89.9 (C-1, ¹*J*_{CH} = 172.6 Hz), 69.5 (C-3), 67.2 (C-4), 67.0 (C-5), 61.0 (C-6), 53.2 (C-2), 19.7, 19.6, 19.5 (3C, C_{Ac}). **11** : Rf = 0,2 (Chex/EtOAc, 1/1).
RMN ¹H (CDCl₃), *δ* 7.19 (d, 1H, *J*_{NH,H-2} = 8.6 Hz, NH), 5.34 (pt, 1H, *J*_{1,2} = 3.3 Hz, H-1), 5.19 (pt, 1H, *J*_{3,4} = 9.5 Hz, H-4), 4.25-4.03 (m, 4H, H-6a, H-5, H-2, H-6b), 3.97 (pt, 1H, *J*_{2,3} = 9.5 Hz, H-3), 2.10, 2.08 (2s, 6H, H_{Ac}).
RMN ¹³C (CDCl₃), *δ* 171.7, 171.4 (2C, C_{Ac}), 163.3 (C_{NTCA}), 92.6 (CCl₃), 91.3 (C-1, ¹*J*_{CH} = 171.9 Hz), 71.5 (C-4), 70.9 (C-3), 68.4 (C-5), 62.7 (C-6), 56.4 (C-2), 21.3, 21.2 (2C, C_{Ac})

| | | |
|---|---|---|
| HRMS (ESI⁺) : | [M+Na]⁺ C₁₂H₁₆NO₈³⁵Cl₃Na | m/z théorique : 429.9839 |
| | | m/z mesurée : 429.9864 |

### ➢ Trichloracétimidate de 3,4,6-tri-O-acétyl-2-désoxy-2-trichloroacétamido-α/β-D-glucopyranose¹ 1 :

### ➢ 2-Trichlorométhyl-(3,4,6-tri-O-acétyl-1,2-didésoxy-α-D-glucopyrano)-[2,1-d]-2-oxazoline¹ 1' :

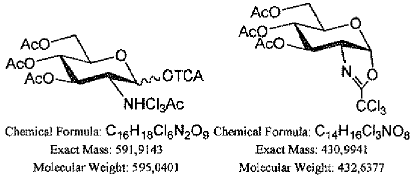

**Voie 1** : L'hémiacétal **10** (4,0 g, 8,9 mmol) est solubilisé dans du DCE (30 mL) et agité sous argon à -5°C puis de la DBU (372 µL, 2,5 mmol, 0,28 éq.) et du trichloroacétonitrile (4,4 mL, 44,4 mmol, 5 éq.) sont ajoutés. Le milieu réactionnel est agité à cette température pendant 15 min puis il est déposé, sans aucun traitement, sur une colonne de gel de silice (flash chromatographie, Chex/EtOAc+5% NEt₃, 7/3→1/1) pour obtenir le trichloroacétimidate **1** sous forme d'un solide blanc (4,6 g, 87%).
**Voie 2** : L'hémiacétal **10** (1,5 g, 3,3 mmol) est solubilisé dans du DCM (15 mL). Du trichloroacétonitrile (1,7 mL, 16,7 mmol, 5 éq.) est additionné au milieu réactionnel puis du Cs₂CO₃ (217 mg, 670 µmol, 0,2 éq.) est ajouté par petites fractions. Le milieu réactionnel est agité à TA et la réaction est suivie par CCM (Chex/EtOAc, 1/1). Après 1h30 d'agitation, la transformation de **10** en un produit moins polaire (Rf= 0,8) est totale. Le résidu obtenu est filtré et lavé avec du DCM. Le filtrat est alors co-évaporé au Tol à l'évaporateur rotatif. Le trichloroacétimidate 1 sous forme d'un solide jaune obtenu (1,9 g, 98%) est engagé directement dans l'étape suivante après contrôle par RMN ¹H.
**1** : Rf = 0,8 (Chex/EtOAc, 1/1)
RMN ¹H (CDCl₃), *δ* : α : 8.85 (s, 1H, NH), 7.00 (d, 1H, *J*_{NH,2} = 8.5 Hz, NH), 6.5 (d, 1H, J_{1,2} = 3.6 Hz, H-1), 5.44 (pt, 1H, *J*_{2,3} = 9.7 Hz, H-3), 5.3 (pt, 1H, *J*_{3,4} = 10.0 Hz, H-4), 4.45 (ddd, 1H, H-2), 4.28 (dd, 1H, *J*_{5,6a} = 4.2 Hz, *J*_{6a,6b} = 12.7 Hz, H-6a), 4.18-4.12 (m, 2H, H-5, H-6b), 2.11, 2.08, 2.06 (3s, 9H, H_{Ac}).
RMN ¹³C (CDCl₃), *δ*: a : 171.8, 170.9, 169.5 (3C, C_{Ac}), 162.4 (C=NH), 160.3 (C_{NTCA}), 94.0 (C-1, ¹*J*_{CH}= 181.4Hz), 92.1 (CCl₃), 90.8 (CCl₃), 70.8 (C-5), 70.5 (C-3), 68.2 (C-4), 61.6 (C-6), 54.2 (C-2), 21.1, 21.0, 20.9 (3C, C_{Ac})

L'oxazoline **1',** résultant de la dégradation de **1** durant la purification, est isolé comme le second produit à éluer lors de chromatographie sur gel de silice.
**1'** : Rf = 0,65 (Chex/EtOAc, 1/1)
RMN ¹H (CDCl₃), *δ* : oxazoline : 6.35 (d, 1H, *J*_{1,2} = 7.4 Hz, H-1), 5.41 (pt, 1H, *J*_{2,3} = 2.3 Hz, H-3), 4.96 (m, 1H, *J*_{4,5} = 8.2 Hz, *J*_{2,4} = 1.6 Hz, H-4), 4.49 (m, 1H, H-2), 4.29 (dd, 1H, *J*_{5,6a} = 3.0 Hz, *J*_{6a,6b} = 12.1 Hz, H-6a), 4.20 (dd, 1H, *J*_{5,6b} = 6.0 Hz, H-6b), 3.80 (m, 1H, H-5), 2.15, 2.13, 2.11 (3s, 9H, H_{Ac}).
RMN ¹³C (CDCl₃), *δ*: oxazoline : 170.8, 169.8, 169.3 (3C, C_{Ac}), 163.4 (N=C), 103.5 (C-1, ¹*J*_{CH} = 183.6 Hz), 86.5 (CCl₃), 69.4 (C-5), 69.1 (C-3), 68.0 (C-4), 65.1 (C-2), 63.8 (C-6), 21.2, 21.1, 21.0 (3C, C_{Ac}).

### ➢ 2-Désoxy-4,6-O-isopropylidène-2-trichloroacétamido-α/β-D-glucopyranose 12 :

### ➢ 1,3-Di-O-acétyl-2-désoxy-4,6-O-isopropylidène-2-trichloroacétamido-α/β-D-glucopyranose 14 :

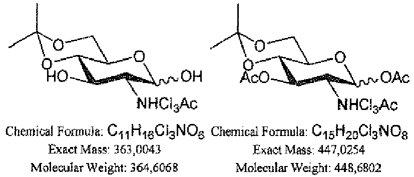

Du chlorhydrate de D-glucosamine commercial **4** (11,6 g, 54,0 mmol) est protégé en position 2_{D} par un groupement trichloroacétamide comme décrit précédemment.

Le brut obtenu **9** (54,0 mmol) est dissous dans du DMF (185 mL) contenant du 2-méthoxypropène (10,3 mL, 108,0 mmol, 2 éq.). Du CSA (24,0 g, 108,0 mmol, 2 éq.) est ajouté par fractions au milieu réactionnel jusqu'à obtenir un pH de 2. Le milieu réactionnel est agité à TA et la réaction est suivie par CCM (DCM/MeOH, 8/2 et Chex/EtOAc, 1/1). Après 2h30 d'agitation, le MeOH formé est évaporé à l'évaporateur rotatif et du 2-méthoxypropène (2 mL, 21,6 mmol, 0,4 éq.) est de nouveau ajouté. Après 1 h d'agitation, la transformation de **9** en un produit moins polaire (Rf= 0,3 dans Chex/EtOAc, 1/1) est quasi-totale. Le milieu réactionnel est neutralisé avec de la triéthylamine (0,2 mL) puis concentré à l'évaporateur rotatif pour conduire à un solide jaune, correspondant à un mélange d'anomères **12_{α/β}** (9/1).
Rf = 0,3 (Chex/EtOAc, 1/1).
RMN ¹H (DMSO-d6), *δ*: α : 8.37 (d, 1H, *J*_{NH,2} = 7.8 Hz, NH), 6.88 (d, 1H, *J*_{1,2} = 3.9 Hz, H-1), 5.0 (m, 2H, OH), 3.77 (m, 1H, H-3), 3.71-3.65 (m, 4H, H-2, H-5, H-6a, H-6b), 3.50 (m, 1H, H-4), 1.44 (s, 3H, H_{iPr}), 1.25 (s, 3H, B_{iPr}).
RMN ¹³C (DMSO-d6), *δ*: α : 162.4 (C_{NTCA}), 99.9 (C_{iPr}), 93.3 (CCl₃), 91.2 (C-1, ¹*J*_{CH} = 168.2 Hz), 75.0 (C-4), 67.5 (C-3), 63.9 (C-5), 62.3 (C-6), 58.0 (C-2), 29.8 (C_{iPr}), 21.6 (C_{iPr}).

| | | |
|---|---|---|
| HRMS (ESI⁺) : | [M+Na]⁺ C₁₁H₁₆NO₆³⁵Cl₃Na | m/z théorique : 385.9941 |
| | | m/z mesurée : 385.9962 |

Le brut réactionnel **12** obtenu est dissous dans un mélange pyridine/anhydride acétique (1/1, 100 mL) à 0°C et le milieu réactionnel est laissé sous agitation pendant une nuit à TA. Après avoir vérifié par un suivi par CCM (Chex/EtOAc, 1/1) l'apparition de deux nouveaux composés (Rf = 0,7 et 0,75), le milieu réactionnel est concentré à l'évaporateur rotatif puis coévaporé au Tol (3 x 200 mL) et au DCM (3 x 200 mL). Le résidu est séché à la pompe à palettes pour conduire au dérivé attendu sous forme d'un solide. Le solide obtenu est purifié par chromatographie sur gel de silice (Chex/EtOAc, 8/2→75/25) pour conduire au mélange d'anomères **14_{a/β}** dans les proportions 55/45 sous forme d'un solide blanc (17,0 g, 70%)
**14_{α}** : Rf = 0,7 (Chex/EtOAc, 1/1).
RMN ¹H (CDCl₃), *δ* 7.01 (d, 1H, *J*_{NH,2} = 8.4 Hz, NH), 6.23 (d, 1H, *J*_{1,2} = 3.9 Hz, H-1), 5.30 (pt, 1H, *J*_{2,3} = 9.5 Hz, H-3), 4.28 (ddd, 1H, H-2), 3,93-3.83 (m, 2H, H-4, H-6a), 3.79-3.74 (m, 2H, H-5, H-6b), 2.16, 2.09 (2s, 6H, H_{Ac}), 1.51 (s, 3H, H_{iPr}), 1.39 (s, 3H, H_{jPr}).
RMN ¹³C (CDCl₃), δ 172.2, 169.4 (2C, C_{Ac}),162.4 (C_{NTCA}), 100.4 (C_{iPr}), 92.2 (CCl₃), 90.5 (C-1, ¹*J*_{CH} = 178.6 Hz), 71.8 (C-4), 70.0 (C-3), 66.4 (C-5), 62.2 (C-6), 53.8 (C-2), 29.2 (C_{iPr}), 21.2, 21.1 (2C, C_{Ac}), 19.4 (C_{iPr}).
**14_{β}** Rf= 0,75 (Chex/EtOAc, 1/1).
RMN ¹H (CDCl₃), *δ* 7.03 (d, 1H, *J*_{NH,2} = 9.7 Hz, NH), 5.78 (d, 1H, *J*_{1,2} = 8.7 Hz, H-1), 5.24 (pt, 1H, *J*_{2,3} = 9.6 Hz, H-3), 4.25 (m, 1H, H-2), 4.01 (m, 1H, H-6a), 3.88-3.83 (m, 2H, H-4, H-6b), 3.55 (m, 1H, H-5), 2.11 (s, 3H, H_{Ac}), 2.09 (s, 3H, H_{Ac}), 1.52 (s, 3H, H_{iPr}), 1.45 (s, 3H, B_{iPr}).
RMN ¹³C (CDCl₃), *δ* 71.8, 169.5 (2C, C_{Ac}), 162.8 (C_{NTCA}), 100.5 (C_{iPr}), 93.2 (C-1), 92.2 (CCl₃), 71.6 (C-4), 71.3 C-3), 69.1 (C-5), 62.1 (C-6), 55.9 (C-2), 30.1 (C_{iPr}), 21.2, 21.1 (2C, C_{Ac}), 19.3 (C_{iPr}).

| | | |
|---|---|---|
| HRMS (ESI⁺) : | [M+Na]⁺ C₁₅H₂₀NO₈³⁵Cl₃Na | m/z théorique : 470.0152 |
| | | m/z mesurée : 470.0123 |

### ➢ 3-O-Acétyl-2-désoxy-4,6-O-isopropylidène-2-trichloroacétamido-α/β-D-glucopyranose 15 :

### ➢ 2,5-Hydroxy-4,6-O-isopropylidène-hex-2-enal 16 :

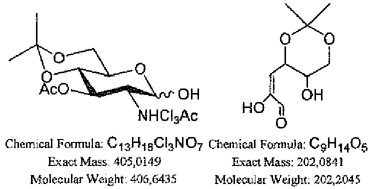

A une solution d'éthylènediamine (177 µL, 2,4 mmol, 1,2 éq.) dans du THF (40 mL) est additionné lentement de l'acide acétique (162 µL, 2,8 mmol, 1,4 éq.). Il en résulte immédiatement la formation d'un précipité. Ensuite le di-*O*-acétyle **14** (900 mg, 2,0 mmol) est additionné au milieu réactionnel qui est agité à TA et la réaction est suivie par CCM (DCM/MeOH, 95/5 et Chex/EtOAc, 1/1). Après 20 h, la transformation de **14** (Rf= 0,7 et 0,75 dans Chex/EtOAc, 1/1) en un composé plus polaire est totale (Rf= 0,6 dans DCM/MeOH, 95/5 et 0,5 dans Chex/EtOAc, 1/1). Le milieu réactionnel est repris à l'eau froide (25 mL) et à l'acétate d'éthyle (25 mL) et la phase aqueuse est extraite à l'acétate d'éthyle (3 x 20 mL). Les phases organiques sont regroupées et lavées avec une solution de NaHCO₃ 5% (3 x 15 mL), avec une solution de NaClₛₐₜ (3 x 15 mL), avec de l'H₂O (3 x 15 mL) puis séchées sur Na₂SO₄ et concentrées à l'évaporateur rotatif. Le solide obtenu est purifié par chromatographie sur gel de silice (Chex/EtOAc, 8/2→ 1/1) pour conduire au mélange d'anomères **15_{α/β}** dans les proportions 95/5 sous forme d'un solide blanc (710 mg, 87%) suivi du céto-aldéhyde **16** (20 mg, 5%).
**15_{α} :** Rf= 0,5 (Chex/EtOAc, 1/1).
RMN ¹H (CDCl₃), *δ* 7.11 (d, 1H, *J*_{NH,2} = 8.9 Hz, NH), 5.37-5.34 (m, 2H, H-1, H-3), 4.20 (ddd, 1H, *J*_{1,2} = 3.7 Hz, *J*_{2,3} = 9.5 Hz, *J*_{2,OH} = 1.8 Hz, H-2), 4.02 (ddd, 1H, H-5), 3.91 (dd, 1H, *J*_{5,6a} = 5.2 Hz, *J*_{6a,6b} = 10.6 Hz, H-6a), 3.86-3.77 (m, 2H, *J*_{3,4} = 10.6 Hz, H-4, H-6b), 2.09 (s, 3H, H_{Ac}), 1.53 (s, 3H, H_{iPr}), 1.45 (s, 3H, H_{iPr}).
RMN ¹³C (CDCl₃), *δ* 171.8 (C_{Ac}), 162.5 (C_{NTCA}), 100.4 (C_{iPr}), 92.4 (CCl₃), 92.1 (C-1, ¹*J*_{CH} = 173.5 Hz), 72.0 (C-4), 70.2 (C-3), 64.3 (C-5), 62.6 (C-6), 55.3 (C-2), 29.3 (C_{iPr}), 21.3 (C_{Ac}), 19.4 (C_{iPr}).

| | | |
|---|---|---|
| HRMS (ESI⁺) : | [M+Na]⁺ C₁₃H₁₈NO₇³⁵Cl₃Na | m/z théorique : 428.0047 |
| | | m/z mesurée : 428.0048 |

**16 :** Rf = 0,25 (Chex/EtOAc, 6/4).
RMN ¹H (CDCl₃), *δ* 9.43 (s, 1H, H-1), 6.45 (d, 1H, *J*_{3,4} = 5.7 Hz, H-3), 4.74 (m, 1H, H-4), 3.93 (m, 1H, H-6a), 3.77-3.69 (m, 2H, H-5, H-6b), 1.54 (s, 3H, H_{iPr}), 1.43 (s, 3H, H_{iPr});
RMN ¹³C (CDCl₃), *δ* 187.0 (CHO), 137.3 (C-3), 135.8 (C-2), 99.8 (C_{iPr}), 72.0 (C-4), 67.2 (C-5), 65.1 (C-6), 29.0 (C_{iPr}), 19.5 (C_{iPr});

### ➢ Trichloracétimidate de 3-O-acétyl-2-désoxy-4,6-O-isopropylidène-2-trichloroacétamido-α/β-D-glucopyranose 2 :

### ➢ 2-Trichlorométhyl-(3-0-acétyl-1,2-didésoxy-4,6-O-isopropylidène-α-D-glucopyrano)-[2,1-d]-2-oxazoline 2':

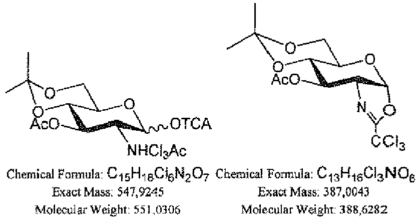

**Voie 1** : L'hémiacétal **15** (3,0 g, 7,4 mmol) est dissous dans du DCE (12 mL) et agité sous argon à -5°C, puis de la DBU (310 µL, 2,1 mmol, 0,28 éq.) et du trichloroacétonitrile (3,7 mL, 36,9 mmol, 5 éq.) sont ajoutés. Le milieu réactionnel est agité à cette température pendant 15 min puis il est déposé, sans aucun traitement, sur une colonne de gel de silice (flash chromatographie, Chex/EtOAc+5% NEt₃, 7/3) pour obtenir le trichloroacétimidate **2** sous forme d'un solide blanc (3,5 g, 87%).
**Voie 2 :** A une solution d'éthylènediamine (1,0 mL, 14,5 mmol, 1,4 éq.) dans du THF (180 mL) est additionné lentement de l'acide acétique (1,0 mL, 17,6 mmol, 1,7 éq.). Il en résulte immédiatement la formation d'un précipité. Ensuite le di-*O*-acétyle **14** (4,6 g, 10,4 mmol) est additionné au milieu réactionnel qui est agité à TA et la réaction est suivie par CCM (DCM/MeOH, 95/5 et Chex/EtOAc, 1/1). Après 20 h, la transformation de **14** (Rf = 0,7 et 0,75 dans Chex/EtOAc, 1/1) en un composé plus polaire est totale (Rf = 0,6 dans DCM/MeOH, 95/5 et 0,5 dans Chex/EtOAc, 1/1). Le milieu réactionnel est repris à l'acétate d'éthyle (150 mL) et est lavé à l'eau froide (2 x 25 mL) et la phase aqueuse est extraite à l'acétate d'éthyle (2 x 50 mL). Les phases organiques sont regroupées puis séchées sur Na₂SO₄ et concentrées à l'évaporateur rotatif pour conduire au composé désiré sous forme d'un solide blanc (4,2 g).

Le brut précédent (4,2 g, 10,4 mmol) est solubilisé dans du DCE (15 mL) et agité sous argon à -5°C puis de la DBU (434 µL, 2,9 mmol, 0,28 éq.) et du trichloroacétonitrile (4,5 mL, 44,6 mmol, 4,3 éq.) sont ajoutés. Le milieu réactionnel est agité à cette température pendant 15 min puis il est déposé, sans aucun traitement, sur une colonne de gel de silice (flash chromatographie, Chex/EtOAc+5% NEt₃, 7/3) pour obtenir le trichloroacétimidate **2** sous forme d'un solide blanc (4,6 g, 80%).
**2** : Rf = 0,5 (Chex/EtOAc, 7/3).
RMN ¹H (CDCl₃), *δ*: α : 8.78 (s, 1H, NH), 7.10 (d, 1H, *J*_{NH,2} = 8.5 Hz, NH), 6.45 (d, 1H, *J*_{1,2} = 3.7 Hz, H-1), 5.40 (pt, 1H, *J*_{2,3} = 9.2 Hz, H-3), 4.39 (m, 1H, H-2), 3.98-3.91 (m, 3H, H-4, H-5, H-6a), 3.81 (m, 1H, H-6b), 2.11 (s, 3H, H_{Ac}), 1.54 (s, 3H, H_{iPr}), 1.43 (s, 3H, B_{iPr}).
RMN ¹³C (CDCl₃), *δ*: α : 171.9 (C_{Ac}), 162.5 (C_{NTCA}), 160.7 (C=NH), 100.5 (C_{iPr}), 94.7 (C-1, ¹*J*_{CH} = 180.1 Hz), 92.2 (CCl₃), 91.0 (CCl₃), 71.4 (C-4), 69.9 (C-3), 67.0 (C-5), 62.3 (C-6), 55.0 (C-2), 29.3 (C_{iPr}), 21.2 (C_{Ac}), 19.4 (C_{iPr}).

L'oxazoline **2'**, résultant de la dégradation de **2** durant la purification, est isolé comme le second produit à éluer lors de chromatographie sur gel de silice.
**2'** : Rf = 0,4 (Chex/EtOAc, 7/3).
RMN ¹H (CDCl₃), *δ*: oxazoline : 7.18 (d, 1H, *J*_{NH,2} = 8.5 Hz, NH), 6.28 (d, 1H, *J*_{1,2} = 7.53Hz, H-1), 5.05 (m, 1H, H-3), 4.34 (m, 1H, H-2), 4.05 (m, 1H, H-6a), 3.88 (m, 1H, H-4), 3.76 (m, 1H, H-6b), 3.59 (m, 1H, H-5), 2.11 (s, 3H, H_{Ac}), 1.52 (s, 3H, H_{iPr}), 1.43 (s, 3H, H_{iPr}).
RMN ¹³C (CDCl₃), *δ*: oxazoline : 170.0 (C_{Ac}), 163.1 (N=C), 105.9 (C-1), 100.5 (C_{iPr}), 92.2 (CCl₃), 74.2 (C-3), 70.8 (C-4), 68.5 (C-2), 65.2 (C-5), 62.1 (C-6), 29.0 (C_{iPr}), 21.4 (C_{Ac}), 19.3 (C_{iPr}).

### ➢ Allyl 3-O-acétyl-2-désoxy-4,6-O-isopropylidène-2-trichloroacétamido-β-D-glucopyranoside 17 :

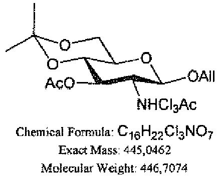

Du TMSOTf (360,0 µL, 2,0 mmol, 0,2 éq.) est additionné à une solution d'alcool allylique (1,4 mL, 20,1 mmol, 2 éq.) et de donneur **2** (5,5 g, 10,1 mmol, 1 éq.) dans du DCM (700 mL), en présence de tamis moléculaire 4 Å (3,9 g), agitée sous argon à - 78°C. Après 30 min sous agitation le suivi par CCM (Tol/EtOAc, 8/2) indique la disparition du donneur (Rf = 0,65) et l'apparition d'un nouveau composé plus polaire (Rf = 0,45). La réaction est stoppée par l'addition de triéthylamine (0,5 mL) puis le milieu réactionnel est filtré et concentré à l'évaporateur rotatif. L'huile jaune obtenue est purifiée par chromatographie sur gel de silice (Tol/EtOAc, 91→75/25) pour obtenir le glycoside d'allyle **17** sous forme d'un solide blanc (3,7 g, 82%).
Rf= 0,45 (Tol/EtOAc, 1/1).
RMN ¹H (CDCl₃), δ7.65 (d, 1H, *J*_{NH,2} = 9.6 Hz, NH), 5.81 (m, 1H, CH=), 5.38 (pt, 1H, *J*_{2,3} = 9.3 Hz, H-3), 5.25 (m, 1H, *J*ₜᵣₐₙₛ = 17.2 Hz, =CH₂), 5.15 (m, 1H, *J*_{cis} = 9.2 Hz, =CH₂), 4.62 (d, 1H, *J*_{1,2} = 8.3 Hz, H-1), 4.29 (m, 1H, H_{All}), 4.18 (m, 1H, H-2), 4.10 (m, 1H, H_{All}), 3.99 (dd, 1H, *J*_{5,6a} = 5.5 Hz, *J*_{6a,6b} = 10.8 Hz, H-6a), 4.17 (m, 1H, H-6b), 3.81 (pt, 1H, *J*_{3,4} = 9.5 Hz, H-4), 3.75 (ddd, 1H, *J*_{4,5} = 9.9 Hz, H-5), 2.08 (s, 3H, H_{Ac}), 1.51 (s, 3H, R_{iPr}), 1.38 (s, 3H, H_{iPr}).
RMN ¹³C (CDCl₃), *δ* 172.0 (C_{Ac}), 162.7 (C_{NTCA}), 133.7 (CH=), 118.0 (=CH₂), 100.9 (C-1, ¹*J*_{CH} = 158.1 Hz), 100.1 (C_{iPr}), 93.1 (CCl₃), 72.5 (C-3), 72.1 (C-4), 70.6 (C_{All}), 67.4 (C-5), 62.2 (C-6), 56.1 (C-2), 29.1 (C_{iPr}), 21.1 (C_{Ac}), 19.3 (C_{iPr}).

| | | |
|---|---|---|
| HRMS (ESI⁺) : | [M+Na]⁺ C₁₆H₂₂NO₇³⁵Cl₃Na | m/z théorique : 468.0359 |
| | | m/z mesurée : 468.0359 |

### ➢ Allyl 3,4,6-tri-O-acétyl-2-désoxy-2-trichloroacétamido-β-D-glucopyranoside 18 :

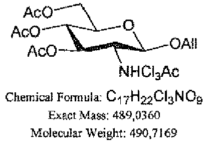

Du TMSOTf (830,0 µL, 4,6 mmol, 0,2 éq.) est additionné à une solution d'alcool allylique (3,1 mL, 46,0 mmol, 2 éq.) et de donneur **1** (13,7 g, 23,0 mmol, 1 éq.) dans du DCM (160 mL), en présence de tamis moléculaire 4 Å (8,9 g), agitée sous argon à - 78°C. Après 30 min sous agitation le suivi par CCM (Tol/EtOAc, 8/2) indique la disparition du donneur (Rf = 0,5) et l'apparition d'un nouveau composé plus polaire (Rf = 0,35). La réaction est stoppée par l'addition de triéthylamine (1 mL) puis le milieu réactionnel est filtré et concentré à l'évaporateur rotatif. L'huile jaune obtenue est purifiée par chromatographie sur gel de silice (Tol/EtOAc, 9/1→75/25) pour obtenir le glycoside d'allyle **18** sous forme d'un solide blanc (9,4 g, 83%).
Rf = 0,35 (Tol/EtOAc, 1/1).
RMN ¹H (CDCl₃), *δ* 6.98 (d, 1H, *J*_{NH,2} = 8.9 Hz, NH), 5.84 (m, 1H, CH=), 5.39 (pt, 1H, *J*_{2,3} = 9.3 Hz, H-3), 5.28 (m, 1H, *J*ₜᵣₐₙₛ = 17.2 Hz, =CH₂), 5.19 (m, 1H, *J*_{cis} = 10.4 Hz, =CH₂), 5.12 (pt, 1H, *J*_{3,4} = 9.6 Hz, H-4), 4.74 (d, 1H, *J*_{1,2} = 8.3 Hz, H-1), 4.36 (m, 1H, H_{All}), 4.29 (dd, 1H, *J*_{5,6a} = 5.0 Hz, *J*_{6a,6b} = 12.3 Hz, H-6a), 4.17 (dd, 2H, *J*_{5,6b} = 2.4 Hz, H-6b), 4.10 (m, 1H, H_{All}), 4.03 (m, 1H, H-2), 3.75 (ddd, 1H, *J*_{4,5} = 9.9 Hz, H-5), 2.10, 2.04, 2.03 (3s, 9H, H_{Ac}).
RMN ¹³C (CDCl₃), *δ* 171.3, 171.1, 169.7 (3C, C_{Ac}), 162.4 (C_{NTCA}), 133.6 (CH=), 118.4 (=CH₂), 99.7 (C-1, ¹*J*_{CH} = 155.5 Hz), 92.7 (CCl₃), 72.4 (C-5), 72.0 (C-3), 70.6 (C_{All}), 69.0 (C-4), 62.5 (C-6), 56.3 (C-2), 21.1, 21.0, 20.9 (3C, C_{Ac}).

| | | |
|---|---|---|
| HRMS (ESI⁺) : | [M+Na]⁺ C₁₇H₂₃NO₉³⁵Cl₃Na | m/z théorique : 512.0258 |
| | | m/z mesurée : 512.0258 |

### ➢ Allyl 2-acétamido-2-désoxy-4,6-0-isopropylidène-β-D-glucopyranoside⁴ 19

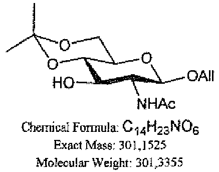

Après addition de NaOH 1 M (6,7 mL, 6,7 mmol, 3 éq.) au glycoside d'allyle **17** (1,0 g, 2,2 mmol) en solution dans du MeOH (40 mL), le milieu réactionnel est agité pendant une nuit et son évolution est suivie par CCM (DCM/MeOH, 9/1). Après avoir observé la disparition de **17** (Rf= 0,95) et l'apparition d'un produit plus polaire (Rf= 0,5), de l'anhydride acétique (630 µL, 6,7 mmol, 3 éq.) est ajouté au milieu réactionnel. Après 3 h d'agitation, Le solide jaune obtenu est purifié par chromatographie sur gel de silice (DCM/MeOH, 98/2→9/1) pour obtenir l'alcool **19** sous forme d'un solide blanc (476 mg, 71%).
Rf = 0,5 (DCM/MeOH, 9/1).
RMN ¹H (CDCl₃), *δ* 5.90 (m, 1H, CH=), 5.74 (d, 1H, *J*_{NH,2} = 4.6 Hz, NH), 5.32 (m, 1H, *J*ₜᵣₐₙₛ= 17.2 Hz, =CH₂), 5.26 (m, 1H, *J*_{cis}= 10.4 Hz, =CH₂), 4.68 (d, 1H, *J*_{1,2} = 8.3 Hz, H-1), 4.37 (m, 1H, H_{All}), 4.10 (m, 1H, H_{All}), 4.00-3.93 (m, 2H, H-3, H-6a), 3.82 (m, 1H, H-6b), 3.61 (pt, 1H, *J*_{3,4} = 9.4 Hz, H-4), 3.49 (m, 1H, H-2), 3.31 (ddd, 1H, *J*_{5,6b} = 5.4 Hz, *J*_{4,5} = 10.0 Hz, H-5), 2.07 (s, 3H, H_{NAc}), 1.55 (s, 3H, H_{iPr}), 1.47 (s, 3H, H_{iPr}).
RMN ¹³C (CDCl₃), *δ* 162.7 (C_{NAc}), 134.2 (CH=), 117.8 (=CH₂), 100.9 (C-1, ¹*J*_{CH} = 162.1 Hz), 100.1 (C_{iPr}), 74.6 (C-4), 71.8 (C-3), 70.5 (C_{All}), 67.5 (C-5), 62.4 (C-6), 57.9 (C-2), 29.4 (C_{iPr}), 23.7 (C_{NAc}), 19.5 (C_{iPr}).

| | | |
|---|---|---|
| HRMS (ESI⁺) : | [M+Na]⁺ C₁₄H₂₃NO₆Na | m/z théorique : 324.123 |
| | | m/z mesurée : 321.1412 |
| | [M+H]⁺ C₁₄H₂₃NO₆ | m/z théorique : 302.1604 |
| | | m/z mesurée: 302.1599 |

### ➢ Allyl 2-désoxy-4,6-O-isopropylidène-2-trichloroacétamido-β-D-glucopyranoside 20 :

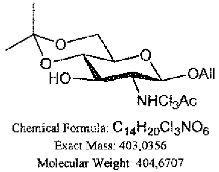

**Voie 1** : Après addition de NaOMe 0,5 M (42,0 mL, 20,9 mmol, 1,1 éq.) au glycoside d'allyle **18** (9,3 g, 19,0 mmol) en solution dans du MeOH (40 mL), le milieu réactionnel est agité pendant une nuit et son évolution est suivie par CCM (Tol/EtOAc, 8/2, DCM/MeOH, 9/1). Après avoir observé la disparition de **18** (Rf= 0,35 et 0,85, respectivement) et l'apparition d'un produit plus polaire (Rf= 0 et 0,3, respectivement), le milieu réactionnel est neutralisé par addition de résine échangeuse d'ion DOWEX (H⁺), puis filtré sur fritté. La résine est lavée avec un mélange DCM/MeOH et les solvants sont évaporés. Le solide jaune obtenu est engagé directement dans l'étape suivante (6,8 g).
   Dans du DMF (30 mL) est dissous le brut précédent (6,8 g, 19,0 mmol) et le 2-méthoxypropène (3,4 mL, 38,0 mmol, 2 éq.). Du CSA (1,2 g, 5,2 mmol, 0,15 éq.) est ajouté par fractions au milieu réactionnel. Sous agitation à TA, la réaction est suivie par CCM (Tol/EtOAc, 6/4 et DCM/MeOH, 95/5). Après 2h30 d'agitation, le MeOH formé est évaporé à l'évaporateur rotatif et du 2-méthoxypropène (1,7 mL, 19,0 mmol, 1 éq.) est de nouveau ajouté. Après 1 h d'agitation, la transformation du produit de départ en un produit moins polaire (Rf = 0,3 dans Tol/EtOAc, 6/4) est quasi-totale. Le milieu réactionnel est neutralisé avec de la triéthylamine (1 mL) puis concentré à l'évaporateur rotatif. Le solide obtenu est purifié par chromatographie sur gel de silice (Tol/EtOAc, 8/2→7/3) pour conduire à l'alcool **20** sous forme d'un solide blanc (6,2 g, 81%).
**Voie 2 :** Le glycoside d'allyle **17** (395 mg, 880 µmol) est dissous dans du MeOH (5 mL) puis du K₂CO₃ (37 mg, 260 µmol, 0,3 éq.) est ajouté au milieu réactionnel. Après 25 min sous agitation, le suivi par CCM (Chex/EtOAc, 6/4) indique la disparition de **17** (Rf= 0,45) et l'apparition d'un produit plus polaire (Rf = 0,25). Le milieu réactionnel est évaporé sous pression réduite. Le solide obtenu est purifié par chromatographie sur gel de silice (Chex/EtOAc, 8/2→1/1) pour conduire à l'alcool **20** sous forme d'un solide blanc (335 mg, 94%).
Rf = 0,3 (Tol/EtOAc, 6/4).
RMN ¹H (CDCl₃), δ7.26 (d, 1H, *J*_{NH,2} = 7.4 Hz, NH), 5.84 (m, 1H, CH=), 5.27 (m, 1H, *J*ₜᵣₐₙₛ = 17.2 Hz, =CH₂), 5.19 (m, 1H, *J*_{cis} = 10.4 Hz, =CH₂), 4.89 (d, 1H, *J*_{1,2} = 8.3 Hz, H-1), 4.33 (m, 1H, H_{All}), 4.20 (pt, 1H, *J*_{2,3} = 9.2 Hz, H-3), 4.08 (m, 1H, H_{All}), 3.93 (dd, 1H, *J*_{5,6a} = 5.4 Hz, *J*_{6a,6b} = 10.8 Hz, H-6a), 3.79 (m, 1H, H-6b), 3.56 (pt, 1H, *J*_{3□,4} = 9.3 Hz, H-4), 3.50 (m, 1H, H-2), 3.32 (ddd, 1H, J_{4,5} = 10.0 Hz, H-5), 1.52 (s, 3H, H_{iPr}), 1.42 (s, 3H, R_{iPr}).
RMN ¹³C (CDCl₃), *δ* 162.3 (C_{NTCA}), 133.3 (CH=), 118.4 (=CH₂), 99.9 (C_{iPr}), 98.9 (C-1, ¹*J*_{CH} = 165.0 Hz), 92.5 (CCl₃), 74.4 (C-4), 70.6 (C_{All}), 69.8 (C-3), 67.0 (C-5), 61.9 (C-6), 59.3 (C-2), 29.0 (C_{iPr}), 19.1 (C_{iPr}).

| | | |
|---|---|---|
| HRMS (ESI⁺) : | [M+Na]⁺ C₁₄H₂₀NO₆³⁵Cl₃Na | m/z théorique : 426.0254 |
| | | m/z mesurée : 426.0252 |

### ➢ Allyl 2-désoxy-4,6-O-isopropylidène-3-O-lévulinoyl-2-trichloroacétamido-β-D-glucopyranoside 24 :

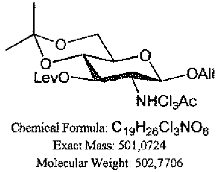

L'alcool **20** (5,9 g, 14,6 mmol) est dissous dans du DCM (100 mL) puis la DMAP (3,6 g, 29,3 mmol, 2 éq.) est ajouté au milieu réactionnel. Dans un autre ballon, la DCC (4,5 g, 22,0 mmol, 1,5 éq.), et l'acide lévulinique (2,7 mL, 26,4 mmol, 1,8 éq.) sont agités dans du DCM (100 mL) puis ajoutés au milieu réactionnel. Le milieu réactionnel est alors agité durant 30 min, temps au bout duquel le suivi par CCM (Tol/EtOAc, 7/3) montre la formation d'un composé moins polaire majoritaire (Rf = 0,35) et la disparition de **20** (Rf= 0,25). La DCU est filtrée sur célite et le filtrat est repris à l'H₂O (50 mL). La phase aqueuse est extraite au DCM (3 x 200 mL). Les phases organiques sont regroupées et lavées avec une solution de NaHCO₃ₛₐₜ (3 x 100 mL), une solution de NaClₛₐₜ (3 x 100 mL), filtrées sur filtre séparateur de phases et concentrées à l'évaporateur rotatif. L'huile obtenue est purifiée par chromatographie sur gel de silice (Chex/EtOAc, 85/15→75/25) pour conduire à l'ester de lévulinoyle **24** (7,3 g, 98%).
Rf = 0,35 (Tol/EtOAc, 7/3).
RMN ¹H (CDCl₃), *δ* 7.50 (d, 1H, *J*_{NH,2} = 9.4 Hz, NH), 5.82 (m, 1H, CH=), 5.34 (pt, 1H, *J*_{2□,3} = 9.6 Hz, H-3), 5.24 (m, 1H, *J*ₜᵣₐₙₛ = 17.2 Hz, =CH₂), 5.13 (m, 1H, *J*_{cis} = 10.5 Hz, =CH₂), 4.66 (d, 1H, *J*_{1,2} = 8.3 Hz, H-1), 4.29 (m, 1H, H_{All}), 4.11 (m, 1H, H-2), 4.06 (m, 1H, H_{All}), 3.96 (dd, 1H, *J*_{5,6a} = 5.4 Hz, *J*_{6a,6b} = 10.7 Hz, H-6a), 3.81 (m, 1H, H-6b), 3.78 (pt, 1H, *J*_{3□,4} = 9.6 Hz, H-4), 3.60 (ddd, 1H, *J*_{4,5} = 9.9 Hz, H-5), 2.72 (m, 2H, CH_{2Lev}), 2.58 (m, 2H, CH_{2Lev}), 2.14 (s, 3H, CH_{3Lev}), 1.50 (s, 3H, H_{iPr}), 1.36 (s, 3H, H_{iPr}).
RMN ¹³C (CDCl₃), *δ* 205.5 (C_{Lev}), 173.1 (C_{Lev}), 162.3 (C_{NTCA}), 133.3 (CH=), 117.5 (=CH₂), 100.4 (C-1, ¹*J*_{CH} = 162.5 Hz), 99.8 (C_{iPr}), 92.7 (CCl₃), 72.1 (C-3), 71.7 (C-4), 70.4 (C_{All}), 66.9 (C-5), 61.9 (C-6), 56.0 (C-2), 37.9 (CH_{2Lev}), 29.6 (CH_{3Lev}), 28.7 (C_{iPr}), 28.1 (CH_{2Lev}), 18.9 (C_{iPr}).

| | | |
|---|---|---|
| HRMS (ESI⁺) : | [M+Na]⁺ C₁₉H₂₆NO₈³⁵Cl₃Na | m/z théorique : 524.0622 |
| | | m/z mesurée : 524.0657 |
| | [M+NH₄]⁺ C₁₉H₂₆NO₈³⁵Cl₃NH₄ | m/z théorique : 519.1068 |
| | | m/z mesurée : 519.1097 |

### ➢ -Désoxy-4,6-O-isopropylidène-3-O-lévulinoyl-2-trichloroacétamido-β-D-glucopyranose 25 :

### ➢ Trichloracétimidate de 2-désoxy-4,6-O-isopropylidène-3-O-lévulinoyl-2-trichioroacétamido-α-D-glucopyranose 3 :

### ➢ 2-Trichlorométhyl-(1,2-didésoxy-4,6-O-isopropylidène-3-O-lévulinoyl-α-D-glucopyrano)-[2,1-d]-2-oxazoline 3':

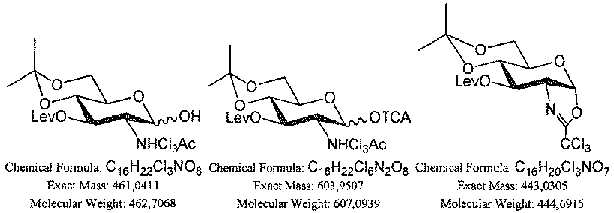

Le (1,5-cyclooctadiènebis(méthyldiphénylphosphine)iridium(I)hexafluorophosphate) (360 mg) est dissous dans du THF (100 mL) sous agitation. Le milieu subit 5 cycles dégazage vide/argon, 5 cycles dégazage vide/hydrogène puis est mis sous hydrogène pendant 15 min. Le milieu subit alors une seconde fois 5 cycles dégazage vide/argon. La solution de l'ester lévulinoyle **24** (6,0 g, 11,9 mmol) dans du THF (15 mL) est transféré sur la solution de catalyseur activé. Le suivi par CCM (Tol/EtOAc, 6/4) montre la disparition de **24** (Rf= 0,4) et l'apparition d'un produit un peu moins polaire (Rf = 0,45).

Du diiode (6,0 g, 23,8 mmol, 2 éq.) et du NaHCO₃ (3,0 g, 35,7 mmol, 3 éq.) en solution dans un mélange THF/H₂O (8/2, 70 mL) est ajouté au milieu réactionnel. Le suivi par CCM (Tol/EtOAc, 6/4) indique la disparition de l'intermédiaire (Rf = 0,45) et l'apparition d'un nouveau composé beaucoup plus polaire (Rf = 0.3). Pour stopper la réaction, une solution aqueuse de NaHSO₃ 10% (60 mL) est ajoutée par fractions au milieu jusqu'à disparition de la coloration noire. Le milieu réactionnel est repris avec du DCM et la phase aqueuse est extraite au DCM (3 x 200 mL). Les phases organiques sont regroupées et lavées avec une solution de NaClₛₐₜ (3 x 100 mL), avec de l'H₂O (3 x 100 mL) puis séchées sur filtre séparateur de phases et concentrée à l'évaporateur rotatif. L'huile jaune obtenue (5,5 g), correspondant à l'hémiacétal **25,** est engagée directement sans aucune purification dans l'étape suivante. **25** : Rf = 0,3 (Tol/EtOAc, 6/4).
RMN ¹H (CDCl₃), *δ* 7.16 (m, 1H, NH), 5.33-5.28 (m, 2H, H-3, H-1), 4.44 (bs, 1H, OH), 4.16 (dd, 1H, *J*_{1,2} = 3.6 Hz, *J*_{2,3} = 10.0 Hz, H-2), 3.99 (ddd, 1H, *J*_{4,5} = 10.1 Hz, H-5), 3.88 (dd, 1H, *J*_{5,6a} = 5.2 Hz, *J*_{6a,6b} = 10.7 Hz, H-6a), 3.82-3.76 (m, 2H, H-4, H-6b), 2.71 (m, 2H, CH_{2Lev}), 2.61 (m, 2H, CH_{2Lev}), 2.17 (s, 3H, CH_{3Lev}), 1.51 (s, 3H, H_{iPr}), 1.39 (s, 3H, H_{iPr}).
RMN ¹³C (CDCl₃), *δ* 206.4 (C_{Lev}), 173.0 (C_{Lev}), 162.2 (C_{NTCA}), 100.0 (C_{iPr}), 92.1 (CCl₃), 91.6 (C-1, ¹*J*_{CH} = 158.9 Hz), 71.8 (C-4), 70.3 (C-3), 63.7 (C-5), 62.2 (C-6), 54.8 (C-2), 37.9 (CH_{2Lev}), 29.8 (CH_{3Lev}), 28.9 (C_{iPr}), 28.0 (CH_{2Lev}), 19.1 (C_{iPr}).

| | | |
|---|---|---|
| HRMS (ESI⁺) : | [M+Na]⁺ C₁₆H₂₂NO₈³⁵Cl₃Na | m/z théorique : 484.0309 |
| | | m/z mesurée : 484.0332 |
| | [M+NH₄]⁺ C₁₉H₂₆NO₈³⁵Cl₃NH₄ | m/z théorique : 479.0755 |
| | | m/z mesurée : 479.0780 |

Le brut **25** (5,5 g, 11,9 mmol) est solubilisé dans du DCE (50 mL) et agité sous argon à -5°C puis de la DBU (500 µL, 3,3 mmol, 0,28 éq.) et du trichloroacétonitrile (5,9 mL, 59,5 mmol, 5 éq.) sont ajoutés. Le milieu réactionnel est agité à cette température pendant 15 min puis il est déposé, sans aucun traitement, sur une colonne de gel de silice (flash chromatographie, Tol/EtOAc+5% NEt₃, 9/1→7/3) pour obtenir le trichloroacétimidate **3** sous forme d'un solide blanc (6,1 g, 84%).
Rf = 0,55 (Tol/EtOAc, 6/4).
RMN ¹H (CDCl₃), *δ* 8.76 (s, 1H, NH), 7.09 (d, 1H, *J*_{NH,2} = 8.3 Hz, NH), 6.42 (d, 1H, *J*_{1,2} = 3.7 Hz, H-1), 5.33 (pt, 1H, *J*_{2,3} = 10.4 Hz, *J*_{3,4} = 9.2 Hz, H-3), 4.36 (m, 1H, H-2), 3.92 (dd, 1H, *J*_{5,6a} = 4.7 Hz, *J*_{6a,6b} = 9.2 Hz, H-6a), 3.90-3.84 (m, 2H, H-4, H-5), 3.81 (m, 1H, H-6b), 2.72 (m, 2H, CH_{2Lev}), 2.61 (m, 2H, CH_{2Lev}), 2.15 (s, 3H, CH_{3Lev}), 1.51 (s, 3H, H_{iPr}), 1.39 (s, 3H, H_{iPr}).
RMN ¹³C (CDCl₃), *δ* 205.8 (C_{Lev}), 173.6 (C_{Lev}), 162.1 (C_{NTCA}), 160.2 (C=NH), 100.2 (C_{iPr}), 94.2 (C-1, ¹*J*_{CH} = 180.9 Hz), 91.7 (CCl₃), 90.5 (CCl₃), 71.0 (C-4), 69.7 (C-3), 66.5 (C-5), 61.8 (C-6), 54.6 (C-2), 37.8 (CH_{2Lev}), 29.7 (CH_{3Lev}), 28.8 (C_{iPr}), 27.9 (CH_{2Lev}), 19.0 (C_{iPr}).

L'oxazoline **3',** résultant de la dégradation de **3** durant la purification, est isolé comme le second produit à éluer lors de chromatographie sur gel de silice.
Rf = 0,45 (Tol/EtOAc, 6/4).
RMN ¹H (CDCl₃), *δ* 6.26 (d, 1H, *J*_{1,2} = 7.5 Hz, H-1), 5.01 (dd 1H, *J*_{2□,3} = 3.4 Hz, *J*_{3,4} = 7.8 Hz, H-3), 4.37 (dd, 1H, H-2), 4.00 (m, 1H, *J*_{5,6a} = 5.6 Hz, *J*_{6a,6b} = 11.0 Hz, H-6a), 3.90 (pt, 1H, *J*_{4,5} = 10.2 Hz, H-4), 3.76 (pt, 1H, H-6b), 3.49 (m, 1H, H-5), 2.81 (m, 2H, CH_{2Lev}), 2.64 (m, 2H, CH_{2Lev}), 2.19 (s, 3H, CH_{3Lev}), 1.50 (s, 3H, H_{iPr}), 1.39 (s, 3H, H_{iPr}).
RMN ¹³C (CDCl₃), *δ* 206.6 (C_{Lev}), 171.9 (C_{Lev}), 162.7 (N=C), 105.2 (C-1), 100.1 (C_{iPr}), 86.2 (CCl₃), 74.0 (C-3), 70.5 (C-4), 68.0 (C-2), 64.4 (C-5), 61.7 (C-6), 38.0 (CH_{2Lev}), 29.8 (CH_{3Lev}), 28.7 (C_{iPr}), 28.0 (CH_{2Lev}), 18.8 (C_{iPr}).

### Partie II - Synthèse des cibles I, II, III, IV, V et VI

Dans cette seconde partie sont décrits les procédés conduisant aux saccharides I, II, III, IV, V VI.

Cette partie présente la synthèse des fragments di-, tri-, tétra- et pentasaccharides cibles en série **A** réducteur avec ou non la présence de l'acétyle en position 2_{C}.

| | Accepteur | Donneur | Cible |
|---|---|---|---|
| Procédé 1 | 31 | 33 | ***I*** |
| Procédé 2 | 21 | 1 ou 2 | ***II*** |
| Procédé 3 | 38 | 39 | ***III*** |
| Procédé 4 | 38 | 39 | ***IV*** |
| Procédé 5 | 38 | 46 ou 47 | ***V*** |
| Procédé 6 | 38 | 46 ou 47 | ***VI*** |

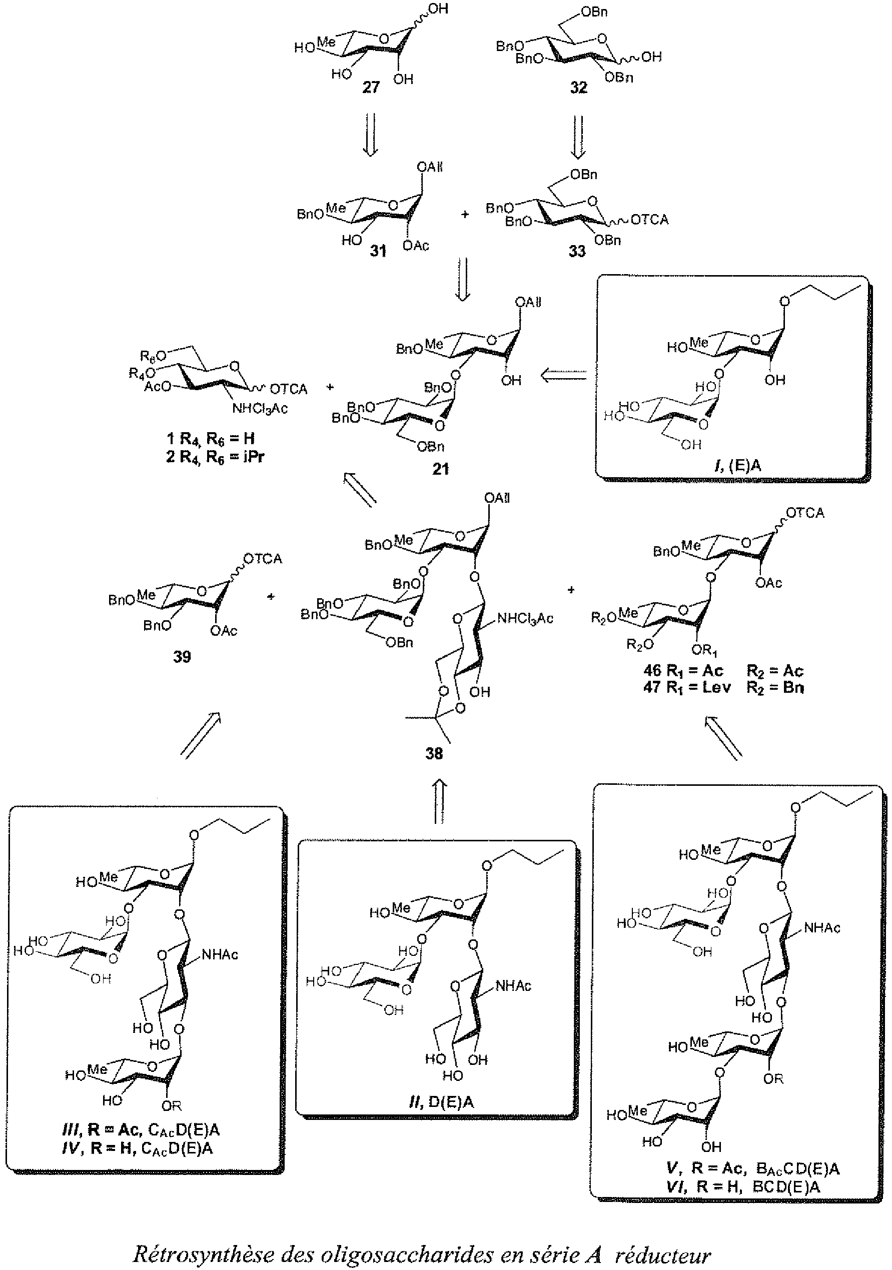

### Procédé 1 :

Selon un autre de ses objets, la présente description se rapporte à un procédé de préparation du disaccharide (E)A (I), produit intermédiaire dans la synthèse d'un saccharide (oligo- ou polysaccharide) tel que défini dans la liste L1, caractérisé en ce qu'il comprend les étapes suivantes :
- condensation du monosaccharide accepteur **31** avec le monosaccharide donneur **33** conduisant au disaccharide **35,** de préférence la réaction de condensation est réalisée dans un solvant tel que le dichlorométhane, le 1,2-dichloroéthane, l'éther, le toluène, l'acétonitrile ou tout autre solvant équivalent, en présence d'un acide de Lewis tel que le TMSOTf ou l'acide triflique, de préférence, l'éther et à une température inférieure ou égale à -20°C (schémas 14 et 15) ;
- désacétylation du disaccharide **35** pour conduire au disaccharide **21,** de préférence, en présence de NaOMe à reflux du méthanol (schémas 14 et 15) ;
- déprotection du disaccharide **21** par hydrogénolyse des groupes benzyles pour conduire au disaccharide (E)A, de préférence, sous pression d'hydrogène dans un alcool tel que l'éthanol en présence de dérivé de palladium, par exemple le palladium sur charbon (schéma 16).

### 1. Synthèse de l'accepteur 31

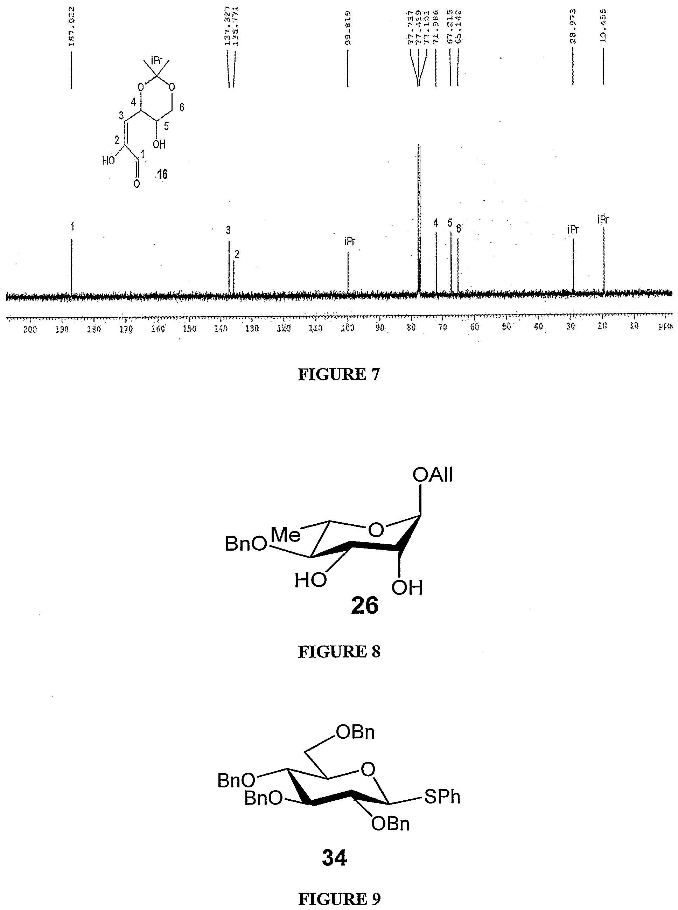

Dans un premier temps, une synthèse rapide et efficace de quantités importantes du diol **26** a été mise au point en s'appuyant sur les nombreuses données disponibles dans la littérature et les travaux antérieurs du laboratoire⁴²⁻⁴⁵ (Figure 8).

La synthèse est réalisée en 4 étapes.⁴² Pour la première étape de glycosylation, de nombreux acides ont été utilisés, dans la littérature, pour catalyser cette réaction tels que l'acide trifluoromethanesulfonique⁴³ (TMSOTf) ou l'acide chlorhydrique.⁴² D'autres réactifs permettant de générer *in situ* de l'acide chlorhydrique ont aussi été employés comme par exemple le chlorure d'acétyle.⁴⁶ Dans notre cas, la glycosylation du L-rhamnose monohydraté **27,** en présence d'un excès d'alcool allylique, a été testée en présence de deux promoteurs différents : le TMSOTf et l'acide chlorhydrique issu de l'action de l'alcool allylique sur le chlorure d'acétyle.

L'emploi du TMSOTf a permis d'isoler le triol **28** avec un rendement de 64%. Le chlorure d'acétyle s'est montré plus efficace. En effet, dans ce cas, l'anomère α est obtenu avec un rendement de 89% dans un rapport α/β de 90/10 soit un rendement global de 98%. Le glycoside d'allyle **28** obtenu est ensuite protégé régiosélectivement en position 2 et 3 par une fonction isopropylidène en présence de 2,2-diméthoxypropane et d'un catalyseur acide (APTS) dans l'acétone. L'acétal est introduit au niveau des positions 2 et 3 car il s'agit de diols *cis*-vicinaux et ces derniers forment des cycles à 5 plus stables que les diols *trans*-vicinaux, L'acétal **29,** obtenu avec un rendement de 84%, est ensuite benzylé sur la fonction hydroxyle en position 4 par action de l'alcoolate, issu de la déprotonation de l'alcool **29** par l'hydrure de sodium, sur le bromure de benzyle. L'intermédiaire complètement protégé **30** est isolé avec un rendement de 82%. Enfin, la fonction isopropylidène de l'intermédiaire **30** est clivée par hydrolyse acide pour régénérer les fonctions hydroxyles libres en position 2 et 3 et conduire au diol **26** souhaité avec un rendement de 72% (Schéma 10).

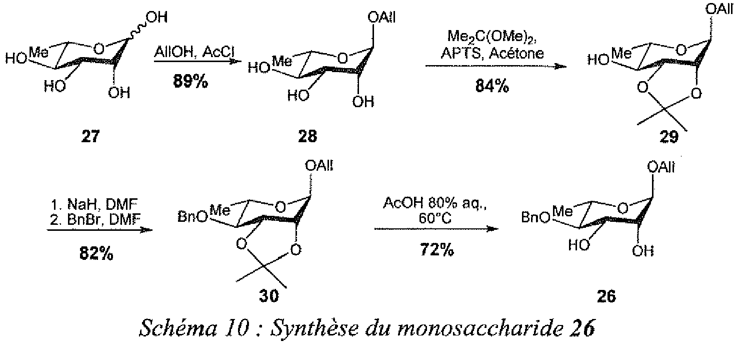

Après avoir réalisé la synthèse du diol **26** en purifiant et caractérisant chacun des intermédiaires **28, 29** et **30,** la synthèse a été reproduite sans chromatographie intermédiaire. Le diol attendu **26** est simplement isolé par recristallisation avec un rendement global de 78% nettement supérieur à celui obtenu précédemment (44% sur les 4 étapes) et celui de la littérature, qui enchaînait aussi les quatre étapes⁴³ (46%) (Schéma 11). De façon intéressante, le caractère cristallin du diol **26** permet d'enchaîner la séquence des 4 étapes de protection/déprotection sur des quantités importantes (20 g, par exemple).

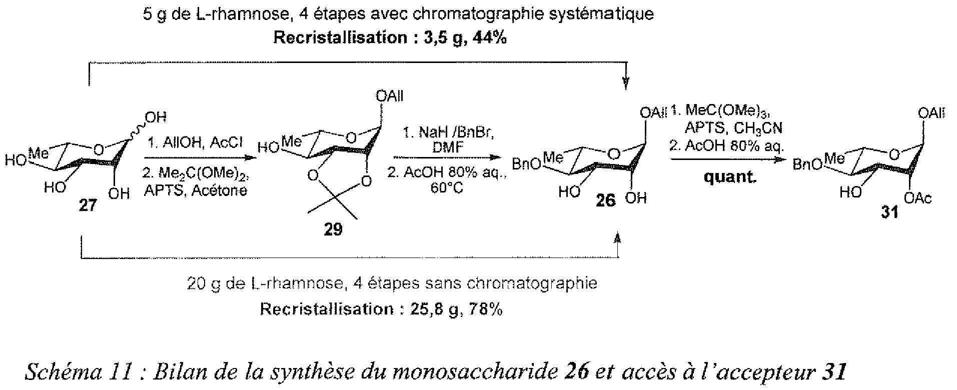

La dernière étape avant le couplage glycosidique avec un donneur glucose consiste en une protection régiosélective de la position 2.^{47,48} Du fait du caractère *cis-*vicinal des hydroxyles 2 et 3 et de leur différence de basicité, la position 2 peut aisément être protégée sélectivement par ouverture régiosélective d'un orthoester cyclique intermédiaire. Ainsi, le diol **26** réagit avec l'orthoacétate de triméthyle en présence d'APTS, pour être converti en accepteur **31** *via* une étape d'hydrolyse acide. L'accepteur **31** est obtenu sous forme d'un mélange de régioisomères 2-OAc/3-OAc dans un rapport 97/3 (rapport déterminé par RMN ¹H en se basant sur le signal du H-6 du rhamnose). Cet accepteur n'étant pas très stable sur silice, il est utilisé tel quel dans l'étape de couplage (une migration de la fonction acétyle du OH-2 vers le OH-3 en milieu acide a été observée précédemment au laboratoire^{45, 49}). Un spectre RMN ¹H effectuée avant le couplage permet de vérifier que l'acétyle n'a pas migré (Schéma 11).

### 2. Donneurs de glucose 33 et 34

Le 2,3,4,6-tétra-*O*-benzyl-β-D-glucopyranose commercial **32** réagit à température ambiante avec du trichloroacétonitrile en présence de K₂CO₃ dans du dichlorométhane pour engendrer le donneur **33,** activé en position anomérique. La réaction est réalisée sous contrôle cinétique,^{50,34} à TA et en présence d'une base faible afin de favoriser la formation de l'anomère β³⁵ qui cristallise, légèrement contaminé par l'anomère α (α/β : 7/93 rapport déterminé par RMN ¹H en se basant sur le signal du proton anomérique). Le donneur **33** est obtenu avec un rendement de 76% (Schéma 12). Le trichloroacétimidate est instable sur silice, ce qui rend le suivi de la réaction difficile, mais, une fois cristallisé, il peut être conservé au congélateur pendant de longues périodes.

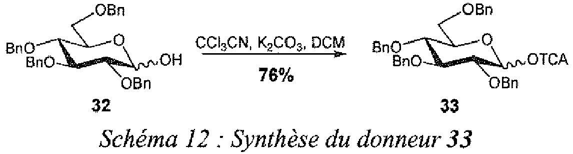

Pour connaître l'influence du donneur utilisé lors des réactions de glycosylation, le donneur thiophényle **34** (Figure 9), disponible au laboratoire, a aussi été utilisé.

### 3. Disaccharide (E)A accepteur 21

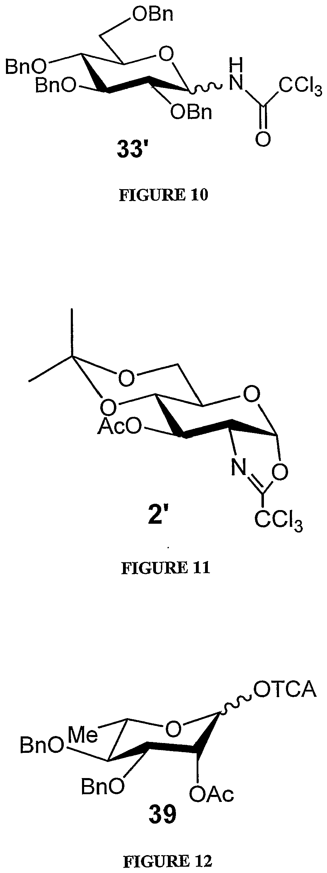

Le disaccharide **35**^{45,49,51} est obtenu par condensation de l'accepteur **31** et du donneur **33.** Il a été montré, dans la littérature qu'une liaison de type 1,2*-cis* est obtenu avec une meilleure stéréosélectivité en utilisant la procédure inverse de Sehmidt^{23, 52} *i .e.* le donneur est ajouté goutte à goutte au milieu réactionnel comprenant accepteur et activateur. Ainsi, le réarrangement du donneur **33** en **33**'⁵³ selon le réarrangement dit « de Chapman »⁵⁴⁻⁵⁶ est limité (Figure 10).

Le mécanisme simplifié de la réaction implique une compétition entre une réaction de type S_{N}¹ et une réaction de type S_{N}² comme le montre le Schéma 13.

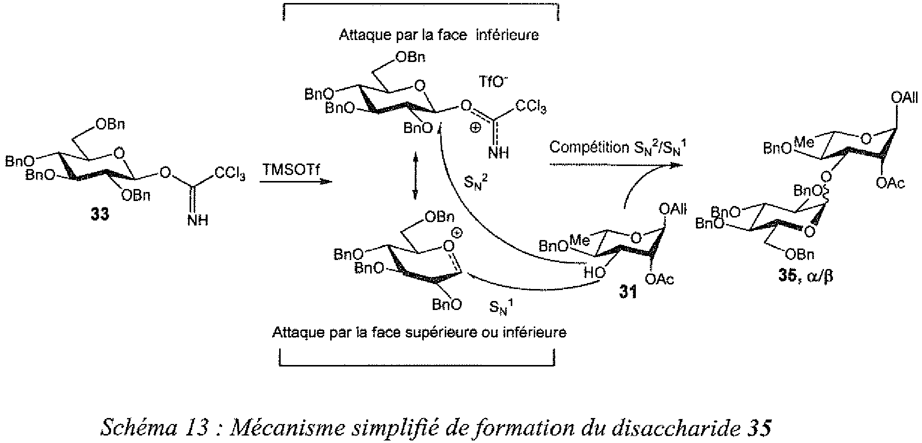

Pour obtenir majoritairement une liaison 1,2*-cis,* le mécanisme de type S_{N}² doit être favorisé. Ainsi, les influences du solvant (Ether ou Toluène), du catalyseur (TMSOTf ou BF₃.OEt₂), de la température d'addition du donneur et de la stéréochimie du donneur ont été étudiées (Tableau 2).

Au vu du Tableau 2, les conclusions suivantes peuvent être tirées :
- En comparant les entrées 1 à 5 et 6 à 10, les meilleures stéréosélectivités sont observées en utilisant du TMSOTf comme catalyseur et le toluène comme solvant de glycosylation ;
- Les entrées 4 et 9 montrent qu'augmenter la quantité de catalyseur ou ajouter du tamis moléculaire n'augmente ni la stéréosélectivité ni le rendement ;
- La comparaison entre les entrées 2 et 7 avec, respectivement, les entrées 1 et 6 montre que plus la température d'addition du donneur est basse, plus la stéréosélectivité est bonne ce qui prouve qu'un mécanisme de type S_{N}² est favorisé à basse température ;
- Comme attendu, si le mécanisme de type S_{N}² est favorisé, alors utiliser un donneur sous forme d'un mélange α/β n'améliore pas la stéréosélectivité (entrée 3) ;
- Le donneur thiophényle ne permet pas d'obtenir une meilleure stéréosélectivité que le donneur trichloroacétimidate (entrée 5).

**Tableau 2 : Optimisation de la glycosylation du disaccharide 35**

| **Entrée** | **Solvant** | **Catalyseur** | **Donneur**** | **Température d'ajout du donneur***** | **Rendement et Rapport α/β*** |
|---|---|---|---|---|---|
| **1** | Et₂O | TMSOTf 0,02 eq. | **33β** 1,2 éq. | TA | n. d., 60/40 |
| **2** | | | | -20°C | 80%, 70/30 |
| **3** | | | **33_{α/β}** (4/6) 1,2 éq. | | 58%, 70/30 |
| **4** | | TMSOTf 0,5 éq. | **33_{β}** 1,2 éq. | -78°C | n. d., 65/35 |
| **5** | | NIS TMSOTf 0,3 éq. | 1,2 éq. | 0°C | 77%, 55 /45 |
| **6** | Tol | **TMSOTf 0,02 éq.** | **33_{β} 1,2 éq.** | -40°C | 70%, 80/20 |
| **7** | | | | **-78°C** | **82%, 85/15** |
| **8** | | | **33_{β}** 1,5 éq. | | 79%, 85/15 |
| **9** | | TMSOTf 0,1 éq. (Tamis) | **33**_{β} 1,2 éq. | -78°C | 80%, 75/25 |
| **10** | | BF₃.OEt₂ 1 éq. | **33_{β}** 1,2 éq. | -45°C | 35%, 75/25 |

| | | | | | |
|---|---|---|---|---|---|
| * n. d. : non déterminé. ** Le donneur est solubilisé dans un minimum de DCM puis dissout dans le solvant de couplage (2/1). *** Toutes les réactions ont été arrêtées après retour à TA. | | | | | |

Au vu de ces résultats, les conditions optimums retenues sont donc : toluène comme solvant, donneur sous sa forme anomère β, TMSOTf (0,02 éq.) comme catalyseur, utilisation de la procédure inverse de Schmidt.

Après avoir isolé grossièrement un mélange d'anomères α/β (35), difficilement séparables, ce mélange est désacétylé en présence de NaOMe à reflux du méthanol car la fonction acétyle à cliver est isolée et encombrée. Les diastéréoisomères sont isolés par chromatographie sur gel de silice à cette étape (Schéma 14). Le disaccharide **21** de configuration α (¹*J*_{CH}= 166,3 Hz) est obtenu avec un rendement de 58% et le disaccharide **36** de configuration β (*J*_{1,2} = 7,5 Hz, ¹*J*_{CH} = 159,5 Hz) avec un rendement de 12%.

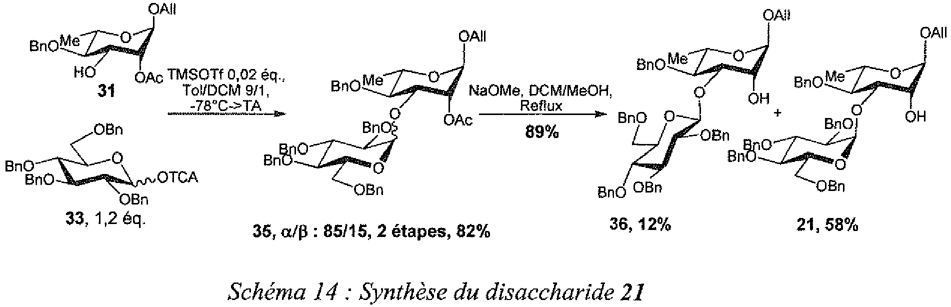

Dans un deuxième temps, ce couplage a été réalisé sur 8 g (27 mmol) d'accepteur **1** au lieu de 500 mg. Le rendement en disaccharide **21** est légèrement augmenté (61%) sur les 3 étapes soit 48% de rendement en anomère α à partir du L-rhamnose commercial. De plus, lors de la purification grossière à l'étape de désacétylation, 10% de diol **26,** issu de la transestérification de **31** qui n'a pas réagi lors de l'étape de couplage sont isolés, ce qui conduit à un rendement corrigé en disaccharide **21** de 69% sur les 3 étapes (Schéma 15).

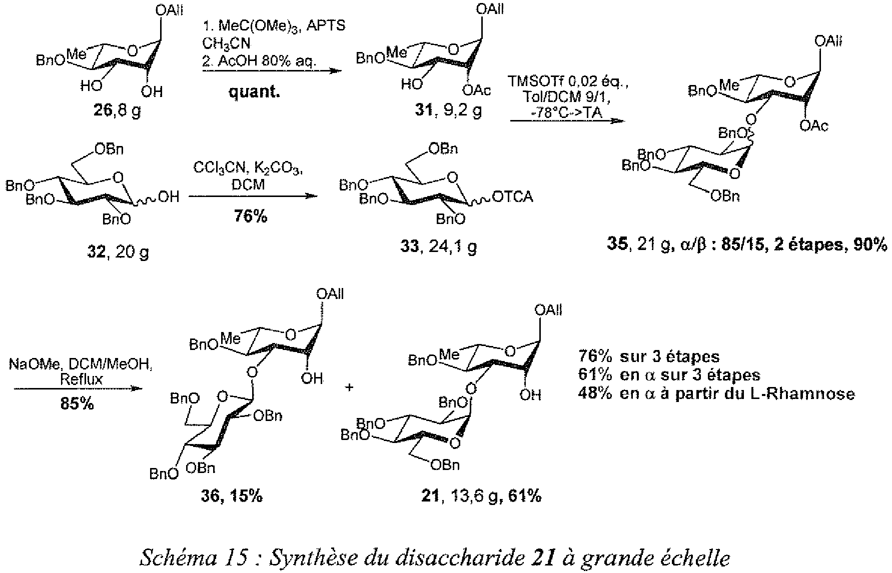

Une autre possibilité d'accès au disaccharide **21** est inspirée des travaux de Nifant'ev *et al.*⁵⁷ Ce dernier a montré que des couplages en position 3 du rhamnose pouvaient être réalisés sur le diol *cis* sans protection préalable de la fonction hydroxyle axiale. L'avantage de cette stratégie est de réduire le nombre d'étapes de protection/déprotection même si la formation de régioisomères, couplés en position 2 ou 3, et du trisaccharide est observée. Les premiers essais de couplage réalisés au laboratoire entre le donneur **6** et le diol accepteur **1** ont montré la présence d'un mélange complexe de produits. Il est évident qu'en l'absence de groupement participant, la formation des deux régioisomères α, couplés en position 2 ou 3, ainsi que celle des deux stéréoisomères β correspondants, pouvaient être attendues. Cette stratégie a donc été rapidement abandonnée.

### 4. Déprotection finale du disaccharide (E)A 21

L'hydrogénolyse^{58, 59} des groupes benzyles du disaccharide **21** est effectuée sous pression d'hydrogène dans l'éthanol en présence de palladium sur charbon en milieu légèrement acide pour conduire au glycoside de propyle ***I*** avec un rendement de 83%.

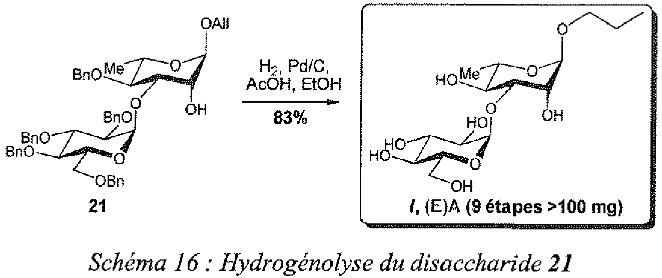

### Procédé 2 :

La description se rapporte également au procédé de préparation du trisaccharide D(E)A (II), oligosaccharide produit intermédiaire dans la synthèse d'un oligo- ou polysaccharide tel que défini dans la liste L1, caractérisé en ce qu'il comprend les étapes suivantes :
- condensation du monosacccharide donneur **1** avec le disaccharide accepteur **21** conduisant au trisaccharide **22,** de préférence la réaction de condensation est réalisée dans un solvant tel que le dichlorométhane, le 1,2-dichloroéthane, l'éther, le toluène, l'acétonitrile ou tout autre solvant équivalent, de préférence, dans le dichlorométhane en présence d'un acide de Lewis tel que le TMSOTf ou l'acide triflique (schéma 17) ;
- désacétylation du trisaccharide **22** conduisant au trisaccharide **37,** de préférence, en présence de NaOMe (schéma 19) ;
- déprotection du trisaccharide **37** par hydrogénolyse acide suivie d'une hydrodéchloration pour conduire au trisaccharide D(E)A, de préférence, sous pression d'hydrogène en présence de palladium sur charbon en milieu basique (schéma 20).

La présente description se rapporte encore au procédé de préparation du trisaccharide D(E)A (II), oligosaccharide produit intermédiaire dans la synthèse d'un oligo- ou polysaccharide tel que défini dans la liste L1 comprenant les étapes suivantes :
- condensation du monosacccharide donneur **2** avec le disaccharide accepteur **21** conduisant au trisaccharide **23,** de préférence la réaction de condensation est réalisée dans un solvant tel que le dichlorométhane, le 1,2-dichloroéthane, l'éther, le toluène, l'acétonitrile ou tout autre solvant équivalent, en présence d'un acide de Lewis tel que le TMSOTf ou l'acide triflique, de préférence, dans le dichlorométhane, en présence de TMSOTf à 0,3 eq (schéma 18) ;
- désacétylation du trisaccharide **23** conduisant au trisaccharide accepteur **38,** de préférence, en présence de NaOMe (schéma 19) ;
- clivage du groupement isopropylène du trisaccharide **38** par hydrolyse acide conduisant au trisaccharide **37** ;
- déprotection du trisaccharide **37** pour conduire au trisaccharide D(E)A, de préférence par hydrogénolyse acide suivie d'une hydrodéchloration sous pression d'hydrogène en présence d'un dérivé de palladium tel que le palladium sur charbon en milieu basique.

### 5. Trisaccharide D(E)A accepteur 38

### a. Accès au trisaccharide accepteur 38 via le glycoside d'allyle 22

Outre la synthèse d'un trisaccharide accepteur pouvant être allongé en position 3_{D} ou 1_{A}, notre objectif a été de déterminer si l'introduction d'une fonction isopropylidène sur les positions 4_{D} et 6_{D} est plus efficace au stade du monosaccharide ou au stade du trisaccharide.

Dans un premier temps, la condensation entre le donneur **1** et l'accepteur **21** est réalisée dans le dichlorométhane en présence de TMSOTf (0,2 éq.) et de tamis moléculaire. Le trisaccharide **22** est obtenu avec la configuration β attendue (¹*J*_{CH} = 161,0 Hz) et un rendement de 95%, confirmant le rôle de groupe participant du trichloroacétamide (Schéma 17) ainsi que l'efficacité, comme déjà observé dans les séries précédentes, de **1** en tant que donneur.

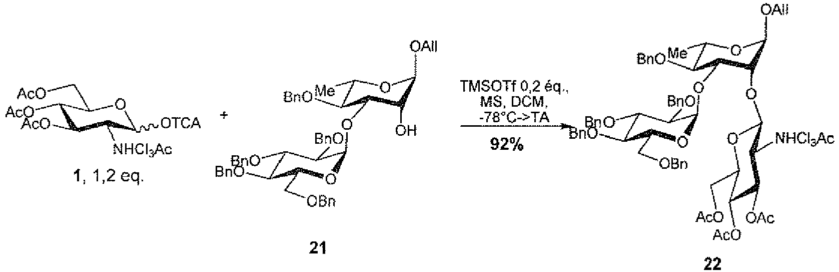

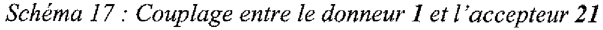

Le glycoside d'allyle **22** est désacétylé en présence de NaOMe dans les conditions de Zemplen⁶⁰ pour obtenir le triol **37** avec un rendement de 94%. Ce dernier est ensuite protégé régiosélectivement en position 4_{D} et 6_{D} par un groupement isopropylidène en présence de 2-méthoxypropène^{30, 31} et de CSA pour conduire à l'accepteur **38** avec un rendement de 76% (Schéma 19). Il est à noter, que l'isopropylidène de l'accepteur **38** s'est révélé instable en milieu légèrement acide (par exemple, le CDCl₃ utilisé pour l'analyse RMN). Un équilibre entre **38** et le triol **37** (Rapport 65/35 : **38/37)** formé de façon plus ou moins rapide en fonction de la température de l'échantillon mais aussi de sa concentration est observé.

### b. Accès au trisaccharide accepteur 38 via le glycoside d'allyle 23

Lorsque les conditions de couplage utilisées précédemment (dichlorométhane, 0,2 éq. de TMSOTf, tamis moléculaire) ont été appliquées au donneur **2,** la formation de l'oxazoline **2'** issue du donneur **2** a été observée comme confirmée par la présence du signal du proton anomérique à 7,18 ppm en RMN ¹H caractéristique d'une oxazoline³³ (Figure 11).

Pour compenser cette différence de réactivité, les couplages suivants ont donc été réalisés avec 0,3 éq. de TMSOTf pour permettre l'ouverture de l'oxazoline formée.⁶¹ Dans ce cas, la condensation entre le donneur **11** et l'accepteur **9** permet d'obtenir le trisaccharide **23** avec la configuration β attendue (¹*J*_{CH} = 164,4 Hz) et un rendement de 92% (Schéma 18).

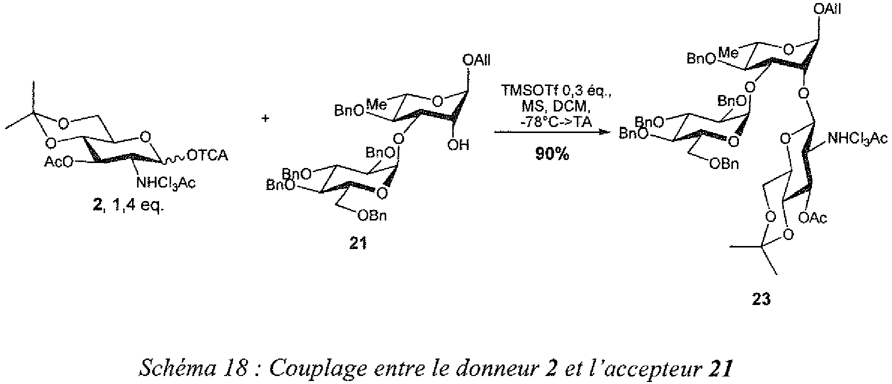

Augmenter les quantités de TMSOTf et de donneur **2** est donc nécessaire pour introduire un résidu glucosamine pré-équipé à des positions stériquement encombrées comme ici, la position 2_{A}. Si on prend en compte les conditions de glycosylation, la combinaison des deux exemples présentés permet de valider le potentiel donneur du composé **2** déjà fonctionnalisé permettant l'allongement, après couplage et simple déprotection sélective, en position 3_{D} sans autres étapes supplémentaires de protection à un stade avancé de la synthèse.

L'avantage de cette deuxième stratégie est que l'isopropylidène a déjà été introduit au niveau de la synthèse du donneur **2.** Ainsi, le glycoside d'allyle **23** est désacétylé dans les conditions de Zemplen ⁶⁰ pour obtenir, en une étape, l'accepteur **38** avec un rendement de 84% (Schéma 19).

### c. Conclusion sur la synthèse du trisaccharide D(E)A

N'impliquant cette fois que deux étapes de purification par chromatographie, les deux voies de synthèse de l'accepteur trisaccharide **38** ont été reproduites à l'échelle de 5 g et comparées (Schéma 19). Au vu de ces résultats, il se révèle donc plus avantageux d'introduire la fonction isopropylidène au stade du trisaccharide plutôt qu'au stade du monosaccharide. En effet, les rendements des étapes de couplage et de protection/déprotection ne changent pas mais la préparation du donneur **2** (56% en 5 étapes) porteur d'une fonction isopropylidène est moins performante que celle du triacétate **1** (72% en 4 étapes) (Partie 1).

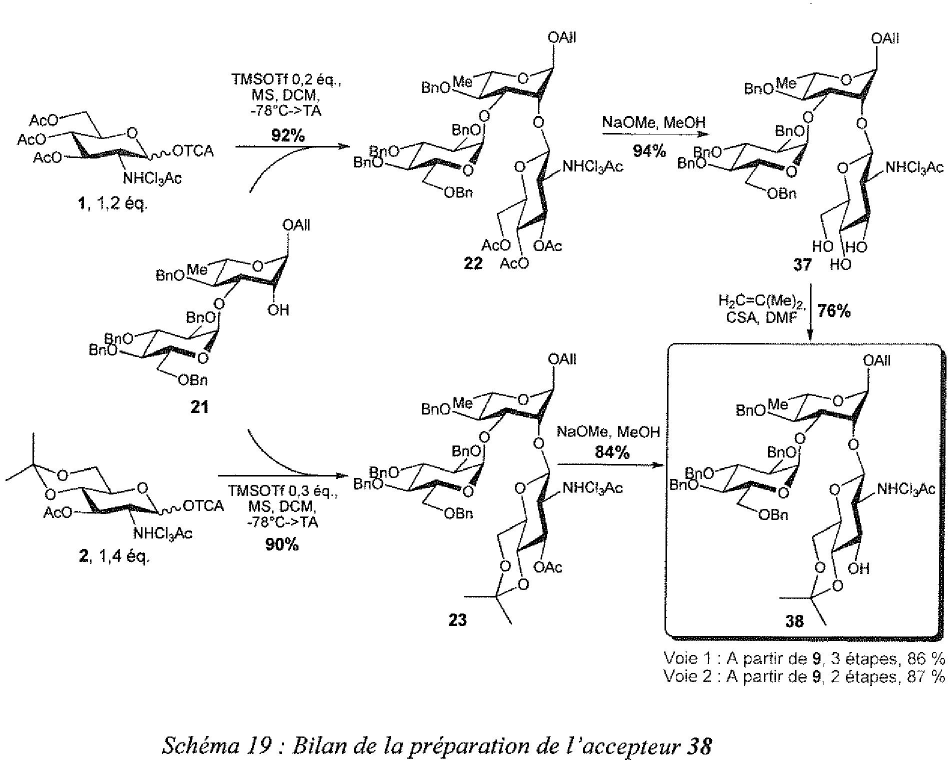

### 6. Déprotection finale du trisaccharide D(E)A 37

Pour le trisaccharide **37,** deux réactions sont nécessaires : une hydrogénolyse acide suivi d'une hydrodéchloration.^{62, 1} Ainsi, **37** est d'abord traité dans des conditions identiques à **21,** puis la réduction de la fonction trichloroacétamide en acétamide est effectuée sous pression d'hydrogène en présence de palladium sur charbon en milieu basique (NEt₃). Le glycoside de propyle attendu ***II*** est obtenu avec un rendement de 69% (Schéma 20).

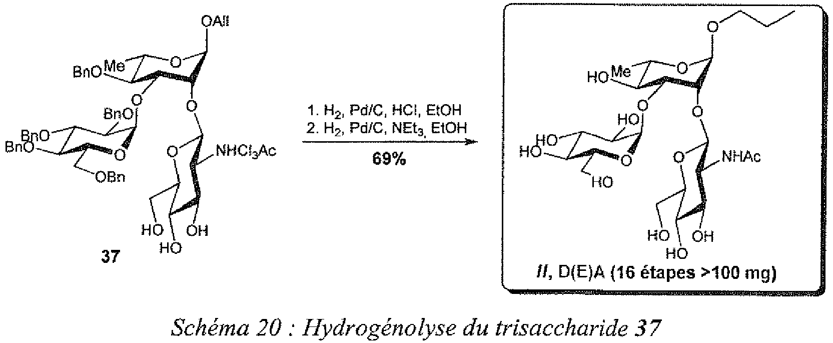

II est à noter qu'une synthèse de l'analogue de ***II*** en série glycoside de méthyle a déjà été publiée dans les années 80 par Kochetkov et *al*.⁶³⁻⁶⁵ Outre le choix de réaliser la synthèse de ***II*** en série glycoside de propyle, possédant l'avantage d'avoir des intermédiaires de synthèse à la fois potentiellement donneur et accepteur, la différence entre les deux stratégies réside dans le choix des groupements protecteurs. Effectivement, Kochetkov et al. ont travaillé en utilisant la chimie des bromure de glycosyle développée par Koenigs-Knorr⁶⁶, et le phtalimide comme groupe protecteur de la fonction amine. En 8 étapes, le trisaccharide est obtenu avec un rendement de 11% , à partir du méthyl 4-*O*-benzyl-α-L-rhamnopyranoside (Schéma 21), alors que la synthèse proposée ci-dessus permet d'obtenir le trisaccharide **D(E)A** avec un rendement de 29% en 6 étapes à partir de l'allyl 4-*O*-benzyl-α-L-rhamnopyranoside. On remarquera toutefois que la différence de rendement majeure réside dans l'étape d'acétylation régiosélective de l'accepteur **A.**

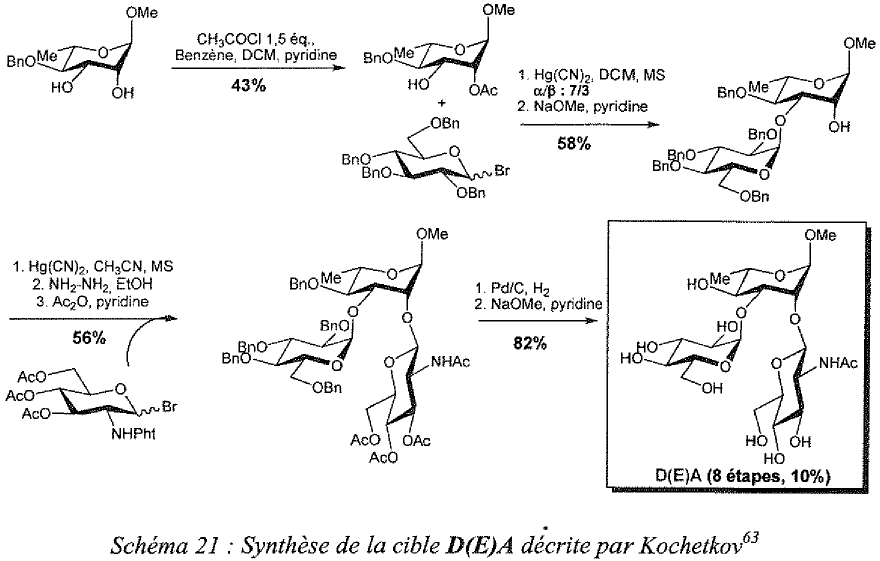

### Procédé 3 :

Un autre des objets de la description se rapporte au procédé de préparation du tétrasaccharide _{Ac}CD(E)A (III), tel que défini dans la liste L1, comprenant les étapes suivantes :
- condensation du monosaccharide donneur **39** avec le trisaccharide accepteur **38** conduisant aux tétrasaccharides **40** et **41,** de préférence la réaction de condensation est réalisée dans un solvant tel que le dichlorométhane, le 1,2-dichloroéthane, l'éther, le toluène, l'acétonitrile ou tout autre solvant équivalent, en présence d'un acide de Lewis tel que le TMSOTf ou l'acide triflique, de préférence, dans le toluène, en présence de TMSOTf et à -78 °C (schéma 22) ;
- clivage du groupement isopropylidène du tétrasaccharide **40** par hydrolyse acide conduisant au tétrasaccharide **41** ;
- déprotection des groupement benzyle et réduction concomitante du trichloroacétamide du tétrasaccharide **41** pour conduire au tétrasaccharide _{Ac}CD(E)A, de préférence sous pression d'hydrogène dans un alcool, par exemple, l'éthanol en présence de dérivé de palladium tel que le palladium sur charbon.

### 7. Tétrasaccharide CD(E)A

Les cibles tétrasaccharides ***III*** et ***IV*** sont issus de la condensation du donneur **39**⁶⁷ (Figure 12), disponible au laboratoire, et du trisaccharide accepteur **38.**

Lors du premier essai de glycosylation, dans les conditions utilisées pour le couplage du donneur **2** avec l'accepteur **21** (dichlorométhane, TMSOTf 0,3 éq., tamis moléculaire), trois produits ont pu être isolés (entrée 1 du Tableau 3 et Schéma 22), dont le produit de couplage **40** souhaité avec la configuration α (¹*J*_{CH}= 169,1 Hz).

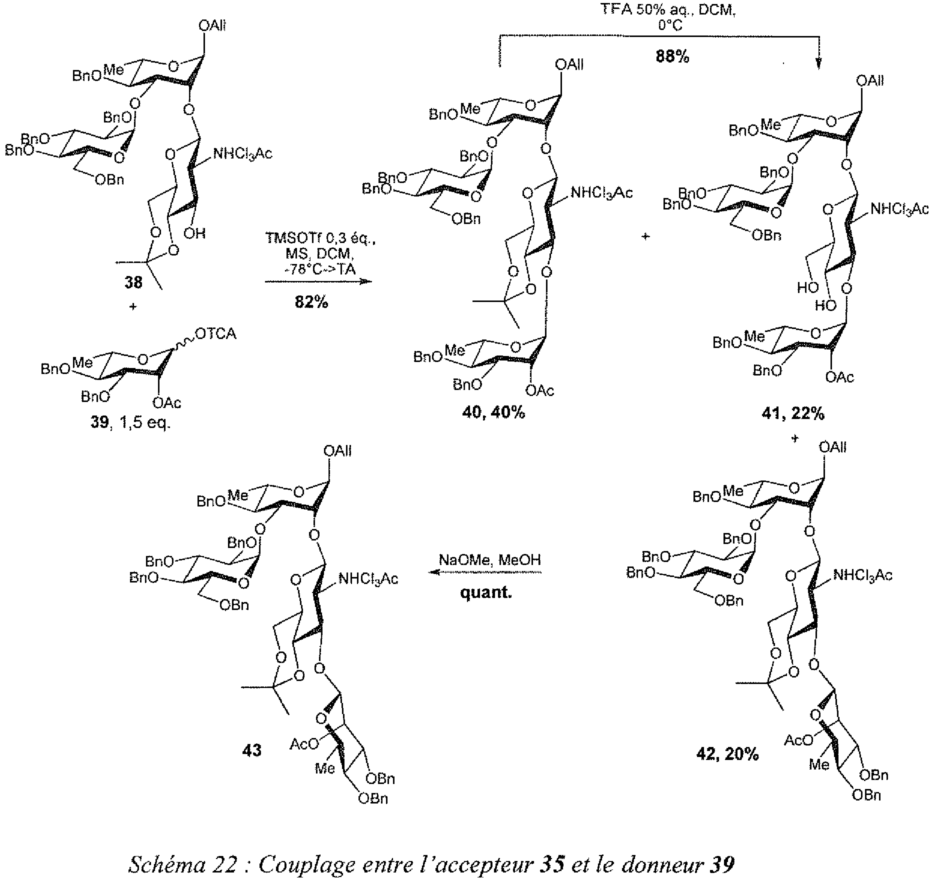

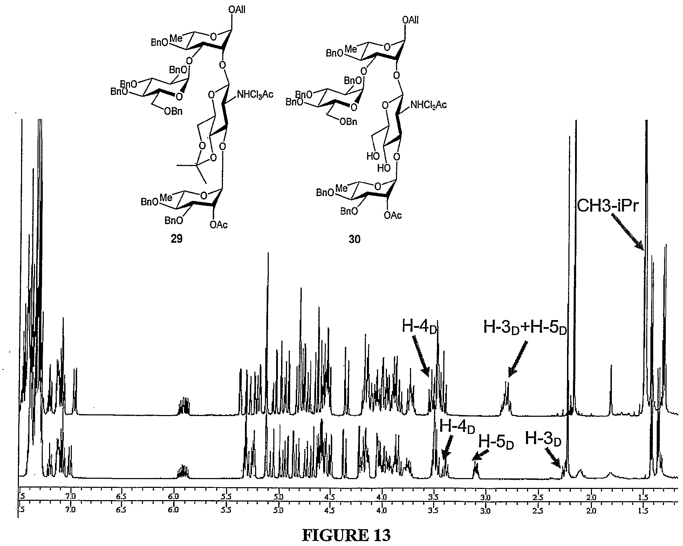

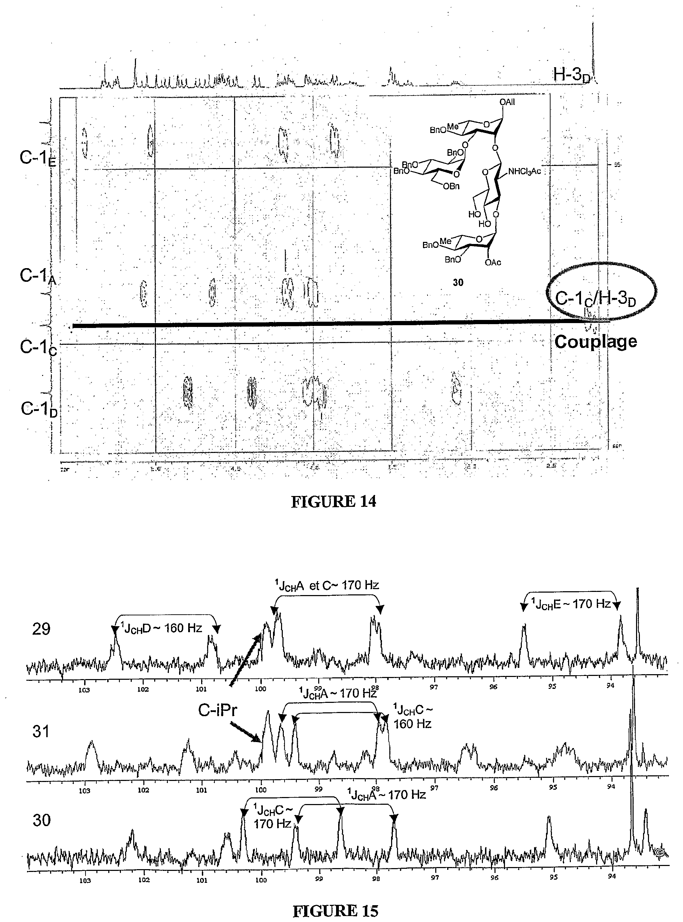

Le glycoside d'allyle **41** possède aussi la configuration α (¹*J*_{CH} = 169,8 Hz) mais a perdu, dans les conditions acides de couplage, la fonction isopropylidène comme l'indique l'absence des pics caractéristiques de l'isopropylidène sur les spectres du proton et du carbone (**41**, Figure 13). Un spectre RMN HMBC a aussi permis de montrer que le couplage avait bien eu lieu en position 3_{d} (Figure 14) préalablement à la désacétalation. Pour confirmer cette réaction secondaire et donc la perte de l'isopropylidène du tétrasaccharide **41** dans les conditions acides de couplage, le produit de couplage **40** est traité par une solution aqueuse de TFA 50% dans le dichlorométhane à 0°C. Après 4h, Le produit isolé est équivalent d'après les analyses en CCM et en RMN au glycoside d'allyle **41.** Ce résultat montre que la glycosylation entre **38** et **39** est antérieure à la perte de l'isopropylidène.

En revanche, l'hypothèse faite sur le produit **42** isolé est la formation d'un tétrasaccharide dont l'unité **C** est liée en position 3_{D} par une liaison β. Pour valider cette hypothèse, et notamment la présence d'un acétyle en position 2_{C}, le produit **42** est désacétylé en présence de NaOMe pour donner, après purification, l'alcool **43** attendu confirmé par une analyse complète RMN (absence d'acétyle et présence d'isopropylidène avec un pic caractéristique à 99,8 ppm en carbone). La superposition des trois spectres C¹³GD de **40, 41** et **42** permet de visualiser les liaisons α de **40** et **41** et la liaison β de **42** (Figure 15).

La présence de **42** et donc la formation de cette liaison β (¹*J*_{CH} = 156,6 Hz) prouve que l'acétyle en position 2_{C} n'a pas joué entièrement son rôle de groupe participant comme l'ont déjà montré des exemples de la littérature.⁶⁸⁻⁷⁵ Des conditions de couplage ont été recherchées afin d'augmenter la stéréosélectivité mais aussi de limiter la dégradation du produit de couplage **40** voulu. Dans certains travaux de la littérature, l'utilisation du toluène permet d'obtenir de meilleurs résultats de couplage que l'utilisation du dichlorométhane.^{69, 76-79} Ainsi, le toluène mais aussi l'éther diéthylique ont été testés (Tableau 3).

**Tableau 3 : Influence du solvant sur le couplage entre l'accepteur 38 et le donneur 39**

| **Entrée** | **Solvants** | **Température** | **40** | **41** | **42** |
|---|---|---|---|---|---|
| **1** | DCM | -78°C→TA | 40% | 22% | 20% |
| **2** | Et₂O | | 58% | 21% | n. o. |
| **3** | Toluène | | n. o. | n. o. | n. o. |
| **4** | Toluène | -78°C | 72% | 5% | n. o. |

| | | | | | |
|---|---|---|---|---|---|
| * n. o. : non observé. | | | | | |

La formation du tétrasaccharide attendu n'a pas été observée dans l'éther diéthylique (entrée 2 du Tableau 3). En revanche, réaliser le couplage dans le toluène permet d'isoler les composés **40** et **41** dans un rapport 75/25 avec un rendement global de 79% (entrée 3). La réaction de couplage est cette fois stéréosélective, aucune trace d'anomère βC n'étant observée (Schéma 23). Pour limiter la dégradation de la fonction isopropylidène, la réaction est poursuivie à -78°C pendant 15 minutes et le milieu réactionnel est neutralisé par ajout de triéthylamine à cette température. Dans ce cas, un mélange de produits **40** et **41** est alors obtenu dans un rapport 95/5 avec un rendement de 77% (entrée 4).

L'isopropylidène porté par **40** est clivé par hydrolyse acide^{80, 81} pour conduire au diol **41** (88%), soit un rendement de 66% sur les 2 étapes (Schéma 23).

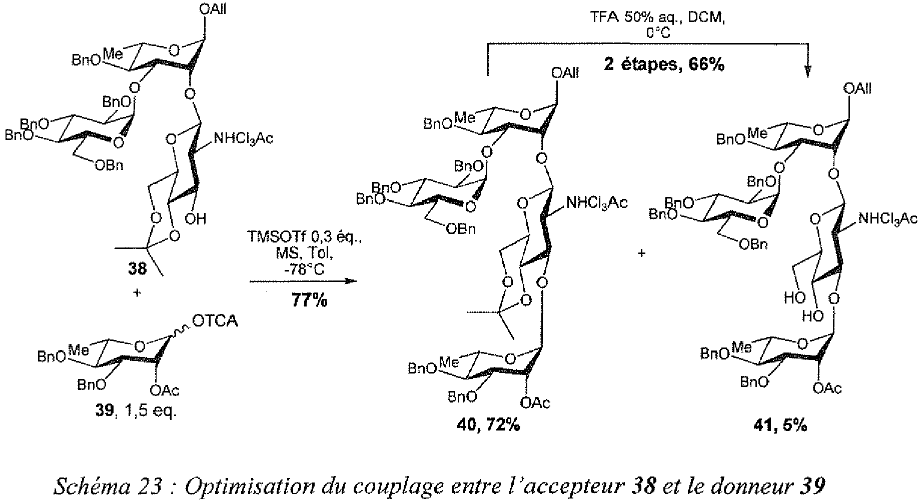

### 8. Déprotection finale des tétrasaccharides en série A réducteur

Dans le cas de l'oligosaccharide **41,** la possibilité de clivage de la fonction acétyle dans des conditions acides ou basiques, exclue l'hydrogénolyse acide et l'hydrodéchloration basique. De plus, comme la cible ***III*** possède une fonction hydroxyle libre en position vicinale de la position 2_{C}, le risque de migration existe. La mise au point des conditions adéquates d'hydrogénation a donc été effectuée sur le pentasaccharide **56** avant d'hydrogéner le tétrasaccharide **41.**

Les différents essais réalisés, dans ces conditions mises au point sur le pentasaccharide **56,** n'ont pas permis d'isoler la cible ***III*** sous une seule forme mais, comme on pouvait s'y attendre, sous forme d'un mélange de deux régioisomères, acétylés en position 2_{C} ou 3_{C} avec un rendement de 76% (Schéma 24).

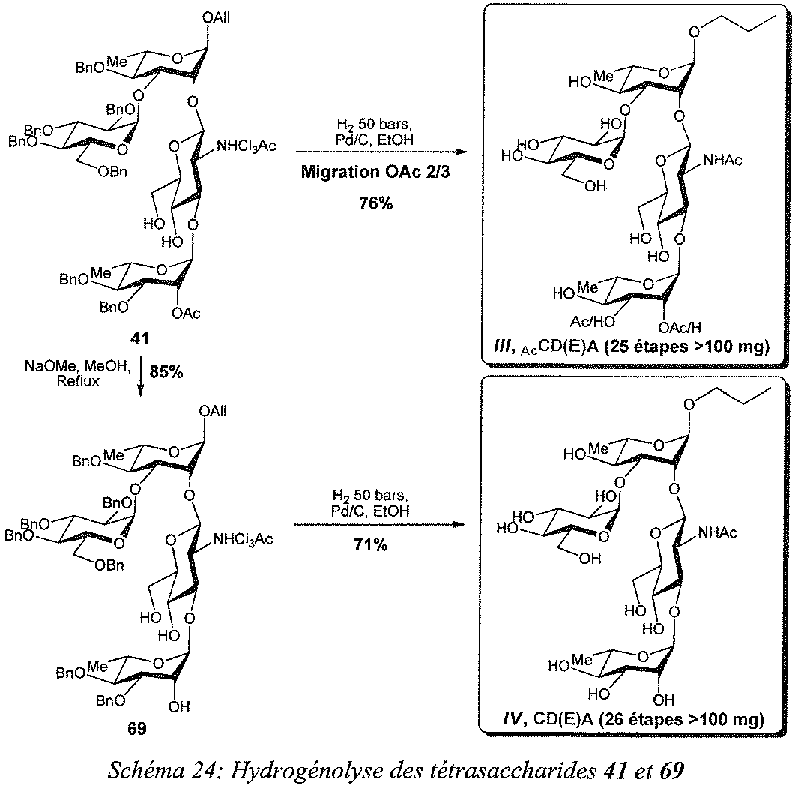

### Procédé 4 :

La description se rapporte également au procédé de préparation du composé tétrasaccharide CD(E)A (IV), tel que défini dans la liste L1, comprenant les étapes suivantes :
- condensation du monosaccharide donneur **39** avec le trisaccharide accepteur **38** conduisant aux tétrasaccharides **40** et **41,** de préférence la réaction de condensation est réalisée dans un solvant tel que le dichlorométhane, le 1,2-dichloroéthane, l'éther, le toluène, l'acétonitrile ou tout autre solvant équivalent, en présence d'un acide de Lewis tel que le TMSOTf ou l'acide triflique, de préférence dans le dichlorométhane, en présence de TMSOTf à 0,2 éq. comme catalyseur et à -78 °C (schéma 22) ;
- clivage du groupement isopropylidène du tétrasaccharide **40** par hydrolyse acide conduisant au tétrasaccharide **41 ;**
- désacétylation du tétrasaccharide **41** conduisant au tétrasaccharide **69,** de préférence en présence de NaOMe à reflux du méthanol (85%) (schéma 24) ;
- déprotection par hydrogénolyse du tétrasaccharide **69,** de préférence sous pression d'hydrogène dans l'alcool, par exemple l'éthanol, en présence d'un dérivé de palladium tel que le palladium sur charbon pour conduire au tétrasaccharide CD(E)A.
L'analogue non-acétylé est obtenu après désacétylation du diol **41** en présence de NaOMe à reflux du méthanol (85%). Pour finir, l'hydrogénation de **69** est effectuée sous 50 bars d'hydrogène. Le glycoside de propyle ***IV*** est isolé avec un rendement de 71% (Schéma 24).

### Procédé 5 :

La description se rapporte au procédé de préparation du pentasaccharide B_{Ac}CD(E)A (V) tel que défini dans la liste L1 comprenant les étapes suivantes :
- condensation du disaccharide donneur **46** avec le trisaccharide accepteur **38** conduisant au pentasaccharide **52,** de préférence la réaction de condensation est réalisée dans un solvant tel que le dichlorométhane, le 1,2-dichloroéthane, l'éther, le toluène, l'acétonitrile ou tout autre solvant équivalent, en présence d'un acide de Lewis tel que le TMSOTf ou l'acide triflique, encore préférentiellement, dans le toluène, en présence de TMSOTf à 0,3 éq. et à -78 °C (schéma 27);
- désacétylation du pentasaccharide **52** conduisant au pentasaccharide **54,** de préférence en conditions basiques avec du K₂CO₃ (schéma 28) ;
- clivage du groupement isopropylidène du pentasaccharide **54** conduisant au pentasaccharide **56,** de préférence en solution aqueuse de TFA 50% dans le dichlorométhane à 0°C (schéma 29) ;
- déprotection du pentasaccharide **56** pour conduire au pentasaccharide B_{Ac}CD(E)A de préférence sous pression d'hydrogène dans l'alcool, par exemple l'éthanol, en présence de dérivé de palladium tel que le palladium sur charbon (schéma 35).

La présente description se rapporte également au procédé de préparation du pentasaccharide B_{Ac}CD(E)A (V) tel que défini dans la liste L1 comprenant les étapes suivantes :
- condensation du disaccharide donneur **47** avec le trisaccharide accepteur **38** conduisant au pentasaccharide **64,** de préférence, la réaction de condensation est réalisée dans un solvant tel que le dichlorométhane, le 1,2-dichloroéthane, l'éther, le toluène, l'acétonitrile ou tout autre solvant équivalent, en présence d'un acide de Lewis tel que le TMSOTf ou l'acide triflique, plus préférentiellement, dans le toluène, en présence de TMSOTf à 0,3 éq. et à -40 °C (schéma 33) ;
- déprotection du groupement lévulinoyle du pentasaccharide **64** conduisant au pentasaccharide **66** de préférence en milieu tamponné (pyridine/AcOH) en présence d'hydrazine monohydraté (schéma 33) ;
- clivage du groupement isopropylidène du pentasaccharide **66** conduisant au pentasaccharide **67** de préférence en solution aqueuse de TFA 50% dans le dichlorométhane à 0°C (schéma 34) ;
- déprotection du pentasaccharide **67** pour conduire au pentasaccharide B_{Ac}CD(E)A de préférence sous pression d'hydrogène dans l'éthanol en présence de palladium sur charbon (schéma 36).

### 9. Pentasaccharide B_{Ac}CD(E)A V

Notre analyse rétrosynthétique ayant pris en compte l'accès à des oligosaccharides d'ordre supérieur, la synthèse proposée pour la cible ***V*** implique le synthon donneur **47.** En l'absence d'élongation en position 2_{B}, le donneur **46,** plus facile d'accès que **47,** peut également être employé avec succès (Schéma 25). Les deux voies ont été étudiées.

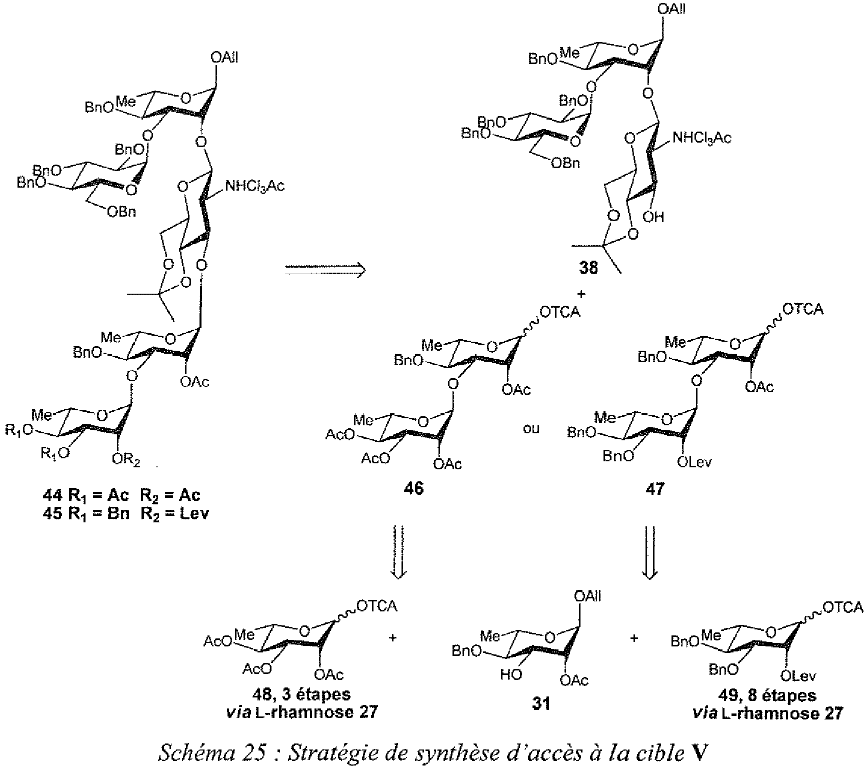

### a. Voie B tritri-O-acétyl

Par analogie avec la synthèse du disaccharide **35,** l'accepteur **31** est condensé sur le donneur **48,** disponible au laboratoire (3 étapes, 76%^{82, 49}). La réaction de glycosylation est réalisée dans le dichlorométhane en présence de TMSOTf (0,3 éq.) et de tamis moléculaire. Le glycoside d'allyle **50** est obtenu avec la configuration α attendue (*J*_{1,2} =1,5 Hz, ¹*J*_{CH} = 173,0 Hz) et un rendement de 95% (Schéma 26). Le disaccharide **50** est déallylé^{39,40} en hémiacétal **51** (91%) et ensuite converti par action de trichloroacétonitrile et DBU,^{22,23} en dormeur **46** avec un rendement de 89%.

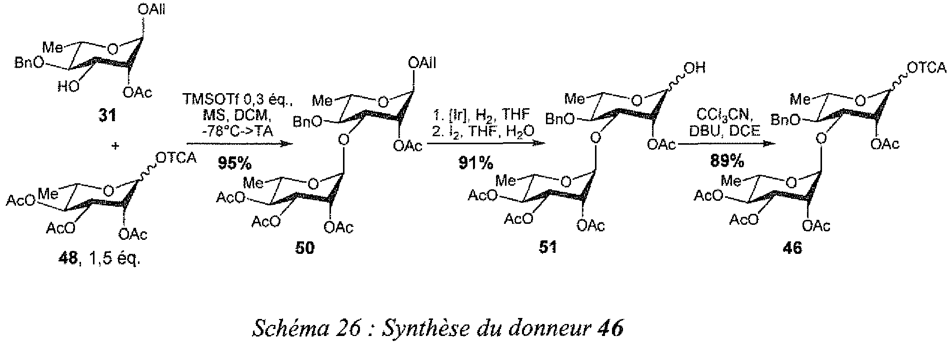

Le choix stratégique initial impose que la désacétylation sélective vis-à-vis d'un acétyle isolé soit réalisable. Le disaccharide **50,** synthon clé de la préparation du donneur **46,** a servi de modèle pour confirmer que tous les acétyles du rhamnose **B** peuvent être clivés sélectivement en présence de celui en position 2_{C}. Le clivage sélectif a donc été testé dans différentes conditions basiques ou acides (Tableau 4). Le meilleur résultat a été observé dans des conditions basiques avec l'utilisation du K₂CO₃. Le triol **50'** et le tétraol **50"** sont obtenus dans un rapport 70/30

**Tableau 4 : Étude de la désacétylation sélective du disaccharide 50**

| **Conditions** | **Température** | **Temps de Réaction** | **Rapport 50'/50"** |
|---|---|---|---|
| NaOMe/MeOH 0,2 éq.⁶⁰ | TA | 2h30 | 60/40 |
| NaOMe/MeOH 0,2 éq.⁶⁰ | 0°C | >2 jours | n. d. |
| NEt₃/MeOH/H₂O^{83,84} | 0°C | 2 jours | 55/45 |
| K₂CO₃/MeOH 1 éq.⁸⁵ | TA | 15 min | 70/30 |
| K₂CO₃/MeOH 1 éq.⁸⁵ | 0°C | 1h30 | 65/35 |
| HBF₄ dans Et₂O/MeOH^{86,87} | TA | >7 jours | n. d. |

| | | | |
|---|---|---|---|
| * n. d. : non déterminé. | | | |

Le donneur **46** étant maintenant disponible et la désacétylation sélective du disaccharide **50** en acétate **50'** étant réalisable, le couplage pour accéder à la cible pentasaccharide a été envisagé avec l'accepteur **38.**

Les conditions mises au point, lors de la synthèse du tétrasaccharide **40** (toluène, TMSOTf 0,3 éq., tamis moléculaire) ont été appliquées à cette réaction de condensation (Schéma 27). Deux composés ont pu être isolés après chromatographie, dans un rapport 78/22 avec un rendement de 90%, le produit de couplage **52,** possédant bien la configuration α souhaité (¹*J*_{CH} = 170,5 Hz), et le diol **53.** Comme dans le cas de **41,** ce dernier, dont la structure a pu être confirmée par une analyse complète en RMN, résulte de la perte de l'isopropylidène « post-condensation » dans les conditions acides de couplage. De la même façon que pour le tétrasaccharide **40,** pour limiter la perte de l'isopropylidène, la température est maintenue à -78°C pendant 15 minutes puis la réaction est stoppée par ajout de triéthylamine. Un mélange de pentasaccharides **52** et **53** est obtenu, cette fois, dans un rapport 92/8 avec un rendement de 87%.

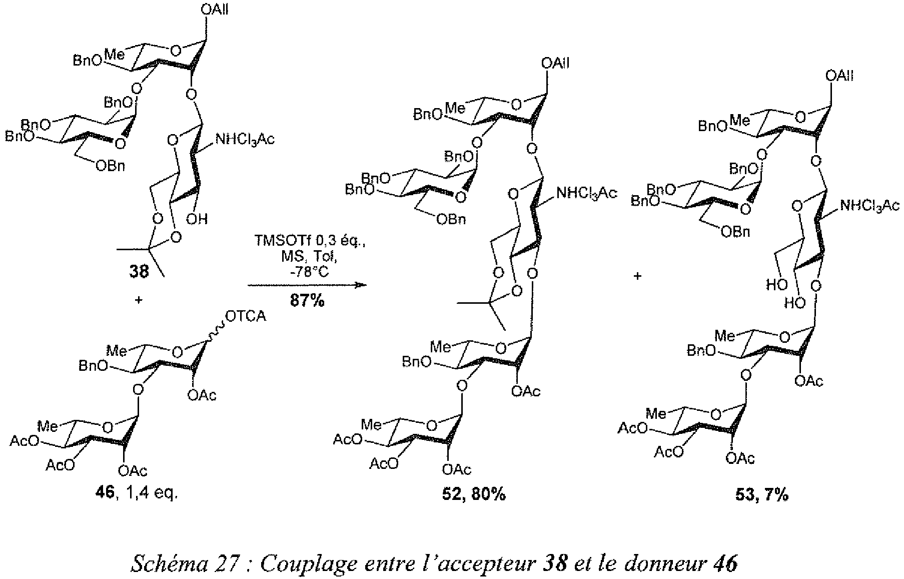

Le pentasaccharide **52** est ensuite désacétylé, dans les conditions mises au point sur le disaccharide **50** (K₂CO₃, MeOH) pour obtenir le triol **54** et le tétraol **55** dans un rapport 92/8 avec un rendement de 92% (Schéma 28). Cette désacétylation sélective est plus efficace sur le pentasaccharide **52** que sur le disaccharide **50,** sûrement, comme cela pouvait être anticipé, du fait de l'encombrement stérique plus important au niveau de la fonction acétyle en position 2_{C}.

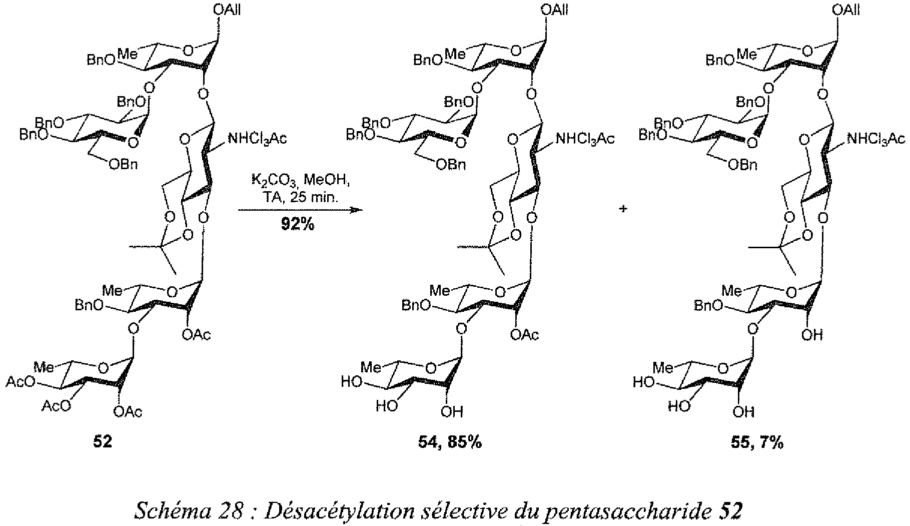

Traités par une solution aqueuse de TFA 50% dans le dichlorométhane à 0°C,^{80,81} les composés **54** et **55** conduisent respectivement aux pentasaccharides **56** et **57** avec un rendement identique de 95% (Schéma 29).

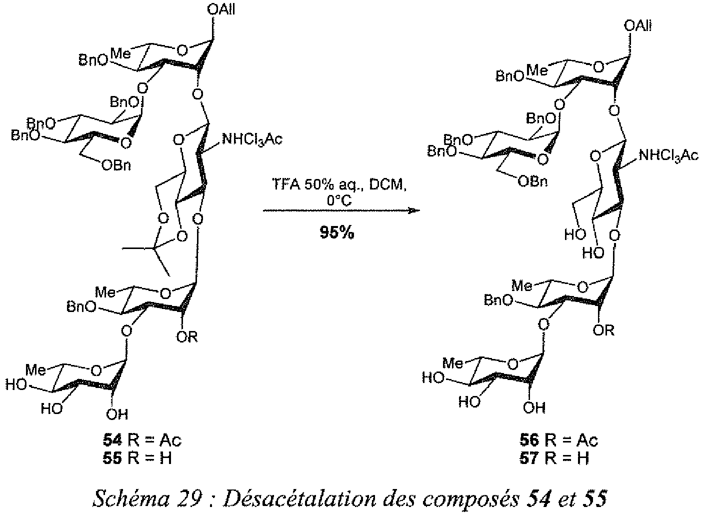

### b. Série B 2-O-lévulinoyl

Pour réaliser la synthèse du building block **47,** la synthèse du précurseur **49** (Schéma 25) a été envisagée. En effet, comme nous l'avons vu dans la partie rétrosynthèse, le choix d'un groupe protecteur temporaire et participant, en position 2_{B}, s'est porté sur le groupe lévulinoyle.^{88, 89}

Plusieurs stratégies sont envisageables mais pour valoriser la synthèse à grande échelle du diol **26,** une protection régiosélective de la position 3 de ce diol par un groupement benzyle semble être la meilleure stratégie. Le diol **26** est ainsi chauffé à reflux du toluène au soxhlet en présence d'oxyde de dibutylétain utilisé en quantité stoechiométrique dans le toluène pour former l'intermédiaire dibutylstannylène.⁹⁰ Après formation de cet intermédiaire, l'attaque régiosélective de l'hydroxyle 3, sur le bromure de benzyle, a été testée en présence d'iodure de *tertio-butyl* ammonium,⁹¹ ou de fluorure de césium.⁹²⁻⁹⁴ Ce dernier permet d'obtenir l'accepteur **58**⁹⁵ avec un rendement de 88% supérieur au 70% obtenu avec l'iodure de *tertio*-butyl ammonium.

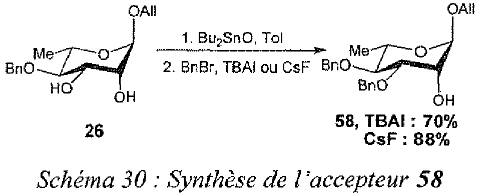

L'accepteur **58** est, converti en ester de lévulinoyle **59** avec un rendement de 89% par action d'acide lévulinique en présence de DCC et de DMAP dans le dichlorométhane⁸⁹ (Schéma 31) puis déallylé^{96, 39, 40} en deux étapes (Partie Rétrosynthèse) permettant d'isoler l'hémiacétal **60** avec un rendement de 94%. Ce dernier est mis en réaction dans le 1,2-dichloroéthane en présence de DBU et de trichloroacétonitrile^{22, 23} pour conduire au trichloroacétimidate **49** avec un rendement de 92%.

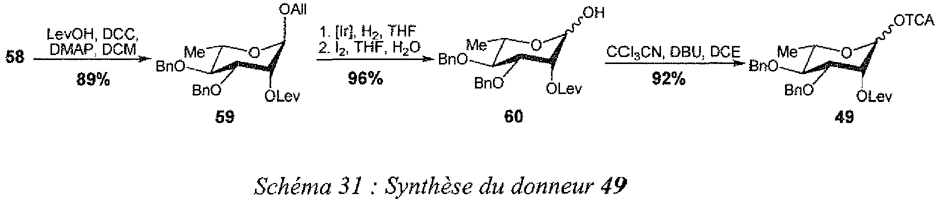

Le couplage entre le donneur **49** et l'accepteur **31** a été étudié en utilisant différentes conditions de solvant.^{69, 76-79} Si la condensation est réalisée dans le dichlorométhane en présence de TMSOTf (0,3 éq.) et de tamis moléculaire, le disaccharide **61** est obtenu avec la configuration α attendue (J_{1,2} = 1,5 Hz, ¹*J*_{CH} = 169,5 Hz) et un rendement de 66% confirmant le rôle de groupe participant du lévulinoyle (Schéma 32). Dans le toluène en présence de TMSOTf (0,3 éq.) et de tamis moléculaire, le rendement en disaccharide **61** est accru (77%) et atteint 90% quand le couplage est réalisé à grande échelle (7 g d'accepteur **5**). Puis, comme pour le disaccharide **50,** le disaccharide **61** est converti en donneur **47** en deux étapes (Schéma 32), une étape de déallylation^{39, 40} (93%) suivie d'une étape d'activation ^{22, 23} (84%).

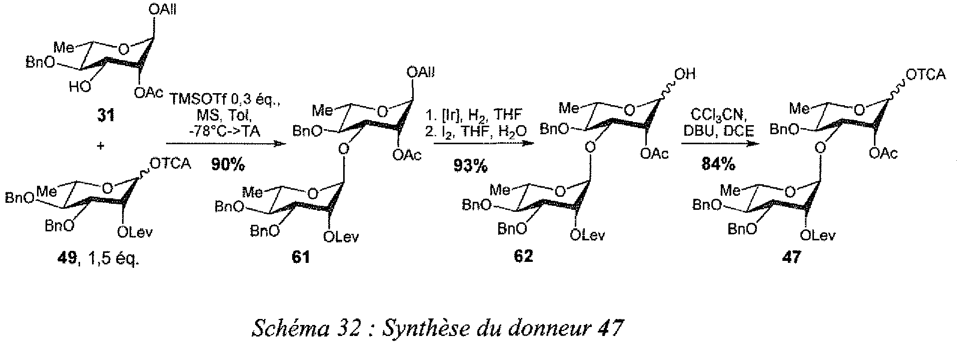

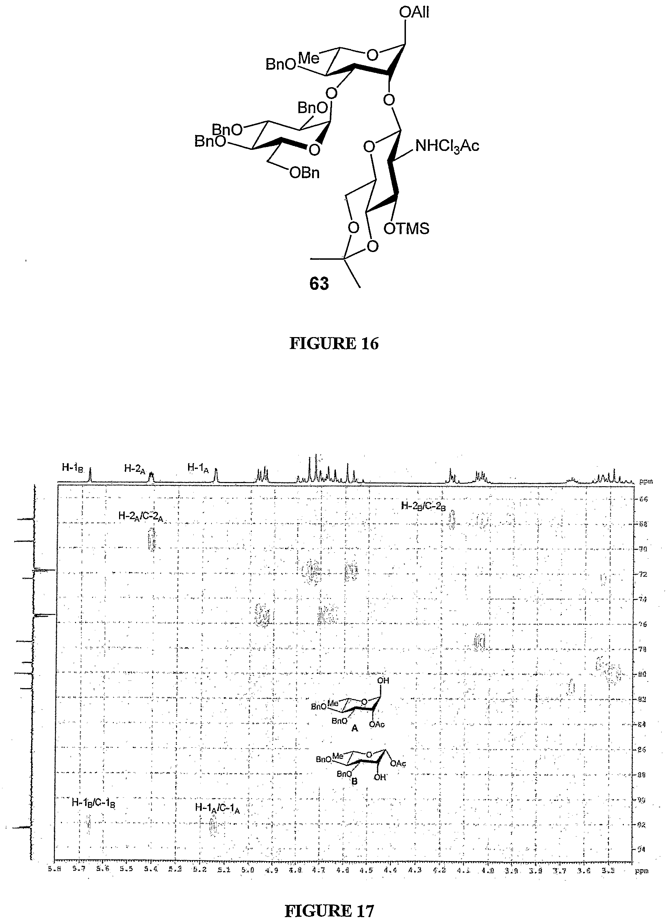

Différentes conditions de solvant^{69, 76-79} (dichlorométane, toluène) ont été testées lors du couplage entre le donneur disaccharide **47** et l'accepteur trisaccharide **38** (Schéma 33). Les deux premières entrées du Tableau 5 montrent, encore une fois, l'efficacité du toluène par rapport au dichlorométhane mais aussi la stabilité de la fonction isopropylidène à -78°C. En revanche lorsque le couplage a été réalisé à plus grande échelle (de l'ordre de 2 g d'accepteur **38**), l'accepteur silylé **63** et la dégradation du donneur **47,** probablement liées à la faible réactivité du donneur et/ou de l'accepteur, sont observées (Figure 16).

Deux hypothèses ont été émises :
1. Le milieu réactionnel n'est pas assez acide pour permettre l'attaque l'accepteur silylé **63** sur le donneur ;
2. Il n'y a pas assez de donneur dans le milieu réactionnel,

Aucune amélioration n'est observée lorsque le couplage est réalisé en conditions plus acides (0,4 éq de TMSOTf) et, par ailleurs, l'addition de donneur supplémentaire conduit à sa dégradation immédiate. Ces deux hypothèses ne permettant pas d'éluder le problème de réactivité du donneur **47,** un autre facteur, l'influence de la température a donc été étudié. Effectivement, les entrées 3 et 4 du Tableau 5 indiquent qu'à -40°C ou à -20°C, ni accepteur de départ **38** ni accepteur silylé **63** ne sont observés mais, à nouveau, comme pour les couplages précédents, la perte d'isopropylidène est bien réelle. En revanche, si la température est trop augmentée, le rendement chute. L'analyse par RMN, HMBC notamment, a permis de confirmer la structure du pentasaccharide **64** et du pentasaccharide **65,** ayant perdu l'isopropylidène. De nouveau, la glycosylation précède la perte de cette fonction.

**Tableau 5 : Étude du couplage entre le donneur 47 et l'accepteur 38**

| **Entrée** | **Solvants** | **Température** | **Temps de réaction** | **Accepteur 38 restant** | **64** | **65** |
|---|---|---|---|---|---|---|
| **1** | DCM | -78°C | 1h. | 30% | 42% | n.o. |
| **2** | Toluène | -78°C | 1h. | 16% | 63% | n.o. |
| **3** | Toluène | -40°C | 30 min. | n.o. | 66% | 22% |
| **4** | Toluène | -20°C | 30 min. | n.o. | 66% | 13% |
| **5** | Toluène | -40°C | 10 min. | n.o. | 75% | n.o. |

| | | | | | | |
|---|---|---|---|---|---|---|
| * n. o. : non observé. | | | | | | |

En conclusion, si la température est trop faible, le donneur s'hydrolyse plus vite qu'il ne réagit avec l'accepteur **38** ou l'accepteur silylé **63** et, à l'inverse, si la température est trop élevée, le couplage a bien lieu mais avec un rendement qui décroit avec l'augmentation de la température. Le contrôle de la température est donc déterminant comme illustré dans le Schéma 33. De plus, pour limiter la dégradation de l'isopropylidène lors du couplage, un autre facteur a été étudié : le temps de réaction avant neutralisation du milieu réactionnel. Le entrées 3 et 5 du Tableau 5 permettent de confirmer que ce facteur influence la perte de l'isopropylidène. La condensation entre le donneur **47** et l'accepteur **38** est donc réalisée dans le toluène en présence de TMSOTf (0,3 éq.) et de tamis moléculaire à -40°C. Après 10 min (entrée 5 du Tableau 5), la réaction est stoppée et le pentasaccharide **64,** doté de la configuration α attendue (¹*J*_{CH} = 167.6 Hz), est isolé avec un rendement de 75%.

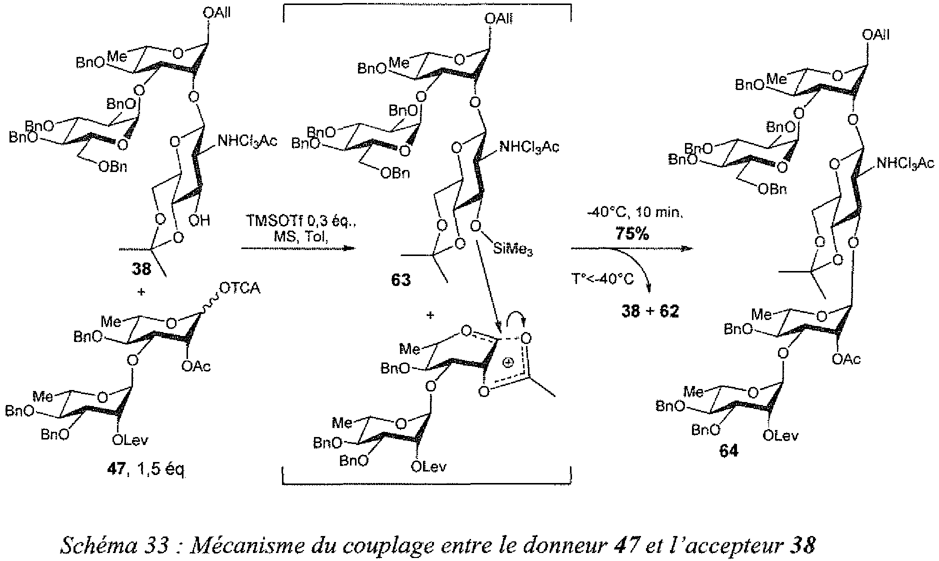

La déprotection du groupement lévulinoyle du pentasaccharide **64** est réalisée en milieu tamponné (pyridine/AcOH) en présence d'hydrazine monohydraté.^{89, 97-101} L'alcool **66** est obtenu avec un rendement de 94%. Traité par une solution aqueuse de TFA 50% dans le dichlorométhane à 0°C,^{80, 81} ce dernier conduit au pentasaccharide **67** avec un rendement de 84% (Schéma 34).

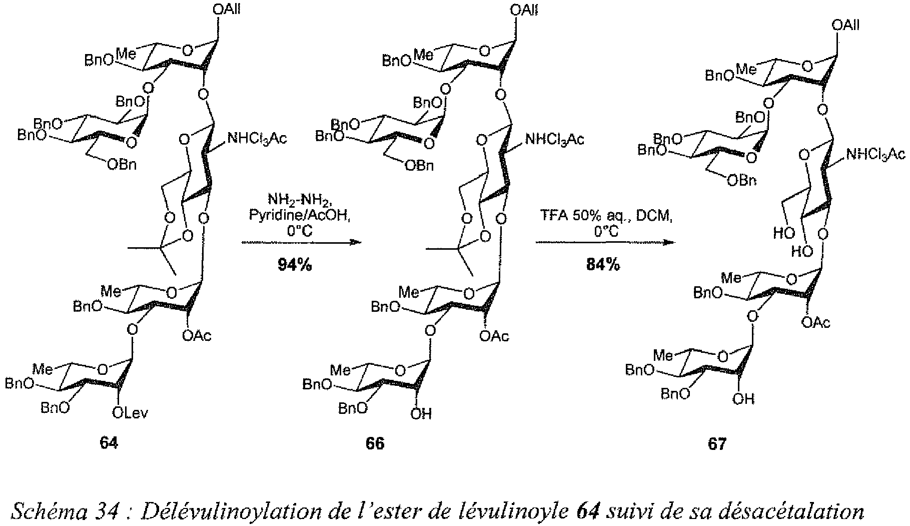

### 10. Déprotection finale des pentasaccharides en série A réducteur

La possibilité de clivage de la fonction acétyle de **56** dans des conditions acides ou basiques, excluant l'hydrogénolyse acide et l'hydrodéchloration basique, une mise au point des conditions adéquates d'hydrogénation a été effectuée. L'utilisation de la haute pression (50 bars) a été préférée à une étude sur le choix du catalyseur (Schéma 35).

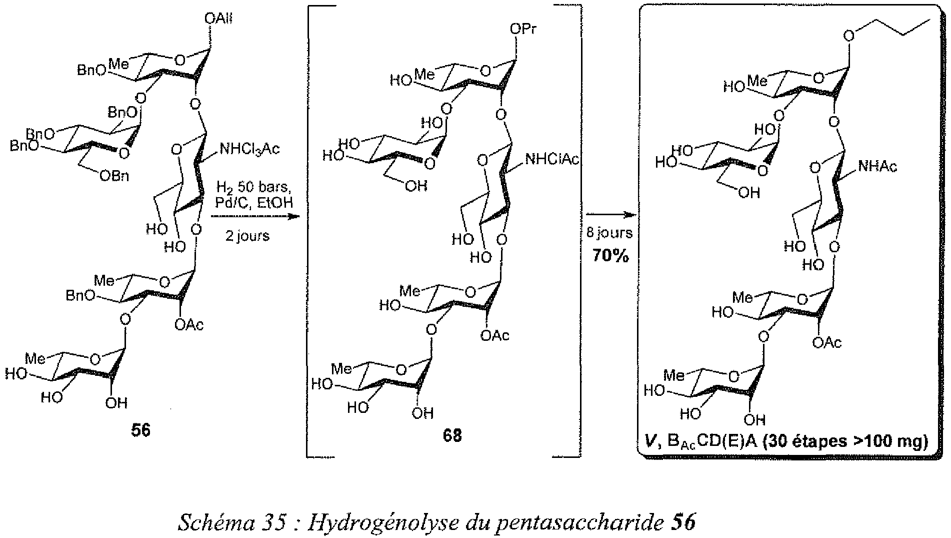

Après deux jours, l'intermédiaire chloroacétamide **68** a été observé lors du suivi par spectrométrie de masse (M = 940.3429, trouvé *m*/*z* = 940.3425) et par RMN (singulet à 4,1 ppm en proton et un signal à 42,5 ppm en carbone) (Schéma 35). Après huit jours supplémentaires, la conversion en acétamide est totale. Le glycoside de propyle ***V*** est isolé avec un rendement de 70%. L'autre possibilité est d'utiliser le pentasaccharide **67** issu du couplage avec le synthon disaccharide **47.** Le glycoside de propyle attendu ***V*** est obtenu, dans les mêmes conditions, avec un rendement de 75% (Schéma 36).

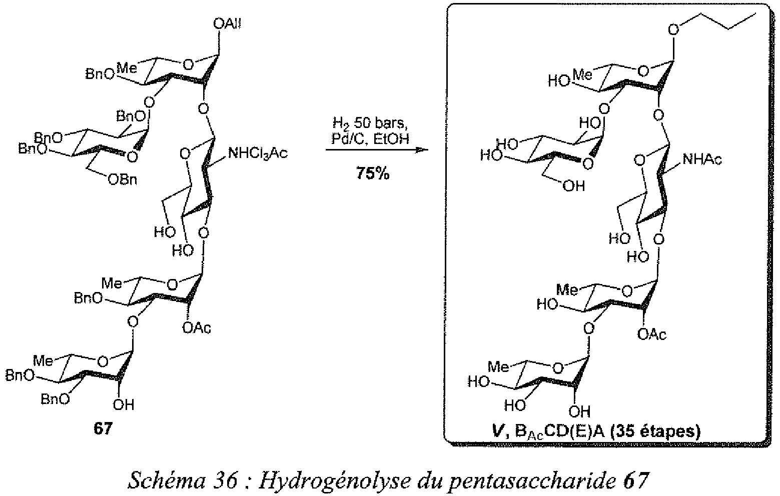

### Procédé 6 :

La description se rapporte encore au procédé de préparation du composé VI pentasaccharide BCD(E)A (VI) tel que défini dans la liste L1 comprenant les étapes suivantes :
- condensation du disaccharide donneur **46** avec le trisaccharide accepteur **38** conduisant au pentasaccharide **52** de préférence la réaction de condensation est réalisée dans un solvant tel que le dichlorométhane, le 1,2-dichloroéthane, l'éther, le toluène, l'acétonitrile ou tout autre solvant équivalent, en présence d'un acide de Lewis tel que le TMSOTf ou l'acide triflique, préférentiellement dans le toluène, en présence de TMSOTf à 0,3 éq. et à -78 °C (schéma 27) ;
- désacétylation du pentasaccharide **52** conduisant au pentasaccharide **55** de préférence en conditions basiques, notamment avec du K₂CO₃ (schéma 28) ;
- désacétylation du pentasaccharide **55** conduisant au pentasaccharide **57** de préférence en solution aqueuse de TFA 50% dans le dichlorométhane à 0°C (schéma 29) ;
- déprotection du pentasaccharide **57** pour conduire au pentasaccharide BCD(E)A de préférence sous pression d'hydrogène dans l'alcool, par exemple l'éthanol, en présence de dérivé de palladium, tel que le palladium sur charbon (schéma 37).
En effet, par analogie au procédé précédent, l'hydrogénation sous 50 bars de pression, de l'alcool **57** conduit au glycoside de propyle ***VI**,* isolé avec un rendement de 71% (Schéma 37).

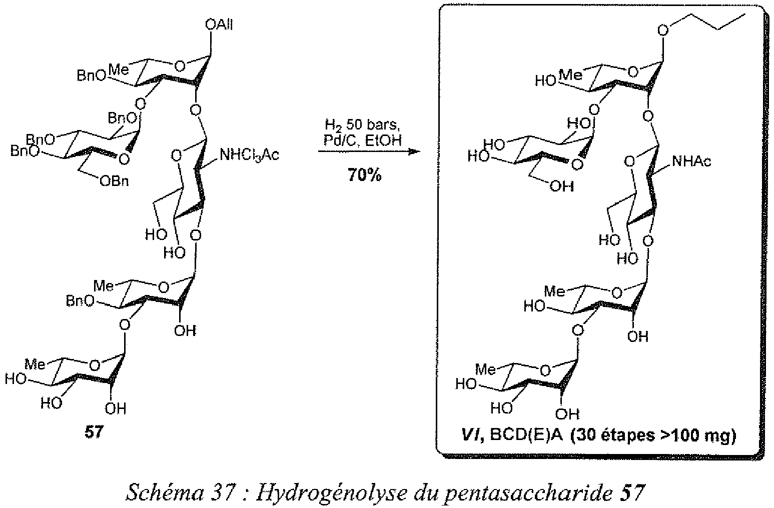

### 11. Conclusion

Dans cette partie, la synthèse de six oligosaccharides en série **A** réductrice, mono-acétylé ou non correspondant aux sérotypes 3a ou X de *S. flexneri,* incluant un di-, un tri-, deux tétra-, et deux pentasaccharides ainsi qu'une étude comparative d'accès à la cible pentasaccharide *V* ont été réalisées. D'après le Schéma 38, l'accès à cette cible, issu du couplage entre le trisaccharide accepteur **38** et le disaccharide donneur **46,** est un peu plus efficace que celle utilisant le donneur **47.** Mais l'avantage de la seconde voie de synthèse est l'allongement possible du synthon disaccharide **61** ainsi que du pentasaccharide **64,** en position réductrice ou non-réductrice, pour accéder à des cibles oligosaccharidiques de plus grande taille.

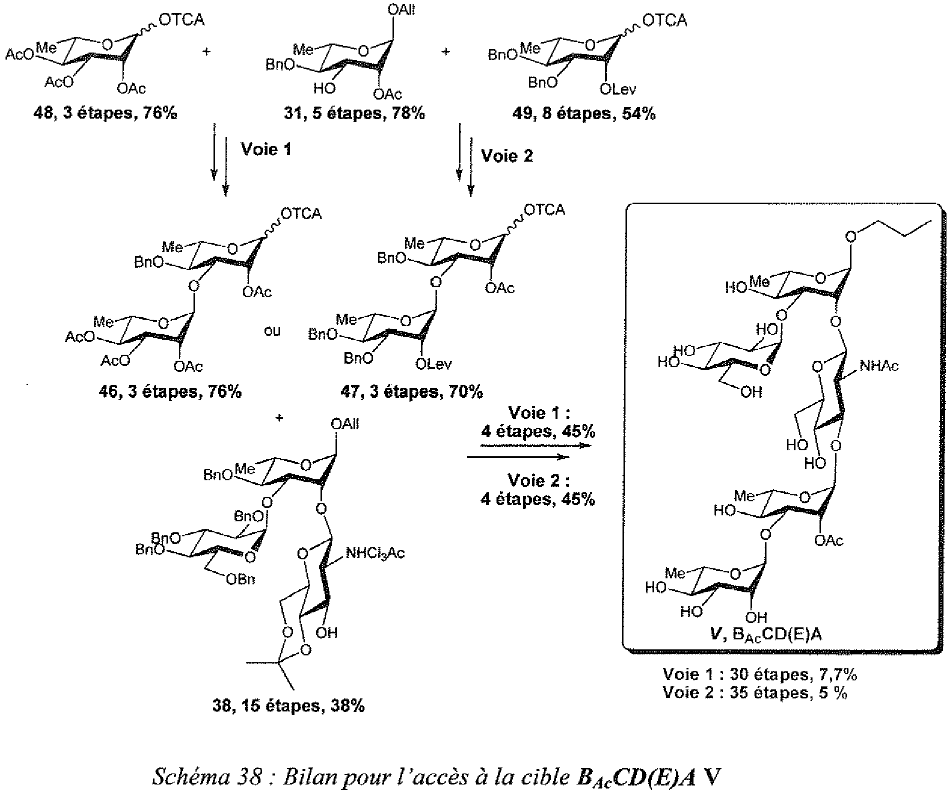

**Mise en oeuvre expérimentales des procédés 1 à 6**

**Procédé 1 :**

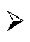 **Allyl α-L-rhamnopyranoside⁴² 28 :**

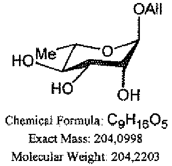

Du chlorure d'acétyle (19,6 mL, 270,0 mmol, 2,5 éq.) est ajouté par portions à de l'alcool allylique (250 mL) à 0°C durant 15 min, puis le L(+)-rhamnose monohydrate commercial **27** (20,0 g, 110,0 mmol) est ajouté au milieu réactionnel. Le milieu réactionnel est ensuite chauffé à 70°C pendant 2,5 h puis à 40°C durant une nuit. Le suivi de la réaction par CCM (DCM/MeOH, 9/1) montre la disparition de **27** (Rf= 0,1) et la présence d'un produit majoritaire moins polaire (Rf= 0,45). Le milieu réactionnel est refroidi à 0°C, neutralisé par addition de NaHCO₃ puis filtré sur célite et enfin concentré à l'évaporateur rotatif. Les volatiles sont coévaporés avec du Tol (2 x 100 mL) pour conduire à une huile jaune. L'huile jaune est purifiée par chromatographie sur gel de silice (DCM/MeOH, 98/2→8/2) pour obtenir un mélange du glycoside d'allyle **28_{α}** (19,8 g, 88%) et son anomère **28_{β}** (2,4 g, 11%) sous forme d'une huile jaune.
**28_{α}** : Rf = 0.45 (DCM/MeOH, 9/1).
RMN ¹H (CDCl₃), *δ* 5.92 (m, 1H, CH=), 5.30 (m, 1H, *J*ₜᵣₐₙₛ = 15.7 Hz, =CH₂), 5.20 (m, 1H, *J*_{cis} = 10.4 Hz, =CH₂), 4.81 (bs, 1H, H-1), 4.5 (bs, 1H, OH), 4.19-4.10 (m, 2H, H_{All}, OH), 3.98 (m, 1H, H_{All}), 3.96 (bs, 1H, H-2), 3.79 (m, 1H, H-3), 3.68 (dq, 1H, *J*_{4,5} = 9.4 Hz, H-5), 3.50 (m, 2H, H-4, OH), 1.32 (d, 3H, *J*_{5,6} = 6.2 Hz, H-6).
RMN ¹³C (CDCl₃), *δ* 134.0 (CH=), 118.0 (=CH₂), 99.3 (C-1, ¹*J*_{CH} = 164.2 Hz), 73.1 (C-4), 72.4 (C-3), 71.5 (C-2), 68.5 (C-5), 68.4 (C_{All}), 17.9 (C-6).

### ➢ Allyl 2,3-O-isopropylidène-α-L-rhamnopyranoside⁴² 29 :

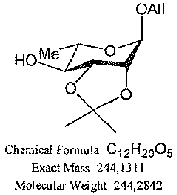

Le glycoside d'allyle **28** (3,5 g, 17,1 mmol) est dissous dans un mélange d'acétone (16 mL) et de 2,2-diméthoxypropane (6,9 mL, 51,3 mmol, 3 éq.) agité sous atmosphère inerte à TA. De l'APTS (162 mg, 0,84 mmol, 0,05 éq.) est additionné et l'agitation est poursuivie pendant 2 h Le suivi par CCM (DCM/MeOH, 9/1) montre la disparition de **28** (Rf= 0,45) et la formation d'un composé moins polaire majoritaire (Rf = 0,85). Le milieu réactionnel est neutralisé par addition de triéthylamine (1 mL) puis concentré à l'évaporateur rotatif. L'huile jaune est reprise dans du DCM (100 mL) et lavée avec de l'H₂O (3 x 50 mL). Les phases organiques sont regroupées, séchées sur filtre séparateur de phases et concentrées à l'évaporateur rotatif pour conduire à une huile jaune. L'huile jaune est purifiée par chromatographie sur gel de silice (Chex/EtOAc, 9/1→6/4) pour obtenir l'alcool **29** sous forme d'une huile jaune (3,5 g, 84%).
Rf = 0.85 (DCM/MeOH, 9/1).
RMN ¹H (CDCl₃), *δ* 5.89 (m, 1H, CH=), 5.32 (m, 1H, *J*ₜᵣₐₙₛ = 15.6 Hz, =CH₂), 5.22 (m, 1H, *J*_{cis} = 10.6 Hz, =CH₂), 5.01 (bs, 1H, H-1), 4.19 (m, 1H, H_{All}), 4.16 (d, 1H, *J*_{1,2} = 0.6 Hz, *J*_{2,3} = 5.8 Hz, H-2), 4.09 (pt, 1H, *J*_{3,4} = 5.8 Hz, H-3), 4.01 (m, 1H, H_{All}), 3.68 (dq, 1H, *J*_{4,5} = 9.3 Hz, H-5), 3.39 (m, 1H, H-4), 2.9 (s, 1H, OH), 1.53 (s, 3H, H_{iPr}), 1.35 (s, 3H, H_{iPr}), 1.32 (d, 3H, *J*_{5,6} = 6.3 Hz, H-6).
RMN ¹³C (CDCl₃), *δ* 134.0 (CH=), 118.2 (=CH₂), 110.0 (C_{iPr}), 96.6 (C-1), 78.5 (C-3), 76.2 (C-2), 74.7 (C-4), 68.3 (C_{All}), 66.2 (C-5), 28.3 (C_{iPr}), 26.5 (C_{iPr}), 17.8 (C-6).

### ➢ Allyl 4-O-benzyl-2,3-O-isopropylidène-α-L-rhamoopyranoside⁴² 30 :

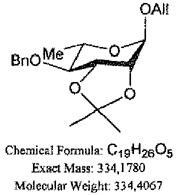

Dans un ballon tricol, l'alcool **29** (3,5 g, 14,4 mmol) est dissous dans du DMF (80 mL) sous argon et refroidi à -5°C puis du NaH 60% dans l'huile (1,7 g, 41,8 mmol, 2,9 éq.) est additionné par petites fractions. L'agitation est poursuivie à TA pendant 1 h Après retour à -5°C, le bromure de benzyle (1,9 mL, 15,8 mmol, 1,1 éq.) est additionné goutte à goutte. Après 4 heures d'agitation à TA, le suivi par CCM (Chex/EtOAc, 8/2) montre la formation d'un composé moins polaire majoritaire (Rf= 0,7). Après avoir placé le ballon dans un bain de glace, du MeOH (1,2 mL) est additionné lentement et l'agitation est maintenue pendant 2 h à TA. De l'H₂O est ajoutée (20 mL) puis le milieu réactionnel est transféré dans une ampoule à décanter et la phase aqueuse est extraite à l'acétate d'éthyle (3 x 100 mL). Les phases organiques sont regroupées et lavées successivement à l'H₂O (2 x 20 mL), à HCl 5% (2 x 20 mL), à NaClₛₐₜ (2 x 20 mL), puis séchées sur filtre séparateur de phases et enfin concentrées à l'évaporateur rotatif. L'huile jaune obtenue est purifiée par chromatographie sur gel de silice (Chex/EtOAc, 99/1 →8/2) pour obtenir le composé **30** sous forme d'une huile jaune (3,9 g, 82%).
Rf= 0,7 (Chex/EtOAc, 8/2).
RMN ¹H (CDCl₃), *δ* 7.41-7.31 (m, 5H, CH_{Ph}), 5.92 (m, 1H, CH=), 5.33 (m, 1H, *J*ₜᵣₐₙₛ = 17.2 Hz, =CH₂), 5.23 (m, 1H, *J*_{cis} = 10.4 Hz, =CH₂), 5.05 (bs, 1H, H-1), 4.94 (d, 1H, *J* = 11.6 Hz, H_{Bn}), 4.67 (d, 1H, H_{Bn}), 4.32 (pt, 1H, *J*_{3,4} = 6.8 Hz, H-3), 4.20 (m, 2H, H-2, H_{All}), 4.01 (m, 1H, H_{All}), 3.76 (dq, *J*_{4,5} = 9.8 Hz, 1H, H-5), 3.25 (pt, 1H, H-4), 1.54 (s, 3H, H_{iPr}), 1.41 (s, 3H, H_{iPr}), 1.32 (d, 3H, *J*_{5,6} = 6.3 Hz, H-6).
RMN ¹³C (CDCl₃), *δ* 138.8 (C_{Ph}), 134.1 (CH=), 128.8-128.0 (CH_{Ph}), 118.1 (=CH₂), 109.5 (C_{iPr}), 96.5 (C-1, ¹*J*_{CH} = 167.8 Hz), 81.5 (C-4), 79.1 (C-3), 76.5 (C-2), 73.3 (C_{Bn}), 68.2 (C_{All}), 64.8 (C-5), 28.4 (C_{iPr}), 26.7 (C_{iPr}), 18.2 (C-6).

### ➢ Allyl 4-O-benzyl-α-L-rhamnopyranoside⁴² 26 :

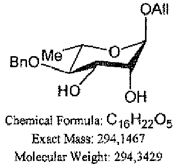

**Voie 1** : Le composé **30** (3,9 g, 11,8 mmol) est dissous dans une solution d'acide acétique à 80% aq. (60 mL) et porté à 60°C. Après 3 h de réaction, le suivi par CCM (DCM/EtOAc, 1/1) montre la formation d'un composé plus polaire majoritaire (Rf= 0,6). La réaction est refroidie à TA puis concentrée et coévaporée avec un mélange Tol/Chex (1/1, 3 x 100 mL). Une recristallisation est réalisée dans un mélange iPr₂O/EP (8/2, 10 mL). Après une nuit en chambre froide, les cristaux blancs sont filtrés et lavés par un mélange iPr₂O/EP (1/1, 10 mL) pour obtenir le diol **26** sous forme de cristaux blancs (2,5 g, 72% soit 44% sur 4 étapes).
**Voie 2 :** Du chlorure d'acétyle (19,6 mL, 270,0 mmol, 2,5 éq.) est ajouté par portions à de l'alcool allylique (250 mL) à 0°C durant 15 min, puis le L(+)-rhamnose monohydrate commercial **27** (20,0 g, 110,0 mmol) est ajouté au milieu réactionnel. Le milieu réactionnel est ensuite chauffé à 70°C pendant 2,5 h puis à 40°C durant une nuit. Le suivi de la réaction par CCM (DCM/MeOH, 9/1) montre la disparition de **27** (Rf= 0,1) et la présence d'un produit majoritaire moins polaire (Rf= 0,45). Le milieu réactionnel est refroidi à 0°C, neutralisé par addition de NaHCO₃ puis filtré sur célite et enfin concentré à l'évaporateur rotatif. Les volatiles sont coévaporés avec du Tol (2 x 100 mL) pour conduire à une huile jaune (27,6 g).

Le brut réactionnel obtenu (27,6 g, 110,0 mmol) est dissous dans un mélange d'acétone (121 mL) et de 2,2-diméthoxypropane (40,5 mL, 330,0 mmol, 3 éq.) agité sous atmosphère inerte à TA. De l'APTS (208 mg, 1,1 mmol, 0,01 éq.) est additionné et l'agitation est poursuivie pendant 2 h Le suivi par CCM (DCM/MeOH, 9/1) montre la disparition de **28** (Rf= 0,45) et la formation d'un composé moins polaire majoritaire (Rf= 0,85). Le milieu réactionnel est neutralisé par addition de triéthylamine (2 mL) puis concentré à l'évaporateur rotatif. L'huile jaune est reprise dans du DCM (200 mL) et lavée avec de l'H₂O (3 x 100 mL). Les phases organiques sont regroupées, séchées sur filtre séparateur de phases et concentrées à l'évaporateur rotatif pour conduire à une huile jaune (25,1 g).

Dans un ballon tricol, le brut réactionnel obtenu (25,1 g, 110,0 mmol) est dissous dans du DMF (320 mL) sous argon et refroidi à -5°C puis du NaH 60% dans l'huile (13,2 g, 330,0 mmol, 3 éq.) est additionné par petites fractions. L'agitation est poursuivie à TA pendant 1 h Après retour à -5°C, le bromure de benzyle (25,5 mL, 210,0 mmol, 2 éq.) est additionné goutte à goutte. Après 4 heures d'agitation à TA, le suivi par CCM (Chex/EtOAc, 8/2) montre la formation d'un composé moins polaire majoritaire (Rf= 0,7). Après avoir placé le ballon dans un bain de glace, du MeOH (9 mL) est additionné lentement et l'agitation est maintenue pendant 2 h à TA. De l'H₂O est ajoutée (50 mL) puis le milieu réactionnel est transféré dans une ampoule à décanter et la phase aqueuse est extraite à l'acétate d'éthyle (3 x 250 mL). Les phases organiques sont regroupées et lavées successivement à l'H₂O (2 x 50 mL), à HCl 5% (2 x 50 mL), à NaClₛₐₜ (2 x 50 mL), puis séchées sur filtre séparateur de phases et enfin concentrées à l'évaporateur rotatif pour conduire à une huile jaune (35,0 g).

Le brut réactionnel obtenu (35,0 g, 110,0 mmol) est dissous dans une solution d'acide acétique à 80% aq. (280 mL) et porté à 60°C. Après 3 h de réaction, le suivi par CCM (DCM/EtOAc, 1/1) montre la formation d'un composé plus polaire majoritaire (Rf= 0,6). La réaction est refroidie à TA puis concentrée et coévaporée avec un mélange Tol/Chex (1/1, 3 x 250 mL). Une recristallisation est réalisée dans un mélange iPr₂O/EP (8/2, 100 mL). Après une nuit en chambre froide, les cristaux blancs sont filtrés et lavés par un mélange iPr₂O/EP (1/1). Les eaux mères sont concentrées et purifiées par chromatographie sur gel de silice (DCM/EtOAc, 9/1→7/3). L'huile jaune obtenue est recristallisée dans un mélange iPr₂O/EP pour obtenir le diol **26** sous forme de cristaux blancs (25,8 g, 78%).
Rf = 0,6 (DCM/EtOAc, 8/2).
RMN ¹H (CDCl₃), *δ* 7.40-7.33 (m, 5H, CH_{Ph}), 5.88 (m, 1H, CH=), 5,31 (m, 1H, *J*ₜᵣₐₙₛ = 17.2 Hz, =CH₂), 5.20 (m, 1H, *J*_{cis}= 10.4 Hz, =CH₂), 4.83 (s, 1H, H-1), 4.81 (d, 1H, *J=* 11.3 Hz, H_{Bn}), 4.72 (d, 1H, H_{Bn}), 4.19 (m, 1H, H_{All}), 4.00-3.96 (m, 2H, H-3, H_{All}), 3.95 (bs, 1H, H-2), 3.78 (dq, *J*_{4,5} = 9.4 Hz, 1H, H-5), 3.37 (pt, 1H, *J*_{3,4} = 9.0 Hz, H-4), 3.05 (bs, 2H, OH), 1.31 (d, 3H, *J*_{5,6} = 6.3 Hz, H-6).
RMN ¹³C (CDCl₃), *δ* 138.6 (C_{Ph}), 134.1 (CH=), 129.0-128.4 (CH_{Ph}), 117.7 (=CH₂), 98.8 (C-1, ¹*J*_{CH} = 168.2 Hz), 82.1 (C-4), 75.5 (C_{Bn}), 71.8, 71.6 (C-2*, C-3*), 68.3 (C_{All}), 67.6 (C-5), 18.4 (C-6).

### ➢ Trichloracétimidate de 2,3,4,6-tétra-O-benzyl-α/β-D-glucopyranose³⁵ 33 :

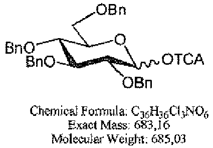

Le 2,3,4,6-tétra-*O*-benzyl-β-D-glucopyranose commercial **32** (25,0 g , 46,3 mmol) sont dissous dans du DCM (250 mL). Sont alors ajoutés du K₂CO₃ (25,0 g, 180,0 mmol, 3,9 éq.) préalablement séché une nuit à 300°C, puis du trichloroacétonitrile (25,0 mL, 250,0 mmol, 5,4 éq.). Le milieu réactionnel est laissé sous forte agitation pendant 3 h à TA. La réaction est suivie par CCM (Chex/EtOAc + 5% NEt₃, 8/2). Bien qu'elle ne soit pas terminée, la réaction est stoppée afin d'éviter l'isomérisation du trichloroacétimidate β (Rf= 0,6) en α (Rf = 0,7). Le milieu réactionnel est filtré, concentré sous vide. Le trichloroacétimidate est obtenu par recristallisation dans un mélange iPr₂O/EP (6/4, 100 mL). La première filtration, après 1 h dans un bain de glace, permet de récupérer le produit de départ sous forme de flocons (1,1 g, 2 mmol), et la seconde, après une semaine en chambre froide, le produit d'arrivée sous forme de billes. Il s'agit majoritairement de l'anomère **33_{β}** (22,4 g, 71%) et minoritairement de l'anomère **33_{α}** (1,7 g, 5%).
**33_{β}**□ : Rf = 0,6 (Chex/EtOAc, 8/2).
RMN ¹H (CDCl₃), *δ*: β : 8.72 (s, 1H, NH), 7.36-7.29 (m, 20H, CH_{Ph}), 5.83 (d, 1H, *J*₁₋₂ = 7.3 Hz, H-1), 4.98-4.55 (m, 8H, H_{Bn}), 3.80-3.74 (m, 5H, H-2, H-3, H-4, H-6a, H-6b), 3.67 (m, 1H, H-5).
RMN ¹³C (CDCl₃), *δ* : β : 161.6 (C=NH), 138.8-138.3 (C_{Ph}), 128.7-127.9 (CH_{Ph}), 98.7 (C-1, ¹*J*_{CH} = 167.2 Hz), 84.9 (C-4), 81.3 (C-3), 77.7 (C-2), 76.3 (C-5), 76.0, 75.4, 75.3, 73.8 (4C, C_{Bn}), 68.6 (C-6).

### ➢ Allyl 2-O-acétyl-4-O-benzyl-α-L-rhamnopyranoside⁴¹ 31 :

### ➢ Allyl (2,3,4,6-tétra-O-benzyl-α-D-glucopyranosyl)-(1→3)-2-O-acétyl-4-O-benzyl-α-L-rhamnopyranoside³¹ 35 :

### ➢ Allyl (2,3,4,6-tétra-O-benzyl-α-D-glucopyranosyl)-(1→3)-4-O-benzyl-α-L-*rhamnopyranoside⁴⁵ 21 :

### ➢ Allyl (2,3,4,6-tétra-O-benzyl-β-D-glucopyranosyl)-(1→3)-4-O-benzyl-α-L-hanmopyranoside⁴⁵ 36 :

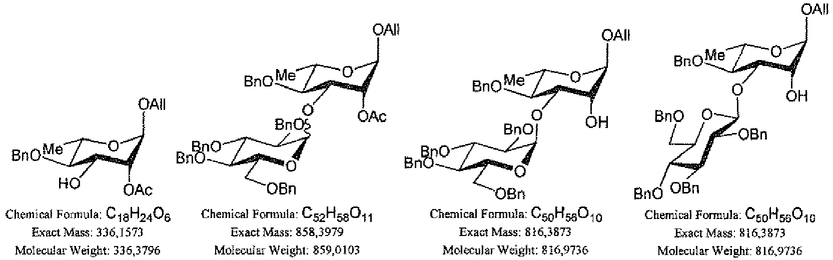

L'orthoacétate de triméthyle (6,6 mL, 51,7 mmol, 1,9 éq.) et l'APTS (72 mg, 400 µmol, 0,014 éq.) sont ajoutés au diol **26** (8,0 g, 27,2 mmol) en solution dans l'acétonitrile (15 mL). Le milieu réactionnel est agité à TA et la réaction est suivie par CCM (Tol/EtOAc, 9/1, DCM/EtOAc, 1/1). Après 1 h, la transformation du diol **26** (Rf= 0,5 dans DCM/EtOAc, 1/1) en orthoester moins polaire (Rf= 0,6 dans Tol/EtOAc, 9/1) est totale et le ballon est plongé dans un bain de glace. L'agitation est maintenue pendant 10 min, puis de l'acide acétique 80% aqueux (15 mL) sont ajoutés. Après 1 h sous forte agitation à TA, le suivi par CCM (Tol/EtOAc, 8/2) indique la transformation complète de l'orthoester en un composé plus polaire (Rf= 0,25). Le milieu réactionnel est repris à l'eau froide (50 mL) et la phase aqueuse est extraite rapidement au DCM (3 x 100 mL). Les phases organiques sont regroupées et lavées avec une solution de NaClₛₐₜ (3 x 20 mL), filtrées sur filtre séparateur de phases et concentrées à l'évaporateur rotatif. L'huile incolore (9,1 g, 98%) est engagée directement dans l'étape suivante après contrôle par RMN ¹H. Ce dernier indique la présence de l'accepteur **31** voulu et du régioisomère acétylé en position 3 dans un rapport 97/3.
Rf = 0,25 (Tol/EtOAc, 8/2).
RMN ¹H (CDCl₃), *δ* 7.38-7.32 (m, 5H, CH_{Ph}), 5.88 (m, 1H, CH=), 5.31 (m, 1H, *J*ₜᵣₐₙₛ = 17.2 Hz, =CH₂), 5.21 (m, *J*_{cis} = 10.4 Hz, =CH₂), 5.14 (dd, 1H, *J*_{1,2} = 1.7 Hz, *J*_{2,3} = 3,6 Hz, H-2), 4.86 (d, 1H, *J=* 11.1 Hz, H_{Bn}), 4.80 (d, 1H, H-1), 4.73 (d, 1H, H_{Bn}), 4.17 (m, 2H, H-3, H_{All}), 3.98 (m, 1H, H_{All}), 3.78 (dq, 1H, *J*_{4,5} = 9,4 Hz, H-5), 3.37 (pt, 1H, *J*_{3,4} = 9.5 Hz, H-4), 2.19 (s, 3H, H_{Ac}), 1.37 (d, 3H, *J*_{5,6} = 6.3 Hz, H-6).
RMN ¹³C (CDCl₃), *δ* 168.9 (C_{Ac}), 136.3 (C_{Ph}), 131.6 (CH=), 126.6-126.0 (CH_{Ph}), 115.6 (=CH₂), 94.6 (C-1), 79.9 (C-4), 73.4 (C_{Bn}), 70.9 (C-2), 68.3 (C-3), 66.2 (C_{All}), 65.6 (C-5), 19.2 (C_{Ac}), 16.1 (C-6).

Du TMSOTf (98,0 µL, 550 µmol, 0,02 éq.) est additionné à une solution de l'huile brute obtenue (9,1 g, 27,2 mmol) dans du Tol (112 mL), agitée sous argon à -78°C. Après 30 min sous agitation à cette température, une solution du donneur **33** (22,4 g, 32,6 mmol, 1,2 éq.) dans un mélange Tol/DCM (2/1, 24 mL) est ajoutée goutte à goutte. Le bain éthanol-carboglace est retiré et l'agitation est poursuivie pendant 1 h, temps au bout duquel le suivi par CCM (Tol/EtOAc, 9/1, Chex/EtOAc, 73/27 et Tol/EtOAc, 8/2) indique la disparition de l'accepteur **31** (Rf = 0,15 et 0,2, respectivement) et l'apparition d'un composé moins polaire (Rf= 0,4 et 0,55, respectivement). La réaction est stoppée par l'addition de triéthylamine (0,2 mL) puis le milieu réactionnel est concentré à l'évaporateur rotatif. L'huile jaune obtenue est purifiée grossièrement par flash-chromatographie (Tol/EtOAc, 97/3→94/6). Le mélange d'anomères **35_{α}** et **35_{β}** sous forme d'une huile jaune (21,0 g, 85/15, αE/βE, 90%) est engagée dans l'étape suivante sans autre purification.
Rf= 0,55 (Chex/EtOAc, 73/27).
RMN ¹H (CDCl₃), *δ*: α : 7.46-7.15 (m, 25H, CH_{Ph}), 5.88 (m, 1H, CH=), 5.43 (dd, 1H, H-2_{A}), 5.31 (m, 1H, *J*ₜᵣₐₙₛ = 17.3 Hz, =CH₂), 5.24-5.21 (m, 2H, H-1_{E}, =CH₂), 5.06 (d, 1H, *J* = 11.0 Hz, H_{Bn}), 4.99 (d, 1H, *J =* 10.2 Hz, H_{Bn}), 4.92 (d, 1H, H_{Bn}), 4.89 (d, 1H, H_{Bn}), 4.82 (d, 1H, *J*_{1,2} = 1.6 Hz, H-1_{A}), 4.77 (d, 1H, *J* = 11.9 Hz, H_{Bn}), 4.72 (d, 1H, H_{Bn}), 4.65 (d, 1H, H_{Bn}), 4.63 (d, 1H, H_{Bn}), 4.52 (d, 1H, *J* = 11.0 Hz, H_{Bn}), 4.40 (d, 1H, *J* = 12.1 Hz, H_{Bn}), 4.37 (dd, 1H, *J*_{2,3} = 3.3 Hz, *J*_{3,4} = 9.6 Hz, H-3_{A}), 4.19 (m_{overlaped,} 1H, H_{All}), 4.14 (pt_{overlaped}, 1H, *J*_{3,4} = 9.5 Hz, H-3_{E}), 4.10 (m_{overlaped,} 1H, H-5_{E}), 4.00 (m, 1H, H_{All}), 3.84 (dq, 1H, *J*_{4,5} = 9.4 Hz, H-5_{A}), 3.85 (pt, 1H, *J*_{4,5} = 9.4 Hz, H-4_{E}), 3.68-3.61 (m, 3H, H-2_{E}, H-6a_{E}, H-4_{A}), 3.57 (dd, 1H, *J*_{5,6b} = 1.8 Hz, *J*_{6a,6b} = 10.9 Hz, H-6b_{E}), 1.99 (s, 3H, H_{Ac}), 1.43 (d, 3H, *J*_{5,6} = 6.3 Hz, H-6_{A}).
RMN ¹³C (CDCl₃), *δ* : β : 170.5 (C_{Ac}), 138.9-137.7 (C_{Ph}), 133.6 (CH=), 128.6-127.4 (CHPh), 117.5 (=CH₂), 96.7 (C-1_{A}, ¹*J*_{CH} = 170.4 Hz), 92.7 (C-1_{E}, ¹*J*_{CH} = 167.0 Hz), 82.2 (C-3_{E}), 79.9 (C-4_{A}), 79.4 (C-2_{E}), 77.9 (C-4_{E}), 76.2, 75.5, 75.0, 73.3, 73.0 (5C, C_{Bn}), 72.2 (C-3_{A}), 70.2 (C-5_{E}), 68.3 (C-6_{E}), 68.1 (C_{All}), 68.0 (C-5_{A}), 67.9 (C-2_{A}), 20.9 (C_{Ac}), 18.0 (C-6_{A}).

Après addition de NaOMe 0,5 M (53,8 mL, 26,9 mmol, 1,1 éq.) à l'huile obtenue (21,0 g, 24,5 mmol) en solution dans un mélange DCM/MeOH (11/8, 250 mL), le milieu réactionnel est porté à reflux. Après 2 h, la réaction n'évoluant plus (Tol/EtOAc, 9/1, Chex/EtOAc, 73/27), elle est arrêtée Après retour à TA, il est neutralisé par addition de résine échangeuse d'ion DOWEX (H⁺), puis filtré sur fritté. La résine est lavée avec un mélange DCM/MeOH et les solvants sont évaporés. L'huile jaune obtenue est purifiée par chromatographie sur gel de silice (Tol/EtOAc 92/8→9/1) pour conduire par ordre d'élution au disaccharide **21** désiré (Rf = 0,3 et 0,45, 13,6 g, 61% sur trois étapes) puis au stéréoisomère β **36** (Rf= 0,15 et 0,35, 3,4 g, 15% sur trois étapes). Les deux disaccharides **21** et **36** sont isolés sous forme d'huiles incolores. Les produits de condensation sont obtenus avec un rendement global de 76% sur trois étapes, dans un rapport α/β de 8/2.
**21** : Rf = 0,45 (Chex/EtOAc, 73/27)
RMN ¹H (CDCl₃), *δ* 7.25-7.02 (m, 25H, CH_{Ph}), 5.78 (m, 1H, CH=), 5.18 (m, 1H, *J*ₜᵣₐₙₛ = 17.3 Hz, =CH₂), 5.10 (m, 1H, *J*_{cis}= 10.4 Hz, =CH₂), 4.85 (d, 1H, *J=* 11.0 Hz, H_{Bn}), 4.81 (m, 3H, H-1_{E}, H-1_{A}, H_{Bn}), 4.73 (d, 2H, *J* = 11.5 Hz, H_{Bn}), 4.60 (m, 3H, H_{Bn}), 4.39 (d, 2H, *J* = 12.0 Hz, H_{Bn}), 4.30 (d, 1H, H_{Bn}), 4.07 (m, 1H, H_{All}), 3.99 (pt, 1H, H-3_{E}), 3.95-3.92 (m, 2H, H-3_{A}, H_{All}), 3.90-3.83 (m, 2H, *J*_{4,5} = 9.1 Hz, H-2_{A}, H-5_{E}), 3.68 (dq, 1H, *J*_{4,5} = 9.3 Hz, H-5_{A}), 3.63 (pt, 1H, *J*_{3,4} = 9.7 Hz, H-4_{E}), 3.51 (dd, 1H, *J*_{1,2} = 3.6 Hz, *J*_{2,3} = 9.6 Hz, H-2_{E}), 3.41 (pt, 1H, *J*_{3,4} = 9.4 Hz, H-4_{A}), 3.37 (dd, 1H, *J*_{5,6b} = 2.7 Hz, *J*_{6a,6b} = 10.9 Hz, H-6b_{E}), 3.30 (bs, 1H, OH), 3.24 (dd, 1H, *J*_{5,6a} = 1.8 Hz, H-6a_{E}), 1.27 (d, 3H, *J*_{5,6} = 6.2 Hz, H-6_{A}).
RMN ¹³C (CDCl₃), *δ* : 139.0-137.9 (C_{Ph}), 134.2 (CH=), 129.4-127.9 (CH_{Ph}), 117.7 (=CH₂), 98.6 (C-1_{E}, ¹*J*_{CH} = 166.3 Hz), 94.4 (C-1_{A}, ¹*J*_{CH} = 166.9 Hz), 82.8 (C-3_{E}), 79.7 (C-4_{A}), 79.3 (C-2_{E}), 78.1 (C-4_{E}), 77.7 (C-3_{A}), 77.0, 76.0, 75.3, 74.7, 73.8 (5C, C_{Bn}), 71.1 (C-5_{E}), 68.3 (C-6_{E}), 68.2 (C_{All}), 67.8 (C-2_{A}), 67.6 (C-5_{A}), 18.3 (C-6_{A}).
**36** : Rf = 0,35 (Chex/EtOAc, 73/27)
RMN ¹H (CDCl₃), *δ* 57.43-7.28 (m, 25H, CH_{Ph}), 5.95 (m, 1H, CH=), 5.36 (m, 1H, *J*ₜᵣₐₙₛ = 17.2 Hz, =CH₂), 5.26 (m, 1H, *J*_{cis}= 10.4 Hz, =CH₂), 5.04 (d, 1H, *J* = 11.1 Hz, H_{Bn}), 4.98 (m, 2H, H_{Bn}), 4.93 (bs, 1H, H-1_{A}), 4.90 (m, 2H, H_{Bn}), 4.81 (d, 1H, *J*_{1,2} = 7.5 Hz, H-1_{E}), 4.81 (m, 1H, *J* = 11.2 Hz, H_{Bn}), 4.65 (m, 4H, H_{Bn}), 4.26 (m, 1H, H_{All}), 4.25-4.21 (m, 2H, H-3_{A}, H-2_{A}), 4.05 (m, 1H, H_{All}), 3.89 (m, 1H, H-5_{A}), 3.80-3.63 (m, 6H, H-6a_{E}, H-3_{E}, H-4_{E}, H-6b_{E}, H-4_{A}, H-2_{E}), 3.60 (m, 1H, H-5_{E}), 3.33 (bs, 1H, OH), 1.40 (d, 3H, *J*_{5,6} = 6.3 Hz, H-6_{A}).
RMN ¹³C (CDCl₃), *δ* : 138.9-138.3 (C_{Ph}), 134.4 (CH=), 129.5-127.9 (CH_{Ph}), 117.8 (=CH₂), 103.0 (C-1_{E}, ¹*J*_{CH} = 159.5 Hz), 99.1 (C-1_{A}, ¹*J*_{CH} = 167.6 Hz), 85.3 (C-3_{E}), 82.6 (C-2_{E}), 81.4 (C-3_{A}), 80.5 (C-4_{A}), 78.2 (C-4_{E}), 76.0, 75.5, 75.4, 75.1 (4C, C_{Bn}), 75.0 (C-5_{E}), 74.0 (C_{Bn}), 70.5 (C-2_{A}), 69.3 (C-6_{E}), 68.3 (C_{All}), 68.0 (C-5_{A}), 18.4 (C-6_{A}).

### ➢ Propyl α-D-glucopyranosyl-(1→3)-α-L-rhamnopyranoside I :

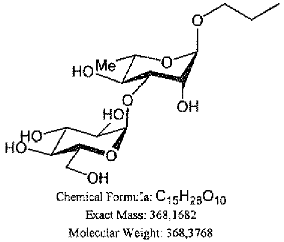

Du Pd-C 10% (420 mg) est ajouté à une solution dégazée de disaccharide **21** (500 mg, 610 µmol) dans un mélange éthanol (24 mL) et acide acétique (70,0 µL, 1,2 mmol, 2 éq.). La suspension est saturée avec de l'hydrogène à pression atmosphérique et agitée à TA pendant 1 nuit. Le suivi par CCM (iPrOH/H₂O/NH₃, 7/1/2 et Chex/EtOAc, 73/27) montre la disparition de **21** (Rf= 1 et 0,45, respectivement) et l'apparition d'un nouveau composé plus polaire (Rf = 0,6 et 0, respectivement). Ensuite le milieu réactionnel est filtré sur célite puis concentré à l'évaporateur rotatif. Le résidu obtenu est purifié sur colonne C-18 (H₂O/CH₃CN, 100/0→9/1) pour conduire à la cible ***I*** sous forme d'un solide blanc (187 mg, 83%).
Rf = 0,6 (iPrOH/H₂O/NH₃, 7/1/2).
RMN ¹H (D₂O), *δ* 5.03 (d, 1H, *J*_{1,2} = 3.8 Hz, H-1_{E}), 4.81 (d, 1H, *J*_{1,2} = 1.6 Hz, H-1_{A}), 4.10 (dd, 1H, *J*_{2,3} = 2.2 Hz, H-2_{A}), 3.92 (m, 1H, H-5_{E}), 3.80-3.70 (m, 5H, H-3_{A}, H-3_{E}, H-6a_{E}, H-6b_{E}, H-5_{A}), 3.62 (m, 1H, H_{Pr}), 3.56-3.45 (m, 3H, H-2_{E}, H-4_{A}, H_{Pr}), 3.42 (pt, 1H, *J*_{3,4} = 9.3 Hz, H-4_{E}), 1.63 (sex, 2H, CH₂), 1.27 (d, 3H, *J*_{5,6} = 6.2 Hz, H-6_{A}), 0.88 (t, 3H, *J* = 7.4 Hz, CH₃).
RMN ¹³C (D₂O), *δ* 99.6 (C-1_{A}, ¹*J*_{CH} = 170.2 Hz), 95.9 (C-1_{E}, ¹*J*_{CH} = 172.7 Hz), 76.1 (C-3_{A}), 73.3 (C-3_{E}), 72.1 (C-5_{E}), 71.8 (C-2_{E}), 70.7 (C-4_{A}), 70.0 (C_{Pr}), 69.8 (C-4_{E}), 69.0 (C-5_{A}), 67.3 (C-2_{A}), 60.7 (C-6_{E}), 22.4 (CH₂), 17.1 (C-6_{A}), 10.3 (CH₃).

| | | |
|---|---|---|
| HRMS (ESI⁺) : | [M+Na]⁺ C₁₅H₂₈O₁₀Na | m/z |
| théorique : 391.1580 | | m/z mesurée : 391.1556 |

### Procédé 2 :

### ➢ Allyl (3,4,6-tri-O-acétyl-2-désoxy-2-trichloroacétamido-β-D-glucopyranosyl)-(1→2)-[2,3,4,6-tétra-O-benzyl-α-D-glucopyranosyl-(1→3)]-4-O-benzyl-α-L-rhamnopyranoside 22 :

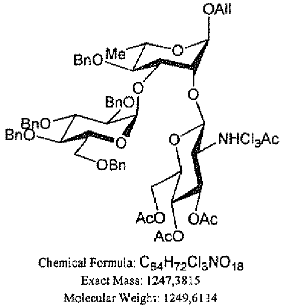

Du TMSOTf (40,0 µL, 230 µmol, 0,2 éq.) est additionné à une solution d'accepteur **21⁴⁵** (920 mg, 1,13 mmol) et de donneur **1** (800 mg, 1,3 mmol, 1,2 éq.) dans du DCM (24 mL), en présence de tamis moléculaire 4 Å (1,0 g), agitée sous argon à -78°C. Après 3 h sous agitation, le bain éthanol-carboglace est retiré et l'agitation est poursuivie pendant 30 min, temps au bout duquel le suivi par CCM (Chex/EtOAc, 7/3) indique la disparition de l'accepteur (Rf = 0,5) et l'apparition d'un nouveau composé plus polaire (Rf= 0,2). La réaction est stoppée par l'addition de triéthylamine (0,2 mL) puis le milieu réactionnel est filtré puis concentré à l'évaporateur rotatif. L'huile jaune obtenue est purifiée par chromatographie sur gel de silice (Chex/EtOAc, 8/2→7/3) pour obtenir le glycoside d'allyle **22** sous forme d'un solide blanc (1,3 g, 92%).
Rf = 0,2 (Chex/EtOAc, 7/3).
RMN ¹H (CDCl₃), *δ* 7.40-7.03 (m, 26H, NH, CH_{Ph}), 5.88 (m, 1H, CH=), 5.29 (m, 1H, *J*ₜᵣₐₙₛ = 17.2 Hz, =CH₂), 5.20 (m, 1H, *J_{cis}* = 10.4 Hz, =CH₂), 5.22-5.16 (m, 3H, H-1_{E}, 2HBn), 5.06 (s, 2H, H_{Bn}), 5.01 (pt, 1H, *J*_{3,4} = 9.7 Hz, H-4_{D}), 4.85 (d, 1H, *J*_{1,2} = 1.5 Hz, H-1_{A}), 4.86-4.75 (m, 3H, H-1_{D}, H-3_{D}, H_{Bn}), 4.72 (d, 1H, *J =* 10.1 Hz, H_{Bn}), 4.55 (m, 2H, H_{Bn}), 4.45 (d, 1H, *J=* 10.9 Hz, H_{Bn}), 4.30 (d, 1H, *J=* 12.0 Hz, H_{Bn}), 4.25-4.12 (m, 4H, H-2_{D}, H-3_{A}, H-3_{E}, H_{All}), 4.07-3.99 (m, 3H, H-6a_{D}, H-6b_{D}, H-5_{E}), 3.98-3.90 (m, 2H, H-2_{A}, H_{All}), 3.91 (dd, 1H, *J*_{1,2} = 3.6 Hz, H-2_{E}), 3.80 (pt, 1H, *J*_{3,4} = 9.2 Hz, H-4_{E}), 3.71 (m, 1H, H-5_{A}), 3.45 (pt, 1H, *J*_{3,4} = 9.8 Hz, H-4_{A}), 3.37 (m, 2H, H-6a_{E}, H-6b_{E}), 2.94 (m, 1H, H-5_{D}), 2.12, 2.02 (2s, 9H, H_{Ac}), 1.40 (d, 3H, *J*_{5,6} = 6.2 Hz, H-6_{A}).
RMN ¹³C (CDCl₃), *δ* 170.6, 170.5, 169.1 (3C, C_{Ac}), 161.9 (C_{NTCA}), 138.5-137.6 (C_{Ph}), 133.7 (CH=), 128.9-127.2 (CH_{Ph}), 117.2 (=CH₂), 100.8 (C-1_{D}, ¹*J*_{CH} = 161.0 Hz), 98.1 (C-1_{A}, ¹*J*_{CH} = 172.2 Hz), 94.5 (C-1_{E}, ¹*J*_{CH} = 165.5 Hz), 92.7 (CCl₃), 83.4 (C-3_{E}), 79.6 (C-4_{A}), 78.9 (C-2_{E}), 78.5 (C-4_{E}), 76.0, 75.1, 74.8 (3C, C_{Bn}), 74.6 (C-3_{A}), 74.4 C-2_{A}), 73.9, 73.3 (2C, C_{Bn}), 73.1 (C-3_{D}), 71.6 (C-5_{D}), 69.8 (C-5_{E}), 68.1 (C-5_{A}), 67.9 (C_{All}), 67.8 (C-4_{D}), 67.7 (C-6_{E}), 61.6 (C-6_{D}), 55.5 (C-2_{D}), 20.7, 20.6, 20.5 (3C, C_{Ac}), 17.8 (C-6_{A}).

| | | |
|---|---|---|
| HRMS (ESI⁺) : | [M+Na]⁺ C₆₄H₇₂NO₁₈³⁵Cl₃Na | m/z théorique : 1270.3713 |
| | | m/z mesurée : 1270.3662 |
| | [M+NH₄]⁺ C₆₄H₇₂NO₁₈³⁵Cl₃NH₄ | m/z théorique : 1265.4159 |
| | | m/z mesurée : 1265.4180 |

### ➢ Allyl (3-O-acétyl-2-désoxy-4,6-O-isopropylidène-2-trichloroacétamido-β-D-glucopyranosyl)-(1→2)-[2,3,4,6-tétra-O-benzyl-α-D-glucopyranosyl-(1→3)]-4-O-benzyl-α-L-rhamnopyranoside 23 :

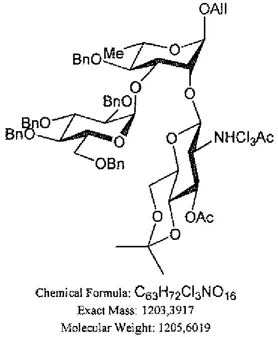

Du TMSOTf (59,0 µL, 330 µmol, 0,3 éq.) est additionné à une solution d'accepteur **21⁴⁵** (900 mg, 1,1 mmol) et de donneur **2** (850 mg, 1,5 mmol, 1,4 éq.) dans du DCM (30 mL), en présence de tamis moléculaire 4 Å (0,9 g), agitée sous argon à -78°C. Après 6 h sous agitation, le bain éthanol-carboglace est retiré et l'agitation est poursuivie pendant 30 min, temps au bout duquel le suivi par CCM (Chex/EtOAc, 7/3) indique la disparition presque totale de l'accepteur (Rf = 0,5) et l'apparition d'un nouveau composé plus polaire (Rf = 0,35). La réaction est stoppée par l'addition de triéthylamine (0,1 mL) puis le milieu réactionnel est filtré et concentré à l'évaporateur rotatif. L'huile jaune obtenue est purifiée par chromatographie sur gel de silice (Chex/EtOAc, 8/2→7/3) pour obtenir le glycoside d'allyle **23** sous forme d'un solide blanc (1,2 g, 90%).
Rf = 0,35 (Chex/EtOAc, 7/3).
RMN ¹H (CDCl₃), *δ*7.42-7.04 (m, 26H, NH, CH_{Ph}), 5.89 (m, 1H, CH=), 5.28 (m, 1H, *J*ₜᵣₐₙₛ = 17.2 Hz, =CH₂), 5.19 (m, 1H, *J*_{cis} = 10.4 Hz, =CH₂), 5.13-5.1 (m, 3H, *J*_{1,2} = 3.3 Hz, 2H_{Bn}, H-1_{E}), 5.04 (d, 2H, H_{Bn}), 4.79-4.74 (m, 3H, H-3_{D}, H-1_{A}, H_{Bn}), 4.71 (m, 2H, H-1_{D}, H_{Bn}), 4.58-4.51 (m, 2H, H_{Bn}), 4.45 (d, 1H, *J* = 10.9 Hz, H_{Bn}), 4.29 (d, 1H, *J*=12.0 Hz, H_{Bn}), 4.18-4.10 (m, 4H, H-3_{A}, H-3_{E}, H_{All}, H-2_{D}), 4.07 (m, 1H, H-5_{E}), 3.97 (bs, 1H, H-2_{A}), 3.94 (m, 1H, H_{All}), 3.91-3.83 (m, 2H, H-2_{E}, H-6a_{D}), 3.78 (pt, 1H, *J*_{3,4} = 9.4 Hz, H-4_{E}), 3.75-3.64 (m, 3H, H-6b_{D}, H-5_{A}, H-4_{D}), 3,43 (pt, 1H, *J*_{3,4} = 9.6 Hz, H-4_{A}), 3.41 (m, 1H, H-6a_{E}, H-6b_{E}), 2.71 (m, 1H, H-5_{D}), 2.07 (s, 3H, H_{Ac}), 1.45 (s, 3H, H_{iPr}), 1.40 (s, 3H, H_{iPr}), 1.39 (d, 3H, *J*_{5,6} = 6.2 Hz, H-6_{A}).
RMN ¹³C (CDCl₃), *δ*171.2 (C_{Ac}), 162.5 (C_{NTCA}), 138.8-137.8 (C_{Ph}), 134.1 (CH=), 129.5-127.7 (CH_{Ph}), 117.6 (=CH₂), 101.8 (C-1_{D}, ¹*J*_{CH} = 164.4 Hz), 100.1 (C_{iPr}), 98.7 (C-1_{A}, ¹J_{CH} = 173.7 Hz), 94.9 (C-1_{E}, ¹*J*_{CH} = 168.6 Hz), 93.1 (CCl₃), 83.8 (C-3_{E}), 80.1 (C-4_{A}) 78.9 (2C, C-2_{E}, C-4_{E}), 76.5, 75.5, 75.3 (3C, C_{Bn}), 74.7 (C-3_{A}), 74.6 (C-2_{A}), 74.4, 73.8 (2C, C_{Bn}), 72.8 (C-3_{D}), 71.5 (C-4_{D}), 70.1 (C-5_{E}), 68.6 (C-5_{A}), 68.2 (2C, C_{All}, C-6_{E}), 67.6 (C-5_{D}), 62.4 (C-6_{D}), 56.8 (C-2_{D}), 29.4 (C_{iPr}), 21.3 (C_{Ac}), 19.3 (C_{iPr}), 18.3 (C-6_{A}).

| | | |
|---|---|---|
| HRMS (ESI⁺) : | [M+Na]⁺ C₆₃H₇₂NO₁₆³⁵Cl₃Na | m/z théorique : 1226.3814 |
| | | m/z mesurée : 1226.3860 |
| | [M+NH₄]⁺ C₆₃H₇₂NO₁₆³⁵Cl₃NH₄ | m/z théorique : 1221.4260 |
| | | m/z mesurée : 1221.4227 |
| | [M+K]⁺ C₆₃H₇₂NO₁₆³⁵Cl₃K | m/z théorique : 1242.3553 |
| | | m/z mesurée : 1242.3586 |

### ➢ Allyl (2-désoxy-2-trichloroacétamido-β-D-glucopyranosyl)-(1→2)-[2,3,4,6-tétra-O-benzyl-α-D-glucopyranosyl-(1→3)]-4-O-benzyl-α-L-rhamnopyranoside 37 :

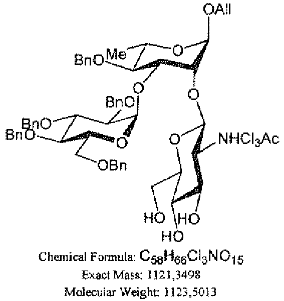

Après addition de NaOMe 0,5 M (160 µL, 8,0 µmol, 0,2 éq.) au tri-*O*-acétyle **22** (500 mg, 40 µmol) en solution dans du MeOH (3 mL), le milieu réactionnel est agité pendant 15 min et son évolution est suivie par CCM (Chex/EtOAc, 1/1, DCM/MeOH, 95/5). Après avoir observé la disparition de **22** (Rf = 0,7 et 0,95, respectivement) et l'apparition d'un produit plus polaire (Rf = 0,05 et 0,3, respectivement), le milieu réactionnel est neutralisé par addition de résine échangeuse d'ion DOWEX (H⁺), puis filtré sur fritté. La résine est lavée avec un mélange DCM/MeOH et les solvants sont évaporés. L'huile jaune obtenue est purifiée par chromatographie sur gel de silice (DCM/MeOH, 99/1→96/4) pour conduire au triol **37** sous forme d'un solide blanc (420 mg, 94%).
Rf= 0,3 (DCM/MeOH, 7/3).
RMN ¹H (CDCl₃), *δ* 7.38-7.06 (m, 26H, NH, CH_{Ph}), 5.88 (m, 1H, CH=), 5.29 (m, 1H, *J*ₜᵣₐₙₛ = 17.2 Hz, =CH₂), 5.20 (m, 1H, *J*_{cis} = 10.4 Hz, =CH₂), 5.10 (d, 1H, *J*_{1,2} = 3.7 Hz, H-1_{E}), 5.07 (m, 3H, *J*= 10.0 Hz, H_{Bn}), 4.90-4.87 (m, 2H, H-1_{A}, H_{Bn}), 4.79-4.70 (m, 2H, *J*= 11.0 Hz, H_{Bn}), 4.62 (d, 1H, *J*_{1,2} = 8.3 Hz, H-1_{D}), 4.61-4.46 (m, 3H, *J* = 11.0 Hz, H_{Bn}), 4.30 (d, 1H, *J*= 12.0 Hz, H_{Bn}), 4.17-4.09 (m, 4H, H-3_{A}, H-5_{E}, H_{All}, H-3_{E},), 4.01 (m, 2H, H-2_{A}, H_{All}), 3.84-3.78 (m, 5H, H-2_{E}, H-4_{E}, H-2_{D}, H-6a_{D}, H-6b_{D}), 3.63 (m, 1H, H-5_{A}), 3.51 (pt, H, *J*_{3,4} = 9.2 Hz, H-4_{D}), 3.44 (m, 3H, H-4_{A}, H-6a_{E}, H-6b_{E}), 3.01 (m, 2H, H-5_{D}, OH), 2.57 (pt, 1H, *J*_{2,3} = 9.6 Hz, H-3_{D}), 2.56 (bs, 1H, OH), 1.40 (d, 3H, *J*_{5,6}= 6.2 Hz, H-6_{A}).
RMN ¹³C (CDCl₃), *δ* 164.4 (C_{NTCA}), 138.9-137.8 (C_{Ph}), 129.5 (CH=), 129.1-127.7 (CH_{Ph}), 117.7 (=CH₂), 101.3 (C-1_{D}, ¹*J*_{CH} = 160.3 Hz), 98.6 (C-1_{A}, ¹*J*_{CH} = 171.6 Hz), 94.8 (C-1_{E}, ¹*J*_{CH} = 166.4 Hz), 92.9 (CCl₃), 83.6 (C-3_{E}), 80.2 (C-4_{A}), 79.5, 79.0 (2C, C-2_{E}, C-4_{E}), 76.6 (C_{Bn}), 76.0 (C-3_{D}), 75.6 (C_{Bn}), 75.5 (C-5_{D}), 75.4, 75.3 (2C, C_{Bn}), 74.8 (C-3_{A}), 74.6 (C-2_{A}), 73.8 (C_{Bn}), 71.6 (C-4_{D}), 70.3 (C-5_{E}), 68.7 (C-5_{A}), 68.3 (C_{All}), 66.2 (C-6_{E}), 62.4 (C-6_{D}), 58.6 (C-2_{D}), 18.3 (C-6_{A}).

| | | |
|---|---|---|
| HRMS (ESI⁺) : | [M+Na]⁺ C₅₈H₆₆NO₁₅³⁵Cl₃Na | m/z théorique : 1144.3396 |
| | | m/z mesurée : 1144.3389 |
| | [M+NH₄]⁺ C₅₈H₆₆NO₁₅³⁵Cl₃NH₄ | m/z théorique : 1139.3842 |
| | | m/z mesurée : 1139.3890 |

### ➢ Propyl (2-acétamido-2-désoxy-β-D-glucopyranosyl)-(1→2)-[α-D-glucopyranosyl-(1→3)]-4-O-benzyl-α-L-rhamnopyranoside II :

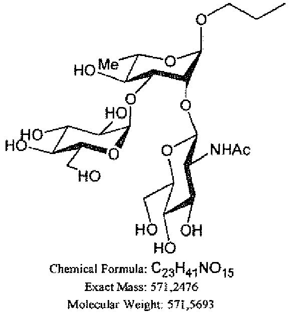

Du Pd-C 10% (280 mg) est ajouté à une solution dégazée de trisaccharide **37** (412 mg, 360 µmol) dans un mélange éthanol (30 mL) et HCl 1M (280 µL). La suspension est saturée avec de l'hydrogène à pression atmosphérique et agitée à TA pendant 1 nuit. Le suivi par CCM (iPrOH/H₂O/NH₃, 7/1/2 et DCM/MeOH, 92/8) montre la disparition de **37** (Rf = 1 et 0,35, respectivement) et l'apparition d'un nouveau composé plus polaire (Rf = 0,45 et 0, respectivement). Ensuite le milieu réactionnel est filtré sur célite puis concentré à l'évaporateur rotatif. Le résidu obtenu est alors dissous dans un mélange éthanol (30 mL) et triéthylamine (153 µL, 1,1 mmol, 3 éq.). Du Pd-C 10% (280 mg) est ajouté au milieu réactionnel. La suspension est alors saturée avec de l'hydrogène à pression atmosphérique et agitée à TA pendant 1 nuit. Le suivi par CCM (iPrOH/H₂O/NH₃, 7/1/2) montre la disparition de l'intermédiaire (Rf = 0,45) et l'apparition d'un nouveau composé plus polaire (Rf = 0,4). Ensuite le milieu réactionnel est filtré sur célite puis concentré à l'évaporateur rotatif. Le résidu obtenu est purifié sur colonne C-18 (H₂O/CH₃CN, 100/0→9/1) pour conduire à la cible ***II*** sous forme d'un solide blanc (144 mg, 69%).
Rf = 0,45 (iPrOH/H₂O/NH₃, 7/1/2).
RMN ¹H (D₂O), *δ* 5.10 (d, 1H, *J*_{1,2} = 3.7 Hz, H-1_{E}), 4.89 (d, 1H, *J*_{1,2} = 1.8 Hz, H-1_{A}), 4.79 (d, 1H, *J*_{1,2} = 8.5 Hz, H-1_{D}), 4.23 (dd, 1H, *J*_{2,3} = 2.3 Hz, H-2_{A}), 4.01 (m, 1H, H-5_{E}), 3.89-3.86 (m, 3H, H-3_{A}, H-6a_{D}, H-6b_{D}), 3.80 (pt, 1H, *J*_{3,4} = 9.7 Hz, H-3_{E}), 3.78-3.64 (m, 6H, H-6b_{D}, H-6a_{E}, H-6b_{E}, H-2_{D}, H-2_{E}, H-5_{A}), 3.60 (m, 1H, H_{Pr}), 3.50-3.44 (m, 2H, H-4_{E}, H_{Pr}), 3.42-3.38 (m, 3H, H-5_{D}, H-3_{D}, H-4_{D}), 3.32-3.34 (pt, 1H, *J*_{3,4} = 9.7 Hz, H-4_{A}), 2.10 (s, 3H, H_{Ac}), 1.57 (sex, 2H, CH₂), 1.24 (d, 3H, *J*_{5,6} = 6.2 Hz, H-6_{A}), 0.87 (t, 3H, *J* = 7.4 Hz, CH₃).
RMN ¹³C (D₂O), *δ* 174.8 (C_{NAc}), 102.3 (C-1_{D}, ¹*J*_{CH} = 163.4 Hz), 99.6 (C-1_{A}, ¹*J*_{CH} = 171.3 Hz), 95.1 (C-1_{E}, ¹*J*_{CH} = 169.5 Hz), 76.3 (C-4_{D}), 74.6 (2C, C-2_{A}, C-3_{D}), 74.5 (C-3_{A}), 73.5 (C-3_{E}), 71.8 (C-5_{E}), 71.7 (C-2_{E}), 71.3 (C-4_{A}), 70.2 (C5-_{D}), 70.0 (C_{Pr}), 69.8 (C-4_{E}), 69.3 (C-5_{A}), 61.0 (C-6_{D}), 60.7 (C-6_{E}), 56.0 (C-2_{D}), 23.0 (C_{NAc}), 22.4 (CH₂), 17.1 (C-6_{A}), 10.3 (CH₃).

| | | |
|---|---|---|
| HRMS (ESI⁺) : | [M+Na]⁺ C₂₃H₄₁NO₁₅Na | m/z théorique : 594.2374 |
| | | m/z mesurée : 594.2379 |

### ➢ Allyl (2-désoxy-4,6-O-isopropylidène-2-trichloroacétamido-β-D-glucopyranosyl)-(1→2)-[2,3,4,6-tétra-O-benzyl-α-D-glucopyranosyl-(1→3)]-4-O-benzyl-α-L-rhamnopyranoside 38 :

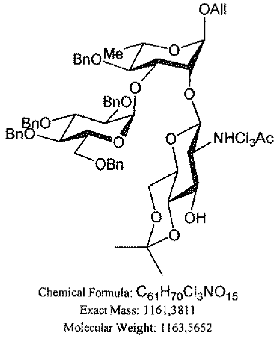

**Voie 1 :** Dans du DMF (3 mL) est dissous le triol 37 (500 mg, 450 µmol) et le 2-méthoxypropène (85 µL, 890 µmol, 2 éq.). Du CSA (10 mg, 23 µmol, 0,1 éq.) est ajouté par fractions au milieu réactionnel. Sous agitation à TA, la réaction est suivie par CCM (Chex/EtOAc, 1/1 et DCM/MeOH, 96/4). Après 6 h d'agitation, la transformation de **37** (Rf = 0,05 et 0,1, respectivement) en un produit moins polaire (Rf= 0,75 et 0,85, respectivement) est presque totale. Le milieu réactionnel est neutralisé avec de la triéthylamine (0,2 mL) puis concentré à l'évaporateur rotatif. Le solide obtenu est purifié par chromatographie sur gel de silice (Chex/EtOAc, 9/1→1/1) pour conduire à l'alcool **38** sous forme d'un solide blanc (390 mg, 76%).
**Voie 2** : Du TMSOTf (65,7 µL, 366 µmol, 0,2 éq.) est additionné à une solution d'accepteur **21** (1,5 g, 1,8 mmol) et de donneur **1** (1,3 g, 2,2 mmol, 1,2 éq.) dans du DCM (37 mL), en présence de tamis moléculaire 4 Å (1,45 g), agitée sous argon à - 78°C. Après 3 h sous agitation, le bain éthanol-carboglace est retiré et l'agitation est poursuivie pendant 30 min, temps au bout duquel le suivi par CCM (Chex/EtOAc, 7/3) indique la disparition de **21** (Rf = 0,5) et l'apparition d'un nouveau composé plus polaire (Rf = 0,2). La réaction est stoppée par l'addition de triéthylamine (0,2 mL) puis le milieu réactionnel est filtré puis concentré à l'évaporateur rotatif. L'huile jaune obtenue est purifiée par chromatographie sur gel de silice (Chex/EtOAc, 8/2→7/3) pour obtenir un solide blanc (2,5 g).
   Après addition de NaOMe 0,5 M (800 µL, 400 µmol, 0,2 éq.) au brut précédent (2,5 g, 2,0 mmol) en solution dans du MeOH (5 mL), le milieu réactionnel est agité pendant 15 min et son évolution est suivie par CCM (Chex/EtOAc, 1/1, DCM/MeOH, 95/5). Après avoir observé la disparition du produit de départ (Rf= 0,7 et 0,95, respectivement) et l'apparition d'un produit plus polaire (Rf = 0,05 et 0,3, respectivement), le milieu réactionnel est neutralisé par addition de résine échangeuse d'ion DOWEX (H⁺), puis filtré sur fritté. La résine est lavée avec un mélange DCM/MeOH et les solvants sont évaporés. L'huile jaune obtenue est engagée directement dans l'étape suivante (2,2 g).
   Le brut précédent (2,2 g, 2,0 mmol) est dissous dans du DMF (15 mL) et le 2-méthoxypropène (385 µL, 4,0 mmol, 2 éq.). Du CSA (93 mg, 400 µmol, 0,2 éq.) est ajouté par fractions au milieu réactionnel. Sous agitation à TA, la réaction est suivie par CCM (Chex/EtOAc, 1/1 et DCM/MeOH, 96/4). Après 6 h d'agitation, la transformation du produit de départ (Rf = 0,05 et 0,12, respectivement) en un produit moins polaire (Rf= 0,75 et 0,85, respectivement) est presque totale. Le milieu réactionnel est neutralisé avec de la triéthylamine (0,2 mL) puis concentré à l'évaporateur rotatif. Le solide obtenu est purifié par chromatographie sur gel de silice (Chex/EtOAc, 9/1→1/1) pour conduire à l'alcool **38** sous forme d'un solide blanc (1,8 g, 86%).
**Voie 3** : Après addition de NaOMe 0,5 M (108 µL, 54 µmol, 0,2 éq.) au glycoside d'allyle **23** (328 mg, 270 µmol) en solution dans du MeOH (5 mL), le milieu réactionnel est agité à TA et la réaction est suivie par CCM (Chex/EtOAc, 1/1, Chex/EtOAc, 7/3). Après une nuit, la transformation de **23** (Rf= 0,75 et 0,5, respectivement) en un produit moins polaire (Rf= 0,6 et 0,2, respectivement) est totale. Le milieu réactionnel est donc neutralisé par addition de résine échangeuse d'ion DOWEX (H⁺), puis filtré sur fritté. La résine est lavée avec un mélange DCM/MeOH et les solvants sont évaporés. Le produit obtenu est purifié par chromatographie sur gel de silice (Chex/EtOAc+NEt₃, 7/3→6/4) pour conduire à l'alcool **38** sous forme d'un solide blanc (241 mg, 76%).
**Voie 4 :** Du TMSOTf (245,0 µL, 1,4 mmol, 0,3 éq.) est additionné à une solution d'accepteur **21** (3,6 g, 4,5 mmol) et du donneur **2** (3,5 g, 6,3 mmol, 1,4 éq.) dans du DCM (8 mL), en présence de tamis moléculaire 4 Å (3,7 g), agitée sous argon à - 78°C. Après 6 h sous agitation, le bain éthanol-carboglace est retiré et l'agitation est poursuivie pendant 30 min, temps au bout duquel le suivi par CCM (Chex/EtOAc, 7/3) indique la disparition de **21** (Rf = 0,5) et l'apparition d'un nouveau composé plus polaire (Rf = 0,35). La réaction est stoppée par l'addition de triéthylamine (0,1 mL) puis le milieu réactionnel est filtré et concentré à l'évaporateur rotatif. L'huile jaune obtenue est purifiée par chromatographie sur gel de silice (Chex/EtOAc, 8/2→7/3) pour obtenir un solide blanc (5,3 g).

Après addition de NaOMe 0,5 M (4,5 mL, 2,2 mmol, 0,5 éq.) au brut précédent (5,3 g, 4,5 mmol) en solution dans du MeOH (60 mL), le milieu réactionnel est agité à TA et la réaction est suivie par CCM (Chex/EtOAc, 6/4). Après 4 h, la transformation du produit de départ (Rf= 0,7) en un produit moins polaire (Rf = 0,5) est totale. Le milieu réactionnel est donc neutralisé par addition de résine échangeuse d'ion DOWEX (H⁺), puis filtré sur fritté. La résine est lavée avec un mélange DCM/MeOH et les solvants sont évaporés. Le produit obtenu est purifié par chromatographie sur gel de silice (Chex/EtOAc+NEt₃, 7/3→6/4) pour conduire à l'alcool **38** sous forme d'un solide blanc (4,5 g, 87%).
Rf = 0,45 (Chex/EtOAc, 6/4).
RMN ¹H (CDCl₃), *δ* 7.42-7.08 (m, 26H, NH, CH_{Ph}), 5.89 (m, 1H, CH=), 5.29 (m, 1H, *J*ₜᵣₐₙₛ = 15.6 Hz, =CH₂), 5.21 (m, 1H, *J*_{cis} = 10.4 Hz, =CH₂), 5.10 (d, 1H, *J*_{1,2} = 3.6 Hz, H-1_{E}), 5.15-4.91 (m, 3H, H_{Bn}), 4.90 (d, 1H, *J* = 12.1 Hz, H_{Bn}), 4.82-4.79 (m, 2H, H-1_{A}, H_{Bn}), 4.74 (d, 1H, *J* = 10.0 Hz, H_{Bn}), 4.62-4.49 (m, 4H, H_{Bn}, H-1_{D}, 2H_{Bn}), 4.34 (d, 1H, *J*=10.9 Hz, H_{Bn}), 4.18-4.11 (m, 4H, H-3_{A}, H-5_{E}, H_{All}, H-3_{E}), 4.02 (bs, 1H, H-2_{A}), 4.01 (m, 1H, H_{All}), 3.91-3.82 (m, 4H, H-2_{E}, H-4_{E}, H-2_{D}, H-6a_{D}), 3.76-3.71 (m, 2H, H-6a_{D}, H-5_{A}), 3.51-3.42 (m, 4H, H-4_{A}, H-6a_{E}, H-6b_{E}, H-4_{D}), 2.84 (m, 2H, H-5_{D}, OH), 2.48 (pt, 1H, *J*_{2,3} = 9.4 Hz, H-3_{D}), 1.51 (s, 3H, H_{iPr}), 1.50 (s, 3H, H_{iPr}), 1.40 (d, 3H, *J*_{5,6} = 6.2 Hz, H-6_{A}).
RMN ¹³C (CDCl₃), *δ* 164.1 (C_{NTCA}), 138.8-137.8 (C_{Ph}), 134.2 (CH=), 129.3-127.7 (CH_{Ph}), 117.6 (=CH₂), 101.5 (C-1_{D}, ¹*J*_{CH} = 163.6 Hz), 100.1 (C_{iPr}), 98.7 (C-1_{A}, ¹*J*_{CH} = 174.0 Hz), 94.5 (C-1_{E}, ¹*J*_{CH} = 167.5 Hz), 92.9 (CCl₃), 83.6 (C-3_{E}), 80.2 (C-4_{A}), 80.1 C-2_{E}), 79.1 (C-4_{E}), 76.6, 75.8, 75.7, 75.3 (4C, C_{Bn}), 74.7 (C-3_{A}), 74.4 (C-4_{D}), 74.0 (C-2_{A}), 73.8 (C_{Bn}), 73.4 (C-3_{D}), 70.2 (C-5_{E}), 68.7 (C-5_{A}), 68.2 (2C, C_{All}, C-6_{E}), 67.4 (C-5_{D}), 62.3 (C-6_{D}), 59.1 (C-2_{D}), 27.3 (C_{iPr}), 19.3 (C_{iPr}), 18.3 (C-6_{A}).

| | | |
|---|---|---|
| HRMS (ESI⁺) : | [M+Na]⁺ C₆₁H₇₀NO₁₅³⁵Cl₃Na | m/z théorique : 1184.3709 |
| | | m/z mesurée : 1184.3669 |
| | [M+NH₄]⁺ C₆₁H₇₀NO₁₅³⁵Cl₃NH₄ | m/z théorique : 1179.4155 |
| | | m/z mesurée : 1179.4165 |

### Procédé 3 :

### ➢ Allyl (2-O-acétyl-3,4-di-O-benzyl-α-L-rhamnopyranosyl)-(1→3)-(2-désoxy-4,6-O-isopyopylidène-2-trichloroacetamido-β-D-glucopyranosyl)-(1→2)-[2,3,4,6-tétra-O-benzyl-α-D-glucopyranosyl-(1→3)]-4-O-benzyl-α-L-rhamnopyranoside 40 :

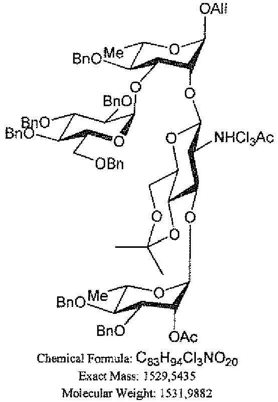

Du TMSOTf (24,0 µL, 131 µmol, 0,3 éq.) est additionné à une solution d'accepteur **38** (510 mg, 440 µmol) et du donneur **39**⁶⁷ (350 mg, 660 µmοl, 1,5 éq.) dans du Tol (11 mL), en présence de tamis moléculaire 4 Å (350 mg), agitée sous argon à -78°C. Après 30 min sous agitation à -78°C, le suivi par CCM (Chex/EtOAc, 75/25 et Chex/EtOAc, 6/4) indique la disparition de **38** (Rf = 0,1 et 0,45, respectivement) et l'apparition d'un nouveau composé plus polaire (Rf = 0,3 et 0,7, respectivement). La réaction est stoppée par l'addition de triéthylamine (0,1 mL) puis le milieu réactionnel est filtré et concentré à l'évaporateur rotatif. L'huile jaune obtenue est purifiée par chromatographie sur gel de silice (Chex/EtOAc, 6,5/3,5→1/1) pour conduire par ordre d'élution au glycoside d'allyle **40** sous forme d'un solide blanc (480 mg, 72%) puis au diol **41** sous forme d'un solide blanc (33 mg, 5%).
Rf = 0,7 (Chex/EtOAc, 6/4).
RMN ¹H (CDCl₃), *δ* 7.45-7.10 (m, 35H, CH_{Ph}), 6.98 (d, 1H, *J*_{NH,2} = 8.6 Hz, NH), 5.92 (m, 1H, CH=), 5.37 (m, 1H, H-2c), 5.29 (m, 1H, *J*ₜᵣₐₙₛ = 16.9 Hz, =CH₂), 5.21 (m, 1H, *J*_{cis} *=* 10.6 Hz, =CH₂), 5.17 (d, 1H, *J*_{1,2} = 3.6 Hz, H-1_{E}), 5.05-4.90 (m, 3H, H_{Bn}), 4.83-4.69 (m, 6H, H-1_{C}, 2H_{Bn}, H-1_{A}, 2H_{Bn}), 4.64-4.50 (m, 7H, 3H_{Bn}, H-1_{D}, 3H_{Bn}), 4.34 (d, 1H, *J* = 12.0 Hz, H_{Bn}), 4.17-4.14 (m, 3H, H-3_{E}, H_{All}, H-3_{A}), 4.12-4.03 (m, 3H, H-5_{E}, H-5_{C}, H-2_{D}), 4.00 (bs, 1H, H-2_{A}), 3.99-3.89 (m, 2H, H_{All}, H-3_{C}), 3.90-3.86 (m, 2H, H-2_{E}, H-6a_{D}), 3.84 (pt, 1H, *J*_{3,4} = 9.8 Hz, H-4_{E}) 3.74-3.69 (m, 2H, H-6b_{D}, H-5_{A}), 3.50 (pt, 1H, *J*_{3,4} = 9.4 Hz, H-4_{D}), 3.49-3.41 (m, 3H, H-4_{A}, H-6a_{E}, H-6b_{E}), 3.40 (pt, 1H, *J*_{3,4} = 9.5 Hz, H-4_{C}), 2.84-2.76 (m, 2H, H-5_{D}, H-3_{D}), 2.16 (s, 3H, H_{Ac}), 1.51 (s, 3H, H_{iPr}), 1.50 (s, 3H, H_{iPr}), 1.41 (d, 3H, *J*_{5,6} = 6.2 Hz, H-6_{A}), 1.41 (d, 3H, *J*_{5,6} = 6.2 Hz, H-6_{C}). RMN ¹³C (CDCl₃), *δ* 170.1 (C_{Ac}), 162.2 (C_{NTCA}), 139.0-137.8 (C_{Ph}), 134.3 (CH=), 129.8-127.7 (CH_{Ph}), 117.5 (=CH₂), 101.7 (C-1_{D}, ¹*J*_{CH} = 161.0 Hz), 99.9 (C_{iPr}), 98.9 (C-1_{C}, ¹*J*_{CH} = 169.1 Hz), 98.8 (C-1_{A}, ¹*J*_{CH} = 171.2 Hz), 94.6 (C-1_{E}, ¹*J*_{CH} = 167.6 Hz), 93.5 (CCl₃), 83.5 (C-3_{E}), 80.6 (C-2_{E}), 80.2 (C-4_{C}), 80.1 (C-4_{A}), 79.1 (C-4_{E}), 79.0 (C-3_{D}), 78.5 (C-3_{C}), 76.5, 75.7, 75.6, 75.3, 75.2 (5C, C_{Bn}), 74.8 (C-3_{A}), 74.1 (C-2_{A}), 73.8 (C_{Bn}), 73.0 (C-4_{D}), 72.2 (C_{Bn}), 70.3 (C-5_{E}), 69.7 (C-2_{C}), 68.7 (C-5_{A}), 68.3 (C-6_{E}), 68.3 (C-5_{C}), 68.2 (C_{All}), 67.4 (C-5_{D}), 62.5 (C-6_{D}), 57.8 (C-2_{D}), 29.5 (C_{iPr}), 21.5 (C_{Ac}), 19.3 (C_{iPr}), 18.4, 18.3 (C-6_{A}, C-6_{C}).

| | | |
|---|---|---|
| HRMS (ESI⁺) : | [M+Na]⁺ C₈₃H₉₄NO₂₀³⁵Cl₃Na | m/z théorique : 1552.5332 |
| | | m/z mesurée : 1552.5293 |
| | [M+NH₄]⁺ C₈₃H₉₄NO₂₀³⁵Cl₃NH₄ | m/z théorique : 1547.5779 |
| | | m/z mesurée : 1547.5818 |
| | [M+K]⁺ C₈₃H₉₄NO₂₀³⁵Cl₃K | m/z théorique : 1568.5072 |
| | | m/z mesurée : 1568.5120 |

### ➢ Allyl (2-O-acétyl-3,4-di-O-benzyl-α-L-rhamnopyranosyl)-(1→3)-(2-désoxy-2-trichloroacétamido-β-D-glucopyranosyl)-(1→2)-[2,3,4,6-tétra-O-benzyl-α-D-glucopyranosyl-(1→3)]-4-O-benzyl-α-L-rhamnopyranoside 41 :

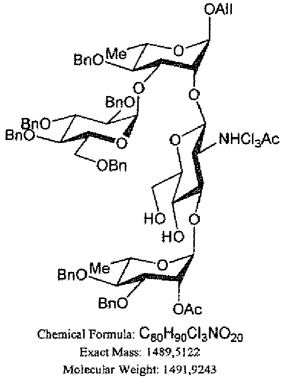

Une solution aqueuse de TFA 50% (1,6 mL) est additionnée lentement à une solution de glycoside d'allyle **40** (79 mg, 52,0 µmol) dans du DCM (4 mL) à 0°C. Le milieu réactionnel est agité pendant 4 h à cette température, temps au bout duquel le suivi par CCM (Chex/EtOAc, 6/4) indique la disparition totale de **40** (Rf = 0,65) et l'apparition d'un nouveau composé plus polaire (Rf = 0,3). Le milieu réactionnel est concentré par coévaporation avec du Tol (3 x 10 mL). L'huile jaune obtenue est purifiée par chromatographie sur gel de silice (Chex/EtOAc, 7/3→6/4) pour obtenir un solide blanc correspondant au diol **41** attendu (68 mg, 88%).
Rf = 0,3 (Chex/EtOAc, 6/4).
RMN ¹H (CDCl₃), *δ* 7.40-7.07 (m, 35H, CH_{Ph}), 6.99 (d, 1H, *J*_{NH,2} = 8.3 Hz, NH), 5.91 (m, 1H, CH=), 5.31 (m, 1H, H-2_{C}), 5.29 (m, 1H, *J*ₜᵣₐₙₛ = 16.9 Hz, =CH₂), 5.25-5.23 (m, 2H, =CH₂, H-1_{E}), 5.12 (m, 2H, H_{Bn}), 5.08 (d, 1H, *J* = 12.6 Hz, H_{Bn}), 4.99-4.91 (m, 2H, H_{Bn}), 4.85 (d, 1H, *J*_{1,2} = 1.5 Hz, H-1_{A}), 4.82 (d, 1H, *J* = 11.1 Hz, H_{Bn}), 4.73 (d, 1H, *J* = 10.1 Hz, H_{Bn}), 4.67 (d, 1H, *J* = 11.4 Hz, H_{Bn}), 4.62-4.51 (m, 6H, 2H_{Bn}, H-1_{D}, 3H_{Bn}) 4.49 (d, 1H, *J*_{1,2} = 1.8 Hz, H-1_{C}), 4.35 (d, 1H, *J* = 11.9 Hz, H_{Bn}), 4.22-4.13 (m, 5H, OH-4_{D}, H-3_{A}, H-3_{E}, H_{All}, H-5_{E}), 4.04-4.01 (m, 3H, H_{All}, H-2_{A}, H-3_{C}), 3.98 (m, 1H, H-2_{D}), 3.96 (m, 1H, H-5_{C}) 3.87-3.82 (m, 3H, H-6a_{D}, H-2_{E}, H-4_{E}), 3.80-3.70 (m, 2H, H-6b_{D}, H-5_{A}), 3.52-3.49 (m, 3H, H-4_{A}, H-6a_{E}, H-6b_{E}), 3.46 (pt, 1H, *J*_{3,4} = 9.4 Hz, H-4_{C}), 3.39 (pt, 1H, *J*_{3,4} = 9.4 Hz, H-4_{D}), 3.09 (m, 1H, H-5_{D}), 2.09 (m, 4H, H_{Ac}, H-3_{D}), 1.41 (d, 3H, *J*_{5,6} = 6.2 Hz, H-6_{A}), 1.34 (d, 3H, *J*_{5,6} = 6.2 Hz, H-6_{C}).
RMN ¹³C (CDCl₃), *δ* 170.5 (C_{Ac}), 162.3 (C_{NTCA}), 138.8 -137.8 (C_{Ph}), 134.3 (CH=), 129.7-127.8 (CH_{Ph}), 117.8 (=CH₂), 101.4 (C-1_{D}, ¹*J*_{CH} = 158.6 Hz), 99.5 (C-1_{C}, ¹*J*_{CH} = 169.8 Hz), 98.6 (C-1_{A}, ¹*J*_{CH} = 174.0 Hz), 94.3 (C-1_{E}, ¹*J*_{CH} = 169.3 Hz), 93.7 (CCl₃), 87.3 (C-3_{D}), 83.4 (C-3_{E}), 81.0 (C-2_{E}), 80.2 (C-4_{A}), 79.6 (C-4_{C}), 79.2 (C-4_{E}), 77.5 (C-3_{C}), 76.6, 75.7, 75.6, 75.5, 75.3 (5C, C_{Bn}), 75.2 (C-5_{D}), 74.6 (C-3_{A}) 74.3 (C-2_{A}), 73.8, 72.2 (2C, C_{Bn}), 71.2 (C-4_{D}), 70.3 (C-5_{E}), 69.8 (C-5_{C}), 69.4 (C-2_{C}), 68.8 (C-5_{A}), 68.3 (C-6_{E}), 68.1 (C_{All}), 63.3 (C-6_{D}), 55.6 (C-2_{D}), 21.4 (C_{Ac}), 18.4 (C-6_{A}), 18.2 (C-6_{C}).

| | | |
|---|---|---|
| HRMS (ESI⁺) : | [M+Na]⁺ C₈₀H₉₀NO₂₀³⁵Cl₃Na | m/z théorique : 1512.5019 |
| | | m/z mesurée : 1512.4950 |
| | [M+NH₄]⁺ C₈₀H₉₀NO₂₀³⁵Cl₃NH₄ | m/z théorique : 1507.5465 |
| | | m/z mesurée : 1507.5507 |
| | [M+K]⁺ C₈₀H₉₀No₂₀³⁵Cl₃K | m/z théorique : 1528.4758 |
| | | m/z mesurée : 1528.4808 |

### ➢ Allyl (3,4-di-O-benzyl-β-L-rhamnopyranosyl)-(1→3)-(2-désoxy-4,6-O-isopropylidène-2-trichloroacétamido-β-D-glucopyranosyl)-(1→2)-[2,3,4,6-tétra-O-benzyl-α-D-glucopyranosyl-(1→3)]-4-O-benzyl-α-L-rhamnopyranoside 43 :

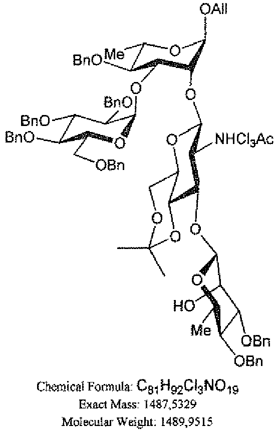

Du TMSOTf (25,0 µL, 140 µmol, 0,5 éq.) est additionné à une solution d'accepteur **38** (300 mg, 260 µmol) et du donneur **39**⁶⁷ (190 mg, 360 µmol, 1,4 éq.) dans du DCM (5 mL), en présence de tamis moléculaire 4 Å (350 mg), agitée sous argon à -78°C. Après 30 min sous agitation, le bain éthanol-carboglace est retiré et l'agitation est poursuivie pendant 30 min, temps au bout duquel le suivi par CCM (Chex/EtOAc, 75/25 et Chex/EtOAc, 6/4) indique la disparition de **38** (Rf = 0,1 et 0,45, respectivement). La réaction est stoppée par l'addition de triéthylamine (0,1 mL) puis le milieu réactionnel est filtré et concentré à l'évaporateur rotatif. L'huile jaune obtenue est purifiée par chromatographie sur gel de silice (Chex/EtOAc, 6,5/3,5→1/1) pour conduire par ordre d'élution au glycoside d'allyle **40** sous forme d'un solide blanc (170 mg, 40%), au composé **42** (80 mg, 20%), légèrement contaminé, puis au diol **41** sous forme d'un solide blanc (85 mg, 22%). Après addition de NaOMe 0,5 M (800 µL, 400 µmol) à **42** (25 mg) en solution dans du MeOH (1 mL), le milieu réactionnel est agité à TA et son évolution est suivie par CCM (Chex/EtOAc, 6/4). Après 4 h, la disparition de **42** (Rf = 0,65) et l'apparition d'un nouveau composé plus polaire (Rf = 0,5) sont observées. Le milieu réactionnel est neutralisé par addition de résine échangeuse d'ion DOWEX (H⁺), puis le milieu réactionnel est filtré sur fritté, La résine est lavée avec un mélange DCM/MeOH et les solvants sont évaporés. L'huile jaune obtenue est purifiée par chromatographie sur gel de silice (Chex/EtOAc, 8/2→6/4) pour conduire à l'alcool **43** sous forme d'un solide blanc (8 mg).
Rf = 0,5 (Chex/EtOAc, 6/4).
RMN ¹H (CDCl₃), *δ*7.55 (d, 1H, *J*_{NH,2} = 8.3 Hz, NH), 7.40-7.03 (m, 35H, CH_{Ph}), 5.88 (m, 1H, CH=), 5.26 (m, 1H, *J*ₜᵣₐₙₛ = 17.2 Hz, =CH₂), 5.18 (m, 1H, *J*_{cis} = 10.4 Hz, =CH₂), 5.10-5.05 (m, 3H, H_{Bn}, H-1_{E}, H_{Bn}), 5.00-4.92 (m, 3H, H_{Bn}), 4.86 (d, 1H, *J* = 12.1 Hz, H_{Bn}), 4.76 (m, 1H, *J*_{1,2} = 1.5 Hz, H-1_{A}), 4.75-4.69 (m, 4H, H_{Bn}, H-1_{D}, 3H_{Bn}), 4.66-4.50 (m, 4H, 3H_{Bn}, H-1_{C}), 4.45 (d, 1H*, J* = 10.9 Hz, H_{Bn}), 4.30 (d, 1H, *J*= 11.9 Hz, H_{Bn}), 4.17-4.02 (m, 6H, H-3_{E}, H-3_{A}, H_{All}, H-5_{E}, H-2_{C}, H-2_{D}), 3.99 (m, 1H, H-2_{A}), 3.91 (m, 1H, H_{All}), 3.85-3.68 (m, 5H, H-6a_{D}, H-2_{E}, H-4_{E}, H-6b_{D}, H-5_{A}), 3.62 (pt, 1H, *J*_{3,4} = 9.4 Hz, H-4_{D}), 3.52-3.39 (m, 6H, H-4_{C}, H-3_{D}, H-4_{A}, H-3_{C}H-6a_{E}, H-6b_{E}), 3.24 (m, 1H, H-5_{C}), 2.72 (m, 1H, H-5_{D}), 1.45 (s, 6H, H_{iPr}), 1.40 (d, 3H, *J*_{5,6} = 6.3 Hz, H-6_{A}), 1.18 (d, 3H, *J*_{5,6}= 6.3 Hz, H-6_{C}).
RMN ¹³C (CDCl₃), *δ*163.2 (C_{NTCA}), 138.8-137.8 (C_{Ph}), 134.2 (CH=), 129.5-127.6 (CH_{Ph}), 117.6 (=CH₂), 101.8 (C-1_{D}, ¹*J*_{CH} = 160.3 Hz), 99.8 (C_{iPr}), 99.1 (C-1c, ¹*J*_{CH} = 156.6 Hz), 98.7 (C-1_{A}, ¹*J*_{CH} = 172.7 Hz), 95.0 (C-1_{E}, ¹*J*_{CH}= 166.1 Hz), 93.4 (CCl₃), 83.8 (C-3_{E}), 81.7 (C-3_{C}), 80.1 (C-4_{A}) 79.7 (C-4_{C}), 79.1 (C-4_{E}), 78.5 (C-2_{E}), 77.1 (C-3_{D}), 76.5, 75.8, 75.5, 75.1 (4C, C_{Bn}), 75.1 (C-3_{A}), 74.6 (C-4_{D}), 74.4 (C-2_{A}), 74.3, 73.8 (2C, C_{Bn}), 71.7 (C-5_{C}), 71.1 (C_{Bn}), 70.7 (C-5_{E}), 68.7 (C-5_{A}), 68.2 (C-2_{C}), 68.2, 68.1 (C-6_{E}, C_{All}), 67.1 (C-5_{D}), 62.4 (C-6_{D}), 56.6 (C-2_{D}), 29.4 (C_{iPr}), 19.5 (C_{iPr}), 18.5 (C-6_{C}), 18.2 (C-6_{A}).

### ➢ Propyl (2/3-O-acétyl-α-L-rhamoopyranosyl)-(1→3)-(2-acétamido-2-désoxy-β-D-glucopyranosyl)-(1→2)-[α-D-glucopyratiosyl-(1→3)]-α-L-rhamnopyranoside III :

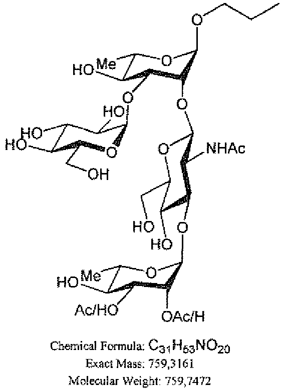

Du Pd-C 10% (300 mg) est ajouté à une solution de tétrasaccharide **41** (340 mg, 320 µmol) dans de l'éthanol (30 mL). La suspension est saturée avec de l'hydrogène à 50 bars et agitée à TA pendant 10 j. Le suivi par CCM (iPrOH/H₂O/NH₃, 4/1/0,5 et Chex/EtOAc, 6/4) montre la disparition de **41** (Rf = 1 et 0,2, respectivement) et l'apparition d'un nouveau composé plus polaire (Rf = 0, 5 et 0, respectivement). Le milieu réactionnel est filtré sur un filtre Acrodisc LC 25 mm, et le filtrat est concentré à l'évaporateur rotatif. Le résidu obtenu est purifié sur colonne C-18 (H₂O/CH₃CN, 100/0→7/3) pour conduire à la cible ***III*** sous forme d'un mélange de deux régioisomères 2_{C}- et 3_{C}-*O*-acétyle et d'un solide blanc (132 mg, 76%).
Rf = 0,5 (iPrOH/H₂O/NH₃, 4/1/0,5).
RMN ¹H (D₂O), *δ*(partial) 5.05 (m, 1H, H-1_{E}), 4.83-4.72 (m, 4H, H-1_{A}, H-1_{C}, H-1_{D}, H-2_{C}), 4.17 (m, 1H, H-2_{A}), 3.93 (m, 1H, H-5_{E}), 3.82-3.3.79 (m, 2H, H-3_{A}, H-5_{C}), 3.73-3.52 (m, 10H, H-2_{D}, H-6a_{D}, H-3_{E}, H-6a_{E}, H-6b_{E}, H-6b_{D}, H-3_{C}H-5_{A}, H-2_{E}, H_{Pr}), 3.42-3.32 (m, 6H, H-4_{E}, H_{Pr}, H-5_{D}, H-4_{C}, H-4_{D}, H-3_{D}), 3.21 (pt, 1H, J_{3,4} = 9.7 Hz, H-4_{A}), 2.07-2.2.01 (m, 6H, H_{AC}, H_{NAC}), 1.48 (sex, 2H, CH₂), 1.17-1.11 (d, 6H, H-6_{A}, H-6_{C}), 0.80 (t, 3H, *J* = 7.4 Hz, CH₃).
RMN ¹³C (D₂O), *δ*(partial) 174.9, 174.5, 174.1, 173.6 (C_{NAc}, C_{Ac}), 101.8 (C-1_{D}), 101.5, 101.2 (C-1_{C}99.0, 98.8 (C-1_{A}), 94.8, 94.7 (C-1_{E}), 82.6, 81.7 (C-3_{D}), 76.4 (C-4_{D}), 74.7 (C-2_{A}), 74.3-73.9 (C-3_{A}, C-2_{C}, C-3_{C}), 73.5, 73.3 (C-3_{E}), 72.4 (C-4_{C}), 71.7, 71.6 (C-5_{E}, C-2_{E}), 71.0 (C-4_{A}), 70.0 (C_{Pr}), 69.6-68.5 (C-4_{E}, C-5_{A}, C-5c, C-5_{D}), 60.9 (C-6_{D}), 60.6 (C-6_{E}), 55.8 (C-2_{D}), 23.1, 22.8 (C_{NAc}), 22.3 (CH₂), 20.8, 20.6 (C_{Ac}), 17.1-16.6 (C-6_{A}, C-6_{C}), 10.2 (CH₃).

| | | |
|---|---|---|
| HRMS (ESI⁺) : | [M+Na]⁺ C₃₁H₅₃NO₂₀Na | m/z théorique : 782.3058 |
| | | m/z mesurée : 782.3066 |

### Procédé 4 :

### ➢ Allyl (3,4-di-O-benzyl-α-L-rhamnopyranosyl)-(1→3)-(2-désoxy-2-trichloroacétamido-β-D-glucopyranosyl)-(1→2)-[2,3,4,6-tétra-O-benzyl-α-D-glucopyranosyl-(1→3)]-4-O-benzyl-α-L-rhamnopyranoside 69 :

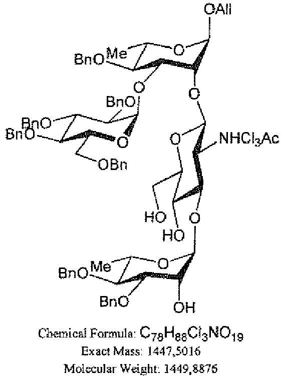

Après addition de NaOMe 0,5 M (102 µL, 51 µmol, 1,1 éq.) au diol **41** (69 mg, 46 µmol) en solution dans du MeOH (3 mL), le milieu réactionnel est porté à reflux. Après 2 h, le suivi par CCM (Chex/EtOAc, 6/4) indique la disparition du produit de départ (Rf = 0,3) et l'apparition d'un produit plus polaire (Rf= 0,2). Après retour à TA, il est neutralisé par addition de résine échangeuse d'ion DOWEX (H⁺), puis filtré sur fritté. La résine est lavée avec un mélange DCM/MeOH et les solvants sont évaporés. Le solide obtenu est purifié par chromatographie sur gel de silice (Chex/EtOAc, 6/4→1/1) pour conduire à un solide blanc correspondant au triol 69 (57 mg, 85%).
Rf= 0,2 (Chex/EtOAc, 6/4).
RMN ¹H (CDCl₃), *δ* 7.41-7.07 (m, 35H, CH_{Ph}), 6.93 (d, 1H, *J*_{NH,2} = 7.7 Hz, NH), 5.89 (m, 1H, CH=), 5.29 (m, 1H, *J*ₜᵣₐₙₛ = 17.2 Hz, =CH₂), 5.23 (m, 1H, *J*_{cis} = 10.4 Hz, =CH₂), 5.21 (d, 1H, *J*_{1,2}= 3.7 Hz, H-1_{E}), 5.13-4.88 (m, 5H, H_{Bn}), 4.84 (d, 1H, *J*_{1,2} = 1.7 Hz, H-1_{A}), 4.79 (d, 1H, *J* = 11.0 Hz, H_{Bn}), 4.73-4.67 (m, 3H, H_{Bn}), 4.62 (m, 1H, H_{Bn}), 4.58 (d, 1H, *J*_{1,2}= 8.5 Hz, H-1_{D}), 4.58 (m, 1H, H_{Bn}), 4.55 (m, 1H, H-1_{C}), 4.51 (d, 2H, *J* = 11.0 Hz, H_{Bn}), 4.34 (d, 1H, *J* = 12.0 Hz, H_{Bn}), 4.19-4.11 (m, 4H, H-3_{A}, H_{All}, H-3_{E}, H-5_{E}), 4.03-4.00 (m, 3H, H-2_{A}, H-2_{C}, H_{All}), 3.96 (m, 1H, H-2_{D}), 3.93-3.87 (m, 3H, H-5_{C}, H-3_{C}H-6a_{D}), 3.85 (m, 1H, H-2_{E}), 3.83 (m, 1H, H-4_{E}), 3.77 (m, 1H, H-6b_{D}), 3.73 (m, 1H, H-5_{A}), 3.50-3.44 (m, 4H, H-4_{C}, H-6a_{E}, H-6b_{E}, H-4_{A}), 3.38 (pt, 1H, *J*_{3,4} = 8.9 Hz, H-4_{D}), 3.08 (m, 1H, H-5_{D}), 2.30 (m, 1H, *J*_{3,4} = 8.8 Hz, H-3_{D}), 1.40 (d, 3H, *J*_{5,6} = 6.2 Hz, H-6_{A}), 1.32 (d, 3H, *J*_{5,6} = 6.2 Hz, H-6_{C}).
RMN ¹³C (CDCl₃), *δ* 162.0 (C_{NTCA}), 138.7-138.2 (C_{Ph}), 134.3 (CH=), 129.7-127.6 (CH_{Ph}), 117.8 (=CH₂), 101.4 (C-1_{D}, ¹*J*_{CH}= 161.7 Hz), 101.0 (C-1c, ¹*J*_{CH}= 168.3 Hz), 98.6 (C-1_{A}, ¹*J*_{CH} = 168.3 Hz), 94.3 (C-1_{E}, ¹*J*_{CH} = 17.6 Hz), 93.7 (CCl₃), 87.1 (C-3_{D}), 83.5 (C-3_{E}), 80.8 (C-2_{E}), 80.2 (C-4_{A}), 79.5 (C-4_{C}), 79.4 (C-3_{C}), 79.3 (C-4_{E}), 76.6, 75.7, 75.6, 75.5, 75.3 (5C, C_{Bn}), 75.3 (C-5_{D}), 74.5 (2C, C-2_{A}, C-3_{A}), 73.8, 72.2 (2C, C_{Bn}), 71.1 (C-4_{D}), 70.3 (C-5_{E}), 69.5 (C-5_{C}), 68.9 (C-2_{C}), 68.8 (C-5_{A}), 68.4 (C-6_{E}), 68.1 (C_{All}), 63.3 (C-6_{D}), 55.9 (C-2_{D}), 21.4 (C_{Ac}), 18.3, 18.2 (C-6_{A}* C-6_{C}*).

| | | |
|---|---|---|
| HRMS (ESI⁺) : | [M+Na]⁺ C₇₈H₈₈NO₁₉³⁵Cl₃Na | m/z théorique : 1470.4913 |
| | | m/z mesurée : 1470.5050 |
| | [M+NH₄]⁺ C₇₈H₈₈NO₁₉³⁵Cl₃NH₄ | m/z théorique : 1465.5360 |
| | | m/z mesurée : 1465.5448 |
| | [M+K]⁺ C₇₈H₈₈NO₁₉³⁵Cl₃K | m/z théorique : 1486.4653 |
| | | m/z mesurée : 1486.4777 |

### ➢ Propyl α-L-rhamnopyranosyl-(1→3)-(2-acétamido-2-désoxy-β-D-glucopyranosyl)-(1→2)-[α-D-glucopyranosyl-(1→3) 1-α-L-rhamnopyranoside IV :

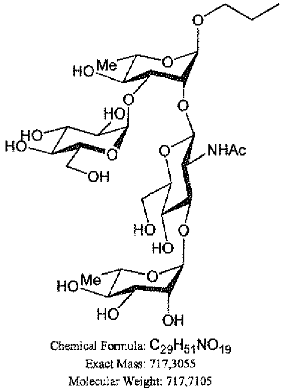

Du Pd-C 10% (300 mg) est ajouté à une solution dégazée de tétrasaccharide **68** (320 mg, 220 µmol) dans de l'éthanol (30 mL). La suspension est saturée avec de l'hydrogène à 50 bars et agitée à TA pendant 2j. Le suivi par CCM (iPrOH/H₂O/NH₃, 4/1/0,5 et Chex/EtOAc, 6/4) montre la disparition de **68** (Rf = 1 et 0,2, respectivement) et l'apparition d'un nouveau composé plus polaire (Rf = 0,65 et 0, respectivement). Le milieu réactionnel est filtré sur un filtre Acrodisc LC 25 mm, et le filtrat est concentré à l'évaporateur rotatif. Le résidu obtenu est purifié sur colonne C-18 (H₂O/CH₃CN, 100/0→7/3) pour conduire à la cible *IV* sous forme d'un solide blanc (119 mg, 71%).
Rf = 0,65 (iPrOH/H₂O/NH₃, 4/1/0,5).
RMN ¹H (D₂O), *δ* 5.06 (d, 1H, *J*_{1,2} = 3.7 Hz, H-1_{E}), 4.83 (d, 1H, *J*_{1,2} = 1.6 Hz, H-1_{A}), 4.76 (d, 1H, *J*_{1,2} = 1.3 Hz, H-1_{C}), 4.73 (d, 1H, *J*_{1,2} = 8.6 Hz, H-1_{D}), 4.18 (m, 1H, H-2_{A}), 3.95 (m, 1H, H-5_{E}), 3.88 (m, 1H, H-5_{C}), 3.83-3.79 (m, 2H, H-3_{A}, H-6a_{D}), 3.74 (m, 1H, H-2_{D}), 3.72 (m, 1H, H-3_{E}), 3.70-3.67 (m, 3H, H-2_{C}, H-6a_{E}, H-6b_{E}), 3.65-3.59 (m, 4H, H-6b_{D}, H-3_{C}H-5_{A}, H-2_{E}), 3.52 (m, 1H, R_{Pr}), 3.42-3.28 (m, 6H, H-4_{E}, H_{Pr}, H-4_{D}, H-5_{D}, H-3_{D}, H-4_{C}), 3.23 (pt, 1H, *J*_{3,4} = 9.7 Hz, H-4_{A}), 2.01 (s, 3H, H_{NAc}), 1.49 (sex, 2H, CH₂), 1.16 (d, 3H, *J*_{5,6} = 6.2 Hz, H-6_{A}), 1.12 (d, 3H, *J*_{5,6} = 6.3 Hz, H-6_{C}), 0.80 (t, 3H, *J* = 7.4 Hz, CH₃).
RMN ¹³C (D₂O), *δ* 174.5 (C_{NAc}), 101.8 (C-1_{D}, ¹*J*_{CH}= 160.3 Hz), 101.6 (C-1c, ¹*J*_{CH} = 170.5 Hz), 99.0 (C-1_{A}, ¹*J*_{CH} = 172.7 Hz), 94.8 (C-1_{E}, ¹*J*_{CH} = 169.8 Hz), 81.7 (C-3_{D}), 76.4 (C-4_{D}), 74.7 (C-2_{A}), 74.1 (C-3_{A}), 73.5 (C-3_{E}), 72.2 (C-4_{C}), 71.7 (C-5_{E}), 71.6 (C-2_{E}), 71.2 (C-2_{C}), 71.0 (C-4_{A}), 70.5 (C-3_{C}), 70.0 (C_{Pr}), 69.6 (C-5_{D}), 69.3 (C-5_{C}), 69.1 (C-5_{A}), 68.6 (C-4_{E}), 60.9 (C-6_{D}), 60.6 (C-6_{E}), 55.9 (C-2_{D}), 22.8 (C_{NAc}), 22.3 (CH₂), 17.0, 16.7 (C-6_{A}, C-6_{C}), 10.2 (CH₃).

| | | |
|---|---|---|
| HRMS (ESI⁺) : | [M+Na]⁺ C₂₉H₅₁NO₁₉Na | m/z théorique : 740.2953 |
| | | m/z mesurée : 740.2941 |

### Procédé 5 :

### ➢ Allyl (2,3,4-tri-O-acétyl-α-L-rhamnopyranosyl)-(1→3)-2-O-acétyl-4-O-benzyl-α-L-rhamnopyranoside 50 :

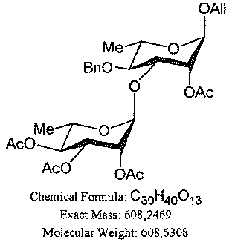

Du TMSOTf (124,0 µL, 690 µmol, 0,3 éq.) est additionné à une solution d'accepteur **31** (780 mg, 2,3 mmol) et de donneur **48**^{82,49} (1,5 g, 3,5 mmol, 1,5 éq.) dans du DCM (60 mL), en présence de tamis moléculaire 4 Å (2,0 g), agitée sous argon à -78°C. Après 6 h, à cette température, le bain éthanol-carboglace est retiré et l'agitation est poursuivie pendant 30 min, temps au bout duquel le suivi par CCM (Tol/EtOAc, 8/2) indique la disparition de **31** (Rf = 0,3) et l'apparition d'un nouveau composé moins polaire (Rf= 0,35). La réaction est stoppée par addition de triéthylamine (0,1 mL) puis le milieu réactionnel est filtré et concentré à l'évaporateur rotatif. L'huile jaune obtenue est purifiée par chromatographie sur gel de silice (Tol/EtOAc, 9/1→8/2) pour obtenir le glycoside d'allyle **50** sous forme d'un solide blanc (1,3 g, 95%).
Rf = 0,35 (Tol/EtOAc, 8/2).
RMN ¹H (CDCl₃), *δ*7.34-7.02 (m, 5H, CH_{Ph}), 5.87 (m, 1H, CH=), 5.33 (m, 1H, H-2_{B}), 5.28 (m, 1H, =CH₂), 5.25 (m, 1H, H-3_{B}), 5.20 (m, 1H, =CH₂), 5.18 (m, 1H, H-2_{C}), 5.07 (pt, 1H, *J*_{3,4} = 9.9 Hz, H-4_{B}), 5.04 (d, 1H, *J*_{1,2} = 1.5 Hz, H-1_{B}), .82 (d, 1H, *J* = 10.8 Hz, H_{Bn}), 4.75 (d, 1H, *J*_{1,2} = 1.6 Hz, H-1_{C}), 4.67 (d, 1H, *J* = 10.9 Hz, H_{Bn}), 4.17-4.12 (m, 2H, H-3_{C}H_{All}), 4.00-3.91 (m, 2H, H_{All}, H-5_{B}), 3.77 (m, 1H, H-5_{C}), 3.51 (pt, 1H, *J*_{3,4} = 9.5 Hz, H-4_{C}), 2.21, 2.08, 2.07, 1.99 (4s, 12H, H_{Ac}), 1.34 (d, 3H, *J*_{5,6} = 6.2 Hz, H-6_{C}), 1.21 (d, 3H, *J*_{5,6} = 6.3 Hz, H-6_{B}).
RMN ¹³C (CDCl₃), δ 170.9, 170.4, 170.3, 170.2 (4C, C_{Ac}), 138.3 (C_{Ph}), 133.8 (CH=), 129.4-128.1 (CH_{Ph}), 117.9 (=CH₂), 99.8 (C-1_{B}, ¹*J*_{CH} = 173.0 Hz), 96.8 (C-1_{C}, ¹*J*_{CH} = 170.0 Hz), 81.1 (C-4_{C}), 77.2 (C-3_{C}), 75.6 (C_{Bn}), 72.5 (C-2_{C}), 71.1 (C-4_{B}), 70.2 (C-2_{B}), 69.5 (C-3_{B}), 68.5 (C_{All}), 68.3 (C-5c), 67.6 (C-5_{B}), 21.4, 21.2, 21.1, 21.0 (4C, C_{Ac}), 18.3 (C-6_{C}), 17.7 (C-6_{B}).

| | | |
|---|---|---|
| HRMS (ESI⁺) : | [M+Na]⁺ C₃₀H₄₀O)₃Na | m/z théorique : 631.2367 |
| | | m/z mesurée : 631.2368 |
| | [M+K]⁺ C₃₀H₄₀O₁₃K | m/z théorique : 647.2106 |
| | | m/z mesurée : 647.2124 |

### ➢ (2,3,4-Tri-O-acétyl-α-L-rhamnopyranosyl)-(1→3)-2-O-acétyl-4-O-benzyl-α-L-rhamnopyranose 51 :

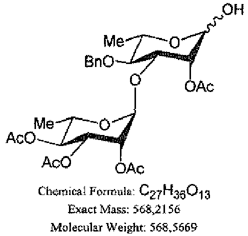

Le (1,5-cyclooctadiènebis(méthyldiphénylphosphine)iridium(I)hexafluorophosphate) (170 mg) est dissous dans du THF (16 mL) sous agitation. Le milieu subit 5 cycles dégazage vide/argon, 5 cycles dégazage vide/hyrdogène puis est mis sous hydrogène pendant 15 min. Le milieu subit alors une seconde fois 5 cycles dégazage vide/argon. La solution de glycoside d'allyle **50** (1,3 g, 2,2 mmol) dans du THF (8 mL) est transférée sur la solution de catalyseur activé. Le suivi par CCM (Tol/EtOAc, 8/2) montre la disparition de **50** (Rf= 0,35) et l'apparition d'un produit proche moins polaire (Rf= 0,4).

Du diiode (1,1 g, 4,4 mmol) en solution dans un mélange THF/H₂O (8/2, 15 mL) est ajouté au milieu réactionnel. Le suivi par CCM (Tol/EtOAc, 8/2 et DCM/MeOH, 95/5) indique la disparition de l'intermédiaire (Rf= 0,35 et 0,95, respectivement) et l'apparition d'un nouveau composé beaucoup plus polaire (Rf= 0 et 0,55, respectivement). Pour stopper la réaction, une solution aqueuse de NaHSO₃ 10% (10 mL) est ajoutée par fractions au milieu jusqu'à disparition de la coloration noire. Le milieu réactionnel est repris avec du DCM (30 mL) et la phase aqueuse est extraite au DCM (3 x 20 mL). Les phases organiques sont regroupées et lavées avec une solution de NaClₛₐₜ (3 x 15 mL), avec de l'H₂O (3 x 15 mL) puis séchées sur filtre séparateur de phases et concentrées à l'évaporateur rotatif. L'huile jaune obtenue est purifiée par chromatographie sur gel de silice (DCM/MeOH, 99/1→9/1) pour obtenir l'hémiacétal **51** sous forme d'une huile jaune (1,1 g, 91%).
Rf = 0,55 (DCM/MeOH, 95/5).
RMN ¹H (CDCl₃), *δ*: α : 7.33-7.26 (m, 5H, CH_{Ph}), 5.34 (m, 1H, H-2B), 5.25 (m, 1H, H-3_{B}), 5.18 (m, 1H, H-2_{C}), 5.10 (s, 1H, H-1_{C}), 5.05 (pt, 1H, *J*_{3,4} = 9.9 Hz, H-4_{B}), 5.04 (d, 1H, *J*_{1,2} = 1.7 Hz, H-1_{B}), 4.80 (d, 1H, *J* = 10.9 Hz, H_{Bn}), 4.65 (d, 1H, *J* = 10.9 Hz, H_{Bn}), 4.19 (m, 1H, H-3_{C}), 4.01-3.91 (m, 2H, H-5_{C}, H-5_{B}), 3.58 (s, 1H, OH), 3.49 (pt, 1H, *J*_{3,4} = 9.5 Hz, H-4_{C}), 2.21, 2.08, 2.07, 1.99 (4s, 12H, H_{Ac}), 1.31 (d, 3H, *J*_{5,6}= 6.2 Hz, H-6_{C}), 1.19 (d, 3H, *J*_{5,6}= 6.3 Hz, H-6_{B}).
RMN ¹³C (CDCl₃), *δ*: α : 171.7, 171.0, 170.6, 170.3 (4C, C_{Ac}), 138.3-137.9 (C_{Ph}), 128.8-128.1 (CH_{Ph}), 99.7 (C-1_{B}, ¹*J*_{CH}= 172.7 Hz), 92.3 (C-1c, ¹*J*_{CH}= 170.5 Hz), 81.1 (C-4_{C}), 76.6 (C-3_{C}), 75.9 (C_{Bn}), 72.9 (C-2_{C}), 71.1 (C-4_{B}), 70.2 (C-2_{B}), 69.5 (C-3_{B}), 68.2 (C-5_{C}), 67.6 (C-5_{B}), 21.4, 21.2, 21.1, 21.0 (4C, C_{Ac}), 18.3 (C-6_{C}), 17.6 (C-6_{B}).

| | | |
|---|---|---|
| HRMS (ESI⁺) : | [M+Na]⁺ C₂₇H₃₆O₁₃Na | m/z théorique : 591.2054 |
| | | m/z mesurée: 591.2037 |
| | [M+K]⁺ C₂₇H₃₆O₁₃K | m/z théorique : 607.1793 |
| | | m/z mesurée : 607.1838 |

### ➢ Tricholroacétimidate de (2,3,4-tri-O-acétyl-α-L-rhamnopyranosyl)-(1→3)-2-O-acétyl-4-O-benzyl-α/β-L-rhamnopyranose 46 :

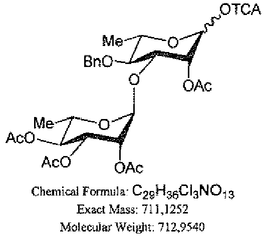

L'hémiacétal **51** (1,0 g, 1,7 mmol) est solubilisé dans du DCE (7 mL) et agité sous argon à -5°C puis de la DBU (72 µL, 476 µmol, 0,28 éq.) et du trichloroacétonitrile (2,2 mL, 21,5 mmol, 12 éq.) sont ajoutés. Le milieu réactionnel est agité à cette température pendant 15 min puis il est déposé, sans aucun traitement, sur une colonne de gel de silice (flash chromatographie, Chex/EtOAc+5% NEt₃, 7/3) pour obtenir le trichloroacétimidate 46 sous forme d'une huile jaune (1,1 g, 89%).
Rf = 0,5 (Chex/EtOAc, 6/4).
RMN ¹H (CDCl₃), *δ* : α : 8.70 (s, 1H, NH), 7.33-7.27 (m, 5H, CH_{Ph}), 6.18 (d, 1H, *J*_{1,2} = 1.7 Hz, H-1_{C}), 5.35 (m, 2H, H-2_{B}, H-2_{C}), 5.22 (m, 1H, H-3_{B}), 5.08 (s, 1H, J_{1,2} = 1.5 Hz, H-1_{B}), 5.07 (pt, 1H, *J*_{3,4} = 9.9 Hz, H-4_{B}), 4.80 (d, 1H, *J* = 10.8 Hz, H_{Bn}), 4.68 (d, 1H, *J* = 10.8 Hz, H_{Bn}), 4.23 (m, 1H, H-3_{C}), 4.00-3.92 (m, 2H, H-5_{C}, H-5_{B}), 3.62 (pt, 1H, *J*_{3,4} = 9.6 Hz, H-4_{C}), 2.26, 2.08, 2.07, 1.99 (4s, 12H, H_{Ac}), 1.37 (d, 3H, *J*_{5,6} = 6.3 Hz, H-6_{C}), 1.19 (d, 3H, *J*_{5,6} = 6.3 Hz, H-6_{B}).
RMN ¹³C (CDCl₃), *δ*: α : 170.6, 170.5, 170.4, 170.2 (4C, C_{Ac}), 164.0 (C=NH), 137.8 (C_{Ph}), 128.8-128.3 (CH_{Ph}), 99.6 (C-1_{B}, ¹*J*_{CH} = 173.4 Hz), 95.1 (C-1c, ¹*J*_{CH}= 178.6 Hz), 91.2 (CCl₃), 80.5 (C-4_{C}), 76.2 (C_{Bn}), 75.6 (C-3_{C}), 71.3 (C-5_{C}), 70.9 (C-4_{B}), 70.7 (C-2_{C}), 70.0 (C-2_{B}), 69.4 (C-3_{B}), 67.8 (C-5_{B}), 21.3, 21.2, 21.1, 21.0 (4C, C_{Ac}), 18.3 (C-6_{C}), 17.7 (C-6_{B}).

### ➢ Allyl (2,3,4-tri-O-acétyl-α-L-rhamnopyranosyl)-(1→3)-(2-O-acétyl-4-O-benzyl-α-L-rhamnopyranosyl)-(1→3)-(2-désoxy-4,6-O-isopropylidène-2-trichloroacétamido-β-D-glucopyranosyl)-(1→2)-[2,3,4,6-tétra-O-benzyl-α-D-glucopyranosyl-(1→3)1-4-O-benzyl-α-L-rhamnopyranoside 52 :

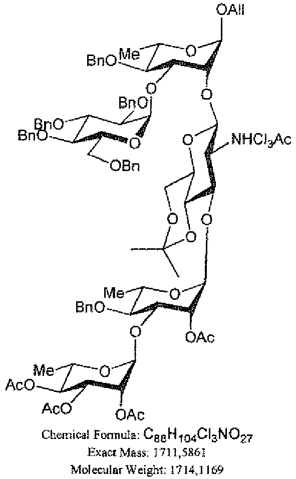

Du TMSOTf (50,0 µL, 280 µmol, 0,3 éq.) est additionné à une solution d'accepteur **38** (1,1 g, 935 µmol) et de donneur **46** (935 mg, 1,3 mmol, 1,4 éq.) dans du Tol (5,6 mL), en présence de tamis moléculaire 4 Å (765 mg), agitée sous argon à -78°C. Après 15 min sous agitation, le suivi par CCM (Tol/EtOAc, 7/3) indique la disparition de **38** (Rf = 0,35) et l'apparition d'un nouveau composé plus polaire (Rf= 0,55). La réaction est stoppée par l'addition de triéthylamine (0,1 mL) puis le milieu réactionnel est filtré et concentré à l'évaporateur rotatif. L'huile jaune obtenue est purifiée par chromatographie sur gel de silice (Tol/EtOAc, 9/1→4/6) pour obtenir par ordre d'élution un solide blanc correspondant au glycoside d'allyle **52** (1,3 g, 80%) puis au diol **53** à savoir l'allyl (2,3,4-tri-*O*-acétyl-α-L-rhamnopyranosyl)-(1→3)-(2-*O-*acétyl-4-*O*-benzyl-α-L-rhamnopyranosyl)-(1→3)-(2-désoxy-2-trichloroacétamido-β-D-glucopyranosyl)-(1→2)-[2,3,4,6-tétra-*O*-benzyl-α-D-glucopyranosyl-(1→3)]-4-*O-*benzyl-α-L-rhamnopyranoside (Rf = 0,25, 108 mg, 7%) contaminé par le donneur hydrolysé.
Rf = 0,55 (Tol/EtOAc, 7/3).
RMN ¹H (CDCl₃), *δ*7.48-7.12 (m, 35H, CH_{Ph}), 6.95 (d, 1H, *J*_{NH,2} = 8.8 Hz, NH), 5.92 (m, 1H, CH=), 5.38 (m, 1H, H-2_{B}), 5.29 (m, 1H, *J*ₜᵣₐₙₛ= 16.9 Hz, =CH₂), 5.28 (m, 1H, H-3_{B}), 5.22 (d, 1H, *J*_{1,2} = 4.1 Hz, H- 1_{E}), 5.21 (m, 1H, *J*_{cis} = 10.6 Hz, =CH₂), 5.17 (m, 1H, H-2_{C}), 5.15 (m, 4H, H-1_{B}, H-4_{B}, 2H_{Bn}), 5.05 (d, 1H, *J* = 12.2 Hz, H_{Bn}), 4.95 (d, 1H, *J* = 12.2 Hz, H_{Bn}), 4.86-4.82 (m, 3H, H-1_{A}, 2H_{Bn}), 4.76-4.70 (m, 3H, H_{Bn}, H-1_{C}, H_{Bn}), 4.62-4.53 (m, 4H, 2H_{Bn}, H-1_{D}, H_{Bn}), 4.36 (d, 1H, *J* = 12.0 Hz, H_{Bn}), 4.22-4.14 (m, 6H, H-3_{E}, H_{All}, H-3_{C}H-3_{A}, H-5_{B}, H-5_{E}), 4.09-4.04 (m, 3H, H-2_{D}, H-5_{C}, H-2_{A}), 3.99 (m, 1H, H_{All}), 3.93-3.88 (m, 2H, H-2_{E}, H-6a_{D}), 3.85 (pt, 1H, *J*_{3,4} = 9.6 Hz, H-4_{E}), 3.77-3.72 (m, 2H, H-5_{A}, H-6b_{D}), 3.57 (pt, 1H, *J*_{3,4} = 9.2 Hz, H-4_{D}), 3.52-3.45 (m, 4H, H-4_{C}, H-4_{A}, H-6a_{E}, H-6b_{E}), 2.84-2.80 (m, 2H, H-5_{D}, H-3_{D}), 2.26, 2.13, 2.11, 2.03 (4s, 12H, H_{Ac}), 1.52 (s, 3H, H_{iPr}), 1.50 (s, 3H, H_{iPr}), 1.44 (d, 3H, *J*_{5,6} = 6.2 Hz, H-6_{A}), 1.37 (d, 3H, *J*_{5,6}= 6.2 Hz, H-6_{B}), 1.33 (d, 3H, *J*_{5,6} = 6.2 Hz, H-6_{C}).
RMN ¹³C (CDCl₃), *δ* 170.6, 170.5, 170.3, 170.2 (4C, C_{Ac}), 162.4 (C_{NTCA}), 138.6-137.8 (C_{Ph}), 134.3 (CH=), 129.8-127.7 (CH_{Ph}), 117.6 (=CH₂), 101.6 (C-1_{D}, ¹*J*_{CH} = 164.7 Hz), 99.9 (C_{iPr}), 99.2 (C-1_{B}, ¹*J*_{CH} = 174.2 Hz), 98.8 (C-1_{A}, ¹*J*_{CH} = 171.2 Hz), 98.4 (C-1_{C}, ¹*J*_{CH} = 170.5 Hz), 94.7 (C-1_{E}, ¹*J*_{CH} = 166.9 Hz), 93.4 (CCl₃), 83.5 (C-3_{E}), 81.5 (C-4_{C}), 80.7 (C-2_{E}), 80.2 (C-4_{A}), 79.2 (C-4_{E}), 78.7 (C-3_{D}), 76.5 (C_{Bn}), 75.6 (C-3_{C}), 75.7, 75.6, 75.4, 75.3 (4C, C_{Bn}), 74.9 (C-3_{A}), 74.1 (C-2_{A}), 73.8 (C_{Bn}), 72.9 (C-4_{D}), 72.5 (C-2_{C}), 71.2 (C-4_{B}), 70.3 (2C, C-2_{B}, C-5_{E}), 69.6 (C-3_{B}), 68.8 (C-5_{A}), 68.4 (C-6_{E}), 68.3 (C-5_{C}), 68.2 (C_{All}), 67.8 (C-5_{B}), 67.4 (C-5_{D}), 62.5 (C-6_{D}), 58.0 (C-2_{D}), 29.6 (C_{iPr}), 21.4, 21.3, 21.2, 21.1 (4C, C_{Ac}), 19.5 (C_{iPr}), 18.5 (C-6_{C}), 18.3 (C-6_{A}), 17.7 (C-6_{B}).

| | | |
|---|---|---|
| HRMS (ESI⁺) : | [M+Na]⁺ C₈₈H₁₀₄³⁵Cl₃NO₂₇Na | m/z théorique : 1734.5759 |
| | | m/z mesurée: 1734.5825 |
| | [M+NH₄]⁺ C₈₈H₁₀₄³⁵Cl₃NO₂₇NH₄ | m/z théorique : 1729.6205 |
| | | m/z mesurée : 1729.6278 |

### ➢ Allyl (α- L-rhamnopyranosyl)-(1→3)-(2-O-acétyl-4-O-benzyl-α-L-hamnopyranosyl)-(1→3)-(2-désoxy-4,6-O-isopropylidène-2-trichloroacétamido-β-D-glucopyranosyl)-(1→2)-[2,3,4,6-tétra-O-benzyl-α-D-glucopyranosyl-(1→3)]-4-O-benzyl-α-L-rhamnopyranoside 54 :

### ➢ Allyl α-L-rhamnopyranosyl-(1→3)-(4-O-benzyl-α-L-rhamnopyranosyl)-(1→3)-(2-désoxy-4,6-O-isopropylidène-2-trichloroacétamido-β-D-glucopyranosyl)-(1→2)-[2,3,4,6-tétra-O-benzyl-α-D-glucopyranosyl-(1→3)]-4-O-benzyl-α-L-rhamnopyranoside 55 :

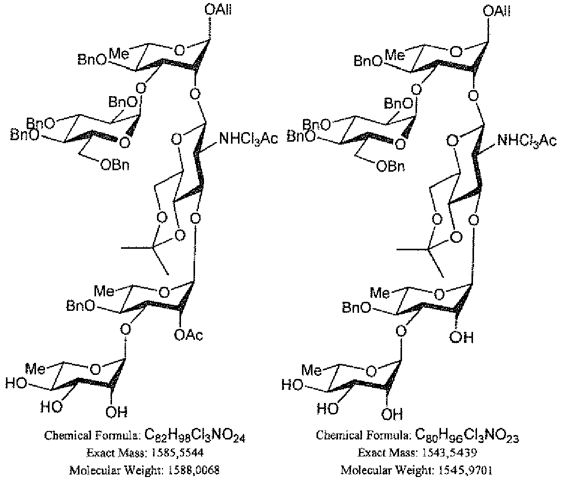

Le glycoside d'allyle **52** (525 mg, 300 µmol) est dissous dans du MeOH (20 mL) puis du carbonate de potassium (42 mg, 300 µmol, 1 éq.) est ajouté au milieu réactionnel. Après 25 min sous agitation à TA, le suivi par CCM (DCM/MeOH, 96/4 et Tol/EtOAc, 8/2) indique la disparition de **52** (Rf= 0,35 et 1, respectivement) et l'apparition de deux produits plus polaires (Rf = 0,3 et 0,25 dans Tol/EtOAc, 8/2). Le milieu réactionnel est alors neutralisé par addition de résine échangeuse d'ion DOWEX (H⁺), puis filtré sur fritté. La résine est lavée avec un mélange DCM/MeOH et les solvants sont évaporés. L'huile jaune obtenue est purifiée par chromatographie sur gel de silice (Chex/Acétone, 6/4→4/6) pour conduire par ordre d'élution au produit mono-acétylé **54** sous forme d'un solide blanc (414 mg, 85%) et au produit non-acétylé **55** sous forme d'un solide blanc (33 mg, 7%). **54 :** Rf= 0,3 (DCM/MeOH, 96/4).
RMN ¹H (CDCl₃), *δ* 7.42-7.07 (m, 35H, CH_{Ph}), 6.95 (d, 1H, *J*_{NH,2} = 8.8 Hz, NH), 5.92 (m, 1H, CH=), 5.26 (m, 1H, *J*ₜᵣₐₙₛ = 17.2 Hz, =CH₂), 5.18 (m, 1H, *J*_{cis} = 10.4 Hz, =CH₂), 5.15 (d, 1H, *J*_{1,2} = 3.4 Hz, H-1_{E}), 5.09-5.05 (m, 4H, H-2_{C}, H-1_{B}, 2H_{Bn}), 4.98 (d, 1H, *J =* 12.2 Hz, H_{Bn}), 4.87 (d, 1H, *J* = 12.2 Hz, H_{Bn}), 4.80-4.74 (m, 3H, H-1_{A}, 2H_{Bn}), 4.70 (d, 1H, *J* = 11.2 Hz, H_{Bn}), 4.66-4.63 (m, 3H, H_{Bn}, H-1_{C}, H_{Bn}), 4.56-4.47 (m, 3H, 2H_{Bn}, H-1_{D}), 4.30 (d, 1H, *J* = 11.9 Hz, H_{Bn}), 4.14-4.05 (m, 5H, H_{All}, H-3_{E}, H-3_{A}, H-5_{E}, H-3_{C}), 4.00-3.94 (m, 4H, H-2_{D}, H_{All}, H-2_{A}, H-5_{C}), 3.87-3.84 (m, 4H, H-6a_{D}, H-2_{B}, H-5_{B}, H-2_{E}), 3.79 (pt, 1H, *J*_{3,4} = 9.1 Hz, H-4_{E}), 3.70-3.65 (m, 3H, H-6b_{D}, H-5_{A}, H-3_{B}), 3.53 (pt, 1H, *J*_{3,4} = 9.2 Hz, H-4_{D}), 3.46-3.37 (m, 5H, H-4_{B}, H-6a_{E}, H-6b_{E}, H-4_{C}, H-4_{A}), 2.81-2.71 (m, 2H, H-5_{D}, H-3_{D}), 2.14 (s, 3H, H_{Ac}), 1.47 (s, 3H, H_{iPr}), 1.43 (s, 3H, H_{iPr}), 1.38 (d, 3H, *J*_{5,6} = 5.9 Hz, H-6_{A}), 1.36 (d, 3H, *J*_{5,6} = 6.0 Hz, H-6_{B}), 1.25 (d, 3H, *J*_{5,6} = 6.2 Hz, H-6_{C}).
RMN ¹³C (CDCl₃), *δ* 170.5 (C_{Ac}), 162.6 (C_{NTCA}), 138.7-138.1 (C_{Ph}), 134.2 (CH=), 129.8-127.7 (CH_{Ph}), 117.7 (=CH₂), 101.6 (C-1_{B}, ¹*J*_{CH}= 170.5 Hz), 101.4 (C-1_{D}, ¹*J*_{CH} = 159.5 Hz), 99.9 (C_{iPr}), 98.7 (C-1_{A}, ¹*J*_{CH} = 172.0 Hz), 98.2 (C-1_{C}, ¹*J*_{CH} = 169.1 Hz), 94.6 (C-1_{E}, ¹*J*_{CH} = 166.9 Hz), 93.3 (CCl₃), 83.4 (C-3_{E}), 81.3 (C-4_{C}), 80.6 (C-2_{E}), 80.1 (C-4_{A}), 79.1 (C-4_{E}), 78.7 (C-3_{D}), 76.5 (C_{Bn}), 76.0 (C-3_{C}), 75.7, 75.6, 75.5, 75.3 (4C, C_{Bn}), 74.8 (C-3_{A}), 73.9 (C-2_{A}), 73.8 (C-4_{B}), 73.7 (C_{Bn}), 72.9 (C-2_{C}), 72.7 (C-4_{D}), 71.8 (C-3_{B}), 71.4 (C-2_{B}), 70.2 (C-5_{E}), 69.2 (C-5_{B}), 68.7 (C-5_{A}), 68.3 (C-5_{C}), 68.2 (2C, C-6_{E}, C_{All}), 67.3 (C-5_{D}), 62.4 (C-6_{D}), 57.9 (C-2_{D}), 29.6 (C_{iPr}), 21.6 (C_{Ac}), 19.5 (C_{iPr}), 18.4 (C-6_{A}), 18.3 (C-6_{C}), 17.8 (C-6_{B}).

| | | |
|---|---|---|
| HRMS (ESI⁺) : | [M+Na]⁺ C₈₂H₉₈³⁵Cl₃NO₂₄Na | m/z théorique : 1608.5542 |
| | | m/z mesurée : 1608.5582 |
| | [M+NH₄]⁺ C₈₂H₉₈³⁵Cl₃NO₂₄NH₄ | m/z théorique : 1603.5889 |
| | | m/z mesurée : 1603.6035 |
| | [M+K]⁺ C₈₂H₉₈³⁵Cl₃NO₂₄K | m/z théorique : 1624.5182 |
| | | m/z mesurée : 1624.5436 |

**55** : Rf = 0,25 (DCM/MeOH, 96/4).
RMN ¹H (CDCl₃), *δ* 7.38-7.04 (m, 35H, CH_{Ph}), 6.93 (d, 1H, *J*_{NH,2} = 8.8 Hz, NH), 5.86 (m, 1H, CH=), 5.26 (m, 1H, *J*ₜᵣₐₙₛ = 17.2 Hz, =CH₂), 5.18 (m, 1H, *J*_{cis} = 10.4 Hz, =CH₂), 5.15 (d, 1H, *J*_{1,2} = 3.5 Hz, H-1_{E}), 5.09-4.95 (m, 4H, H-1_{B}, 3H_{Bn}), 4.89 (d, 1H, *J* = 12.3 Hz, H_{Bn}), 4.78 (d, 1H, *J* = 11.0 Hz, H_{Bn}), 4.75 (d, 1H, *J*_{1,2} = 1.0 Hz, H-1_{A}), 4.73-4.61 (m, 4H, 2H_{Bn}, H-1_{C}, H_{Bn}), 4.57 (d, 1H, *J* = 12.0 Hz, H_{Bn}), 4.51-4.47 (m, 2H, H_{Bn}), 4.41 (d, 1H, *J*_{1,2} = 8.4 Hz, H-1_{D}), 4.32 (d, 1H, *J* = 12.0 Hz, H_{Bn}), 4.14-4.07 (m, 4H, H_{All}, H-3_{A}, H-3_{E}, H-5_{E}), 4.03 (d, 1H, *J*_{1,2} = 9.2 Hz, H-2_{D}), 4.00-3.91 (m, 4H, H-5_{C}, H-2_{A}, H-3_{C}, H_{All}), 3.89-3.79 (m, 6H, H-2_{C}, H-2_{B}, H-2_{E}, H-6a_{D}, H-4_{E}, H-3_{B}), 3.75-3.64 (m, 3H, H-5_{B}, H-6b_{D}, H-5_{A}), 3.52-3.48 (pt, 2H, *J*_{3,4} = 9.2 Hz, H-4_{D}, H-4_{B}), 3.44 (m, 2H, H-6a_{E}, H-6b_{E}), 3.48 (pt, 1H, *J*_{3,4} = 9.9 Hz, H-4_{C}), 3.48 (pt, 1H, *J*_{3,4} = 9.5 Hz, H-4_{A}), 2.74-2.69 (m, 2H, H-3_{D}, H-5_{D}), 1.46 (s, 3H, H_{iPr}), 1.42 (s, 3H, H_{iPr}), 1.41 (d, 3H, *J*_{5,6} = 6.0 Hz, H-6_{B}), 1.37 (d, 3H, *J*_{5,6} = 6.2 Hz, H-6_{A}), 1.22 (d, 3H, *J*_{5,6} = 6.2 Hz, H-6_{C}). RMN ¹³C (CDCl₃), *δ* 162.3 (C_{NTCA}), 138.8-138.2 (C_{Ph}), 134.2 (CH=), 129.8-127.7 (CH_{Ph}), 117.6 (=CH₂), 102.0 (C-1_{B}, ¹*J*_{CH} = 175.6 Hz), 101.4 (C-1_{D}, ¹*J*_{CH} = 162.5 Hz), 100.2 (C-1_{C}, ¹*J*_{CH} = 171.2 Hz), 99.8 (C_{iPr}), 98.7 (C-1_{A}, ¹*J*_{CH} = 172.0 Hz), 94.4 (C-1_{E}, ¹*J*_{CH} = 166.1 Hz), 93.3 (CCl₃), 83.5 (C-3_{E}), 80.7 (C-4_{C}), 80.5 (C-2_{E}), 80.1 (C-4_{A}), 79.4 (C-3_{C}), 79.1 (C-4_{E}), 78.0 (C-3_{D}), 76.6, 75.8, 75.6, 75.4, 75.3 (5C, C_{Bn}), 74.5 (C-3_{A}), 73.8 (C-4_{B}), 73.7 (C_{Bn}), 73.6 (C-2_{A}), 72.5 (C-4_{D}), 72.0 (C-3_{B}), 71.5 (C-2_{C}), 71.4 (C-2_{B}), 70.1 (C-5_{E}), 69.2 (C-5_{B}), 68.7 (C-5_{A}), 68.2 (2C, C-6_{E}, C_{All}), 68.0 (C-5_{C}), 67.3 (C-5_{D}), 62.4 (C-6_{D}), 58.1 (C-2_{D}), 30.1 (C_{iPr}), 19.5 (C_{iPr}), 18.3, 18.2 (C-6_{A}^{*},C-6c^{*}), 17.8 (C-6_{B}).

| | | |
|---|---|---|
| HRMS (ESI⁺) : | [M+Na]⁺ C₈₀H₉₆³⁵Cl₃NO₂₃Na | m/z théorique : 1566.5337 |
| | | m/z mesurée : 1566.5404 |
| | [M+NH₄]⁺ C₈₀H₉₆³⁵Cl₃NO₂₃NH₄ | m/z théorique : 1561.5782 |
| | | m/z mesurée : 1561.5812 |

### ➢ Allyl (α-L-rhamnopyranosyl)-(1→3)-(2-O-acétyl-4-O-benzyl-α-L-rhamnopyranosyl)-(1→3)-(2-désoxy-2-trichloroacétamido-β-D-glucopyranosyl)-(1→2)-[2,3,4,6-tétra-O-benzyl-α-D-glucopyranosyl-(1→3)]-4-O-benzyl-α-L-rhamnopyranoside 56 :

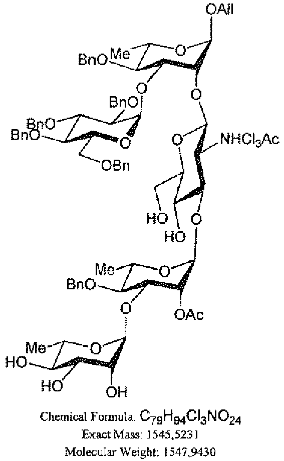

Une solution aqueuse de TFA 50% (2 mL) est additionnée lentement à une solution de triol **54** (292 mg, 180 µmol) dans du DCM (5 mL) à 0°C. Le milieu réactionnel est agité pendant 2 h à cette température, temps au bout duquel le suivi par CCM (DCM/MeOH, 95/5) indique la disparition totale de **54** (Rf = 0,35) et l'apparition d'un nouveau composé plus polaire (Rf = 0,3). Le milieu réactionnel est concentré par coévaporation avec du Tol (3 x 10 mL). L'huile jaune obtenue est purifiée par chromatographie sur gel de silice (DCM/MeOH, 95/5→93/7) pour obtenir le mono-acétylé **56** sous forme d'un solide blanc (274 mg, 95%).
Rf = 0,3 (DCM/MeOH, 95/5).
RMN ¹H (CDCl₃), *δ* 7.39-7.05 (m, 36H, CH_{Ph}, NH), 5.88 (m, 1H, CH=), 5.28 (m, 1H, *J*ₜᵣₐₙₛ = 17.2 Hz, =CH₂), 5.22-5.20 (m, 2H, H-1_{E}, =CH₂), 5.11 (m, 1H, H-2_{C}), 5.09-5.02 (m, 3H, H_{Bn}), 4.98 (m, 1H, H-1_{B}), 4.93 (d, 1H, *J* = 12.5 Hz, H_{Bn}), 4.84 (d, 1H, *J*_{1,2} = 1.3 Hz, H-1_{A}), 4.81-4.73 (m, 3H, H_{Bn}), 4.63-4.60 (m, 3H, H_{Bn}, H-1_{D}, H_{Bn}), 4.57 (m, 1H, H-1_{C}), 4.54-4.49 (m, 2H, H_{Bn}), 4.33 (d, 1H, *J* = 12.0 Hz, H_{Bn}), 4.17-4.07 (m, 6H, H-3_{A}, H_{All}, H-3_{E}, H-5_{E}, H-3_{C}, H-2_{A}), 4.00-3.95 (m, 2H, H-2_{D}, H_{All}), 3.90 (m, 1H, H-5_{C}), 3.88-3.85 (m, 2H, H-2_{B}, H-6a_{D}), 3.84-3.78 (m, 2H, H-2_{E}, H-4_{E}), 3.77-3.61 (m, 4H, H-6b_{D}, H-5_{B}, H-3_{B}, H-5_{A}), 3.51-3.41 (m, 5H, H-4_{A}, H-6a_{E}, H-6b_{E}, H-4_{C}, H-4_{B}), 3.53 (pt, 1H, *J*_{3,4} = 8.9 Hz, H-4_{D}), 3.04 (m, 1H, H-5_{D}), 2.29 (m, 1H, H-3_{D}), 2.19 (s, 3H, H_{Ac}), 1.25 (d, 3H, *J*_{5,6} = 6.2 Hz, H-6_{B}), 1.36 (d, 3H, *J*_{5,6} = 6.2 Hz, H-6_{C}), 1.38 (d, 3H, *J*_{5,6} = 6.2 Hz, H-6_{A}).
RMN ¹³C (CDCl₃), *δ* 170.5 (C_{Ac}), 162.8 (C_{NTCA}), 138.9-138.2 (C_{Ph}), 134.3 (CH=), 129.7-127.8 (CH_{Ph}), 117.7 (=CH₂), 102.1 (C-1_{B}, ¹*J*_{CH}= 167.9 Hz), 101.1 (C-1_{D}, ¹*J*_{CH} = 164.0 Hz), 98.7 (C-1_{C}, ¹*J*_{CH} = 170.3 Hz), 98.6 (C-1_{A}, ¹*J*_{CH} = 170.3 Hz), 94.5 (C-1_{E}, ¹*J*_{CH} = 167.9 Hz), 93.2 (CCl₃), 86.6 (C-3_{D}), 83.4 (C-3_{E}), 80.7 (C-2_{E}), 80.3 (C-4_{C}), 79.9 (C-4_{A}), 79.1 (C-4_{E}), 77.7 (C-3_{C}), 76.6, 75.9, 75.7, 75.4, 75.3 (5C, C_{Bn}), 75.2 (C-5_{D}), 74.8 (C-3_{A}), 74.0 (C-2_{A}), 73.8 (C-4_{B}), 73.7 (C_{Bn}), 72.5 (C-2_{C}), 71.9 (C-3_{B}), 71.4 (C-2_{B}), 70.9 (C-4_{D}), 70.3 (C-5_{E},), 69.5 (C-5_{C}), 69.4 (C-5_{A}), 68.8 (C-5_{B}), 68.4 (C-6_{E}), 68.2 (C_{All}), 63.1 (C-6_{D}), 55.8 (C-2_{D}), 21.4 (C_{Ac}), 18.4 (C-6_{C}), 18.3 (C-6_{B}), 17.8 (C-6_{A}).

| | | |
|---|---|---|
| HRMS (ESI⁺) : | [M+Na]⁺ C₇₉H₉₄³⁵Cl₃NO₂₄Na | m/z théorique : 1568.5129 |
| | | m/z mesurée : 1568.5162 |
| | [M+NH₄]⁺ C₇₉H₉₄³⁵Cl₃NO₂₄NH₄ | m/z théorique : 1563.5575 |
| | | m/z mesurée : 1563.5681 |

### ➢ Allyl α-L-rhamnopyranosyl-(1→3)-(4-O-benzyl-α-L-rhamnopyranosyl)-(1→3)-(2-désoxy-2-trichloroacétamido-β-D-glucopyranosyl)-(1→2)-[2,3,4,6-tétra-O-benzyl-α-D-glucopyranosyl-(1→3)]-4-O-benzyl-α-L-rhamnopyranoside 57 :

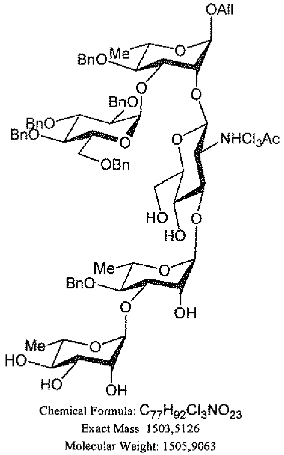

Une solution aqueuse de TFA 50% (2 mL) est additionnée lentement à une solution de tétraol **55** (340 mg, 180 µmol) dans du DCM (5 mL) à 0°C. Le milieu réactionnel est agité pendant 2 h à cette température, temps au bout duquel le suivi par CCM (DCM/MeOH, 94/6) indique la disparition totale de **55** (Rf = 0,35) et l'apparition d'un nouveau composé plus polaire (Rf = 0,2). Le milieu réactionnel est concentré par coévaporation avec du ToI (3 x 10 mL). L'huile jaune obtenue est purifiée par chromatographie sur gel de silice (DCM/MeOH, 95/5→93/7) pour obtenir le non-acétylé **57** sous forme d'un solide blanc (314 mg, 95%).
Rf = 0,2 (DCM/MeOH, 94/6).
RMN ¹H (CDCl₃), *δ* 7.34-7.05 (m, 35H, CH_{Ph}, NH), 6.93 (d, 1H, *J*_{NH,2} = 8.7 Hz, 5.86 (m, 1H, CH=), 5.26 (m, 1H, *J*ₜᵣₐₙₛ = 17.2 Hz, =CH₂), 5.20 (m, 1H, *J*_{cis} = 10.4 Hz, =CH₂), 5.17 (d, 1H, *J*_{1,2} = 3.6 Hz, H-1_{E}), 5.06 (d, 1H, *J* = 11.0 Hz, H_{Bn}), 5.02 (m, 1H, H-1_{B}), 5.01-4.97 (m, 2H, H_{Bn}), 4.88 (d, 1H, *J* = 12.3 Hz, H_{Bn}), 4.81 (d, 1 H, *J*_{1*,*2} = 1.4 Hz, H-1_{A}), 4.78-4.69 (m, 3H, H_{Bn}), 4.62-4.56 (m, 3H, H_{Bn}, H-1_{D}, H_{Bn}), 4.51-4.47 (m, 3H, H_{Bn}, H-1_{C}, H_{Bn}), 4.32 (d, 1H, *J* = 12.0 Hz, H_{Bn}), 4.15-4.13 (m, 2H, H-3_{A}, H_{All}), 4.11-4.07 (m, 2H, H-3_{E}, H-5_{E}), 4.02 (m, 1H, H-2_{A}), 3.98-3.95 (m, 2H, H-3_{C}, H_{All}), 3.93-3.87 (m, 4H, H-2_{C} H-2_{D}, H-5_{C}, H-2_{B}), 3.84 (m, 1H, H-6a_{D}), 3.82-3.71 (m, 5H, H-5_{B}, H-2_{E}, H-4_{E}, H-6b_{D}, H-3_{B}), 3.69 (m, 1H, H-5_{A}), 3.48 (pt, 1H, *J*_{3,4} = 9.2 Hz, H-4_{B}), 3.47-3.37 (m, 5H, H-4_{C}, H-6a_{E}, H-6b_{E}, H-4_{A}, H-4_{D}), 3.04 (m, 1H, H-5_{D}), 2.38 (pt, 1H, *J*_{3,4} = 9.1 Hz, H-3_{D}), 1.27 (d, 3H, *J*_{5,6} = 6.5 Hz, H-6_{C}), 1.36 (d, 3H, *J*_{5,6} = 6.2 Hz, H-6_{A}), 1.37 (d, 3H, *J*_{5,6}= 6.2 Hz, H-6_{B}).
RMN ¹³C (CDCl₃), δ 162.4 (C_{NTCA}), 138.2-137.9 (C_{Ph}), 134.2 (CH=), 129.2-127.7 (CH_{Ph}), 117.7 (=CH₂), 101.4 (C-1_{B}, ¹*J*_{CH} = 167.6 Hz), 101.1 (C-1_{D}, ¹*J*_{CH} = 159.5 Hz), 100.9 (C-1_{C}, ¹*J*_{CH} = 168.3 Hz), 98.6 (C-1_{A}, ¹*J*_{CH} = 174.2 Hz), 94.3 (C-1_{E}, ¹*J*_{CH}= 169.1 Hz), 93.3 (CCl₃), 85.8 (C-3_{D}), 83.4 (C-3_{E}), 80.6 (C-2_{E}), 80.3 (C-4_{A}), 80.0 (C-4_{C}), 79.2 (C-4_{E}), 78.8 (C-3c), 76.6, 75.7, 75.6, 75.5 (4C, C_{Bn}), 75.3 (C-5_{D}), 75.2 (C_{Bn}), 74.5 (C-3_{A}), 74.0 (C-2_{A}), 73.7 (C_{Bn}), 73.6 (C-4_{B}), 71.9 (C-3_{B}), 712 (C-2_{B}), 70.9 (C-2_{C}), 70.6 (C-4_{D}), 70.3 (C-5_{E},), 69.4 (2C, C-5_{B}, C-5_{C}), 68.8 (C-5_{A}), 68.3 (C-6_{E}), 68.1 (C_{All}), 63.0 (C-6_{D}), 56.3 (C-2_{D}), 18.2, 18.1, 18.0 (C-6_{A}^{*}, C-6_{B}^{*}, C-6_{C}^{*}).

| | | |
|---|---|---|
| HRMS (ESI⁺) : | [M+Na]⁺ C₇₇H₉₂³⁵Cl₃NO₂₃Na | m/z théorique : 1526.5023 |
| | | m/z mesurée : 1526.5076 |
| | [M+NH₄]⁺ C₇₇H₉₂³⁵Cl₃NO₂₃NH₄ | m/z théorique : 1521.5470 |
| | | m/z mesurée: 1521.5582 |
| | [M+K]⁺ C₇₇H₉₂³⁵Cl₃NO₂₃K | m/z théorique : 1542.4763 |
| | | m/z mesurée : 1542.4871 |

### ➢ Allyl 3,4-di-O-benzyl-α-L-rhamnopyranoside^{96,102} 58 :

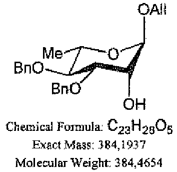

Le diol **26** (1,9 g, 6,6 mmol) et l'oxyde de dibutylétain (1,8 g, 7,2 mmol, 1,1 éq.) sont dissous dans du Tol (52 mL). Le milieu réactionnel est chauffé à reflux durant 2 h surmonté d'un soxhlet contenant du tamis moléculaire activé. La solution est refroidie à 60°C et le soxhlet est enlevé. Du flurorure de césium (1,5 mL, 13,1 mmol, 2 éq.) est ajouté et le milieu réactionnel est évaporé. Le résidu est repris au DMF (50 mL) et du bromure de benzyle (1,6 mL, 13,1 mmol, 2 éq.) est additionné. Le milieu réactionnel est alors agité durant une nuit à TA, temps au bout duquel le suivi par CCM (Chex/EtOAc, 7/3) montre l'apparition d'un nouveau composé moins polaire (Rf = 0,35). Le milieu réactionnel est alors filtré, repris au dichlorométhane (200 mL) et lavé avec une solution de NaCl sat (3 x 50 mL), de l'H₂O (3 x 50 mL) puis séché sur Na₂SO₄ et concentré à l'évaporateur rotatif. L'huile jaune obtenue est purifiée par chromatographie sur gel de silice (Chex/EtOAc, 8/2→4/6) pour conduire à l'alcool **58** sous forme d'une huile incolore (2,2 g, 88% soit 68% *via* **27**).
Rf = 0,35 (Chex/EtOAc, 7/3).
RMN ¹H (CDCl₃), *δ* 7.41-7.36 (m, 10H, CH_{Ph}), 5.93 (m, 1H, CH=), 5.32 (m, 1H, *J*ₜᵣₐₙₛ = 17.2 Hz, =CH₂), 5.23 (m, 1H, *J*_{cis} = 10.4 Hz, =CH₂), 4.94 (d, 1H, *J* = 10.9 Hz, H_{Bn}), 4.90 (d, 1H, *J*_{1,2} = 1.5 Hz, H-1), 4.74 (m, 2H, H_{Bn}), 4.70 (d, 1H, *J* = 10.9 Hz, H_{Bn}), 4.19 (m, 1H, H_{All}), 4.10 (m, 1H, H-2), 4.03 (m, 1H, H_{All}), 3.93 (dd, 1H, *J*_{2,3} = 3.3 Hz, *J*_{3,4} = 9.1 Hz, H-3), 3.81 (m, 1H, H-5), 3.53 (pt, 1H, *J*_{4,5} = 9.3 Hz, H-4), 2.69 (bs, 1H, OH), 1.38 (d, 3H, *J*_{5,6} = 6.3 Hz, H-6).
RMN ¹³C (CDCl₃), *δ* 138.8-138.4 (C_{Ph}), 134.3 (CH=), 128.9-128.1 (CH_{Ph}), 117.7 (=CH₂), 98.7 (C-1, ¹*J*_{CH}= 167.6 Hz), 80.5 (C-4), 80.4 (C-3), 75.8, 72.4 (2C, C_{Bn}), 69.0 (C-2), 68.3 (C_{All}), 67.8 (C-5), 18.3 (C-6).

### ➢ Allyl 3,4-di-O-benzyl-2-O-lévulinoyl-α-L-rhamnopyranoside 59 :

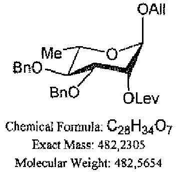

L'alcool **58** (2,2 g, 5,7 mmol) est dissous dans du DCM (120 mL) puis la DCC (1,8 g, 8,5 mmol, 1,5 éq.), la DMAP (1,4 g, 11,3 mmol, 2 éq.), et l'acide lévulinique (1,1 mL, 10,2 mmol, 1,8 éq.) sont ajoutés par portions. Le milieu réactionnel est agité durant une nuit, temps au bout duquel le suivi par CCM (DCM/EtOAc, 9/1) montre la formation d'un composé moins polaire majoritaire (Rf = 0,75) et la disparition de **58** (Rf = 0,65). La DCU est filtrée sur célite et le filtrat est repris à l'H₂O (50 mL). La phase aqueuse est extraite au DCM (3 x 200 mL). Les phases organiques sont regroupées et lavées avec une solution d'HCl 10% (3 x 100 mL), une solution de NaHCO₃ₛₐₜ (3 x 100 mL), une solution de NaClₛₐₜ (3 x 100 mL), filtrées sur filtre séparateur de phases et concentrées à l'évaporateur rotatif. L'huile obtenue est purifiée par chromatographie sur gel de silice (Chex/EtOAc, 85/15→8/2) pour conduire à l'ester de lévulinoyle **59** sous forme d'une huile incolore (2,4 g, 89%).
Rf = 0,4 (Chex/EtOAc, 7/3).
RMN ¹H (CDCl₃), *δ* 7.39-7.30 (m, 10H, CH_{Ph}), 5.91 (m, 1H, CH=), 5.43 (m, 1H, H-2), 5.28 (m, 1H, *J*ₜᵣₐₙₛ = 15.8 Hz, =CH₂), 5.22 (m, 1H, *J*_{cis} = 10.4 Hz, =CH₂), 4.95 (d, 1H, *J* = 10.8 Hz, H_{Bn}), 4.81 (d, 1H, *J*_{1,2} = 1.5 Hz, H-1), 4.72 (d, 1H, *J* = 11.2 Hz, H_{Bn}), 4.65 (d, 1H, *J* = 10.8 Hz, H_{Bn}), 4.55 (d, 1H, *J* = 11.2 Hz, H_{Bn}), 4.16 (m, 1H, H_{All}), 3.99 (m, 2H, H_{All}, H-3), 3.81 (m, 1H, H-5), 3.46 (pt, 1H, *J*_{4,5} = 9.4 Hz, H-4), 2.74 (m, 4H, 2CH_{2Lev}), 2.19 (s, 3H, CH_{3Lev}), 1.37 (d, 3H, *J*_{5,6} = 6.2 Hz, H-6).
RMN ¹³C (CDCl₃), *δ* 206.6 (C_{Lev}), 172.5 (C_{Lev}), 138.9-138.5 (C_{Ph}), 133.9 (CH=), 128.9-128.1 (CH_{Ph}), 118.0 (=CH₂), 97.1 (C-1, ¹*J*_{CH} = 169.8 Hz), 80.5 (C-4), 78.5 (C-3), 75.8, 72.0 (2C, C_{Bn}), 69.5 (C-2), 68.4 (C_{All}, 68.1 (C-5), 38.4 (CH_{2Lev}), 30.2 (CH_{3Lev}), 28.6 (CH_{2Lev}), 18.4 (C-6).

| | | |
|---|---|---|
| HRMS (ESI⁺) : | [M+Na]⁺ C₂₈H₃₄O₇Na | m/z théorique : 505.2202 |
| | | m/z mesurée : 505.2201 |

### ➢ 3,4-Di-O-benzyl-2-O-lévulinoyl-α/β-L-rhamnopyranose 60 :

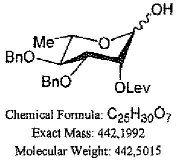

Le (1,5-cyclooctadiènebis(méthyldiphénylphosphine)iridium(I)hexafluorophosphate) (120 mg) est dissous dans du THF (60 mL) sous agitation. Le milieu subit 5 cycles dégazage vide/argon, 5 cycles dégazage vide/hyrdogène puis est mis sous hydrogène pendant 15 min. Le milieu subit alors une seconde fois 5 cycles dégazage vide/argon. La solution de glycoside d'allyle **59** (2,7 g, 5,7 mmol) dans du THF (8 mL) est transféré sur la solution de catalyseur activé. Le suivi par CCM (Chex/EtOAc, 7/3) montre la disparition de **59** (Rf = 0,4) et l'apparition d'un produit un peu moins polaire (Rf = 0,5).

Du diiode (2,9 g, 11,3 mmol, 2 éq.) en solution dans un mélange THF/H₂O (8/2, 30 mL) est ajouté au milieu réactionnel. Le suivi par CCM (Chex/EtOAc, 7/3 et DCM/MeOH, 98/2) indique la disparition de l'intermédiaire (Rf = 0,5 et 0,8, respectivement) et l'apparition d'un nouveau composé beaucoup plus polaire (Rf = 0,05 et 0,25, respectivement). Pour stopper la réaction, une solution aqueuse de NaHSO₃ 10% (20 mL) est ajoutée par fractions au milieu jusqu'à disparition de la coloration noire. Le milieu réactionnel est repris avec du DCM (50 mL) et la phase aqueuse est extraite au DCM (3 x 30 mL). Les phases organiques sont regroupées et lavées avec une solution de NaClₛₐₜ (3 x 30 mL), avec de l'H₂O (3 x 30 mL) puis séchées sur filtre séparateur de phases et concentrées à l'évaporateur rotatif. L'huile jaune obtenue est purifiée par chromatographie sur gel de silice (DCM/MeOH, 99/1→9/1) pour obtenir un mélange d'anomères α/β dans les proportions 75/25 de l'hémiacétal **60** sous forme d'une huile jaune (2,4 g, 96%).
Rf = 0,25 (DCM/MeOH, 98/2)
**60**_{α}: RMN ¹H (CDCl₃), *δ* 7.37-7.28 (m, 10H, CH_{Ph}), 5.40 (m, 1H, H-2), 5.13 (m, 1H, H-1), 4.95 (d, 1H, *J* = 10.9 Hz, H_{Bn}), 4.75-4.51 (m, 2H, H_{Bn}), 4.20 (d, 1H, *J* = 10.7 Hz, H_{Bn}), 4.03-3.98 (m, 2H, H-3, H-5), 3.43 (pt, 1H, *J*_{3,4} = 9.4 Hz, H-4), 2.73 (m, 4H, 2CH_{2Lev}), 2.18 (s, 3H, CH_{3Lev}), 1.33 (d, 3H, *J*_{5,6}= 6.2 Hz, H-6).
RMN ¹³C (CDCl₃), *δ* 206.8 (C_{Lev}), 172.5 (C_{Lev}), 138.9-138.0 (C_{Ph}), 128.8-128.0 (CH_{Ph}), 92.7 (C-1, ¹*J*_{CH} = 170.3 Hz), 80.5 (C-4), 77.9 (C-3), 75.7, 72.0 (2C, C_{Bn}), 70.0 (C-2), 68.1 (C-5), 38.5 (CH_{2Lev}), 30.1 (CH_{3Lev}), 28.6 (CH_{2Lev}), 18.4 (C-6).
**60**_{β} : RMN ¹H (CDCl₃), *δ* 737-7.28 (m, 10H, CH_{Ph}), 5.56 (m, 1H, H-2), 4.95 (d, 1H, *J* = 10.9 Hz, H_{Bn}), 4.77 (d, 1H, H-1), 4.75-4.51 (m, 2H, H_{Bn}), 4.20 (d, 1H, *J* = 10.7 Hz, H_{Bn}), 3.66 (dd, 1H, *J*_{2,3} = 3.3 Hz, *J*_{3,4} = 8.9 Hz, H-3), 3.38 (m, 1H, H-5), 3.21 (m, 1H, H-4), 2.73 (m, 4H, 2CH_{2Lev}), 2.20 (s, 3H, CH_{3Lev}), 1.38 (d, 3H, *J*_{5,6} = 5.9 Hz, H-6).
RMN ¹³C (CDCl₃), *δ* 208.2 (C_{Lev}), 173.0 (C_{Lev}), 138.9-138.0 (C_{Ph}), 128.8-128.0 (CH_{Ph}), 93.5 (C-1β, ¹*J*_{CH} = 158.8 Hz), 80.4 (C-3), 79.9 (C-4) 75.8, 71.9 (2C, C_{Bn}), 72.1 (C-5), 70.5 (C-2), 39.0 (CH_{2Lev}), 30.2 (CH_{3Lev}), 28.8 (CH_{2Lev}), 18.4 (C-6).

| | | |
|---|---|---|
| HRMS (ESI⁺) : | [M+Na]⁺ C₂₅H₃₀O₇Na | m/z théorique : 465.1889 |
| | | m/z mesurée : 465.1876 |

### ➢ Trichloracétimidate de 3,4-di-O-benzyl-2-O-lévulinoyl-α-L-rhamnopyranose 49 :

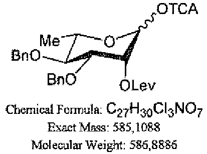

L'hémiacétal **60** (2,1 g, 4,8 mmol) est solubilisé dans du DCE (15 mL) et agité sous argon à -5°C puis de la DBU (202 µL, 1,3 mmol, 0,28 éq.) et du trichloroacétonitrile (2,4 mL, 24,2 mmol, 5 éq.) sont ajoutés. Le milieu réactionnel est agité à cette température pendant 15 min puis il est déposé, sans aucun traitement, sur une colonne de gel de silice (flash chromatographie, Chex/EtOAc+5% NEt₃, 7/3→1/1) pour obtenir le trichloroacétimidate **49** sous forme d'une huile jaune (2,6 g, 92%).
Rf = 0,4 (Chex/EtOAc, 7/3).
RMN ¹H (CDCl₃), *δ*8.68 (s, 1H, NH), 7.37-7.28 (m, 10H, CH_{Ph}), 6.20 (d, 1H, *J*_{1,2} = 1.9 Hz, H-1), 5.50 (m, 1H, H-2), 4.97 (d, 1H, *J=* 10.8 Hz, H_{Bn}), 4.74 (d, 1H, *J* = 11.3 Hz, H_{Bn}), 4.67 (d, 1H, *J* = 10.8 Hz, H_{Bn}), 4.58 (d, 1H, *J* = 11.3 Hz, H_{Bn}), 4.03-3.94 (m, 2H, H-3, H-5), 3.53 (pt, 1H, *J*_{4,5} = 9.5 Hz, H-4), 2.78 (m, 4H, 2CH_{2Lev}), 2.19 (s, 3H, CH_{3Lev}), 1.37 (d, 3H, *J*_{5,6}= 6.2 Hz, H-6).
RMN ¹³C (CDCl₃), *δ*206.4 (C_{Lev}), 172.2 (C_{Lev}), 160.7 (C=NH), 138.5-138.0 (C_{Ph}), 128.8-128.2 (CH_{Ph}), 95.5 (C-1, ¹*J*_{CH} = 178.6 Hz), 91.3 (CCl₃), 79.7 (C-4), 77.6 (C-3), 76.0, 72.3 (2C, C_{Bn}), 71.1 (C-5), 68.2 (C-2), 38.4 (CH_{2Lev}), 30.2 (CH_{3Lev}), 28.5 (CH_{2Lev}), 18.4 (C-6).

### ➢ Allyl (3,4-di-O-benzyl-2-O-lévulinoyl-α-L-rhamnopyranosyl)-(1→3)-2-O-acétyl-4-O-benzyl-α-L-rhamnopyranoside 61 :

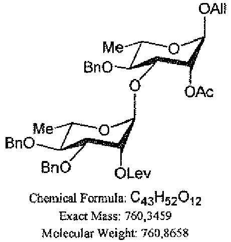

Du TMSOTf (1,1 mL, 6,0 mmol, 0,3 éq.) est additionné à une solution d'accepteur **31** (6,8 g, 20,2 mmol) et de donneur **49** (14,2 g, 24,2 mmol, 1,2 éq.) dans du Tol (250 mL), en présence de tamis moléculaire 4 Å (17 g), agitée sous argon à -78°C. Après 1 h à cette température, le bain éthanol-carboglace est retiré et l'agitation est poursuivie pendant 30 min, temps au bout duquel le suivi par CCM (Tol/EtOAc, 8/2, Chex/EtOAc, 6/4) indique la disparition de **31** (Rf = 0,3 et 0,4, respectivement) et l'apparition d'un nouveau composé (Rf = 0,45 et 0,5, respectivement). La réaction est stoppée par addition de triéthylamine (1 mL) puis le milieu réactionnel est filtré et concentré à l'évaporateur rotatif. L'huile jaune obtenue est purifiée par chromatographie sur gel de silice (Tol/EtOAc, 95/5→85/15) pour obtenir le glycoside d'allyle **61** sous forme d'une huile incolore (13,3 g, 89%).
Rf = 0,5 (Chex/EtOAc, 6/4).
RMN ¹H (CDCl₃), *δ*7.39-7.27 (m, 15H, CH_{Ph}), 5.89 (m, 1H, CH=), 5.46 (m, 1H, H-2_{B}), 5.29 (m, 1H, *J*ₜᵣₐₙₛ = 17.2 Hz, =CH₂), 5.21 (m, 1H, *J*_{cis}= 10.4 Hz, =CH₂), 5.17 (m, 1H, H-2_{C}), 5.05 (d, 1H, *J*_{1,2} = 1.5 Hz, H-1_{B}), 4.91 (d, 1H, *J* = 11.0 Hz, H_{Bn}), 4.87 (d, 1H, *J* = 10.9 Hz, H_{Bn}), 4.80 (d, 1H, *J*_{1,2} = 1.5 Hz, H-1_{C}), 4.67-4.60 (d, 3H, H_{Bn}), 4.45 (d, 1H, *J=* 11.3 Hz, H_{Bn}), 4.20-4.13 (m, 2H, H-3_{C}, H_{All}), 3.98 (m, 1H, H_{All}), 3.90 (dd, 1H, *J*_{2,3} = 3.3 Hz, *J*_{3,4} = 9.3 Hz, H-3_{B}), 3.83-3.74 (m, 2H, H-5_{B}, H-5_{C}), 3.47 (pt, 1H, *J*_{3,4} = 9.5 Hz, H-4_{C}), 3.43 (pt, 1H, *J*_{3,4} = 9.4 Hz, H-4_{B}), 2.71 (m, 4H, 2CH_{2Lev}), 2.18 (s, 3H, CH_{3Lev}), 2.14 (s, 3H, H_{Ac}), 1.31 (m, 6H, H-6_{C}, H-6_{B}).
RMN ¹³C (CDCl₃), *δ*206.4 (C_{Lev}), 172.2 (C_{Lev}), 170.6 (C_{Ac}), 138.9-138.4 (C_{Ph}), 133.9 (CH=), 128.9-128.0 (CH_{Ph}), 117.8 (=CH₂), 100.0 (C-1_{B}, ¹*J*_{CH} = 169.5 Hz), 96.6 (C-1_{C}, ¹J_{CH} = 170.0 Hz), 80.7 (C-4_{C}80.2 (C-4_{B}), 78.1 (C-3_{C}), 78.0 (C-3_{B}), 75.9, 75.6 (2C, C_{Bn}), 72.7 (C-2_{C}), 71.9 (C_{Bn}), 69.7 (C-2_{B}), 69.0 (C-5_{B}), 68.6 (C_{All}), 68.2 (C-5_{C}), 38.4 (CH_{2Lev}), 30.2 (CH_{3Lev}), 28.6 (CH_{2Lev}), 21.4 (C_{Ac}), 18.3(2C, C-6_{B}, C-6_{C}).

| | | |
|---|---|---|
| HRMS (ESI⁺) : | [M+Na]⁺ C₄₃H₅₂O₁₂Na | m/z théorique : 783.3356 |
| | | m/z mesurée : 783.3364 |
| | [M+NH₄]⁺ C₄₃H₅₂O₁₂NH₄ | m/z théorique : 778.3802 |
| | | m/z mesurée : 778.3829 |

### ➢ (3,4-Di-O-benzyl-2-O-lévulinoyl-α-L-rhamnopyranosyl)-(1→3)-2-O-acétyl-4-O-benzyl-α/β-L-rhamnopyranose 62 :

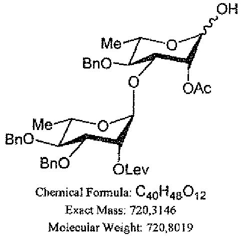

Le (1,5-cyclooctadiènebis(méthyldiphénylphosphine)iridium(I)hexafluorophosphate) (175 mg) est dissous dans du THF (90 mL) sous agitation. Le milieu subit 5 cycles dégazage vide/argon, 5 cycles dégazage vide/hyrdogène puis est mis sous hydrogène pendant 15 min. Le milieu subit alors une seconde fois 5 cycles dégazage vide/argon. La solution de glycoside d'allyle **61** (8,0 g, 10,6 mmol) dans du THF (15 mL) est transféré sur la solution de catalyseur activé. Le suivi par CCM (Tol/EtOAc, 8/2) montre la disparition du produit de départ (Rf= 0,45) et l'apparition d'un produit un peu moins polaire (Rf= 0,5).

Du diiode (5,4 g, 21,2 mmol, 2 éq.) en solution dans un mélange THF/H₂O (8/2, 30 mL) est ajouté au milieu réactionnel. Le suivi par CCM (Tol/EtOAc, 8/2 et DCM/MeOH, 98/2) indique la disparition de l'intermédiaire (Rf= 0,5 et 0,95, respectivement) et l'apparition d'un nouveau composé beaucoup plus polaire (Rf= 0,05 et 0,45, respectivement). Pour stopper la réaction, une solution aqueuse de NaHSO₃ 10% (25 mL) est ajoutée par fractions au milieu jusqu'à disparition de la coloration noire. Le milieu réactionnel est repris avec du DCM (100 mL) et la phase aqueuse est extraite au DCM (3 x 100 mL). Les phases organiques sont regroupées et lavées avec une solution de NaClₛₐₜ (3 x 50 mL), avec de l'H₂O (3 x 50 mL) puis séchées sur filtre séparateur de phases et concentrées à l'évaporateur rotatif. L'huile jaune obtenue est purifiée par chromatographie sur gel de silice (DCM/MeOH, 99/1→9/1) pour obtenir majoritairement l'hémiacétal **62** sous forme d'une huile jaune (7,1 g, 93%).
Rf = 0,45 (DCM/MeOH, 98/2).
RMN ¹H (CDCl₃), *δ*7.39-7.28 (m, 15H, CH_{Ph}), 5.44 (dd, 1H, *J*_{1,2} = 1.9 Hz, *J*_{2,3} = 3.2 Hz, H-2_{B}), 5.16 (dd, 1H, *J*_{1,2} = 1.8 Hz, *J*_{2,3} = 3.1 Hz, H-2_{C}), 5.12 (d, 1H, *J*_{1,2} = 1.5 Hz, H-1_{C}), 5.05 (d, 1H, *J*_{1,2} = 1.5 Hz, H-1_{B}), 4.92 (d, 1H, *J* = 11.0 Hz, H_{Bn}), 4.83 (d, 1H, *J* = 10.9 Hz, H_{Bn}), 4.67-4.60 (m, 3H, H_{Bn}), 4.45 (d, 1H, *J* = 11.3 Hz, H_{Bn}), 4.20 (dd, 1H, *J*_{2,3} = 3.3 Hz, *J*_{3,4} = 9.5 Hz, H-3_{C}), 3.98 (dq, 1H, H-5_{C}), 3.89 (dd, 1H, *J*_{2,3} = 3.3 Hz, *J*_{3,4} = 9.3 Hz, H-3_{B}), 3.83 (dq, 1H, H-5_{B}), 3.77 (m, 1H, OH), 3.47 (pt, 1H, *J*_{3,4}= 9.5 Hz, H-4_{C}), 3.43 (pt, 1H, J_{3,4} = 9.3 Hz, H-4_{B}), 2.70 (m, 4H, 2CH_{2Lev}), 2.18 (s, 3H, CH_{3Lev}), 2.14 (s, 3H, H_{Ac}), 1.29 (d, 3H, *J*_{5,6} = 6.3 Hz, H-6_{B}), 1.30 (d, 3H, *J*_{5,6}= 6.2 Hz, H-6_{C}). RMN ¹³C (CDCl₃), *δ*206.8 (C_{Lev}), 172.6 (C_{Lev}), 171.6 (C_{Ac}), 138.8-138.4 (C_{Ph}), 128.9-128.1 (CH_{Ph}), 99.9 (C-1_{B}, ¹*J*_{CH}= 173.3 Hz), 92.0 (C-1_{C}, ¹*J*_{CH} = 170.0 Hz), 80.7 (C-4_{C}), 80.2 (C-4_{B}), 78.0 (C-3_{B}), 77.7 (C-3_{C}), 75.8, 75.6 (2C, C_{Bn}), 73.2 (C-2_{C}), 72.0 (C_{Bn}), 69.7 (C-2_{B}), 69.0 (C-5_{B}), 68.0 (C-5_{C}), 38.4 (CH_{2Lev}), 30.2 (CH_{3Lev}), 28.5 (CH_{2Lev}), 21.5 (C_{Ac}), 18.5, 18.4 (C-6B*, C-6_{C}*).

| | | |
|---|---|---|
| HRMS (ESI⁺) : | [M+Na]⁺ C₄₀H₄₈O₁₂Na | m/z théorique : 743.3043 |
| | | m/z mesurée : 743.3173 |
| | [M+NH₄]⁺ C₄₃H₅₂O₁₂NH₄ | m/z théorique : 738.3489 |
| | | m/z mesurée : 738.3627 |

### ➢ Trichloroacétimidate de (3,4-di-O-benzyl-2-O-lévulinoyl-α-L-rhamnopyranosyl)-(1→3)-2-O-acétyl-4-O-benzyl-α/β-L-rhamnopyranose 47 :

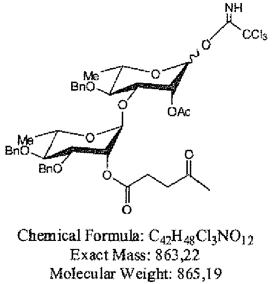

L'hémiacétal **62** (8,7 g, 12,1 mmol) est solubilisé dans du DCE (50 mL) et agité sous argon à -5°C puis de la DBU (508 µL, 3,3 mmol, 0,28 éq.) et du trichloroacétonitrile (6,1 mL, 60,7 mmol, 5 éq.) sont ajoutés. Le milieu réactionnel est agité à cette température pendant 15 min puis il est déposé, sans aucun traitement, sur une colonne de gel de silice (flash chromatographie, Chex/EtOAc+5% NEt₃, 7/3→1/1) pour obtenir le trichloroacétimidate **47** sous forme d'une huile jaune (8,7 g, 84%).
Rf = 0,5 (Chex/EtOAc, 7/3).
RMN ¹H (CDCl₃), *δ*8.72 (s, 1H, NH), 7.42-7.29 (m, 15H, CH_{Ph}), 6.22 (d, 1H, *J*_{1,2} = 1.9 Hz, H-1_{C}), 5.48 (dd, 1H, *J*_{1,2} = 1.8 Hz, *J*_{2,3} = 3.2 Hz, H-2_{B}), 5.31 (dd, 1H, *J*_{1,2} = 2.1 Hz, *J*_{2,3} = 3.1 Hz, H-2_{C}), 5.10 (d, 1H, *J*_{1,2} = 1.4 Hz, H-1_{B}), 4.92 (d, 1H, *J* = 11.0 Hz, H_{Bn}), 4.85 (d, 1H, *J* = 10.8 Hz, H_{Bn}), 4.68-4.62 (m, 3H, H_{Bn}), 4.51 (d, 1H*, J* = 11.3 Hz, H_{Bn}), 4.26 (dd, 1H, *J*_{2,3} = 3.3 Hz, *J*_{3,4} = 9.5 Hz, H-3_{C}), 3.96 (dq, 1H, H-5_{C}), 3.89 (dd, 1H, *J*_{2,3} = 3.3 Hz, *J*_{3,4} = 9.3 Hz, H-3_{B}), 3.82 (dq, 1H, H-5_{B}), 3.58 (pt, 1H, *J*_{3,4} = 9.5 Hz, H-4_{C}), 3.44 (pt, 1H, *J*_{3,4} = 9.4 Hz, H-4_{B}), 2.72 (m, 4H, 2CH_{2Lev}), 2.18 (s, 3H, CH_{3Lev}), 2.17 (s, 3H, H_{Ac}), 1.36 (d, 3H, *J*_{5,6} = 6.2 Hz, H-6_{C}), 1.29 (d, 3H, *J*_{5,6} = 6.2 Hz, H-6_{B}). RMN ¹³C (CDCl₃), *δ*206.5 (C_{Lev}), 172.3 (C_{Lev}), 170.3 (C_{Ac}), 160.5 (C=NH), 138.8-138.0 (C_{Ph}), 129.2-127.9 (CH_{Ph}), 99.9 (C-1_{B}, ¹*J*_{CH} = 137.3 Hz), 94.5 (C-1_{C}, ¹*J*_{CH} = 177.8 Hz), 91.2 (CCl₃), 80.2 (C-4_{C}), 80.1 (C-4_{B}), 77.9 (C-3_{B}), 76.5 (C-3_{C}), 76.1, 75.7, 72.0 (3C, C_{Bn}), 71.2 (C-2_{C}), 71.1 (C-5_{C}), 69.6 (C-2_{B}), 69.1 (C-5_{B}), 38.4 (CH_{2Lev}), 30.2 (CH_{3Lev}), 28.5 (CH_{2Lev}), 21.3 (C_{Ac}), 18.4, 18.3 (C-6_{B}* , C-6_{C}*).

### ➢ Allyl (3,4-di-O-benzyl-2-O-lévulinoyl-α-L-rhamnopyranosyl)-(1→3)-(2-O-cétyl-4-O-benzyl-α-L-rhamnopyranosyl)-(1→3)-(2-désoxy-4,6-O-isopropylidène-2-trichloroacétamido-β-D-glucopyranosyl)-(1→2)-[2,3,4,6-tétra-O-benzyl-α-D-glucopyranosyl-(1→3)]-4-O-benzyl-α-L-rhamnopyranoside 64 :

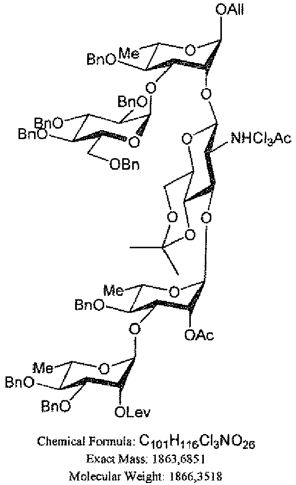

Du TMSOTf (49,0 µL, 270 µmol, 0,3 éq.) est additionné à une solution d'accepteur **38** (1,1 g, 0,9 mmol) et de donneur **47** (1,2 g, 1,4 mmol, 1,5 éq.) dans du Tol (30 mL), en présence de tamis moléculaire 4 Å (1,1 g), agitée sous argon à -40°C. Après 15 min sous agitation, le suivi par CCM (Tol/EtOAc, 8/2) indique la disparition de **38** (Rf = 0,25) et l'apparition d'un nouveau composé plus polaire (Rf= 0,35). La réaction est stoppée par l'addition de triéthylamine (0,1 mL) puis le milieu réactionnel est filtré et concentré à l'évaporateur rotatif. L'huile jaune obtenue est purifiée par chromatographie sur gel de silice (Tol/EtOAc, 9/1→7/3) pour obtenir le glycoside d'allyle **64** sous forme d'un solide blanc (1,4 g, 75%).
Rf= 0,35 (Tol/EtOAc, 8/2).
RMN ¹H (CDCl₃), *δ*7.51-7.13 (m, 40H, CH_{Ph}), 6.95 (d, 1H, *J*_{NH,2} = 8.0 Hz, NH), 5.94 (m, 1H, CH=), 5.48 (dd, 1H, *J*_{1,2} = 1.1 Hz, *J*_{2,3} = 3.0 Hz, H-2_{B}), 5.33 (m, 1H, *J*ₜᵣₐₙₛ = 17.2 Hz, =CH₂), 5.24 (m, 1H, *J*_{cis} = 10.4 Hz, =CH₂), 5.24 (d, 1H, *J*_{1,2} = 3.5 Hz, H-1_{E}), 5.16 (m, 2H, H_{Bn}), 5.12 (dd, 1H, *J*_{2,3} = 2,8 Hz, H-2_{C}), 5.11 (d, 1H, *J*_{1,2} = 1.3 Hz, H-1_{B}), 5.07 (d, 1H, *J* = 12.2 Hz, H_{Bn}), 4.96 (m, 2H, H_{Bn}), 4.87 (d, 2H, *J=* 11.0 Hz, H_{Bn}), 4.84 (d, 1H, *J*_{1,2} = 1.7 Hz, H-1_{A}), 4.79 (d, 1H, *J*_{1,2} = 1.6 Hz, H-1_{C}), 4.77-4.54 (d, 8H, H_{Bn}, H-1_{d}), 4.46 (d, 1H, *J* = 11.3 Hz, H_{Bn}), 4.36 (d, 1H, *J* = 12.0 Hz, H_{Bn}), 4.24-4.15 (m, 5H, H-3_{E}, H-3_{C}, H_{All}, H-3_{A}, H-5_{E}), 4.09-3.98 (m, 5H, H-2_{D}, H-2_{A}, H-5_{C}, H_{All}, H-5_{B}), 3.95 (dd, 1H, *J*_{2,3} = 3.2 Hz, *J*_{3,4} = 9.2 Hz, H-3_{B}), 3.91-3.85 (m, 3H, H-6a_{D}, H-2_{E}, H-4_{E}), 3.78-3.73 (m, 2H, H-5_{A}, H-6b_{D}), 3.57 (pt, 1H, *J*_{3,4} = 9.2 Hz, H-4_{D}), 3.52 (pt, 1H, *J*_{3,4} = 9.6 Hz, H-4_{A}), 3.50-3.44 (m, 4H, H-6a_{E}, H-6b_{E}, H-4_{C}, H-4_{B}), 2.87 (m, 1H, H-5_{D}), 2.77 (m, 5H, H-3_{D}, 2CH_{2Lev}), 2.21 (s, 3H, CH_{3Lev}), 2.16 (s, 3H, H_{Ac}), 1.52 (s, 3H, H_{iPr}), 1.50 (s, 3H, H_{iPr}), 1.46 (d, 3H, *J*_{5,6} = 6.2 Hz, H-6_{A}), 1.40 (d, 3H, *J*_{5,6} = 6.2 Hz, H-6_{B}), 1.31 (d, 3H, *J*_{5,6} = 6.2 Hz, H-6_{C}).
RMN ¹³C (CDCl₃), *δ*206.5 (C_{Lev}), 172.1 (C_{Lev}), 169.9 (C_{Ac}), 162.4 (C_{NTCA}), 139.1-138.8 (C_{Ph}), 134.4 (CH=), 129.5-127.8 (CH_{Ph}), 117.5 (=CH₂), 101.7 (C-1_{D}, ¹*J*_{CH} = 163.9 Hz), 99.9 (C_{iPr}), 99.4 (C-1_{B}, ¹*J*_{CH} = 174.7 Hz), 98.8 (C-1_{A}, ¹*J*_{CH} = 171.5 Hz), 98.1 (C-1_{C}, ¹*J*_{CH} = 167.6 Hz), 94.9 (C-1_{E}, ¹*J*_{CH} = 165.3 Hz), 93.5 (CCl₃), 83.5 (C-3_{E}), 80.9 (C-2_{E}), 80.8 (C-4_{B}), 80.3 (C-4_{C}80.1 (C-4_{A}), 79.2 (C-3_{D}), 79.1 (C-4_{E}), 78.0 (C-3_{B}),77.1 (C-3_{C}), 76.4, 75.8, 75.7, 75.4, 75.3, 75.2 (6C, C_{Bn}), 75.1 (C-3_{A}), 74.2 (C-2_{A}), 73.8 (C_{Bn}), 73.1 (C-4_{D}), 72.8 (C-2_{C}), 71.9 (C_{Bn}), 70.3 (C-5_{E}), 69.9 (C-2_{B}), 69.0 (C-5_{B}), 68.8 (C-5_{A}), 68.4 (C-6_{E}), 68.3 (C-5_{C}), 68.2 (C_{All}), 67.4 (C-5_{D}), 62.5 (C-6_{D}), 57.8 (C-2_{D}), 38.6 (CH_{2Lev}), 30.3 (CH_{3Lev}), 29.6 (C_{iPr}), 28.7 (CH_{2Lev}), 21.5 (C_{Ac}), 19.4 (C_{iPr}), 18.5 (C-6_{C}), 18.4 (C-6_{A}), 18.3 (C-6_{B}).

| | | |
|---|---|---|
| HRMS (ESI⁺) : | [M+Na]⁺ C₁₀₁H₁₁₆³⁵Cl₃NO₂₆Na | m/z théorique : 1886.6749 |
| | | m/z mesurée : 1886.6445 |
| | [M+NH₄]⁺ C₈₈H₁₀₄³⁵Cl₃NO₂₇NH₄ | m/z théorique : 1881.7195 |
| | | m/z mesurée : 1881.6897 |

### ➢ Allyl (2-désoxy-4,6-O-isopropylidène-2-trichloroacétamido-3-triméthylsillyl-β-D-glucopyranosyl)-(1→2)-[2,3,4,6-tétra-O-benzyl-α-D-glucopyranosyl-(1→3)]-4-O-benzyl-α-L-rhamnopyranoside 63 :

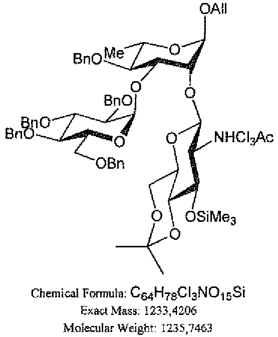

Du TMSOTf (49,0 µL, 270 µmol, 0,3 éq.) est additionné à une solution d'accepteur **38** (2,0 g, 1,7 mmol) et de donneur **47** (2,3 g, 2,6 mmol, 1,5 éq.) dans du Tol (30 mL), en présence de tamis moléculaire 4 Å (1,1 g), agitée sous argon à -78°C. Après 15 min sous agitation, le suivi par CCM (Tol/EtOAc, 8/2) indique la disparition de **38** (Rf = 0,25) et l'apparition d'un nouveau composé plus polaire (Rf= 0,35). La réaction est stoppée par l'addition de triéthylamine (0,1 mL) puis le milieu réactionnel est filtré et concentré à l'évaporateur rotatif.

L'huile jaune obtenue est purifiée par chromatographie sur gel de silice (Tol/AcOEt : 9/1→7/3) pour obtenir par ordre d'élution le composé silylé **63** (233 mg, 11%) puis le glycoside d'allyle **64** (1,4 g, 66%).
Rf= 0,5 (Tol/EtOAc, 8/2).
RMN ¹H (CDCl₃), *δ*7.47-7.08 (m, 26H, NH, CH_{Ph}), 5.89 (m, 1H, CH=), 5.32 (m, 1H, *J*ₜᵣₐₙₛ = 17.2 Hz, =CH₂), 5.23 (m, 1H, *J*_{cis}= 10.6 Hz, =CH₂), 5.12 (d, 1H, *J*_{1,2} = 3.6 Hz, H-1_{E}), 5.11-5.05 (m, 3H, H_{Bn}), 4.93 (d, 1H, *J* = 12.8 Hz, H_{Bn}), 4.86 (d, 1H, *J*_{1,2} = 1.6 Hz, H-1_{A}), 4.83-4.76 (m, 3H, H_{Bn}, H-1_{D}, H_{Bn}), 4.61-4.56 (m, 2H, H_{Bn}), 4.50 (d, 1H, *J* = 11.0 Hz, H_{Bn}), 4.32 (d, 1H, *J*=12.0 Hz, H_{Bn}), 4.22-4.10 (m, 4H, H-3_{E}, H_{All}, H-3_{A}, H-5_{E}), 4.08-3.97 (m, 3H, H-2_{A}, H-2_{D}, H_{All}), 3.90 (dd, 1H, *J*_{5,6a} = 5.3 Hz, *J*_{6a,6b} = 10.7 Hz, H-6a_{D}), 3.83-3.72 (m, 4H, H-2_{E}, H-4_{E}, H-6b_{D}, H-5_{A}), 3.55 (pt, 1H, *J*_{3,4} = 9.1 Hz, H-4_{D}), 3.54 (pt, 1H, *J*_{3,4} = 9.5 Hz, H-4_{A}), 3.43 (m, 2H, H-6a_{E}, H-6b_{E}), 3.36 (pt, 1H, *J*_{2,3} = 9.2 Hz, H-3_{D}), 2.94 (m, 1H, H-5_{D}), 1.51 (s, 3H, H_{iPr}), 1.45 (s, 3H, H_{iPr}), 1.44 (d, 3H, *J*_{5,6} = 6.2 Hz, H-6_{A}), 0.13 (s, 9H, H_{Si}).
RMN ¹³C (CDCl₃), *δ*161.7 (C_{NTCA}), 138.7-137.7 (C_{Ph}), 134.0 (CH=), 129.1-127.3 (CH_{Ph}), 117.1 (=CH₂), 101.5 (C-1_{D}, ¹*J*_{CH} = 163.6 Hz), 99.4 (C_{iPr}), 98.5 (C-1_{A}, *¹J*_{CH} = 174.0 Hz), 95.2 (C-1_{E}, ¹*J*_{CH} = 167.5 Hz), 93.3 (CCl₃), 83.3 (C-3_{E}), 79.8 (C-4_{A}), 78.7, 78.6 (2C, C-2_{E}*, C-4_{E}*), 75.9 (C_{Bn}), 75.4 (C-3_{A}), 75.3, 74.9 (2C, C_{Bn}), 74.3 (C-3_{D}), 74.3 (C_{Bn}), 74.2 (C-4_{D}), 74.1 (C-2_{A}), 73.4 (C_{Bn}), 70.0 (C-5_{E}), 68.5 (C-5_{A}), 67.9 (C-6_{E}), 67.8 (C_{All}), 67.2 (C-5_{D}), 62.1 (C-6_{D}), 58.6 (C-2_{D}), 29.1 (C_{iPr}), 19.0 (C_{iPr}), 18.0 (C-6_{A}), 0.7 (C_{Si}).

| | | |
|---|---|---|
| HRMS (ESI⁺) : | [M+Na]⁺ C₆₄H₇₈NO₁₅³⁵Cl₃SiNa | m/z théorique : 1256.4104 |
| | | m/z mesurée: 1256.4188 |
| | [M+NH₄]⁺ C₆₄H₇₈NO₁₅³⁵Cl₃SiNH₄ | m/z théorique : 1251.4550 |
| | | m/z mesurée : 1251.4548 |

### ➢ Allyl (3,4-di-O-benzyl-α-L-rhamnopyranosyl)-(1→3)-(2-O-acétyl-4-O-benzyl-α-L-rhamnopyranosyl)-(1→3)-(2-désoxy-4,6-O-isopropylidene-2-trichloroacétamido-β-D-glucopyranosyl)-(1→2)-(2,3,4,6-tétra-O-benzyl-α-D-glucopyranosyl-(1→3)]-4-O-benzyl-α-L-rhamnopyranoside 66 :

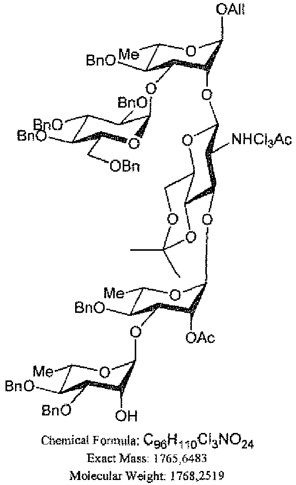

Le glycoside d'allyle **64** (1,2 g, 570 µmol) est dissous dans un mélange pyridine/acide acétique (3/2, 10 mL). De l'hydrazine monohydraté (280 µL, 5,7 mmol, 10 éq.) est ajouté goutte à goutte au milieu réactionnel. Après 25 min d'agitation à 0°C, le suivi par CCM (Tol/EtOAc, 7/3) indique la disparition de **64** (Rf = 0, 5) et l'apparition d'un produit plus polaire (Rf= 0,35). Le milieu réactionnel est alors repris à l'eau froide (10 mL) et la phase aqueuse est extraite rapidement au DCM (3 x 50 mL). Les phases organiques sont regroupées et lavées avec une solution de NaClₛₐₜ, filtrées sur filtre séparateur de phase et concentrées à l'évaporateur rotatif. L'huile obtenue est purifiée par chromatographie sur gel de silice (Tol/EtOAc, 9/1→7/3) pour obtenir l'alcool **66** sous forme d'un solide blanc (950 mg, 94%).
Rf = 0,35 (Tol/EtOAc, 7/3).
RMN ¹H (CDCl₃), *δ*7.48-7.06 (m, 40H, CH_{Ph}), 6.87 (d, 1H, *J*_{NH,2} = 8.9 Hz, NH), 5.90 (m, 1H, CH=), 5.29 (m, 1H, *J*ₜᵣₐₙₛ = 17.2 Hz, =CH₂), 5.24 (m, 1H, *J*_{cis} = 11.5 Hz, =CH₂), 5.16 (d, 1H, *J*_{1,2} = 3.6 Hz, H-1_{E}), 5.10-5.07 (m, 4H, H_{Bn}, H-1_{B}, H-2_{C}, H_{Bn}), 5.02 (d, 1H, *J* = 12.1 Hz, H_{Bn}), 4.89 (m, 2H, H_{Bn}), 4.81 (d, 1H, *J* = 11.0 Hz, H_{Bn}), 4.78 (d, 1H, *J*_{1,2} = 1.7 Hz, H-1_{A}), 4.76 (d, 1H, *J*_{1,2} = 1.7 Hz, H-1_{C}), 4.75-4.49 (m, 10H, 9H_{Bn}, H-1_{D}), 4.32 (d, 1H, *J=* 11.9 Hz, H_{Bn}), 4.19-4.10 (m, 5H, H-3_{E}, H_{All}, H-3_{A}, H-3_{C}, H-5_{E}), 4.04-3.93 (m, 5H, H-2_{D}, H-2_{A}, H_{All}, H-5_{C}, H-2_{B}), 3.90-3.82 (m, 4H, H-5_{B}, H-6a_{D}, H-2_{E}, H-4_{E}), 3.77 (dd, 1H, *J*_{2,3} = 3.2 Hz, *J*_{3,4} = 9.1 Hz, H-3_{B}), 3.74-3.67 (m, 2H, H-6b_{D}, H-5_{A}), 3.54 (pt, 1H, *J*_{3,4} = 9.2 Hz, H-4_{D}), 3.51-3.44 (m, 4H, H-4_{A}, H-4_{B}, H-6a_{E}, H-6b_{E}) 3.40 (pt, 1H, *J*_{3,4} = 9.4 Hz, H-4_{C}), 2.81 (m, 1H, H-5_{D}), 2.68 (pt, 1H, *J*_{2,3} = 9.2 Hz, H-3_{D}), 2.13 (s, 3H, H_{Ac}), 1.48 (s, 3H, H_{iPr}), 1.46 (s, 3H, H_{iPr}), 1.40 (d, 3H, *J*_{5,6} = 6.2 Hz, H-6_{A}), 1.33 (d, 3H, *J*_{5,6} = 6.2 Hz, H-6_{B}), 1.25 (d, 3H, *J*_{5,6} = 6.2 Hz, H-6_{C}). RMN ¹³C (CDCl₃), *δ*169.6 (C_{Ac}), 162.0 (C_{NTCA}), 138.6-137.6 (C_{Ph}), 133.9 (CH=), 129.4-127.4 (CH_{Ph}), 117.1 (=CH₂), 101.2 (C-1_{D}, ¹*J*_{CH}= 158.9 Hz), 100.7 (C-1_{B}, ¹*J*_{CH}= 172.6 Hz), 99.4 (C_{iPr}), 98.4 (C-1_{A}, ¹*J*_{CH} = 172.4 Hz), 97.4 (C-1_{C}, ¹*J*_{CH} = 170.7 Hz), 94.3 (C-1_{E}, ¹*J*_{CH}= 166.4 Hz), 93.0 (CCl₃), 83.1 (C-3_{E}), 80.4 (C-4_{C}80.3 (C-2_{E}), 79.9, 79.7 (2C, C-4_{A}*, C-4_{B}*), 79.6 (C-3_{B}), 78.7 (C-4_{E}), 78.5 (C-3_{D}), 77.0 (C-3_{C}), 76.0, 75.3, 75.4, 75.0, 74.9, 74.8 (6C, C_{Bn}), 74.5 (C-3_{A}), 73.7 (C-2_{A}), 73.3 (C_{Bn}), 72.6 (C-4_{D}), 72.4 (C-2_{C}), 71.8 (C_{Bn}), 69.9 (C-5_{E}), 69.0 (C-2_{B}), 68.4 (C-5_{A}), 68.2 (C-5_{B}), 68.0 (C-6_{E}), 67.8 (C-5_{C}), 67.7 (C_{All}), 66.9 (C-5_{D}), 62.1 (C-6_{D}), 57.3 (C-2_{D}), 29.2 (C_{iPr}), 21.1 (C_{Ac}), 19.0 (C_{iPr}), 18.1, 17.9, 17.8 (C-6_{A}*, C-6_{B}*, C-6_{C}*).

| | | |
|---|---|---|
| HRMS (ESI⁺) : | [M+Na]⁺ C₉₆H₁₁₀³⁵Cl₃NO₂₄Na | m/z théorique : 1788.6381 |
| | | m/z mesurée : 1788.6537 |
| | [M+H]⁺ C₉₆H₁₁₀³⁵Cl₃NO₂₄ | m/z théorique : 1766.6561 |
| | | m/z mesurée : 1766.6667 |

### ➢ Allyl (3,4-di-O-benzyl-α-L-rhamnopyranosyl)-(1→3)-(2-O-acétyl-4-O-benzyl-α-L-rhamnopyranosyl)-(1→3)-(2-désoxy-2-trichloroacétamido-β-D-glucopyranosyl)-(1→2)-[2,3,4,6-tétra-O-benzyl-α-D-glucopyranosyl-(1→3)1-4-O-benzyl-α-L-rhamnopyranoside 67 :

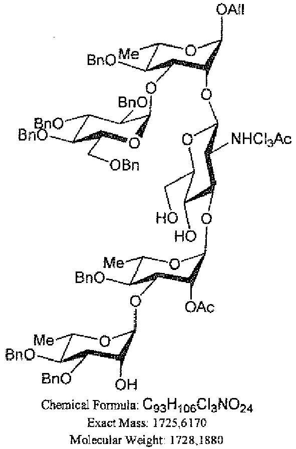

Une solution aqueuse de TFA 50% (4 mL) est additionnée lentement à une solution d'alcool **66** (367 mg, 210 µmol) dans du DCM (10 mL) à 0°C. Le milieu réactionnel est agité pendant 2 h à cette température, temps au bout duquel le suivi par CCM (DCM/MeOH, 95/5) indique la disparition totale du produit de départ (Rf = 0,85) et l'apparition d'un nouveau composé plus polaire (Rf = 0,3). Le milieu réactionnel est concentré par coévaporation avec du Tol (3 x 10 mL). L'huile jaune obtenue est purifiée par chromatographie sur gel de silice (Tol/EtOAc, 7/3→6/4) pour obtenir le triol **67** sous forme d'un solide blanc (300 mg, 84%).
Rf = 0,3 (DCM/MeOH, 95/5).
RMN ¹H (CDCl₃), *δ* 7.44-7.06 (m, 40H, CH_{Ph}), 7.02 (d, 1H, *J*_{NH,2} = 8.8 Hz, NH), 5.90 (m, 1H, CH=), 5.30 (m, 1H, *J*ₜᵣₐₙₛ = 18.7 Hz, =CH₂), 5.24-5.22 (m, 2H, =CH₂, H-1_{E}), 5.14 (dd, 1H, *J*_{1,2} = 2. 0 Hz, *J*_{2,3} = 2.9 Hz, H-2_{C}), 5.10 (d, 1H, *J*_{1,2} = 1.1 Hz, H-1_{B}), 5.10-5.05 (m, 2H, H_{Bn}), 4.96 (d, 1H, *J* = 12.7 Hz, H_{Bn}), 4.90 (d, 1H, *J* = 11.0 Hz, H_{Bn}), 4.84 (d, 1H, *J*_{1,2} = 1.6 Hz, H-1_{A}), 4.83-4.73 (m, 4H, H_{Bn}), 4.66 (d, 1H, *J* = 10.9 Hz, H_{Bn}), 4.51-4.50 (m, 8H, H_{Bn}, H-1_{C}, H_{Bn}, H-1_{D}, 4H_{Bn}), 4.34 (d, 1H, *J* = 12.0 Hz, H_{Bn}), 4.21 (bs, 1H, OH-4_{D}), 4.20-4.12 (m, 5H, H-3_{A}, H_{All}, H-3_{E}, H-3_{C}, H-5_{E}), 4.00-3.91 (m, 5H, H-2_{B}, H-2_{A}, H_{All}, H-2_{D}, H-5_{C}), 3.86-3.69 (m, 7H, H-6a_{D}, H-2_{E}, H-3_{B}, H-4_{E}, H-5_{B}, H-6b_{D}, H-5_{A}), 3.50-3.45 (m, 4H, H-6a_{E}, H-6b_{E}, H-4_{A}, H-4_{B}), 3.40 (pt, 1H, *J*_{3,4} = 9.5 Hz, H-4_{C}), 3.36 (pt, 1H, *J*_{3,4} = 9.2 Hz, H-4_{D}), 3.03 (m, 1H, H-5_{D}), 2.31 (bs, 1H, OH-4_{D}), 2.24 (pt, 1H, *J*_{2,3} = 9.8 Hz, H-3_{D}), 2.18 (s, 3H, H_{Ac}), 1.41 (d, 3H, *J*_{5,6} = 6.2 Hz, H-6_{A}), 1.30 (m, 3H, H-6_{C}), 1.26 (d, 3H, *J*_{5,6} = 6.2 Hz, H-6_{B}).
RMN ¹³C (CDCl₃), *δ* 170.0 (C_{Ac}), 162.2 (C_{NTCA}), 138.5-137.6 (C_{Ph}), 133.9 (CH=), 129.4-127.4 (CH_{Ph}), 117.3 (=CH₂), 101.0 (C-1_{D}, ¹*J*_{CH} = 160.2 Hz), 100.7 (C-1_{B}, ¹*J*_{CH} = 165.1 Hz), 98.2 (2C, C-1_{A}, C-1_{C}, ¹*J*_{CH} = 169.6 Hz), 94.3 (C-1_{E}, ¹*J*_{CH} = 166.7 Hz), 93.0 (CCl₃), 86.7 (C-3_{D}), 83.1 (C-3_{E}), 80.4 (C-2_{E}), 79.9, 79.7 (C-4_{A}*, C-4_{B}*), 79.6 (C-4_{E}), 79.2 (C-4_{C}), 78.7 (C-3_{B}), 77.9 (C-3_{C}), 76.2, 75.4, 75.3, 75.0 (6C, C_{Bn}), 74.6 (C-5_{D}), 74.3 (C-3_{A}), 74.2 (C-2_{A}), 73.4 (C_{Bn}), 72.0 (C-2_{C}), 71.6 (C_{Bn}), 70.8 (C-4_{D}), 69.9 (C-5_{E}), 69.1 (C-5_{C}), 68.7 (C-2_{B}), 68.5 (C-5_{A}), 68.4 (C-5_{B}), 68.0 (C-6_{E}), 67.7 (C_{All}), 62.9 (C-6_{D}), 55.1 (C-2_{D}), 21.1 (C_{Ac}), 18.0, 17.9, 17.8 (C-6_{A}*, C-6_{B}*, C-6_{C}*).

| | | |
|---|---|---|
| HRMS (ESI⁺) : | [M+Na]⁺ C₉₃H₁₀₆³⁵Cl₃NO₂₄Na | m/z théorique : 1748.6068 |
| | | m/z mesurée : 1748.6008 |

### ➢ Propyl α-L-rhamnopyranosyl-(1→3)-(2-O-acétyl-α-L-rhamnopyranosyl)-(1→3)-(2-acétamido-2-désoxy-β-D-glucopyranosyl)-(1→2)-[α-D-glucopyranosyl-(1→3)]-α-L-rhamnopyranoside V :

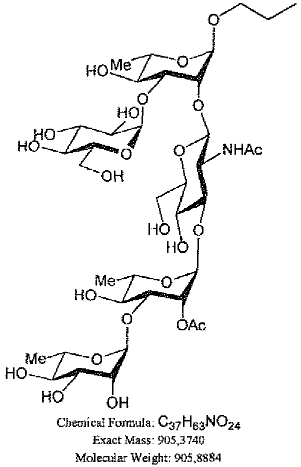

**Voie 1** : Du Pd-C 10% (280 mg) est ajouté à une solution dégazée de pentasaccharide **56** (270 mg, 180 µmol) dans de l'éthanol (20 mL). La suspension est saturée avec de l'hydrogène à 50 bars et agitée à TA pendant 10 j. Le suivi par CCM (iPrOH/H₂O/NH₃, 4/1/0,5 et DCM/MeOH, 95/5) montre la disparition de **56** (Rf = 1 et 0,3, respectivement) et l'apparition d'un nouveau composé plus polaire (Rf = 0,3 et 0, respectivement). Le milieu réactionnel est filtré sur un filtre Acrodisc LC 25 mm, et le filtrat est concentré à l'évaporateur rotatif. Le résidu obtenu est purifié sur colonne C-18 (H₂O/CH₃CN, 100/0**→**7/3) pour conduire à la cible ***V*** sous forme d'un solide blanc (111 mg, 70%).
**Voie 2** : Du Pd-C 10% (70 mg) est ajouté à une solution dégazée de pentasaccharide **67** (100 mg, 58 µmol) dans de l'éthanol (10 mL). La suspension est saturée avec de l'hydrogène à 50 bars et agitée à TA pendant 10 j. Le suivi par CCM (iPrOH/H₂ONH₃, 4/1/0,5 et DCM/MeOH, 95/5) montre la disparition de **67** (Rf = 1 et 0,3, respectivement) et l'apparition d'un nouveau composé plus polaire (Rf = 0,3 et 0, respectivement). Le milieu réactionnel est filtré sur un filtre Acrodisc LC 25 mm, et le filtrat est concentré à l'évaporateur rotatif. Le résidu obtenu est purifié sur colonne C-18 (H₂O/CH₃CN, 100/0**→**7/3) pour conduire à la cible ***V*** sous forme d'un solide blanc (40 mg, 75%).
Rf = 0,3 (iPrOH/H₂O/NH₃, 4/1/0,5).
RMN ¹H (D₂O), *δ* 5.04 (d, 1H, *J*_{1,2} = 3.7 Hz, H-1_{E}), 4.91 (m, 1H, H-2_{C}), 4.90 (d, 1H, *J*_{1,2} = 1.4 Hz, H-1_{B}), 4.83 (d, 1H, *J*_{1,2} = 1.7 Hz, H-1_{A}), 4.79 (d, 1H, *J*_{1,2} = 1.4 Hz, H-1_{C}), 4.72 (d, 1H, H-1_{D}), 4.17 (m, 1H, H-2_{A}), 4.00 (m, 1H, H-5_{C}), 3.96 (m, 1H, H-5_{E}), 3.91 (m, 1H, H-2_{B}), 3.86 (dd, 1H, *J*_{2,3} = 3.2 Hz, *J*_{3,4} = 9.7 Hz, H-3_{C}), 3.82-3.79 (m, 2H, H-3_{A}, H-6a_{D}), 3.76 (m, 1H, H-2_{D}), 3.71 (pt, 1H, *J*_{3,4} = 9.5 Hz, H-3_{E}), 3.69 (m, 2H, H-6a_{E}, H-6b_{E}), 3.67-3.52 (m, 5H, H-6b_{D}, H_{Pr}, H-5_{A}, H-2_{E}, H-3_{B}), 3.48 (pt, 1H, *J*_{3,4} = 9.9 Hz, H-4_{C}), 3.44 (m, 1H, H-5_{B}), 3.40 (m, 1H, H_{Pr}), 3.38 (m, 1H, H-4_{E}), 3.33 (m, 1H, H-4_{B}), 3.33-3.28 (m, 3H, H-5_{D}, H-4_{D}, H-3_{D}), 3.23 (pt, 1H, *J*_{3,4} = 9.7 Hz, H-4_{A}), 2.07 (s, 3H, H_{Ac}), 2.04 (s, 3H, H_{NAc}), 1.49 (sex, 2H, CH₂), 1.17 (d, 3H, *J*_{5,6} = 6.1 Hz, H-6_{A}), 1.16 (m, 3H, *J*_{5,6} = 6.0 Hz, H-6_{B}), 1.15 (d, 3H, *J*_{5,6} = 6.4 Hz, H-6_{C}), 0.80 (t, 3H, *J* = 7.4 Hz, CH₃).
RMN ¹³C (D₂O), δ 174.7 (C_{NAc}), 173.5 (C_{Ac}), 103.1 (C-1_{B}, ¹*J*_{CH} = 172.0 Hz), 101.9 (C-1_{D}, ¹*J*_{CH} = 163.9 Hz), 99.0 (C-1_{A}, ¹*J*_{CH} = 174.2 Hz), 98.7 (C-1_{C}, ¹*J*_{CH} = 171.2 Hz), 94.7 (C-1_{E}, ¹*J*_{CH} = 170.5 Hz), 83.0 (C-3_{D}), 77.4 (C-3_{C}), 76.4 (C-4_{D}), 74.7 (C-2_{A}), 74.0 (C-3_{A}), 73.5 (C-3_{E}), 72.8 (C-2_{C}), 72.1 (C-4_{B}), 71.7 (C-5_{E}), 71.6 (C-2_{E}), 71.5 (C-4_{C}), 71.2 (C-4_{A}), 70.5 (C-2_{B}), 70.3 (C-3_{B}), 70.0 (C_{Pr}), 69.4 (2C, C-4_{E}, C-5_{D}), 69.2 (C-5_{C}), 69.1 (C-5_{A}), 68.5 (C-5_{B}), 61.0 (C-6_{D}), 60.6 (C-6_{E}), 55.8 (C-2_{D}), 23.1 (C_{NAc}), 22.3 (CH₂), 20.6 (C_{Ac}), 17.1, 16.9, 16.7 (C-6_{A}*, C-6_{B}*, C-6_{C}*), 10.2 (CH₃).

| | | |
|---|---|---|
| HRMS (ESI⁺) : | [M+H]⁺ C₃₇H₆₄NO₂₄ | m/z théorique : 906.3818 |
| | | m/z mesurée: 906.3823 |
| [M+Na]⁺ C₃₇H₆₃NO₂₄Na | | m/z théorique : 928.3638 |
| | | m/z mesurée : 928.3630 |

### Procédé 6 :

### ➢ Propyl α-L-rhamnopyranosyl-(1→3)-α-L-rhanmopyranosyl-(1→3)-(2-acétamido-2-désoxy-β-D-glucopyranosyl)-(1→2)-[α-D-glucopyranosyl-(1→3)]-α-L-rhaminopyranoside VI :

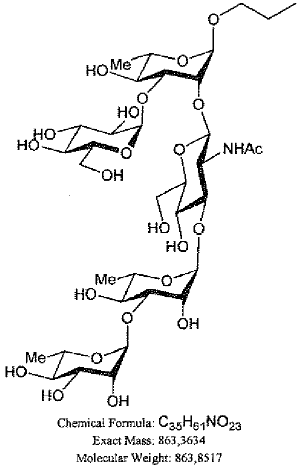

Du Pd-C 10% (250 mg) est ajouté à une solution dégazée de pentasaccharide 57 (200 mg, 130 µmol) dans de l'éthanol (15 mL). La suspension est saturée avec de l'hydrogène à 50 bars et agitée à TA pendant 2 j. Le suivi par CCM (iPrOH/H₂O/NH₃, 4/1/0,5 et DCM/MeOH, 95/5) montre la disparition de **57** (Rf = 1 et 0,2, respectivement) et l'apparition d'un nouveau composé plus polaire (Rf = 0,2 et 0, respectivement). Le milieu réactionnel est filtré sur un filtre Acrodisc LC 25 mm, et le filtrat est concentré à l'évaporateur rotatif. Le résidu obtenu est purifié sur colonne C-18 (H₂O/CH₃CN, 100/0→7/3) pour conduire à la cible ***VI*** sous forme d'un solide blanc (81 mg, 71%).
Rf = 0,2 (iPrOH/H₂O/NH₃, 4/1/0,5).
RMN ¹H (D₂O), *δ* 4.94 (d, 1H, *J*_{1,2} = 3.6 Hz, H-1_{E}), 4.78 (bs, 1H, H-1_{B}), 4.71 (bs, 1H, H-1_{A}), 4.63 (bs, 1H, H-1_{D}), 4.61 (bs, 1H, H-1_{C}), 4.07 (m, 1H, H-2_{A}), 3.83-3.78 (m, 3H, H-2_{B}, H-5_{E}, H-5_{C}), 3.71-3.68 (m, 2H, H-3_{A}, H-6a_{D}), 3.66-3.64 (m, 2H, H-2_{C}, H-2_{D}), 3.62-3.59 (m, 2H, H-3_{E}, H-3_{B}), 3.59-3.52 (m, 5H, H-6a_{E}, H-6b_{E}, H-3_{C}, H-5_{B}, H-6b_{D}), 3.50-3.39 (m, 2H, H-5_{A}, H-2_{E}), 3.41 (m, 1H, H_{Pr}), 3.32-3.18 (m, 7H, H-4_{E}, H-4_{C}, H_{Pr}, H-3_{D}, H-5_{D}, H-4_{B}, H-4_{D}), 3.10 (pt, 1H, *J*_{3,4} = 9.7 Hz, H-4_{A}), 1.89 (s, 3H, H_{NAc}), 1.38 (sex, 2H, CH₂), 1.09 (m, 3H, *J*_{5,6} = 6.2 Hz, H-6_{B}), 1.05 (d, 3H, *J*_{5,6} = 6.2 Hz, H-6_{A}), 1.01 (d, 3H, *J*_{5,6} = 6.2 Hz, H-6_{C}), 0.29 (t, 3H, *J* = 7.4 Hz, CH₃).
RMN ¹³C (D₂O), *δ* 174.4 (C_{NAc}), 102.8 (C-1_{B}, ¹*J*_{CH} = 172.0 Hz), 102.0 (C-1_{D}, ¹*J*_{CH} = 163.2 Hz), 101.7 (C-1_{C}, ¹*J*_{CH} = 171.2 Hz), 99.1 (C-1_{A}, ¹*J*_{CH} = 172.7 Hz), 95.0 (C-1_{E}, ¹*J*_{CH} = 170.5 Hz), 82.1 (C-3_{D}), 78.5 (C-3_{C}), 76.5 (C-4_{D}), 74.8 (C-2_{A}), 74.4 (C-3_{A}), 73.6 (C-3_{E}), 72.5 (C-4_{B}), 71.9 (C-5_{E}), 71.8 (C-2_{E}), 71.7 (C-4_{C}), 71.3 (C-4_{A}), 71.0 (C-2_{C}), 70.7 (C-3_{B}), 70.6 (C-2_{B}), 70.1 (C_{Pr}), 69.9 (C-5_{D}), 69.5 (2C, C-5_{B}, C-5_{C}), 69.3 (C-5_{A}), 68.8 (C-4_{E}), 61.2 (C-6_{D}), 60.8 (C-6_{E}), 55.9 (C-2_{D}), 23.1 (C_{NAc}), 22.5 (CH₂), 17.2, 17.1, 16.9 (C-6_{A}*, C-6_{B}*, C-6_{C}*), 10.3 (CH₃).

| | | |
|---|---|---|
| HRMS (ESI⁺) : | [M+H]⁺ C₃₅H₆₂NO₂₃ | m/z théorique : 864.3713 |
| | | m/z mesurée : 864.3652 |
| | [M+Na]⁺ C₃₅H₆₁NO₂₃Na | m/z théorique : 886.3532 |
| | | m/z mesurée : 886.3461 |

### Partie III - Synthèse des cibles XI, XII, XIII, XIV, XV et XVI

Dans cette partie, nous décrirons la synthèse de l'ensemble des cibles tétra- et pentasaccharides porteurs d'un résidu C ou D à leur extrémité réductrice. Comme précédemment, les cibles ont été synthétisées sous forme de glycoside de propyle de manière à bloquer la position réductrice conformément à l'anomérie adoptée par le résidu au sein du polysaccharide naturel. Le résidu C est acétylé ou non, correspondant respectivement à des fragments représentatifs des antigènes O des sérotypes 3a et X de *S. flexneri.*

| | Accepteur | Donneur | Cible |
|---|---|---|---|
| Procédé 7 | 79 | 80 | ***XI*** |
| Procédé 8 | 79 | 80 | ***XII*** |
| Procédé 9 | 85 | 1 | ***XIII*** |
| Procédé 10 | 85 | 1 | ***XIV*** |
| Procédé 11 | 86 | 19 | ***XV*** |
| Procédé 12 | 86 | 19 | ***XVI*** |

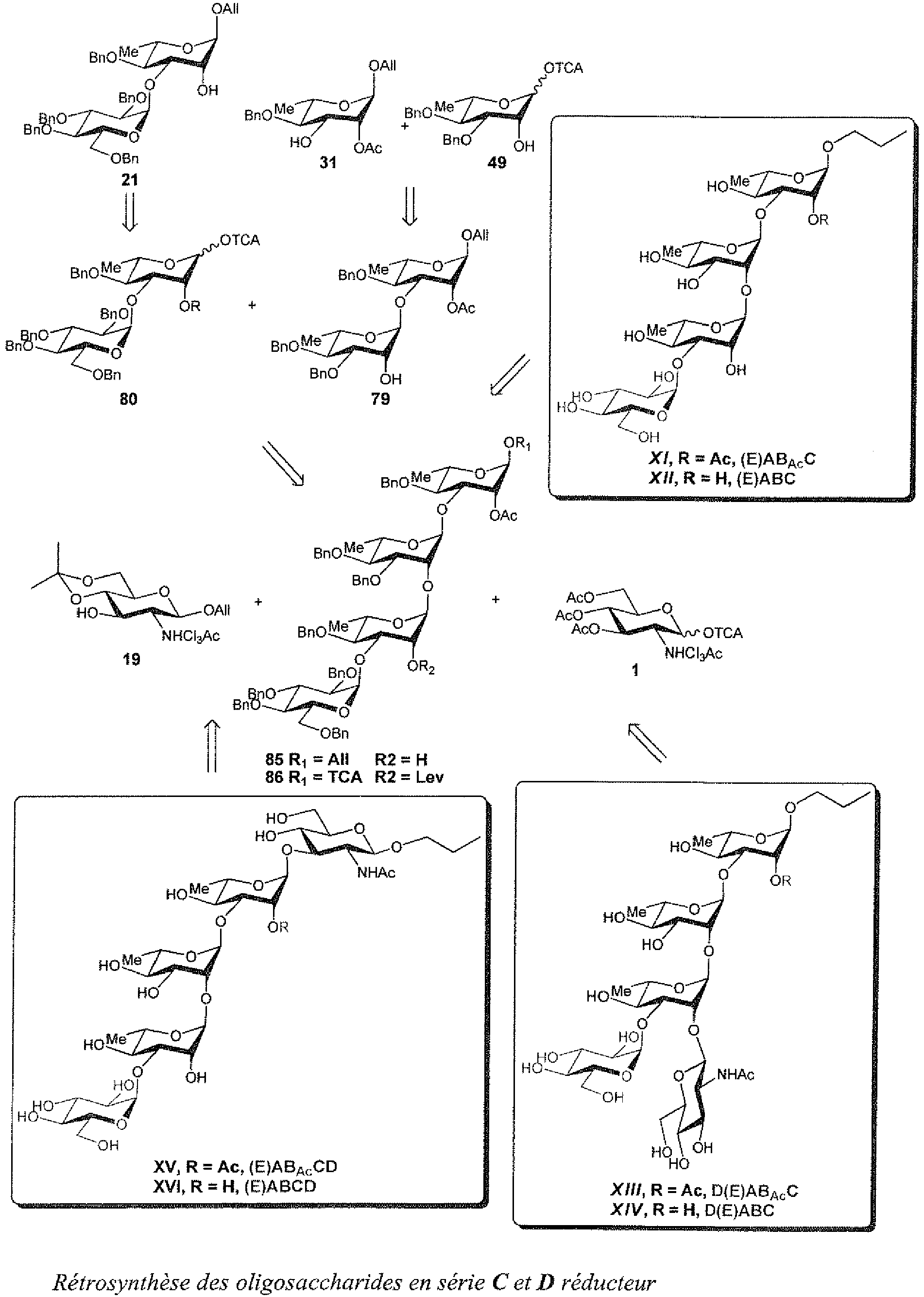

### Procédé 7 :

La description se rapporte au procédé de préparation du composé XI (tétrasaccharide (E)AB_{Ac}C) tel que défini dans la liste L1 comprenant les étapes suivantes :
- condensation du disaccharide accepteur **79** (BC) avec le disaccharide donneur **80** ((E)A) conduisant au tétrasaccharide **87** de préférence la réaction de condensation est réalisée dans un solvant tel que le dichlorométhane, le 1,2-dichloroéthane, l'éther, le toluène, l'acétonitrile ou tout autre solvant équivalent, en présence d'un acide de Lewis tel que le TMSOTf ou l'acide triflique, préférentiellement, dans le toluène ou le dichlorométhane en présence de TMSOTf (schéma 49) ;
- déprotection du tétrasaccharide **87** conduisant au tétrasaccharide accepteur **85** de préférence dans un mélange pyridine/AcOH en présence d'hydrazine monohydraté (schéma 49) ;
- déprotection par hydrogénolyse des groupes benzyles du tétrasaccharide **85** conduisant au tétrasaccharide (E)AB_{Ac}C de préférence sous atmosphère atmosphérique d'hydrogène dans l'éthanol en présence de palladium en conditions neutres (schéma 50).

### 1. Monosaccharide B donneur

L'accès au synthon **BC 61** implique le donneur **49** protégé en position 2 par un groupe lévulinoyle^{88,89} (partie rétrosynthèse). Une synthèse de ce dernier, valorisant le succès de la synthèse du diol **26** à grande échelle, est décrite dans la partie 1. Ici, nous rapportons l'optimisation de la synthèse de l'accepteur **69,** précurseur du donneur **49.**

Une méthode d'accès rapide à un composé totalement protégé avec un rendement de l'ordre de 94% à partir du L-rhamnose commercial **27** issu d'un intermédiaire clé, l'orthoester de méthyle **70** a été décrite⁶⁷ (Schéma 39). En procédant selon le protocole décrit,⁶⁷ notre rendement en orthoester di-*O*-benzyl **70** fut seulement de 75%. Dans la mesure où notre objectif était d'accéder au synthon **BC 61,** à la fois accepteur et donneur, la préparation de l'orthoester d'allyle, a été préférée à celle de l'orthoester de méthyle, dans le but de synthétiser l'accepteur **58,** après ouverture régiosélective de l,orthoester.¹⁰³

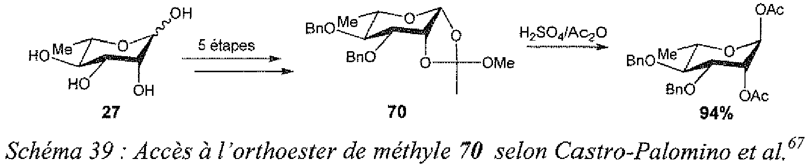

Ainsi le rhamnose commercial **27** est péracétylé en un mélange d'anomères (8/2) **71,** puis activé en précurseur **72** par action du bromure d'hydrogène dans l'acide acétique.¹⁰⁴ Le brut réactionnel, traité par l'alcool allylique dans la lutidine,¹⁰⁵ conduit à l'orthoester di-*O*-acétylé **73.** La désacétylation de ce dernier par action du carbonate de potassium dans le méthanol donne de façon quantitative le diol **74,** qui est di-O-benzylé par action du dialcoolate correspondant sur le bromure de benzyle. L'orthoester clé **75** est isolé par chromatographie sur gel de silice (76%) (Schéma 40).

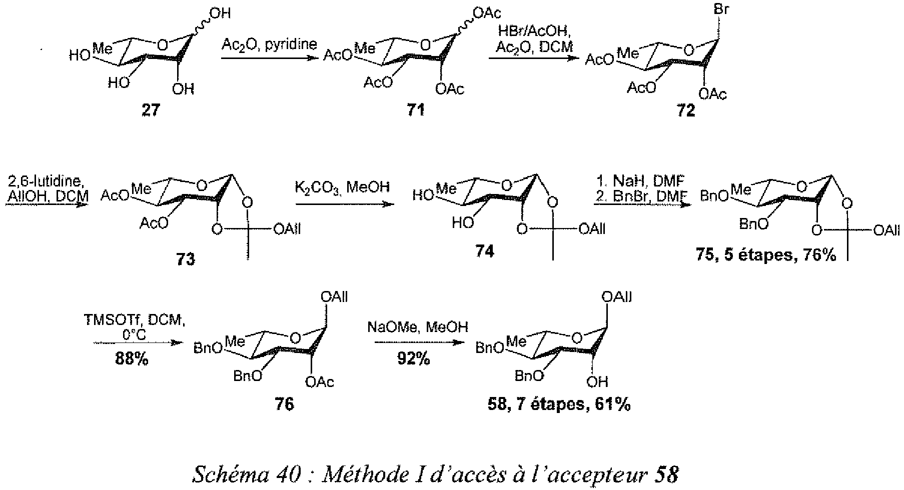

Selon cette stratégie, la première option consiste en une ouverture régiosélective en milieu acide. Différentes méthodes ont été décrites¹⁰³ parmi lesquelles, l'utilisation du TMSOTf.¹⁰⁶ En présence de ce promoteur, l'ouverture le de l'orthoester **75** est régio- et stéréosélective. En effet, seul l'anomère α **76** est isolé (88%) (Schéma 40). Un mécanisme permettant d'expliquer cette bonne stéréosélectivité a été proposé¹⁰⁶ (Schéma 41). De façon forte intéressante, la présence de fonctions benzyles semble primordiale. En effet, traité dans les mêmes conditions, l'analogue di-*O*-acétylé réagit de façon très différente.¹⁰⁶

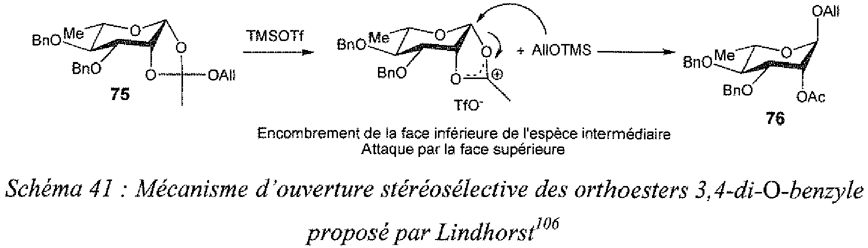

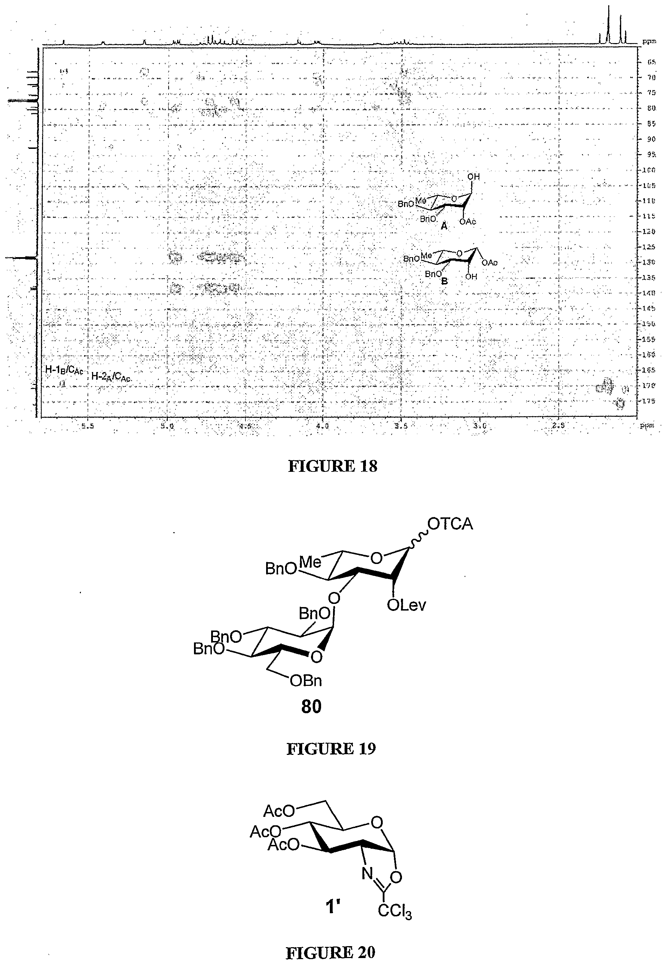

La simple désacétylation de **76** réalisée en présence de NaOMe conduit à l'alcool **58** avec un rendement de 92%, soit 61% en 7 étapes à partir du L-rhamnose commercial. Par ailleurs, nous avons pu observer que le traitement de l'orthoester d'allyle **75** par une solution acide d'HCl à 10% conduit à un mélange de deux régioisomères monoacétylés **77** et **78** moins polaires, difficilement séparables, dans un rapport 3/2 (Schéma 43). En outre, le spectre C¹³GD montre que le composé majoritaire **77** correspond à un anomère a alors que **78** correspond à un anomère β. D'autre part, les spectres HMBC et HSQC (Figure 17 et Figure 18), permettent de prouver que le composé **77** est acétylé en position 2 alors que le composé **78** l'est en position 1. Cette analyse par RMN complète a permis de confirmer la structure des deux composés 77 et **78,** à savoir le 2-*O*-acétyl-3,4-di-*O*-benzyl-α-L-rhamnopyranose et le 1-*O*-acétyl-3,4-di-*O*-benzyl-β-L-rhamnopyranose, respectivement. Ces résultats peuvent être expliqués sur la base des données de la littérature.^{107, 108}

Un mécanisme de formation des composés **77** et **78** est proposé (Schéma 42) sur la base des travaux de Kong,¹⁰³.

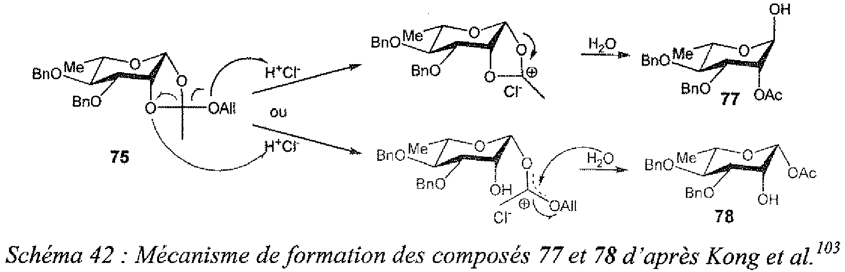

Lorsque le mélange des composés **77** et **78** est chauffé dans l'alcool allylique en présence de chlorure d'acétyle, le glycoside d'allyle **58,** désacétylé en position 2, est obtenu avec un rendement de 85%, soit 65% en 6 étapes partir du L-rhamnose commercial (Schéma 43). Effectivement, la fonction acétyle, instable en milieu acide alcoolique, est probablement clivée lors de cette étape d'allylation anomérique. Cette voie s'est avérée plus efficace que la voie I puisqu'elle permet d'isoler l'alcool **58** avec un rendement plus élevée en moins d'étapes.

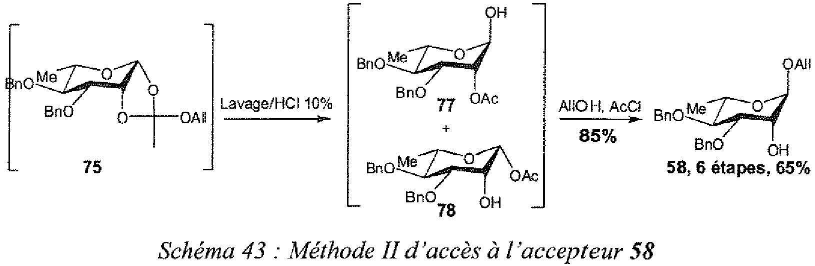

Dans la partie précédente, l'accepteur **58** était obtenu avec un rendement de 68% en 5 étapes impliquant le diol **26** (Schéma 44). Malgré ce rendement légèrement supérieur, la voie II, proposé ci-dessus, reste la plus efficace car l'utilisation de l'étain est évitée et les 6 étapes peuvent être enchaînées sans aucune purification intermédiaire. Dans ce cas, l'alcool **58** est isolé, par simple chromatographie avec un rendement global de 72%, au lieu des 65% obtenus sinon (Schéma 44).

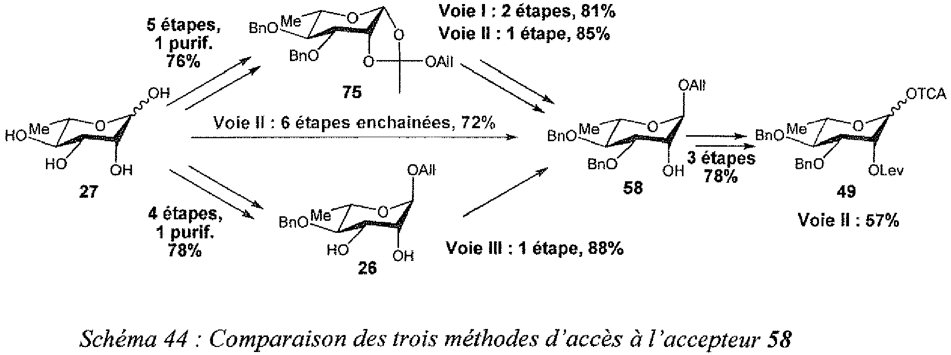

Pour rappel (Partie 2), l'accepteur **58** est transformé en donneur **49** en trois étapes, lévulinoylation, déallylation, puis activation avec un rendement de 78% (Schéma 44). La synthèse de l'alcool **58** ayant été optimisée, le donneur **49** est maintenant accessible en 9 étapes avec un rendement de 57%.

### 2. Disaccharide BC accepteur

Le disaccharide totalement protégé **61** (Partie 2) peut être converti en accepteur **79** après déprotection du groupement lévulinoyle (81%) dans un mélange pyridine/AcOH en présence d'hydrazine monohydraté¹⁰⁹ (Schéma 45).

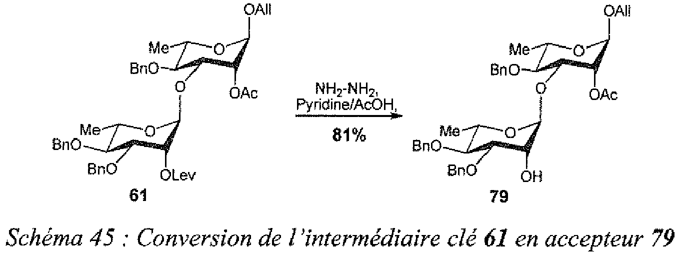

### 3. Disaccharide (E)A donneur

La fonction lévulinoyle est le groupe protecteur orthogonal à la fonction acétyle, dit « participant », retenu à l'issu de l'analyse rétrosynthétique menée sur l'antigène O de *S. flexneri* 3a. En conséquence, le disaccharide donneur **80** est l'un des synthons clés, impliqués dans la préparation des cibles ***XI*** et ***XII*** (Figure 19).

Le premier test de lévulinoylation de l'accepteur **21** (Partie 2) est effectué dans les conditions utilisées pour préparer l'ester de lévulinoyle **81.** à savoir par action sur l'acide lévulinique en présence de DCC et de DMAP dans le dichlorométhane.⁸⁹ Mais, cette fois, la réaction n'est pas totale (Schéma 46).

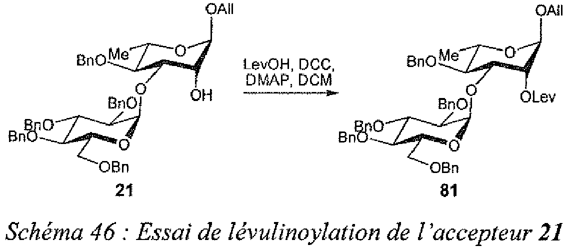

Varier différents paramètres, tels le solvant ou la température de réaction, n'a permis au mieux que d'obtenir l'ester de lévulinoyle **81** en mélange avec l'alcool **21** dans un rapport 4/1 (Tableau 6). Ce résultat peut être expliqué par l'encombrement présumé de la fonction hydroxyle en position 2_{A}.

**Tableau 6 : Lévulinoylation du glycoside d'allyle 21**

| **Solvants** | **Température** | **Temps de réaction** | **Conversion*** |
|---|---|---|---|
| DCM | TA | 3 jours | 60% |
| | 50°C | 5 jours | 80% |
| DCE | 80°C | 5 jours | 80% |
| CH₃CN | | / | 0% |
| DMF | 80°C | / | 0% |
| DCE | 90°C | 5 jours | 80% |

| | | | |
|---|---|---|---|
| * Les taux de conversion sont déterminés par CCM | | | |

La séparation de l'accepteur **21** et du lévulinate **81** étant extrêmement difficile, d'autres conditions d'introduction du groupement lévulinoyle ont été testées (Schéma 47). Elles impliquent le chlorure de lévulinoyle^{110, 111} ou l'anhydride lévulinique.¹¹²

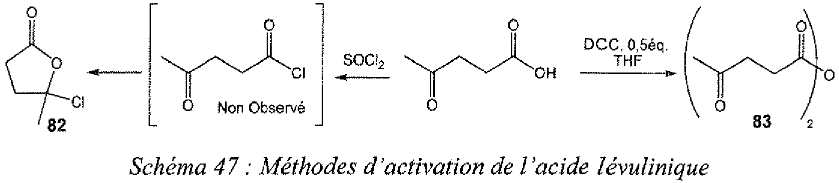

L'action du chlorure de thionyle sur l'acide lévulinique ne permet pas d'isoler le chlorure de lévulinoyle **82,** comme attendu, mais le produit de cyclisation confirmée par l'analyse RMN.¹¹³ Ce dernier ne réagissant pas avec l'accepteur **21**¹¹⁰ (Schéma 47), cette voie a été abandonnée. En revanche, lorsque l'acide lévulinique est mis en réaction avec la DCC,¹¹² l'anhydride lévulinique **83** est obtenu de manière quantitative après contrôle RMN (Schéma 47). L'action de cet anhydride **83** sur l'accepteur **21** a été étudiée dans des conditions classiques d'estérification, pyridine et DMAP (Tableau 7). La comparaison des entrées 1 et 2 du Tableau 7 permet de confirmer qu'augmenter la température lors de cette estérification, augmente le taux de conversion alors que l'entrée 3 montre que la DMAP active la réaction. La quantité d'anhydride lévulinique ajouté a aussi été variée. Effectivement, l'augmentation des quantités de DMAP et d'anhydride lévulinique dans le milieu réactionnel (entrée 3, 4 et 5 du Tableau 7) est corrélée avec le taux de conversion de **21** en **81.**

**Tableau 7 : Optimisation de la lévulinoylation de l'accepteur 21**

| **Entrée** | **Anhydride lévulinique (éq.)** | **DMAP (éq.)** | **Température** | **Conversion*** |
|---|---|---|---|---|
| **1** | 4 | 0.5 | TA | 1 0% |
| **2** | | | 50°C | 30% |
| **3** | | 2 | | 60% |
| **4** | 8 | 2 | | 80% |
| **5** | 10 | 5 | | 90% |
| **6** | | | 50°C** | 100% |

| | | | | |
|---|---|---|---|---|
| * Les taux de conversion sont déterminés par CCM ** Dans ce cas, l'anhydride lévulinique est ajouté au milieu réactionnel à 50°C. | | | | |

Au vu des résultats du Tableau 7 (entrée 5 et 6), si 10 équivalents d'anhydride lévulinique sont ajoutés à 50°C alors la conversion est maximale et permet d'isoler l'ester de lévulinoyle **81** voulu avec un rendement de 95%. Ensuite, le donneur **80** est préparé en deux étapes. Le disaccharide **81** est déallylé en hémiacétal **84** avec un rendement de 92%.^{39, 40} Ce dernier est activé par action du trichloroacétonitrile pour obtenir le donneur **80** avec un rendement de 97%.^{22, 23}

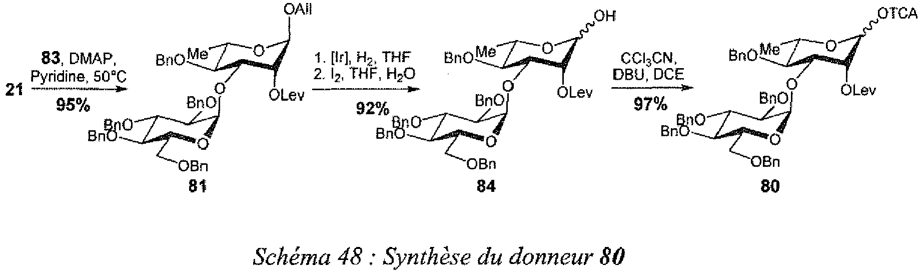

### 4. Tétrasaccharide (E)ABC

Une fois les disaccharides **BC** accepteur **79** et **(E)A** donneur **80** disponibles, la synthèse des tétrasaccharides **(E)ABC** accepteur **85** et donneur **86** a été réalisée.

### a. Série (E)ABC Accepteur

De même que pour les synthèses du partie 2, la réaction de glycosylation entre le donneur **80** et l'accepteur **79** a été étudiée dans différents solvants.^{69, 76-79} Si la condensation est réalisée dans le dichlorométhane en présence de TMSOTf (0,3 éq.) et de tamis moléculaire, le tétrasaccharide **87** est obtenu avec la configuration α attendue (J_{1,2} = 1,6 Hz, ¹J_{CH} = 176,6 Hz) et un rendement de 69% (Schéma 49). En revanche, dans le toluène, le tétrasaccharide **87** est obtenu avec un meilleur rendement (75%), qui atteint 92% quand la synthèse est reproduite à grande échelle (4g d'accepteur **79** au lieu de 200 mg). Ce système de solvant a donc été adopté.
La déprotection du groupement lévulinoyle, porté par **87** dans un mélange pyridine/AcOH en présence d'hydrazine monohydraté¹⁰⁹ conduit à l'accepteur **85** requis avec un rendement de 89% confirmant le caractère orthogonal du système de groupes protecteurs mis en place (Schéma 49).

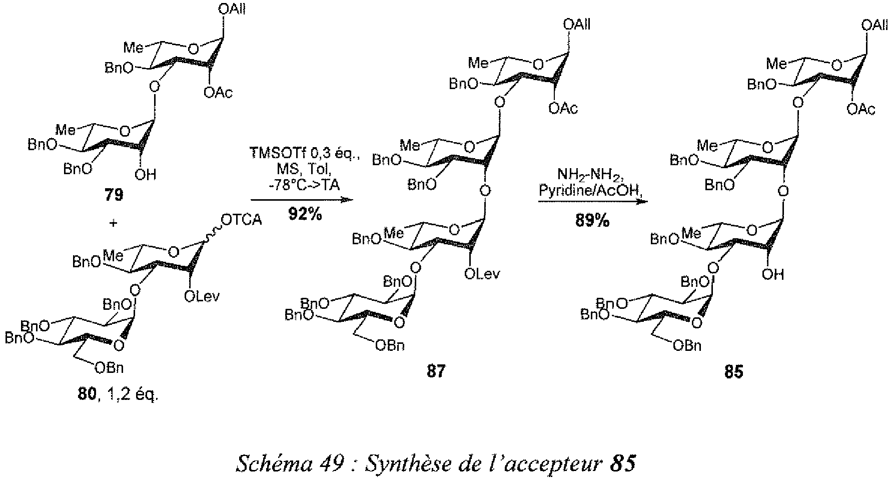

### b. Accès aux cibles XI et XII

La présence d'un groupement acétyle sur le tétrasaccharide **85** exclu, à priori des conditions d'hydrogénation en milieu acide. L'hydrogénolyse des groupes benzyles du tétrasaccharide **85** est donc conduite sous atmosphère atmosphérique d'hydrogène dans l'éthanol en présence de palladium en condition neutre. Le glycoside de propyle ***XI*** est isolé avec un rendement de 76% (Schéma 50).

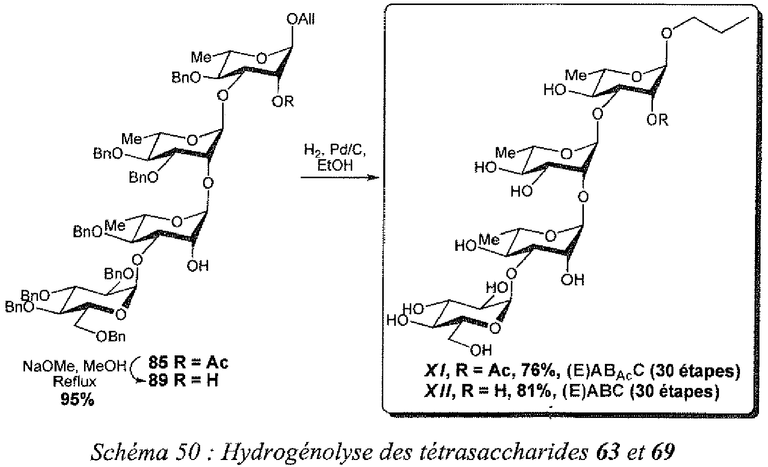

### Procédé 8 :

La description se rapporte aussi au procédé de préparation du composé XII (tétrasaccharide (E)ABC) tel que défini dans la liste L1 comprenant les étapes suivantes :
- condensation du disaccharide accepteur **79** (BC) avec le disaccharide donneur **80** ((E)A) conduisant au tétrasaccharide **87** de préférence la réaction de condensation est réalisée dans un solvant tel que le dichlorométhane, le 1,2-dichloroéthane, l'éther, le toluène, l'acétonitrile ou tout autre solvant équivalent, en présence d'un acide de Lewis tel que le TMSOTf ou l'acide triflique, préférentiellement, dans le toluène ou le dichlorométhane en présence de TMSOTf (schéma 49) ;
- déprotection du tétrasaccharide **87** conduisant au tétrasaccharide accepteur **85** de préférence dans un mélange pyridine/AcOH en présence d'hydrazine monohydraté (schéma 49) ;
- désacétylation du tétrasaccharide accepteur **85** au tétrasaccharide **89** de préférence en présence de NaOMe à reflux conduisant ;
- déprotection par hydrogénolyse des groupes benzyles du tétrasaccharide **89** conduisant au tétrasaccharide (E)ABC de préférence sous atmosphère atmosphérique d'hydrogène dans l'éthanol en présence de palladium en conditions neutres (schéma 50).
Pour obtenir la cible ***XII*,** représentative de l'antigène O de la bactérie *S. flexneri* X, une étape de désacétylation est nécessaire. L'accepteur **85** est désacétylé en présence de NaOMe à reflux pour obtenir le diol **89** (95%), puis débenzylé par hydrogénolyse. La cible attendu ***XII*** est obtenue sous forme de glycoside de propyle avec un rendement de 81% (Schéma 50).

### Procédé 9 :

La description se rapporte au procédé de préparation du composé XIII (pentasaccharide D(E)AB_{Ac}C) tel que défini dans la liste L1 comprenant les étapes suivantes :
- condensation du monosaccharide donneur **1** et du tétrasaccharide accepteur **85** conduisant au pentasaccharide **90** de préférence la réaction de condensation est réalisée dans un solvant tel que le dichlorométhane, le 1,2-dichloroéthane, l'éther, le toluène, l'acétonitrile ou tout autre solvant équivalent, en présence d'un acide de Lewis tel que le TMSOTf ou l'acide triflique, préférentiellement, dans le toluène ou le dichlorométhane en présence de TMSOTf (schéma 51) ;
- désacétylation du pentasaccharide **90** conduisant au pentasaccharide **91** de préférence dans du Iméthanol en présence de NaOMe (schéma 52) ;
- déprotection du pentasaccharide **91** pour conduire au pentasaccharide D(E)AB_{Ac}C de préférence sous pression d'hydrogène dans l'éthanol en présence de palladium sur charbon (schéma 53).

### 5. Pentasaccharide D(E)ABC

Le pentasaccharide **90** est obtenu par condensation du donneur **1** et de l'accepteur **85.** Différentes conditions de solvant ont été comparées^{69, 76-79} (Tableau 8). Dans les conditions de couplage « habituelles » (dichlorométhane ou toluène, 0,3 éq. de TMSOTf, tamis moléculaire, entrées 1 et 2), l'oxazoline **1',**¹ déjà décrite, est isolée (δ_{H-1} = 6,35 ppm en RMN ¹H) (Figure 20). Les couplages suivants ont été réalisés avec 0,5 éq. de TMSOTf pour permettre l'ouverture de l'oxazoline.⁶¹

La comparaison des entrées 1 et 6 avec, respectivement les entrées 2 et 7 montrent, encore une fois, que l'utilisation du toluène (30% et 72%) est préférable à celle du dichlorométhane (20% et 67%) (Tableau 8).

**Tableau 8 : Optimisation du couplage entre l'accepteur 63 et le donneur 10**

| **Entrée** | **TMSOTf** | **Température** | **Donneur 1** | **Solvants** | **Rendement** |
|---|---|---|---|---|---|
| **1** | 0,3 éq. | -78°C | 1,3 éq. | D ichlorométhane | 20% |
| **2** | | | | Toluène | 30% |
| **3** | 0,5 éq. | -78°C | 1,3 éq. | Toluène | 50% |
| **4** | | -40°C | | Toluène | 65% |
| **5** | | -20°C | | Toluène | 60% |
| **6** | | -40°C | 1,6 éq. | Dichlorométhane | 67% |
| **7** | | | | Toluène | 72% |
| **8** | | | 2 éq. | Toluène | 82% |

Des trois conditions de température testées, les meilleurs résultats sont obtenus à -40°C, c'est donc la température retenue (entrée 3, 4 et 5). En effet, à -20°C, le donneur **1** est vraisemblablement moins stable qu'à -40°C ce qui explique le rendement plus faible à cette température alors qu'à -78°C, l'accepteur **85** n'est pas assez réactif et l'hydrolyse du donneur **1** domine. Mais, lors de tous ces couplages, le tétrasaccharide **85** accepteur n'est pas totalement consommé. En revanche, si 2 éq. de donneur **1** sont employés (entrée 7), le glycoside d'allyle **90** est isolé avec un rendement de 82% et la configuration β attendue (¹J_{CH} = 161.0 Hz) (Schéma 51).

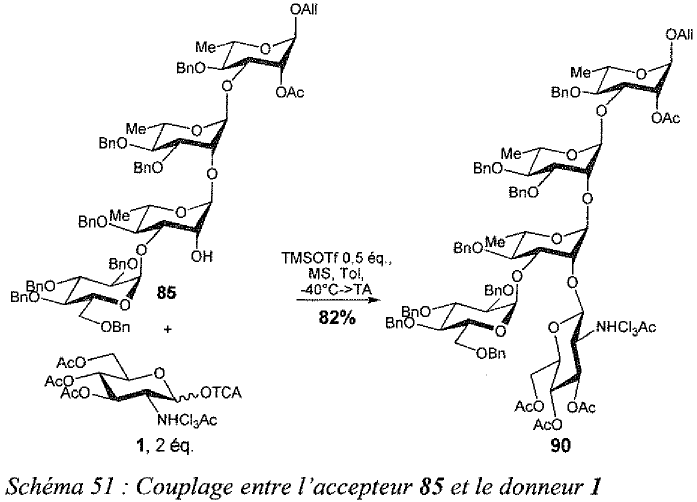

Dans un second temps, le clivage sélectif des acétyles présents sur l'unité glucosamine **D** a été testé dans différentes conditions (Tableau 9). Contrairement aux résultats obtenus lors de la desacétylation de **52,** le carbonate de potassium ne permet pas une désacétylation sélective et le tétraol **92** est formé en quantités importantes aux dépens du triol **91** (entrée 1). Le même phénomène est observé si la triéthylamine est utilisée (entrée 6). Dans les conditions de Zemplen⁶⁰ (entrée 2), la réaction est trop lente. Si la quantité de NaOMe est augmentée (entrée 3, 4 et 5), les résultats sont identiques (rapport 65/35), seule la durée de la réaction varie. Cette différence de sélectivité par rapport aux résultats décrits en série **A** réducteur (Partie 2) peut être expliquée par un encombrement beaucoup moins important de la fonction acétyle en position 2_{C}. En effet, le résidu concerné est en position terminale et donc plus accessible

**Tableau 9 : Conditions de désacétylation sélective**

| **Entrée** | **Conditions** | **Temps de réaction** | **Rapport 91/92** |
|---|---|---|---|
| **1** | K₂CO₃/MeOH 1 éq.⁸⁵ | 30 min. | 40/60 |
| **2** | NaOMe/MeOH 0,2 éq.⁶⁰ | 1 jour | 65/35 |
| **3** | NaOMe/MeOH 1 éq. | 2 h. | 65/35 |
| **4** | NaOMe/MeOH 2 éq. | 45 min. | 65/35 |
| **5** | NaOMe/MeOH 3 éq. | 20 min. | 65/35 |
| **6** | NEt₃/MeOH/H₂O^{83, 84} | 7 h. | 60/40 |

Le produit **90** est donc désacétylé sélectivement dans du méthanol en présence de NaOMe (2 éq.) pour obtenir le composé mono-acétylé **91** et le tétraol **92** dans un rapport 65/35 avec un rendement de 86% (Schéma 52).

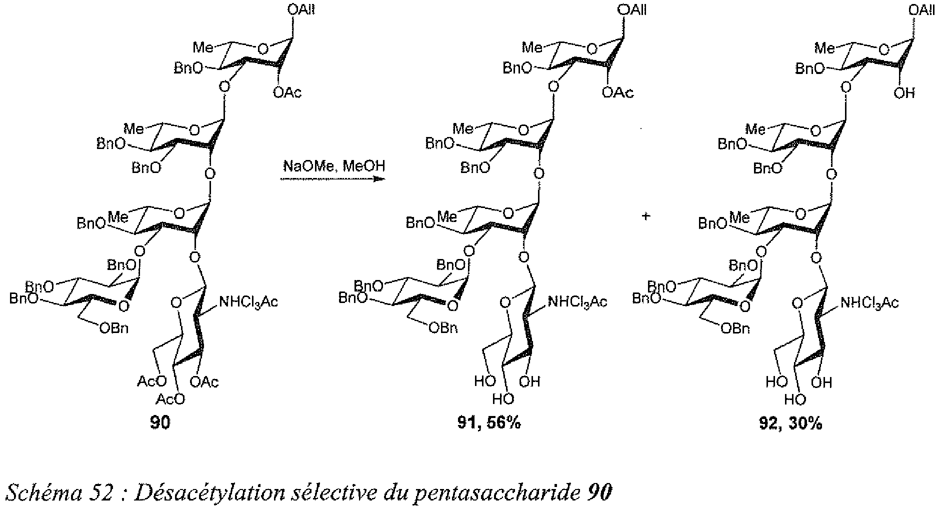

Les conditions d'hydrogénolyse d'un oligosaccharide porteur, à la fois, d'une fonction trichloroacétamide et de fonction benzyles mises au point sur **56** (Partie 2) ont été appliquées aux intermédiaires de synthèse **91** et **92** sous 50 bars de pression. L'intermédiaire chloroacétamide a toujours été observé mais la poursuite de la réaction sur plusieurs jours, a permis la conversion totale de ce dernier en acétamide. Cette réaction peut, en effet, être suivie par masse ou par analyse RMN grâce au déplacement des protons de la fonction C(O)CH₂Cl (δ_{CH2} = 4,1 ppm) et du carbone (δ_{CH2} = 42,5 ppm). Ainsi, l'hydrogénolyse des deux pentasaccharides **91** et **92** sous pression d'hydrogène (50 bars) permet d'isoler, après purification par chromatographie phase inverse (C18), les deux glycosides de propyle ***XIII*** et ***XIV*** avec le même rendement de 74% (Schéma 53).

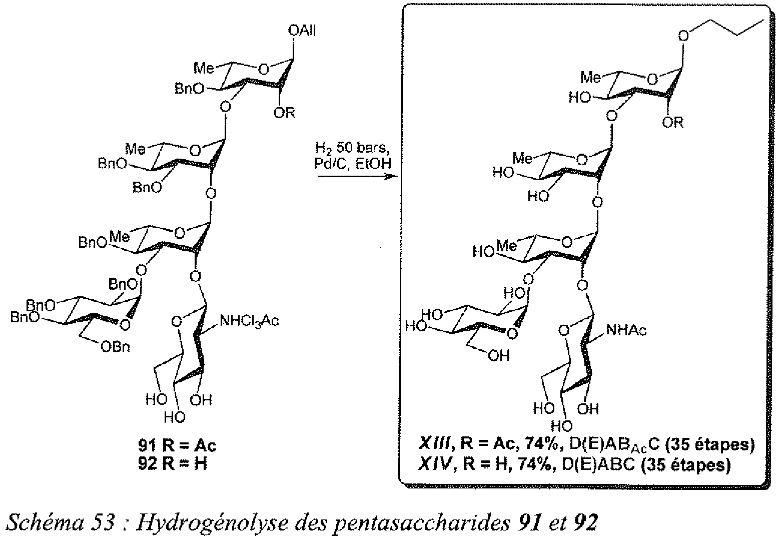

Il est à noter que l'hydrogénolyse de ces deux pentasaccharides **91** et **92** s'est faite, respectivement en 2 et un jour alors que pour les deux pentasaccharides **56** et **57,** en 10 et 2 jours, respectivement. Cette différence de vitesse de conversion du trichloroacétamide en acétamide peut s'expliquer par l'encombrement plus important de l'unité **D,** comportant la fonction trichloroacétamide, des pentasaccharides en série **A** réducteur par rapport à ceux en série C réducteur.

### Procédé 10 :

La description se rapporte encore au procédé de préparation du composé XIV (pentasaccharide D(E)ABC) tel que défini dans la liste L1 comprenant les étapes suivantes :
- condensation du monosaccharide donneur **1** et du tétrasaccharide accepteur **85** conduisant au pentasaccharide **90** de préférence la réaction de condensation est réalisée dans un solvant tel que le dichlorométhane, le 1,2-dichloroéthane, l'éther, le toluène, l'acétonitrile ou tout autre solvant équivalent, en présence d'un acide de Lewis tel que le TMSOTf ou l'acide triflique, préférentiellement, dans le toluène ou le dichlorométhane, en présence de TMSOTf (schéma 51) ;
- désacétylation du pentasaccharide **90** conduisant au pentasaccharide **92** de préférence dans du méthanol en présence de NaOMe (schéma 52) ;
- déprotection du pentasaccharide **92** pour conduire au pentasaccharide D(E)ABC de préférence sous pression d'hydrogène dans l'éthanol en présence de palladium sur charbon (schéma 53).

### Procédé 11 :

La description se rapporte au procédé de préparation du composé XV (pentasaccharide (E)AB_{Ac}CD) tel que défini dans la liste L1 comprenant les étapes suivantes :
- condensation du monosaccharide accepteur **19** et du tétrasaccharide donneur **86** conduisant au pentasaccharide **93** de préférence la réaction de condensation est réalisée dans un solvant tel que le dichlorométhane, le 1,2-dichloroéthane, l'éther, le toluène, l'acétonitrile ou tout autre solvant équivalent, en présence d'un acide de Lewis tel que le TMSOTf ou l'acide triflique, préférentiellement, dans le toluène à 70°C (schéma 55) ;
- clivage de la fonction lévulinoyle du pentasaccharide **93** conduisant au pentasaccharide **94,** de préférence dans un mélange pyridine/AcOH en présence d'hydrazine monohydraté (schéma 56) ;
- clivage du groupement isopropylidène du pentasaccharide **94** par hydrolyse acide conduisant au pentasaccharide **95** ;
- hydrogénolyse du pentasaccharide **95** conduisant au pentasaccharide (E)AB_{Ac}CD, de préférence sous atmosphère d'hydrogène dans l'alcool, par exemple l'éthanol en présence de dérivé de palladium, tel que le palladium sur charbon(schéma 58).

### 6. Série (E)ABC Donneur

Le donneur **86** est préparé en deux étapes, à partir du même intermédiaire **87.** Après déallylation,^{39, 40} Phémiacétal obtenu **88** (90%) est activé en trichloroacétimidate **86** avec un rendement de 88%^{22, 23}.

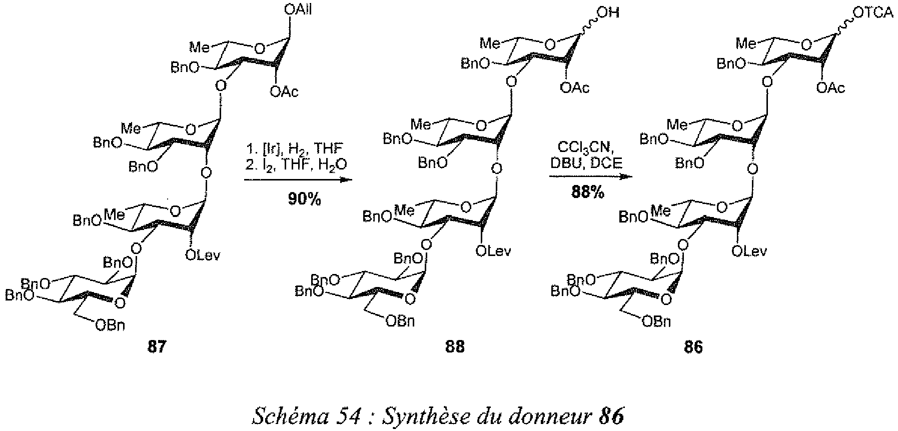

### 7. Pentasaccharide (E)ABCD

Le pentasaccharide **93** est obtenu par condensation de l'accepteur **19** et du donneur **86.** Or les résultats de la littérature,¹¹⁴ appuyés des résultats obtenus au laboratoire,^{115, 62} suggèrent que la réactivité d'un accepteur de structure proche de celle de **19,** peu soluble dû fait de la présence d'une fonction acétamide,³⁸ pourrait être améliorée en augmentant la température de la réaction lors du couplage. Suite à cette observation, le couplage entre **19** et **86** a donc été réalisé à 70°C dans le toluène, sur la base des études de solvants de glycosylation précédentes (Partie 2). De plus, comme le donneur est un tétrasaccharide « couteux », il est utilisé en défaut, de façon à assurer sa consommation totale. Lors du premier essai de couplage, le rendement en pentasaccharide **93** est de 43% et 36% de donneur hydrolysé **88** ont été récupéré, après purification. En revanche, en présence de 2,5 éq. d'accepteur, le donneur **86** est consommé en totalité et le pentasaccharide **93** est isolé avec un rendement de 78%. Ainsi les meilleurs résultats sont obtenus dans des conditions identiques à celle retenues dans une étude analogue.⁶²

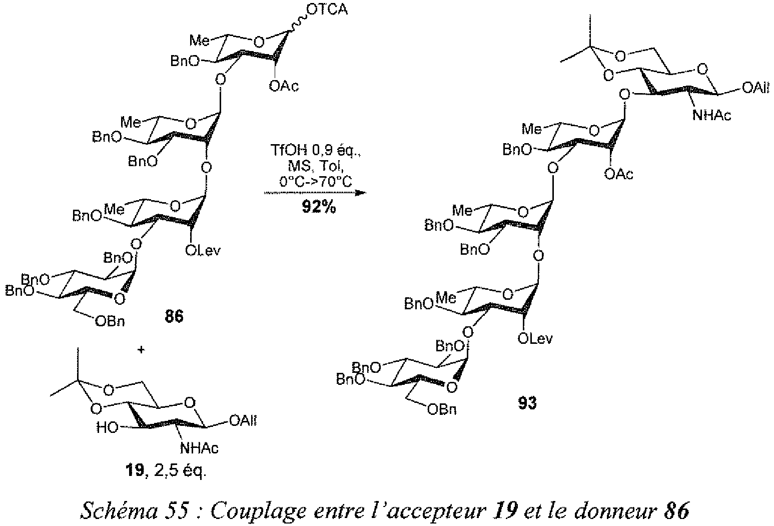

Le clivage de la fonction lévulinoyle de **93,** traité dans un mélange pyridine/AcOH en présence d'hydrazine monohydraté, fournit le monoacétate **94**¹⁰⁹ (85%), transformé en triol **95** après hydrolyse acide (92%) (Schéma 56).

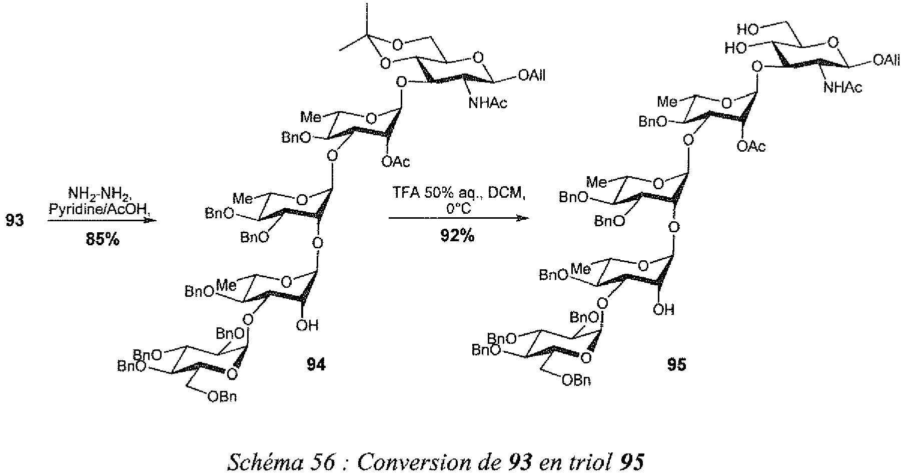

Par analogie, **93** traité par du NaOMe à reflux du méthanol fournit cette fois le diol **96** (94%), ensuite converti en tétraol **97** avec un rendement de 70% (Schéma 57).

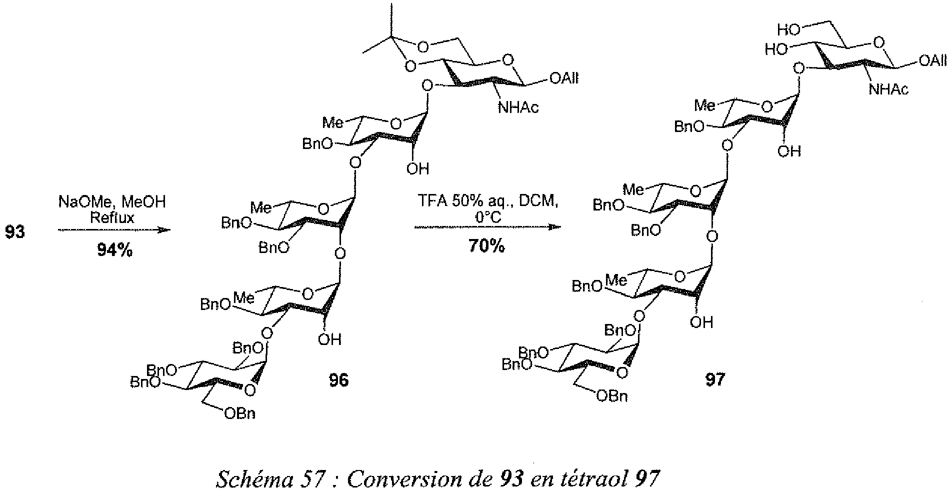

De la même façon que pour l'accès aux cibles ***XI*** et ***XII***, l'hydrogénolyse de **95** et **97** conduit aux pentasaccharides cibles ***XV*** et ***XVI*** sous forme de glycoside de propyle avec des rendements respectifs de 77% et 70% (Schéma 58).

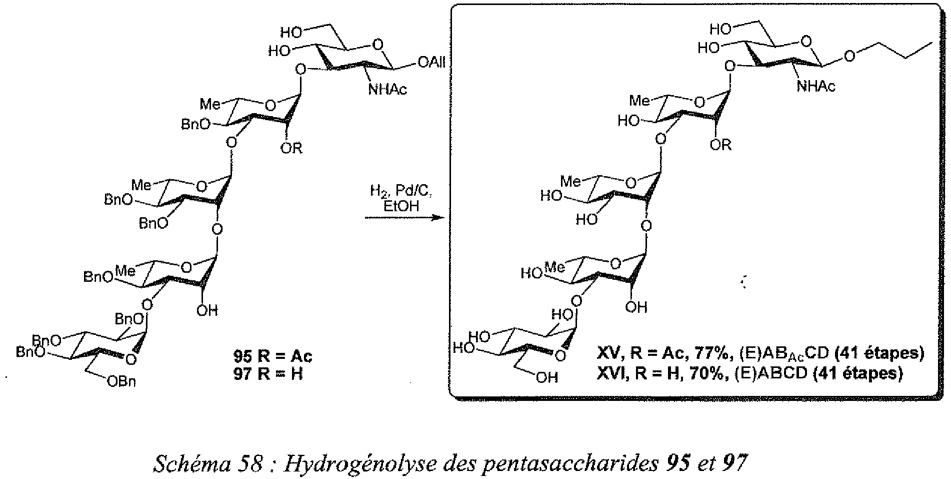

### Procédé 12 :

La description se rapporte au procédé de préparation du composé XVI (pentasaccharide (E)ABCD) tel que défini dans la liste L1 comprenant les étapes suivantes :
- condensation du monosaccharide accepteur **19** et du tétrasaccharide donneur **86** conduisant au pentasaccharide **93** de préférence la réaction de condensation est réalisée dans un solvant tel que le dichlorométhane, le 1,2-dichloroéthane, l'éther, le toluène, l'acétonitrile ou tout autre solvant équivalent, en présence d'un acide de Lewis tel que le TMSOTf ou l'acide triflique, préférentiellement, dans le toluène à 70°C (schéma 55) ;
- déprotection du pentasaccharide **93** conduisant au pentasaccharide **96,** de préférence par transestérification (schéma 57) ;
- clivage du groupement isopropylidène du pentasaccharide **96** par hydrolyse acide conduisant au pentasaccharide **97**, de préférence en solution aqueuse de TFA 50% dans le dichlorométhane à 0°C (schéma 57) ;
- hydrogénolyse du pentasaccharide **97** conduisant au pentasaccharide (E)ABCD, de préférence sous atmosphère d'hydrogène dans l'alcool en présence de palladium sur charbon (schéma 58).

### 8. Conclusion

Dans cette partie, la synthèse d'un tétrasaccharide, à la fois potentiellement accepteur et donneur, a été réalisée en 27 étapes avec un rendement de 13% (Schéma 38). Ce synthon clé a permis l'accès à six cibles, incluant deux tétra- et quatre pentasaccharides, mono-acétylé ou non correspondant aux fragments de l'antigène O des bactéries *S. flexneri* 3a et/ou X.

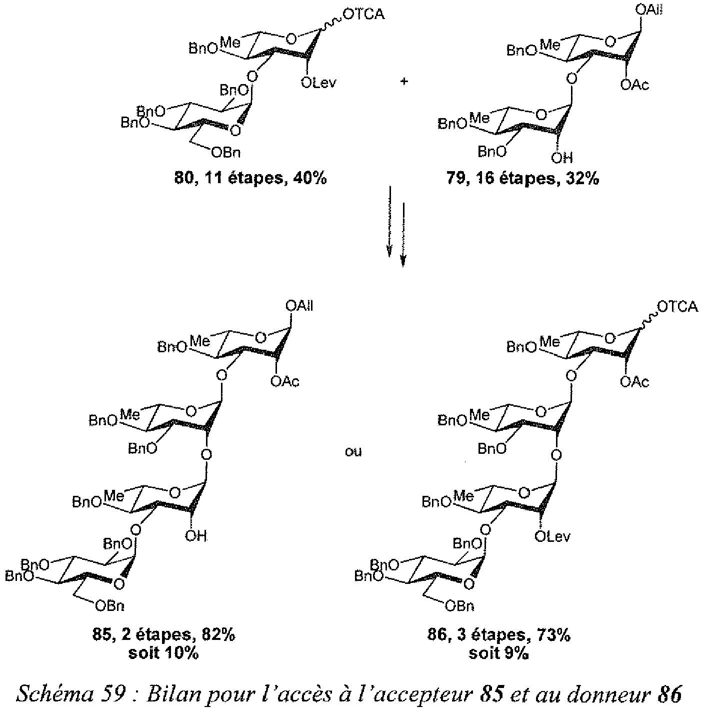

### Mise en oeuvre expérimentale des procédés 7 à 12

### Procédé 7 :

### ➢ 3,4-Di-O-benzyl-1,2-(méthyloxyéthylidène)-β-L-rhamnopyranose⁶⁷ 70 :

### ➢ 1,2,3,4-Tétra-O-acétyl-α,β-L-rhamnopyranose 71 :

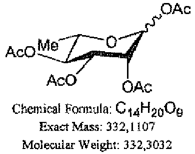

Du L(+)-rhamnose monohydraté commercial **27** (36,4 g, 200,0 mmol) est dissous dans un mélange pyridine/anhydride acétique (1/1, 200 mL). A la fin de l'addition, le milieu réactionnel est laissé sous agitation pendant une nuit à TA. Après avoir vérifié par un suivi CCM (DCM/MeOH, 8/2) la disparition de **27** (Rf= 0,2) et l'apparition d'un nouveau composé (Rf = 0,9), le milieu réactionnel est concentré à l'évaporateur rotatif puis coévaporé au Tol (3 x 100 mL) et au DCM (3 x 100 mL). Le résidu est séché à la pompe à palettes pour conduire au per-acétylé **71** sous forme d'un mélange d'anomères α/β (8/2) et d'une huile jaune (66,3 g, quant.).
**71_{α}** : Rf = 0,3 (Chex/EtOAc, 6/4).
RMN ¹H (CDCl₃), *δ* 5.98 (d, 1H, *J*_{1,2} = 1.9 Hz, H-1), 5.27 (dd, 1H, *J*_{3,4} = 10.1 Hz, *J*_{2,3} = 3.5 Hz, H-3), 5.22 (m, 1H, H-2), 5.09 (pt, 1H, *J*_{4,5} = 9.9 Hz, H-4), 3.91 (dq, 1H, H-5), 2.14, 2.13, 2.05, 1.98 (4s, 12H, H_{Ac}), 1.20 (d, 3H, *J*_{5,6} = 6.3 Hz, H-6).
RMN ¹³C (CDCl₃), *δ* 170.5, 170.2, 170.0, 168.6 (4C, C_{Ac}), 91.0 (C-1, ¹*J*_{CH} = 177.0 Hz), 71.8 (C-5_{β}), 70.8 (C-4), 69.1 (C-3), 69.1 (C-5), 69.0 (C-2), 21.4, 21.2, 21.0, 20.8 (8C, C_{Ac}), 17.8 (C-6_{α}), 17.7 (C-6).
**71_{β}** : Rf = 0,3 (Chex/EtOAc, 6/4).
RMN ¹H (CDCl₃), *δ* 5.84 (d, 1H, *J*_{1,2} = 1.2 Hz, H-1), 5.45 (m, 1H, H-2), 5.05 (m, 2H, H-3, H-4), 3.65 (dq, 1H, H-5), 2.20, 2.12, 2.07, 1.95 (4s, 12H, H_{Ac}), 1.26 (d, 3H, *J*_{5,6} = 6.2 Hz, H-6).
RMN ¹³C (CDCl₃), *δ* 170.3, 170.1, 169.9, 168.7 (4C, C_{Ac}), 91.0 (C-1, ¹*J*_{CH} = 162.6 Hz), 71.8 (C-5), 71.1, 70.6 (2C, C-3*, C-4*), 68.9 (C-2), 21.3, 21.1, 20.9, 20.7 (8C, C_{Ac}), 17.8 (C-6_{α}), 17.7 (C-6).

### ➢ Bromure de 2,3,4-O-acetyl-α-L-rhamnopyranose 72 :

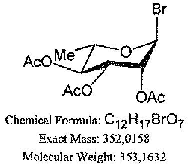

Le per-acétylé **71** (66,3 g, 200,0 mmol) est dissous dans du DCM (500 mL) puis est ajouté de l'acide bromhydrique (100 mL) et de l'anhydride acétique (23 mL) à 0°C. A la fin de l'addition, le milieu réactionnel est laissé sous agitation pendant une nuit à TA. Après avoir vérifié par un suivi CCM (Chex/EtOAc, 6/4) la disparition de **71** (Rf = 0,25) et l'apparition d'un nouveau composé (Rf = 0,35), l'excès d'acide bromhydrique est évaporé à la trompe à eau puis le milieu réactionnel est concentré à l'évaporateur rotatif et coévaporé au Tol (3 x 100 mL). Le résidu est séché à la pompe à palettes pour conduire au bromure **72** sous forme d'une huile jaune (70,6 g, quant.) qui est engagée directement dans l'étape suivante après contrôle par RMN ¹H.
Rf = 0,35 (Chex/EtOAc, 6/4).
RMN ¹H (CDCl₃), *δ* 6.2δ (d, 1H, *J*_{1,2} = 0.8 Hz, H-1), 5.66 (dd, 1H, *J*_{3,4} = 10.2 Hz, *J*_{2,3} = 3.4 Hz, H-3), 5.44 (m, 1H, H-2), 5.14 (pt, 1H, *J*_{4,5} = 10.0 Hz, H-4), 4.10 (dq, 1H, H-5), 2.14,2.06, 1.99 (3s, 9H, H_{Ac}), 1.25 (d, 3H, *J*_{5,6} = 6.3 Hz, H-6).
RMN ¹³C (CDl₃), *δ* 170.1, 170.0, 169.9 (3C, C_{Ac}), 84.1 (C-1, ¹*J*_{CH} = 184.3 Hz), 72.8 (C-2), 71.5 (C-5), 70.7 (C-4), 68.3 (C-3), 21.1, 21.0, 20.9 (3C, C_{Ac}), 17.3 (C-6).

### ➢ 3,4-Di-O-acétyl-1,2-(allyloxyéthylidène)-β-L-rhamnopyranose 73 :

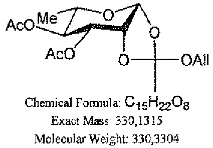

A une solution du bromure **72** (70,6 g, 200,0 mmol) dans un mélange alcool allylique/DCM (1/1, 400 mL) est ajouté de la 2,6-lutidine (28 mL). A la fin de l'addition, le milieu réactionnel est laissé sous agitation pendant une nuit à TA. Après avoir vérifié par un suivi CCM (Chex/EtOAc, 7/3) la disparition de **72** (Rf = 0,25) et l'apparition d'un nouveau composé (Rf = 0,35), le milieu réactionnel est repris avec un mélange DCM/H₂O (2/1, 150 mL) et la phase aqueuse est extraite au DCM (3 x 150 mL). Les phases organiques sont regroupées et lavées avec une solution d'acide citrique 10% (3 x 50 mL), une solution de NaHCO₃ₛₐₜ (3 x 50 mL), et une solution d'H₂O (3 x 30 mL) puis séchées sur filtre séparateur et concentrées à l'évaporateur rotatif. Le résidu est séché à la pompe à palettes pour conduire à l'orthoester **73** sous forme d'une huile jaune (66,0 g) qui est engagée directement dans l'étape suivante après contrôle par RMN ¹H.
Rf = 0,35 (Chex/EtOAc, 7/3).
RMN ¹H (CDCl₃), *δ* 5.85 (m, 1H, CH=), 5.39 (d, 1H, *J*_{1,2} = 2.4 Hz, H-1), 5.22 (m, 1H, *J*ₜᵣₐₙₛ = 17.2 Hz, =CH₂), 5.09 (m, 1H, *J*_{cis} = 11.8 Hz, =CH₂), 5.06 (dd, 1H, *J*_{2,3} = 3.9 Hz, *J*_{3,4} = 9.7 Hz, H-3), 5.01 (pt, 1H, *J*_{4,5} = 9.9 Hz, H-4), 4.56 (dd, 1H, *J*_{1,2} = 2.4 Hz, *J*_{2,3} = 3.9 Hz, H-2), 4.00 (m, 2H, H_{All}), 3.49 (dq, 1H, H-5), 2.07, 2.02 (2s, 6H, H_{Ac}), 1.72 (s, 3H, CH₃ₒᵣₜₕₒ), 1.20 (d, 3H, *J*_{5,6} = 6.2 Hz, H-6).
RMN ¹³C (CDl₃), *δ* 170.7, 170.3 (2C, C_{Ac}), 134.5 (CH=), 124.5 (Cₒᵣₜₕₒ), 116.9 (=CH₂), 97.4 (C-1, ¹*J*_{CH} = 175.0 Hz), 77.0 (C-2), 71.1 (C-3), 70.7 (C-4), 69.5 (C-5), 64.0 (C_{All}), 24.5 (CH₃ₒᵣₜₕₒ), 21.2, 21.0 (2C, C_{Ac}), 17.8 (C-6).

| | | |
|---|---|---|
| HRMS (ESI⁺) : | [M+Na]⁺ C₁₅H₂₂O₈Na | m/z théorique : 353.1212 |
| | | m/z mesurée : 353.1235 |

### ➢ 1,2-(Allyloxyéthylidène)-β-L-rhamnopyranose 74 :

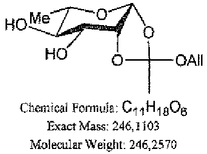

L'orthoester **73** (66,0 g, 200,0 mmol) est dissous dans du MeOH (250 mL) puis du carbonate de potassium (1,1 g, 8,0 mmol, 0.04 éq.) est ajouté au milieu réactionnel. Après 3 h sous agitation, le suivi par CCM (Chex/EtOAc, 7/3 et DCM/MeOH, 95/5) indique la disparition de **73** (Rf = 0,4 et 1, respectivement) et l'apparition d'un produit plus polaire (Rf = 0 et 0,4, respectivement). Le milieu réactionnel est évaporé sous pression réduite pour conduire au diol **74** sous forme d'un solide jaunâtre (49,2 g) qui est engagée directement dans l'étape suivante après contrôle par RMN ¹H.
Rf = 0,5 (DCM/MeOH, 9/1).
RMN ¹H (CDCl₃), *δ* 5.85 (m, 1H, CH=), 5.39 (d, 1H, *J*_{1,2} = 2.0 Hz, H-1), 5.26 (m, 1H, *J*ₜᵣₐₙₛ = 17.2 Hz, =CH₂), 5.15 (m, 1H, *J*_{cis} = 10.4 Hz, =CH₂), 4.48 (m, 1H, H-2), 4.07 (m, 2H, H_{All}), 3.70 (dd, 1H, *J*_{3,4} = 9.2 Hz, *J*_{2,3} = 4.0 Hz, H-3), 3.42 (pt, 1H, *J*_{4,5} = 9.2 Hz, H-4), 3,30 (dq, 1H, H-5), 1.71 (s, 3H, CH₃ₒᵣₜₕₒ), 1.31 (d, 3H, *J*_{5,6} = 6.0 Hz, H-6). RMN ¹³C (CDl₃), *δ* 134.4 (CH=), 124.0 (Cₒᵣₜₕₒ), 116.8 (=CH₂), 97.7 (C-1, ¹*J*_{CH} = 175.1 Hz), 79.6 (C-2), 72.9 (C-3), 72.7 (C-4), 71.1 (C-5), 64.0 (C_{All}), 25.5 (CH₃ₒᵣₜₕₒ), 17.8 (C-6).

### ➢ 3,4-Di-O-benzyl-1,2-(allyloxyéthylidène)-β-L-rhamnopyranose 75 :

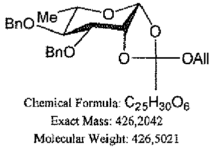

A une solution de diol **74** (49,2 g, 200,0 mmol) dans du DMF (300 mL) est ajouté par petites portions du NaH 60% dans l'huile (24,0 g, 600,0 mmol, 3 éq.) à 0°C. Le milieu réactionnel est alors laissé sous agitation pendant 15 min puis replacé à 0°C. Du bromure de benzyle (57,3 mL, 480,0 mmol, 2,4 éq.) est ajouté goutte à goutte puis le milieu réactionnel est agité pendant une nuit à TA. Après avoir vérifié par un suivi CCM (DCM/MeOH, 9/1 et Chex/EtOAc, 7/3) la disparition de **73** (Rf = 0,5 et 0, respectivement) et l'apparition d'un nouveau composé (Rf= 0,9 et 0,5, respectivement), le milieu réactionnel est placé à 0°C et du MeOH (30 mL) est ajouté. Le milieu réactionnel est alors repris par un mélange EtOAc/H₂O (3/1, 400mL) et la phase aqueuse est extraite à l'EtOAc (3 x 150 mL). Les phases organiques sont regroupées et lavées avec une solution de NaClₛₐₜ (3 x 50 mL), et une solution d'H₂O (3 x 50 mL) puis séchées sur Na₂SO₄ et concentrées à l'évaporateur rotatif. L'huile obtenue est purifiée par chromatographie sur gel de silice (Chex/EtOAc, 9/1→7/3) pour conduire au di-*O*-benzyle **75** sous forme d'une huile incolore (64,8 g, 76%, 5 étapes).
Rf = 0,5 (Chex/EtOAc, 7/3).
RMN ¹H (CDCl₃), *δ* 7.47-7.35 (m, 10H, CH_{Ph}), 5.96 (m, 1H, CH=), 5.33 (m, 1R, *J*ₜᵣₐₙₛ = 17.2 Hz, =CH₂), 5.31 (d, 1H, *J*_{1,2} = 1.7 Hz, H-1), 5.22 (m, 1H, *J*_{cis} = 10.4 Hz, =CH₂), 4.99 (d, 1H, *J* = 10.8 Hz, H_{Bn}), 4.80 (m, 2H, H_{Bn}), 4.71 (d, 1H, *J* = 10.8 Hz, H_{Bn}), 4.41 (dd, 1H, *J*_{1,2} = 1.7 Hz, *J*_{2,3} = 4.1 Hz, H-2), 4.08 (m, 2H, H_{All}), 3.73 (dd, 1H, *J*_{3,4} = 9.1 Hz, *J*_{2,3} = 4.1 Hz, H-3), 3.52 (pt, 1H, *J*_{4,5} = 9.1 Hz, H-4), 3.37 (dq, 1H, H-5), 1.80 (s, 3H, CH₃ₒᵣₜₕₒ), 1.34 (d, 3H, *J*_{5,6} = 6.2 Hz, H-6).
RMN ¹³C (CDl₃), *δ* 138.7-138.3 (C_{Ph}), 135.0 (CH=), 129.0-128.3 (CH_{Ph}), 124.1 (Cₒᵣₜₕₒ), 116.9 (=CH₂), 97.8 (C-1, ¹*J*_{CH} = 173.6 Hz), 79.9 (C-4), 79.6 (C-3), 77.5 (C-2), 76.6, 72.6 (2C, C_{Bn}), 70.7 (C-5), 64.0 (C_{All}), 25.4 (CH₃ₒᵣₜₕₒ), 18.4 (C-6).

| | | |
|---|---|---|
| HRMS (ESI⁺) : | [M+Na]⁺ C₂₅H₃₀O₆Na | m/z théorique : 449.1940 |
| | | m/z mesurée: 449.1980 |

### ➢ Allyl 2-O-acétyl-3,4-di-O-benzyl-α-L-rhamnopyranoside 76 :

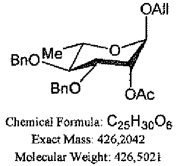

A une solution du di-*O*-benzyle **75** (53,8 g, 126,0 mmol) dans du DCM (300 mL) est ajouté du TMSOTf (4,5 mL, 25,2 mmol, 0,2 éq.), en présence de tamis moléculaire 4 Å (55 g), agitée sous argon à 0°C. Après 3 h sous agitation à TA, le suivi par CCM (Chex/EtOAc, 7/3) indique la disparition de **75** (Rf = 0,5) et l'apparition d'un nouveau composé moins polaire (Rf= 0,6). La réaction est stoppée par l'addition de triéthylamine (2 mL) puis le milieu réactionnel est filtré et concentré à l'évaporateur rotatif. L'huile obtenue est purifiée par chromatographie sur gel de silice (Chex/EtOAc, 9/1→7/3) pour conduire au glycoside d'allyle **76** sous forme d'une huile incolore (47,5 g, 88%).
Rf = 0,6 (Chex/EtOAc, 7/3).
RMN ¹H (CDCl₃), *δ* 7.42-7.33 (m, 10H, CH_{Ph}), 5.90 (m, 1H, CH=), 5.42 (dd, 1H, *J*_{1,2} = 1.8 Hz, *J*_{2,3} = 3.4 Hz, H-2), 5.29 (m, 1H, *J*ₜᵣₐₙₛ = 17.2 Hz, =CH₂), 5.21 (m, 1H, *J*_{cis} = 10.4 Hz, =CH₂), 4.95 (d, 1H, *J* = 10.8 Hz, H_{Bn}), 4.80 (d, 1H, *J*_{1,2} = 1.7 Hz, H-1), 4.73 (d, 1H, *J* = 11.2 Hz, H_{Bn}), 4.64 (d, 1H, *J* = 10.8 Hz, H_{Bn}), 4.56 (d, 1H, *J* = 11.2 Hz, H_{Bn}), 4.19 (m, 1H, H_{All}), 4.01-3.97 (m, 2H, H_{All}, H-3), 3.37 (dq, 1H, H-5), 3.47 (pt, 1H, *J*_{4,5} = 9.4 Hz, H-4), 2.17 (s, 3H, H_{Ac}), 1.36 (d, 3H, *J*_{5,6} = 6.2 Hz, H-6).
RMN ¹³C (CDl₃), *δ* 170.7 (C_{Ac}), 139.0-138.5 (C_{Ph}), 134.0 (CH=), 128.9-128.1 (CH_{Ph}), 118.0 (=CH₂), 97.3 (C-1, ¹*J*_{CH} = 169.6 Hz), 80.5 (C-4), 78.6 (C-3), 75.8, 72.2 (2C, C_{Bn}), 69.5 (C-2), 68.4 (C_{All}), 68.2 (C-5), 21.5 (C_{Ac}), 18.4 (C-6).

| | | |
|---|---|---|
| HRMS (ESI⁺) : | [M+Na]⁺ C₂₅H₃₀O₆Na | m/z théorique : 449.1940 |
| | | m/z mesurée : 449.1951 |

### ➢ Allyl 3,4-di-O-benzyl-α-L-rhamnopyranoside^{96, 102} 58 :

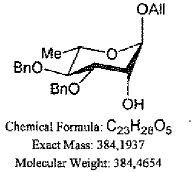

**Voie 1** : Après addition de NaOMe 0,5 M (222,7 mL, 111,0 mmol, 1 éq.) au glycoside d'allyle **76** (47,5 g, 111,0 mmol) en solution dans du MeOH (150 mL), le milieu réactionnel est agité pendant 2 h et son évolution est suivie par CCM (Chex/EtOAc, 7/3). Après avoir observé la disparition de **76** (Rf = 0,6) et l'apparition d'un produit plus polaire (Rf = 0,35), le milieu réactionnel est neutralisé par addition de résine échangeuse d'ion DOWEX (H⁺), puis filtré sur fritté. La résine est lavée avec un mélange DCM/MeOH et les solvants sont évaporés. L'huile jaune obtenue est purifiée par chromatographie sur gel de silice (Chex/EtOAc, 8/2→7/3) pour conduire à l'alcool **58** sous forme d'une huile incolore (39,2 g, 92% soit 61% à partir du L-Rhamnose commercial **27).**
**Voie 2** : Le brut de **76** (65,2 g, 200,0 mmol) est repris par de l'EtOAc (300 mL). La phase organique est lavée avec une solution de HCl 10% (3 x 50 mL), une solution de NaClₛₐₜ (3 x 50 mL), et une solution d'H₂O (3 x 50 mL) puis séchées sur Na₂SO₄ et concentrées à l'évaporateur rotatif. Du chlorure d'acétyle est ajouté (35,5 mL, 500,0 mmol, 2,5 éq.) par portions à d'alcool allylique (300 mL) à 0°C durant 15 min, puis le brut précédent est ajouté au milieu réactionnel. Le milieu réactionnel est ensuite chauffé à 70°C pendant 2,5 h puis à 40°C durant une nuit. Le suivi de la réaction par CCM (Chex/EtOAc, 7/3) montre la disparition de **76** (Rf = 0,25) et la présence d'un produit majoritaire moins polaire (Rf = 0,35). Le milieu réactionnel est refroidi à 0°C, neutralisé par addition de NaHCO₃ puis filtré sur célite et enfin concentré à l'évaporateur rotatif. Les volatiles sont coévaporés avec du Tol (2 x 100 mL) pour conduire à une huile jaune. L'huile jaune est purifiée par chromatographie sur gel de silice (Chex/EtOAc, 812→1/1) pour conduire à l'alcool **58** sous forme d'une huile incolore (55 g, 72% à partir du L-Rhamnose commercial **27**).
**Voie 3** : Partie 2

### ➢ Allyl (3,4-di-O-benzyl-α-L-rhamnopyranosyl)-(1→3)-2-O-acétyl-4-O-benzyl-α-L-rhamnopyranoside 79 :

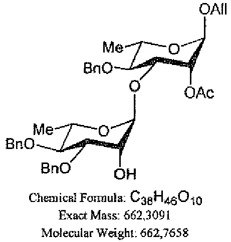

Le glycoside d'allyle **61** (Partie 2) (172 mg, 230 µmol) est dissous dans un mélange pyridine/acide acétique (3/2, 5mL). De l'hydrazine monohydraté (55 µL, 1,1 mmol, 5 éq.) est ajouté goutte à goutte au milieu réactionnel. Après 30 min d'agitation à TA, le suivi par CCM (Tol/EtOAc, 8/2) indique la disparition de **61** (Rf = 0,45) et l'apparition d'un produit plus polaire (Rf = 0,3). Le milieu réactionnel est alors repris à l'eau froide (10 mL) et la phase aqueuse est extraite rapidement au DCM (3 x 50 mL). Les phases organiques sont regroupées et lavées avec une solution de NaClₛₐₜ, filtrées sur filtre séparateur de phase et concentrées à l'évaporateur rotatif. L'huile obtenue est purifiée par chromatographie sur gel de silice (Tol/EtOAc, 9/1→75/25) pour obtenir l'accepteur **79** sous forme une huile incolore (121 mg, 81%).
Rf= 0,3 (Tol/EtOAc, 8/2).
RMN ¹H (CDCl₃), *δ* 7.40-7.28 (m, 15H, CH_{Ph}), 5.89 (m, 1H, CH=), 5.29 (m, 1H, *J*ₜᵣₐₙₛ = 17.2 Hz, =CH₂), 5.21 (m, 1H, *J*_{cis} = 10.4 Hz, =CH₂), 5.17 (m, 1H, H-2_{C}), 5.10 (d, 1H, *J*_{1,2} = 1.5 Hz, H-1_{B}), 4.88 (d, 1H, *J* = 11.0 Hz, H_{Bn}), 4.80 (d, 1H, *J*_{1,2} = 1.6 Hz, H-1_{C}), 4.87 (d, 1H, *J* = 11.0 Hz, H_{Bn}), 4.69-4.60 (d, 4H, H_{Bn}), 4.18-4.13 (m, 2H, H-3_{C}, H_{All}), 4.01-3.96 (m, 2H, H-2_{B}, H_{All}), 3.83-3.74 (m, 3H, H-3_{B}, H-5_{B}, H-5_{C}), 3.48 (pt, 1H, *J*_{3,4} = 9.1 Hz, H-4_{B}), 3.46 (pt, 1H, *J*_{3,4} = 9.4 Hz, H-4_{C}), 2.14 (s, 3H, H_{Ac}), 1.31 (m, 6H, H-6_{B}, H-6_{C}).
RMN ¹³C (CDCl₃), *δ* 170.7 (C_{Ac}), 138.8-138.3 (C_{Ph}), 133.9 (CH=), 129.4-128.1 (CH_{Ph}), 117.9 (=CH₂), 101.8 (C-1_{B}, ¹*J*_{CH} = 171.0 Hz), 96.6 (C-1_{C}, ¹*J*_{CH} = 169.2 Hz), 80.7 (C-4_{C}), 80.2 (C-4_{B}), 80.1 (C-3_{B}), 78.5 (C-3_{C}), 75.9, 75.6 (2C, C_{Bn}), 72.9 (C-2_{C}), 72.4 (C_{Bn}), 69.4 (C-2_{B}), 68.7 (C-5_{B}), 68.6 (C_{All}), 68.1 (C-5_{C}), 21.5 (C_{Ac}), 18.3, 18.2 (C-6_{B}*, C-6_{C}*).

| | | |
|---|---|---|
| HRMS (ESI⁺) : | [M+Na]⁺ C₃₈H₄₆O₁₀Na | m/z théorique : 685.2989 |
| | | m/z mesurée : 685.2993 |
| | [M+NH₄]⁺ C₃₈H₄₆O₁₀NH₄ | m/z théorique : 680.3434 |
| | | m/z mesurée : 680.3472 |
| | [M+K]⁺ C₃₈H₄₆O₁₀K | m/z théorique : 701.2728 |
| | | m/z mesurée : 701.2731 |

### ➢ 5-Chloro-5-méthyl-dihydro-furan-2-one¹¹³ 82 :

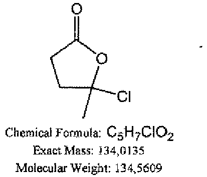

A une solution d'acide lévulinique (5,0 mL, 48,6 mmol, 1 éq.) est ajouté lentement du chlorure de thionyle (3,9 mL, 53,0 mmol, 1,1 éq.). Le milieu réactionnel est agité à TA pendant 1h puis porté à 50°C pendant 30 min. L'excès de chlorure de thionyle est alors évaporé. L'huile obtenue contrôlé par RMN ¹H et les données obtenues sont identiques à la littérature.¹¹³

### ➢ Anhydride Lévulinique¹¹² 83 :

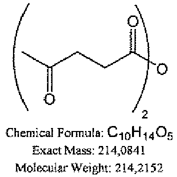

A une solution d'acide lévulinique (79,5 mL, 780,0 mmol, 2 éq.) dans le THF (500 mL) est ajouté de la DCC (80 g, 390 mmol) et le milieu réactionnel est agité à TA pendant 1 nuit. La DCU formé est alors filtrée sur célite et le filtrat est concentré à l'évaporateur rotatif. Le solide blanc obtenu (89,0 g) est engagé directement dans l'étape suivante après contrôle par RMN ¹H.

### ➢ Allyl (2,3,4,6-tétra-O-benzyl-α-D-glucopyranosyl)-(1→3)-4-O-benzyl-2-O-lévulinoyl-α-L-rhamnopyranoside 81 :

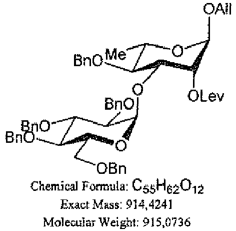

L'alcool **21** (18,6 g, 22,8 mmol) est dissous dans de la pyridine (150 mL) puis de la DMAP (13,9 g, 114 mmol, 5 éq.) est ajouté et le milieu réactionnel est porté à 50°C. De l'anhydride lévulinique **83** (48,8 g, 228,0 mmol, 10 éq.) dissout dans la pyridine (200 mL) est ajouté goutte à goutte pendant 1h30. Après 3 h, le suivi par CCM (Chex/EtOAc, 7/3) indique la disparition de **21** (Rf = 0,40) et l'apparition d'un produit plus polaire (Rf = 0,3). Le milieu réactionnel est alors concentré à l'évaporateur rotatif. L'huile obtenue est purifiée par chromatographie sur gel de silice (Chex/EtOAc, 9/1→75/25) pour obtenir l'ester de lévulinoyle **81** sous forme d'une huile incolore (19,7 g, 95%).
Rf = 0,3 (Chex/EtOAc, 7/3).
RMN ¹H (CDCl₃), *δ* 7.40-7.10 (m, 25H, CH_{Ph}), 5.87 (m, 1H, CH=), 5.39 (m, 1H, H-2_{A}), 5.28 (m, 1H, *J*ₜᵣₐₙₛ = 17.2 Hz, =CH₂), 5.19 (d, 1H, *J*_{1,2} = 5.6 Hz, H-1_{E}), 5.28 (m, 1H, *J*_{cis} = 10.3 Hz, =CH₂), 5.02 (d, 1H, *J* = 11.0 Hz, H_{Bn}), 4.96-4.85 (m, 3H, H_{Bn}), 4.78 (d, 1H, *J*_{1,2} = 1.8 Hz, H-1_{A}), 4.78-4.59 (m, 4H, H_{Bn}), 4.50 (d, 1H, *J* = 11.0 Hz, H_{Bn}), 4.36 (d, 1H, *J* = 12.0 Hz, H_{Bn}), 4.26 (dd, 1H, *J*_{2,3} = 3.2 Hz, *J*_{3,4} = 9.6 Hz, H-3_{A}), 4.18-4.04 (m, 3H, H_{All}, H-3_{E}, H-5_{E}), 3.97 (m, 1H, H_{All}), 3.80 (m, 1H, H-5_{A}), 3.77 (pt, 1H, *J*_{4,5} = 9.2 Hz, H-4_{E}), 3.64-3.52 (m, 4H, H-2_{E}, H-6a_{E}, H-4_{A}, H-6b_{E}), 2.55 (m, 4H, 2CH_{2Lev}), 2.09 (s, 3H, CH_{3Lev}), 1.40 (d, 3H, *J*_{5,6} = 6.2 Hz, H-6_{A}).
RMN ¹³C (CDCl₃), *δ* 206.5 (C_{Lev}), 172.5 (C_{Lev}), 139.2-138.1 (C_{Ph}), 134.0 (CH=), 129.0-127.8 (CH_{Ph}), 117.8 (=CH₂), 97.0 (C-1_{E}, ¹*J*_{CH} = 170.2 Hz), 93.3 (C-1_{A}, ¹*J*_{CH} = 167.5 Hz), 82.5 (C-3_{E}), 80.3 (C-4_{A}), 80.0 (C-2_{E}), 78.3 (C-4_{E}), 76.5, 75.9, 75.3, 73.7, 72.8 (5C, C_{Bh}), 72.8 (C-3_{A}), 70.6 (C-5_{E}), 68.7 (C-6_{E}), 68.6 (C-2_{A}), 68.5 (C_{All}), 68.4 (C-5_{A}), 38.2 (CH_{2Lev}), 30.0 (CH_{3Lev}), 28.4 (CH_{2Lev}), 18.3 (C-6_{A}).

| | | |
|---|---|---|
| HRMS (ESI⁺) : | [M+Na]⁺ C₅₅H₆₂O₁₂Na | m/z théorique : 937.4139 |
| | | m/z mesurée : 937.4109 |
| | [M+NH₄]⁺ C₅₅H₆₂O₁₂NH₄ | m/z théorique : 932.4585 |
| | | m/z mesurée : 932.4542 |

### ➢ (2,3,4,6-Tétra-O-benzyl-α-D-glucopyranosyl)-(1→3)-4-O-benzyl-2-O-évulinoyl-α/β-L-rhamnopyranose 84 :

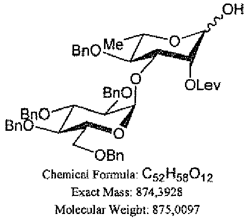

Le (1,5-cyclooctadiènebis(méthyldiphénylphosphine)iridium(I)hexafluorophosphate) (250 mg) est dissous dans du THF (100 mL) sous agitation. Le milieu subit 5 cycles dégazage vide/argon, 5 cycles dégazage vide/hyrdogène puis est mis sous hydrogène pendant 15 min. Le milieu subit alors une seconde fois 5 cycles dégazage vide/argon. La solution d'ester de lévulinoyle **81** (8,5 g, 9,3 mmol) dans du THF (15 mL) est transféré sur la solution de catalyseur activé. Le suivi par CCM (Chex/EtOAc, 7/3) montre la disparition de **81** (Rf= 0,3) et l'apparition d'un produit un peu moins polaire (Rf = 0,35).

Du diiode (4,7 g, 18,6 mmol, 2 éq.) en solution dans un mélange THF/H₂O (8/2, 50 mL) est ajouté au milieu réactionnel. Le suivi par CCM (Chex/EtOAc, 7/3 et DCM/MeOH, 98/2) indique la disparition de l'intermédiaire (Rf = 0,35 et 0,85, respectivement) et l'apparition d'un nouveau composé beaucoup plus polaire (Rf= 0,05 et 0,2, respectivement). Pour stopper la réaction, une solution aqueuse de NaHSO₃ 10% (40 mL) est ajoutée par fractions au milieu jusqu'à disparition de la coloration noire. Le milieu réactionnel est repris avec du DCM (100 mL) et la phase aqueuse est extraite au DCM (3 x 100 mL). Les phases organiques sont regroupées et lavées avec une solution de NaClₛₐₜ (3 x 50 mL), avec de l'H₂O (3 x 50 mL) puis séchées sur filtre séparateur de phases et concentrées à l'évaporateur rotatif. L'huile jaune obtenue est purifiée par chromatographie sur gel de silice (DCM/MeOH, 99/1→9/1) pour obtenir un mélange d'anomères α/β de l'hémiacétal **83** dans les proportions 7/3 sous forme d'une huile jaune (7,5 g, 92%).
Rf = 0,2 (DCM/MeOH, 98/2).
**83_{α}** : RMN ¹H (CDCl₃), *δ* 7.43-7.14 (m, 25H, CH_{Ph}), 5.43 (m, 1H, H-2_{A}), 5.25 (d, 1H, *J*_{1,2} = 3.4 Hz, H-1_{E}), 5.15 (d, 1H, *J*_{1,2} = 1.7 Hz, H-1_{A}), 5.07-4.87 (m, 3H, H_{Bn}), 4.80 (d, 1H, *J,* = 12.2 Hz, H_{Bn}), 4.74-4.59 (m, 3H, H_{Bn}), 4.52 (d, 1H, *J* = 11.0 Hz, H_{Bn}), 4.46-4.36 (d, 2H, H_{Bn}), 4.34 (m, 1H, H-3_{A}), 4.20-4.10 (m, 2H, H-3_{E}, H-5_{E}), 4.05 (m, 1H, H-5_{A}), 3.78-3.73 (m, 2H, OH, H-4_{E}), 3.70-3.55 (m, 4H, H-2_{E}, H-6a_{E}, H-4_{A}, H-6b_{E}), 2.55 (m, 4H, 2CH_{2Lev}), 2.11 (s, 3H, CH_{3Lev}), 1.41 (d, 3H, *J*_{5,6} = 6.2 Hz, H-6_{A}).
RMN ¹³C (CDCl₃), *δ* 206.9 (C_{Lev}), 172.6 (C_{Lev}), 139.2-138.9 (C_{Ph}), 129.0-127.8 (CH_{Ph}), 93.0 (C-1_{E}, ¹*J*_{CH} = 169.0 Hz), 92.4 (C-1_{A}, ¹*J*_{CH} = 170.7 Hz), 82.5 (C-3_{E}), 80.3 (C-4_{A}), 79.9 (C-2_{E}), 78.3 (C-4_{E}), 76.5, 75.9, 75.4, 73.6, 73.2 (5C, C_{Bn}), 72.3 (C-3_{A}), 70.5 (C-5_{E}), 69.0 (C-2_{A}), 68.7 (C-6_{E}), 68.3 (C-5_{A}), 38.3 (CH_{2Lev}), 30.1 (CH_{3Lev}), 28.6 (CH_{2Lev}), 18.5 (C-6_{A}).
**83_{β}** : RMN ¹H (CDCl₃), *δ* 7.43-7.14 (m, 25H, CH_{Ph}), 5.62 (d, 1H, *J*_{2,3} = 2.7 Hz, H-2_{A}), 5.38 (d, 1H, *J*_{1,2} = 3.4 Hz, H-1_{E}), 5.07-4.87 (m, 3H, H_{Bn}), 4.80 (d, 1H, *J* = 12.2 Hz, H_{Bn}), 4.79 (s, 1H, H-1_{A}), 4.74-4.59 (m, 3H, H_{Bn}), 4.53 (d, 1H, *J* = 11.0 Hz, H_{Bn}), 4.46-4.36 (d, 2H, H_{Bn}), 4.20-4.10 (m, 2H, H-3_{E}, H-5_{E}), 4.00 (m, 1H, H-3_{A}), 3.75 (m, 1H, H-4_{E}), 3.70-3.55 (m, 3H, H-2_{E}, H-6a_{E}, H-6b_{E}), 3.53 (pt, 1H, *J*_{4,5} = 9.3 Hz, H-4), 3.44 (m, 1H, H-5_{A}), 2.55 (m, 4H, 2CH_{2Lev}), 2.14 (s, 3H, CH_{3Lev}), 1.47 (d, 3H, *J*_{5,6} = 6.0 Hz, H-6_{A}).
RMN ¹³C (CDCl₃), *δ* 208.6 (C_{Lev}), 173.1 (C_{Lev}), 139.2-138.9 (C_{Ph}), 129.0-127.8 (CH_{Ph}), 93.6 (C-1_{A}, ¹*J*_{CH} = 160.9 Hz), 92.6 (C-1_{E}, ¹*J*_{CH} = 169.1 Hz), 82.4 (C-3_{E}), 79.6 (C-4_{A}), 80.0 (C-2_{E}), 78.4 (C-4_{E}), 76.6, 76.0, 75.5 (3C, C_{Bn}), 74.2 (C-3_{A}), 73.6, 73.4 (2C, C_{Bn}), 72.3 (C-5_{A}), 70.5 (C-5_{E}), 69.0 (C-2_{A}), 68.8 (C-6_{E}), 39.1 (CH_{2Lev}), 30.0 (CH_{3Lev}), 28.5 (CH_{2Lev}), 18.5 (C-6_{A}).

| | | |
|---|---|---|
| HRMS (ESI⁺) : | [M+Na]⁺ C₅₂H₅₈O₁₂Na | m/z théorique : 897.3826 |
| | | m/z mesurée : 897.3777 |
| | [M+NH₄]⁺ C₅₂H₅₈O₁₂NH₄ | m/z théorique : 892.4272 |
| | | m/z mesurée : 892.4234 |

### ➢ Trichloracétimidate de (2,3,4,6-tétra-O-benzyl-α-D-glucopyranosyl)-(1→3)-4-O-benzyl-2-O-lévulinoyl-α/β-L-rhamnopyranose 80 :

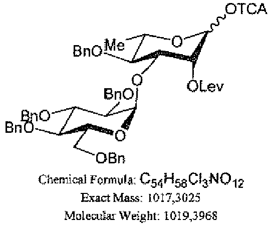

L'hémiacétal **83** (7,1 g, 8,1 mmol) est solubilisé dans du DCE (30 mL) et agité sous argon à -5°C puis de la DBU (340 µL, 2,3 mmol, 0,28 éq.) et du trichloroacétonitrile (4,0 mL, 40,5 mmol, 5 éq.) sont ajoutés. Le milieu réactionnel est agité à cette température pendant 15 min puis il est déposé, sans aucun traitement, sur une colonne de gel de silice (flash chromatographie, Chex/EtOAc+5% NEt₃, 7/3→1/1) pour obtenir le trichloroacétimidate **80** sous forme d'une huile jaune (8,0 g, 97%).
Rf = 0,35 (Chex/EtOAc, 7/3).
RMN ¹H (CDCl₃), *δ* 8.72 (s, 1H, NH), 7.42-7.14 (m, 25H, CH_{Ph}), 6.24 (d, 1H, *J*_{1,2} = 2.0 Hz, H-1_{A}), 5.61 (m, 1H, H-2_{A}), 5.27 (d, 1H, *J*_{1,2} = 3.4 Hz, H-1_{E}), 5.05-4.99 (m, 2H, H_{Bn}), 4.92-4.88 (m, 2H, H_{Bn}), 4.80 (d, 1H, *J* = 12.0 Hz, H_{Bn}), 4.74-4.53 (m, 4H, H_{Bn}), 4.42 (d, 1H, *J* = 12.2 Hz, H_{Bn}), 4.34 (dd, 1H, *J*_{2,3} = 3.1 Hz, *J*_{3,4} = 9.7 Hz, H-3_{A}), 4.16-4.06 (m, 2H, H-3_{E}, H-5_{E}), 4.02 (m, 1H, H-5_{A}), 3.79 (pt, 1H, *J*_{3,4} = 9.3 Hz, H-4_{E}), 3.71-3.62 (m, 3H, H-4_{A}, H-6a_{E}, H-2_{E}), 3.52 (m, 1H, H-6b_{E}), 2.59 (m, 4H, 2CH_{2Lev}), 2.11 (s, 3H, CH_{3Lev}), 1.45 (d, 3H, *J*_{5,6} = 6.2 Hz, H-6_{A}).
RMN ¹³C (CDCl₃), *δ* 206.3 (C_{Lev}), 172.5 (C_{Lev}), 160.5 (C=NH), 139.1-137.9 (C_{Ph}), 129.2-127.8 (CH_{Ph}), 95.4 (C-1_{A}, ¹*J*_{CH} = 178.9 Hz), 93.4 (C-1_{E}, ¹*J*_{CH} = 168.3 Hz), 91.3 (CCl₃), 82.4 (C-3_{E}), 79.8 (C-2_{E}), 79.5 (C-4_{A}), 78.2 (C-4_{E}), 76.8, 75.9, 75.4, 73.8, 73.2 (5C, C_{Bn}), 72.5 (C-3_{A}), 71.4 (C-5_{A}), 70.8 (C-5_{E}), 68.4 (C-6_{E}), 66.9 (C-2_{A}), 38.2 (CH_{2Lev}), 30.0 (CH_{3Lev}), 28.4 (CH_{2Lev}), 18.4 (C-6_{A}).

### Allyl (2,3,4,6-tétra-O-benzyl-α-D-glucopyranosyl)-(1→3)-(4-O-benzyl-2-O-lévulinoyl-α-L-rhamnopyranosyl)-(1→2)-(3,4-di-O-benzyl-α-L-rhamnopyranosyl)-(1→3)-2-O-acétyl-4-O-benzyl-α-L-rhamnopyranoside 87 :

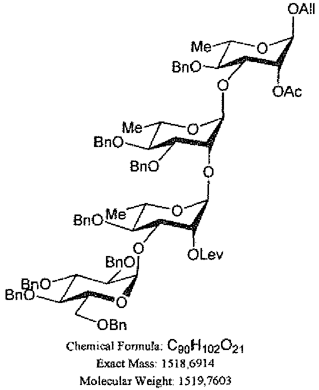

Du TMSOTf (340,0 µL, 1,9 mmol, 0,3 éq.) est additionné à une solution d'accepteur **79** (4,2 g, 6,3 mmol) et de donneur **80** (8,0 g, 7,8 mmol, 1,2 éq.) dans du Tol (100 mL), en présence de tamis moléculaire 4 Å (5,3 g), agitée sous argon à -78°C. Après 1 h à cette température, le bain éthanol-carboglace est retiré et l'agitation est poursuivie pendant 30 min, temps au bout duquel le suivi par CCM (Tol/EtOAc, 8/2, Chex/EtOAc, 7/3) indique la disparition de **79** (Rf = 0,35 et 0,4, respectivement) et l'apparition d'un nouveau composé (Rf = 0,65 et 0,45, respectivement). La réaction est stoppée par addition de triéthylamine (0,5 mL) puis le milieu réactionnel est filtré et concentré à l'évaporateur rotatif. L'huile jaune obtenue est purifiée par chromatographie sur gel de silice (Tol/EtOAc, 95/5→85/15) pour obtenir le glycoside d'allyle **87** une huile incolore (8,8 g, 92%).
Rf = 0,45 (Chex/EtOAc, 7/3).
RMN ¹H (CDCl₃), *δ* 7.42-7.15 (m, 40H, CH_{Ph}), 5.89 (m, 1H, CH=), 5.58 (m, 1H, H-2_{A}), 5.31 (m, 1H, *J*ₜᵣₐₙₛ = 17.2 Hz, =CH₂), 5.28 (d, 1H, *J*_{1,2} = 3.4 Hz, H-1_{E}), 5.21 (m, 1H, *J*_{cis} = 10.4 Hz, =CH₂), 5.19 (m, 1H, H-2_{C}), 5.04 (d, 1H, *J* = 11.0 Hz, H_{Bn}), 5.01 (d, 1H, *J*_{1,2} = 1.4 Hz, H-1_{B}), 5.00 (d, 1H, *J*_{1,2} = 1.6 Hz, H-1_{A}), 4.98-4.87 (m, 4H, H_{Bn}), 4.84 (d, 1H, *J*_{1,2} = 1.6 Hz, H-1_{C}), 4.82-4.61 (m, 8H, H_{Bn}), 4.57 (d, 1H, *J* = 11.1 Hz, H_{Bn}), 4.52 (d, 1H, *J* = 11.0 Hz, H_{Bn}), 4.36 (d, 1H, *J* = 12.0 Hz, H_{Bn}), 4.28 (dd, 1H, *J*_{2,3} = 3.1 Hz, *J*_{3,4} = 9.6 Hz, H-3_{A}), 4.19 (m, 1H, H_{All}), 4.17-4.07 (m, 3H, H-3_{E}, H-3_{C}, H-5_{E}), 4.04 (m, 1H, H_{All}), 3.99 (m, 1H, H-2_{B}), 3.90 (m, 1H, H-5_{A}), 3.88-3.81 (m, 2H, H-3_{B}, H-4_{E}), 3.79-3.74 (m, 3H, H-5_{C}), 3.73 (m, 1H, H-5_{B}), 3.69-3.65 (m, 2H, H-2_{E}, H-6a_{E}), 3.60-3.56 (m, 2H, H-6b_{E}, H-4_{A}), 3.54 (pt, 1H, *J*_{3,4} = 9.4 Hz, H-4_{B}), 3.45 (pt, 1H, *J*_{3,4} = 9.5 Hz, H-4_{C}), 2.57 (m, 4H, 2CH_{2Lev}), 2.17 (s, 3H, H_{Ac}), 2.11 (s, 3H, CH_{3Lev}), 1.35 (d, 3H, *J*_{5,6} = 6.2 Hz, H-6_{A}), 1.30 (m, 6H, H-6_{C}, H-6_{B}).
RMN ¹³C (CDCl₃), *δ* 206.6 (C_{Lev}), 172.1 (C_{Lev}), 170.8 (C_{Ac}), 138.6-138.4 (C_{Ph}), 134.0 (CH=), 129.0-127.9 (CH_{Ph}), 117.9 (=CH₂), 101.7 (C-1_{B}, ¹*J*_{CH} = 169.9 Hz), 99.5 (C-1_{A}, ¹*J*_{CH} = 176.6 Hz), 96.5 (C-1_{C}, ¹*J*_{CH} = 170.6 Hz), 93.3 (C-1_{E}, ¹*J*_{CH} = 168.8 Hz), 82.6 (C-3_{E}), 80.7 (C-4_{B}), 80.3 (C-4_{C}), 80.2 (C-4_{A}), 79.8 (C-2_{E}), 79.7 (C-3_{B}), 79.4 (C-3_{C}), 78.2 (C-4_{E}), 76.5, 76.0, 75.9 (3C, C_{Bn}), 75.8 (C-2_{B}), 75.7, 75.4, 73.8, 73.2 (4C, C_{Bn}), 72.9 (C-2_{C}), 72.6 (C-3_{A}), 72.5 (C_{Bn}), 70.6 (C-5_{E}), 69.4 (C-5_{B}), 69.0 (C-5_{A}), 68.7 (C_{All}), 68.6 (C-6_{E}), 68.4 (C-2_{A}), 68.1 (C-5_{C}), 38.3 (CH_{2Lev}), 30.1 (CH_{3Lev}), 28.5 (CH_{2Lev}), 21.5 (C_{Ac}), 18.4, 18.3, 18.2 (C-6_{A}*, C-6_{B}*, C-6_{C}*).

| | | |
|---|---|---|
| HRMS (ESI⁺) : | [M+Na]⁺ C₉₀H₁₀₂O₂₁Na | m/z théorique : 1541.6812 |
| | | m/z mesurée : 1541.6769 |
| | [M+NH₄]⁺ C₉₀H₁₀₂O₂₁NH₄ | m/z théorique : 1536.7257 |
| | | m/z mesurée: 1536.7207 |

### ➢ Allyl (2,3,4,6-tétra-O-benzyl-α-D-glucopyranosyl)-(1→3)-(4-O-benzyl-α-L-rhamnopyranosyl)-(1→2)-(3,4-di-O-benzyl-α-L-rhamnopyranosyl)-(1→3)-2-O-acétyl-4-O-benzyl-α-L-rhamnopyranoside 85 :

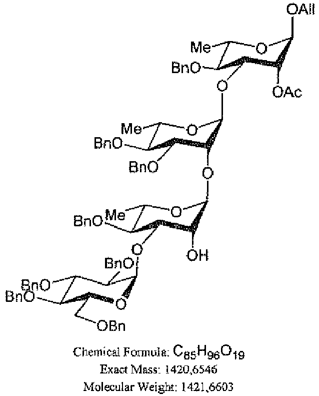

Le glycoside d'allyle **87** (552 mg, 360 µmol) est dissous dans un mélange pyridine/acide acétique (3/2, 5mL). De l'hydrazine monohydraté (88 µL, 1,8 mmol, 5 éq.) est ajouté goutte à goutte au milieu réactionnel. Après 30 min d'agitation à TA, le suivi par CCM (Tol/EtOAc, 8/2) indique la disparition de **87** (Rf = 0,65) et l'apparition d'un produit moins polaire (Rf= 0,7). Le milieu réactionnel est alors repris à l'eau froide (10 mL) et la phase aqueuse est extraite rapidement au DCM (3 x 100 mL). Les phases organiques sont regroupées et lavées avec une solution de NaClₛₐₜ, filtrées sur filtre séparateur de phase et concentrées à l'évaporateur rotatif. L'huile obtenue est purifiée par chromatographie sur gel de silice (Tol/EtOAc, 9/1) pour obtenir l'accepteur **85** sous forme d'une huile incolore (456 mg, 89%).
Rf = 0,7 (Tol/EtOAc, 8/2).
RMN ¹H (CDCl₃), *δ* 7.43-7.23 (m, 40H, CH_{Ph}), 5.89 (m, 1H, CH=), 5.35 (m, 1H, *J*ₜᵣₐₙₛ = 17.2 Hz, =CH₂), 5.25 (m, .1H, *J*_{cis} = 10.4 Hz, =CH₂), 5.22 (m, 1H, H-2_{C}), 5.19 (s, 1H, H-1_{A}), 5.06 (d, 1H, *J*_{1,2} = 1.4 Hz, H-1_{B}), 5.03-4.96 (m, 3H, H_{Bn}), 4.97 (d, 1H, *J*_{1,2} = 3.7 Hz, H-1_{E}), 4.91-4.89 (m, 3H, H_{Bn}), 4.81 (d, 1H, *J*_{1,2} = 1.3 Hz, H-1_{C}), 4.81 (d, 1H, *J* = 10.7 Hz, H_{Bn}), 4.75-4.68 (m, 3H, H_{Bn}), 4.66-4.53 (m, 5H, H_{Bn}), 4.36 (d, 1H, *J* = 11.9 Hz, H_{Bn}), 4.19 (m, 1H, H_{All}), 4.17-4.08 (m, 5H, H-3_{C}, H-2_{A}, H-2_{B}, H-3_{E}, H-3_{A}), 4.03 (m, 1H, H_{All}), 4.01 (m, 1H, H-5_{E}), 3.93 (m, 1H, H-5_{A}), 3.89 (dd, 1H, *J*_{2,3} = 2.8 Hz, *J*_{3,4} = 9.4 Hz, H-3_{B}), 3.85 (m, 3H, H-5_{C}), 3.79 (pt, 1H, *J*_{3,4} = 9.7 Hz, H-4_{E}), 3.74 (m, 1H, H-5_{B}), 3.66 (dd, 1H, *J*_{1,2} = 3.6 Hz, *J*_{2,3} = 9.6 Hz, H-2_{E}), 3.57 (pt, 1H, *J*_{3,4} = 9.3 Hz, H-4_{A}), 3.54-3.43 (m, 4H, H-6a_{E}, H-4_{B}, H-4_{C}, H-6b_{E}), 2.19 (s, 3H, H_{Ac}), 1.37 (d, 3H, *J*_{5,6} = 6.3 Hz, H-6_{A}), 1.35 (d, 3H, *J*_{5,6} = 6.2 Hz, H-6_{B}), 1.33 (d, 3H, *J*_{5,6} = 6.2 Hz, H-6_{C}).
RMN ¹³C (CDCl₃), *δ* 170.8 (C_{Ac}), 138.8-138.6 (C_{Ph}), 134.0 (CH=), 129.1-128.1 (CH_{Ph}), 117.9 (=CH₂), 101.9 (C-1_{B}, ¹*J*_{CH} = 168.4 Hz), 101.3 (C-1_{A}, ¹*J*_{CH} = 173.1 Hz), 96.6 (C-1_{C}, *¹J*_{CH} = 170.4 Hz), 94.3 (C-1_{E}, ¹*J*_{CH} = 167.2 Hz), 82.8 (C-3_{E}), 80.8 (C-4_{B}), 80.4 (C-4_{C}), 80.0 (C-3_{B}), 79.6 (C-4_{A}), 79.4 (2C, C-2_{E}, C-3_{C}), 78.2 (C-4_{E}), 76.8 (C-3_{A}), 76.0, 75.9, 75.7 (4C, C_{Bn}), 75.6 (C-2_{B}), 75.3, 74.8, 73.8 (3C, C_{Bn}), 73.0 (C-2_{C}), 72.7 (C_{Bn}), 71.1 (C-5_{E}), 69.4 (C-5_{B}), 68.7 (C_{All}), 68.7 (C-6_{E}), 68.3 (C-5_{A}), 68.2 (C-5_{C}), 67.8 (C-2_{A}), 21.6 (C_{Ac}), 18.4, 18.3, 18.2 (C-6_{A}*, C-6_{B}*, C-6_{C}*).

| | | |
|---|---|---|
| HRMS (ESI⁺) : | [M+Na]⁺ C₈₅H₉₆O₁₉Na | m/z théorique : 1443.6444 |
| | | m/z mesurée : 1443.6505 |
| | [M+NH₄]⁺ C₈₅H₉₆O₁₉NH₄ | m/z théorique : 1438.6890 |
| | | m/z mesurée : 1438.6960 |

### ➢ Propyl α-D-glucopyranosyl-(1→3)-α-L-rhamnopyranosyl-(1→2)-α-L-rhamnopyranosyl-(1→3)-2-O-acétyl-α-L-rhamnopyranoside XI :

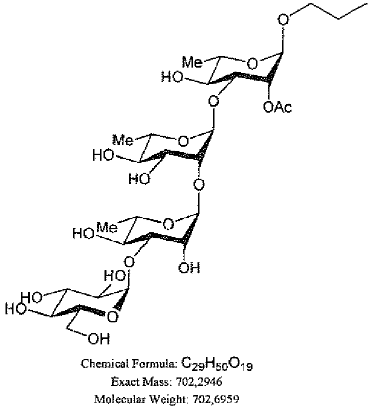

Du Pd-C 10% (300 mg) est ajouté à une solution dégazée d'alcool **85** (400 mg, 281 µmol) dans de l'éthanol (30 mL). La suspension est saturée avec de l'hydrogène à pression atmosphérique et agitée à TA pendant 1 nuit. Le suivi par CCM (iPrOH/H₂O/NH₃, 4/1/0,5 et Tol/EtOAc, 8/2) montre la disparition de **85** (Rf = 1 et 0,7, respectivement) et l'apparition d'un nouveau composé plus polaire (Rf = 0,8 et 0, respectivement). Le milieu réactionnel est filtré sur célite, et le filtrat est concentré à l'évaporateur rotatif. Le résidu obtenu est purifié sur colonne C-18 (H₂O/CH₃CN, 100/0→8/2) pour conduire à la cible ***XI*** sous forme d'un solide blanc (150 mg, 76%). Rf = 0,8 (iPrOH/H₂O/NH₃, 4/1/0,5).
RMN ¹H (D₂O), *δ* 5.09 (d, 1H, *J*_{1,2} = 1.0 Hz, H-1_{B}), 5.07 (m, 1H, H-2_{C}), 5.00 (d, 1H, *J*_{1,2} = 3.8 Hz, H-1_{E}), 4.88 (d, 1H, *J*_{1,2} = 1.5 Hz, H-1_{A}), 4.72 (d, 1H, *J*_{1,2} = 1.5 Hz, H-1_{c}), 4.17 (dd, 1H, *J*_{2,3} = 2.5 Hz, H-2_{A}), 3.91-3.83 (m, 3H, H-2_{B}, H-3_{C}, H-5_{E}), 3.75-3.61 (m, 7H, H-3_{A}, H-5_{C}, H-6a_{E}, H-6b_{E}, H-3_{E}, H-5_{A}, H-3_{B}), 3.60-3.54 (m, 2H, H_{Pr}, H-5_{B}), 3.49 (pt, 1H, *J*_{3,4} = 9.7 Hz, H-4_{C}), 3.48-3.37 (m, 3H, H-2_{E}, H-4_{A}, H_{Pr}), 3.42 (pt, 1H, *J*_{3,4} = 10.0 Hz, H-4_{B}), 3.42 (pt, 1H, *J*_{3,4} = 9.2 Hz, H-4_{E}), 2.07 (s, 3H, H_{Ac}), 1.60 (sex, 2H, CH₂), 1.27 (d, 3H, *J*_{5,6} = 6.2 Hz, H-6_{C}), 1.18-1.16 (m, 6H, H-6_{A}, H-6_{B}), 0.88 (t, 3H, *J* = 7.4 Hz, CH₃).
RMN ¹³C (D₂O), *δ* 173.4 (C_{Ac}), 102.4 (C-1_{A}, ¹*J*_{CH} = 175.5 Hz), 101.2 (C-1_{B}, ¹*J*_{CH} = 173.2 Hz), 97.2 (C-1_{C}, ¹*J*_{CH} = 172.3 Hz), 95.8 (C-1_{E}, ¹*J*_{CH} = 167.3 Hz), 78.8 (C-2_{B}), 76.2 (C-3_{C}), 75.7 (C-3_{A}), 73.3 (C-3_{E}), 72.5 (C-4_{C}), 72.4 (C-4_{E}), 72.3 (C-2_{C}), 72.1 (C-5_{E}), 71.8 (C-2_{E}), 70.7 (C-4_{A}), 70.3 (C_{Pr}), 70.2 (C-5_{A}), 69.8 (2C, C-4_{B}, C-5_{B}), 69.7 (C-3_{B}), 69.0 (C-5_{C}), 67.1 (C-2_{A}), 60.7 (C-6_{E}), 22.4 (CH₂), 20.7 (C_{Ac}), 17.2, 17.1, 16.9 (C-6_{A}*, C-6_{B}*, C-6_{C}*), 10.3 (CH₃).

| | | |
|---|---|---|
| HRMS (ESI⁺) : | [M+Na]⁺ C₂₉H₅₀O₁₉Na | m/z théorique : 725.2844 |
| | | m/z mesurée : 725.2827 |

### Procédé 8 :

### ➢ Allyl (2,3,4,6-tétra-O-benzyl-α-D-glucopyranosyl)-(1→3)-(4-O-benzyl-α-L-rhamnopyranosyl)-(1→2)-(3,4-di-O-benzyl-α-L-rhamnopyranosyl)-(1→3)-4-O-henzyl-α-L-rhamnopyranoside 89 :

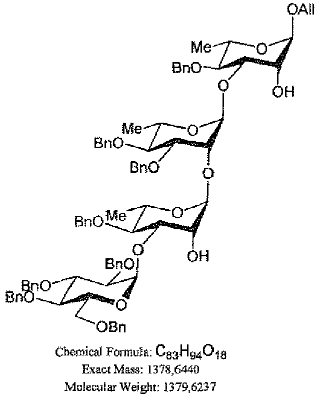

Après addition de NaOMe 0,5 M (770 µL, 390 µmol, 1,1 éq.) à l'alcool 85 (500 mg, 350 µmol) en solution dans du MeOH (10 mL). Le milieu réactionnel est porté à reflux et agité pendant 3 h, temps au bout duquel le suivi par CCM (Chex/EtOAc, 7/3), indique la disparition de **85** (Rf = 0,5) et l'apparition d'un produit plus polaire (Rf = 0,3). Après retour à TA, le milieu réactionnel est neutralisé par addition de résine échangeuse d'ion DOWEX (H⁺), puis filtré sur fritté. La résine est lavée avec un mélange DCM/MeOH et les solvants sont évaporés. L'huile jaune obtenue est purifiée par chromatographie sur gel de silice (Chex/EtOAc, 75/25→7/3) pour conduire au diol **89** sous forme d'un solide blanc (461 mg, 95%).
Rf = 0,3 (Chex/EtOAc, 7/3).
RMN ¹H (CDCl₃), δ 7.42-7.20 (m, 40H, CH_{Ph}), 5.93 (m, 1H, CH=), 5.33 (m, 1H, *J*ₜᵣₐₙₛ = 17.2 Hz, =CH₂), 5.23 (m, 1H, *J*_{cis} = 10.4 Hz, =CH₂), 5.18 (s, 1H, H-1_{A}), 5.10 (d, 1H, *J*_{1,2} = 1.5 Hz, H-1_{B}), 5.03-4.98 (m, 3H, H_{Bn}), 4.95 (d, 1H, *J*_{1,2} = 3.3 Hz, H-1_{E}), 4.92-4.87 (m, 3H, H_{Bn}), 4.84 (d, 1H, *J*_{1,2} = 1.5 Hz, H-1_{C}), 4.79 (d, 1H, *J* = 11.4 Hz, H_{Bn}), 4.75-4.69 (m, 5H, H_{Bn}), 4.60-4.56 (m, 2H, H_{Bn}), 4.52 (d, 1H, *J* = 11.0 Hz, H_{Bn}), 4.33 (d, 1H, *J* = 12.0 Hz, H_{Bn}), 4.19 (m, 1H, H_{All}, 4.13-4.07 (m, 5H, H-2_{A}, H-3_{E}, H-2_{B}, H-3_{A}, H-2_{C}), 4.03 (m, 1H, H_{All}), 4.01-3.98 (m, 2H, H-3_{C}, H-5_{E}), 3.93-3.87 (m, 3H, H-3_{B}, H-5_{B}, H-5_{A}), 3.81-3.76 (m, 2H, H-4_{E}, H-5_{C}), 3.65 (dd, 1H, J_{1,2} = 3.5 Hz, *J*_{2,3} = 9.7 Hz, H-2_{E}), 3.56 (pt, 1H, *J*_{3,4} = 9.5 Hz, H-4_{A}), 3.54-3.43 (m, 4H, H-4_{B}, H-6a_{E}, H-4_{C}, H-6b_{E}), 2.19 (s, 1H, OH), 1.37 (d, 3H, *J*_{5,6} = 6.2 Hz, H-6_{A}), 1.34 (d, 3H, H-6_{A}, *J*_{5,6} = 6.3 Hz, H-6_{B}), 1.30 (d, 3H, *J*_{5,6} = 6.3 Hz, H-6_{C}).
RMN ¹³C (CDCl₃), *δ* 138.6-138.2 (C_{Ph}), 134.0 (CH=), 129.0-128.1 (CH_{Ph}), 117.7 (=CH₂), 101.5 (C-1_{B}, ¹*J*_{CH} = 170.1 Hz), 10143 (C-1_{A}, ¹*J*_{CH} = 170.1 Hz), 98.8 (C-1_{C}, ¹*J*_{CH} = 167.2 Hz), 94.3 (C-1_{E}, ¹*J*_{CH} = 169.2 Hz), 82.8 (C-3_{E}), 81.5 (C-3_{C}), 80.7 (C-4_{B}), 80.2 (C-4_{C}), 80.0 (C-3_{B}), 79.6 (C-4_{A}), 79.3 (C-2_{E}), 78.2 (C-4_{E}), 76.8 (C-3_{A}), 76.0, 75.9, 75.8 (4C, C_{Bn}), 75.9 (C-2_{B}), 75.3, 74.8, 73.8, 72.7 (4C, C_{Bn}), 71.3 (C-2_{C}), 71.1 (C-5_{E}), 69.6 (C-5_{B}), 68.4 (C_{All}), 68.3 (C-6_{E}), 68.3 (C-5_{A}), 68.0 (C-5_{C}), 67.7 (C-2_{A}), 18.4, 18.3, 18.2 (C-6_{A}*, C-6_{B}*, C-6_{C}*).

| | | |
|---|---|---|
| HRMS (ESI⁺) : | [M+Na]⁺ C₈₃H₉₄O₁₈Na | m/z théorique : 1401.6338 |
| | | m/z mesurée : 1401.6403 |
| | [M+NH₄]⁺ C₈₃H₉₄O₁₈NH₄ | m/z théorique : 1396.6783 |
| | | m/z mesurée : 1396.6854 |

### ➢ Propyl α-D-glucopyranosyl-(1→3)-α-L-rhamnopyranosyl-(1→2)-α-L-rhamnopyranosyl-(1→3)-α-L-rhamnopyranoside XII :

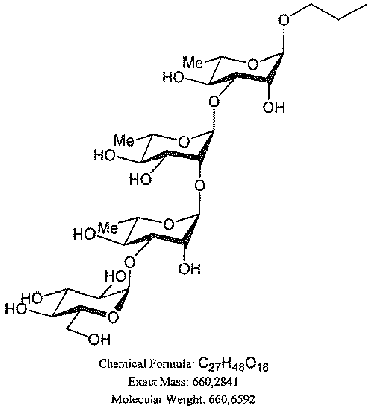

Du Pd-C 10% (300 mg) est ajouté à une solution dégazée de diol **89** (360 mg, 261 µmol) dans de l'éthanol (30 mL). La suspension est saturée avec de l'hydrogène à pression atmosphérique et agitée à TA pendant 1 nuit. Le suivi par CCM (iPrOH/H₂O/NH₃, 4/1/0,5 et Chex/EtOAc, 7/3) montre la disparition de **89** (Rf = 1 et 0,3, respectivement) et l'apparition d'un nouveau composé plus polaire (Rf = 0,5 et 0, respectivement). Le milieu réactionnel est filtré sur célite, et le filtrat est concentré à l'évaporateur rotatif. Le résidu obtenu est purifié sur colonne C-18 (H₂O/CH₃CN, 100/0→8/2) pour conduire au à la cible ***XII*** sous forme d'un solide blanc (140 mg, 81%).
Rf = 0,5 (iPrOH/H₂O/NH₃, 4/1/0,5).
RMN ¹H (D₂O), *δ* 5.10 (bs, 1H, H-1_{B}), 4.99 (d, 1H, *J*_{1,2} = 3.8 Hz, H-1_{E}), 4.89 (d, 1H, *J*_{1,2} = 1.5 Hz, H-1_{A}), 4.65 (d, 1H, *J*_{1,2} = 1.4 Hz, H-1_{C}), 4.17 (dd, 1H, *J*_{2,3} = 2.4 Hz, H-2_{A}), 3.95 (m, 1H, H-2_{B}) 3.88-3.81 (m, 3H, H-2_{C}, H-5_{E}, H-3_{B}), 3.73 (dd, 1H, *J*_{2,3} = 3.0 Hz, *J*_{3,4} = 9.7 Hz, H-3_{A}), 3.71-3.59 (m, 7H, H-3_{C}, H-6a_{E}, H-6b_{E}, H-5_{B}, H-3_{E}, H-5_{C}, H-5_{A}), 3.53 (m, 1H, H_{Pr}), 3.48-3.37 (m, 5H, H-2_{E}, H-4_{A}, H-4_{C}, H_{Pr}, H-4_{B}), 3.34 (pt, 1H, *J*_{3,4} = 9.3 Hz, H-4_{E}), 1.50 (sex, 2H, CH₂), 1.18-1.16 (m, 9H, H-6_{A}, H-6_{B}, H-6_{C}), 0.81 (t, 3H, *J* = 7.4 Hz, CH₃).
RMN ¹³C (D₂O), *δ* 102.3 (C-1_{A}, ¹*J*_{CH}= 167.2 Hz), 101.0 (C-1_{B}, ¹*J*_{CH} = 173.1 Hz), 99.8 (C-1_{C}, ¹*J*_{CH} = 169.4 Hz), 95.7 (C-1_{E}, ¹*J*_{CH} = 169.5 Hz), 78.8 (C-2_{B}), 77.7 (C-3_{C}), 75.5 (C-3_{A}), 73.2 (C-3_{E}), 72.4 (C-4_{B}), 72.2 (C-4_{C}), 72.0 (C-5_{E}), 71.7 (C-4_{A}), 70.6 (C-2_{E}), 70.3 (C-2_{C}), 70.2 (C-3_{B}), 69.9 (C_{Pr}), 69.7 (C-5_{B}), 69.6 (C-4_{E}), 69.5 (C-5_{C}), 68.9 (C-5_{A}), 67.0 (C-2_{A}), 60.6 (C-6_{E}), 22.3 (CH₂), 17.1, 17.0, 16.8 (C-6_{A}*, C-6_{B}*, C-6_{C}*), 10.2 (CH₃).

| | | |
|---|---|---|
| HRMS (ESI⁺) : | [M+Na]⁺ C₂₇H₄₈O₁₈Na | m/z théorique : 683.2739 |
| | | m/z mesurée : 683.2729 |

### Procédé 9 :

### ➢ Allyl (3,4,6-tri-O-acétyl-2-désoxy-2-trichloroacétamido-β-D-glucopyranosyl)-(1→2)-[2,3,4,6-tétra-O-henzyl-α-D-glucopyranosyl-(1→3)]-(4-O-benzyl-α-L-rhamnopyranosyl)-(1→2)-(3,4-di-O-benzyl-α-L-rhamnopyranosyl)-(1→3)-2-O-acétyl-4-O-benzyl-α-L-rhamrnopyranoside 90 :

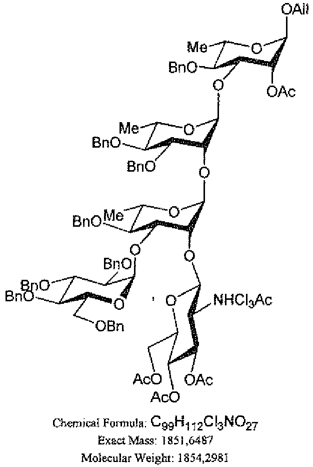

Du TMSOTf (28,0 µL, 160 µmol, 0,5 éq.) est additionné à une solution d'accepteur **85** (442 mg, 310 µmol) et de donneur **1** (370 mg, 620 µmol, 2 éq.) dans du Tol (8 mL), en présence de tamis moléculaire 4 Å (253 mg), agitée sous argon à -40°C. Après 1 h, à cette température, le bain éthanol-carboglace est retiré et l'agitation est poursuivie pendant 30 min, temps au bout duquel le suivi par CCM (Tol/EtOAc, 8/2) indique la quasi-disparition de **85** (Rf = 0,65) et l'apparition d'un nouveau composé (Rf = 0,45). La réaction est stoppée par addition de triéthylamine (0,1 mL) puis le milieu réactionnel est filtré et concentré à l'évaporateur rotatif. L'huile jaune obtenue est purifiée par chromatographie sur gel de silice (Tol/EtOAc, 9/1→7/3) pour obtenir le glycoside d'allyle **90** sous forme d'un solide blanc (469 mg, 82%).
Rf = 0,45 (Tol/EtOAc, 8/2).
RMN ¹H (CDCl₃), *δ* 7.43-7.09 (m, 41H, CH_{Ph}, NH), 5.89 (m, 1H, CH=), 5.31 (m, 1H, *J*ₜᵣₐₙₛ= 17.2 Hz, =CH₂), 5.26 (d, 1H, *J*_{1,2} = 3.5 Hz, H-1_{E}), 5.22 (m, 1H, *J*_{cis} = 10.4 Hz, =CH₂), 5.18 (m, 2H, H_{Bn}), 5.16 (m, 1H, H-2_{C}), 5.19 (d, 1H, *J*_{1,2} = 1.1 Hz, H-1_{A}), 5.06 (m, 1H, H-4_{D}), 5.05 (m, 2H, H_{Bn}), 5.00 (d, 1H, *J*_{1,2} = 1.0 Hz, H-1_{B}), 4.96 (m, 1H, H_{Bn}), 4.94 (m, 1H, H-1_{D}), 4.88 (pt, 1H, *J*_{3,4} = 10.4 Hz, H-3_{D}), 4.81 (d, 1H, *J*_{1,2} = 1.6 Hz, H-1_{C}), 4.77-4.85 (m, 4H, H_{Bn}), 4.62-4.56 (m, 4H, H_{Bn}), 4.52-4.47 (m, 2H, H_{Bn}), 4.32 (d, 1H, *J* = 11.9 Hz, H_{Bn}), 4.26 (m, 1H, H-2_{D}), 4.20 (m, 1H, H-2_{A}), 4.18-4.14 (m, 3H, H_{All}, H-3_{A}, H-3_{E}), 4.12-4.08 (m, 2H, H-5_{E}, H-3_{C}), 4.01 (m, 1H, H_{All}), 3.98 (m, 1H, H-6a_{D}), 3.96-3.92 (m, 2H, H-2_{B}, H-2_{E}), 3.87-3.82 (m, 4H, H-6b_{D}, H-4_{E}, H-5_{A}, H-3_{B}), 3.79 (m, 3H, H-5_{C}), 3.74 (m, 1H, H-5_{B}), 3.50 (pt, 1H, *J*_{3,4} = 9.3 Hz, H-4_{B}), 3.48 (pt, 1H, *J*_{3,4} = 9.4 Hz, H-4_{A}), 3.54-3.40 (m, 3H, H-6a_{E}, H-6b_{E}, H-4_{C}), 2.97 (m, 1H, H-5_{D}), 2.15, 2.08, 2.03, 1.90 (4s, 12H, H_{Ac}), 1.34 (d, 3H, *J*_{5,6} = 6.2 Hz, H-6_{A}), 1.32 (d, 3H, *J*_{5,6} = 6.2 Hz, H-6_{B}) 1.27 (d, 3H, *J*_{5,6}= 6.2 Hz, H-6_{C}).
RMN ¹³C (CDCl₃), *δ* 171.0, 170.9, 170.8, 169.6 (4C, C_{Ac}), 162.6 (C_{NTCA}), 139.0-138.0 (C_{Ph}), 134.0 (CH=), 129.0-127.8 (CH_{Ph}), 117.9 (=CH₂), 101.7 (C-1_{B}, ¹*J*_{CH} = 173.2 Hz), 101.3 (C-1_{A}, ¹*J*_{CH}= 171.0 Hz), 101.2 (C-1_{D}, ¹*J*_{CH}= 161.0 Hz), 96.5 (C-1_{C}, ¹*J*_{CH}= 168.8 Hz), 95.1 (C-1_{E}, ¹*J*_{CH}= 164.5 Hz), 93.1 (CCl₃), 82.8 (C-3_{E}), 80.8 (C-4_{B}), 80.3 (C-4_{C}), 80.1 (C-4_{A}), 79.3 (C-3_{C}), 79.2 (C-2_{E}), 79.1 (C-4_{E}), 78.9 (C-3_{B}), 76.4 (C_{Bn}), 76.0 (C-2_{B}), 75.9, 75.7, 75.5, 75.3 (4C, C_{Bn}), 75.1 (C-3_{A}), 74.4 (C-2_{A}), 74.3, 73.8 (2C, C_{Bn}), 73.6 (C-3_{D}), 72.9 (C-2_{C}), 72.3 (C_{Bn}), 72.2 (C-5_{D}), 70.4 (C-5_{E}), 69.2 (C-5_{B}), 69.1 (C-5_{A}), 68.6 (C_{All}), 68.2 (C-4_{D}), 68.2 (C-6_{E}), 68.1 (C-5_{C}), 61.9 (C-6_{D}), 56.2 (C-2_{D}), 21.5, 21.0, 20.9, 20.8 (4C, C_{Ac}), 18.3, 18.2, 18.1 (C-6_{A}*, C-6_{B}*, C-6_{C}*).

| | | |
|---|---|---|
| HRMS (ESI⁺) : | [M+Na]⁺ C₉₉H₁₁₂³⁵Cl₃NO₂₇Na | m/z théorique : 1874.6385 |
| | | m/z mesurée : 1874.6638 |
| | [M+NH₄]⁺ C₉₉H₁₁₂³⁵Cl₃NO₂₇NH₄ | m/z théorique : 1869.6831 |
| | | m/z mesurée : 1869.6978 |

### ➢ Allyl (2-désoxy-2-trichloroacétamido-β-D-glucopyranosyl)-(1→2)-[2,3,4,6-tétra-O-benzyl-α-D-glucopyranosyl-(1→3)1-(4-O-benzyl-α-L-rhamnopyranosyl)-(1→2)-(3,4-di-O-benzyl-α-L-rhamnopyranosyl)-(1→3)-2-O-acétyl-4-O-benzyl-α-L-rhamnopyranoside 91 :

### ➢ Allyl (2-désoxy-2-trichloroacétamido-β-D-glucopyranosyl)-(1→2)-[2,3,4,6-tétra-O-benzyl-α-D-glucopyranosyl-(1→3)]-(4-O-benzyl-α-L-rhamnopyranosyl)-(1→2)-(3,4-di-O-benzyl-α-L-rhamnopyranosyl)-(1→3)-4-O-benzyl-α-L-rhamnopyranoside 92 :

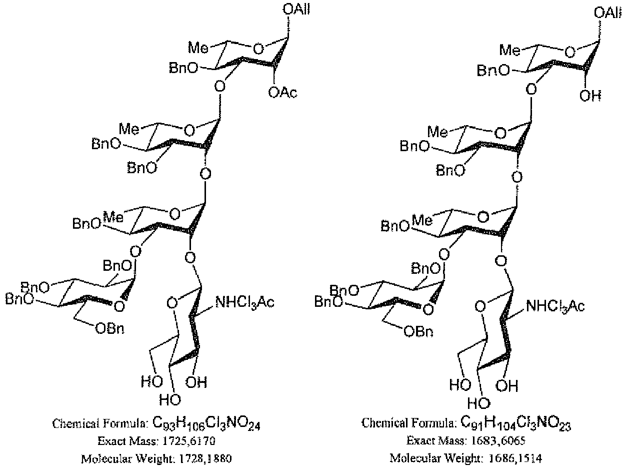

Le glycoside d'allyle **90** (200 mg, 100 µmol) est dissous dans du MeOH (12,5 mL) puis du NaOMe 0,5 M (431 µL, 200 µmol, 2 éq.) est ajouté au milieu réactionnel. Après 25 min sous agitation, le suivi par CCM (DCM/MeOH, 95/5 et Chex/EtOAc, 6/4) indique la disparition de **90** et l'apparition de deux produits plus polaires (Rf= 0,3 et 0,2 dans DCM/MeOH, 95/5). Le milieu réactionnel est alors neutralisé par addition de résine échangeuse d'ion DOWEX (H⁺), puis filtré sur fritté. La résine est lavée avec un mélange DCM/MeOH et les solvants sont évaporés. L'huile jaune obtenue est purifiée par chromatographie sur gel de silice (Tol/EtOAc, 7/3→1/1) pour conduire par ordre d'élution au produit mono-acétylé **91** sous forme d'un solide blanc (103 mg, 56%) et au produit non-acétylé **92** sous forme d'un solide blanc (55 mg, 30%).
**91 :** Rf = 0,3 (DCM/MeOH, 95/5).
RMN ¹H (CDCl₃), δ 7.41 (d, 1H, *J*_{2,NH} = 6.1 Hz, NH), 7.39-7.08 (m, 40H, CH_{Ph}), 5.94 (m, 1H, CH=), 5.38 (bs, 1H, H-1_{A}), 5.33 (m, 1H, *J*ₜᵣₐₙₛ = 17.2 Hz, =CH₂), 5.23 (m, 1H, *J*_{cis} = 10.4 Hz, =CH₂), 5.19-5.15 (m, 4H, H-2_{C}, H-1_{E}, 2H_{Bn}), 5.12-5.01 (m, 2H, H_{Bn}), 4.96 (d, 1H, *J* = 10.7 Hz, H_{Bn}), 4.91 (bs, 1H, H-1_{B}), 4.82 (d, 1H, J_{1,2} = 1.5 Hz, H-1_{C}), 4.80-4.64 (m, 5H, H_{Bn}), 4.55 (m, 1H, H-1_{D}), 4.54-4.49 (m, 5H, H_{Bn}), 4.32 (d, 1H, *J* = 11.9 Hz, H_{Bn}), 4.20 (m, 1H, H-2_{B}), 4.17 (m, 1H, H_{All}), 4.15 (m, 1H, H-2_{A}), 4.13-4.08 (m, 4H, H-3_{E}, H-3_{A}, H-3_{C}, H-5_{E}), 4.11(m, 1H, H_{All}), 3.89-3.82 (m, 3H, H-4_{E}, H-3_{B}, H-2_{E}), 3.79-3.69 (m, 4H, H-2_{D}, H-5_{C}, H-5_{A}, H-5_{B}), 3.63 (m, 1H, H-6a_{D}), 3.52-3.36 (m, 5H, H-4_{B}, H-4_{A}, H-6a_{E}, H-6b_{E}, H-4_{C}), 3.12 (pt, 1H, *J*_{3,4} = 9.3 Hz, H-4_{D}), 3.05 (m, 1H, H-5_{D}), 2.92 (m, 1H, H-6b_{D}), 2.37 (m, 1H, H-3_{D}), 2.14 (s, 3H, H_{Ac}), 1.34-1.27 (m, 6H, H-6_{A}, H-6_{B}), 1.28 (d, 3H, *J*_{5,6} = 6.1 Hz, H-6_{C}).
RMN ¹³C (CDCl₃), *δ* 170.8 (C_{Ac}), 164.9 (C_{NTCA}), 138.7-138.1 (C_{Ph}), 134.0 (CH=), 129.5-127.7 (CH_{Ph}), 118.2 (=CH₂), 102.0 (C-1_{B}, ¹*J*_{CH} = 170.5 Hz), 101.3 (C-1_{D}, ¹*J*_{CH} = 161.0 Hz), 100.4 (C-1_{A}, ¹*J*_{CH} = 171.2 Hz), 96.5 (C-1_{C}, ¹*J*_{CH} = 171.2 Hz), 94.6 (C-1_{E}, ¹*J*_{CH} = 169.8 Hz), 92.9 (CCl₃), 83.4 (C-3_{E}), 80.6 (C-4_{B}), 80.5 (C-4_{E}), 80.1 (C-4_{C}), 80.1 (C-3_{C}), 79.9 (C-3_{B}), 79.4 (C-4_{A}), 79.0 (C-2_{E}), 76.6 (C_{Bn}), 76.5 (C-3_{D}), 76.1 (C-5_{D}), 75.9, 75.6, 75.4, 75.3 (5C, C_{Bn}), 74.4 (C-3_{A}), 74.1 (C-2_{A}), 73.9, 73.2 (2C, C_{Bn}), 72.9 (C-4_{D}), 72.8 (C-2_{C}), 72.1 (C-2_{B}), 70.4 (C-5_{B}), 69.7 (C-5_{E}), 69.2 (C-5_{A}), 68.8 (C_{All}), 68.0 (C-6_{E}), 67.9 (C-5_{C}), 62.9 (C-6_{D}), 58.8 (C-2_{D}), 21.5 (C_{Ac}), 18.3, 18.2, 18.1 (C-6_{A}*, C-6_{B}*, C-6_{C}*).

| | | |
|---|---|---|
| HRMS (ESI⁺) : | [M+Na]⁺ C₉₃H₁₀₆³⁵Cl₃NO₂₄Na | m/z théorique : 1748.6068 |
| | | m/z mesurée : 1748.6366 |
| | [M+NH₄]⁺ C₉₃H₁₀₆³⁵Cl₃NO₂₄NH₄ | m/z théorique : 1743.6514 |
| | | m/z mesurée : 1743.6545 |

**92 :** Rf= 0,2 (DCM/MeOH, 95/5).
RMN ¹H (CDCl₃), *δ* 7.41 (d, 1H, *J*_{2,NH} = 6.6 Hz, NH), 7.40-7.11 (m, 40H, CH_{Ph}), 5.92 (m, 1H, CH=), 5.38 (bs, 1H, H-1_{A}), 5.31 (m, 1H, *J*ₜᵣₐₙₛ= 17.2 Hz, =CH₂), 5.23 (m, 1H, *J*_{cis} = 10.4 Hz, =CH₂), 5.19 (d, 1H, *J*_{1,2} = 3.5 Hz, H-1_{E}), 5.14-4.98 (m, 4H, H_{Bn}), 4.96 (bs, 1H, H-1_{B}), 4.93 (d, 1H, *J* = 12.3 Hz, H_{Bn}), 4.81 (d, 1H, *J*_{1,2} = 1.3 Hz, H-1_{C}), 4.80 (d, 1H, *J* = 10.7 Hz, H_{Bn}), 4.74-4.66 (m, 6H, H_{Bn}), 4.55 (m, 1H, *J*_{1,2} = 4.4 Hz, H-1_{D}), 4.55-4.69 (m, 3H, H_{Bn}), 4.37 (d, 1H, *J* = 11.9 Hz, H_{Bn}), 4.20 (m, 1H, H_{All}), 4.18-4.10 (m, 5H, H-2_{B}, H-2_{A}, H-3_{A}, H-3_{E}, H-5_{E}), 4.07 (m, 1H, H-2_{C}), 4.11(m, 1H, H_{All}), 3.97 (m, 1H, H-3_{C}), 3.92 (m, 1H, H-3_{B}), 3.89-3.83 (m, 3H, H-4_{E}, H-2_{E}, H-2_{D}), 3.88 (m, 1H, H-5_{B}), 3.76 (m, 1H, H-5_{C}), 3.72 (m, 1H, H-5_{A}), 3.67 (m, 1H, H-6a_{D}), 3.56 (pt, 1H, *J*_{3,4} = 9.4 Hz), 3.53-3.39 (m, 4H, H-6a_{E}, H-6b_{E}, H-4_{A}, H-4_{C}), 3.18 (pt, 1H, *J*_{3,4} = 8.9 Hz, H-4_{D}), 3.13 (m, 1H, H-5_{D}), 3.08 (m, 1H, H-6b_{D}), 2.48 (m, 1H, H-3_{D}), 1.28 (d, 3H, *J*_{5,6} = 6.2 Hz, H-6_{B}), 1.28 (d, 3H, *J*_{5,6} = 6.2 Hz, H-6_{A}), 1.28 (d, 3H, *J*_{5,6} = 6.2 Hz, H-6_{C}), RMN ¹³C (CDCl₃), *δ* 164.7 (C_{NTCA}), 138.7-138.2 (C_{Ph}), 134.2 (CH=), 129.6-127.7 (CH_{Ph}), 117.9 (=CH₂), 101.7 (C-1_{B}, ¹*J*_{CH}= 170.5 Hz), 101.4 (C-1_{D}, ¹*J*_{CH}= 158.1 Hz), 100.6 (C-1_{A}, ¹*J*_{CH}= 173.4 Hz), 98.8 (C-1_{C}, ¹*J*_{CH}= 169.8 Hz), 94.7 (C-1_{E}, ¹*J*_{CH}= 168.3 Hz), 93.0 (CCl₃), 83.7 (C-3_{E}), 81.9 (C-3_{C}), 80.7 (C-4_{B}), 80.3 (C-3_{B}), 80.1 (C-4_{A}), 80.0 (C-4_{C}), 79.4 (C-4_{E}), 79.1 (C-2_{E}), 76.6 (C_{Bn}), 76.5 (C-3_{D}), 76.2 (C-5_{D}), 75.8, 75.7, 75.4, 75.3 (5C, C_{Bn}), 74.4 (C-3_{A}), 74.3 (C-2_{A}), 73.9, 73.4 (2C, C_{Bn}), 72.8 (C-4_{D}), 72.7 (C-2_{B}), 71.3 (C-2_{C}), 70.4 (C-5_{E}), 69.7 (C-5_{B}), 69.3 (C-5_{A}), 68.4 (C-6_{E}), 68.2 (C_{All}), 67.9 (C-5_{C}), 63.0 (C-6_{D}), 58.8 (C-2_{D}), 18.4, 18.3, 18.2 (C-6_{A}* , C-6_{B}*, C-6_{C}*).

| | | |
|---|---|---|
| HRMS (ESI⁺) : | [M+Na]⁺ C₉₁H₁₀₄³⁵Cl₃NO₂₃Na | m/z théorique : 1706.5962 |
| | | m/z mesurée : 1706.6206 |
| | [M+NH₄]⁺ C₉₁H₁₀₄³⁵Cl₃NO₂₃NH₄ | m/z théorique : 1701.6409 |
| | | m/z mesurée : 1701.6571 |

### ➢ Propyl (2-acétamido-2-désoxy-β-D-glucopyranosyl)-(1→2)-[α-D-glucopyranosyl-(1→-3)]-α-L-rhamnopyranosyl-(1→2)-α-L-rhamnopyranosyl-(1→3)-2-O-acétyl-α-L-rhamnopyranoside XIII :

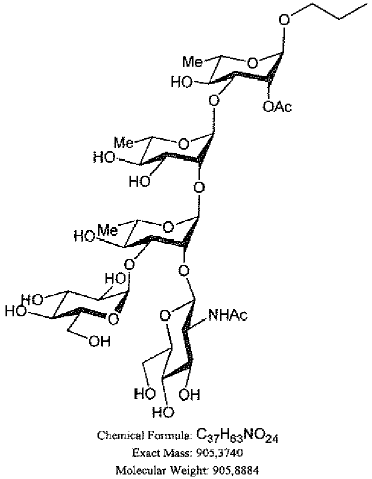

Du Pd-C 10% (300 mg) est ajouté à une solution dégazée de triol 91 (310 mg, 180 µmol) dans de l'éthanol (20 mL). La suspension est saturée avec de l'hydrogène à 50 bars et agitée à TA pendant 2 j. Le suivi par CCM (iPrOH/H₂O)/NH₃, 4/1/0,5 et DCM/MeOH, 95/5) montre la disparition de **91** (Rf = 1 et 0,3, respectivement) et l'apparition d'un nouveau composé plus polaire (Rf = 0,2 et 0, respectivement). Le milieu réactionnel est filtré sur un filtre Acrodisc LC 25 mm, et le filtrat est concentré à l'évaporateur rotatif. Le résidu obtenu est purifié sur colonne C-18 (H₂O/CH₃CN, 100/0→7/3) pour conduire à la cible *XIII* sous forme d'un solide blanc (119 mg, 74%).
Rf= 0,2 (iPrOH/H₂O/NH₃, 4/1/0,5).
RMN ¹H (D₂O), *δ*5.09 (bs, 1H, H-1_{B}), 5.08 (m, 1H, H-2_{C}), 5.07 (d, 1H, *J*_{1,2} = 3.5 Hz, H-1_{E}), 4.99 (d, 1H, *J*_{1,2} = 1.5 Hz, H-1_{A}), 4.73 (bs, 1H, H-1_{C}), 4.72 (d, 1H, *J*_{1,2} = 8.5 Hz, H-1_{D}), 4.33 (m, 1H, H-2_{A}), 3.95 (m, 1H, H-5_{E}), 3.93 (m, 1H, H-2_{B}), 3.88 (dd, 1H, *J*_{2,3} = 3.4 Hz, *J*_{3,4} = 9.6 Hz, H-3_{C}), 3.83-3.79 (m, 2H, H-3_{A}, H-6a_{D}), 3.76-3.68 (m, 4H, H-3_{E}, H-5_{C}, H-6a_{E}, H-6b_{E}), 3.66-3.52 (m, 7H, H-6b_{D}, H-2_{D}, H-3_{B}, H-5_{A}, H-2_{E}, H_{Pr}, H-5_{B}), 3.49 (pt, 1H, *J*_{3,4} = 9.7 Hz, H-4_{C}), 3.42 (m, 1H, H_{Pr}), 3.38 (m, 1H, H-4_{E}), 3.37-3.29 (m, 4H, H-4_{B}, H-5_{D}, H-4_{D}, H-3_{D}), 3.26 (pt, 1H, *J*_{3,4} = 9.7 Hz, H-4_{A}), 2.08 (s, 3H, H_{Ac}), 2.00 (s, 3H, H_{NAc}), 1.53 (sex, 2H, CH₂), 1.22 (d, 3H, *J*_{5*,*6} = 6.2 Hz, H-6_{C}), 1.17 (m, 3H, *J*_{5,6} = 6.4 Hz, H-6_{B}), 1.15 (d, 3H, *J*_{5,6} = 6.2 Hz, H-6_{A}), 0.83 (t, 3H, *J=* 7.4 Hz, CH₃).
RMN ¹³C (D₂O), *δ*174.8 (C_{NAc}), 173.3 (C_{Ac}), 102.2 (C-1_{D}, ¹*J*_{CH} = 163.9 Hz), 101.7 (C-1_{A}, ¹*J*_{CH}= 173.4 Hz), 101.0 (C-1_{B}, *¹J*_{CH}= 172.7 Hz), 97.1 (C-1_{C}, *¹J_{CH}*= 172.0 Hz), 94.9 (C-1_{E}, ¹*J*_{CH}=170.5 Hz), 78.7 (C-2_{B}), 76.3 (C-4_{D}), 76.0 (C-3_{C}), 74.5 (C-3_{D}), 74.4 (C-2_{A}), 74.1 (C-3_{A}), 73.4 (C-3_{E}), 72.5 (C-4_{C}), 72.3 (C-2_{C}), 72.2 (C-4_{B}), 71.7 (C-5_{E}), 71.6 (C-2_{E}), 71.1 (C-4_{A}), 70.2 (C_{Pr}), 70.1 (C-5_{D}), 69.8 (C-3_{B}), 69.7 (3C, C-4_{E}, C-5_{A}, C-5_{B}), 68.9 (C-5_{C}), 61.0 (C-6_{D}), 60.6 (C-6_{E}), 56.0 (C-2_{D}), 23.0 (C_{NAc}), 22.3 (CH₂), 20.7 (C_{Ac}), 17.2, 17.0, 16.8 (C-6_{A}*, C-6_{B}*, C-6_{C}*), 10.2 (CH₃).

| | | |
|---|---|---|
| HRMS (ESI⁺) : | [M+H]⁺ C₃₇H₆₄NO₂₄ | m/z théorique : 906.3818 |
| | | m/z mesurée : 906.3831 |
| | [M+Na]⁺ C₃₇H₆₃NO₂₄Na | m/z théorique : 928.3638 |
| | | m/z mesurée : 928.3652 |

### Procédé 10 :

### ➢ Propyl (2-acétamido-2-désoxy-β-D-glucopyranosyl)-(1→2)-[α-D-lucopyranosyl-(1→3)]-α-L-rhamnopyranosyl-(1→2)-α-L-rhamnopyranosyl-(1→3)-α-L-rhamnopyranoside XIV :

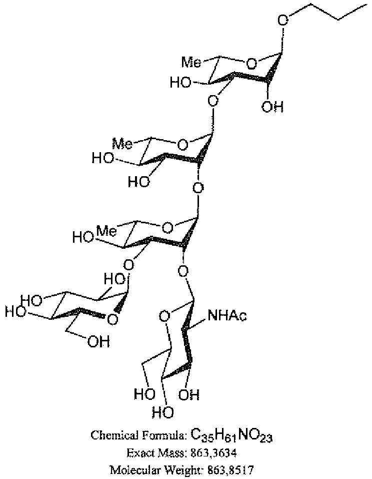

Du Pd-C 10% (250 mg) est ajouté à une solution dégazée de tétraol **92** (240 mg, 143 µmol) dans de l'éthanol (15 mL). La suspension est saturée avec de l'hydrogène à 50 bars et agitée à TA pendant 24 h. Le suivi par CCM (iPrOH/H₂O/NH₃, 4/1/0,5 et DCM/MeOH, 95/5) montre la disparition de **92** (Rf = 1 et 0,2, respectivement) et l'apparition d'un nouveau composé plus polaire (Rf = 0,15 et 0, respectivement). Le milieu réactionnel est filtré sur un filtre Acrodisc LC 25 mm, et le filtrat est concentré à l'évaporateur rotatif. Le résidu obtenu est purifié sur colonne C-18 (H₂O/CH₃CN, 100/0→7/3) pour conduire à la cible ***XIV*** sous forme d'un solide blanc (91 mg, 74%). Rf=0,15 (iPrOH/H₂O/NH₃, 4/1/0,5).
RMN ¹H (D₂O), *δ*5.17 (bs, 1H, H-1_{B}), 5.14 (d, 1H, *J*_{1,2} = 3.6 Hz, H-1_{E}), 5.06 (d, 1H, *J*_{1,2} = 1.2 Hz, H-1_{A}), 4.78 (d, 1H, *J*_{1,2}=8.5 Hz, H-1_{D}), 4.73 (bs, 1H, H-1_{C}), 4.39 (m, 1H, H-2_{A}), 4.03 (m, 1H, H-2_{B}), 4.00 (m, 1H, H-5_{E}), 3.95 (m, 1H, H-2_{C}), 3.90 (m, 1H, H-3_{B}), 3.89-3.85 (m, 2H, H-3_{A}, H-6a_{D}), 3.82-3.72 (m, 5H, H-3_{E}, H-6a_{E}, H-6b_{E}, H-5_{B}, H-3_{C}), 3.73-3.65 (m, 5H, H-2_{D}, H-6b_{D}, H-5_{A}, H-2_{E}, H-5_{C}), 3.61 (m, 1H, H_{Pr}), 3.51 (pt, 1H, *J*_{3,4} = 9.6 Hz, H-4_{C}), 3.47 (m, 1H, H_{Pr}), 3.45 (m, 1H, H-4_{E}), 3.43 (m, 1H, H-4_{B}), 3.41-3.3 (m, 3H, H-5_{D}, H-4_{D}, H-3_{D}), 3.32 (pt, 1H, *J*_{3,4} = 9.7 Hz, H-4_{A}), 2.06 (s, 3H, H_{NAc}), 1.57 (sex, 2H, CH₂), 1.25 (m, 6H, H-6_{B}, H-6_{C}), 1.22 (d, 3H, *J*_{5,6} = 6.2 Hz, H-6_{A}), 0.88 (t, 3H, *J =* 7.4 Hz, CH₃).
RMN ¹³C (D₂O), *δ* 174.8 (C_{NAc}), 102.3 (C-1_{D}, ¹*J*_{CH}=163.2 Hz), 101.7 (C-1_{A}, ¹*J*_{CH} = 170.5 Hz), 101.0 (C-1_{B}, ¹*J*_{CH} = 172.7 Hz), 99.9 (C-1_{C}, ¹*J*_{CH}=169.8 Hz), 95.0 (C-1_{E}, ¹*J*_{CH}=170.5 Hz), 78.9 (C-2_{B}), 77.8 (C-3_{C}), 76.3 (C-4_{D}), 74.6 (C-3_{D}), 74.5 (C-2_{A}), 74.2 (C-3_{A}), 73.5 (C-3_{E}), 72.6 (C-4_{B}), 72.5 (C-4_{C}), 71.8 (C-5_{E}), 71.7 (C-2_{E}), 71.2 (C-4_{A}), 70.4 (C-2_{C}), 70.2 (2C, C-5_{D}, C-3_{B}), 70.0 (C_{Pr}), 69.9 (2C, C-5_{A}, C-4_{E}), 69.5 (C-5_{B}), 69.0 (C-5_{C}), 61.1 (C-6_{D}), 60.7 (C-6_{E}), 56.0 (C-2_{D}), 23.0 (C_{NAc}), 22.4 (CH₂), 17.2, 17.1, 16.9 (C-6_{A}*, C-6_{B}*, C-6_{C}*), 10.3 (CH₃).

| | | |
|---|---|---|
| HRMS (ESI⁺) : | [M+H]⁺ C₃₅H₆₂NO₂₃ | m/z théorique : 864.3713 |
| | | m/z mesurée : 864.3726 |
| | [M+Na]⁺ C₃₅H₆₁NO₂₃Na | m/z théorique : 886.3532 |
| | | m/z mesurée : 886.3522 |

### Procédé 11 :

### ➢ (2,3,4,6-Tétra-O-benzyl-α-D-glucopyranosyl)-(1→3)-(4-O-benzyl-2-O-évulinoyl-α-L-rhamnopyranosyl)-(1→2)-(3,4-di-O-benzyl-α-L-rhamnopyranosyl)-(1→3)-2-0-acétyl-4-O-benzyl-α/β-L-rhamnopyranose88 :

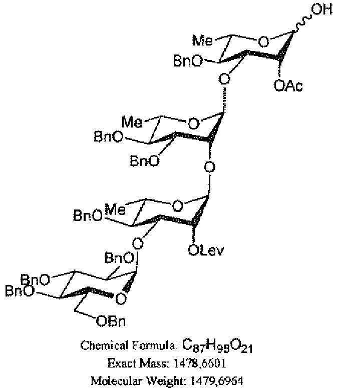

Le (1,5-cyclooctadiènebis(méthyldiphénylphosphine)iridium(I)hexafluorophosphate) (25 mg) est dissous dans du THF (90 mL) sous agitation. Le milieu subit 5 cycles dégazage vide/argon, 5 cycles dégazage vide/hyrdogène puis est mis sous hydrogène pendant 15 min. Le milieu subit alors une seconde fois 5 cycles dégazage vide/argon. La solution du glycoside d'allyle **87** (1,9 g, 1,2 mmol) dans du THF (15 mL) est transféré sur la solution de catalyseur activé. Le suivi par CCM (Tol/EtOAc, 9/1) montre la disparition de **87** (Rf=0,3) et l'apparition d'un produit un peu moins polaire (Rf=0,35).

Du diiode (340 mg, 2,5 mmol, 2 éq.) en solution dans un mélange THF/H₂O (8/2, 10 mL) est ajouté au milieu réactionnel. Le suivi par CCM (Tol/EtOAc, 9/1 et DCM/MeOH, 99/1) indique la disparition de l'intermédiaire (Rf=0,35 et 0,95, respectivement) et l'apparition d'un nouveau composé beaucoup plus polaire (Rf= 0,05 et 0,25, respectivement). Pour stopper la réaction, une solution aqueuse de NaHSO₃ 10% (2 mL) est ajoutée par fractions au milieu jusqu'à disparition de la coloration noire. Le milieu réactionnel est repris avec du DCM (50 mL) et la phase aqueuse est extraite au DCM (3 x 50 mL). Les phases organiques sont regroupées et lavées avec une solution de NaClₛₐₜ (3 x 25 mL), avec de l'H₂O (3 x 25 mL) puis séchées sur filtre séparateur de phases et concentrées à l'évaporateur rotatif. L'huile jaune obtenue est purifiée par chromatographie sur gel de silice (DCM/MeOH, 100/0-99/1) pour obtenir majoritairement l'anomère α de l'hémiacétal **88** sous forme d'une huile jaune (1,7 g, 90%).
Rf=0,25 (DCM/MeOH, 99/1).
RMN ¹H (CDCl₃), *δ*7.42-7.15 (m, 40H, CH_{Ph}), 5.56 (m, 1H, H-2_{A}), 5.26 (d, 1H, *J*_{1,2} = 3.4 Hz, H-1_{E}), 5.19 (m, 2H, H-2_{C}, H-1_{C}), 5.04 (d, 1H, *J*=10.9 Hz, H_{Bn}), 5.01 (d, 1H, *J*_{1,2}=1.2 Hz, H-1_{B}), 4.97 (d, 1H, *J*_{1,2} =1.4 Hz, H-1_{A}), 4.96-4.81 (m, 14H, H_{Bn}), 4.35 (d, 1H, *J* = 12.0 Hz, H_{Bη}), 4.28 (dd, 1H, *J*_{2,3} = 3.0 Hz, *J*_{3,4} = 9.6 Hz, H-3_{A}), 4.17-3.97 (m, 5H, H-3_{C}, H-3_{E}, H-5_{E}, H-5_{C}, H-2_{B}), 3.88 (m, 1H, H-5_{A}), 3.86-3.78 (m, 2H, H-3_{B}, H-4_{E}), 3.71 (m, 1H, H-5_{B}), 3.67-3.63 (m, 2H, H-2_{E}, H-6a_{E}), 3.56 (pt, 1H, *J*₃,₄ = 9.3 Hz, H-4_{A}), 3.55 (m, 1H, H-6b_{E}), 3.51 (pt, 1H, *J*_{3,4} = 9.4 Hz, H-4_{B}), 3.43 (pt, 1H, *J*_{3,4} = 9.4 Hz, H-4_{C}), 3.13 (m, 1H, OH), 2.53 (m, 4H, 2CH_{2Lev}), 2.16 (s, 3H, H_{Ac}), 2.10 (s, 3H, CH_{3Lev}), 1.34 (d, 3H, *J*_{5,6} = 6.2 Hz, H-6_{A}), 1.29 (d, 3H, *J*_{5,6} = 6.1 Hz, H-6_{B}), 1.27 (d, 3H, *J*_{5,6} = 6.2 Hz, H-6_{C}).
RMN ¹³C (CDCl₃), *δ*206.7 (C_{Lev}), 172.1 (C_{Lev}), 170.9 (C_{Ac}), 139.1-138.8 (C_{Ph}), 129.1-127.9 (CH_{Ph}), 101.6 (C-1_{B}, ¹*J*_{CH} = 171.8 Hz), 99.5 (C-1_{A}, ¹*J*_{CH} = 169.9 Hz), 93.2 (C-1_{E}, ¹*J*_{CH} = 169.6 Hz), 92.0 (C-1_{C}, ¹*J*_{CH} = 170.2 Hz), 82.5 (C-3_{E}), 80.6 (C-4_{B}), 80.2 (C-4_{A}), 80.1 (C-4_{C}), 79.7 (C-2_{E}), 79.6 (C-3_{B}), 78.8 (C-3_{C}), 78.1 (C-4_{E}), 76.6, 76.0 (2C, C_{Bn}), 75.8 (C-2_{B}), 75.8, 75.7, 75.4, 74.0 (4C, C_{Bn}), 73.2 (C-2_{C}), 73.1 (C_{Bn}), 72.6 (C-3_{A}), 72.4 (C_{Bn}), 70.5 (C-5_{E}), 69.3 (C-5_{B}), 69.0 (C-5_{A}), 68.6 (C-6_{E}), 68.4 (C-2_{A}), 68.1 (C-5_{C}), 38.3 (CH_{2Lev}), 30.1 (CH_{3Lev}), 28.5 (CH_{2Lev}), 21.5 (C_{Ac}), 18.4, 18.3, 18.2 (C-6_{A}*, C-6_{B}*, C-6_{C}*).

| | | |
|---|---|---|
| HRMS (ESI⁺) : | [M+Na]⁺ C₈₇H₉₈O₂₁Na | m/z théorique : 1501.6498 |
| | | m/z mesurée: 1501.6665 |
| | [M+NH₄]⁺ C₈₇H₉₈O₂₁NH₄ | m/z théorique : 1496.6945 |
| | | m/z mesurée : 1496.7114 |

### ➢ Trichloroacétimidate (2,3,4,6-tétra-O-benzyl-α-D-glucopyranosyl)-(1→3)-(4-O-benzyl-2-O-lévulinoyl-α-L-rhamnopyranosyl)-(1→2)-(3,4-di-O-benzyl-α-L-rhamnopyranosyl)-(1→3)-2-O-acétyl-4-O-benzyl-α/β-L-rhamnopyranose 86 :

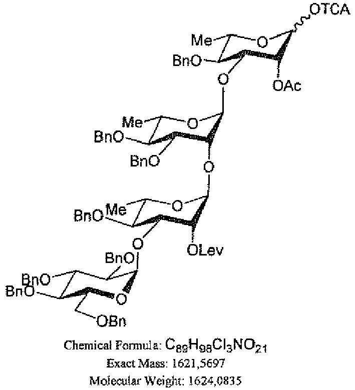

L'hémiacétal 88 (1,5 g, 1,0 mmol) est solubilisé dans du DCE (5 mL) et agité sous argon à -5°C puis de la DBU (42 µL, 280 µmol, 0,28 éq.) et du trichloroacétonitrile (520 µL, 5,2 mmol, 5 éq.) sont ajoutés. Le milieu réactionnel est agité à cette température pendant 15 min puis il est déposé, sans aucun traitement, sur une colonne de gel de silice (flash chromatographie, Chex/EtOAc+5% NEt₃, 8/2→7/3) pour obtenir le trichloroacétimidate **86** sous forme d'une huile jaune (1,5 g, 88%).
Rf =0,35 (Chex/EtOAc, 7/3).
RMN ¹H (CDCl₃), *δ*8.73 (s, 1H, NH), 7.37-7.14 (m, 40H, CH_{Ph}), 6.24 (d, 1H, *J*_{1,2} = 1.9 Hz, H-1_{C}), 5.56 (dd, 1H, *J*_{1,2} = 2.7 Hz, *J*_{1,2} = 4.9 Hz, H-2_{A}), 5.32 (dd, 1H, *J*_{1,2} = 2.2 Hz, *J*_{1,2} = 3.1 Hz, H-2_{C}), 5.26 (d, 1H, *J*_{1,2} = 3.4 Hz, H-1_{E}), 5.04 (d, 1H, *J* = 10.9 Hz, H_{Bn}), 5.03 (d, 1H, *J*_{1,2} = 1.3 Hz, H-1_{B}), 4.99 (d, 1H, *J*_{1,2} = 1.6 Hz, H-1_{A}), 4.97-4.79 (m, 6H, H_{Bn}), 4.73-4.49 (m, 8H, H_{Bn}), 4.35 (d, 1H, *J* = 12.0 Hz, H_{Bn}), 4.25 (dd, 1H, *J*_{2,3} = 3.1 Hz, *J*_{3,4} = 9.6 Hz, H-3_{A}), 4.19 (dd, 1H, *J*_{2,3} = 3.3 Hz, *J*_{3,4}= 9.5 Hz, H-3_{C}), 4.14 (pt, 1H, *J*_{3,4}= 9.3 Hz, H-3_{E}), 4.08 (m, 1H, H-5_{E}), 3.98-3.88 (m, 3H, H-2_{B}, H-5_{C}, H-5_{A}), 3.86-3.81 (m, 2H, H-3_{B}, H-4_{E}), 3.73 (m, 1H, H-5_{B}), 3.67-3.64 (m, 2H, H-2_{E}, H-6a_{E}), 3.58 (m, 1H, H-6b_{E}), 3.57 (pt, 1H!; *J*_{3,4} = 9.6 Hz, H-4_{A}), 3.54 (pt, 1H, *J*_{3,4} = 9.5 Hz, H-4_{C}), 3.52 (pt, 1H, *J*_{3,4} = 9.4 Hz, H-4_{B}), 2.55 (m, 4H, 2CH_{2Lev}), 2.19 (s, 3H, H_{Ac}), 2.10 (s, 3H, CH_{3Lev}), 1.34 (d, 3H, *J*_{5,6} = 6.3 Hz, H-6_{A}), 1.31 (d, 3H, *J*_{5,6} = 6.1 Hz, H-6_{B}), 1.28 (d, 3H, *J*_{5,6} = 6.0 Hz, H-6_{C}).

RMN ¹³C (CDCl₃), *δ*206.7 (C_{Lev}), 172.2 (C_{Lev}), 170.3 (C_{Ac}), 160.5 (C=NH), 139.1-138.8 (C_{Ph}), 129.1-127.9 (CH_{Ph}), 101.5 (C-1_{B}, ¹*J*_{CH} = 178.7 Hz), 99.8 (C-1_{A}, ¹*J*_{CH} = 174.2 Hz), 94.6 (C-1_{C}, ¹*J*_{CH} = 178.5 Hz), 93.3 (C-1_{E}, ¹*J*_{CH} = 170.0 Hz), 93.3 (CCl₃), 82.5 (C-3_{E}), 80.6 (C-4_{C}), 80.1 (C-4_{A}), 79.8 (C-4_{B}), 79.6 (C-2_{E}), 79.4 (C-3_{B}), 78.2 (C-3_{C}), 78.1 (C-4_{E}), 76.7 (C_{Bn}), 76.4 (C-2_{B}), 76.0, 75.9, 75.8, 75.4, 73.8, 73.2 (6C, C_{Bn}), 72.6 (C-3_{A}), 72.6 (C_{Bn}), 71.3 (C-2_{C}), 71.0 (C-5_{C}), 70.6 (C-5_{E}), 69.5 (C-5_{B}), 68.9 (C-5_{A}), 68.5 (C-6_{E}), 68.4 (C-2_{A}), 38.3 (CH_{2Lev}), 30.1 (CH_{3Lev}), 28.5 (CH_{2Lev}), 21.4 (C_{Ac}), 18.4, 18.3, 18.2 (C-6_{A}*, C-6_{B}*, C-6_{C}*).

### ➢ Allyl (2,3,4,6-tétra-O-benzyl-α-D-glacopyranosyl)-(1->3)-(4-O-benzyl-2-O-évulinoyl-α-L-rhamnopyranosyl)-(1→2)-(3,4-di-O-benzyl-α-L-rhamnopyranosyl)-(1→3)-(2-O-acétyl-4-O-benzyl-α-L-rhamnopyranosyl)-(1→3)-2-acétamido-2-désoxy-4,6-O-isopropylidène-β-D-glucopyranoside 93 :

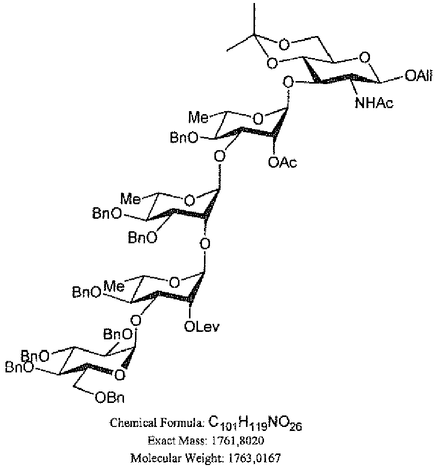

Du TfOH (45,0 µL, 515 µmol, 0,9 éq.) est additionné à une solution d'accepteur 19 (432 mg, 1,4 mmol, 2,5 éq.) et de donneur 86 (926 mg, 571 µmol, 1 éq.) dans du Tol (25 mL), en présence de tamis moléculaire 4 Å (3,2 g), agitée sous argon à 0°C. Le milieu réactionnel est alors chauffé à 75°C. Après 1 h à cette température, le suivi par CCM (Tol/EtOAc, 7/3) indique l'apparition d'un nouveau composé (Rf = 0,25). La réaction est stoppée par addition de triéthylamine (0,2 mL) puis le milieu réactionnel est filtré et concentré à l'évaporateur rotatif. L'huile jaune obtenue est purifiée par chromatographie sur gel de silice (Tol/EtOAc, 7/3→6/4) pour obtenir le glycoside d'allyle **93** sous forme d'un solide blanc (780 mg, 78%).
Rf = 0,45 (Tol/EtOAc, 6/4).
RMN ¹H (CDCl₃), *δ*7.42-7.10 (m, 40H, CH_{Ph}), 5.89 (m, 2H, NH, CH=), 5.56 (dd, 1H, *J*_{1,2} = 2.2 Hz, H-2_{A}), 5.30 (m, 1H, *J*ₜᵣₐₙₛ = 17.2 Hz, =CH₂), 5.25 (d, 1H, *J*_{1,2} = 3.4 Hz, H-1_{E}), 5.21 (m, 1H, *J*_{cis} = 10.4 Hz, =CH₂), 5.15 (d, 1H, *J*_{1,2} = 8.3 Hz, H-1_{D}), 5.07 (dd, 1H, *J*_{1,2} = 1.6 Hz, *J*_{1,2} = 3.2 Hz, H-2_{C}), 5.02 (d, 1H, *J =* 11.0 Hz, H_{Bn}), 4.98 (d, 1H, *J*_{1,2} = 1.5 Hz, H-1_{A}), 4.96 (d, 1H, *J*_{1,2} = 1.3 Hz, H-1_{B}), 4.97-4.86 (m, 4H, H_{Bn}), 4.80 (bs, 1H, H-1_{C}), 4.78 (d, 1H, *J*= 11.9 Hz, H_{Bn}), 4.73 (d, 1H*, J* = 11.5 Hz, H_{Bn}), 4.67 (d, 1H, *J* = 13.4 Hz, H_{Bn}), 4.62 (d, 1H, *J* = 12.3 Hz, H_{Bn}), 4.61-4.53 (m, 6H, H_{Bn}), 4.50 (d, 1H, *J =* 11.0 Hz, H_{Bn}), 4.39 (pt, 1H, *J*_{3,4} = 9.2 Hz, H-3_{D}), 4.31 (m, 1H, H_{All}), 4.26 (dd, 1H, *J*_{2,3} = 3.0 Hz, *J*_{3,4} = 9.6 Hz, H-3_{A}), 4.15-4.06 (m, 3H, H-3_{E}, H_{All}, H-5_{E}),4.04-3.94 (m, 4H, H-5_{C}, H-3_{C}, H-2_{B}, H-6a_{D}), 3.88 (dq, 1H, *J*_{4,5} = 9.5 Hz, H-5_{A}), 3.84-3.77 (m, 3H, H-3_{B}, H-4_{E}, H-6b_{D}), 3.71-3.62 (m, 3H, H-5_{B}, H-6a_{E}, H-2_{E}), 3.60-3.52 (m, 3H, H-6b_{E}, H-4_{A}, H-4_{D}), 3.50 (pt, 1H, *J*_{3,4} = 9.4 Hz, H-4_{B}), 3.40 (m, 1H, H-5_{D}), 3.38 (pt, 1H, *J*_{3,4} = 9.6 Hz, H-4_{C}), 3.49 (m, 1H, *J*_{2,NH} = 8.0 Hz, H-2_{D}), 2.53 (m, 4H, 2CH_{2Lev}), 2.11 (s, 3H, H_{NAc}), 2.09 (s, 3H, CH_{3Lev}), 2.04 (s, 3H, H_{Ac}), 1.50 (s, 3H, H_{iPr}), 1.42 (s, 3H, H_{iPr}), 1.35 (d, 3H, *J*_{5,6} = 6.2 Hz, H-6_{A}), 1.26 (d, 3H, *J*_{5,6} = 6.2 Hz, H-6_{B}), 1.20 (d, 3H, *J*_{5,6} = 6.2 Hz, H-6_{C}).
RMN ¹³C (CDCl₃), *δ*206.1 (C_{Lev}), 171.7 (C_{Lev}), 171.3 (C_{Ac}), 170.5 (C_{NAc}), 138.8-137.6 (C_{Ph}), 133.8 (CH=), 129.1-127.4 (CH_{Ph}), 117.5 (=CH₂), 101.2 (C-1_{B}, ¹*J*_{CH}= 169.4 Hz), 99.4 (C_{iPr}), 99.0 (C-1_{A}, ¹*J*_{CH}= 173.7 Hz), 98.9 (C-1_{D}, ¹*J*_{CH} = 162.7 Hz), 97.6 (C-1_{C}, ¹*J*_{CH} = 171.0 Hz), 92.8 (C-1_{E}, ¹*J*_{CH} = 167.6 Hz), 82.1 (C-3_{E}), 80.2 (C-4_{B}), 79.8 (C-4_{A}), 79.6 (C-4_{C}), 79.5 (C-2_{E}), 79.4 (2C, C-3_{B}*, C-3_{C}*), 77.8 (C-4_{E}), 76.3 (C_{Bn}), 76.1 (C-3_{D}), 75.6, 75.5, 75.3 (3C, C_{Bn}), 75.2 (C-2_{B}), 75.1, 75.0, 73.4 (3C, C_{Bn}), 73.1 (C-4_{D}), 72.8 (C-2_{C}), 72.8 (C_{Bn}), 72.2 (C-3_{A}), 72.0 (C_{Bn}), 70.5 (C_{All}), 70.3 (C-5_{E}), 69.0 (C-5_{B}), 68.6 (C-5_{A}), 68.3 (C-6_{E}), 68.0 (C-2_{A}), 67.6 (C-5_{C}), 67.0 (C-5_{D}), 62.3 (C-6_{D}), 59.6 (C-2_{D}), 37.9 (CH_{2Lev}), 29.7 (CH_{3Lev}), 29.1 (C_{iPr}), 28.1 (CH_{2Lev}), 23.5 (C_{Ac}), 21.2 (C_{NAc}), 19.3 (C_{iPr}), 18.0, 17.9, 17.8 (C-6_{A}*, C-6_{B}*, C-6_{C}*).

| | | |
|---|---|---|
| HRMS (ESI⁺) : | [M+Na]⁺ C)₁₀₁H₁₁₉NO₂₆Na | m/z théorique : 1784.7917 |
| | | m/z mesurée : 1784.7917 |
| | [M+H]⁺ C₁₀₁H₁₁₉NO₂₆ | m/z théorique : 1762.8098 |
| | | m/z mesurée : 1762.8038 |
| | [M+NH₄]⁺ C₁₀₁H₁₁₉NO₂₆NH₄ | m/z théorique : 1779.8364 |
| | | m/z mesurée : 1779.8289 |

### ➢ Allyl (2,3,4,6-tétra-O-benzyl-α-D-glucopyranosyl)-(1→3)-(4-O-benzyl-α-L-rhamnopyranosyl)-(1→2)-(3,4-di-O-benzyl-α-L-rhamnopyranosyl)-(1→3)-(2-O-acétyl-4-O-benzyl-α-L-rhamnopyranosyl)-(1→3)-2-acétamido-2-désoxy-4,6-O-isopropylidène-β-D-glucopyranoside 94 :

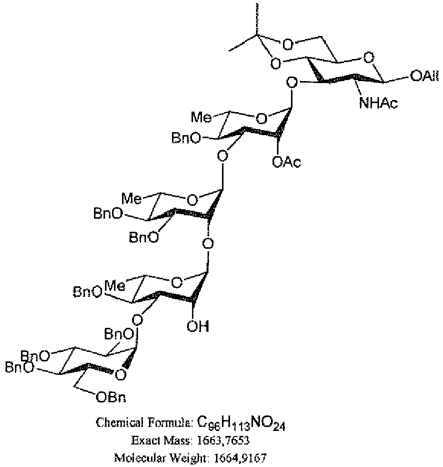

Le glycoside d'allyle **93** (720 mg, 409 µmol) est dissous dans un mélange pyridine/acide acétique (3/2, 20 mL). De l'hydrazine monohydraté (200 µL, 4,0 mmol, 10 éq.) est ajouté goutte à goutte au milieu réactionnel. Après 30 min d'agitation à 0°C, le milieu réactionnel est repris à l'eau froide (20 mL) et la phase aqueuse est extraite rapidement au DCM (3 x 100 mL). Les phases organiques sont regroupées et lavées avec une solution de NaClₛₐₜ (3 x 50 mL), filtrées sur filtre séparateur de phase et concentrées à l'évaporateur rotatif. L'huile obtenue est purifiée par chromatographie sur gel de silice (Tol/EtOAc, 9/1→75/25) pour obtenir l'alcool **94** sous forme d'un solide blanc (580 mg, 85%).
Rf=0,45 (Tol/EtOAc, 6/4).
RMN ¹H (CDCl₃), δ7.44-7.18 (m, 40H, CH_{Ph}), 5.96 (d, 1H, J_{NH,2} = 6.8 Hz, NH), 5.91 (m, 1H, CH=), 5.32 (m, 1H, *J*ₜᵣₐₙₛ = 17.2 Hz, =CH₂), 5.24 (m, 1H, *J*_{cis} = 10.4 Hz, =CH₂), 5.20 (bs, 1H, H-1_{A}), 5.16 (d, 1H, *J*_{1,2} = 8.4 Hz, H-1_{D}), 5.12 (dd, 1H, *J*_{1,2} = 1.6 Hz, *J*_{1,2} = 3.2 Hz, H-2_{C}), 5.03 (d, 1H, *J*_{1,2} = 1.3 Hz, H-1_{B}), 5.02-4.95 (d, 3H, *J* = 11.0 Hz, H_{Bn}), 4.95 (d, 1H, *J*_{1,2} = 3.9 Hz, H-1_{E}), 4.91-4.86 (m, 3H, H_{Bn}), 4.86 (bs, 1H, H-1c), 4.84-4.81 (m, 2H, H_{Bn}), 4.72-4.70 (m, 3H, H_{Bn}), 4.63-4.58 (m, 4H, H_{Bn}), 4.53 (d, 1H, *J=* 11.0 Hz, H_{Bn}), 4.41 (pt, 1H, *J*_{3,4}= 9.3 Hz, H-3_{D}), 4.34 (m, 1H, H_{All}), 4.15-3.96 (m, 9H, H_{All}, H-2_{B}, H-3_{E}, H-2_{A}, H-3_{A}, H-5_{C}, H-3_{C}, H-5_{E}, H-6a_{D}), 3.91 (dq, 1H, *J*_{4,5} = 9.6 Hz, H-5_{A}), 3.88 (dd, 1H, *J*_{2,3} = 2.7 Hz, *J*_{3,4} = 9.4 Hz, H-3_{B}), 3.83 (m, 1H, H-6b_{D}), 3.80 (pt, 1H, *J*_{3,4}= 10.1 Hz, H-4_{E}), 3.72 (dq, 1H, *J*_{4,5} = 9.4 Hz, H-5_{B}), 3.66 (dd, 1H, *J*_{2,3} = 3.6 Hz, *J*_{3,4} = 9.6 Hz, H-2_{E}), 3.62-3.56 (m, 2H, H-4_{D}, H-4_{A}), 3.54-3.40 (m, 5H, H-6a_{E}, H-4_{B}, H-6b_{E}, H-4_{C}, H-5_{D}) 3.16 (m, 1H, *J*_{2,NH} = 8.0 Hz, H-2_{D}), 2.16 (s, 3H, H_{Ac}), 2.09 (s, 3H, H_{NAc}), 1.54 (s, 3H, H_{iPr}), 1.46 (s, 3H, H_{iPr}), 1.38 (d, 3H, *J*_{5,6} = 6.2 Hz, H-6_{A}), 1.33 (d, 3H, *J*_{5,6}= 6.5 Hz, H-6_{B}), 1.26 (d, 3H, *J*_{*5*,*6*}= 6.2 Hz, H-6_{C}).
RMN ¹³C (CDCl₃), δ171.3 (C_{NAc}), 170.5 (C_{Ac}), 138.7-137.6 (C_{Ph}), 133.9 (CH=), 129.1-127.7 (CH_{Ph}), 117.6 (=CH₂), 101.6 (C-1_{B}, ¹*J*_{CH} = 171.0 Hz), 100.8 (C-1_{A}, ¹*J*_{CH} = 175.0 Hz), 99.4 (C_{iPr}), 99.0 (C-1_{D}, *¹J*_{CH} = 160.7 Hz), 97.6 (C-1_{C}, ¹*J*_{CH} = 170.8 Hz), 94.0 (C-1_{E}, ¹*J*_{CH} = 166.2 Hz), 82.4 (C-3_{E}), 80.4 (C-4_{B}), 79.7 (C-4_{c}, C-3_{B}), 79.5 (C-3_{C}), 79.3 (C-4_{A}), 79.0 (C-2_{E}), 77.8 (C-4_{E}), 76.5 (C-3_{A}), 76.2 (C-3_{D}), 75.7, 75.6, 75.4, 75.3, 75.0 (5C, C_{Bn}), 74.9 (C-2_{B}), 74.5, 73.5 (2C, C_{Bn}), 73.1 (C-4_{D}), 72.9 (C-2_{C}), 72.3 (C_{Bn}), 70.8 (C-5_{E}), 70.5 (C_{All}), 69.0 (C-5_{B}), 68.0 (C-6_{E}), 67.9 (C-5_{A}), 67.6 (C-5_{C}), 67.4 (C-2_{A}), 67.1 (C-5_{D}), 62.3 (C-6_{D}), 59.7 (C-2_{D}), 29.2 (C_{iPr}), 23.6 (C_{NAc}), 21.2 (C_{Ac}), 19.3 (C_{iPr}), 18.0, 17.9, 17.8 (C-6_{A}*, C-6_{B}*, C-6_{C}*).

| | | |
|---|---|---|
| HRMS (ESI⁺) : | [M+Na]⁺ C₉₆H₁₁₃NO₂₄Na | m/z théorique : 1686.7550 |
| | | m/z mesurée : 1686.7488 |
| | [M+H⁺ C₉₆H₁₁₃NO₂₄ | m/z théorique : 1664.7731 |
| | | m/z mesurée : 1664.7709 |
| | [M+NH₄]⁺ C₉₆H₁₁₃NO₂₄NH₄ | m/z théorique : 1681.7997 |
| | | m/z mesurée : 1681.7993 |

### ➢ Allyl (2,3,4,6-tétra-O-benzyl-α-D-glucopyranosyl)-(1→3)-(4-O-benzyl-α-L-rhamnopyranosyl)-(1→2)-(3,4-di-O-benzyl-α-L-rhamnopyranosyl)-(1→3)-(2-O-acétyl-4-O-benzyl-α-L-rhamnopyranosyl)-(1→3)-2-acétamido-2-désoxy-β-D-glucopyranoside 95 :

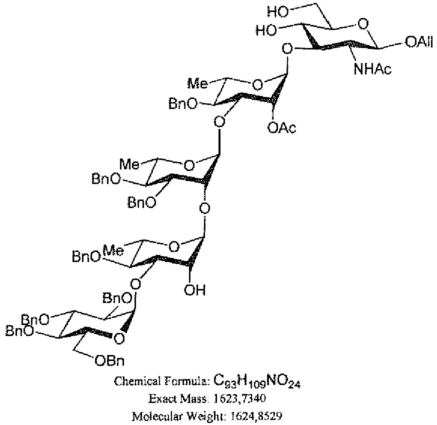

Une solution aqueuse de TFA 50% (4 mL) est additionnée lentement à une solution d'alcool 94 (400 mg, 240 µmol) dans du DCM (10 mL) à 0°C. Le milieu réactionnel est agité pendant 2 h à cette température, temps au bout duquel le suivi par CCM (Tol/EtOAc, 4/6) indique la disparition totale de **94** (Rf = 0,6) et l'apparition d'un nouveau composé plus polaire (Rf = 0,2). Le milieu réactionnel est concentré par coévaporation avec du Tol (3 x 10 mL). L'huile jaune obtenue est purifiée par chromatographie sur gel de silice (DCM/MeOH, 98/2→9/1) pour obtenir le triol **95** solide blanc correspondant au tétrasaccharide attendu (360 mg, 92%).
Rf = 0,55 (DCM/MeOH, 95/5).
RMN ¹H (CDCl₃), *δ* 7.42-7.13 (m, 40H, CH_{Ph}), 6.09 (m, 1H, NH), 5.91 (m, 1H, CH=), 5.27 (m, 1H, =CH₂), 5.22 (bs, 1H, H-1_{A}), 5.18 (m, 1H, *J*_{cis} = 10.4 Hz, =CH₂), 5.16 (m, 2H, H-1_{C}, H-2_{C}), 5.11 (d, 1H, *J*_{1,2} = 8.3 Hz, H-1_{D}), 5.10-4.88 (m, 8H, H-1_{B}, H-1_{E}, H_{Bn}), 4.83-4.49 (m, 9H, H_{Bn}), 4.35-4.29 (m, 2H, H_{All}, H_{Bn}), 4.14-3,96 (m, 9H, H-3_{E}, H_{All}, H-2_{A}, H-5_{C}, H-3_{A}, H-3_{C}, H-5_{E}, H-5_{A}), 3.87-3.73 (m, 4H, H-3_{B}, H-6a_{D}, H-6b_{D}, H-4_{E}), 3.68 (m, 1H, H-5_{B}), 3.65 (dd, 1H, *J*_{2,3} = 3.6 Hz, *J*_{3,4} = 9.6 Hz, H-2_{E}), 3.59 (d, 1H, *J*_{4,5} = 9.3 Hz, H-4_{A}), 3.54-3.36 (m, 6H, H-6a_{E}, H-5_{D}, H-4_{B}, H-4_{C}, H-6b_{E}, H-4_{D}), 3.16 (m, 1H, H-2_{D}), 2.14 (s, 3H, H_{Ac}), 2.10 (s, 3H, H_{NAc}), 1.25 (m, 9H, H-6_{A}, H-6_{B}, H-6_{C}).
RMN ¹³C (CDCl₃), *δ* 170.5 (2C, C_{NAc}, C_{Ac}), 138.6-137.5 (C_{Ph}), 133.9 (CH=), 128.6-127.5 (CH_{Ph}), 117.7 (=CH₂), 101.3 (C-1_{B}), 100.8 (C-1_{A}, C-1_{C}, ¹*J*_{CH} = 168.2 Hz), 98.4 (C-1_{D}, ¹*J*_{CH} = 169.0 Hz), 94.0 (C-1_{E}, ¹*J*_{CH} = 166.3 Hz), 82.4 (C-3_{E}), 80.2 (C-4_{C}), 79.4-79.1 (C-4_{B}*, C-3_{B}*, C-3_{C}*, C-4_{A}*), 79.0 (C-2_{E}), 77.8 (C-4_{E}), 76.5 (C-3_{A}), 75.9, 75.6, 75.4 (3C, C_{Bn}), 75.1 (C-4_{D}), 74.9, 74.5, 74.5, 73.5, 73.5 (5C, C_{Bn}), 72.2 (C-2_{C}), 70.8 (C-5_{E}), 70.5 (C_{All}), 70.3 (C-5_{D}), 69.0 (C-5_{B}), 68.0 (C-5_{A}), 67.9 (C-6_{E}), 67.4 (C-5_{C}, C-2_{A}), 62.3 (C-6_{D}), 59.7 (C-2_{D}), 23.2 (C_{NAc}), 21.1 (C_{Ac}), 18.3, 17.9, 17.7 (C-6_{A}*, C-6_{B}*, C-6_{C}*).

| | | |
|---|---|---|
| HRMS (ESI⁺) : | [M+Na]⁺ C₉₃H₁₀₉NO₂₄Na | m/z théorique : 1646.7238 |
| | | m/z mesurée : 1646.7377 |
| | [M+H]⁺ C₉₃H₁₀₉NO₂₄ | m/z théorique : 1624.7418 |
| | | m/z mesurée : 1624.7565 |
| | [M+NH₄]⁺ C₉₃H₁₀₉NO₂₄NH₄ | m/z théorique : 1641.7683 |
| | | m/z mesurée : 1641.7848 |

### ➢ Propyl α-D-glucopyranosyl-(1→3)-α-L-rhamnopyranosyl-(1→2)-α-L-rhamnopyranosyl-(1→3)-(2-O-acétyl-α-L-rhamnopyranosyl)-(1→3)-2-acétamido-2-désoxy-β-D-glucopyranoside XV :

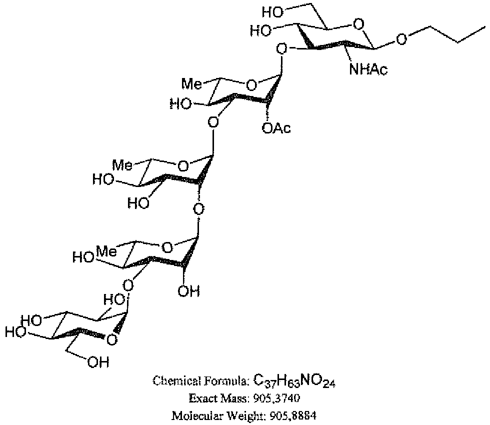

Du Pd-C 10% (200 mg) est ajouté à une solution dégazée de triol 95 (230 mg, 142 µmol) dans de l'éthanol (15 mL). La suspension est saturée avec de l'hydrogène et agitée à TA pendant 2 j. Le suivi par CCM (iPrOH/H₂O/NH₃, 4/1/0,5 et DCM/MeOH, 95/5) montre la disparition de **95** (Rf = 1 et 0,55, respectivement) et l'apparition d'un nouveau composé plus polaire (Rf = 0,35 et 0, respectivement). Le milieu réactionnel est filtré sur un filtre Acrodisc LC 25 mm, et le filtrat est concentré à l'évaporateur rotatif. Le résidu obtenu est purifié sur colonne C-18 (H₂O/CH₃CN, 100/0→7/3) pour conduire à la cible ***XV*** sous forme d'un solide blanc (100 mg, 77%).
Rf = 0,35 (iPrOH/H₂O/NH₃, 4/1/0,5).
RMN ¹H (D₂O), *δ* 5.03 (bs, 1H, H-1_{B}), 5.00 (d, 1H, *J*_{1,2} = 3.7 Hz, H-1_{E}), 4.90 (m, 2H, H-1_{A}, H-2_{C}), 4.76 (bs, 1H, H-1_{C}), 4.40 (d, 1H, *J*_{1,2} = 8.6 Hz, H-1_{D}), 4.17 (m, 1H, H-2_{A}), 3.99 (m, 1H, *J*_{5,6} = 6.3 Hz, *J*_{4,5} = 9.8 Hz, H-5_{C}), 3.92 (dd, 1H, *J*_{2,3} = 5.2 Hz, H-2_{B}), 3.88-3.80 (m, 3H, H-5_{E}, H-3_{C}, H-6a_{D}), 3.76-3.61 (m, 9H, H-3_{A}, H_{Pr}, H-2_{D}, H-6a_{E}, H-6b_{E}, H-3_{E}, H-5_{A}, H-3_{B}, H-6b_{D}), 3.51-3.40 (m, 7H, H-4_{C}, H-4_{A}, H-2_{E}, H-4_{D}, H_{Pr}, H-3_{D}, H-5_{B}), 3.38-3.31 (m, 3H, H-4_{E}, H-4_{B}, H-5_{D}), 2.07 (s, 3H, H_{Ac}), 1.94 (s, 3H, H_{NAc}), 1.49 (sex, 2H, *J* = 7.1 Hz, CH₂), 1.19-1.14 (m, 9H, H-6_{A}, H-6_{B}, H-6_{C}), 0.76 (t, 3H, *J* = 7.4 Hz, CH₃).
RMN ¹³C (D₂O), *δ* 174.4 (C_{NAc}), 173.0 (C_{Ac}), 101.9 (C-1_{A}, ¹*J*_{CH} = 175.6 Hz), 101.0 (C-1_{B}, ¹*J*_{CH} = 172.4 Hz), 100.6 (C-1_{D}), ¹*J*_{CH} = 162.7 Hz), 98.5 (C-1_{C}, ¹*J*_{CH} = 173.4 Hz), 95.3 (C-1_{E}, ¹*J*_{CH} = 170.3 Hz), 82.4 (C-3_{D}), 78.0 (C-2_{B}), 76.2 (C-3_{C}), 76.0 (C-5_{D}), 75.2 (C-3_{A}), 72.9 (C-3_{E}), 72.2 (C_{Pr}), 72.2 (C-2_{C}), 71.8 (C-4_{B}), 71.6 (C-5_{E}), 71.6 (C-4_{C}), 71.4 (C-4_{A}), 70.2 (C-4_{D}), 69.9 (C-3_{B}), 69.4 (C-2_{E}), 69.3 (C-4_{E}), 69.3 (C-5_{A}), 68.8 (C-5_{C}), 68.3 (C-5_{B}), 66.6 (C-2_{A}), 60.7 (C-6_{D}), 60.2 (C-6_{E}), 55.2 (C-2_{D}), 22.2 (C_{NAc}), 22.1 (CH₂), 20.2 (C_{Ac}), 16.7 (C-6_{A}), 16.5 (C-6_{B}), 16.3 (C-6_{C}), 9.6 (CH₃).

| | | |
|---|---|---|
| HRMS (ESI⁺) : | [M+H]⁺ C₃₇H₆₃NO₂₄ | m/z théorique : 906.3818 |
| | | m/z mesurée : 906.3821 |
| | [M+Na]⁺ C₃₇H₆₃NO₂₄Na | m/z théorique : 928.3638 |
| | | m/z mesurée : 928.3635 |
| | [M+K]⁺ C₃₇H₆₃NO₂₄K | m/z théorique : 944.3377 |
| | | m/z mesurée : 944.3279 |

### Procédé 12 :

### ➢ Allyl (2,3,4,6-tétra-O-benzyl-β-D-glucopyranosyl)-(1→3)-(4-O-benzyl-α-L-rhamnopyranosyl)-(1→2)-(3,4-di-O-benzyl-α-L-rhamnopyranosyl)-(1→3)-(4-O-benzyl-α-L-rhamnopyranosyl)-(1→3)-2-acétamido-2-désoxy-4,6-O-isopropylidène-β-D-glucopyranoside 96 :

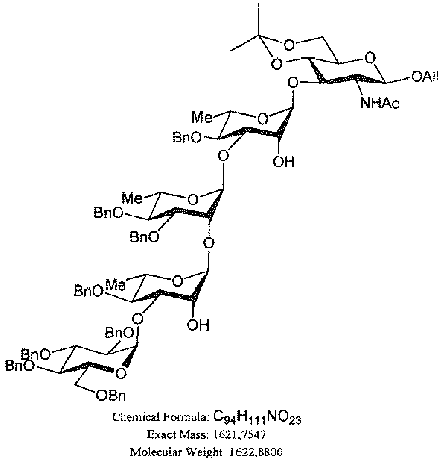

Après addition de NaOMe 0,5 M (830 µL, 414 µmol, 1 éq.) au glycoside d'allyle **93** (729 mg, 414 µmol) en solution dans du MeOH (20 mL). Le milieu réactionnel est porté à reflux et agité pendant 3 h, temps au bout duquel le suivi par CCM (Tol/EtOAc, 1/1), indique la disparition de **93** (Rf = 0,5) et l'apparition d'un produit plus polaire (Rf = 0,2). Après retour à TA, le milieu réactionnel est neutralisé par addition de résine échangeuse d'ion DOWEX (H⁺), puis filtré sur fritté. La résine est lavée avec un mélange DCM/MeOH et les solvants sont évaporés. L'huile jaune obtenue est purifiée par chromatographie sur gel de silice (Tol/EtOAc, 1/1→4/6) pour conduire au diol **96** sous forme d'un solide blanc (631 mg, 94%).
Rf = 0,2 (Tol/EtOAc, 1/1).
RMN ¹H (CDCl₃), *δ* 7.41-7.16 (m, 40H, CH_{Ph}), 5.90 (m, 1H, CH=), 5.81 (d, 1H, *J*_{NH,2} = 7.9 Hz, NH), 5.30 (m, 1H, *J*ₜᵣₐₙₛ = 17.2 Hz, =CH₂), 5.22 (m, 1H, *J*_{cis} = 10.4 Hz, =CH₂), 5.17 (bs, 1H, H-1_{B}), 5.07 (bs, 1H, H-1_{A}), 5.02-4.97 (m, 2H, H_{Bn}), 4.94 (d, 1H, *J*_{1,2} = 4.0 Hz, H-1_{E}), 4.90-4.86 (m, 4H, 2H_{Bn}, H-1_{D}, H-1_{C}), 4.80-4.66 (d, 8H, H_{Bn}), 4.59-4.51 (m, 3H, H_{Bn}), 4.38-4.32 (m, 2H, H_{All}, H_{Bn}), 4.21 (pt, 1H, *J*_{3,4} = 9.4 Hz, H-3_{D}), 4.12-4.05 (m, 6H, H_{All}, H-2_{B}, H-3_{E}, H-2_{A}, H-3_{A}, H-5_{C}), 3.99-3.84 (m, 7H, H-2_{C}, H-5_{E}, H-6a_{D}, H-3_{B}*, H-3_{C}*, H-5_{A}, H-5_{B}), 3.83 (m, 1H, H-6b_{D}), 3.78 (pt, 1H, *J*_{3,4} = 9.6 Hz, H-4_{E}), 3.66-3.61 (m, 2H, H-2_{E}, H-4_{D}), 3.57-3.52 (m, 2H, H-4_{A}, H-4_{B}), 3.52-3.49 (m, 2H, H-2_{D}, H-6a_{E}), 3.43-3.35 (m, 3H, H-6b_{E}, H-4_{C}, H-5_{D}), 2.02 (s, 3H, H_{NAc}), 1.53 (s, 3H, H_{iPr}), 1.43 (s, 3H, H_{iPr}), 1.41, 1.28 (m, 6H, H-6_{A}*, H-6_{B}*), 1.23 (d, 3H, *J*_{5,6} = 6.2 Hz, H-6_{C}).
RMN ¹³C (CDCl₃), *δ* 170.4 (C_{NAc}), 138.6-137.6 (C_{Ph}), 133.8 (CH=), 129.1-127.5 (CH_{Ph}), 117.6 (=CH₂), 101.0 (C-1_{A}, ¹*J*_{CH} = 168.4 Hz), 100.9 (C-1_{B}, ¹*J*_{CH} = 172.3 Hz), 99.8 (C-1_{C}, ¹*J*_{CH} = 170.6 Hz), 99.6 (C-1_{D}, ¹*J*_{CH} = 160.5 Hz), 99.5 (C_{iPr}), 94.0 (C-1_{E}, ¹*J*_{CH} = 167.5. Hz), 82.4 (C-3_{E}), 80.4 (1C, C-4_{A}*, C-4_{B}*), 80.3 (1C, C-3_{B}*, C-3_{C}*), 79.7 (C-4_{C}), 79.5 (1C, C-3_{B}*, C-3_{C}*), 79.3 (1C, C-4_{A}*, C-4_{B}*), 79.0 (C-2_{E}), 77.8 (C-4_{E}), 76.5 (C-3_{A}), 76.0 (C-3_{D}), 75.6 (3C, C_{Bn}), 75.4 (C-2_{A}), 75.0, 74.9, 74.4, 73.5 (4C, C_{Bn}), 73.0 (C-4_{D}), 72.5 (C_{Bn}), 71.2 (C-2_{C}), 70.7 (C-5_{E}), 70.0 (C_{All}), 69.1 (1C, C-5_{A}*, C-5_{B}*), 68.0 (C-6_{E}), 67.9 (1C, C-5_{A}*, C-5_{B}*), 67.7 (C-5_{C}), 67.4 (C-2_{B}), 67.3 (C-5_{D}), 62.3 (C-6_{D}), 58.2 (C-2_{D}), 29.1 (C_{iPr}), 23.5 (C_{NAc}), 19.2 (C_{iPr}), 18.0, 17.8 (3C, C-6_{A}*, C-6_{B}*, C-6_{C}*).

| | | |
|---|---|---|
| HRMS (ESI⁺) : | [M+H]⁺ C₉₄H₁₁₁NO₂₃ | m/z théorique : 1622.7626 |
| | | m/z mesurée : 1622.7649 |
| | [M+Na]⁺ C₉₄H₁₁₁NO₂₃Na | m/z théorique : 1644.7445 |
| | | m/z mesurée : 1644.7562 |

### ➢ Allyl (2,3,4,6-tétra-O-benzyl-α-D-glucopyranosyl)-(1→3)-(4-O-benzyl-α-L-rhamnopyranosyl)-(1→2)-(3,4-di-O-benzyl-α-L-rhamnopyranosyl)-(1→3)-(4-O-benzyl-α-L-rhamnopyranosyl)-(1→3)-2-acétamido-2-désoxy-β-D-glucopyranoside 97 :

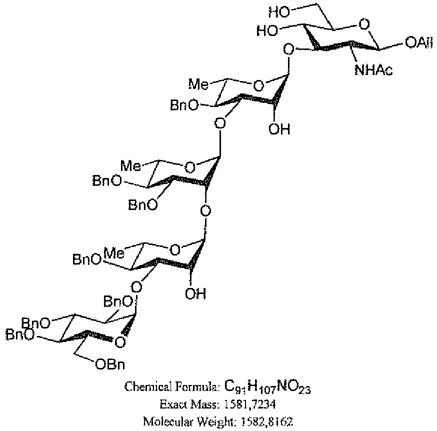

Une solution aqueuse de TFA 50% (4 mL) est additionnée lentement à une solution de diol **96** (515 mg, 318 µmol) dans du DCM (10 mL) à 0°C. Le milieu réactionnel est agité pendant 2 h à cette température, temps au bout duquel le suivi par CCM (DCM/MeOH, 95/5) indique la disparition totale de **96** (Rf = 0,35) et l'apparition d'un nouveau composé plus polaire (Rf = 0,2). Le milieu réactionnel est concentré par coévaporation avec du Tol (3 x 10 mL). L'huile jaune obtenue est purifiée par chromatographie sur gel de silice (Tol/EtOAc, 3/7**→**1/9) pour obtenir au tétraol 97 sous forme d'un solide blanc (330 mg, 66%).
Rf = 0,2 (DCM/MEOH, 95/5).
RMN ¹H (CDCl₃), *δ* 7.42-7.13 (m, 40H, CH_{Ph}), 5.90 (m, 1H, CH=), 5.78 (d, 1H, *J*_{NH,2} = 7.2 Hz, NH), 5.30 (m, 1H, *J*ₜᵣₐₙₛ = 17.3 Hz, =CH₂), 5.21 (m, 1H, *J*_{cis} = 10.4 Hz, =CH₂), 5.17 (bs, 1H, H-1_{B}), 5.01-4.94 (m, 4H, H-1_{D}, H-1_{A}, 2H_{Bn}), 4.92 (d, 1H, *J*_{1,2} = 3.7 Hz, H-1_{E}), 4.89-4.86 (m, 2H, H_{Bn}), 4.83 (m, 1H, *J*_{1,2} = 2.4 Hz, H-1_{C}), 4.78-4.65 (d, 7H, H_{Bn}), 4.57-4.49 (m, 3H, H_{Bn}), 4.37-4.20 (m, 4H, H_{All}, 2H_{Bn}, H-3_{D}), 4.13 (m, 1H, H_{All}), 4.09-4.02 (m, 4H, H-3_{E}, H-2_{B}, H-2_{A}, H-3_{A}), 4.00-3.91 (m, 5H, H-2_{C}, H-5_{E}, H-5_{B}, H-3_{B}, H-6a_{D}), 3.88-3.82 (m, 4H, H-3_{C}, H-5_{C}, H-5_{A}, H-6b_{D}), 3.77 (pt, 1H, *J*_{3,4} = 9.7 Hz, H-4_{E}), 3.63 (dd, 1H, *J*_{2,3} = 9.6 Hz, H-2_{E}), 3.56-3.49 (m, 3H, H-4_{A}, H-6a_{E}, H-4_{B}), 3.47-3.37 (m, 4H, H-4_{D}, H-5_{D}, H-4_{C}, H-6b_{E}), 3.15 (m, 1H, H-2_{D}), 1.97 (s, 3H, H_{NAc}), 1.34-1.28 (m, 9H, H-6_{A}*, H-6_{B}*, H-6_{C}*).
RMN ¹³C (CDCl₃), *δ* 170.6 (C_{NAc}), 138.6-137.5 (C_{Ph}), 133.9 (CH=), 128.6-127.5 (CH_{Ph}), 117.5 (=CH₂), 101.5 (C-1_{C}, ¹*J*_{CH} = 171.3 Hz), 100.9 (C-1_{A}, ¹*J*_{CH} = 168.8 Hz), 100.9 (C-1_{B}, ¹*J*_{CH} = 168.8 Hz), 98.7 (C-1_{D}, ¹*J*_{CH} = 163.1 Hz), 94.0 (C-1_{E}, ¹*J*_{CH} = 165.5. Hz), 84.1 (C-3_{D}), 82.4 (C-3_{E}), 80.2 (C-4_{B}), 79.8 (C-3_{B}), 79.4 (C-3_{C}), 79.3 (C-4_{A}), 79.2 (C-4_{C}), 79.0 (C-2_{E}), 77.8 (C-4_{E}), 76.5 (C-3_{A}), 75.8, 75.6, 75.5 (3C, C_{Bn}), 75.1 (C-4_{D}), 75.0 (C-2_{A}), 75.0, 74.5, 73.4, 72.6 (5C, C_{Bn}), 71.1 (C-5_{D}), 70.8 (2C, C-2_{C}, C-5_{E}), 70.3 (C_{All}), 69.3 (C-5_{B}), 69.0 (C-5_{C}), 68.0 (C-6_{E}), 67.9 (C-5_{A}), 67.3 (C-2_{B}), 62.8 (C-6_{D}), 57.3 (C-2_{D}), 23.6 (C_{NAc}), 18.0, 17.9, 17.8 (C-6_{A}*, C-6_{B}*, C-6_{C}*).

| | | |
|---|---|---|
| HRMS (ESI⁺) : | [M+H]⁺ C₉₁H₁₀₇NO₂₃ | m/z théorique : 1582.7312 |
| | | m/z mesurée: 1582.7300 |
| | [M+Na]⁺ C₉₁H₁₀₇NO₂₃Na | m/z théorique : 1604.7131 |
| | | m/z mesurée: 1604.7180 |

### ➢ Propyl α-D-glucopyranosyl-(1→3)-α-L-rhamnopyranosyl-(1→2)-α-L-rhamnopyranosyl-(1→3)-α-L-rhamnopyranosyl-(1→3)-2-acétamido-2-désoxy-β-D-glucopyranoside- XVI :

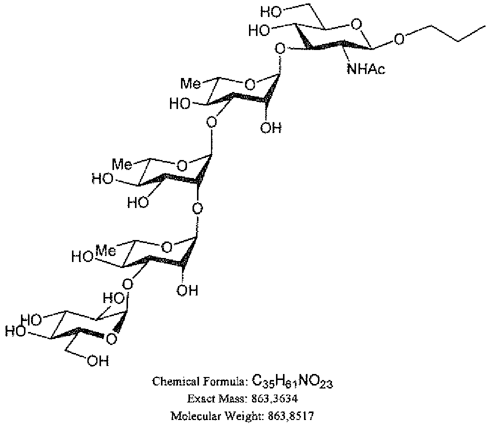

Du Pd-C 10% (200 mg) est ajouté à une solution dégazée de tétraol **97** (260 mg, 164 µmol) dans de l'éthanol (15 mL). La suspension est saturée avec de l'hydrogène et agitée à TA pendant 2 j. Le suivi par CCM (iPrOH/H₂O/NH₃, 4/1/0,5 et DCM/MeOH, 95/5) montre la disparition de **97** (Rf = 1 et 0,2, respectivement) et l'apparition d'un nouveau composé plus polaire (Rf = 0,25 et 0, respectivement). Le milieu réactionnel est filtré sur un filtre Acrodisc LC 25 mm, et le filtrat est concentré à l'évaporateur rotatif. Le résidu obtenu est purifié sur colonne C-18 (H₂O/CH₃CN, 100/0→7/3) pour à la cible ***XVI*** sous forme d'un solide blanc (99 mg, 70%).
Rf = 0,25 (iPrOH/H₂O/NH₃, 4/1/0,5).
RMN ¹H (D₂O), *δ* 5.15 (bs, 1H, H-1_{B}), 5.08 (d, 1H, *J*_{1,2} = 3.8 Hz, H-1_{E}), 4.98 (d, 1H, *J*_{1,2} = 1.5 Hz, H-1_{A}), 4.79 (d, 1H, *J*_{1,2} = 1.2 Hz, H-1_{C}), 4.50 (d, 1H, *J*_{1,2} = 8.6 Hz, H-1_{D}), 4.17 (dd, 1H, *J*_{2,3} = 2.6 Hz, H-2_{A}), 4.03 (dd, 1H, *J*_{2,3} = 3.0 Hz, H-2_{B}), 3.99 (m, 1H, *J*_{4,5} = 9.8 Hz, H-5_{C}), 3.94 (m, 1H, H-5_{E}), 3.92-3.87 (m, 2H, H-3_{B}, H-6a_{D}), 3.85-3.68 (m, 10H, H-3_{A}, H-2_{C}, H_{Pr}, H-2_{D}, H-6a_{E}, H-6b_{E}, H-3_{E}, H-3_{C}, H-6b_{D}, H-5_{A}, H-5_{B}), 3.58-3.40 (m, 9H, H-3_{D}, H-4_{A}, H-2_{E}, H_{Pr}, H-4_{C}, H-4_{D}, H-4_{B}, H-4_{E}, H-5_{D}), 2.01 (s, 3H, H_{NAc}), 1.51 (sex, 2H, *J* = 7.2 Hz, CH₂), 1.29-1.24 (m, 6H, H-6_{A}*, H-6_{B}*), 1.20 (d, 3H, *J*_{5,6} = 6.2 Hz, H-6_{C}), 0.83 (t, 3H, *J* = 7.4 Hz, CH₃).
RMN ¹³C (D₂O), *δ* 174.1 (C_{NAc}), 102.0 (C-1_{A}, ¹*J*_{CH} = 171.5 Hz), 101.3 (C-1_{C}, ¹*J*_{CH} = 173.6 Hz), 100.8 (C-1_{B}, ¹*J*_{CH} = 177.8 Hz), 100.6 (C-1_{D}, ¹*J*_{CH} = 156.2 Hz), 95.4 (C-1_{E}, ¹*J*_{CH} = 169.4 Hz), 81.7 (C-3_{D}), 78.3 (C-2_{B}), 77.3 (C-3_{C}), 76.0 (C-5_{D}), 75.3 (C-3_{A}), 73.0 (C-3_{E}), 72.3 (C_{Pr}), 72.2 (C-4_{B}), 71.7 (2C, C-4_{C}, C-5_{E}), 71.4 (C-4_{A}), 70.6 (C-2_{C}), 70.3 (C-2_{E}), 69.9 (C-3_{B}), 69.4 (C-4_{E}), 69.3 (2C, C-5_{A}, C-5_{B}), 68.9 (C-5_{C}), 68.5 (C-4_{D}), 66.7 (C-2_{A}), 60.9 (C-6_{D}), 60.3 (C-6_{E}), 55.4 (C-2_{D}), 22.1 (2C, C_{NAc}, CH₂), 20.2 (C_{Ac}), 16.8, 16.7 (C-6_{A}*, C-6_{B}*), 16.4 (C-6_{C}), 9.6 (CH₃).

| | | |
|---|---|---|
| HRMS (ESI⁺) : | [M+H]⁺ C₃₅H₆₁NO₂₃ | m/z théorique : 864.3713 |
| | | m/z mesurée : 864.3718 |
| | [M+Na]⁺ C₃₅H₆₁NO₂₃Na | m/z théorique : 886.3532 |
| | | m/z mesurée : 886.3501 |

### Partie IV - Synthèse des cibles VII, VIII, IX, XVII et XVIII

Outre les tri- (*VII*), tétra- (*VIII*) et pentasaccharides (*IX*) comportant un résidu B à leur extrémité réductrice, nous nous sommes, dans cette partie, attachés à synthétiser deux hexasaccharides. Comme précédemment, ces deux cibles, *XVII* en série *S. flexneri* 3a et son correspondant *XVIII* en série *S. flexneri* X, ont été synthétisées sous forme de glycoside de propyle.

| | Accepteur | Donneur | Cible |
|---|---|---|---|
| Procédé 13 | 58 | 80 ou 80' | *VII* |
| Procédé 14 | 58 | 109 | *VIII* |
| Procédé 14bis | 108 | 2 | *VIII* |
| Procédé 14Ter | 108 | 1 | *VIII* |
| Procédé 15 | 116 | 39 | *IX* |
| Procédé 16 | 110 | 47 | *XVII* |
| Procédé 17 | 110 | 47 | *XVIII* |

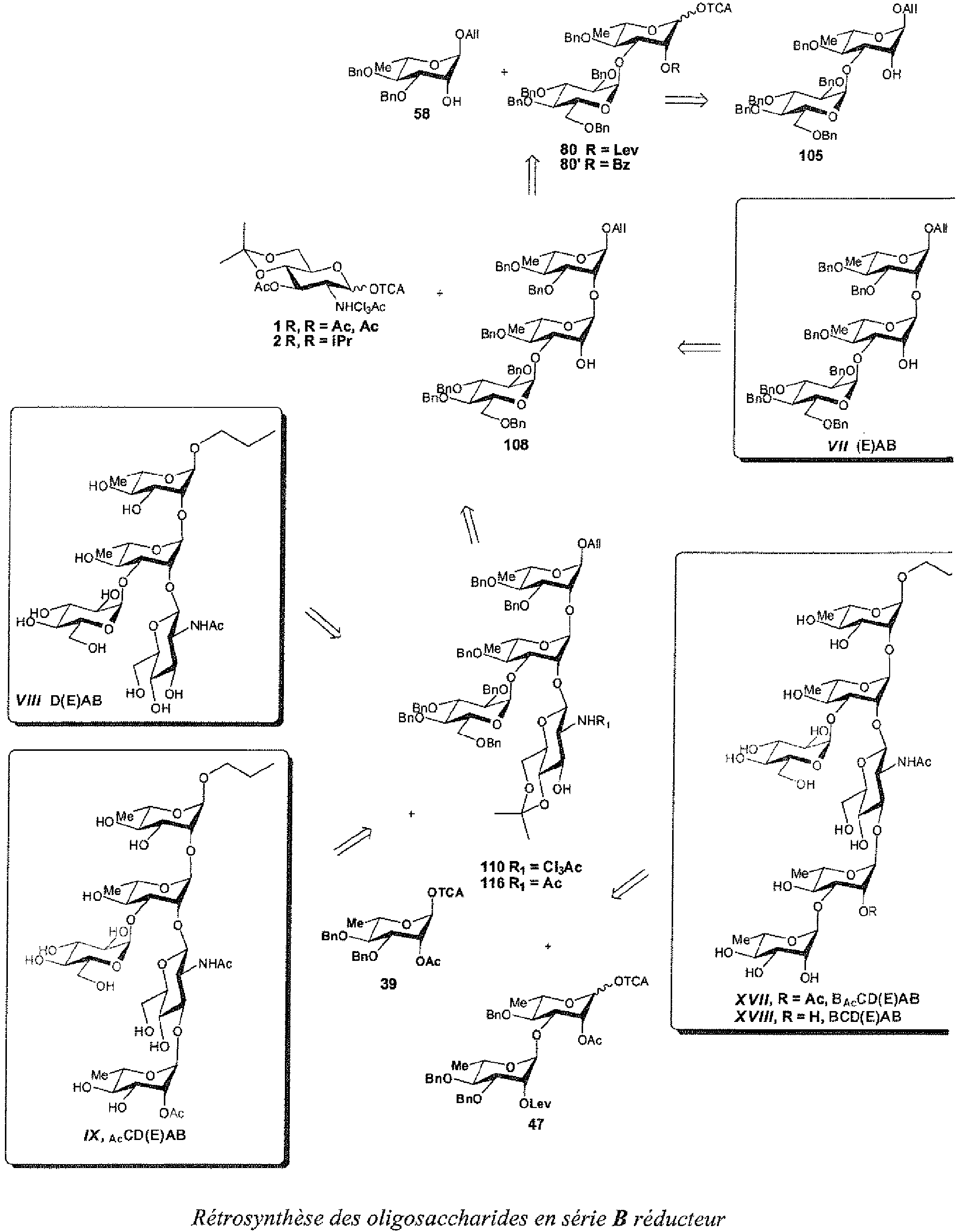

### Procédé 13 :

La description se rapporte au procédé de préparation du composé VII (trisaccharide (E)AB) produit intermédiaire dans la synthèse d'un saccharide tel que défini dans la liste L1 comprenant les étapes suivantes :
- condensation du monosaccharide accepteur **58** et du disaccharide donneur **80'** conduisant au trisaccharide **107'** de préférence la réaction de condensation est réalisée dans un solvant tel que le dichlorométhane, le 1,2-dichloroéthane, l'éther, le toluène, l'acétonitrile ou tout autre solvant équivalent, en présence d'un acide de Lewis tel que le TMSOTf ou l'acide triflique, préférentiellement, dans le toluène en présence d'une quantité catalytique d'acide triflique (schéma 55) ;
- débenzoylation du trisaccharide **107'** conduisant au trisaccharide **108** de préférence par action de MeONa dans un mélange dichlorométhane/méthanol ;
- hydrogénolyse du trisaccharide **108** conduisant au trisaccharide (E)AB de préférence sous atmosphère atmosphérique d'hydrogène dans l'éthanol, par exemple l'éthanol, en présence de palladium (schéma 60).

La description se rapporte également au procédé de préparation du composé VII (trisaccharide (E)AB) produit intermédiaire dans la synthèse d'un saccharide tel que défini dans la liste L1 comprenant les étapes suivantes :
- condensation du monosaccharide accepteur **58** et du disaccharide donneur **80** conduisant au trisaccharide **107** de préférence la réaction de condensation est réalisée dans un solvant tel que le dichlorométhane, le 1,2-dichloroéthane, l'éther, le toluène, l'acétonitrile ou tout autre solvant équivalent, en présence d'un acide de Lewis tel que le TMSOTf ou l'acide triflique, préférentiellement, dans le toluène en présence de TMSOTf à 0,3 éq. (schéma 61) ;
- délévulinoylation du trisaccharide **107'** conduisant au trisaccharide **108** de préférence dans le NaOMe en présence de méthanol ;
- hydrogénolyse du trisaccharide **108** conduisant au trisaccharide (E)AB de préférence sous atmosphère atmosphérique d'hydrogène dans l'alcool, par exemple éthanol, en présence de palladium (schéma 60).

### 1. Trisaccharide (E)AB

### a. Voie 2_{A}-O-benzoyl

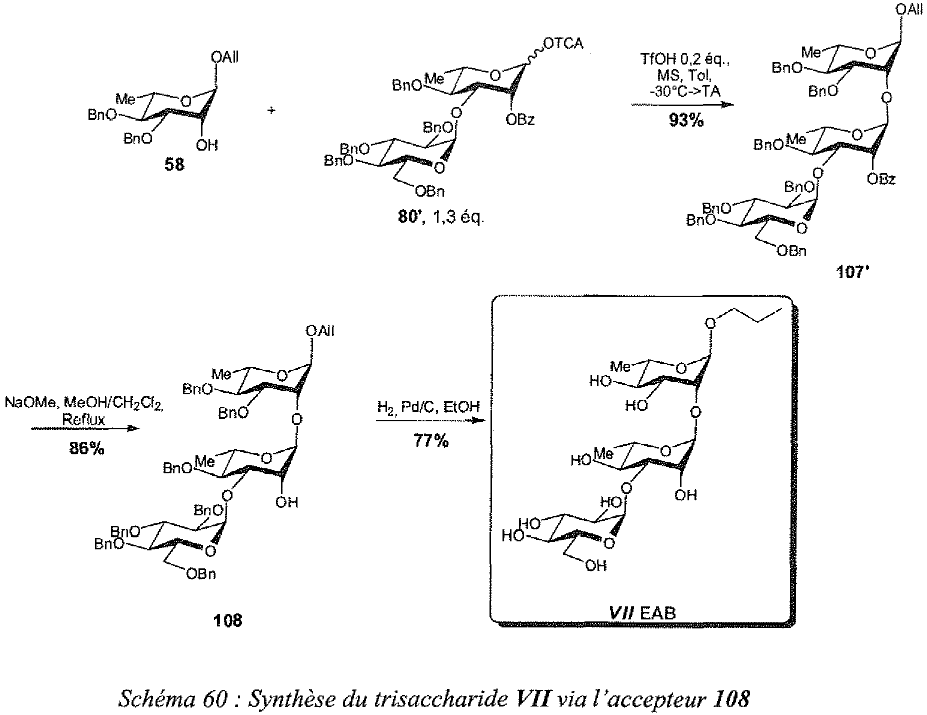

Le disaccharide donneur **80'** porteur d'un groupement benzoyle en position 2_{A} et préparé selon le protocole déjà décrit⁴⁵ est condensé sur l'accepteur **58** dans le toluène en présence d'une quantité catalytique d'acide triflique pour donner le trisaccharide complètement protégé **107'** avec un rendement de 93% (Schéma 60). Ce dernier est débenzoylé par action de MeONa dans un mélange dichlorométhane/méthanol 2/3 porté à reflux pour donner l'accepteur **108** (86%). Celui-ci est débenzylé par hydrogénolyse en présence de palladium sur charbon. Le trisaccharide cible ***VII*** est isolé après purification par HPLC (77%).

### b. Voie 2_{A}-O-levuniloyl

Après avoir développé une nouvelle voie d'accès au disaccharide **21,** précurseur du donneur **80** et optimisé la synthèse de l'accepteur rhamnose **58** dans la partie précédente, l'accès à la cible ***VII*** a été envisagé à partir de ces deux entités. Encore une fois, l'influence du solvant^{69, 76-79} dans l'étape de glycosylation a été évaluée.

Dans le toluène, le trisaccharide 107 est obtenu avec un rendement de 91%, supérieur au rendement obtenu si le dichlorométhane est utilisé (82%). A grande échelle (1,5 g d'accepteur **61**), ce rendement atteint même 92%. La délévulinoylation est cette fois-ci réalisée dans le NaOMe méthanolique, puisqu'il n'y a pas d'acétyle présent sur la molécule, pour accéder à l'accepteur **108** (94%) (Schéma 61).

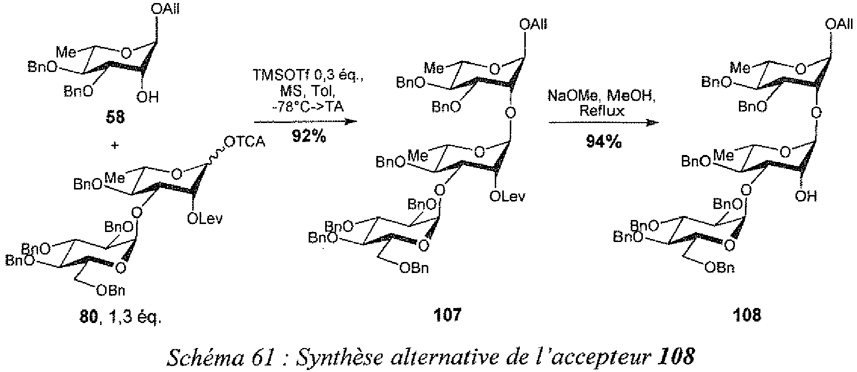

### Procédé 14 :

La description se rapporte au procédé de préparation du composé VIII (tétrasaccharide D(E)AB) tel que défini dans la liste L1 comprenant les étapes suivantes :
- condensation du trisaccharide accepteur **108** avec le monosaccharide donneur **2** conduisant au tétrasaccharide **115** de préférence la réaction de condensation est réalisée dans un solvant tel que le dichlorométhane, le 1,2-dichloroéthane, l'éther, le toluène, l'acétonitrile ou tout autre solvant équivalent, en présence d'un acide de Lewis tel que le TMSOTf ou l'acide triflique, préférentiellement, dans le toluène en présence de TMSOTf à une température comprise entre -20°C et -40°C (schéma 64) ;
- désacétylation du tétrasaccharide **115** conduisant au tétrasaccharide **110** de préférence en présence de carbonate de potassium (schéma 64) ;
- clivage du groupement isopropylidène du tétrasaccharide **110** et déprotection du tétrasaccharide obtenue conduisant au tétrasaccharide D(E)AB de préférence sous atmosphère d'hydrogène d'éthanol en présence de palladium (schéma 29).

La description se rapporte aussi au procédé de préparation du composé VIII (tétrasaccharide D(E)AB) tel que défini dans la liste L1 comprenant les étapes suivantes :
- condensation du trisaccharide accepteur **108** avec le monosaccharide donneur **1** conduisant au tétrasaccharide **115'** de préférence la réaction de condensation est réalisée dans un solvant tel que le dichlorométhane, le 1,2-dichloroéthane, l'éther, le toluène, l'acétonitrile ou tout autre solvant équivalent, en présence d'un acide de Lewis tel que le TMSOTf ou l'acide triflique, préférentiellement, dans le toluène en présence d'une quantité catalytique d'acide triflique (schéma 65) ;
- désacétylation du tétrasaccharide **115'** conduisant au tétrasaccharide **115"** de préférence par une solution de MeONa dans le méthanol ;
- déprotection du tétrasaccharide **115"** conduisant au tétrasaccharide D(E)AB de préférence sous atmosphère atmosphérique d'hydrogène dans l'éthanol en présence de palladium (schéma 65).

### 2. Tétrasaccharide D(E)AB

D'après l'étude rétrosynthétique, la cible ***VIII*** est accessible en couplant l'accepteur **108** avec les donneurs glucosamines **1, 2,** ou 3 décrits dans la première partie (Schéma 62).

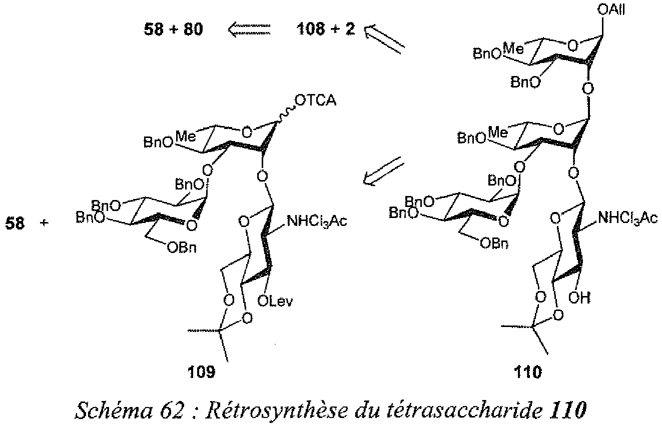

Mais, l'objectif à long terme étant la synthèse de cibles oligosaccharidiques de grande taille, une autre stratégie impliquant un nombre réduit d'étapes a aussi été envisagée. L'accepteur **58** peut-être couplé directement avec un donneur trisaccharide **109** au lieu du disaccharide **80,** issu de la glycosylation avec le donneur **2** (Schéma 62). Ces deux stratégies ont donc été comparées. Il est à noter, que le nouveau donneur **109** ne possède pas de groupe participant en position 2_{A} mais, les données de la littérature suggèrent que dans le cas du rhamnose et du mannose, une bonne stéréosélectivité en faveur de l'anomère α peut être envisagée en l'absence d'assistance anchimérique.

### a. Couplage D(E)A + B

La synthèse du donneur **109** a été entreprise en 3 étapes, lévulinoylation⁸⁹ de l'accepteur **38** (90%), déallylation^{39, 40} du glycoside d'allyle **111** (80%) puis activation^{22, 23} de l'hémiacétal **112** (85%). Sur la base des études précédentes, le couplage entre ce donneur **109** et l'accepteur **54** a été conduit dans le toluène. Mais la dégradation du donneur **109** en hémiacétal **112** ainsi que la présence de l'accepteur de départ **58** ont été observées. En revanche, lorsque la condensation est réalisée dans le dichlorométhane, deux nouveaux composés ont été isolés et caractérisés.

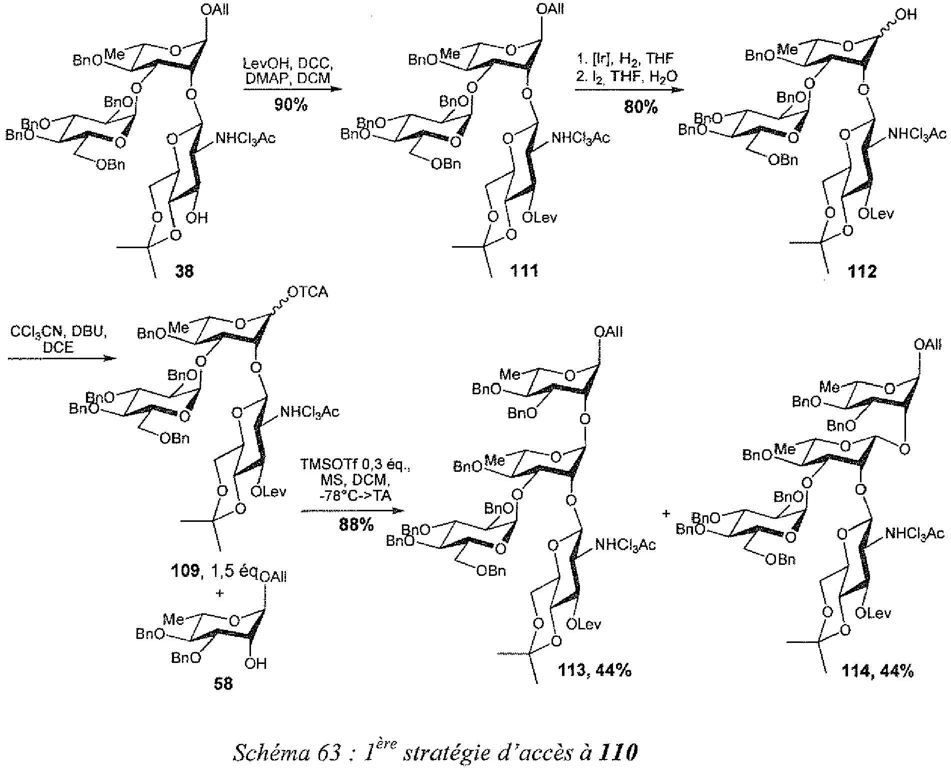

Après une analyse complète en RMN, ces deux derniers correspondent aux deux anomères tétrasaccharides α_{A} **113** et β_{A} **114** obtenus avec un excellent rendement de couplage global de 88% mais dans un rapport 1/1 soit une très mauvaise stéréosélectivité de réaction (Schéma 63). L'influence de l'effet anomère attendu est probablement contrée par un encombrement stérique important, cette voie a donc été abandonnée. En conclusion, ce résultat valide le bien-fondé de notre stratégie initiale malgré le nombre d'étapes plus élevé.

### b. Couplage D + (E)AB

### a. Voie impliquant le donneur 2.

La deuxième voie d'accès au tétrasaccharide est la glycosylation entre l'accepteur **108** et le donneur **2.** Malgré l'utilisation des conditions mises au point dans la partie 3 pour la synthèse du pentasaccharide **90,** une optimisation de ce couplage a de nouveau été nécessaire (Tableau 10). En effet, les deux premiers tests réalisés à -40°C, dans le dichlorométhane et le toluène n'ont pas donné des résultats satisfaisants (entrée 1 et 2). Effectivement, en CCM, l'accepteur **108** est encore présent dans le milieu réactionnel alors que le donneur **2** est consommé. Dans la partie précédente, le donneur glucosamine **2** n'était pas assez réactif à -78°C et se dégradait trop vite à -20°C. Une glycosylation comparative à -20°C et à -40°C a donc été réalisée, en augmentant à la fois la quantité de donneur pour avoir une réaction totale mais aussi la quantité d'acide pour augmenter la vitesse de réaction. Au vu des entrée 3 et 4, les résultats précédents sont, à nouveau, confirmés puisque la température idéale de couplage est -40°C même si la réaction n'est toujours pas totale. En revanche, au vu des entrées 3 et 5, l'augmentation de la quantité d'acide n'a pas eu l'influence souhaitée.

**Tableau 10 : Étude du couplage entre 2 et 108**

| **Entrée** | **TMSOTf** | **Température** | **Donneur 2** | **Solvants** | **Rendement (rendement corrigé)** |
|---|---|---|---|---|---|
| **1** | 0,3 éq. | -40°C | 1,4 éq. | Dichlorométhane | 35% |
| **2** | | | | Toluène | 56% |
| **3** | 0,4 éq. | -40°C | 1,5 éq. | | 77% (84%) |
| **4** | | -20°C | | | 70% (78%) |
| **5** | 0,3 éq. | -40°C | 1,5 éq. | | 75% (82%) |
| **6** | | | 1.7 éq. | | 95% |

La présence d'accepteur **108** de départ lors de ces différents essais, a conduit à augmenter la quantité de donneur (entrée 6 du Tableau 10).

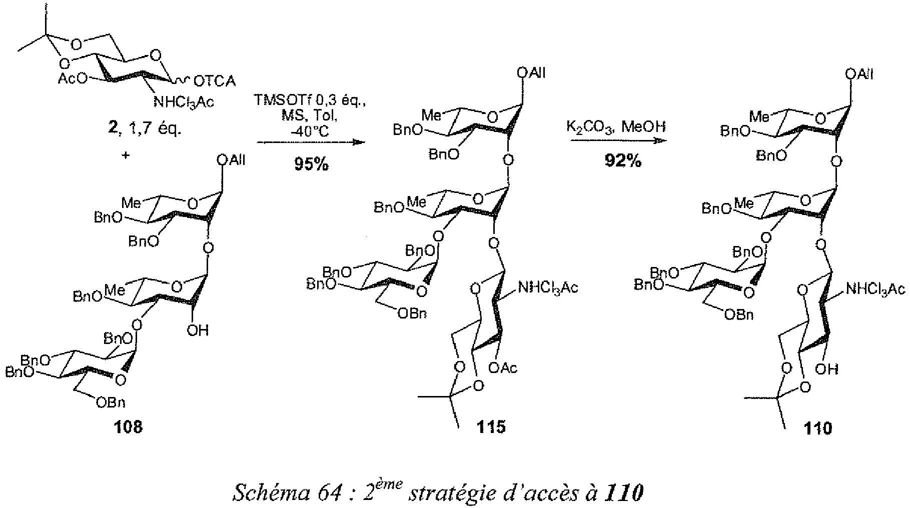

A grande échelle (1,2 g d'accepteur), la réaction est totale et permet d'isoler le tétrasaccharide attendu **115** avec un rendement de 95% (Schéma 64). Ce dernier est désacétylé en présence de carbonate de potassium, ⁸⁵ pour accéder au tétrasaccharide accepteur **110** avec un excellent rendement de 92%.

### b. Voie impliquant le donneur 1.

Si l'élongation de la chaine en position 3_{D} n'est pas requise, il peut être judicieux d'utiliser le donneur de résidu glucosaminyl **1,** plus aisément accessible. Cette voie a également été envisagée (Schéma 65).

Le produit de condensation triacétylé **115'** est isolé (87%) à l'issu du couplage entre l'accepteur **108** avec le donneur **1** est réalisé dans le toluène en présence d'une quantité catalytique d'acide triflique. Traité à température ambiante par une solution de MeONa dans du méthanol, le tétrasaccharide **115'** fournit le triol **115"** (79%). Cet intermédiaire est débenzylé par hydrogénolyse en milieu légèrement acide, puis converti en acétamide par hydrodéchloration en milieu légèrement basique. Le tétrasaccharide libre ***VIII*** est isolé après purification par HPLC (68%).

Une alternative possible consiste à convertir le tétrasaccharide complètement protégé **115'** en acétamido triol par action de MeONa dans un mélange dichlorométhane/méthanol. Cet intermédiaire est débenzylé par hydrogénolyse pour conduire au tétrasaccharide cible ***VIII*** avec un rendement global de 39% à partir de l'accepteur **108.** On remarquera cependant que ce rendement est inférieur au rendement de 46% obtenu sur la séquence couplage/déprotection complète (**108** → ***VIII***) impliquant le trichloroacétamide **115'.**

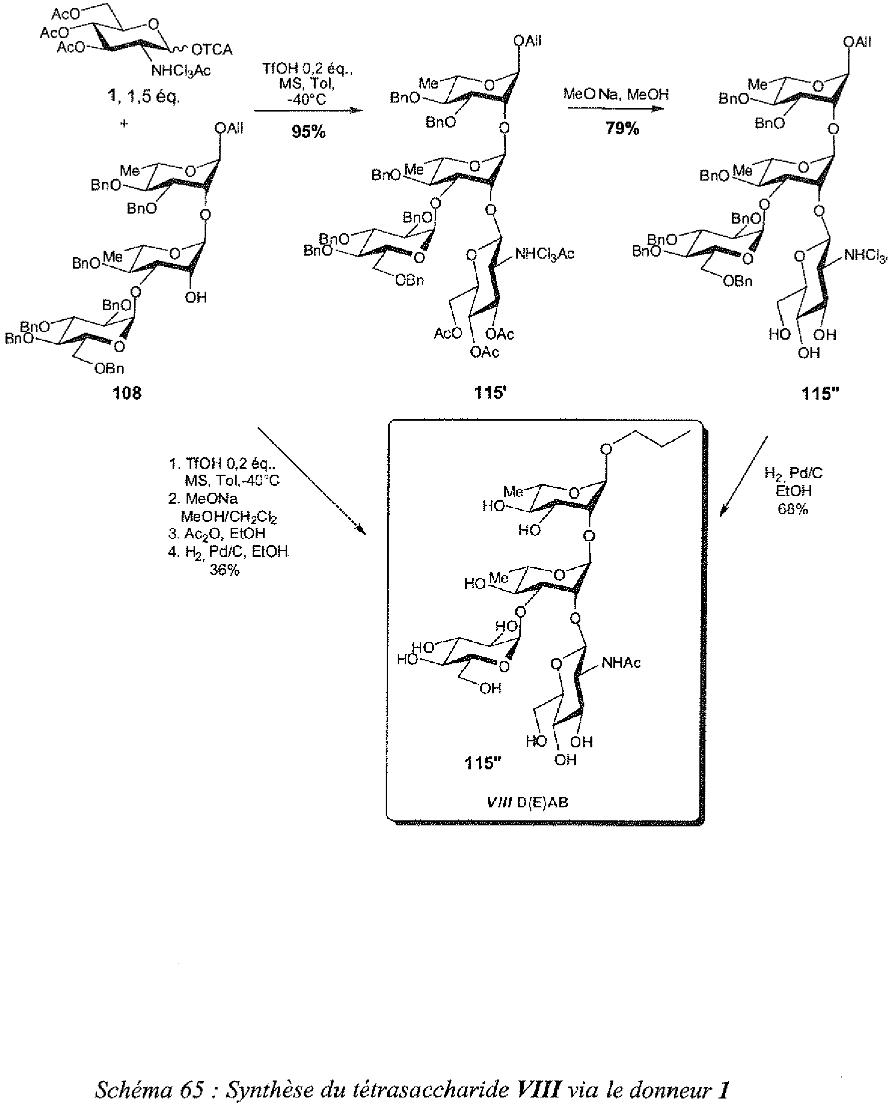

### Procédé 15 :

La description se rapporte encore au procédé de préparation du composé IX (pentasaccharide _{Ac}CD(E)AB) tel que défini dans la liste L1 comprenant les étapes suivantes :
- condensation du tétrasaccharide accepteur **116** et du monosaccharide donneur **39** conduisant au pentasaccharide **116'** de préférence la réaction de condensation est réalisée dans un solvant tel que le dichlorométhane, le 1,2-dichloroéthane, l'éther, le toluène, l'acétonitrile ou tout autre solvant équivalent, en présence d'un acide de Lewis tel que le TMSOTf ou l'acide triflique, préférentiellement, dans le toluène en présence d'acide triflique (schéma 71) ;
- clivage du groupement isopropylidène du pentasaccharide **116'** conduisant au pentasaccharide **116"** ;
- déprotection du pentasaccharide **116"** conduisant au pentasaccharide _{Ac}CD(E)AB de préférence sous atmosphère d'hydrogène dans l'éthanol en présence de palladium sur charbon (schéma 71).

### 3. Nouvelle stratégie : Conversion du trichloroacétamide en acétamide en cours de synthèse

Si on prend en compte la présence de plusieurs fonctions trichloroacétamides sur la même molécule lors de la synthèse de cibles de plus grande taille et les possibles difficultés rencontrées lors de l'hydrogénolyse finale, alors la conversion de la fonction trichloroacétamide en acétamide peut aussi être envisagée avant introduction de la fonction acétyle en position 2_{C} plutôt qu'à la dernière étape. Ainsi la synthèse de l'analogue acétamide de **116** a été envisagée. Dans la littérature, beaucoup de méthodes de conversion existent (partie rétrosynthèse), mais l'étain voulant être évité, une nouvelle méthode a été développée.

### ✔ Mise au point des conditions

Conformément aux résultats qui viennent d'être décrits concernant la transformation de **115'** en ***VIII**,* lors de la désacétylation des trois fonctions acétyles du trisaccharide **22,** un résultat surprenant a été observé puisque, si la réaction est conduite dans du méthanol alors seul le triol **37** est obtenu alors que si du dichlorométhane est ajouté au milieu réactionnel, un composé extrêmement polaire apparait en CCM. Comme ce dernier se révèle à la ninhydrine, l'hypothèse la plus plausible est la présence de la forme amine libre (Schéma 66). Pour le vérifier, une acétylation en milieu alcoolique, sélective de la fonction amine postulée, a été réalisée. La structure du produit de réaction **117** a été déterminée par une analyse complète en RMN et spectrométrie de masse ce qui a permis de confirmer l'hypothèse.

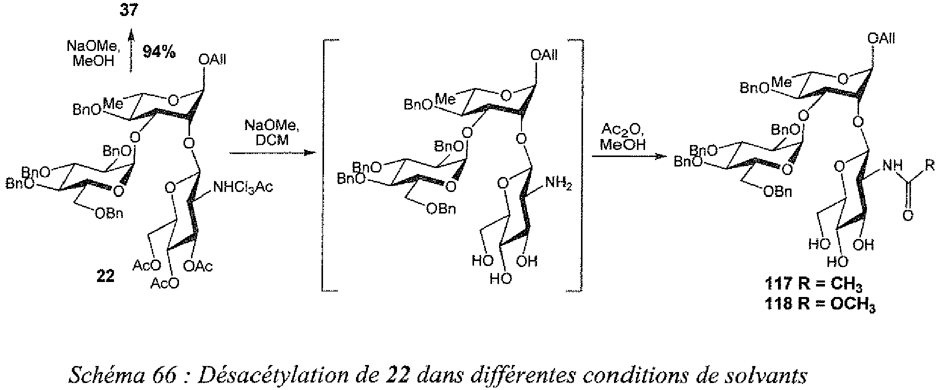

Cependant, la conversion de la fonction trichloroacétamide en acétamide est rendue difficile par la présence d'un composé secondaire **118,** un carbamate de méthyle, dont la structure est très proche de celle de l'acétamide voulue **117.** La formation de ce carbamate **118** peut s'expliquer par un probable passage par un isocyanate instable qui réagit ensuite avec un alcool, ici le méthanol utilisé comme solvant de la réaction, pour donner le carbamate correspondant¹¹⁶ (Schéma 67).

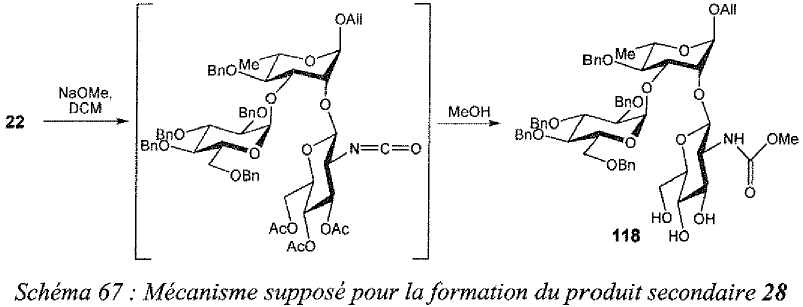

Pour réduire la formation de ce carbamate non désiré, cette réaction a donc été optimisée. En comparant les entrées 1, 2 et 3 du Tableau 11 ainsi qu'au vu de la figure 22, si le volume de NaOMe 0,5M méthanolique est fixe, alors la conversion est optimum lorsque le volume de DCM lui est 8 fois supérieur. Si au contraire le volume de DCM est fixe, alors plus la quantité de NaOMe est importante et plus la réaction est rapide (entrée 4, 5 et 6 du Tableau 11 et de la figure 22). Les conditions optimums sont donc celles de l'entrée 6 car, dans ce cas, la formation du carbamate est minoritaire. Effectivement, à plus grande échelle (5g de **22**), l'acétamide **117** est obtenu avec un rendement de 90%, complété de seulement 5% de carbamate **118.**

**Tableau 11 : Etude de la réduction de la fonction trichloroacétamide**

| **Entrée** | **NaOMe (0.5M)** | **V_{NaOMe}/V_{DCM}** | **Temps de disparition de 22** |
|---|---|---|---|
| **1** | 5 éq. | 4 | 3h45 |
| **2** | | 8 | 2h30 |
| **3** | | 12 | 5h15 |
| **4** | 2 éq. | 8 | 4h |
| **5** | 4 éq. | | 3h15 |
| **6** | 6 éq. | | 2h |

Pour préparer l'équivalent de l'accepteur **38** en série acétamide, le triol **117** est protégé régiosélectivement en position 4_{D} et 6_{D} par un groupement isopropylidène pour conduire à l'accepteur **119** avec un rendement de 85%^{30, 31} (Schéma 68). Il est à noter, que l'isopropylidène de cet accepteur **119** est stable, contrairement à celui de l'accepteur **38** (Partie 2).

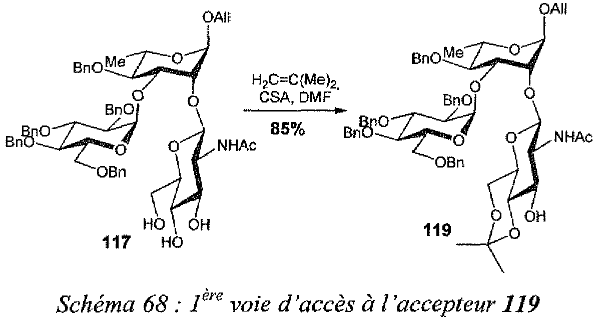

### ✔ Étude de la reproductibilité de la conversion du trichloroacétamide en acétamide sur d'autres synthons

Cette nouvelle méthode de conversion étant particulièrement efficace, elle a été appliquée à d'autres intermédiaires utilisés dans nos différentes synthèses comme par exemple le trisaccharide **23** pour accéder au même accepteur **119.** Ainsi, la désacétylation de la fonction acétyle combinée à la détrichloracétylation de la fonction amine du glycoside d'allyle **23** a permis d'isoler l'accepteur **119** avec un rendement de 90% (Schéma 69). Pour comparer les deux voies d'accès à l'accepteur **119,** les différentes synthèses ont été réalisées à grande échelle (de l'ordre de 5 g) en essayant de limiter le nombre de chromatographies. Les résultats présentés sur le Schéma 69 montrent que les rendements sont comparables pour ces deux voies mais aussi comparables à la synthèse de l'accepteur **38** en série trichloroacétamide (Partie 2).

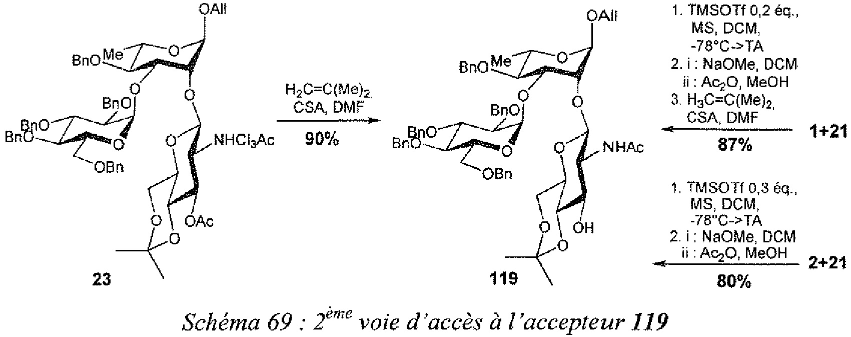

Ensuite deux autres synthons ont été convertis en acétamide, selon le même protocole, le pentasaccharide **90 (D(E)ABC** Partie 3) (66%) et le tétrasaccharide **115** avec un excellent rendement de 83% en **116** (Schéma 70). Cette nouvelle méthode de conversion a donc prouvé tout son potentiel, ce qui permet d'envisager l'allongement vers des oligosaccharides de grande taille.

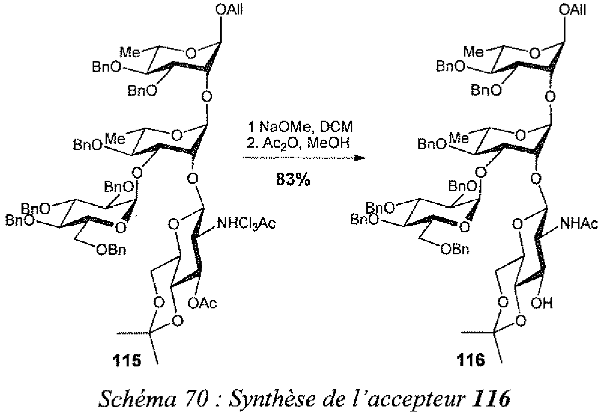

### ✔ Application à la préparation du pentasaccharide IX

Le tétrasaccharide **116** est condensé sur le donneur **39** en présence d'acide triflique (Schéma 71). La réaction menée dans le toluène conduit au pentasaccharide **106'** complètement protégé (71%). La fonction isopropylidène est clivée par hydrolyse acide pour conduire quantitativement au diol **106".** Ce dernier est débenzylé par hydrogénolyse en présence de Pd/C. Conformément au phénomène déjà observé pour le tétrasaccharide ***III**,* le pentasaccharide ***IX*** est isolé après purification sous forme d'un mélange de régioisomères correspondant au produit voulu portant une fonction acétyle en position 2_{C} et au produit de migration de la fonction acétyle sur l'hydroxyle *cis*-vicinal.

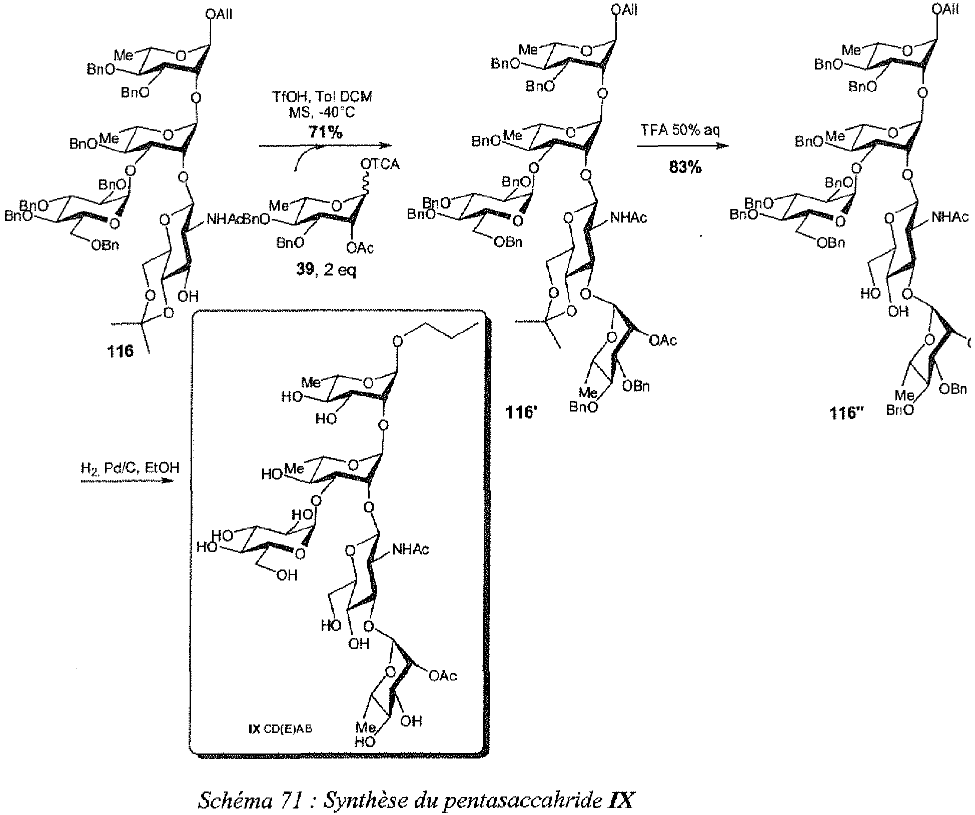

### Procédé 16 :

La description se rapporte au procédé de préparation du composé XVII (hexasaccharide B_{Ac}CD(E)AB) tel que défini dans la liste L1 comprenant les étapes suivantes :
- condensation du tétrasaccharide accepteur **110** et du disaccharide donneur **47** conduisant aux hexasaccharides **122** et **123** de préférence la réaction de condensation est réalisée dans un solvant tel que le dichlorométhane, le 1,2-dichloroéthane, l'éther, le toluène, l'acétonitrile ou tout autre solvant équivalent, en présence d'un acide de Lewis tel que le TMSOTf ou l'acide triflique, préférentiellement, dans le toluène en présence de TMSOTf (schéma 74) ;
- clivage du groupement isopropylidène de l'hexasaccharide **122** conduisant à l'hexasaccharide **123** de préférence dans une solution aqueuse de TFA 50% dans le dichlorométhane (schéma 75) ;
- délévulinoylation de l'hexasaccharide **123** conduisant à l'hexasaccharide **124** de préférence en milieu tamponné en présence de d'hydrazine monohydraté (schéma 75) ;
- déprotection de l'hexasaccharide **124** à l'hexasaccaharide B_{Ac}CD(E)AB de préférence sous 50 bars de pression conduisant (schéma 76).

### 4. Hexasaccharide B_{Ac}CD(E)AB (XVII)

Comme indiqué ci-dessus, dans le cas du dérive ***IX,*** la migration la fonction acétyle de la position 2_{C} sur l'alcool vicinal en 3_{C} est observée. Pour éviter cette migration et avoir des résultats d'antigénicité complets, la synthèse d'un fragment plus long, à savoir l'hexasaccharide **B_{AC}CD(E)AB *XVII*** a été envisagée.

### a. Étude de couplage en série acétamide

Avant de réaliser la synthèse de cet hexasaccharide et pour faire suite aux résultats obtenus dans le cas de la synthèse de ***IX,*** l'étude de la faisabilité de glycosylation en série acétamide a été réalisée dans le cadre de la préparation d'un pentasaccharide issu du couplage entre l'accepteur **119** et le donneur **47** (Schéma 72). Les données de la littérature^{114, 38} et du laboratoire,^{115, 62} complétées des résultats de la partie 3, suggèrent que les accepteurs acétamides sont moins réactifs que leurs analogues trichloroacétamides. Différentes conditions de glycosylation ont été évaluées (Tableau 12).

Les premiers tests à -20°C, dans le toluène et dichlorométhane (entrées 1 et 2), confirment la faible réactivité de l'accepteur **119.** En effet, il est toujours présent dans le milieu réactionnel alors que le donneur **47** se dégrade rapidement. A -5°C, les rendements en **120** sont toujours assez faibles, et ce quel que soit le solvant utilisé (entrées 3, 4 et 5). Pour le couplage entre **19** et **86** (Partie 3), les quantités de tamis moléculaire et d'acide avaient été augmentées pour réaliser la glycosylation à plus haute température selon les conditions mises au point antérieurement au laboratoire.

**Tableau 12 : Optimisation du couplage entre l'accepteur 119 et le donneur 47**

| **Entrée** | **Solvant** | **Température** | **Activateur** | **Rendement** |
|---|---|---|---|---|
| **1** | DCM | -20°C | TMSOTf: 0,3 éq. | 20% |
| **2** | Toluène | -20°C | | 25% |
| **3** | THF | -5°C | | Non observé |
| **4** | Acétonitrile | -5°C | | 35% |
| **5** | Ether | -5°C | | 50% |
| **6** | Toluène | 0°C→70°C | TfOH : 0,9 éq. | 71% |
| **7** | Toluène | 0°C | | 71% |

Ces conditions ont donc été appliquées à ce nouveau couplage (entrées 6 et 7). Les rendements en **120** sont identiques ce qui semble indiquer que la température optimum est de 0°C (Schéma 72).

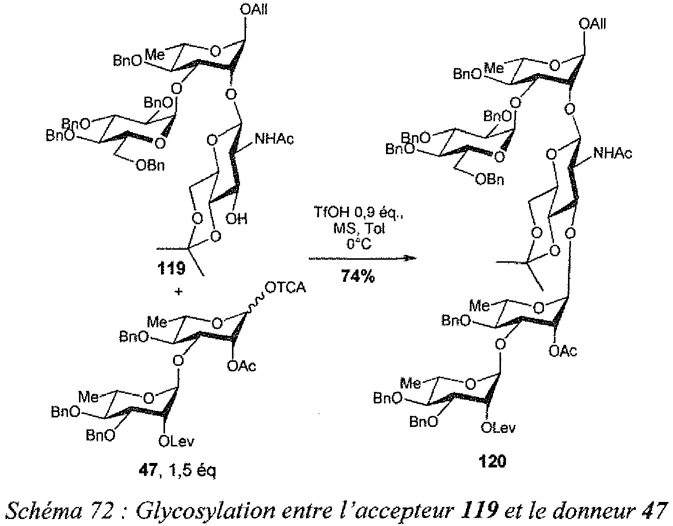

Ce résultat a été confirmé sur l'accepteur acétamide **116**. Effectivement, à 0°C et en présence de TMSOTf (0,9 éq.), l'hexasaccharide **121** est isolé avec un rendement de 65% (Schéma 73). Il est à noter que l'emploi de TMSOTf ou de TfOH (entrée 7 ou Schéma 73), n'a, dans notre cas, aucune influence puisque les rendements sont équivalents. Malgré ces résultats encourageants sur le pentasaccharide **120** et l'hexasaccharide **121,** l'analyse RMN de tous ces produits de glycosylation montre la présence systématique d'un contaminant qui co-élue avec ces derniers. La séparation de ce contaminant a été entreprise lors des étapes de déprotection, soit de l'acétal soit de la fonction lévulinoyle. Cependant, la réaction de délévulinoylation de **120** n'a jamais été totale et la désacétalation de **120** a permis d'obtenir le diol attendu, mais toujours contaminé. Le contaminant n'a pu être ni isolé ni identifié. Ainsi, avant de continuer la synthèse, une comparaison avec la série trichloroacétamide a été réalisée.

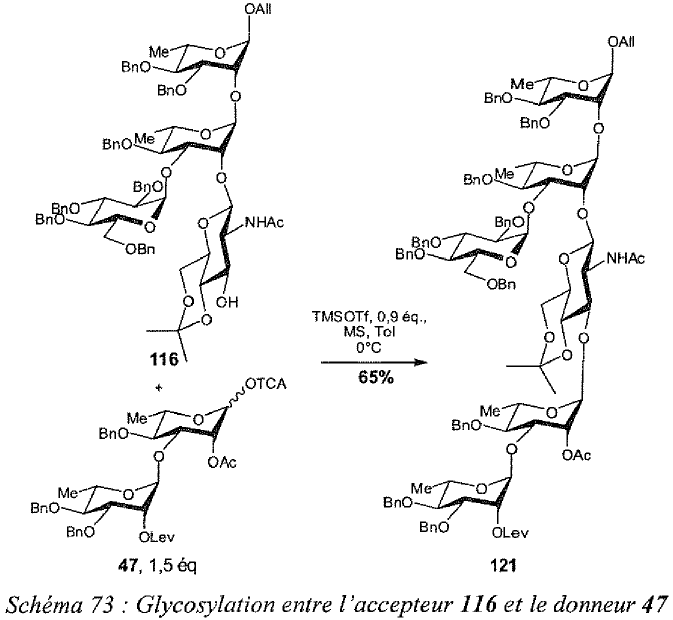

### b. Étude de couplage en série trichloroacétamide

La température optimale (-40°C), déterminée lors de l'étude du couplage entre le trisaccharide **38** et le donneur **47** (Partie 2), a été testée lors de la glycosylation entre le tétrasaccharide **110** et le même donneur **47** (Tableau 13). Dans le dichlorométhane (entrée 1), la dégradation de la fonction isopropylidène est plus importante que dans le toluène (entrée 2), même si le rendement global en produit est proche. Comme précédemment pour **64,** l'analyse RMN de **123,** ainsi que l'isopropylidénation (93%, Schéma 74) de ce dernier a permis de confirmer la perte de l'isopropylidène ainsi que la position de glycosylation sur l'unité glucosamine.

**Tableau 13 : Étude du couplage entre le donneur 47 et l'accepteur 110**

| **Entrée** | **Solvants** | **Temps de réaction** | **122** | **123** |
|---|---|---|---|---|
| **1** | DCM | 25 min. | 20% | 62% |
| **2** | Toluène | 25 min. | 30% | 55% |
| **3** | Toluène | 10 min. | 59% | 23% |
| **4** | Toluène | 40 min. | 59% | 21% |

L'entrée 3 montre, encore une fois, que la dégradation de l'isopropylidène peut être contrôlée en maintenant un temps de réaction très court. Mais l'intermédiaire **123,** étant impliqué dans la synthèse de la cible ***XVII,*** la glycosylation a été reproduite à grande échelle (1,7 g d'accepteur **110)** sans contrôler ce facteur, permettant d'isoler **122** et **123** dans un rapport 75/25 avec un rendement global de 80% (entrée 4, Schéma 74).

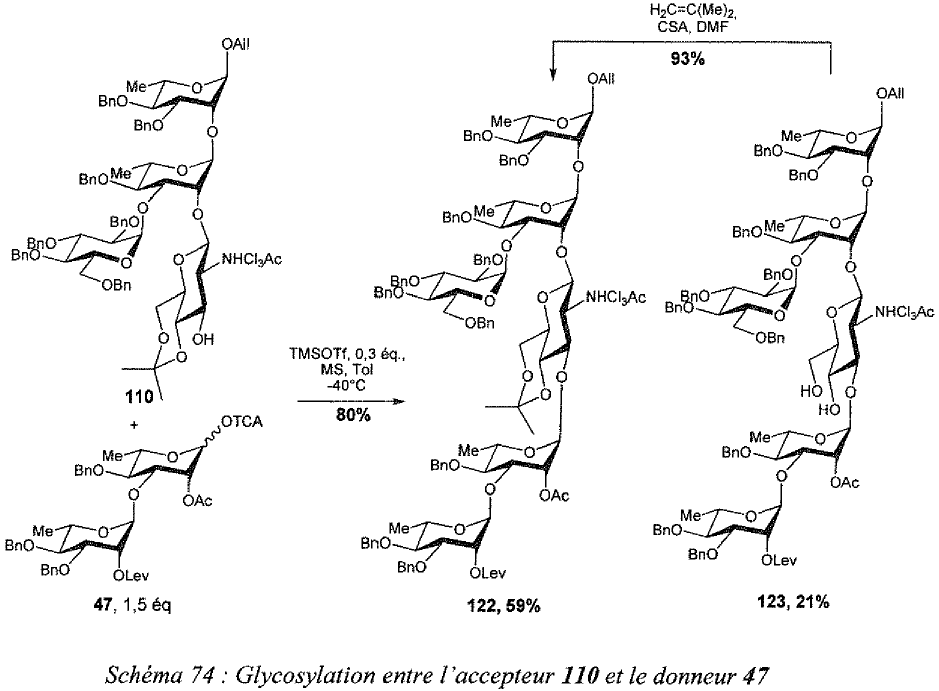

Traité par une solution aqueuse de TFA 50% dans le dichlorométhane,^{80, 81} le pentasaccharide **122** conduit au diol **123** avec un rendement de 89%.

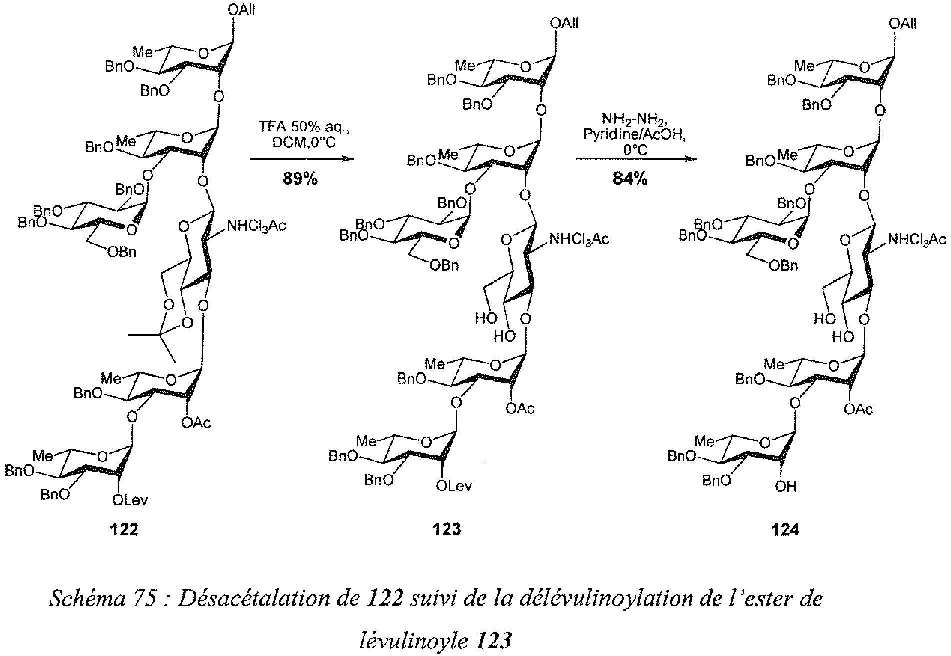

Puis la délévulinoylation est effectuée en milieu tamponné en présence d'hydrazine monohydraté,^{89, 97-101} pour conduire au hexasaccharide **124** (84%). Par analogie avec la synthèse des cibles des Parties 2 et 3, l'hydrogénolyse, sous 50 bars de pression du triol **124** conduit au glycoside de propyle *XVII* avec un rendement de 64% (Schéma 76).

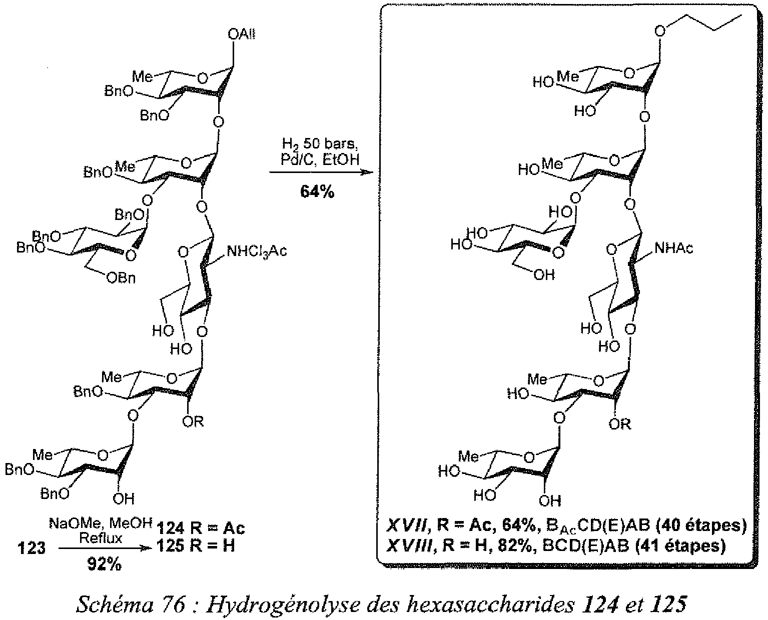

### Procédé 17 :

La description se rapporte au procédé de préparation du composé XVIII (hexasaccharide B_{c}CD(E)AB) tel que défini dans la Isite L1 comprenant les étapes suivantes :
- condensation du tétrasaccharide accepteur **110** et du disaccharide donneur 47 conduisant aux hexasaccharides **122** et **123** de préférence la réaction de condensation est réalisée dans un solvant tel que le dichlorométhane, le 1,2-dichloroéthane, l'éther, le toluène, l'acétonitrile ou tout autre solvant équivalent, en présence d'un acide de Lewis tel que le TMSOTf ou l'acide triflique, préférentiellement dans le toluène en présence de TMSOTf (schéma 74) ;
- clivage du groupement isopropylidène de l'hexasaccharide **122** conduisant à l'hexasaccharide **123** de préférence dans une solution aqueuse de TFA 50% dans le dichlorométhane (schéma 75) ;
- transestérification de l'hexasaccharide **123** conduisant à l'hexasaccharide **125** de préférence en présence de NaOMe à reflux du méthanol (schéma 76) ;
- déprotection de l'hexasaccharide **125** conduisant à l'hexasaccharide B_{Ac}CD(E)AB de préférence sous pression, par exemple sous 50 bars de pression (schéma 76).
La désacétylation du triol **124** en présence de NaOMe à reflux du méthanol conduit au tétraol **125** avec un rendement de 92%. Pour finir, l'hydrogénation, effectuée dans les mêmes conditions que pour la cible ***XVII,*** permet d'isoler le glycoside de propyle ***XVIII*** avec un rendement de 82% (Schéma 76).

### 5. Conclusion

Outre celle des tri- **(VII),** tétra- **(VIII),** et penta- **(IX),** la synthèse des deux hexasaccharides ***XVII*** et ***XVIII.*** La différence de réactivité entre les accepteurs acétamides **119** et **116** avec les accepteurs trichloroacétamides **38** et **110,** respectivement, a aussi été étudiée, permettant de confirmer la plus faible réactivité d'accepteurs de type *N*-acétylglucosamine. Effectivement, dans tous nos essais avec ce type d'accepteurs, les rendements restent plus faibles et la présence d'un contaminant ne permet pas de continuer la synthèse des cibles. Même si la perte de l'isopropylidène est observée en série trichloroacétamide, l'acétylation peut de nouveau être effectuée sur le brut de la glycosylation sans perte de produit.

De plus, les rendements d'hydrogénolyse des dérivés trichloroacétamides **124** ou **125** sont comparables aux rendements de déprotection des intermédiaires acétamides (Partie 2, 3 et 4). Continuer en série trichloroacétamide durant toute la synthèse et réduire cette fonction lors de l'étape de déprotection finale est donc, la meilleure stratégie pour la synthèse d'oligosaccharides d'ordre supérieur.

### Mise en oeuvre expérimentale des procédés 13 à 17

### Procédé 13 :

### ➢ Allyl (2,3,4,6-tétra-O-benzyl-α-D-glucopyranosyl)-(1→3)-(4-O-benzyl-2-O-lévulinoyl-α-L-rhamnopyranosyl)-(1→2)-3,4-di-O-benzyl-α-L-rhamnopyranoside 107 :

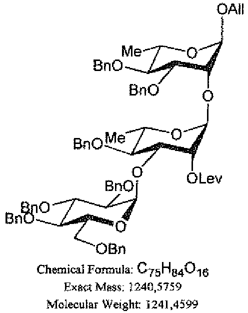

Du TMSOTf (210,0 µL, 1,2 mmol, 0,3 éq.) est additionné à une solution d'accepteur **58** (1,5 g, 3,9 mmol) et de donneur **80** (5,1 g, 5,1 mmol, 1,3 éq.) dans du Tol (100 mL), en présence de tamis moléculaire 4 Å (3,4 g), agitée sous argon à -78°C. Après 15 min à cette température, le bain éthanol-carboglace est retiré et l'agitation est poursuivie pendant 30 min, temps au bout duquel le suivi par CCM (Tol/EtOAc, 85/15) indique la disparition de **58** (Rf = 0,25) et l'apparition d'un nouveau composé moins polaire (Rf = 0,5). La réaction est stoppée par addition de triéthylamine (1 mL) puis le milieu réactionnel est filtré et concentré à l'évaporateur rotatif. L'huile jaune obtenue est purifiée par chromatographie sur gel de silice (Tol/EtOAc, 95/5→85/15) pour obtenir le glycoside d'allyle **107** sous forme d'une huile incolore (4,5 g, 92%).
Rf = 0,5 (Tol/EtOAc, 85/15).
RMN ¹H (CDCl₃), *δ* 7.45-7.12 (m, 35H, CH_{Ph}), 5.87 (m, 1H, CH=), 5.61 (m, 1H, H-2_{A}), 5.32 (d, 1H, *J*_{1,2} = 3.4 Hz, H-1_{E}), 5.31 (m, 1H, *J*ₜᵣₐₙₛ = 17.2 Hz, =CH₂), 5.23 (m, 1H, *J*_{cis} = 10.3 Hz, =CH₂), 5.06 (d, 1H, *J* = 10.5 Hz, H_{Bn}), 5.05 (d, 1H, *J*_{1,2} = 2.2 Hz, H-1_{A}), 5.00-4.89 (m, 5H, H_{Bn}), 4.82 (d, 1H, *J* = 12.2 Hz, H_{Bn}), 4.81 (bs, 1H, H-1_{B}), 4.74-4.64 (m, 5H, H_{Bn}), 4.53 (d, 1H, *J* = 11.0 Hz, H_{Bn}), 4.38 (d, 1H, *J* = 12.0 Hz, H_{Bn}), 4.26 (dd, 1H, *J*_{2,3} = 3.2 Hz, *J*_{3,4} = 9.6 Hz, H-3_{A}), 4.20-4.09 (m, 3H, H_{All}, H-3_{E}, H-5_{E}), 4.03 (dd, 1H, *J*_{2,3} = 2.9 Hz, H-2_{B}), 3.97 (m, 1H, H_{All}), 3.96-3.90 (m, 2H, H-5_{A}, H-3_{B}), 3.83 (pt, 1H, *J*_{4,5} = 9.5 Hz, H-4_{E}), 3.75 (dq, 1H, *J*_{4,5} = 9.4 Hz, H-5_{B}), 3.71-3.66 (m, 2H, H-6a_{E}, H-2_{E}), 3.63-3.57 (m, 2H, H-4_{A}, H-6b_{E}), 3.53 (pt, 1H, *J*_{4,5} = 9.4 Hz, H-4_{B}), 2.55 (m, 4H, 2CH_{2Lev}), 2.11 (s, 3H, CH_{3Lev}), 1.42 (d, 3H, *J*_{5,6} = 6.2 Hz, H-6_{A}), 1.37 (d, 3H, *J*_{5,6} = 6.2 Hz, H-6_{B}).
RMN ¹³C (CDCl₃), *δ* 206.2 (C_{Lev}), 171.8 (C_{Lev}), 138.7-137.7 (C_{Ph}), 133.9 (CH=), 129.1-127.6 (CH_{Ph}), 117.2 (=CH₂), 99.2 (C-1_{A}, ¹*J*_{CH} = 173.4 Hz), 97.9 (C-1_{B}, ¹*J*_{CH} = 170.8 Hz), 92.9 (C-1_{E}, ¹*J*_{CH} = 168.9 Hz), 82.1 (C-3_{E}), 80.5 (C-4_{B}), 79.9 (C-4_{A}), 79.6 (C-3_{B}), 79.5 (C-2_{E}), 77.8 (C-4_{E}), 76.2, 75.6, 75.5, (3C, C_{Bn}), 75.1 (C-2_{B}), 75.0, 73.4, 72.8 (3C, C_{Bn}), 72.3 (C-3_{A}), 72.2 (C_{Bn}), 70.3 (C-5_{E}), 68.4 (C-5_{A}), 68.3 (C-6_{E}), 68.1 (C-5_{B}), 68.0 (C-2_{A}), 67.7 (C_{All}), 38.0 (CH_{2Lev}), 29.7 (CH_{3Lev}), 28.2 (CH_{2Lev}), 18.1, 18.0 (C-6_{A}*, C-6_{B}*).

| | | |
|---|---|---|
| HRMS (ESI⁺) : | [M+Na]⁺ C₇₅H₈₄O₁₆Na | m/z théorique : 1263.5657 |
| | | m/z mesurée : 1263.5437 |
| | [M+NH₄]⁺ C₇₅H₈₄O₁₆NH₄ | m/z théorique : 1258.6104 |
| | | m/z mesurée: 1258.5887 |

### ➢ Allyl (2,3,4,6-tétra-O-benzyl-α-D-glucopyranosyl)-(1→3)-(2-O-benzoyl-4-O-benzyl-α-L-rhamnopyranosyl)-(1→2)-3,4-di-O-benzyl-α-L-rhamnopyranoside 107' :

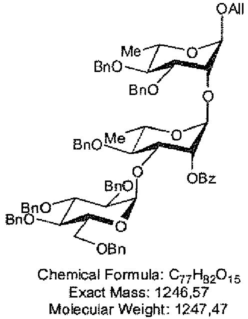

De l'acide triflique (16 µL, 186 µmol, 0,2 éq.) est additionné à une suspension d'accepteur **58** (463 mg, 1,2 mmol), de donneur **80'** (953 mg, 932 µmol) et de tamis moléculaire 4 Å (500 mg) dans du Tol (10 mL), agitée sous argon à -30°C. La réaction est poursuivie pendant que le bain éthanol-carboglace revient à TA (environ 1 h). Le bain est retiré et l'agitation est poursuivie pendant 1 h, temps au bout duquel le suivi par CCM (DCM/EtOAc, 98/2) indique la présence d'un nouveau composé majoritaire moins polaire que **58.** La réaction est stoppée par addition de triéthylamine (300 µL) puis le milieu réactionnel est filtré et concentré à l'évaporateur rotatif. Le brut réactionnel est purifié par chromatographie sur gel de silice (Chex/EtOAc, 100/0→80/20) pour obtenir le glycoside d'allyle **107'** sous forme d'une huile incolore (1,08 g, 93%).
Rf = 0,5 (Chex/EtOAc, 8/2).
RMN ¹H (CDCl₃), *δ* 8.09-7.08 (m, 40H, CH_{Ph}), 5.89 (m, 1H, CH=), 5.84 (dd, 1H, *J*_{1,2} = 2.1 Hz, H-2_{A}), 5.37 (d, 1H, *J*_{1,2} = 3.4 Hz, H-1_{E}), 5.28 (m, 1H, *J*ₜᵣₐₙₛ = 17.3 Hz, =CH₂), 5.20 (m_{superposé}, 1H, =CH₂), 5.19 (m_{superposé}, 1H, H-1_{A}), 5.00 (d, 1H*, J* = 10.2 Hz, H_{Bn}), 4.96 (d, 1H, *J* = 10.8 Hz, H_{Bn}), 4.86 (d, 1H, *J* = 10.9 Hz, H_{Bn}), 4.84 (d, 1H, *J* = 10.9 Hz, H_{Bn}), 4.78 (d, 1H, *J*_{1,2} = 1.8 Hz, H-1_{B}), 4.75 (d, 1H, *J* = 10.9 Hz, H_{Bn}), 4.72 (s, 2H, H_{Bn}), 4.67-4.63 (m, 3H, H_{Bn}), 4.60 (d, 1H, *J* = 12.1 Hz, H_{Bn}), 4.46 (m, 2H, H_{Bn}), 4.39 (dd, 1H, *J*_{2,3} = 3.1 Hz, *J*_{3,4} = 9.8 Hz, H-3_{A}), 4.36 (d, 1H, *J* = 12.0 Hz, H_{Bn}), 4.15 (m, 1H, H_{All}), 4.08-4.03 (m, 3H, H-3_{E}, H-5_{E}, H-2_{B}), 3.97-3.90 (m, 3H, H_{All}, H-5_{A}, H-3_{B}), 3.79 (dd, 1H, *J*_{4,5} = 9.4, *J*_{3,4} = 9.7 Hz, H-4_{E}), 3.76-3.69 (m, 3H, H-5_{B}, H-6a_{E}, H-4_{A}), 3.62 (dd, 1H, *J*_{1,2} = 3.4, *J*_{2,3} = 9.7 Hz, H-2_{E}), 3.59 (m, 1H, *J*_{5,6} = 1.7 Hz, H-6b_{E}), 3.56 (pt, 1H, *J*_{4,5}= 9.4 Hz, H-4_{B}), 1.43 (d, 3H, *J*_{5,6} = 6.2 Hz, H-6_{A}), 1.35 (d, 3H, *J*_{5,6} = 6.2 Hz, H-6_{B}).
RMN ¹³C (CDCl₃), *δ* 166.0 (C_{Bz}), 139.1-127.7 (C_{Ph}), 117.6 (=CH₂), 99.6 (C-1_{A}), 98.2 (C-1_{B}), 93.1 (C-1_{E}), 82.4 (C-3_{E}), 80.9 (C-4_{B}), 80.3 (C-4_{A}), 80.1 (C-3_{B}), 79.3 (C-2_{E}), 77.8 (C-4_{E}), 76.7, 76.0, 75.9, 75.3, (4C, C_{Bn}), 75.1 (C-2_{B}), 73.8 (C_{Bn}), 72.9 (C-3_{A}), 72.6, 72.5 (2C, C_{Bn}), 70.6 (C-5_{E}), 68.8 (2C, C-2_{A}, C-5_{B*}), 68.6 (C-6_{E}), 68.4 (C-5_{A*}), 68.0 (C_{All}), 18.5, 18.4 (C-6_{A}*, C-6_{B}*).

| | | |
|---|---|---|
| HRMS (ESI⁺) : | [M+Na]⁺ C₇₇H₈₂O₁₅Na | m/z théorique : 1269.5552 |
| | | m/z mesurée : 1269.5563 |

### ➢ Allyl (2,3,4,6-tétra-O-benzyl-α-D-glucopyranosyl)-(1→3)-(4-O-benzyl-α-L-rhamnopyranosyl)-(1→2)-3,4-di-O-benzy]-α-L-rhamnopyranoside 108 :

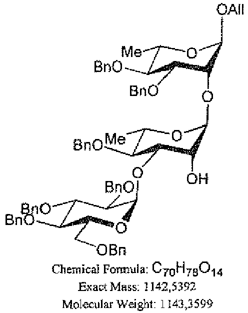

*Voie α.* Après addition de NaOMe méthanolique 0,5 M (2,5 mL, 1,25 mmol, 4 éq.) au glycoside d'allyle **107'** (385 mg, 308 µmol) en solution dans un mélange DCM/MeOH 1/1 (10 mL). Le milieu réactionnel est porté à reflux et agité pendant 3 h, temps au bout duquel le suivi par CCM (Chex/EtOAc, 7/3), indique la disparition de **107'** et l'apparition d'un produit plus polaire (Rf = 0,6). Après retour à TA, le milieu réactionnel est neutralisé par addition de résine échangeuse d'ion Dowex X8 (H⁺), puis filtré sur fritté. La résine est lavée avec un mélange DCM/MeOH et les solvants sont évaporés. L'huile jaune obtenue est purifiée par flash chromatographie sur gel de silice (Chex/EtOAc, 10/0→7/3) pour conduire à l'alcool **108** sous forme d'une huile incolore (303 mg, 86%).
*Voie b.* Après addition de NaOMe méthanolique 0,5 M (12,2 mL, 6,1 mmol, 1,7 éq.) au glycoside d'allyle **107** (4,5 g, 3,6 mmol) en solution dans du MeOH (200 mL). Le milieu réactionnel est porté à reflux et agité pendant 1 h, temps au bout duquel le suivi par CCM (Chex/EtOAc, 7/3), indique la disparition de **107** (Rf= 0,5) et l'apparition d'un produit plus polaire (Rf = 0,6). Après retour à TA, le milieu réactionnel est neutralisé par addition de résine échangeuse d'ion Dowex X8 (H⁺), puis filtré sur fritté. La résine est lavée avec un mélange DCM/MeOH et les solvants sont évaporés. L'huile jaune obtenue est purifiée par chromatographie sur gel de silice (Tol/EtOAc, 98/2→9/1) pour conduire à l'alcool **108** sous forme d'une huile incolore (3,9 g, 94%). Rf = 0,6 (Chex/EtOAc, 7/3).
RMN ¹H (CDCl₃), *δ* 7.47-7.19 (m, 35H, CH_{Ph}), 5.93 (m, 1H, CH=), 5.32 (m, 1H, *J*ₜᵣₐₙₛ = 17.2 Hz, =CH₂), 5.25 (m, 1H, *J*_{cis} = 10.3 Hz, =CH₂), 5.22 (bs, 1H, H-1_{A}), 5.05-4.99 (m, 3H, H_{Bn}), 4.97 (d, 1H, *J*_{1,2} = 3.5 Hz, H-1_{E}), 4.91-4.88 (m, 2H, H_{Bn}), 4.84 (d, 1H, *J*_{1,2} = 1.6 Hz, H-1_{B}), 4.83-4.67 (m, 6H, H_{Bn}), 4.60 (d, 1H, *J* = 12.0 Hz, H_{Bn}), 4.54 (d, 1H, *J* = 11.0 Hz, H_{Bn}), 4.36 (d, 1H, *J* = 12.0 Hz, H_{Bn}), 4.19 (m, 1H, H_{All}), 4.14-4.09 (m, 4H, H-2_{A}, H-2_{B}, H-3_{A}, H-3_{E}), 4.04-3.95 (m, 3H, H_{All}, H-5_{E}, H-3_{B}), 3.92 (dq, 1H, *J*_{4,5} = 9.7 Hz, H-5_{A}), 3.80 (pt, 1H, *J*_{4,5} = 9.8 Hz, H-4_{E}), 3.75 (pt, 1H, *J*_{4,5} = 9.4 Hz, H-5_{B}), 3.66 (dd, 1H, *J*_{2,3} = 3.6 Hz, *J*_{3,4} = 9.6 Hz, H-2_{E}), 3.75 (pt, 1H, *J*_{3,4} = 9.3 Hz, H-4_{A}), 3.55-3.49 (m, 2H, H-6a_{E}, H-4_{B}), 3.45 (dd, 1H, *J*_{5,6aE} = 1.8 Hz, *J*_{6aE,6bE} = 10.8 Hz, H-6b_{E}), 1.43 (d, 3H, *J*_{5,6} = 6.3 Hz, H-6_{A}), 1.39 (d, 3H, *J*_{5,6} = 6.2 Hz, H-6_{B}).
RMN ¹³C (CDCl₃), *δ* 138.7-137.6 (C_{Ph}), 133.9 (CH=), 129.1-127.0 (CH_{Ph}), 117.2 (=CH₂), 101.0 (C-1_{A}, ¹*J*_{CH}= 170.3 Hz), 98.1 (C-1_{B}, ¹*J*_{CH}= 170.3 Hz), 94.0 (C-1_{E}, ¹*J*_{CH} = 166.7 Hz), 82.4 (C-3_{E}), 80.6 (C-4_{B}), 80.0 (C-3_{B}), 79.3 (C-4_{A}), 79.0 (C-2_{E}), 77.8 (C-4_{E}), 76.5 (C-3_{A}), 75.6, 75.6, 75.5 (3C, C_{Bn}), 75.0 (C-2_{B}), 74.9, 74.4, 73.4, 72.5 (4C, C_{Bn}), 70.7 (C-5_{E}), 68.1 (C-5_{B}), 68.0 (C-6_{E}), 67.7 (C-5_{A}), 67.7 (C_{All}), 67.4 (C-2_{A}), 18.0, 17.9 (C-6_{A}*, C-6_{B}*).

| | | |
|---|---|---|
| HRMS (ESI⁺) : | [M+Na]⁺ C₇₀H₇₈O₁₄Na | m/z théorique : 1165.5289 |
| | | m/z mesurée : 1165.5283 |
| | [M+NH₄]⁺ C₇₀H₇₈O₁₄NH₄ | m/z théorique : 1160.5735 |
| | | m/z mesurée : 1160.5674 |

### ➢ Propyl α-D-glucopyranosyl-(1→3)-α-L-rhamnopyranosyl-(1→2)-α-L-rhamnopyranoside VII :

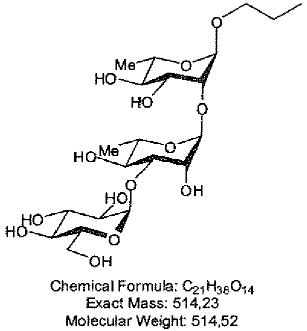

Du Pd/C10% (250 mg) est ajouté à une solution dégazée d'alcool **108** (260 mg, 209 µmol) dans de l'éthanol (10 mL) complété d'acide chlorhydrique 1M (155 µL). La suspension est placée sous atmosphère d'hydrogène (1 bar) et agitée une nuit à TA. Le suivi par CCM (iPrOH/H₂O/NH₃, 7/1/02 montre la disparition de **108** et l'apparition d'un nouveau composé plus polaire. Le milieu réactionnel est filtré sur Célite, et le filtrat est concentré à l'évaporateur rotatif. Le résidu obtenu est purifié par HPLC (colonne C-18 Kromasil, TFA aqueux 0,01M /CH₃CN, 100/0→60/40 en 20 min, 5 mL/min, 215 nm) pour conduire au tétrasaccahride libre ***VII*** sous forme d'une poudre blanche après lyophilisation (83 mg, 77%).
Rf = 0,5 (iPrOH/H₂O/NH₃, 4/1/2).
RMN ¹H (D₂O), δ 5.09 (d, 1H, J_{1,2} = 387 Hz, H-1_{Er}), 4.98 (d, 1H, J_{1,2} = 1.7 Hz, H-1_{A}), 4.88 (d, 1H, J_{1,2} = 1.2 Hz, H-1_{C}), 4.25 (m, 1H, H-2_{A}), 3.94 (m, 1H, H-5_{E}), 3.90 (dd, 1H, H-2_{B}), 3.85-3.49 (m, 6H, H-3_{A}, H-3_{B}, H-3_{E}, H-6a_{E}, H-6b_{E}, H-5_{A}), 3.69 (dq, 1H, , J_{4,5} = 9.6 Hz, H-5_{B}), 3.64-3.48 (m, 4H, OCH₂, H-2_{E}, H-4_{A}, OCH₂), 3.52 (m, 1H, H_{Pr}), 3.44 (t, 2H, H-4_{E}, H-4_{B}) 1.59 (sex, 2H, CH₂), 1.27 (d, 6H, J_{5,6} = 6.2 Hz, H-6_{A}, H-6_{C}), 0.90 (t, 3H, J = 7.4 Hz, CH₃).
RMN ¹³C (D₂O), δ 102.4 (C-1_{C}, ¹J_{CH} = 175 Hz), 99.0 (C-1_{B}, ¹J_{CH}= 170.5 Hz), 95.7 (C-1_{E}, ¹J_{CH}= 168.8 Hz), 79.5 (C-2_{B}), 75.6 (C-3_{A}), 72.6 (C-4₂*), 72.1 (C-2_{E}), 71.8 (C-5_{E}), 70.7 (C-3_{B}), 70.5 (C-4_{A}), 70.2(C_{Pr}), 69.9 (C-4_{E}), 69.7 (C-5_{A}), 69.1 (C-5_{B}), 67.1 (C-2_{A}), 60.8 (C-6_{E}), 22.3 (CH₂), 17.2, 17.0 (C-6_{A}, C-6_{B}), 10.3 (CH₃). MS (ESI⁺) pour C₂₉H₅₁NO₁ m/z théorique 514.2, trouvée 515.1 [M+H]⁺.

### Procédé 14 :

### ➢ Allyl (2-désoxy-4,6-O-isopropylidène-3-O-lévulinoyl-2-trichloroacétamido-β-D-glucopyranosyl)-(1→2)-[2,3,4,6-tétra-O-benzyl-α-D-glucopyranosyl-(1→3)]-4-O-benzy|-α-L-rhamnopyranoside 111 :

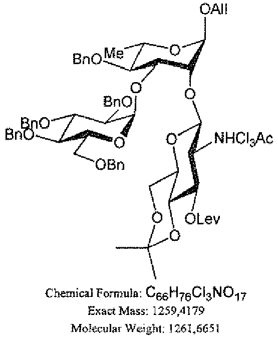

L'alcool **38** (2,0 g, 1,7 mmol) est dissous dans du DCM (15 mL) puis la DMAP (420 mg, 3,4 mmol, 2 éq.) est ajouté au milieu réactionnel. Dans un autre ballon, la DCC (532 mg, 2,6 mmol, 1,5 éq.), et l'acide lévulinique (317 µL, 3,0 mmol, 1,8 éq.) sont agités dans du DCM (15 mL) puis ajoutés au milieu réactionnel. Le milieu réactionnel est alors agité durant 1 h, temps au bout duquel le suivi par CCM (Tol/EtOAc, 7/3) montre la formation d'un composé moins polaire majoritaire (Rf = 0,5) et la disparition de **38** (Rf = 0,35). La DCU est filtrée sur célite et le filtrat est repris à l'H₂O (15 mL). La phase aqueuse est extraite au DCM (3 x 100 mL). Les phases organiques sont regroupées et lavées avec une solution de NaHCO₃ₛₐₜ (3 x 50 mL), une solution de NaClₛₐₜ (3 x 50 mL), filtrées sur filtre séparateur de phases et concentrées à l'évaporateur rotatif. L'huile obtenue est purifiée par chromatographie sur gel de silice (Tol/EtOAc, 85/15→8/2) pour conduire à l'ester de lévulinoyle **111** (1,9 g, 90%).
Rf = 0,5 (Tol/EtOAc, 7/3).
RMN ¹H (CDCl₃), *δ* 7.44-7.08 (m, 26H, NH, CH_{Ph}), 5.94 (m, 1H, CH=), 5.29 (m, 1H, *J*ₜᵣₐₙₛ= 17.2 Hz, =CH₂), 5.21 (m, 1H, *J*_{cis} = 10.4 Hz, =CH₂), 5.11 (d, 1H, *J*_{1,2} = 3.5 Hz, H-1_{E}), 5.10-5.07 (m, 4H, H_{Bn}), 4.80-4.69 (m, 5H, H-1_{A}, H_{Bn}, H-3_{D}, H_{Bn}, H-1_{D}), 4.59-4.54 (m, 2H, H_{Bn})₅ 4.90 (d, 1H, *J* = 11.0 Hz, H_{Bn}), 4.32 (d, 1H, *J* = 12.0 Hz, H_{Bn}), 4.18-4.07 (m, 5H, H_{All}, H-2_{D}, H-3_{E}, H-3_{A}, H-5_{E}), 3.99 (m, 1H, *J*_{1,2} = 2.3 Hz, *J*_{2,3} = 4.8 Hz, H-2_{A}), 3.94 (m, 1H, H_{All}), 3.90 (dd, 1H, *J*_{1,2} = 3.6 Hz, *J*_{2,3} = 9.7 Hz, H-2_{E}), 3.85 (dd, 1H, *J*_{5,6a} = 5.4 Hz, *J*_{6a,6b} = 10.7 Hz, H-6a_{D}), 3.79 (pt, 1H, *J*_{3,4} = 10.0 Hz, H-4_{E}), 3.74-3.69 (m, 2H, H-6b_{D}, H-5_{A}), 3.67 (pt, 1H, *J*_{3,4} = 9.6 Hz, H-4_{D}), 3.45 (pt, 1H, *J*_{3,4} = 9.6 Hz, H-4_{A}), 3.41 (m, 2H, H-6a_{E}, H-6b_{E}), 2.76-2.63 (m, 5H, H-5_{D}, 2CH_{2Lev}), 2.20 (s, 3H, CH_{3Lev}), 1.47 (s, 3H, H_{iPr}), 1.42 (s, 3H, H_{iPr}), 1.39 (d, 3H, *J*_{5,6} = 6.3 Hz, H-6_{A}). RMN ¹³C (CDCl₃), *δ* 206.4 (C_{Lev}), 172.6 (C_{Lev}), 162.5 (C_{NTCA}), 139.0-138.5 (C_{Ph}), 134.2 (CH=), 129.6-127.7 (CH_{Ph}), 117.6 (=CH₂), 101.8 (C-1_{D}, ¹*J*_{CH}= 163.0 Hz), 100.1 (C_{iPr}), 98.7 (C-1_{A}, ¹*J*_{*C*H} = 171.6 Hz), 95.1 (C-1_{E}, ¹*J*_{CH} = 165.2 Hz), 93.2 (CCl₃), 83.8 (C-3_{E}), 80.2 (C-4_{A}), 79.3 (C-2_{E}), 79.1 (C-4_{E}), 76.4, 75.5, 75.3 (3C, C_{Bn}), 75.0 (C-3_{A}), 74.7 (C_{Bn}), 74.6 (C-2_{A}), 73.8 (C_{Bn}), 73.3 (C-3_{D}), 71.6 (C-4_{D}), 70.3 (C-5_{E}), 68.7 (C-5_{A}), 68.3, 68.2 (2C, C_{All}*, C-6_{E}*), 67.5 (C-5_{D}), 62.4 (C-6_{D}), 56.9 (C-2_{D}), 38.4 (CH_{2Lev}), 30.2 (CH_{3Lev}), 29.4 (C_{iPr}), 28.5 (CH_{2Lev}), 19.3 (C_{iPr}), 18.3 (C-6_{A}).

| | | |
|---|---|---|
| HRMS (ESI⁺) : | [M+Na]⁺ C₆₆H₇₆NO₁₇³⁵Cl₃Na | m/z théorique : 1282.4076 |
| | | m/z mesurée : 1282.4189 |
| | [M+NH₄]⁺ C₆₆H₇₆NO₁₇³⁵Cl₃NH₄ | m/z théorique : 1277.4523 |
| | | m/z mesurée : 1277.4634 |

### ➢ (2-Désoxy-4,6-O-isopropylidène-3-O-lévulinoyl-2-trichloroacétamido-β-D-glucopyranosyl)-(1→2)-[2,3,4,6-tétra-O-benzyl-α-D-glucopyranosyl-(1→3)]-4-O-benzyl-α-L-rhamnopyranose 112 :

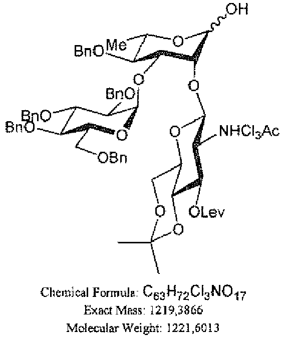

Le (1,5-cyclooetadiènebis(méthyldiphénylphosphine)iridium(I)hexafluorophosphate) (30 mg) est dissous dans du THF (11 mL) sous agitation. Le milieu subit 5 cycles dégazage vide/argon, 5 cycles dégazage vide/hyrdogène puis est mis sous hydrogène pendant 15 min. Le milieu subit alors une seconde fois 5 cycles dégazage vide/argon. La solution d'ester de lévulinoyle **111** (1,8 g, 1,5 mmol) dans du THF (15 mL) est transféré sur la solution de catalyseur activé. Le suivi par CCM (Tol/EtOAc, 8/2) montre la disparition de **111** (Rf= 0,5) et l'apparition d'un produit un peu moins polaire (Rf = 0,55).

Du diiode (740 mg, 2,9 mmol, 2 éq.) en solution dans un mélange THF/H₂O (8/2, 10 mL) est ajouté au milieu réactionnel. Le suivi par CCM (Tol/EtOAc, 8/2 et DCM/MeOH, 98/2) indique la disparition de l'intermédiaire (Rf = 0,55 et 1, respectivement) et l'apparition d'un nouveau composé beaucoup plus polaire (Rf = 0 et 0,3, respectivement). Pour stopper la réaction, une solution aqueuse de NaHSO₃ 10% (4 mL) est ajoutée par fractions au milieu jusqu'à disparition de la coloration noire. Le milieu réactionnel est repris avec du DCM (50 mL) et la phase aqueuse est extraite au DCM (3 x 50 mL). Les phases organiques sont regroupées et lavées avec une solution de NaClₛₐₜ (3 x 15 mL), avec de l'H₂O (3 x 15 mL) puis séchées sur filtre séparateur de phases et concentrées à l'évaporateur rotatif. L'huile jaune obtenue est purifiée par chromatographie sur gel de silice (DCM/MeOH, 99/1→97/3) pour obtenir majoritairement l'anomère α **112** sous forme d'une huile jaune (1,4 g, 80%).
Rf = 0,3 (DCM/MeOH, 98/2).
RMN ¹H (CDCl₃), *δ* 7.45-7.05 (m, 26H, NH, CH_{Ph}), 5.16 (d, 1H, *J*_{1,2} = 2.2 Hz, H-1_{A}), 5.14 (d, 1H, *J*_{1,2} = 3.7 Hz, H-1_{E}), 5.12-5.07 (m, 4H, H_{Bn}), 4.78-4.68 (m, 4H, H-3_{D}, H_{Bn}, H-1_{D}, H_{Bn}), 4.58-4.44 (m, 3H, H_{Bn}), 4.30 (d, 1H, *J* = 12.0 Hz, H_{Bn}), 4.21 (dd, 1H, *J*_{2,3} = 2.9 Hz, *J*_{3,4} = 9.6 Hz, H-3_{A}), 4.16-4.07 (m, 3H, H-2_{D}, H-3_{E}, H-5_{E}), 4.01 (m, 1H, *J*_{1,2} = 2.0 Hz, *J*_{2,3} = 4.5 Hz, H-2_{A}), 3.95 (dq, 1H, *J*_{4,5} = 9.4 Hz, H-5_{A}), 3.90 (dd, 1H, *J*_{1,2} = 2.4 Hz, *J*_{2,3} = 9.6 Hz, H-2_{E}), 3.85 (dd, 1H, *J*_{5,6a} = 5.1 Hz, *J*_{6a,6b} = 10.4 Hz, H-6a_{D}), 3.76 (pt, 1H, *J*_{3,4} = 9.2 Hz, H-4_{E}), 3.70 (m, 1H, H-6b_{D}), 3.66 (pt, 1H, *J*_{3,4} = 9.5 Hz, H-4_{D}), 3.45 (pt, 1H, *J*_{3,4} = 9.5 Hz, H-4_{A}), 3.39 (m, 2H, H-6a_{E}, H-6b_{E}), 3.30 (m, 1H, OH-1), 2.77-2.61 (m, 5H, H-5_{D}, 2CH_{2Lev}), 2.19 (s, 3H, CH_{3Lev}), 1.47 (s, 3H, H_{iPr}), 1.42 (s, 3H, H_{iPr}), 1.38 (d, 3H, *J*_{5,6} = 6.2 Hz, H-6_{A}).
RMN ¹³C (CDCl₃), *δ* 206.2 (C_{Lev}), 172.2 (C_{Lev}), 162.1 (C_{NTCA}), 138.6-137.6 (C_{Ph}), 129.3-127.2 (CH_{Ph}), 101.4 (C-1_{D}, ¹*J*_{CH} = 167.7 Hz), 99.7 (C_{iPr}), 94.6 (C-1_{E}, ¹*J*_{CH} = 167.2 Hz), 93.9 (C-1_{A}, ¹*J*_{CH} = 171.7 Hz), 92.8 (CCl₃), 83.4 (C-3_{E}), 79.7 (C-4_{A}), 78.8 (C-2_{E}), 78.7 (C-4_{E}), 76.2, 75.9, 75.1 (3C, C_{Bn}), 74.5 (C-2_{A}), 74.4 (C_{Bn}), 74.1 (C-3_{A}), 73.3 (C_{Bn}), 72.8 (C-3_{D}), 71.2 (C-4_{D}), 69.9 (C-5_{E}), 68.3 (C-5_{A}), 67.8 (C-6_{E}), 67.1 (C-5_{D}), 62.0 (C-6_{D}), 56.4 (C-2_{D}), 38.0 (CH_{2Lev}), 29.8 (CH_{3Lev}), 28.9 (C_{iPr}), 28.0 (CH_{2Lev}), 18.9 (C_{iPr}), 17.9 (C-6_{A}).

| | | |
|---|---|---|
| HRMS (ESI⁺) : | [M+Na]⁺ C₆₃H₇₂NO₁₇³⁵Cl₃Na | m/z théorique : 1242.3763 |
| | | m/z mesurée : 1242.3856 |
| | [M+NH₄]⁺ C₆₆H₇₆NO₁₇³⁵Cl₃NH₄ | m/z théorique : 1237.4209 |
| | | m/z mesurée : 1237.4213 |

### ➢ Trichloroacétimidate de (2-désoxy-4,6-O-isopropylidène-3-O-lévulinoyl-2-trichloroacétamido-β-D-glucopyranosyl)-(1→2)-[2,3,4,6-tétra-O-benzyl-α-D-glucopyranosyl-(1→3)]-4-O-benzyl-α-L-rhamnopyranose 109 :

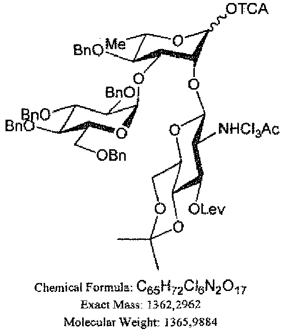

L'hémiacétal 112 (1,3 g, 1,1 mmol) est solubilisé dans du DCE (10 mL) et agité sous argon à -5°C puis de la DBU (44 µL, 308 µmol, 0,28 éq.) et du trichloroacétonitrile (525 µL, 5,2 mmol, 5 éq.) sont ajoutés. Le milieu réactionnel est agité à cette température pendant 15 min puis il est déposé, sans aucun traitement, sur une colonne de gel de silice (flash chromatographie, Chex/EtOAc+5% NEt₃, 7/3→1/1) pour obtenir le trichloroacétimidate **109** une huile jaune (1,2 g, 85%).
Rf = 0,45 (Chex/EtOAc, 6/4).
RMN ¹H (CDCl₃), *δ*8.65 (s, 1H, NH), 7.45-7.06 (m, 26H, NH, CH_{Ph}), 6.22 (s, 1H, H-1_{A}), 5.11-5.01 (m, 5H, H-1_{E}, 4H_{Bn}), 4.80 (pt, 1H, *J*_{3,4} = 9.9 Hz, H-3_{D}), 4.78 (m, 2H, H_{Bn}) 4.73 (d, 1H, *J*_{1,2} = 8.4 Hz, H-1_{D}), 4.57-4.49 (m, 3H, H_{Bn}), 4.33 (d, 1H, *J* = 12.9 Hz, H_{Bn}), 4.18-4.07 (m, 5H, H-2_{D}, H-3_{A}, H-2_{A}, H-3_{E}, H-5_{E}), 3.93-3.85 (m, 3H, H-5_{A}, H-2_{E}, H-6a_{D}), 3.82-3.74 (m, 2H, H-4_{E}, H-6b_{D}), 3.71 (pt, 1H, *J*_{3,4} = 9.6 Hz, H-4_{D}), 3.56 (pt, 1H, *J*_{3,4} = 9.6 Hz, H-4_{A}), 3.42 (m, 2H, H-6a_{E}, H-6b_{E}), 2.85 (m, 1H, H-5_{D}), 2.77-2.58 (m, 4H, 2CH_{2Lev}), 2.20 (s, 3H, CH_{3Lev}), 1.49 (s, 3H, Hi_{Pr}), 1.43 (s, 3H, Hi_{Pr}), 1.42 (d, 3H, *J*_{5,6} = 6.0 Hz, H-6_{A}).
RMN ¹³C (CDCl₃), *δ*206.0 (C_{Lev}), 172.3 (C_{Lev}), 162.1 (C_{NTCA}), 160.2 (C=NH), 138.5-137.4 (C_{Ph}), 129.2-127.4 (CH_{Ph}), 101.3 (C-1_{D}, ¹J_{CH} = 159.2 Hz), 99.8 (C_{iPr}), 96.9 (C-1_{A}, ¹*J*_{CH}= 182.9 Hz), 95.0 (C-1_{E}, ¹*J*_{CH}= 165.6 Hz), 92.7 (CCl₃), 91.1 (CCl₃), 83.3 (C-3_{E}), 79.2 (C-4_{A}), 78.7 (C-2_{E}), 78.6 (C-4_{E}), 76.3, 75.3, 75.0, 74.4 (4C, C_{Bn}), 74.3 (C-3_{A}), 73.3 (C_{Bn}), 72.7 (C-3_{D}), 72.2 (C-2_{A}), 71.2 (C-4_{D}), 71.1 (C-5_{A}), 70.1 (C-5_{E}), 67.9 (C-6_{E}), 67.3 (C-5_{D}), 61.9 (C-6_{D}), 56.5 (C-2_{D}), 38.0 (CH_{2Lev}), 29.8 (CH_{3Lev}), 29.0 (C_{iPr}), 28.1 (CH_{2Lev}), 18.9 (C_{iPr}), 17.9 (C-6_{A}).

### ➢ Allyl (2-désoxy-4,6-O-isopropylidène-3-O-lévulinoyl-2-trichloroacétamido-β-D-glucopyranosyl)-(1→2)-([2,3,4,6-tétra-O-benzyl-α-D-glucopyranosyl-(1→3)]-4-O-benzyl-α-L-rhamnopyranosyl)-(1→2)-3,4-di-O-benzyl-α-L-rhamnopyranoside 113 :

### ➢ Allyl (2-désoxy-4,6-O-isopropylidene-3-O-lévulinoyl-2-trichioroacétamido-β-D-glucopyranosyl)-(1→2)-([2,3,4,6-tétra-O-benzyl-α-D-glucopyranosyl-(1→3)]-4-O-benzyl-β-L-rhamnopyranosyl)-(1→2)-3,4-di-O-benzyl-α-L-rhamnopyranoside 114 :

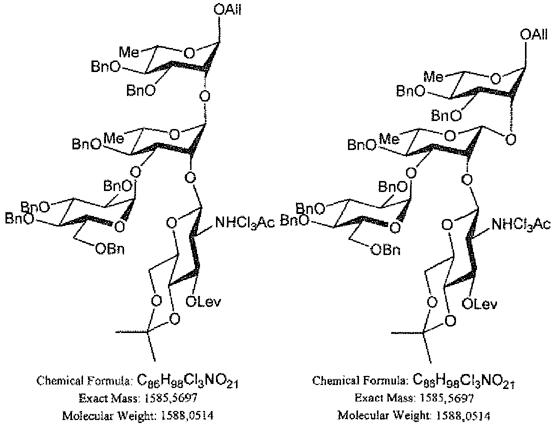

Du TMSOTf (10,0 µL, 56 µmol, 0,3 éq.) est additionné à une solution d'accepteur **58** (71 mg, 185 µmol) et de donneur **109** (380 mg, 280 µmol, 1,5 éq.) dans du DCM (5 mL), en présence de tamis moléculaire 4 Å (160 mg), agitée sous argon à -78°C. Après 15 min sous agitation, le suivi par CCM (Tol/EtOAc, 7/3) indique la disparition de **58** (Rf = 0,5) et l'apparition de deux nouveaux composés plus polaires (Rf = 0,6 et 0,65). La réaction est stoppée par l'addition de triéthylamine (1 mL) puis le milieu réactionnel est filtré et concentré à l'évaporateur rotatif. L'huile jaune obtenue est purifiée par chromatographie sur gel de silice (Tol/EtOAc, 9/1→1/1) pour obtenir par ordre d'élution à l'anomère α **113** sous forme d'une huile incolore (130 mg, 44%) puis à l'anomère β **114** sous forme d'une huile incolore (130 mg, 44%).
**113** : Rf = 0,65 (Tol/EtOAc, 7/3).
RMN ¹H (CDCl₃), *δ*: α : 7.49-7.07 (m, 36H, NH, CH_{Ph}), 5.89 (m, 1H, CH=), 5.27 (m, 1H, *J*tᵣₐₙₛ = 17.2 Hz, =CH₂), 5.21 (m, 1H, *J*_{cis} = 10.3 Hz, =CH₂), 5.16 (d, 1H, *J*_{1,2} = 3.6 Hz, H-1_{E}), 5.13-5.05 (m, 5H, H-1_{A}, 4H_{Bn}), 4.93 (d, 1H, *J* = 10.8 Hz, H_{Bn}), 4.83-4.76 (m, 5H, H-3_{D}, 2H_{Bn}, H-1_{D}, H-1_{B}), 4.73-4.48 (m, 6H, H_{Bn}), 4.32 (d, 1H, *J=* 12.0 Hz, H_{Bn}), 4.20-4.09 (m, 6H, H-3_{E}, H-2_{D}, H-2_{A}, H-3_{A}, H_{All}, H-5_{E}), 4.00 (dd, 1H, *J*_{1,2} = 2.9 Hz, *J*_{2,3} = 5.0 Hz, H-2_{B}), 3.98 (m, 1H, H_{All}), 3.95-3.89 (m, 2H, H-3_{B}, H-2_{E}), 3.83 (pt, 1H, *J*_{3,4} = 9.2 Hz, H-4_{E}), 3.81 (m, 1H, H-5_{B}), 3.73 (dq, 1H, *J*_{4,5} = 9.4 Hz, *J*_{5,6} = 6.1 Hz, H-5_{A}), 3.64 (pt, 1H, *J*_{3,4} = 9.5 Hz, H-4_{D}), 3.54-3.45 (m, 5H, H-6a_{D}, H-4_{A}, H-4_{B}, H-6a_{E}, H-6b_{E}), 3.38 (m, 1H, H-6b_{D}), 2.77 (m, 1H, H-5_{D}), 2.77-2.62 (m, 4H, 2CH_{2Lev}), 2.20 (s, 3H, CH_{3Lev}), 1.44 (s, 3H, R_{iPr}), 1.40 (d, 3H, *J*_{5,6} = 6.3 Hz, H-6_{B}), 1.39 (s, 3H, H_{iPr}), 1.32 (d, 3H, *J*_{5,6}= 6.2 Hz, H-6_{A}).
RMN ¹³C (CDCl₃), *δ* : α : 206.2 (C_{Lev}), 172.3 (C_{Lev}), 162.2 (C_{NTCA}), 138.6-137.5 (C_{Ph}), 133.8 (CH=), 129.2-127.3 (CH_{Ph}), 117.2 (=CH₂), 101.3 (C-1_{D}, ¹*J*_{CH}= 162.0 Hz), 100.9 (C-1_{A}, ¹*J*_{CH} = 171.3 Hz), 99.7 (C_{iPr}), 97.9 (C-1_{B}, ¹*J*_{CH} = 170.2 Hz), 94.6 (C-1_{E}, ¹*J*_{CH}= 165.1 Hz), 92.8 (CCl₃), 83.4 (C-3_{E}), 80.4 (C-4_{B}), 79.8 (C-4_{A}), 79.5 (C-2_{E}), 78.6 (C-4_{E}), 78.4 (C-3_{B}), 76.1, 75.3, 75.2, 74.9 (4C, C_{Bn}), 74.8 (C-2_{B}), 74.4 (C-3_{A}), 74.1 (C_{Bn}), 73.8 (C-2_{A}), 73.5 (C_{Bn}), 72.9 (C-3_{D}), 72.1 (C_{Bn}), 71.3 (C-4_{D}), 69.9 (C-5_{E}), 68.6 (C-5_{B}), 68.0 (C-5_{A}), 67.9 (C-6_{E}), 67.7 (C_{All}), 67.2 (C-5_{D}), 61.7 (C-6_{D}), 56.6 (C-2_{D}), 38.0 (CH_{2Lev}), 29.7 (CH_{3Lev}), 29.0 (C_{iPr}), 28.1 (CH_{2Lev}), 19.0 (C_{iPr}), 18.0 (C-6_{A}), 17.9 (C-6_{B}).

| | | |
|---|---|---|
| HRMS (ESI⁺) : | [M+Na]⁺ C₈₆H₉₈NO₂₁³⁵Cl₃Na | m/z théorique : 1608.5594 |
| | | m/z mesurée : 1608.5730 |
| | [M+NH₄]⁺ C₈₆H₉₈NO₂₁³⁵Cl₃NH₄ | m/z théorique : 1603.6041 |
| | | m/z mesurée : 1603.6112 |

**114 :** Rf = 0,6 (Tol/EtOAc, 7/3).
RMN ¹H (CDCl₃), *δ*: β : 7.66-7.05 (m, 36H, NH, CH_{Ph}), 5.89 (m, 1H, CH=), 5.31 (m, 1H, *J*ₜᵣₐₙₛ = 17.2 Hz, =CH₂), 5.21 (m, 1H, *J*_{cis}= 10.3 Hz, =CH₂), 5.20 (s, 2H, H_{Bn}), 5.11 (d, 1H, *J*_{1,2} = 3.5 Hz, H-1_{E}), 5.05 (d, 2H, H_{Bn}), 4.95-4.89 (m, 4H, H-1_{D}, H-3_{D}, 2H_{Bn}), 4.86 (m, 1H, *J*_{1,2} = 2.7 Hz, H-1_{B}), 4.79 (d, 1H, *J* = 10.5 Hz, H_{Bn}), 4.64-4.48 (m, 7H, 3H_{Bn}, H-1_{A}, 3H_{Bn}), 4.34 (d, 1H, *J=* 12.2 Hz, H_{Bn}), 4.30-4.11 (m, 6H, H-2_{D}, H_{All}, H-2_{B}, H-2_{A}, H-3_{E}, H-5_{E}), 4.00 (m, 1H, H_{All}), 3.99-3.93 (m, 2H, H-3_{B}, H-2_{E}), 3.88 (dd, 1H, *J*_{5,6a} = 5.2 Hz, *J*_{6a,6b} = 10.6 Hz, H-6a_{D}), 3.80 (dd, 1H, *J*_{2,3} = 2.5 Hz, *J*_{3,4} = 9.5 Hz, H-3_{A}), 3.78-3.67 (m, 3H, H-5_{B}, H-4_{E}, H-6b_{D}), 3.60-3.40 (m, 5H, H-4_{D}, H-4_{A}, H-4_{B}, H-6a_{E}, H-6b_{E}), 3.28 (dq, 1H, *J*_{4,5} = 9.2 Hz, *J*_{5,6} = 6.2 Hz, H-5_{A}), 2.83 (m, 1H, H-5_{D}), 2.78-2.64 (m, 4H, 2CH_{2Lev}), 2.18 (s, 3H, CH_{3Lev}), 1.37 (s, 3H, H_{iPr}), 1.40 (m, 6H, H-6_{A}, H-6_{B}), 1.32 (s, 3H, H_{iPr}).
RMN ¹³C (CDCl₃), *δ*: β : 206.1 (C_{Lev}), 172.2 (C_{Lev}), 162.0 (C_{NTCA}), 139.1-137.5 (C_{Ph}), 134.0 (CH=), 129.1-127.3 (CH_{Ph}), 117.2 (=CH₂), 100.7 (C-1_{D}, ¹*J*_{CH}= 163.8 Hz), 99.7 (C_{iPr}), 97.5 (C-1_{A}, ¹*J*_{CH} = 155.1 Hz), 96.9 (C-1_{B}, ¹*J*_{CH} = 167.2 Hz), 94.1 (C-1_{E}, ¹*J*_{CH} = 167.7 Hz), 92.8 (CCl₃), 83.7 (C-3_{E}), 81.0 (C-4_{B}), 79.7 (C-4_{A}), 78.7 (C-4_{E}), 77.8 (C-2_{E}), 77.4 (C-3_{B}), 76.4 (C-3_{A}), 76.2, 75.3, 75.1, 74.6, 73.6 (5C, C_{Bn}), 73.3 (3C, C-2_{A}*, C-2_{B}*, C-3_{D}*), 73.5 (C_{Bn}), 72.4 (C-5_{A}), 71.3 (C-4_{D}), 71.0 (C_{Bn}), 69.9 (C-5_{E}), 68.0 (2C, C-5_{B}*, C-6_{E}*), 67.8 (C_{All}), 67.0 (C-5_{D}), 62.4 (C-6_{D}), 56.7 (C-2_{D}), 38.0 (CH_{2Lev}), 29.8 (CH_{3Lev}), 29.0 (C_{iPr}), 28.1 (CH_{2Lev}), 19.9 (C_{iPr}), 18.4 (C-6_{A}), 18.0 (C-6_{B}).

| | | |
|---|---|---|
| HRMS (ESI⁺) : | [M+Na]⁺ C₈₆H₉₈NO₂₁³⁵Cl₃Na | m/z théorique : 1608.5594 |
| | | m/z mesurée : 1608.5914 |
| | [M+NH₄]⁺ C₈₆H₉₈NO₂₁³⁵Cl₃NH₄ | m/z théorique : 1603.6041 |
| | | m/z mesurée : 1603.6248 |

### Procédé 14bis :

### ➢ Allyl (3-O-acétyl-2-désoxy-4,6-O-isopropylidene-2-trichloroacétamido-β-D-glucopyranosyl)-(1→2)-[2,3,4,6-tétra-O-benzyl-α-D-glucopyranosyl-(1→3)]-(4-O-benzyl-α-L-rhamnopyranosyl)-(1→2)-3,4-di-O-benzyl-α-L-rhamnopyranoside 115 :

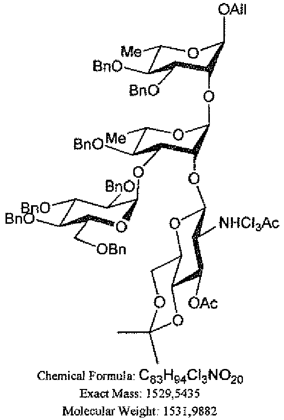

Du TMSOTf (69,0 µL, 380 µmol, 0,3 éq.) est additionné à une solution d'accepteur **108** (1,5 g, 1,3 mmol) et de donneur **2** (1,2 g, 2,2 mmol, 1,7 éq.) dans du Tol (30 mL), en présence de tamis moléculaire **4** Å (1,1 g), agitée sous argon à -40°C. Après 1 h d'agitation, le suivi par CCM (Tol/EtOAc, 85/15) indique la disparition de **108** (Rf = 0,5) et l'apparition d'un nouveau composé plus polaire (Rf = 0,45). La réaction est stoppée par addition de triéthylamine (1 mL) puis le milieu réactionnel est filtré et concentré à l'évaporateur rotatif. L'huile jaune obtenue est purifiée par chromatographie sur gel de silice (Tol/EtOAc, 95/5→1/1) pour obtenir le glycoside d'allyle **115** sous forme d'un solide blanc (1,9 g, 95%).
Rf = 0,45 (Tol/EtOAc, 85/15).
RMN ¹H (CDCl₃), *δ* 7.54-7.09 (m, 36H, NH, CH_{Ph}), 5.91 (m, 1H, CH=), 5.30 (m, 1H, *J*ₜᵣₐₙₛ= 17.2 Hz, =CH₂), 5.23 (m, 1H, *J*_{cis}= 10.5 Hz, =CH₂), 5.23-5.19 (m, 2H, H-1_{E}, H_{Bn}), 5.15 (bs, 1H, H-1_{A}), 5.11 (d, 1H, *J* = 10.9 Hz, H_{Bn}), 5.08 (d, 1H, *J* = 11.0 Hz, H_{Bn}), 4.96 (d, 1H, *J* = 10.8 Hz, H_{Bn}), 4.88-4.78 (m, 5H, H_{Bn}, H-1_{D}, H-3_{D}, 2H_{Bn}), 4.75-4.66 (m, 3H, H_{Bn}), 4.63-4.58 (m, 3H, H-1_{B}, 2H_{Bn}), 4.52 (d, 1H, *J* = 11.0 Hz, H_{Bn}), 4.36 (d, 1H, *J=* 12.0 Hz, H_{Bn}), 4.25-4.12 (m, 6H, H-2_{D}, H-3_{E}, H-2_{A}, H-3_{A}, H_{All}, H-5_{E}), 4.03 (dd, 1H, *J*_{2,3} = 2.8 Hz, H-2_{B}), 4.01-3.92 (m, 3H, H_{All}, H-2_{E}, H-3_{B}), 3.88-3.80 (m, 2H, H-4_{E}, H-5_{A}), 3.75 (dq, 1H, *J*_{4,5} = 9.4 Hz, H-5_{B}), 3.68 (pt, 1H, *J*_{3,4} = 9.5 Hz, H-4_{D}), 3.57-3.40 (m, 6H, H-6a_{D}, H-4_{A}, H-4_{B}, H-6a_{E}, H-6b_{E}, H-6b_{D}), 2.82 (m, 1H, H-5_{D}), 2.11 (s, 3H, H_{Ac}), 1.49 (s, 3H, H_{iPr}), 1.44 (d, 3H, *J*_{5,6} = 6.2 Hz, H-6_{A}), 1.41 (s, 3H, H_{iPr}), 1.36 (d, 3H, *J*_{5,6}= 6.2 Hz, H-6_{B}).
RMN ¹³C (CDCl₃), *δ* 170.8 (C_{Ac}), 162.2 (C_{NTCA}), 138.6-137.6 (C_{Ph}), 133.9 (CH=), 129.1-127.4 (CH_{Ph}), 117.2 (=CH₂), 101.4 (C-1_{D}, ¹*J*_{CH}= 164.1 Hz), 101.0 (C-1_{A}, ¹*J*_{CH} = 172.5 Hz), 99.7 (C_{iPr}), 97.9 (C-1_{B}, ¹*J*_{CH} = 167.9 Hz), 94.6 (C-1_{E}, ¹*J*_{CH} = 167.1 Hz), 92.8 (CCl₃), 83.5 (C-3_{E}), 80.5 (C-4_{B}), 79.8 (C-4_{A}), 79.6 (C-3_{B}), 78.6 (C-4_{E}), 78.2 (C-2_{E}), 76.2, 75.3, 75.2, 75.0 (4C, C_{Bn}), 74.9 (C-2_{B}), 74.4 (C-3_{A}), 74.0 (C_{Bn}), 73.9 (C-2_{A}), 73.5 (C_{Bn}), 72.6 (C-3_{D}), 72.2 (C_{Bn}), 71.3 (C-4_{D}), 70.0 (C-5_{E}), 68.7 (C-5_{A}), 68.0 (C-5_{B}), 67.9 (C-6_{E}), 67.7 (C_{All}), 67.4 (C-5_{D}), 61.8 (C-6_{D}), 56.6 (C-2_{D}), 29.0 (C_{iPr}), 20.9 (C_{Ac}), 19.1 (C_{iPr}), 18.0 (C-6_{B}), 17.9 (C-6_{A}).

| | | |
|---|---|---|
| HRMS (ESI⁺) : | [M+Na]⁺ C₈₃H₉₄NO₂₀³⁵Cl₃Na | m/z théorique **:** 1552.5332 |
| | | m/z mesurée : 1552.5430 |
| | [M+NH₄]⁺ C₈₃H₉₄NO₂₀³⁵Cl₃NH₄ | m/z théorique : 1547.5779 |
| | | m/z mesurée: 1547.5880 |

### ➢ Allyl (2-désoxy-4,6-O-isopropylidène-2-trichloroacétamido-β-D-glucopyranosyl)-(1→2)-[2,3,4,6-tétra-O-benzyl-α-D-glucopyranosyl-(1→3)]-(4-O-benzyl-α-L-rhamnopyranosyl)-(1→2)-3,4-di-O-benzyl-α-L-rhamnopyranoside 110 :

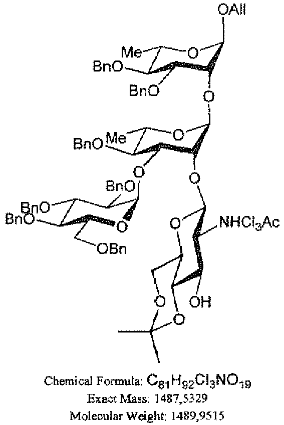

Le glycoside d'allyle **115** (1,9 g, 1,3 mmol) est dissous dans du MeOH (20 mL) puis du carbonate de potassium (175 mg, 1,3 mmol, 1 éq.) est ajouté au milieu réactionnel. Après 1 nuit sous agitation, le suivi par CCM (Tol/EtOAc, 7/3) indique la disparition de **115** (Rf= 0,8) et l'apparition d'un produit plus polaire (Rf= 0,45). Les solvants sont alors évaporés et l'huile jaune obtenue est purifiée par chromatographie sur gel de silice (Tol/EtOAc, 9/1→7/3) pour conduire à l'alcool **110** (1,7 g, 92%) sous forme d'un solide blanc.
Rf = 0,45 (Tol/EtOAc, 7/3).
RMN ¹H (CDCl₃), *δ* 7.43-7.08 (m, 36H, NH, CH_{Ph}), 5.88 (m, 1H, CH=), 5.29 (m, 1H, *J*ₜᵣₐₙₛ = 17.2 Hz, =CH₂), 5.23-5.20 (m, 2H, H-1_{E}, =CH₂), 5.14-5.05 (m, 4H, H_{Bn}, H-1_{A}, 2H_{Bn}), 4.95 (d, 1H, *J* = 10.8 Hz, H_{Bn}), 4.90 (d, 1H*, J* = 12.0 Hz, H_{Bn}), 4.83-4.77 (m, 3H, 2H_{Bn}, H-1_{B}), 4.70-4.61 (m, 5H, 2H_{Bn}, H-1_{D}, 2H_{Bn}), 4.58-4.52 (m, 2H, H_{Bn}), 4.37 (d, 1H, *J* = 12.0 Hz, H_{Bn}), 4.19-4.13 (m, 5H, H-3_{A}, H-2_{A}, H-3_{E}, H_{All}, H-5_{E}), 4.02 (dd, 1H, *J*_{2,3} = 2.8 Hz, H-2_{B}), 4.01-3.92 (m, 5H, H_{All}, H-3_{B}, H-2_{D}, H-4_{E}, H-2_{E}), 3.80 (dq, 1H, *J*_{4,5} = 9.2 Hz, H-5_{A}), 3.73 (dq, 1H, *J*_{4,5} = 9.4 Hz, H-5_{B}), 3.56 (dd, 1H, *J*_{5,6} = 5.3 Hz, *J*_{6a,6b} = 10.8 Hz, H-6a_{D}), 3.52-3.47 (m, 4H, H-6a_{E}, H-6b_{E}, H-4_{A}, H-4_{B}), 3.42-3.35 (m, 2H, H-4_{D}, H-6b_{D}), 2.85 (m, 1H, OH), 2.82 (m, 1H, H-5_{D}), 2.45 (pt, 1H, *J*_{3,4} = 9.7 Hz, H-3_{D}), 1.47 (s, 3H, H_{iPr}), 1.46 (s, 3H, H_{iPr}), 1.41 (d, 3H, *J*_{5,6}= 6.2 Hz, H-6_{A}), 1.35 (d, 3H, *J*_{5,6}= 6.2 Hz, H-6_{B}).
RMN ¹³C (CDCl₃), *δ* 163.7 (C_{NTCA}), 138.6-137.5 (C_{Ph}), 133.8 (CH=), 129.3-127.4 (CH_{Ph}), 117.2 (=CH₂), 101.0 (C-1_{D}, ¹*J*_{CH} = 159.1 Hz), 101.0 (C-1_{A}, ¹*J*_{CH}= 170.3 Hz), 99.7 (C_{iPr}), 97.9 (C-1_{B}, ¹*J*_{CH} = 168.6 Hz), 94.1 (C-1_{E}, ¹*J*_{CH} = 166.2 Hz), 92.7 (CCl₃), 83.3 (C-3_{E}), 80.5 (C-4_{B}), 79.8 (C-4_{A}), 79.7 (C-2_{E}), 79.5 (C-3_{B}), 78.7 (C-4_{E}), 76.3, 75.4, 75.3, 74.9 (5C, C_{Bn}), 74.7 (C-2_{B}), 74.1 (C-3_{A}), 74.0 (C-4_{D}), 73.5 (C_{Bn}), 73.3 (C-2_{A}), 73.0 (C-3_{D}), 72.1 (C_{Bn}), 70.0 (C-5_{E}), 68.7 (C-5_{A}), 68.0 (C-5_{B}), 67.9 (C-6_{E}), 67.7 (C_{All}), 67.1 (C-5_{D}), 61.4 (C-6_{D}), 58.8 (C-2_{D}), 29.1 (C_{iPr}), 19.0 (C_{iPr}), 18.0 (C-6_{B}), 17.8 (C-6_{A}).

| | | |
|---|---|---|
| HRMS (ESI⁺) : | [M+Na]⁺ C₈₁H₉₂NO₁₉³⁵Cl₃Na | m/z théorique : 1510.5227 |
| | | m/z mesurée : 1510.5243 |
| | [M+NH₄]⁺ C₈₁H₉₂NO₁₉³⁵Cl₃NH₄ | m/z théorique : 1505.5673 |
| | | m/z mesurée : 1505.5669 |

### Procédé 14ter :

### ➢ Allyl (3-O-acétyl-2-désoxy-4,6-O-isopropylidène-2-trichloroacétamido-β-D-glucopyranosyl)-(1→2)-[2,3,4,6-tétra-O-benzyl-α-D-glucopyranosyl-(1→3)]-(4-O-benzyl-α-L-rhamnopyranosyl)-(1→2)-3,4-di-O-benzyl-α-L-rhamnopyranoside 115' :

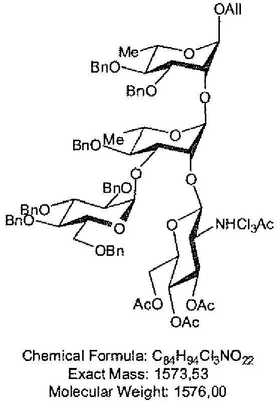

De l'acide triflique (4 µL, 45 µmol, 0,3 éq.) est additionné à une suspension d'accepteur **108** (163 mg, 143 µmol), de donneur **1** (110 mg, 185 µmol, 1,3 éq.) et de tamis moléculaire 4 Å (350 mg), dans du Tol (5 mL), agitée sous argon à -40°C. Après 1 h d'agitation à cette température complétée d'1 h 30 d'agitation à TA, temps au bout duquel du donneur **1** (20 mg, 34 µmol, 0,23 éq.) est ajouté, L'agitation est poursuivie une nuit à TA. Le suivi par CCM (Chex/EtOAc, 7/3) indique la présence d'un nouveau composé plus polaire que **108.** La réaction est stoppée par addition de triéthylamine puis le milieu réactionnel est filtré et concentré à l'évaporateur rotatif. Le brut réactionnel est purifié par flash chromatographie sur gel de silice (Chex/EtOAc, 100/0→70/30) pour obtenir le glycoside d'allyle **115'** (196 mg, 87%).
Rf = 0,33 (Chex/EtOAc, 7/3).
*RMN ¹H (CDCl₃), δ 7.54-7.09 (m, 36H, NH, CH_{Ph}), 5.91 (m, 1H, CH=), 5.30 (m, 1H, Jₜᵣₐₙₛ* = *17.2 Hz,* =*CH₂), 5.23 (m, 1H, J_{cis}* = *10.5 Hz,* =*CH₂), 5.23-5.19 (m, 2H, H-1_{E}, H_{Bn}), 5.15 (bs, 1H, H-1_{A}), 5.11 (d, 1H, J* = *10.9 Hz, H_{Bn}), 5.08 (d, 1H, J* = *11.0 Hz, H_{Bn}), 4.96 (d, 1H, J* = *10.8 Hz, H_{Bn}), 4.88-4.78 (m, 5H, HBₙ, H-1_{D}, H-3_{D}, 2H_{Bn}), 4.75-4.66 (m, 3H, H_{Bn}), 4.63-4.58 (m, 3H, H-1_{B}, 2H_{Bn}), 4.52 (d, 1H, J* = *11.0 Hz, H_{Bn}), 4.36 (d, 1H, J* = *12.0 Hz, H_{Bn}), 4.25-4.12 (m, 6H, H-2_{D}, H-3_{E}, H-2_{A}, H-3_{A}, H_{All}, H-5_{E}), 4.03 (dd, 1H, J_{2,3} = 2.8 Hz, H-2_{B}), 4.01-3.92 (m, 3H, H_{All}, H-2_{E}, H-3_{B}), 3.88-3.80 (m, 2H, H-4_{E}, H-5_{A}), 3.75 (dq, 1H, J_{4,5}* = *9.4 Hz, H-5_{B}), 3.68 (pt, 1H, J_{3,4} = 9.5 Hz, H-4_{D}), 3.57-3.40 (m, 6H, H-6a_{D}, H-4_{A}, H-4_{B}, H-6a_{E}, H-6b_{E}, H-6b_{D}), 2.82 (m, 1H, H-5_{D}), 2.11 (s, 3H, H_{Ac}), 1.49 (s, 3H, H_{iPr}), 1.44 (d, 3H, J_{5,6}* = *6.2 Hz, H-6_{A}), 1.41 (s, 3H, H_{iPr}), 1.36 (d, 3H, J_{5,6}* = *6.2 Hz, H-6_{B}).*
*RMN ¹³C (CDCl₃), δ 170.8 (C_{Ac}), 162.2 (C_{NTCA}), 138.6-137.6 (C_{Ph}), 133.9 (CH*=*), 129.1-127.4 (CH_{Ph}), 117.2 (=CH₂), 101.4 (C-1_{D}, ¹J_{CH} = 164.1 Hz), 101.0 (C-_{1A}, ¹J_{CH}* = *172.5 Hz), 99.7 (C_{iPr}), 97.9 (C-1_{B}, ¹J_{CH}* = *167.9 Hz), 94.6 (C-1_{E}, ¹J_{CH}* = *167.1 Hz), 92.8 (CCl₃), 83.5 (C-3_{E}), 80.5 (C-4_{B}), 79.8 (C-4_{A}), 79.6 (C-3_{B}), 78.6 (C-4_{E}), 78.2 (C-2_{E}), 76.2, 75.3, 75.2, 75.0 (4C, C_{Bn}), 74.9 (C-2_{B}), 74.4 (C-3_{A}), 74.0 (C_{Bn}), 73.9 (C-2_{A}), 73.5* (C_{Bn}), *72.6 (C-3_{D}), 72.2 (C_{Bn}), 71.3 (C-4_{D}), 70.0 (C-5_{E}), 68.7 (C-5_{A}), 68.0 (C-5_{B}), 67.9 (C-6_{E}), 67.7 (C_{All}), 67.4 (C-5_{D}), 61.8 (C-6_{D}), 56.6 (C-2_{D}), 29.0 (C_{iPr}), 20.9 (C_{Ac}), 19.1 (C_{iPr}), 18.0 (C-6_{B}), 17.9 (C-6_{A}).*

| | | |
|---|---|---|
| *HRMS (ESI⁺) :* | *[M+Na]⁺ C₈₄H₉₄NO₂₂³⁵Cl₃Na* | *m*/*z théorique : 1598.5234* |
| | | *m*/*z mesurée : 1598.5170* |

### ➢ Allyl (1-trichloroacétamido-β-D-glucopyranosy)-(1→2)-[2,3,4,6-tétra-O-benzyl-α-D-glucopyranosyl-(1→3)]-(4-O-benzyl-α-L-rhamnopyranosyl)-(1→2)-3,4-di-O-benzyl-α-L-rhamnopyranoside 115" :

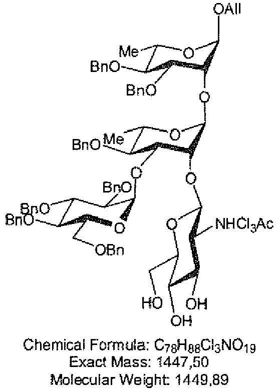

Une solution méthanolique de MeONa 0,5 M (1,4 mL, 0,75 mmol, 6 éq.) est additionnée à une suspension de tétrasaccharide **115'** (185 mg, 117 µmol) dans du méthanol (5 mL). Après 3 h d'agitation à TA, le suivi par CCM (CDCM/MeOH, 95/5) indique la présence d'un nouveau composé plus polaire. Le milieu réactionnel est neutralisé par addition de résine échangeuse d'ions Dowex X8 (H⁺) et filtré. Le filtrat est concentré à l'évaporateur rotatif, puis le brut réactionnel est purifié par flash chromatographie sur gel de silice (DCM/MeOH, 95/5) pour obtenir le triol **115"** (135 mg, 79%).
Rf = 0,58 (DCM/ MeOH, 9/1).
*RMN ¹H (CDCl₃), δ 7.54-7.09 (m, 36H, NH, CH_{Ph}), 5.91 (m, 1H, CH=), 5.30 (m, 1H, Jₜᵣₐₙₛ* = *17.2 Hz,* =*CH₂), 5.23 (m, 1H, J_{cis}* = *10.5 Hz,* =*CH₂), 5.23-5.19 (m, 2H, H-1_{E}, H_{Bn}), 5.15 (bs, 1H, H-1_{A}), 5.11 (d, 1H, J = 10.9 Hz, H_{Bn}), 5.08 (d, 1H, J* = *11.0 Hz, H_{Bn}), 4.96 (d, 1H, J = 10.8 Hz, H_{Bn}), 4.88-4.78 (m, 5H, H_{Bn}, H-1_{D}, H-3_{D}, 2H_{Bn}), 4.75-4.66 (m, 3H, H_{Bn}), 4.63-4.58 (m, 3H, H-1_{B}, 2H_{Bn}), 4.52 (d, 1H, J = 11.0 Hz, H_{Bn}), 4.36 (d, 1H, J = 12.0 Hz, H_{Bn}), 4.25-4.12 (m, 6H, H-2_{D}, H-3_{E}, H-2_{A}, H-3_{A}, H_{All}, H-5_{E}), 4.03 (dd, 1H, J_{2,3}* = *2.8 Hz, H-2_{B}), 4.01-3.92 (m, 3H, H_{All}, H-2_{E}, H-3_{B}), 3.88-3.80 (m, 2H, H-4_{E}, H-5_{A}), 3.75 (dq, 1H, J_{4,5}* = *9.4 Hz, H-5_{B}), 3.68 (pt, 1H, J_{3,4}* = *9.5 Hz, H-4_{D}), 3.57-3.40 (m, 6H, H-6a_{D}, H-4_{A}, H-4_{B}, H-6a_{E}, H-6b_{E}, H-6b_{D}), 2.82 (m, 1H, H-5_{D}), 2.11 (s, 3H, H_{Ac}), 1.49 (s, 3H, H_{iPr}), 1.44 (d, 3H, J_{5,6} = 6.2 Hz, H-6_{A}), 1.41 (s, 3H, H_{iPr}), 1.36 (d, 3H, J_{5,6}* = *6.2 Hz, H-6_{B}).*
*RMN ¹³C (CDCl₃), δ 170.8 (C_{AC}), 162.2 (C_{NTCA}), 138.6-137.6 (C_{Ph}), 133.9 (CH=), 129.1-127.4 (CH_{Ph}), 117.2 (=CH₂), 101.4 (C-1_{D}, ¹J_{CH}* = *164.1 Hz), 101.0 (C-1_{A}, ¹J_{CH} = 172.5 Hz), 99.7 (C_{iPr}), 97.9 (C-1_{B}, ¹J_{CH}* = *167.9 Hz), 94.6 (C-1_{E}, ¹J_{CH}* = *167.1 Hz), 92.8 (CCl₃), 83.5 (C-3_{E}), 80.5 (C-4_{B}), 79.8 (C-4_{A}), 79.6 (C-3_{B}), 78.6 (C-4_{E}), 78.2 (C-2_{E}), 76.2, 75.3, 75.2, 75.0 (4C, C_{Bn}), 74.9 (C-2_{B}), 74.4 (C-3_{A}), 74.0 (C_{Bn}), 73.9 (C-2_{A}), 73.5 (C_{Bn}), 72.6 (C-3_{D}), 72.2 (C_{Bn}), 71.3 (C-4_{D}), 70.0 (C-5_{E}), 68.7 (C-5_{A}), 68.0 (C-5_{B}), 67.9 (C-6_{E}), 67.7 (C_{All}), 67.4 (C-5_{D}), 61.8 (C-6_{D}), 56,6 (C-2_{D}), 29.0 (C_{iPr}), 20.9 (C_{Ac}), 19.1 (C_{iPr}), 18.0 (C-6_{B}), 17.9 (C-6_{A}).*

| | | |
|---|---|---|
| HRMS (ESI⁺) : | [M+Na]⁺ C₇₈H₈₈NO₁₉³⁵Cl₃Na | m/z théorique : 1472.4913 |
| | | m/z mesurée : 1772 .4921 |

### ➢ Propyl 2-acétamido-2-désoxy-β-D-glucopyranosyl-(1→2)-[α-D-glucopyranosyl-(1→3)]-α-L-rhamnopyranosyl-(1→2)-α-L-rhamnopyranoside VIII :

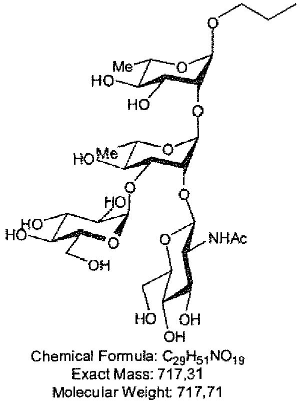

**Voie 1.** Du Pd/C10% (108 mg) est ajouté à une solution dégazée de triol **115"** (108 mg, 75 µmol) dans de l'éthanol (10 mL) complété d'acide chlorhydrique 1M (110 µL). La suspension est placée sous atmosphère d'hydrogène (1 bar) et agitée une nuit à TA. Le suivi par CCM (iPrOH/H₂O/NH₃, 7/1/02 montre la disparition de **115"** et l'apparition d'un nouveau composé plus polaire. Le milieu réactionnel est filtré sur Célite, et le filtrat est concentré à l'évaporateur rotatif. Le résidu est repris dans de l'éthanol (10 mL) auquel est ajouté Et₃N (100 µL). Le milieu est dégazé, du Pd/C 10% (100 mg) est ajouté et le milieu réactionnel est agité sous atmosphère d'hydrogène pendant une nuit. Le suivi par CCM indique de nouveau la présence d'un composé plus polaire. Après filtration de la suspension et évaporation du filtrat, le résidu obtenu est purifié par HPLC (colonne C-18 Kromasil, TFA aqueux 0,01M /CH₃CN, 100/0→60/40 en 20 min, 5 mL/min, 215 nm) pour conduire au tétrasaccharide cible ***VIII*** sous forme d'une poudre blanche après lyophilisation (36 mg, 68%).
**Voie 2.** Une solution d'acide triflique (2 µL, 23 µmol, 0,35 éq.) dans du toluène anhydre (98 µL) est additionnée à une suspension d'accepteur **108** (82 mg, 66 µmol), de donneur 1 (80 mg, 135 µmol, 2 éq.) et de tamis moléculaire 4 Å (200 mg), dans du Tol (2 mL), agitée sous argon à -30°C. Après 1 h d'agitation à cette température complétée d'1 h d'abitation à TA, temps au bout duquel du donneur **1** (10 mg, 17 µmol, 0,25 éq.) est ajouté au milieu réactionnel refroidi à -30°C. L'agitation est poursuivie 3 h alors que le bain revient lentement à TA. La réaction est stoppée par addition de triéthylamine puis le milieu réactionnel est filtré et concentré à l'évaporateur rotatif. Le brut réactionnel est purifié par flash chromatographie sur gel de silice (Chex/EtOAc, 100/0→70/30) pour obtenir **115'** (100 mg). Le glycoside d'allyle est solubilisé dans 1 mL de DCM et une solution 0,5 M de méthoxide de sodium méthanolique (500 µL) est ajoutée. Après 3 h d'agitation à TA, le suivi par CCM (CDCM/MeOH, 9/1) indique la présence d'un nouveau composé plus polaire révélé à la ninhydrine. Le milieu réactionnel est neutralisé par addition de résine échangeuse d'ions Dowex X8 (H⁺) et filtré. Le filtrat est concentré à l'évaporateur rotatif, puis le brut réactionnel est repris dans de l'éthanol (2 mL) et traité par de l'anhydride acétique (1 mL). Après 2 h à TA, les volatiles sont coévaporés au toluène et le brut réactionnel est chromatographié (DCM/MeOH, 95/5) pour obtenir l'acétamidotriol (54 mg, 67 %). Ce dernier est repris dans l'éthanol (5 mL) complété d'acide chlorhydrique 1M (110 µL) et la solution est dégazée. Du Pd/C10% (50 mg) est ajouté et la suspension est placée sous atmosphère d'hydrogène (1 bar) et agitée une nuit à TA. Après filtration, concentration, purification par HPLC et lyophilisation, le tétrasaccharide cible ***VIII*** est isolé (19 mg, 40% à partir de **108).**
*Rf* = *0,32 (iPrOH*/*H₂O*/*NH₃, 4*/*1*/*2).*
*RMN ¹H (D₂O), δ 5.06 (d, 1H, J_{1,2}* = *3.7 Hz, H-1_{E}), 4.83 (d, 1H, J_{1,2}* = *1.6 Hz, H-1_{A}), 4.76 (d, 1H, J_{1,2} = 1.3 Hz, H-1_{C}), 4.73 (d, 1H, J_{1,2}* = *8.6 Hz, H-1_{D}), 4.18 (m, 1H, H-2_{A}), 3.95 (m, 1H, H-5_{E}), 3.88 (m, 1H, H-5_{C}), 3.83-3.79 (m, 2H, H-3_{A}, H-6a_{D}), 3.74 (m, 1H, H-2_{D}), 3.72 (m, 1H, H-3_{E}), 3.70-3.67 (m, 3H, H-2_{C,} H-6a_{E,} H-6b_{E}), 3.65-3.59 (m, 4H, H-6b_{D}, H-3_{C}, H-5_{A}, H-2_{E}), 3.52 (m, 1H, H_{Pr}), 3.42-3.28 (m, 6H, H-4_{E}, H_{Pr}, H-4_{D}, H-5_{D}, H-3_{D}, H-4_{C}), 3.23 (pt, 1H, J_{3,4} = 9.7 Hz, H-4_{A}), 2.01 (s, 3H, H_{NAc}), 1.49 (sex, 2H, CH₂), 1.16 (d, 3H, J_{5,6}* = *6.2 Hz, H-6_{A}), 1.12 (d, 3H, J_{5,6} = 6.3 Hz, H-6_{C}), 0.80 (t, 3H, J = 7.4 Hz, CH₃).*
*RMN ¹³C (D₂O), δ 174. 5 (C_{NAc}), 101.8 (C-1_{D}, ¹J_{CH}* = *160.3 Hz), 101.6 (C-1_{C}, ¹J_{CH}* = *170.5 Hz), 99.0 (C-1_{A}, ¹J_{CH} = 172.7 Hz), 94.8 (C-1_{E}, ¹J_{CH}* = *169.8 Hz), 81.7 (C-3_{D}), 76.4 (C-4_{D}), 74.7 (C-2_{A}), 74.1 (C-3_{A}), 73.5 (C-3_{E}), 72.2 (C-4_{C}), 71.7 (C-5_{E}), 71.6 (C-2_{E}), 71.2 (C-2_{C}), 71.0 (C-4_{A}), 70.5 (C-3_{C}), 70.0 (C_{Pr}), 69.6 (C-5_{D}), 69.3 (C-5_{C}), 69.1 (C-5_{A}), 68.6 (C-4_{E}), 60.9 (C-6_{D}), 60.6 (C-6_{E}), 55.9 (C-2_{D}), 22.8 (C_{NAc}), 22.3 (CH₂), 17.0, 16.7 (C-6_{A}, C-6_{C}), 10.2 (CH₃).* MS (ESI⁺) pour C₂₉H₅₁NO₁₉: m/z théorique 717.32, trouvée 718.3 [M+Na]⁺

### Procédé 15 :

### ➢ Allyl (2-acétamido-2-désoxy-β-D-glucopyranosyl)-(1→2)-[2,3,4,6-tétra-O-benzyl-α-D-glucopyranosyl-(1→3)]-4-O-benzyl-α-L-rhamnopyranoside 117 :

### ➢ Allyl (2-désoxy-2-méthylcarbamate-β-D-glucopyranosyl)-(1→2)-[2,3,4,6-tétra-O-benzyl-α-D-glucopyranosyl-(1→3)]-4-O-benzyl-α-L-rhamnopyranoside 118 :

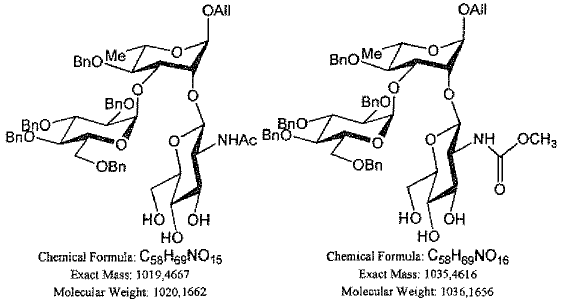

Après addition de NaOMe 0,5 M (4,8 mL, 2,4 mmol, 6 éq.) au tri-*O*-acétyle **22** (500 mg, 400 µmol) en solution dans du DCM (38 mL), le milieu réactionnel est agité pendant 9 h et suivi par CCM (DCM/MeOH, 92/8 et EtOAc/MeOH, 98/2). Après avoir observé la disparition de **22** et l'apparition d'un produit plus polaire révélé par la ninhydrine (Rf = 0,35 et 0,2, respectivement), le milieu réactionnel est dilué par addition de MeOH (77 mL) puis de l'anhydride acétique (300 µL, 3,2 mmol, 8 éq.) est additionné au milieu réactionnel. Après agitation pendant 2 h, on observe l'apparition d'un nouveau produit en CCM (Rf = 0,4 et 0,25, respectivement). Les solvants sont alors évaporés et le solide obtenu est purifié par chromatographie sur gel de silice (DCM/MeOH, 98/2→95/5) pour conduire par ordre d'élution au carbamate non désiré **118** sous forme d'un solide blanc (20 mg, 5%) puis au triol **117** sous forme d'un solide blanc (371 mg, 90%).
**117 :** Rf = 0,4 (DCM/MeOH, 92/8).
RMN ¹H (CDCl₃), *δ* 7.41-7.08 (m, 26H, NH, CH_{Ph}), 5.88 (m, 1H, CH=), 5.29 (m, 1H, *J*ₜᵣₐₙₛ = 17.2 Hz, =CH₂), 5.21 (m, 1H, *J*_{cis} = 10.4 Hz, =CH₂), 5.09-5.06 (m, 2H, H-1_{E}, H_{Bn}), 5.01-4.96 (m, 2H, H-1_{A}, H_{Bn}), 4.91-4.87 (m, 2H, H_{Bn}), 4.81-4.76 (m, 2H, *J =* 10.3 Hz, H_{Bn}), 4.63-4.58 (m, 2H, H_{Bn}), 4.50 (d, 1H, *J=* 10.9 Hz, H_{Bn}), 4.62 (m, 1H, H-1_{D}), 4.34 (m, 1H, H_{Bn}), 4.19-4.08 (m, 4H, H-3_{A}, H-5_{E}, H_{All,} H-3_{E}), 4.01 (m, 1H, H_{All}), 3.91 (bs, 1H, H-2_{A}), 3.87-3.81 (m, 5H, H-2_{E}, H-4_{E}, H-2_{D}, H-6a_{D}, H-6b_{D}), 3.72 (m, 1H, H-5_{A}), 3.51 (pt, 1H, *J*_{3,4} = 9.0 Hz, H-4_{D}), 3.51-3.45 (m, 3H, H-4_{A}, H-6a_{E}, H-6b_{E}), 3.13 (m, 1H, H-5_{D}), 2.80 (pt, 1H, *J*_{2,3} = 9.2 Hz, H-3_{D}), 2.21 (s, 3H, H_{NAc}), 1.40 (d, 3H, *J*_{5,6} = 6.2 Hz, H-6_{A}).
RMN ¹³C (CDCl₃), *δ* 173.3 (C_{NAc}), 138.6-137.5 (C_{Ph}), 134.2 (CH=), 129.5-127.8 (CH_{Ph}), 117.8 (=CH₂), 103.0 (C-1_{D}, ¹*J*_{CH} = 155.6 Hz), 98.5 (C-1_{A}, ¹*J*_{CH} = 174.8 Hz), 94.6 (C-1_{E}, ¹*J*_{CH} = 167.9 Hz), 83.7 (C-3_{E}), 80.2 (C-4_{A}), 79.2, 79.0 (2C, C-2_{E}, C-4_{E}), 77.1 (C-3_{D}), 76.9 (C-2_{A}), 76.5, 75.8 (2C, C_{Bn}), 75.7 (C-5_{D}), 75.6, 75.3 (2C, C_{Bn}), 74.6 (C-3_{A}), 73.8 (C_{Bn}), 71.8 (C-4_{D}), 70.3 (C-5_{E}), 68.7 (C-5_{A}), 68.3 (C_{All}), 68.2 (C-6_{E}), 62.7 (C-6_{D}), 57.4 (C-2_{D}), 23.6 (C_{NAc}), 18.2 (C-6_{A}).

| | | |
|---|---|---|
| HRMS (ESI⁺) : | [M+H]⁺ C₅₈H₇₀NO₁₅ | m/z théorique : 1020.4745 |
| | | m/z mesurée : 1020.4739 |
| | [M+Na]⁺ C₅₈H₆₉NO₁₅Na | m/z théorique : 1042.4565 |
| | | m/z mesurée: 1042.4545 |

**118 :** Rf = 0,45 (DCM/MeOH, 92/8).
RMN ¹H (CDCl₃), *δ* 7.42-7.08 (m, 25H, CH_{Ph}), 6.52 (d, 1H, *J*_{NH,2} = 7.0, NH), 5.90 (m, 1H, CH=), 5.29 (m, 1H, *J*ₜᵣₐₙₛ = 17.2 Hz, =CH₂), 5.20 (m, 1H, *J*_{cis} = 10.4 Hz, =CH₂), 5.11-4.96 (m, 5H, 2H_{Bn}, H-1_{E}, 2H_{Bn}), 4.92 (bs, 1H, H-1_{A}), 4.84-4.79 (m, 2H, H_{Bn}), 4.73 (d, 1H, *J=* 10.4 Hz, H_{Bn}), 4.63-4.55 (m, 2H, H_{Bn}), 4.45-4.42 (m, 2H, H-1_{D}, H_{Bn}), 4.29 (m, 1H, H-3_{E}), 4.15 (m, 1H, H_{All}), 4.12-4.09 (m, 2H, H-3_{A}, H-5_{E}), 3.98 (m, 1H, H_{All}), 4.87 (m, 1H, H-2_{A}), 3.87-3.70 (m, 8H, H-4_{E}, OMe, H-6a_{D}, H-6b_{D}, H-2_{E}, H-5_{A}), 3.63 (m, 1H, H-2_{D}), 3.51 (m, 2H, H-4_{A}, H-4_{D}), 3.33 (m, 2H, H-6a_{E}, H-6b_{E}), 2.90 (m, 1H, H-5_{D}), 2.61 (pt, 1H, *J*_{2,3} = 9.8 Hz, H-3_{D}), 1.40 (d, 3H, *J*_{5,6} = 6.2 Hz, H-6_{A}).
RMN ¹³C (CDCl₃), *δ* 159.4 (C_{NOMe}), 139.7-137.8 (C_{Ph}), 134.2 (CH=), 129.4-127.9 (CH_{Ph}), 117.9 (=CH₂), 103.2 (C-1_{D}, ¹*J*_{CH} = 161.6 Hz), 98.6 (C-1_{A}, ¹*J*_{CH} = 171.5 Hz), 95.8 (C-1_{E}, ¹*J*_{CH} = 170.5 Hz), 83.1 (C-3_{E}), 80.1 (C-2_{E}), 79.9 (C-4_{A}), 78.7 (C-4_{E}), 78.1 (C-2_{A}), 77.1 (C-3_{D}), 76.1 (C_{Bn}), 76.0 (C-3_{A}), 75.7, 75.6, 75.4 (3C, C_{Bn}), 75.2 (C-5_{D}), 73.8 (C_{Bn}), 71.5 (C-4_{D}), 70.6 (C-5_{E}), 69.0 (C-5_{A}), 68.3 (C_{All}), 68.1 (C-6_{E}), 62.7 (C-6_{D}), 58.4 (C-2_{D}), 52.9 (OMe), 18.1 (C-6_{A}).

| | | |
|---|---|---|
| HRMS (ESI⁺) : | [M+Na]⁺ C₅₈H₆₉NO₁₆Na | m/z théorique : 1058.4514 |
| | | m/z mesurée : 1058.4514 |
| | [M+NH₄]⁺ C₅₈H₆₉NO₁₆NH₄ | m/z théorique : 1053.4960 |
| | | m/z mesurée : 1053.4946 |

### ➢ Allyl (2-acétamido-2-désoxy-4,6-O-isopropylidène-β-D-glucopyranosyl)-(1→2)-[2,3,4,6-tétra-O-benzyl-α-D-glucopyranosyl-(1→3)]-4-O-benzyl-α-L-rhamnopyranoside 119 :

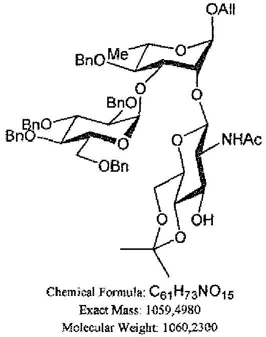

**Voie 1** : Dans du DMF (3 mL) est dissous le triol **117** (270 mg, 265 µmol) et le 2-méthoxypropène (76 µL, 795 µmol, 3 éq.). Du CSA (6,2 mg, 26 µmol, 0,1 éq.) est ajouté par fractions au milieu réactionnel. Le milieu réactionnel est alors agité à TA et la réaction est suivie par CCM (Chex/EtOAc, 1/1 et DCM/MeOH, 92/8). Après une nuit d'agitation, la transformation du produit de départ (Rf = 0,05 et 0,4, respectivement) en un produit moins polaire (Rf = 0,35 et 0,75, respectivement) est presque totale. Le milieu réactionnel est neutralisé avec de la triéthylamine (100 µL) puis concentré à l'évaporateur rotatif. Le solide obtenu est purifié par chromatographie sur gel de silice (Chex/EtOAc, 1/1) pour conduire à l'alcool **119** sous forme d'un solide blanc (240 mg, 85%).
**Voie 2 :** Du TMSOTf (92,3 µL, 514 µmol, 0,2 éq.) est additionné à une solution d'accepteur **21** (2,1 g, 2,6 mmol) et de donneur 1 (1,8 g, 3,1 mmol, 1,2 éq.) dans du DCM (50 mL), en présence de tamis moléculaire 4 Å (2,0 g), agitée sous argon à - 78°C. Après 3 h sous agitation, le bain éthanol-carboglace est retiré et l'agitation est poursuivie pendant 30 min, temps au bout duquel le suivi par CCM (Chex/EtOAc, 7/3) indique la disparition de **21** (Rf = 0,5) et l'apparition d'un nouveau composé plus polaire (Rf= 0,2). La réaction est stoppée par l'addition de triéthylamine (0,2 mL) puis le milieu réactionnel est filtré puis concentré à l'évaporateur rotatif. L'huile jaune obtenue est purifiée par chromatographie sur gel de silice (Chex/EtOAc, 8/2→7/3) pour obtenir à un solide blanc (3,5 g).
   Après addition de NaOMe 0,5 M (33,6 mL, 16,8 mmol, 6 éq.) au brut précédent (3,5 g, 2,8 mmol) en solution dans du DCM (270 mL), le milieu réactionnel est agité pendant 9 h et suivi par CCM (DCM/MeOH, 92/8 et EtOAc/MeOH, 98/2). Après avoir observé la disparition du produit de départ et l'apparition d'un produit plus polaire révélé par la ninhydrine (Rf = 0,35 et 0,2, respectivement), le milieu réactionnel est dilué par addition de MeOH (540 mL) puis de l'anhydride acétique (2,1 mL, 22,4 mmol, 8 éq.) est additionné au milieu réactionnel. Après agitation pendant 2 h, on observe l'apparition d'un nouveau produit en CCM (Rf = 0,4 et 0,25, respectivement). Les solvants sont alors évaporés et le solide obtenu est purifié par chromatographie sur gel de silice (DCM/MeOH, 98/2→95/5) pour conduire à un solide blanc (2,7 g).
   Dans du DMF (17 mL) est dissous le brut précédent (2,7 g, 2,6 mmol) et le 2-méthoxypropène (758 µL, 7,9 mmol, 3 éq.). Du CSA (61,2 mg, 263 µmol, 0,1 éq.) est ajouté par fractions au milieu réactionnel. Le milieu réactionnel est alors agité à TA et la réaction est suivie par CCM (Chex/EtOAc, 1/1 et DCM/MeOH, 92/8). Après 6 h d'agitation, la transformation du produit de départ (Rf = 0,05 et 0,4, respectivement) en un produit moins polaire (Rf = 0,35 et 0,75, respectivement) est presque totale. Le milieu réactionnel est neutralisé avec de la triéthylamine (0,1 mL) puis concentré à l'évaporateur rotatif. Le solide obtenu est purifié par chromatographie sur gel de silice (Chex/EtOAc, 1/1) pour conduire à l'alcool **119** sous forme d'un solide blanc (2,4 g, 87%).
**Voie 3 :** Après addition de NaOMe 0,5 M (3,7 mL, 1,9 mmol, 6 éq.) au glycoside d'allyle **23** (375 mg, 310 µmol) en solution dans du DCM (30 mL), le milieu réactionnel est agité pendant 9 h et suivi par CCM (Chex/EtOAc, 1/1 et Chex/EtOAc, 6/4). Après avoir observé la disparition de **23** et l'apparition d'un produit plus polaire (Rf = 0,2 et 0,15, respectivement), le milieu réactionnel est dilué par addition de MeOH (60 mL) puis de l'anhydride acétique (234 µL, 2,5 mmol, 8 éq.) est additionné au milieu réactionnel. Après agitation pendant 2 h, on observe l'apparition d'un nouveau produit en CCM (Rf = 0,3 dans Chex/EtOAc, 1/1). Les solvants sont alors évaporés et le solide obtenu est purifié par chromatographie sur gel de silice (Chex/EtOAc, 1/1) pour conduire à l'alcool **119** sous forme d'un solide blanc (258 mg, 90%).
**Voie 4 :** Du TMSOTf (16,0 µL, 92 µmol, 0,3 éq.) est additionné à une solution d'accepteur **21** (250 mg, 310 µmol) et de donneur **2** (220 mg, 400 µmol, 1,3 éq.) dans du DCM (8 mL), en présence de tamis moléculaire 4 Å (250 mg), agitée sous argon à -78°C. Après 6 h sous agitation, le bain éthanol-carboglace est retiré et l'agitation est poursuivie pendant 30 min, temps au bout duquel le suivi par CCM (Chex/EtOAc, 7/3) indique la disparition presque totale de l'accepteur (Rf = 0,5) et l'apparition d'un nouveau composé plus polaire (Rf = 0,35). La réaction est stoppée par l'addition de triéthylamine (0,1 mL) puis le milieu réactionnel est filtré puis concentré à l'évaporateur rotatif. L'huile jaune obtenue est purifiée par chromatographie sur gel de silice (Chex/EtOAc, 8/2→7/3) pour obtenir un solide blanc (375 mg).

Après addition de NaOMe 0,5 M (3,7 mL, 1,9 mmol, 6 éq.) au brut précédent (375 mg, 310 µmol) en solution dans du DCM (30 mL), le milieu réactionnel est agité pendant 9 h et suivi par CCM (Chex/EtOAc, 1/1 et Chex/EtOAc, 6/4). Après avoir observé la disparition du produit de départ et l'apparition d'un produit plus polaire (Rf = 0,2 et 0,15, respectivement), le milieu réactionnel est dilué par addition de MeOH (60 mL) puis de l'anhydride acétique (234 µL, 2,5 mmol, 8 éq.) est additionné au milieu réactionnel. Après agitation pendant 2 h, on observe l'apparition d'un nouveau produit en CCM (Rf = 0,3 dans Chex/EtOAc, 1/1). Les solvants sont alors évaporés et le solide obtenu est purifié par chromatographie sur gel de silice (Chex/EtOAc, 1/1) pour conduire à l'alcool **119** sous forme d'un solide blanc (258 mg, 80%).
Rf = 0,35 (Chex/EtOAc, 1/1).
RMN ¹H (CDCl₃), *δ* 7.31-6.96 (m, 26H, NH, CH_{Ph}), 5.88 (m, 1H, CH=), 5.29 (m, 1H, *J*ₜᵣₐₙₛ = 17.3 Hz, =CH₂), 5.21 (m, 1H, *J*_{cis} = 10.4 Hz, =CH₂), 5.09-5.06 (m, 2H, *J*_{1,2} = 5.6 Hz, H-1_{E}, H_{Bn}), 4.98 (m, 1H, *J=* 11.9 Hz, H_{Bn}), 4.86 (m, 2H, H_{Bn}), 4.84 (bs, 1H, *J*_{1,2} = 1.7 Hz, H-1_{A}), 4.80-4.74 (m, 2H, H_{Bn}), 4.63-4.57 (m, 2H, J= 11.9 Hz, H_{Bn}), 4.50 (m, 1H, *J=* 9.6 Hz, H_{Bn}), 4.37-4.30 (m, 2H, *J*_{1,2} = 8.3 Hz, H_{Bn}, H-1_{D}), 4.19-4.07 (m, 4H, H-3_{A}, H-5_{E}, H_{All}, H-3_{E}), 3.96 (m, 1H, H_{All}), 3.89-3.70 (m, 7H, H-2_{A}, H-6a_{D}, H-2_{E}, H-4_{E}, H-2_{D}, H-5_{A}, H-6b_{D}), 3.53 (pt, 1H, *J*_{3,4} = 9.3 Hz, H-4_{D}), 3.51 (m, 2H, H-6a_{E}, H-6b_{E}), 3.45 (pt, 1H, *J*_{3,4} = 9.6Hz, H-4_{A}), 2.87 (m, 1H, H-5_{D}), 2.81 (pt, 1H, *J*_{2,3} = 9.2 Hz, H-3_{D}), 2.20 (s, 3H, H_{NAc}), 1.52 (s, 3H, H_{iPr}), 1.48 (s, 3H, H_{iPr}), 1.39 (d, 3H, *J_{5,6}* = 6.2 Hz, H-6_{A}).
RMN ¹³C (CDCl₃), *δ* 173.3 (C_{NAc}), 138.5-137.5 (C_{Ph}), 134.2 (CH=), 129.5-127.7(CH_{Ph}), 117.7 (=CH₂), 102.8 (C-1_{D}, ¹*J*_{CH} = 155.2 Hz), 100.1 (C_{iPr}), 98.6 (C-1_{A}, ¹*J*_{CH} = 172.4 Hz), 94.6 (C-1_{E}, ¹*J*_{CH} = 169.1 Hz), 83.7 (C-3_{E}), 80.2 (C-4_{A}), 79.9 (C-2_{E}), 79.1 (C-4_{E}), 76.6 (C-2_{A}), 76.5, 76.1, 75.8, 75.4 (4C, C_{Bn}), 74.8 (C-3_{A}), 74.6 (C-3_{D}), 74.5 (C-4_{D}), 73.8 (C_{Bn}), 70.4 (C-5_{E}), 68.8 (C-5_{A}), 68.3 (C_{All}), 68.2 (C-6_{E}), 67.3 (C-5_{D}), 62.3 (C-6_{D}), 58.9 (C-2_{D}), 29.5 (C_{iPr}), 23.6 (C_{NAc}), 19.3 (C_{iPr}), 18.2 (C-6_{A}).

| | | |
|---|---|---|
| HRMS (ESI⁺) : | [M+H]⁺ C₆₁H₇₄NO₁₅ | m/z théorique : 1060.5058 |
| | | m/z mesurée : 1060.5066 |
| | [M+Na]⁺ C₆₁H₇₃NO₁₅Na | m/z théorique : 1082.4878 |
| | | m/z mesurée : 1082.4875 |

### ➢ Allyl (2-désoxy-4,6-O-isopropylidène-2-acétamido-β-D-glucopyranosyl)-(1→2)-[2,3,4,6-tétra-O-benzyl-α-D-glucopyranosyl-(1→3)]-(4-O-benzyl-α-L-rhamnopyranosyl)-(1→2)-3,4-di-O-benzyl-α-L-rhamnopyranoside 116 :

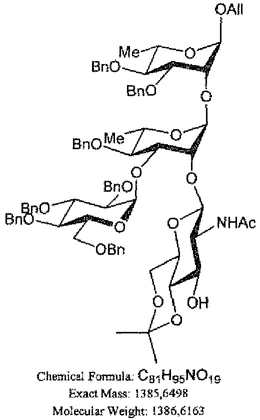

Après addition de NaOMe 0,5 M (5,7 mL, 2,8 mmol, 6,9 éq.) au glycoside d'allyle **115** (630 mg, 412 µmol) en solution dans du DCM (45 mL), le milieu réactionnel est agité pendant 6h30 et suivi par CCM (DCM/MeOH, 98/2 et Tol/EtOAc, 85/15). Après avoir observé la disparition de **115** et l'apparition d'un produit plus polaire révélé par la ninhydrine (Rf = 0,45 et 0, respectivement), le milieu réactionnel est dilué par addition de MeOH (91 mL) puis de l'anhydride acétique (386 µL, 4,1 mmol, 10 éq.) est additionné au milieu réactionnel. Après agitation pendant 2 h, on observe l'apparition d'un nouveau produit en CCM (Rf = 0,5 et 0,1, respectivement). Les solvants sont alors évaporés et le solide obtenu est purifié par chromatographie sur gel de silice (Tol/ EtOAc, 6/4→4/6) pour conduire à l'alcool **116** sous forme d'un solide blanc (470 mg, 83%).
Rf = 0,35 (Tol/ EtOAc, 6/4).
RMN ¹H (CDCl₃), *δ* 7.43-7.07 (m, 36H, NH, CH_{Ph}), 5.89 (m, 1H, CH=), 5.27 (m, 1H, *J*ₜᵣₐₙₛ = 17.2 Hz, =CH₂), 5.20 (m, 1H, *J*_{cis} = 11.7 Hz, =CH₂), 5.18 (d, 1H, *J*_{1,2} = 3.7 Hz, H-1_{E}), 5.11-5.09 (m, 2H, H-1_{A}, H_{Bn}), 5.02-4.88 (m, 4H, H_{Bn}), 4.83-4.79 (m, 2H, H_{Bn}), 4.77 (d, 1H, *J*_{1,2} = 1.6 Hz, H-1_{B}), 4.72-4.53 (m, 6H, H_{Bn}), 4.10-4.35 (m, 2H, H_{Bn}, H-1_{D}), 4.19-4.11 (m, 4H, H-3_{A}, H-5_{E}, H_{All}, H-3_{E}), 4.06 (dd, 1H, *J*_{2,3} = 2.2 Hz, H-2_{A}), 4.02 (dd, 1H, *J*_{2,3} = 2.9 Hz, H-2_{B}), 3.94-3.79 (m, 6H, H_{All}, H-4_{E}, H-3_{B}, H-2_{E}, H-2_{D}, H-5_{A}), 3.73 (dq, 1H, *J*_{4,5} = 9.4 Hz, H-5_{B}), 3.59-3.46 (m, 6H, H-6a_{D}, H-6b_{D}, H-6a_{E}, H-6b_{E}, H-4_{D}, H-4_{A}, H-4_{B}), 2.83 (m, 1H, H-5_{D}), 2.78 (pt, 1H, *J*_{3,4} = 9.2 Hz, H-3_{D}), 1.50 (s, 3H, H_{iPr}), 1.48 (s, 3H, H_{iPr}), 1.40 (d, 3H, *J*_{5,6} = 6.2 Hz, H-6_{A}), 1.35 (d, 3H, *J*_{5,6} = 6.2 Hz, H-6_{B}).
RMN ¹³C (CDCl₃), *δ*172.9 (C_{NAc}), 138.5-137.1 (C_{Ph}), 133.8 (CH=), 129.2-127.4 (CH_{Ph}), 117.2 (=CH₂), 102.4 (C-1_{D}, ¹*J*_{CH} = 162.8 Hz), 1010 (C-1_{A}, ¹*J*_{CH} = 177.5 Hz), 99.6 (C_{iPr}), 97.8 (C-1_{B}, ¹*J*_{CH} = 169.0 Hz), 94.1 CC-1_{E}, ¹*J*_{CH} = 167.0 Hz), 83.3 (C-3_{E}), 80.5 (C-4_{B}), 80.0 (C-4_{A}), 79.4 (C-2_{E}), 79.2 (C-3_{B}), 78.7 (C-4_{E}), 76.3 (C_{Bn}), 75.9 (C-2_{A}), 75.7 (C_{Bn}), 75.5 (C-2_{B}), 75.4 75.3, 75.0 (3C, C_{Bn}), 74.2 (C-3_{A}), 74.1 (C-4_{D}), 74.0 (C-3_{D}), 73.5, 72.0 (2C, C_{Bn}), 70.1 (C-5_{E}), 68.8 (C-5_{A}), 67.9 (2C, C-5_{B}, C-6_{E}), 67.7 (C_{All}), 66.9 (C-5_{D}), 61.7 (C-6_{D}), 58.5 (C-2_{D}), 29.1 (C_{iPr}), 23.2 (C_{NAc}), 19.0 (C_{iPr}), 18.1 (C-6_{B}), 17.7 (C-6_{A}).

| | | |
|---|---|---|
| HRMS (ESI⁺) : | [M+H]⁺ C₈₁H₉₅NO₁₉ | m/z théorique : 1386.6577 |
| | | m/z mesurée : 1386.6625 |
| | [M+Na]⁺ C₈₁H₉₅NO₁₉Na | m/z théorique : 1408.6396 |
| | | m/z mesurée : 1408.6478 |

### ➢ Allyl (2-O-acétyl-3,4-di-O-benzyl-α-L-rhamnopyranosyl)-(1→3)-(2-acétamido-2-désoxy-4,6-O-isopropylidène-β-D-glucopyranosyl)-(1→2)-[2,3,4,6-tétra-O-benzyl-α-D-glucopyranosyl-(1→3)]-(4-O-benzyl-α-L-rhamnopyranosyl)-(1→2)-3,4-di-O-benzyl-α-L-rhamnopyranoside116' :

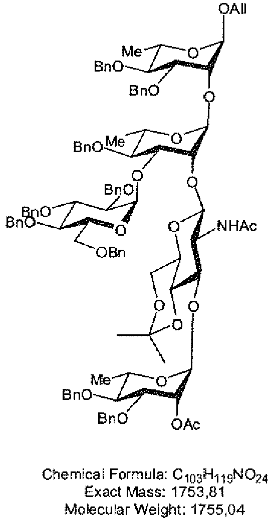

De l'acide triflique (2 µL, 23 µmol, 0,2 éq.) est additionné à une solution d'accepteur 116 (128 mg, 94 µmol), de donneur 39 (97 mg, 188 µmol, 2 éq.) et de tamis moléculaire 4 Å (200 mg), dans du toluène (1,5 mL) agitée sous argon à -40°C. Après 1 h d'agitation à cette température suivie de 2 h d'agitation à TA, le suivi par CCM (DCM/MeOH, 97,5/2,5) indique la présence d'un nouveau composé moins polaire alors que **116** subsiste au mieux à l'état de traces. La réaction est stoppée par addition de triéthylamine (0,2 mL) puis le milieu réactionnel est filtré et concentré à l'évaporateur rotatif. Le brut réactionnel est purifiée par flash chromatographie sur gel de silice (Chex/EtOAc, 100/0→50/50) pour obtenir le glycoside d'allyle **116'** sous forme d'un solide blanc (116 mg, 71%) .
Rf = 0,58 (DCM/MeOH, 98.5/2,5).
RMN ¹H (CDCl₃), δ 7.44-7.06 (m, 50H, CH_{Ph}), 6.38 (d, 1H, NH), 5.85 (m, 1H, CH=), 5.18 (m, 1H, H-1_{E}), 5.04 (s, 1H, H-1_{A}), 5.13 (s, 1H, H-2_{C}), 5.32-4.97 (m, 15H, H_{All}, H_{Bn}), 4.72 (s, 1H, H-1_{B}), 4.63 (s, 1H, H-1_{C}), 4.33 (d, 1H, J = 12.0 Hz, H_{Bn}), 4.27 (d, 1H, J_{1,2} = 8.6 Hz, H-1_{D}), 4.21-4.08 (m, 5H, H-3_{E}, H-5_{E}, H-2_{D}, H-3_{A}, H_{All}), 3.98-3.87 (m, 8H, H-2_{A}, H-3_{C}, H-5_{C}, H-2_{B}, H_{All}, H-3_{B}, H-2_{E}, H-4_{E}), 3.75 (dq, 1H, J_{4,5} = 9.3 Hz, J_{5,6} = 6.6 Hz, H-5_{A}), 3.69 (dq, 1H, J_{4,5} = 9.4 Hz, J_{5,6} = 6.1 Hz, H-5_{B}), 3.52-3.41 (m, 8H, H-6a_{D}, H-6b_{D}, H-6a_{E}, H-6b_{E}, H-4_{A}, H-4_{B}, H-4_{D}, H-4_{C},), 2.74 (m, 1H, *J*_{5,6} = 5.3 Hz, *J*_{4,5} = 9.8 Hz, H-5_{D}), 2.62 (pt, 1H, *J*_{3,4} = *J*_{4,5} *=* 9.4 Hz, H-3_{D}), 2.28 (s, 3H, H_{Ac}), 2.08 (s, 3H, H_{NAc}), 2.05 (s, 3H, H_{Ac}), 1.39 (s, 6H, H_{iPr}), 1.33-1.30 (m, 6H, H-6_{A}, H-6_{B}), 1.24 (d, 3H, J_{5,6} = 6.3 Hz, H-6_{C}).
RMN ¹³C (CDCl₃), δ 170.9 (C_{Ac}), 170.5 (C_{NAc}), 139.2-18.7 (C_{Ph}), 134.2 (CH=), 129.7-127.8 (CH_{Ph}), 117.6 (=CH₂), 103.8 (C-1_{D}), 101.5 (C-1_{A}), 99.6 (C_{iPr}), 98.8 (C-1_{B}), 98.2 (C-1_{C}), 94.4 (C-1_{E}), 83.7 (C-3_{E}), 80.8-78.9 (7C, C-3_{D}, C-4_{B}, C-2_{E}, C-4_{C}, C-4_{A}, C-3_{B}, C-4_{E}), 78.1 (C-3_{C}), 76.6 (C_{Bn}), 76.4 (C-2_{A}), 75.6 (C-2_{B}), 76.0-75.4 (7C, C_{Bn}), 74.6 (C-3_{A}), 73.9 (C_{Bn}), 72.4 (C-4_{D}), 72.3, 71.8 (2C, C_{Bn}), 70.3 (2C, C-2_{C}, C-5_{E}), 69.2 (C-5_{A}), 68.2 (C-5_{B}), 68.0 (2C, C-6_{E}, C_{All}), 67.8 (C-5_{C}), 67.5 (C-5_{D}), 62.3 (C-6_{D}), 55.7 (C-2 _{D}), 29.5 (C_{iPr}), 24.4 (C_{NAc}), 21.5 (C_{Ac}), 19.5 (C_{iPr}), 18.4, 87.3, 18.0 (C-6_{A}, C-6_{B}, C-6c).

| | | |
|---|---|---|
| HRMS (ESI⁺) : | [M+H]⁺ C₁₀₃H₁₁₉NO₂₄ | m/z théorique : 1755.8234 |
| | | m/z mesurée: 1755.8301 |

### ➢ Propyl(2-O-acétyl-α-L-rhamnopyranosyl)-(1→3)-(2-acétamido-2-désoxy-β-D-glucopyranosyl)-(1→2)-[α-D-glucopyranosyl-α-L-rhamoopyranosyl-(1→2)-α-L-rhamnopyranoside IX :

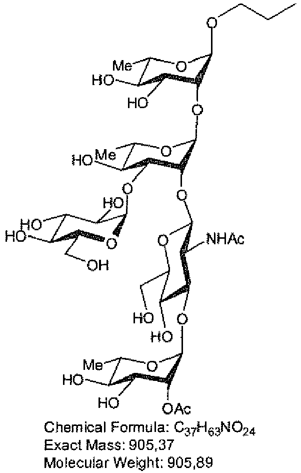

Une solution aqueuse de TFA 50% (2 mL) est additionnée lentement à une solution du glycoside d'allyle **116'** (105 g, 84 µmol) dans du DCM (2 mL) à 0°C. Le milieu réactionnel est agité pendant 1 h à cette température, puis 15 min à TA. Le suivi par CCM (DCM/MeOH, 95/5) indique la disparition totale de **116'** et l'apparition d'un nouveau composé plus polaire **116".** Le milieu réactionnel est concentré par coévaporation avec du toluène (3 x 10 mL). Le brut réactionnel (103 mg) est suffisamment correct d'après les analyses RMN pour être engagé tel que dans l'étape de débenzylation.

Du Pd/C10% (100 mg) est ajouté à une solution dégazée de diol **116"** (102 mg, 60 µmol) dans de l'éthanol (4 mL). La suspension est placée sous atmosphère d'hydrogène (1 bar) et agitée à TA pendant 6 h. Le suivi par CCM (iPrOH/H₂O/NH₃, 7/1/2 montre la disparition de **116"** et la présence d'un composé plus polaire. Le milieu réactionnel est filtré sur Célite, et le filtrat est concentré à l'évaporateur rotatif. Après filtration de la suspension et évaporation du filtrat, le résidu obtenu est purifié par HPLC (colonne C-18 Kromasil, TFA aqueux 0,01M /CH₃CN, 100/0→70/30 en 20 min, 5,5 mL/min, 215 nm) pour conduire au tétrasaccharide cible ***VIII*** sous forme d'une poudre blanche après lyophilisation (36 mg. 68%).
Rf = 0,41 (iPrOH/NH₃/H₂O, 7/1/2).
*RMN ¹H (CDCl₃), δ 7.40-7.06 (m, 50H, CH_{Ph}), 6.38 (d, 1H, J_{NH,2}* = *9.1 Hz, NH), 5.86 (m, 1H, CH*=*), 5.44 (dd, 1H, J_{2,3}* = *3.0 Hz, H-2_{B'}), 5.26 (m, 1H, Jₜᵣₐₙₛ* = *17.2 Hz,* =*CH₂), 5.20-5.17 (m, 2H,* =*CH₂, H-1_{E}), 5.07 (d, 1H, J_{1,2} = 1.6 Hz, H-1_{A}), 5.05 (d, 1H, J_{1,2} = 1.8 Hz, H-1_{B'}), 5.02-4.75 (m, 9H, 4H_{Bn}, H-2_{C,} 4H_{Bn}), 4.75 (d, 1H, J_{1,2}* = *1.4 Hz, H-1_{B}), 4.70 (bs, 1H, H-1_{C}), 4.68-4.57 (m, 9H, H_{Bn}), 4.50 (d, 1H, J = 11.0 Hz, H_{Bn}), 4.42-4.33 (m, 2H, H_{Bn}), 4.28 (d, 1H, J_{1,2}* = *8.6 Hz, H-1_{D}), 4.21-4.08 (m, 6H, H-3_{E}, H-5_{E}, H-2_{D}, H-3_{A}, H_{All,} H-3_{C}), 3.99 (dd, 1H, J_{2,3} = 2.6 Hz, H-2_{A}), 3.96-3.84 (m, 7H, H-5_{C}, H-2_{B}, H_{All}, H-3_{B}, H-3_{B'}, H-2_{E}, H-4_{E}), 3.81-3.73 (m, 2H, H-5_{A}, H-5_{B'}), 3.71 (dq, 1H, J_{4,5} = 9.4 Hz, H-5_{B}), 3.54-3.38 (m, 8H, H-6a_{D}, H-6b_{D}, H-6a_{E}, H-6b_{E}, H-4_{A}, H-4_{B}, H-4_{D}, H-4_{C}, H-4_{B'}), 2.75-2.64 (m, 6H, H-5_{D}, 2CH_{2Lev}, H-3_{D}), 2.31 (s, 3H, H_{NAc}), 2.18 (s, 3H, CH_{3Lev}), 2.05 (s, 3H, H_{Ac}), 1.42 (s, 3H, H_{iPr}), 1.39 (s, 3H, H_{iPr}), 1.35-1.31 (m, 9H, H-6_{A}^{*}, H-6_{B}^{*}*, *H-6_{B'}^{*}), 1.24 (d, 3H, J_{5,6}* = *6.2 Hz, H-6_{C}).*
*RMN ¹³C (CDCl₃), δ206.1 (C_{Lev}), 171.7 (C_{Lev}), 170.4 (C_{Ac}), 170.0 (C_{NAc})*, *138.6-137.5 (C_{Ph}), 133.8 (CH=), 129.3-127.7 (CH_{Ph}), 117.1 (=CH₂), 103.6 (C-1_{D}, ¹J_{CH}* = *159.0 Hz), 101.1 (C-1_{A}, ¹J_{CH}*= *172.8 Hz), 99.5 (C-1_{B'}, ¹J_{CH}* = *167.8 Hz), 99.2 (C_{iPr}), 97.9 (C-1_{B}, ¹J_{CH}*= *172.8 Hz), 97.8 (C-1_{C}, ¹J_{CR}* = *172.8 Hz), 94.2 (C-1_{E}, ¹J_{CR}* = *165.3 Hz), 83.4 (C-3_{E}), 80.5 (C-3_{D}), 80.4 (C-4_{B}), 80.2 (C-2_{E}), 80.1 (C-4_{B'}), 80.0 (C-4_{C}), 79.8 (C-4_{A}), 79.2 (C-3_{B}), 78.5* (C-4_{E}), *78.2 (C-3_{C}), 77.9 (C-3_{B'}), 76.2 (C-2_{A}), 76.1, 75.7, 75.4 (3C, C_{Bn}), 75.3 (C-2_{B}), 75.2, 75.1, 75.0, 74.5 (4C, C_{Bn}), 74.5 (C-3_{A}), 73.5 (C_{Bn}), 73.2 (C-2_{C}), 72.1 (C-4_{D}), 71.9, 71.4 (2C, C_{Bn}), 70.0 (C-5_{E}), 69.3 (C-2_{B'}), 68.9 (C-5_{A}), 68.5 (C-5_{B'}), 67.9 (C-6_{E}), 67.8 (C-5_{B}), 67.7 (C_{All}), 67.3* (C-5_{C}), *67.2 (C-5_{D}), 62.0 (C-6_{D}), 55.0 (C-2 _{D}), 38.1 (CH_{2Lev}), 29.8 (CH_{3Lev}), 29.2 (C_{iPr}), 28.2 (CH_{2Lev}), 24.1 (C_{NAc}), 21.0 (C_{Ac}), 19.1 (C_{iPr}), 18.0, 17.9, 17.8, 17.7 (C-6_{A}*, C-6_{B}*, C-6_{B'}*, C-6_{C}*).*

| | | |
|---|---|---|
| *HRMS (ESI⁺) :* | *[M+Na]⁺ C₃₇H₆₃NO₂₄Na* | *m*/*z théorique : 928.3638* |
| | | *m*/*z mesurée: 928.3660* |

### Procédé 16 :

### ➢ Allyl (3,4-di-O-benzyl-2-O-lévulinoyl-α-L-rhamnopyranosyl)-(1→3)-(2-O-cétyl-4-O-benzyl-α-L-rhamnopyranosyl)-(1→3)-(2-désoxy-4,6-O-isopropylidène-2-acétamido-β-D-glucopyranosyl)-(1→2)-[2,3,4,6-tétra-O-benzy]-α-D-glucopyranosyl-(1→3)]-4-O-benzyl-α-L-rhamnopyranoside 120 :

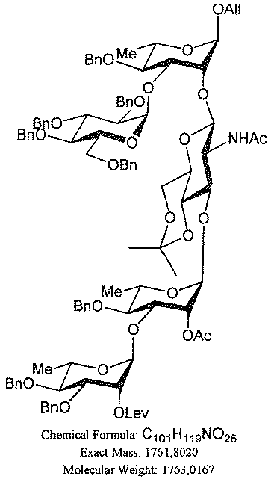

Du TfOH (23,0 µL, 263 µmol, 0,9 éq.) est additionné à une solution d'accepteur **119** (310 mg, 290 µmol et de donneur **47** (379 mg, 440 µmol, 1,5 éq.) dans du ToI (10 mL), en présence de tamis moléculaire 4 Å (1,7 g), agitée sous argon à 0°C. Après 15 min sous agitation, le suivi par CCM (Chex/EtOAc, 1/1) indique la disparition de **119** (Rf = 0,1) et l'apparition d'un nouveau composé plus polaire (Rf = 0,35). La réaction est stoppée par l'addition de triéthylamine (0,2 mL) puis le milieu réactionnel est filtré et concentré à l'évaporateur rotatif. L'huile jaune obtenue est purifiée par chromatographie sur gel de silice (Tol/EtOAc, 8/2→6/4) pour obtenir le glycoside d'allyle **120** sous forme d'un solide blanc (383 mg, 74%) mais légèrement contaminé. Rf=0,35 (Chex/EtOAc, 1/1).
RMN ¹H (CDCl₃), *δ* 7.45-7.07 (m, 40H, CH_{Ph}), 6.47 (d, 1H, *J*_{NH,2} = 9.0 Hz, NH), 5.90 (m, 1H, CH=), 5.46 (dd, 1H, *J*_{2,3} = 3.0 Hz, H-2_{B}), 5.29 (m, 1H, *J*ₜᵣₐₙₛ = 17.2 Hz, =CH₂), 5.20 (m, 1H, *J*_{cis} = 10.4 Hz, =CH₂), 5.15 (d, 1H, *J*_{1,2} = 3.5 Hz, H-1_{E}), 5.09 (d, 1H, *J*_{1,2} = 1.2 Hz, H-1_{B}), 5.07-4.98 (m, 4H, H_{Bn}), 4.94 (dd, 1H, *J_{2,3}* = 2.7 Hz, H-2_{C}), 4.93-4.88 (m, 3H, H_{Bn}), 4.84 (bs, 1H, H-1_{A}), 4.77 (bs, 1H, H-1_{C}), 4.67-4.60 (m, 5H, H_{Bn}), 4.53-4.33 (d, 4H, H_{Bn}), 4.28 (d, 1H, *J*_{1,2} = 8.6 Hz, H-1_{D}), 4.21-4.13 (m, 6H, H-3_{E}, H-2_{D}, H-5_{E}, H-3_{C}, H_{All}, H-3_{A}), 4.00 (dq, 1H, *J*_{4,5} = 9.6 Hz, H-5_{C}), 3.97-3.83 (m, 6H, H_{All}, H-3_{B}, H-2_{E}, H-2_{A}, H-4_{E}, H-6a_{D}), 3.79 (dq, 1H, *J*_{4,5} = 9.4 Hz, *H-5_{B}),* 3.73 (m, 1H, H-6b_{D}), 3.70 (dq, 1H, *J*_{4,5} = 9.5 Hz, H-5_{A}), 3.52 (pt, 1H, *J*_{3,4} = 9.3 Hz, H-4_{D}), 3.58-3.40 (m, 5H, H-4_{A}, H-6a_{E}, H-6b_{E}, H-4_{C}, H-4_{B}), 2.76-2.68 (m, 6H, H-5_{D}, 2CH_{2Lev}, H-3_{D}), 2.35 (s, 3H, H_{NAc}), 2.19 (s, 3H, CH_{3Lev}), 2.06 (s, 3H, H_{Ac}), 1.48 (s, 3H, H_{iPr}), 1.45 (s, 3H, H_{iPr}), 1.38 (d, 3H, *J*_{5,6} = 6.2 Hz, H-6_{A}), 1.34 (d, 3H, J_{5,6} = 6.2 Hz, H-6_{B}), 1.27 (d, 3H, *J*_{5,6}= 6.2 Hz, H-6_{C}).
RMN ¹³C (CDCl₃), *δ*206.1 (C_{Lev}), 171.8 (C_{Lev}), 170.5 (C_{Ac}), 170.1 (C_{NAc}), 138.7-137.5 (C_{Ph}), 134.0 (CH=), 129.4-127.8 (CH_{Ph}), 117.1 (=CH₂), 103.7 (C-1_{D}, ¹*J*_{CH} = 159.9 Hz), 99.5 (C-1_{B}, ¹*J*_{CH} = 169.7 Hz), 99.3 (C_{iPr}), 98.4 (C-1_{A}, ¹*J*_{CH} = 182.1 Hz), 97.5 (C-1_{C}, ¹*J*_{CH}= 170.6 Hz), 94.4 (C-1_{E}, ¹*J*_{CH} = 170.1 Hz), 83.5 (C-3_{E}), 80.7 (C-3_{D}), 80.4 (C-2_{E}), 80.1, 80.0 (C-4_{B}*, C-4_{C}*), 79.8 (C-4_{A}), 78.5 (C-4_{E}), 78.2 (C-3_{C}), 77.9 (C-3_{B}), 76.7 (C-2_{A}), 76.1, 75.9, 75.5, 75.4, 75.1, 75.0 (6C, C_{Bn}), 74.8 (C-3_{A}), 73.4 (C_{Bn}), 73.3 (C-2_{C}72.4 (C-4_{D}), 71.3 (C_{Bn}), 70.0 (C-5_{E}), 69.3 (C-2_{B}), 68.6 (C-5_{A}), 68.5 (C-5_{B}), 67.9 (C-6_{E}), 67.8 (C_{All}), 67.4 (C-5_{D}), 67.3 (C-5_{C}), 62.2 (C-6_{D}), 55.0 (C-2_{D}), 38.1 (CH_{2Lev}), 29.8 (CH_{3Lev}), 29.2 (C_{iPr}), 28.3 (CH_{2Lev}), 24.1 (C_{NAc}), 21.1 (C_{Ac}), 19.1 (C_{iPr}), 17.9, 17.8, 17.7 (C-6_{A}*, C-6_{B}*, C-6_{C}*).

| | | |
|---|---|---|
| HRMS (ESI⁺) : | [M+H]⁺ C₁₀₁H₁₁₉NO₂₆ | m/z théorique : 1762.8098 |
| | | m/z mesurée: 1762.8198 |
| | [M+Na]⁺ C₁₀₁H₁₁₉NO₂₆Na | m/z théorique : 1784.7917 |
| | | m/z mesurée : 1784.7988 |

### ➢ Allyl (3,4-di-O-benzyl-2-O-lévulinoyl-α-L-rhamnopyranosyl)-(1→3)-2-O-acétyl-4-O-benzyl-α-L-rhamnopyranosyl)-(1→3)-(2-désoxy-4,6-O-isopropylidène-2-acétamido-β-D-glucopyranosyl)-(1→2)-[2,3,4,6-tétra-O-benzyl-α-D-glucopyranosyl-(1→3)]-(4-O-benzyl-α-L-rhamnopyranosyl)-(1→2)-3,4-di-O-benzyl-α-L-rhamnopyranoside 121 :

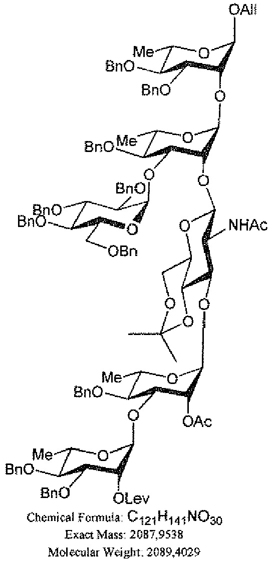

Du TMSOTf (18,0 µL, 100 µmol, 0,9 éq.) est additionné à une solution d'accepteur **116** (150 mg, 110 µmol) et de donneur **47** (140 mg, 160 µmol, 1,5 éq.) dans du Tol (5 mL), en présence de tamis moléculaire 4 Å (620 mg), agitée sous argon à 0°C. Après 45 min d'agitation, le suivi par CCM (Tol/EtOAc, 75/25 et Chex/EtOAc, 1/1) indique la disparition de **116** (Rf = 0,05 et Rf = 0,15, respectivement) et l'apparition d'un nouveau composé moins polaire (Rf = 0,55 et Rf = 0,2). La réaction est stoppée par addition de triéthylamine (0,5 mL) puis le milieu réactionnel est filtré et concentré à l'évaporateur rotatif. L'huile jaune obtenue est purifiée par chromatographie sur gel de silice (Tol/EtOAc, 75/25→1/1) pour obtenir par ordre d'élution le glycoside d'allyle **121** sous forme d'un solide blanc (149 mg, 65%) mais légèrement contaminé.
Rf= 0,55 (Chex/EtOAc, 1/1).
RMN ¹H (CDCl₃), *δ*7.40-7.06 (m, 50H, CH_{Ph}), 6.38 (d, 1H, *J*_{NH,2} = 9.1 Hz, NH), 5.86 (m, 1H, CH=), 5.44 (dd, 1H, *J_{2,3}* = 3.0 Hz, H-2_{B'}), 5.26 (m, 1H, *J*ₜᵣₐₙₛ = 17.2 Hz, =CH₂), 5.20-5.17 (m, 2H, =CH₂, H-1_{E}), 5.07 (d, 1H, *J*_{1,2} = 1.6 Hz, H-1_{A}), 5.05 (d, 1H, *J*_{1,2} = 1.8 Hz, H-1_{B'}), 5.02-4.75 (m, 9H, 4H_{Bn}, H-2_{C}, 4H_{Bn}), 4.75 (d, 1H, *J*_{1,2} = 1.4 Hz, H-1_{B}), 4.70 (bs, 1H, H-1_{C}), 4.68-4.57 (m, 9H, H_{Bn}), 4.50 (d, 1H, *J* = 11.0 Hz, H_{Bn}), 4.42-4.33 (m, 2H, H_{Bn}), 4.28 (d, 1H, *J*_{1,2} = 8.6 Hz, H-1_{D}), 4.21-4.08 (m, 6H, H-3_{E}, H-5_{E}, H-2_{D}, H-3_{A}, H_{All}, H-3_{C}), 3.99 (dd, 1H, *J_{2,3}* = 2.6 Hz, H-2_{A}), 3.96-3.84 (m, 7H, H-5_{C}, H-2_{B}, H_{All}, H-3_{B}, H-3_{B'}, H-2_{E}, H-4_{E}), 3.81-3.73 (m, 2H, H-5_{A}, H-5_{B'}), 3.71 (dq, 1H, *J*_{4,5} = 9.4 Hz, *H-5_{B}),* 3.54-3.38 (m, 8H, H-6a_{D}, H-6b_{D}, H-6a_{E}, H-6b_{E}, H-4_{A}, H-4_{B}, H-4_{D}, H-4_{C}, H-4_{B'}), 2.75-2.64 (m, 6H, H-5_{D}, 2CH_{2Lev}, H-3_{D}), 2.31 (s, 3H, H_{NAc}), 2.18 (s, 3H, CH_{3Lev}), 2.05 (s, 3H, H_{Ac}), 1.42 (s, 3H, H_{iPr}), 1.39 (s, 3H, H_{iPr}), 1.35-1.31 (m, 9H, H-6_{A}*, H-6_{B}*, H-6_{B}*), 1.24 (d, 3H, J_{5,6} = 6.2 Hz, H-6_{C}).
RMN ¹³C (CDCl₃), *δ*206.1 (C_{Lev}), 171.7 (C_{Lev}), 170.4 (C_{Ac}), 170.0 (C_{NAc}), 138.6-137.5 (C_{Ph}), 133.8 (CH=), 129.3-127.7 (CH_{Ph}), 117.1 (=CH₂), 103.6 (C-1_{D}, ¹*J*_{CH} = 159.0 Hz), 101.1 (C-1_{A}, ¹*J*_{CH} = 172.8 Hz), 99.5 (C-1_{B'}, ¹*J*_{CH} = 167.8 Hz), 99.2 (C_{iPr}), 97.9 (C-1_{B}, ¹*J*_{CH} = 172.8 Hz), 97.8 (C-1_{C}, ¹*J*_{CH} = 172.8 Hz), 94.2 (C-1_{E}, ¹*J*_{CH} = 165.3 Hz), 83.4 (C-3_{E}), 80.5 (C-3_{D}), 80.4 (C-4_{B}), 80.2 (C-2_{E}), 80.1 (C-4_{B'}), 80.0 (C-4_{C}), 79.8 (C-4_{A}), 79.2 (C-3_{B}), 78.5 (C-4_{E}), 78.2 (C-3_{C}), 77.9 (C-3_{B'}), 76.2 (C-2_{A}), 76.1, 75.7, 75.4 (3C, C_{Bn}), 75.3 (C-2_{B}), 75.2, 75.1, 75.0, 74.5 (4C, C_{Bn}), 74.5 (C-3_{A}), 73.5 (C_{Bn}), 73.2 (C-2c), 72.1 (C-4_{D}), 71.9, 71.4 (2C, C_{Bn}), 70.0 (C-5_{E}), 69.3 (C-2_{B'}), 68.9 (C-5_{A}), 68.5 (C-5_{B'}), 67.9 (C-6_{E}), 67.8 (C-5_{B}), 67.7 (C_{All}), 67.3 (C-5_{C}), 67.2 (C-5_{D}), 62.0 (C-6 _{D}), 55.0 (C-2 _{D}), 38.1 (CH_{2Lev}), 29.8 (CH_{3Lev}), 29.2 (C_{iPr}), 28.2 (CH_{2Lev}), 24.1 (C_{NAc}), 21.0 (C_{Ac}), 19.1 (C_{iPr}), 18.0, 17.9, 17.8, 17.7 (C-6A*, C-6_{B}*, C-6_{B}*, C-6_{C}*).

| | | |
|---|---|---|
| HRMS (ESI⁺) : | [M+H]⁺ C₁₂₁H₁₄₁NO₃₀ | m/z théorique : 2088.9617 |
| | | m/z mesurée : 2088.9619 |
| | [M+ Na]⁺ C₁₂₁H₁₄₁NO₃₀Na | m/z théorique : 2110.9436 |
| | | m/z mesurée : 2110.9497 |

### ➢ Allyl (3,4-di-O-benzyl-2-O-lévulinoyl-α-L-rhamnopyranosyl)-(1→3)-2-O-acétyl-O-benzyl-α-L-rhamopyranosyl)-(1→3)-(2-désoxy-4,6-O-isopropylidène-2-trichloroacétamido-β-D-glucopyranosyl)-(1→2)-[2,3,4,6-tétra-O-benzyl-α-D-glucopyranosyl-(1→3)]-(4-O-benzyl-α-L-rhamnopyranosyl)-(1→2)-3,4-di-O-benzyl-α-L-rhamnopyranoside 122 :

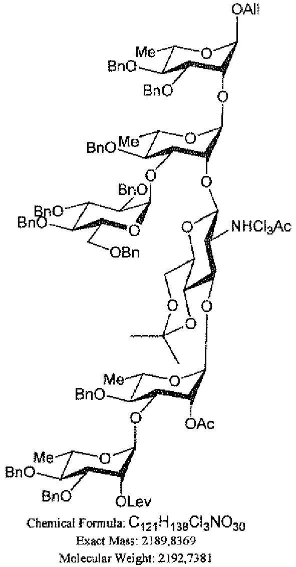

Du TMSOTf (61,0 µL, 340 µmol, 0,3 éq.) est additionné à une solution d'accepteur **110** (1,7 g, 1,1 mmol) et de donneur **47** (1,5 g, 1,7 mmol, 1,5 éq.) dans du Tol (35 mL), en présence de tamis moléculaire 4 Å (975 mg), agitée sous argon à -40°C. Après 1 h d'agitation, le suivi par CCM (Tol/EtOAc, 8/2) indique la disparition de **110** (Rf = 0,3) et l'apparition d'un nouveau composé moins polaire (Rf = 0,45). La réaction est stoppée par addition de triéthylamine (0,5 mL) puis le milieu réactionnel est filtré et concentré à l'évaporateur rotatif. L'huile jaune obtenue est purifiée par chromatographie sur gel de silice (Tol/EtOAc, 95/5→7/3) pour obtenir par ordre d'élution le glycoside d'allyle **122** sous forme d'un solide blanc (1,45 g, 59%) puis le diol **123** sous forme d'un solide blanc (505 mg, 21%).
Rf = 0,45 (Tol/EtOAc, 8/2).
RMN ¹H (CDCl₃), *δ*7.45-7.10 (m, 50H, CH_{Ph}), 6.94 (d, 1H, *J*_{NH,2} = 8.8 Hz, NH), 5.92 (m, 1H, CH=), 5.46 (dd, 1H, *J*_{1,2} = 1.9 Hz, *J_{2,3}* = 3.0 Hz, H-2_{B'}), 5.30 (m, 1H, *J*ₜᵣₐₙₛ = 17.2 Hz, =CH₂), 5.25-5.22 (m, 2H, H-1_{E}, =CH₂), 5.15-5.05 (m, 6H, H-1_{A}, 2H_{Bn}, H-1_{B'}, H-2_{c}, H_{Bn}), 4.98-4.93 (m, 3H, H_{Bn}), 4.89-4.83 (m, 3H, H_{Bn}), 4.81 (d, 1H, *J*_{1,2} = 1.4 Hz, H-1_{B}), 4.76 (d, 1H, *J*_{1,2}= 1.4 Hz, H-1_{C}), 4.72-4.60 (m, 8H, H_{Bn}), 4.58 (d, 1H, *J*_{1,2}= 3.8 Hz, H-1_{D}), 4.54 (d, 1H, *J* = 11.0 Hz, H_{Bn}), 4.45 (d, 1H, *J* = 11.3 Hz, H_{Bn}), 4.37 (d, 1H, *J* = 12.0 Hz, H_{Bn}), 4.21 (pt, 1H, *J*_{3,4}= 9.3 Hz, H-3_{E}), 4.19-4.14 (m, 5H, H-3_{C}, H_{All}, H-3_{A}, H-2_{A}, H-5_{E}), 4.07-3.86 (m, 9H, H-2_{B}, H-2_{D}, H_{All}, H-5_{C}, H-5_{B'}, H-3_{B}, H-3_{B'}, H-2_{E}, H-4_{E}), 3.80 (dq, 1H, *J*_{4,5} = 9.5 Hz, H-5_{A}), 3.74 (dq, 1H, *J*_{4,5} = 9.4 Hz, H-5_{B}), 3.60-3.43 (m, 8H, H-6a_{D}, H-4_{A}, H-4_{B}, H-6a_{E}, H-6b_{E}, H-4_{D}, H-4_{B',} H-4_{C}), 3.35 (m, 1H, *J*_{6a,6b} = 10.4 Hz, H-6b_{D}), 2.82 (m, 1H, H-5_{D}), 2.75-2.69 (m, 5H, 2CH_{2Lev}, H-3_{D}), 2.21 (s, 3H, CH_{3Lev}), 2.14 (s, 3H, H_{Ac}), 1.44-1.43 (m, 9H, H_{iPr}, H-6_{A}), 1.39 (d, 3H, *J*_{5,6} = 6.8 Hz, H-6_{B'}), 1.37 (d, 3H, *J*_{5,6}= 6.2 Hz, H-6_{B}), 1.37 (d, 3H, *J*_{5,6}= 6.2 Hz, H-6_{C}).
RMN ¹³C (CDCl₃), *δ*206.3 (C_{Lev}), 171.8 (C_{Lev}), 169.6 (C_{Ac}), 162.0 (C_{NTCA}), 138.7-137.6 (C_{Ph}), 133.9 (CH=), 129.4-127.5 (CH_{Ph}), 117.2 (=CH₂), 101.2 (C-1_{D}, ¹*J*_{CH} = 163.9 Hz), 100.9 (C-1_{A}, ¹*J*_{CH} = 172.0 Hz), 99.4 (C_{iPr}), 99.0 (C-1_{B'}, ¹*J*_{CH} = 168.3 Hz), 98.0 (C-1_{B'}, ¹*J*_{CH} = 168.7 Hz), 97.6 (C-1_{C}, ¹*J*_{CH} = 170.4 Hz), 94.3 (C-1_{E}, ¹*J*_{CH} = 167.5 Hz), 93.1 (CCl₃), 83.1 (C-3_{E}), 80.4, 80.1, 79.9, 79.7, 79.6, 79.0 (7C, C-3_{B}*, C-3_{B'}*, C-4_{A}*, C-4_{B}*, C-4_{B'}*, C-4_{C}*, C-4_{E*}), 78.7 (C-3_{D}), 77.6 (C-2_{E}), 76.8 (C-3_{C}), 76.2, 75.4, 75.3, 75.2, 75.0, 74.9, 74.8 (7C, C_{Bn}), 74.3 (C-2_{B}), 74.2 (C-3_{A}), 73.5 (C_{Bn}), 73.4 (C-2_{A}), 72.6 (C-4_{D}), 72.1 (C-2_{C}), 72.0, 71.5 (2C, C_{Bn}), 69.9 (C-5_{E}), 69.4 (C-2_{B'}) 68.7 (C-5_{A}), 68.5 (C-5_{B'}), 68.0 (C-5_{B}), 67.9 (C-6_{E}), 67.8 (C-5_{C}), 67.7 (C_{All}), 67.0 (C-5_{D}), 61.8 (C-6_{D}), 57.4 (C-2_{D}), 38.2 (CH_{2Lev}), 29.9 (CH_{3Lev}), 29.2 (C_{iPr}), 28.2 (CH_{2Lev}), 21.2 (C_{Ac}), 19.1 (C_{iPr}), 18.3, 18.1, 18.0, 17.9 (C-6_{A}*, C-6_{B}*, C-6_{B'}*, C-6_{C}*).

| | | |
|---|---|---|
| HRMS (ESI⁺) : | [M+H]⁺ C₁₂₁H₁₃₈NO₃₀³⁵Cl₃ | m/z théorique : 2190.8447 |
| | | m/z mesurée: 2190.8403 |
| | [M+ Na]⁺ C₁₂₁H₁₃₈NO₃₀³⁵Cl₃Na | m/z théorique : 2212.8267 |
| | | m/z mesurée : 2212.8215 |

### ➢ Allyl (3,4-di-O-benzyl-2-O-lévulinoyl-α-L-rhamnopyranosyl)-(1→3)-2-O-acétyl-4-O-benzyl-α-L-rhamnopyranosyl)-(1→3)-(2-désoxy-2-trichloroacétamido-β-D-glucopyranosyl)-(1→2)-[2,3,4,6-tétra-O-benzyl-α-D-glucopyranosyl-(1→3)]-(4-O-benzyl-α-L-rhamnopyranosyl)-(1→2)-3,4-di-O-benzyl-α-L-rhamnopyranoside 123 :

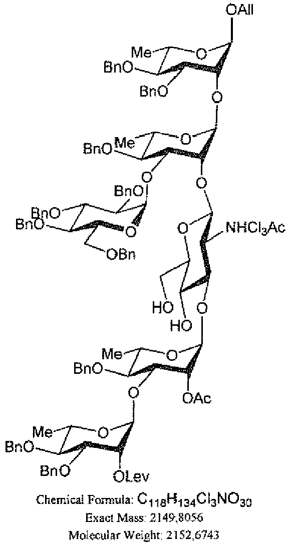

Une solution aqueuse de TFA 50% (8 mL) est additionnée lentement à une solution du glycoside d'allyle **122** (1,9 g, 880 µmol) dans du DCM (30 mL) à 0°C. Le milieu réactionnel est agité pendant 2 h à cette température, temps au bout duquel le suivi par CCM (Tol/ EtOAc, 7/3) indique la disparition totale de **122** (Rf = 0,55) et l'apparition d'un nouveau composé plus polaire (Rf = 0,4). Le milieu réactionnel est concentré par coévaporation avec du Tol (3 x 10 mL). L'huile jaune obtenue est purifiée par chromatographie sur gel de silice (Tol/EtOAc, 8/2→6/4) pour obtenir le diol **123** sous forme d'un solide blanc (1,7 g, 89%).
Rf = 0,4 (Tol/ EtOAc, 7/3).
RMN ¹H (CDCl₃), *δ*7.62-7.13 (m, 50H, CH_{Ph}), 7.04 (d, 1H, *J*_{NH,2}= 8.8 Hz, NH), 5.94 (m, 1H, CH=), 5.53 (dd, 1H, *J*_{2,3} = 3.0 Hz, H-2_{B'}), 5.44 (bs, 1H, H-1_{A}), 5.34 (m, 1H, *J*ₜᵣₐₙₛ = 17.2 Hz, =CH₂), 5.34 (d, 1H, *J*_{1,2} = 3.6 Hz, H-1_{E}), 5.26 (m, 1H, *J*_{cis} = 10.4 Hz, =CH₂), 5.22-5.11 (m, 5H, 2H_{Bn}, H-1_{B'}, H-2_{C}, H_{Bn}), 5.07-4.88 (m, 7H, H_{Bn}), 4.82-4.78 (m, 3H, 2H_{Bn}, H-1_{B}), 4.75-4.64 (m, 8H, H_{Bn}), 4.60 (bs, 1H, H-1_{C}), 4.56 (d, 1H, *J*_{1,2} = 8.8 Hz, H-1_{D}), 4.44 (m, 2H, H_{Bn}), 4.30-4.18 (m, 7H, H-3_{C}, H-2_{A}, H-3_{A}, H-3_{E}, H-5_{E}, H-2_{B}, H_{All}), 4.05-3.70 (m, 7H, H_{All}, H-2_{D}, H-3_{B}, H-3_{B'}, H-5_{C}, H-4_{E}, H-2_{E}), 3.88 (dq, 1H, *J*_{4,5} = 9.4 Hz, H-5_{B'}), 3.81 (dq, 1H, *J*_{4,5} = 9.6 Hz, H-5_{B}), 3.78 (dq, 1H, *J*_{4,5} = 10.0 Hz, H-5_{A}), 3.72 (m, 1H, H-6a_{D}), 3.61-3.56 (m, 4H, H-4_{B}, H-6a_{E}, H-6b_{E}, H-4_{A}), 3.54 (pt, 1H, *J*_{3,4} = 9.5 Hz, H-4_{C}), 3.48 (pt, 1H, *J*_{3,4} = 9.4 Hz, H-4_{B'}), 3.10 (m, 2H, H-4_{D}, H-5_{D}), 3.05 (m, 1H, H-6b_{D}), 2.78 (m, 4H, 2CH_{2Lev}), 2.24 (s, 3H, CH_{3Lev}), 2.23 (s, 3H, H_{Ac}), 2.19 (m, 1H, H-3_{D}), 1.50 (d, 3H, *J*_{5,6}= 6.3 Hz, H-6_{A}), 1.46 (d, 3H, *J*_{5,6} = 6.2 Hz, H-6_{B}), 1.38 (d, 3H, *J*_{5,6} = 6.1 Hz, H-6_{B'}), 1.37 (d, 3H, *J*_{5,6} = 6.1 Hz, H-6_{C}).
RMN ¹³C (CDCl₃), *δ*206.1 (C_{Lev}), 171.7 (C_{Lev}), 170.0 (C_{Ac}), 162.2 (C_{NTCA}), 138.8-137.6 (C_{Ph}), 133.9 (CH=), 129.4-127.5 (CH_{Ph}), 117.1 (=CH₂), 101.1 (C-1_{D}, ¹*J*_{CH} = 165.4 Hz), 100.2 (C-1_{A}, ¹*J*_{CH}= 175.4 Hz), 99.1 (C-1_{B'}, ¹*J*_{CH} = 171.8 Hz), 98.5 (C-1_{C}, ¹*J*_{CH} = 173.3 Hz), 98.3 (C-1_{B}, ¹*J*_{C*H*} = 168.3 Hz), 94.1 (C-1_{E}, ¹*J*_{CH} = 166.9 Hz), 93.3 (CCl₃), 87.2 (C-3_{D}), 83.1 (C-3_{E}), 80.6 (C-3_{B}), 80.5 (2C, C-2_{E}, C-4_{B}), 80.1 (C-4_{B'}), 79.7 (C-4_{A}), 79.5 (C-4_{C}), 78.8 (C-4_{E}), 77.7 (C-3_{B'}), 77.3 (C-3_{C}), 76.3 (C_{Bn}), 75.6 (C-5_{D}), 75.5, 75.4, 75.3, 75.0 (6C, C_{Bn}), 74.1, 74.0 (C-2_{B}*, C-3_{A}*), 73.5, 72.9 (2C, C_{Bn}), 72.1 (C-2_{C}), 71.4 (C_{Bn}), 71.3 (2C, C-2_{A}, C-4_{D}), 70.1 (C-5_{E}), 69.4 (2C, C-2_{B'}, C-5_{C}), 68.9 (C-5_{A}), 68.7 (C-5_{B'}), 68.4 (C-5_{B}), 68.0 (C-6_{E}), 67.6 (C_{All}), 62.8 (C-6_{D}), 55.5 (C-2_{D}), 38.2 (CH_{2Lev}), 29.9 (CH_{3Lev}), 28.3 (CH_{2Lev}), 21.1 (C_{Ac}), 18.1, 18.0, 17.8, 17.9 (C-6_{A}*, C-6_{B}*, C-6_{B'}*, C-6_{C}*).

| | | |
|---|---|---|
| HRMS (ESI⁺) : | [M+H]⁺ C₁₁₈H₁₃₄NO₃₀³⁵Cl₃ | m/z théorique : 2150.8135 |
| | | m/z mesurée : 2150.84857 |
| | [M+ Na]⁺ C₁₁₈H₁₃₄NO₃₀³⁵Cl₃Na | m/z théorique : 2172.7954 |
| | | m/z mesurée: 2172.8081 |

### ➢ Allyl (3,4-di-O-benzyl-2-α-L-rhamnopyranosyl)-(1→3)-2-O-acétyl-4-O-benzyl-α-L-rhamnopyranosyl)-(1→3)-(2-désoxy-2-trichloroacétamido-β-D-glucopyranosyl)-(1→2)-[2,3,4,6-tétra-O-benzyl-α-D-glucopyranosyl-(1→3)]-(4-O-benzyl-α-L-rhamnopyranosyl)-(1→2)-3,4-di-O-benzyl-α-L-rhamnopyranoside 124 :

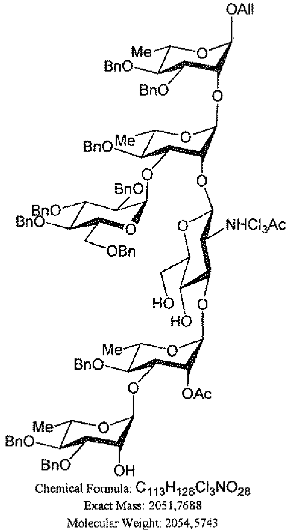

Le diol **123** (700 mg, 320 µmol) est dissous dans un mélange pyridine/acide acétique (3/2, 5 mL). De l'hydrazine monohydraté (158 µL, 3,2 mmol, 10 éq.) est ajouté goutte à goutte au milieu réactionnel. Après 25 min d'agitation à 0°C, le suivi par CCM (Tol/EtOAc, 7/3) indique la disparition de **123** (Rf = 0,4) et l'apparition d'un produit plus polaire (Rf = 0,35). Le milieu réactionnel est alors repris à l'eau froide (10 mL) et la phase aqueuse est extraite rapidement au DCM (3 x 50 mL). Les phases organiques sont regroupées et lavées avec une solution de NaCl sat, filtrées sur filtre séparateur de phase et concentrées à l'évaporateur rotatif. L'huile obtenue est purifiée par chromatographie sur gel de silice (Tol/EtOAc, 9/1→7/3) pour obtenir le triol 124 sous forme d'un solide blanc (560 mg, 84%).
Rf = 0,35 (Tol/ EtOAc, 7/3).
RMN ¹H (CDCl₃), *δ*7.44-7.07 (m, 50H, CH_{Ph}), 7.02 (d, 1H, *J*_{NH,2} = 8.6 Hz, NH), 5.90 (m, 1H, CH=), 5.40 (bs, 1H, H-1_{A}), 5.30 (d, 1H, *J*_{1,2} = 3.6 Hz, H-1_{E}), 5.29 (m, 1H, *J*ₜᵣₐₙₛ= 17.2 Hz, =CH₂), 5.22 (m, 1H, *J*_{cis} = 10.4 Hz, =CH₂), 5.18-5.12 (m, 4H, H_{Bn}, H-1_{B'}, H-2_{C}, H_{Bn}), 5.16-4.99 (m, 3H, H_{Bn}), 4.93 (d, 1H, *J*= 10.9 Hz, H_{Bn}), 4.85-4.81 (m, 2H, H_{Bn}), 4.77-4.66 (m, 7H, 3H_{Bn}, H-1_{B}, 3H_{Bn}), 4.62-4.54 (m, 6H, 5H_{Bn}, H-1_{C}), 4.51 (d, 1H, *J*_{1,2} = 8.4 Hz, H-1_{D}), 4.38 (d, 1H, *J* = 11.9 Hz, H_{Bn}), 4.23-4.13 (m, 7H, H-2_{B}, H-3_{C}, H-3_{E}, H-3_{A}, H_{All}, H-5_{E}, H-2_{A}), 5.53 (m, 1H, H-2_{B'}), 4.00-3.87 (m, 6H, H_{All}, H-2_{D}, H-3_{B}, H-5_{C}, H-4_{E}, H-2_{E}), 3.84-3.81 (m, 1H, H-3_{B'}, H-5_{B'}), 3.78 (m, 1H, H-5_{B}), 3.72 (m, 1H, H-5_{A}), 3.65 (m, 1H, H-6a_{D}), 3.55-3.49 (m, 5H, H-6a_{E}, H-6b_{E}, H-4_{B}, H-4_{A}, H-4_{B'}), 3.47 (pt, 1H, *J*_{3,4} = 9.5 Hz, H-4_{C}), 3.04 (m, 2H, H-4_{D}, H-5_{D}), 2.94 (m, 1H, H-6b_{D}), 2.20 (s, 3H, H_{Ac}), 2.15 (m, 1H, H-3_{D}), 1.46 (d, 3H, *J*_{5,6}= 6.2 Hz, H-6_{A}), 1.42 (d, 3H, *J*_{5,6} = 6.2 Hz, H-6_{B}), 1.33-1.30 (m, 6H, H-6_{C}, H-6_{B'}).
RMN ¹³C (CDCl₃), *δ*170.0 (C_{Ac}), 162.3 (C_{NTCA}), 138.7-137.4 (C_{Ph}), 133.8 (CH=), 129.4-127.4 (CH_{Ph}), 117.1 (=CH₂), 101.1 (C-1_{D}, ¹*J*_{CH} = 161.0 Hz), 100.6 (C-1_{B'}, ¹*J*_{CH} = 171.0 Hz), 100.1 (C-1_{A}, ¹*J*_{CH}= 174.7 Hz), 98.3 (C-1_{C}, ¹*J*_{CH}= 170.4 Hz), 98.2 (C-1_{B}, ¹*J*_{CH}= 170.4 Hz), 94.0 (C-1_{E}, ¹*J*_{CH}= 163.1 Hz), 93.1 (CCl₃), 87.1 (C-3_{D}), 83.1 (C-3_{E}), 80.5 (C-3_{B}), 80.4 (C-4_{B}), 80.3 (C-2_{E}), 80.0 (C-4_{A}), 79.7 (2C, C-3_{B'}, C-4_{B'}), 79.4 (C-4_{C}), 78.7 (C-4_{E}), 77.7 (C-3_{C}), 76.3 (C_{Bn}), 75.5 (C-5_{D}), 75.4, 75.4, 75.3, 75.0, 74.9 (6C, C_{Bn}), 74.1 (C-2_{A}), 73.9 C-3_{A}), 73.5, 72.8 (2C, C_{Bn}), 72.0 (C-2_{C}), 71.7 (C_{Bn}), 71.3 (C-4_{D}), 71.0 (C-2_{B}), 70.0 (C-5_{E}), 69.1 (C-5_{C}), 68.8 (2C, C-2_{B'}, C-5_{A}), 68.4, 68.3 (C-5_{B}*, C-5_{B'}*), 67.8 (C-6_{E}), 67.6 (C_{All}), 62.7 (C-6_{D}), 55.3 (C-2_{D}), 21.2 (C_{Ac}), 18.0, 17.8 (4C, C-6_{A}*, C-6_{B}*, C-6_{B'*}, C-6_{C}*).

| | | |
|---|---|---|
| HRMS (ESI⁺) : | [M+Na]⁺ C₁₁₃H₁₂₈NO₂₈³⁵Cl₃Na | m/z théorique : 2074.7585 |
| | | m/z mesurée : 2074.7581 |
| | [M+NH₄]⁺ C₁₁₃H₁₂₈NO₂₈³⁵Cl₃NH₄ | m/z théorique : 2069.8032 |
| | | m/z mesurée : 2069.7925 |

### ➢ Propylα-L-rhamnopyranosyl-(1→3)-(2-O-acétyl-α-L-rhamnopyranosyl)-(1→3)-(2-acétamido-2-désoxy-β-D-glucopyranosyl)-(1→2)-[α-D-glucopyranosyl-(1→3)]-α-L-rhamnopyranosyl-(1→2)-α-L-rhamnopyranoside XIII :

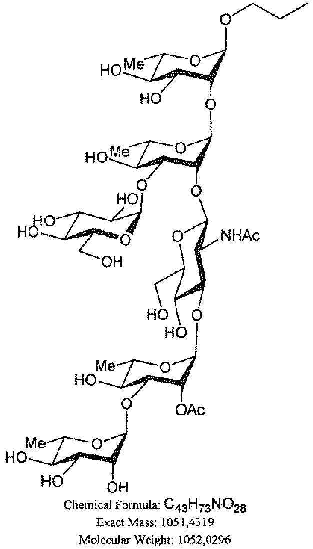

Du Pd-C 10% (400 mg) est ajouté à une solution dégazée de triol **124** (400 mg, 195 µmol) dans de l'éthanol absolu (20 mL). La suspension est saturée avec de l'hydrogène à 50 bar et agitée à TA pendant 10 j. Le suivi par CCM (iPrOH/H₂O/NH₃, 4/1/0,5 et Tol/EtOAc, 7/3) montre la disparition de **124** (Rf = 1 et 0,35, respectivement) et l'apparition d'un nouveau composé plus polaire (Rf = 0,65 et 0, respectivement). Le milieu réactionnel est filtré sur un filtre Acrodisc® LC 25 mm, et le filtrat est concentré à l'évaporateur rotatif. Le résidu obtenu est purifié sur colonne C-18 (H₂O/CH₃CN, 100/0→70/30) pour conduire à la cible ***XIII*** sous forme d'un solide blanc (131 mg, 64%).
Rf = 0,65 (iPrOH/H₂O/NH₃, 4/1/0,5)
RMN ¹H (D₂O), *δ* 5.10 (d, 1H, *J*_{1,2} = 3.7 Hz, H-1_{E}), 5.00 (d, 1H, *J*_{1,2} = 1.6 Hz, H-1_{A}), 4.91 (dd, 1H, *J*_{1,2} = 1.9 Hz, *J*_{2,3} = 3.1 Hz, H-2_{C}), 4.89 (d, 1H, *J*_{1,2} = 1.4 Hz, H-1_{B'}), 4.80 (bs, 2H, H-1_{B}, H-1_{C}), 4.71 (m, 1H, H-1_{D}), 4.35 (m, 1H, H-2_{A}), 4.02-3.92 (m, 3H, H-5_{C}, H-5_{E}, H-2_{B'}), 3.89-3.51 (m, 14H, H-3_{C}, H-2_{B}, H-3_{A}, H-6a_{D}, H-2_{D}, H-3_{B}, H-3_{E}, H-6a_{E}, H-6b_{E}, H-5_{A}, H-6b_{D}, H-2_{E}, H-5_{B}, H-3_{B'}, H_{Pr}), 3.49 (pt, 1H, *J*_{3,4} = 10.0 Hz, H-4_{C}), 3.47-3.30 (m, 8H, H-4_{D}, H_{Pr}, H-5_{B'}, H-4_{E}, H-4_{B}, H-4_{B'}, H-5_{D}, H-3_{D}), 3.26 (pt, 1H, *J*_{3,4} = 9.6 Hz, H-4_{A}), 2.08 (s, 3H, H_{Ac}), 2.03 (s, 3H, H_{NAc}), 1.50 (sex, 2H, CH₂), 1.22-1.13 (m, 12H, H-6_{B}, H-6_{A}, H-6_{B'}, H-6_{C}), 0.82 (t, 3H, *J*= 7.4 Hz, CH₃);
RMN ¹³C (D₂O), *δ* 174.4 (C_{NAc}), 173.2 (C_{Ac}), 102.7 (C-1_{B'}, ¹*J*_{CH} = 170.6 Hz), 101.4 (C-1_{D}, ¹*J*_{CH}= 166.0 Hz), 101.2 (C-1_{A}, ¹*J*_{CH}= 173.5 Hz), 98.4 (C-1_{C}, ¹*J*_{CH}= 167.8 Hz), 98.1 (C-1_{B'}, ¹*J*_{CH}= 171.5 Hz), 94.2 (C-1_{E}, ¹*J*_{CH}= 172.8 Hz), 82.6 (C-3_{D}), 79.1(C-2_{B}), 77.0 (C-3_{C}), 76.0 (C-5_{D}), 74.1 (C-2_{A}), 73.3 (C-3_{A}), 73.0 (C-3_{E}), 72.4 (C-2_{C}), 71.9 (C-4_{B}), 71.7 (C-4_{B'}), 71.3, 71.2, 71.1 (C-5_{E}, C-2_{E}, C-4_{C}), 70.7 (C-4_{A}), 70.1 (C-3_{B}), 69.9 (2C, C-2_{B'}, C-3_{B}), 69.7 (C_{Pr}), 69.4, 69.3 (3C, C-4_{E}, C-5_{A}, C-5_{B'}), 68.8 (C-5_{C}), 68.6 (C-5_{B}), 68.1 (C-4_{D}), 60.6 (C-6_{D}), 60.3 (C-6_{E}), 55.3 (C-2_{D}), 22.7 (C_{NAc}), 21.9 (CH₂), 20.2 (C_{Ac}), 16.8, 16.6, 16.5, 16.3 (C-6_{A}*, C-6_{B}*, C-6_{B'}*, C-6_{C}*), 9.8 (CH₃);

| | | |
|---|---|---|
| HRMS (ESI⁺) : | [M+H]⁺ C₄₃H₇₃NO₂₈ | m/z théorique : 1052.4397 |
| | | m/z mesurée : 1052.4360 |
| | [M+Na]⁺ C₄₃H₇₃NO₂₈Na | m/z théorique : 1074.4216 |
| | | m/z mesurée : 1074.4202 |

### Procédé 17 :

### ➢ Allyl (3,4-di-O-benzyl-2-α-L-rhamnopyranosyl)-(1→3)-4-O-benzyl-α-L-hamnopyranosyl)-(1→3)-(2-désoxy-2-trichloroacétamido-β-D-glucopyranosyl)-(1→2)-[2,3,4,6-tétra-O-benzyl-α-D-glucopyranosyl-(1→3)1-(4-O-benzyl-α-L-rhamnopyranosyl)-(1→2)-3,4-di-O-benzyl-α-L-rhamnopyranoside 125 :

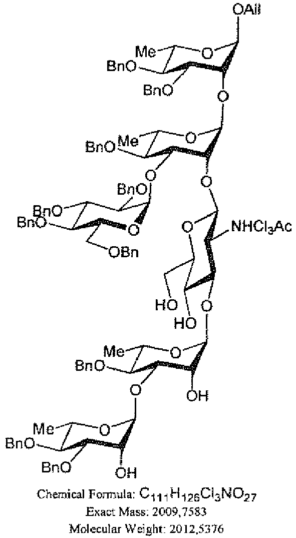

Après addition de NaOMe 0,5 M (745 µL, 370 µmol, 1 éq.) au diol **123** (800 mg, 370 µmol) en solution dans du MeOH (20 mL). Le milieu réactionnel est porté à 60°C et agité pendant 1 h, temps au bout duquel le suivi par CCM (Tol/EtOAc, 6/4), indique la disparition de **123** (Rf = 0,6) et l'apparition d'un produit plus polaire (Rf = 0,45). Après retour à TA, le milieu réactionnel est neutralisé par addition de résine échangeuse d'ion DOWEX (H⁺), puis filtré sur fritté. La résine est lavée avec un mélange DCM/MeOH et les solvants sont évaporés. L'huile jaune obtenue est purifiée par chromatographie sur gel de silice (Tol/EtOAc, 8/2→7/3) pour conduire au tétraol 125 sous forme d'un solide blanc (688 mg, 92%).
Rf = 0,45 (Tol/EtOAc, 6/4)
RMN ¹H (CDCl₃), *δ*7.44-7.05 (m, 50H, CH_{Ph}), 6.86 (d, 1H, *J*_{NH,2} = 8.6 Hz, NH), 5.89 (m, 1H, CH=), 5.36 (bs, 1H, H-1_{A}), 5.28 (m, 2H, =CH₂, H-1_{E}), 5.21 (m, 1H, *J*_{cis} = 10.4 Hz, =CH₂), 5.17 (d, 1H, *J*_{1,2} = 1.6 Hz, H-1_{B'}), 5.14-5.07 (m, 2H, H_{Bn}), 5.03-5.4.90 (m, 4H, H_{Bn}), 4.82 (d, 1H, *J*= 11.0 Hz, H_{Bn}), 4.74-4.51 (m, 13H, H-1_{B}, H_{Bn}), 4.47 (d, 1H, *J*_{1,2} = 7.0 Hz, H-1_{D}), 4.47 (d, 1H, *J*_{1,2} = 1.6 Hz, H-1_{C}), 4.38 (d, 1H, *J* = 11.9 Hz, H_{Bn}), 4.19 (dd, 1H, *J*_{2,3} = 2.4 Hz, H-2_{B}), 4.17-4.12 (m, 5H, H_{All}, H-5_{E}, H-3_{E}, H-3_{A}, H-2_{A}), 4.06-4.03 (m, 2H, H-3_{C}, H-2_{B'}), 3.99-3.82 (m, 9H, H_{All}, H-2_{C}, H-2_{D}, H-3_{B}, H-5_{C}, H-5_{B'}, H-4_{E}, H-2_{E}, H-3_{B'}), 3.78-3.64 (m, 3H, H-5_{B}, H-5_{A}, H-6a_{D}), 3.59-3.45 (m, 6H, H-4_{B'}, H-6a_{E}, H-6b_{E}, H-4_{B}, H-4_{A}, H-4_{C}), 3.07-3.03 (m, 2H, H-5_{D}, H-4_{D}), 2.94 (m, 1H, H-6b_{D}), 2.23 (m, 1H, H-3_{D}), 1.44 (d, 3H, *J*_{5,6}= 6.2 Hz, H-6_{B'}), 1.43 (d, 3H, *J*_{5,6} = 6.1 Hz, H-6_{A}), 1.40 (d, 3H, *J*_{5,6} = 6.2 Hz, H-6_{B}), 1.32 (d, 3H, *J*_{5,6} = 6.1 Hz, H-6_{C}).
RMN ¹³C (CDCl₃), *δ*161.9 (C_{NTCA}), 138.7-137.5 (C_{Ph}), 133.9 (CH=), 129.1-127.3 (CH_{Ph}), 117.1 (=CH₂), 101.0 (C-1_{D}, ¹*J*_{CH} = 162.7 Hz), 100.8 (C-1_{C}, ¹*J*_{CH}= 169.2 Hz), 100.1 (C-1_{A}, ¹*J*_{CH} = 180.3 Hz), 100.0 (C-1_{B'}, ¹*J*_{CH} = 180.3 Hz), 98.3 (C-1_{B'}, ¹*J*_{CH} = 170.3 Hz), 94.0 (C-1_{E}, ¹*J*_{CH} = 168.6 Hz), 93.2 (CCl₃), 86.5 (C-3_{D}), 83.0 (C-3_{E}), 80.5 (C-3_{B}), 80.4 (2C, C-2_{E}, C-4_{B}), 79.8, 79.7, 79.6 (4C, C-4_{A}*, C-3_{B}*, C-4_{B'}*, C-4_{C}*), 78.8 (C-4_{E}), 78.3 (C-3_{C}), 76.3 (C_{Bn}), 75.5 (C-5_{D}), 75.4, 75.3, 75.2, 74.9 (6C, C_{Bn}), 74.0 (C-3_{A}), 73.8 (C-2_{A}), 73.5, 72.8, 72.0 (3C, C_{Bn}), 71.3 (C-2_{B}), 71.1 (C-4_{D}), 70.4 (C-2_{C}), 70.0 (C-5_{E}), 68.9 (C-5_{C}), 68.8 (2C, C-5_{A}, C-2_{B'}), 68.7 (C-5_{B'}), 68.3 (C-5_{B}), 67.9 (C-6_{E}), 67.6 (C_{All}), 62.7 (C-6_{D}), 55.8 (C-2_{D}), 18.1, 18.0, 17.8 (4C, C-6_{A}*, C-6_{B}*, C-6_{B'}*, C-6_{C}*).

| | | |
|---|---|---|
| HRMS (ESI⁺) : | [M+Na]⁺ C₁₁₁H₁₂₆NO₂₇³⁵Cl₃Na | m/z théorique : 2032.7480 |
| | | m/z mesurée : 2032.7448 |

### ➢ Propyl α-L-rhamnopyranosyl-(1→3)-(α-L-rhamnopyranosyl)-(1→3)-(2-acétamido-2-désoxy-β-D-glucopyranosyl)-(1→2)-[α-D-glucopyranosyl-(1→3)]-α-L-rhamnopyranosyl-(1→2)-α-L-rhamnopyranoside XIV :

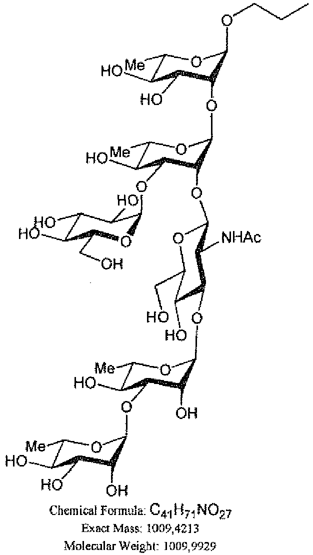

Du Pd-C 10% (340 mg) est ajouté à une solution dégazée de tétraol **125** (580 mg, 289 µmol) dans de l'éthanol absolu (15 mL). La suspension est saturée avec de l'hydrogène à 50 bar et agitée à TA pendant 10 jours. Le suivi par CCM (iPrOH/H₂O/NH₃, 4/1/0,5 et Tol/EtOAc, 6/4) montre la disparition de **125** (Rf = 1 et 0,45, respectivement) et l'apparition d'un nouveau composé plus polaire (Rf = 0.2 et 0, respectivement). Le milieu réactionnel est filtré sur un filtre Acrodisc LC 25 mm, et le filtrat est concentré à l'évaporateur rotatif. Le résidu obtenu est purifié sur colonne C-18 (H₂O/CH₃CN, 100/0→70/30) pour conduire à la cible ***XIV*** sous forme d'un solide blanc (240 mg, 82%).
Rf = 0,2 (iPrOH/H₂O/NH₃, 4/1/0,5)
RMN ¹H (D₂O), *δ* 5.20 (d, 1H, *J*_{1,2} = 3.7 Hz, H-1_{E}), 5.10 (d, 1H, *J*_{1,2} = 1.6 Hz, H-1_{A}), 5.01 (d, 1H, *J*_{1,2} = 1.2 Hz, H-1_{B'}), 4.90 (m, 1H, H-1_{B}), 4.84 (d, 1H, *J*_{1,2} = 1.9 Hz, H-1_{C}), 4.82 (d, 1H, *J*_{1,2} = 9.5 Hz, H-1_{D}), 4.44 (m, 1H, H-2_{A}), 4.06-3.99 (m, 3H, H-2_{B'}, H-5_{E}, H-5_{C}), 3.95-3.61 (m, 17H, H-2_{B}, H-3_{A}, H-6a_{D}, H-2_{C}, H-2_{D}, H-3_{B}, H-3_{E}, H-3_{B'}, H-6a_{E}, H-6b_{E}, H-3_{C}, H-5_{B}, H-5_{A}, H-6b_{D}, H-2_{E}, H-5_{B'}, H_{Pr}), 3.55-3.38 (m, 8H, H_{Pr}, H-4_{C}, H-4_{D}, H-4_{E}, H-3_{D}, H-4_{B}, H-4_{B'}, H-5_{D}), 3.35 (pt, 1H, *J*_{3,4} = 9.7 Hz, H-4_{A}), 2.11 (s, 3H, H_{NAc}), 1.59 (sex, 2H, CH₂), 1.31 (d, 3H, *J*_{5,6} = 6.3 Hz, H-6_{B}), 1.28 (d, 3H, *J*_{5,6} = 6.3 Hz, H-6_{B'}), 1.26 (d, 3H, *J*_{5,6} = 6.3 Hz, H-6_{A}), 1.23 (d, 3H, *J*_{5,6} = 6.3 Hz, H-6_{C}), 0.91 (t, 3H, *J* = 7.4 Hz, CH₃);
RMN ¹³C (D₂O), *δ* 174.0 (C_{NAc}), 102.4 (C-1_{B'}, ¹*J*_{CH} = 171.2 Hz), 101.5 (C-1_{D}, ¹*J*_{CH} = 163.6 Hz), 101.3 (C-1_{C}, ¹*J*_{CH} = 169.8Hz), 101.2 (C-1_{A}, ¹*J*_{CH} = 169.8 Hz), 98.2 (C-1_{B}, ¹*J*_{CH} = 170.5 Hz), 94.4 (C-1_{E}, ¹*J*_{CH} = 171.2 Hz), 81.7 (C-3_{D}), 79.1(C-2_{B}), 78.1 (C-3_{C}), 76.0 (C-5_{D}), 74.2 (C-2_{A}), 73.5 (C-3_{A}), 73.2 (C-3_{E}), 72.2 (C-4_{B}), 72.0 (C-4_{B'}), 71.4 (C-5_{E}), 71.3 (C-2_{E}) 71.3 (C-4_{C}), 70.8 (C-4_{A}), 70.5 (C-2_{C}), 70.2 (C-3_{B'}), 70.2 (C-2_{B'}), 70.0 (C-3_{B}), 69.8 (C_{Pr}), 69.5 (C-4_{E}), 69.4 (C-5_{A}), 69.0 (2C, C-5c, C-5_{B}), 68.7 (C-5_{B'}), 68.3 (C-4_{D}), 60.7 (C-6_{D}), 60.4 (C-6_{E}), 55.5 (C-2_{D}), 22.6 (C_{NAc}), 21.9 (CH₂), 16.8, 16.7, 16.5 (4C, C-6_{A}*, C-6_{B}*, C-6_{B}*, C-6_{C}*), 9.9 (CH₃);

| | | |
|---|---|---|
| HRMS (ESI⁺) : | [M+H]⁺ C₄₁H₇₁NO₂₇ | m/z théorique : 1010.4292 |
| | | m/z mesurée : 1010.4295 |
| | [M+Na]⁺ C₄₁H₇₁NO₂₇Na | m/z théorique : 1032.4111 |
| | | m/z mesurée : 1032.4116 |

### Partie V - Cibles de plus grandes tailles

**Tableau 14 : Oligosaccharides d'ordre supérieur représentatifs des fragments de l'antigène O de S. flexneri sérotype 3a et/ou X**

| Hexasaccharides | Octasaccharides | Décasaccharides | > |
|---|---|---|---|
| **Procédés 16 et 17 : (cf partie 4)** | **Procédés 18 et 19 :** | **Procédés 20 et 21 :** | |
| **B_{Ac}CD(E)AB *XVII*** | **D(E)AB_{Ac}CD(E)A *XIX*** | **B_{Ac}CD(E)AB_{Ac}CD(E)A *XXI*** | |
| **BCD(E)AB *XVIII*** | **D(E)ABCD(E)A *XX*** | **BCD(E)ABCD(E)A *XXII*** | |
| **Procédés 22 et 23 :** | **Procédés24 et 25 :** | **Procédés 26 et 27 :** | |
| **D(E)AB_{Ac}CD *XXIII*** | **B_{Ac}CD(E)AB_{Ac}CD *XXV*** | **D(E)AB_{Ac}CD(E)AB_{Ac}C *XXVII*** | |
| **D(E)ABCD *XXIV*** | **BCD(E)ABCD *XXVI*** | **D(E)AB_{Ac}CD(E)AB_{Ac}C *XXVIII*** | |

Une analyse rétro synthétique prenant en compte les fragments d'ordre supérieur des antigènes O de S. flexneri 3a et X, à savoir comportant 6 résidus au moins, a permis de sélectionner un nombre restreint de synthons communs dont la combinaison selon des séquences adaptées à chacune des cibles, permet d'accéder à l'ensemble des oligosaccharides d'intérêt. Comme indiqué plus haut, les synthons accepteurs peuvent être allongés à leur extrémité non réductrice par incorporation itérative des monosaccharides ou disaccharides donneurs requis fonctionnalisés de façon appropriée. En outre, les glycosides d'allyle peuvent être allongés à leur extrémité réductrice après déallylation sélective et activation, permettant leur couplage sur les monosaccharides ou disaccharides accepteurs requis pré-fonctionnalisés.

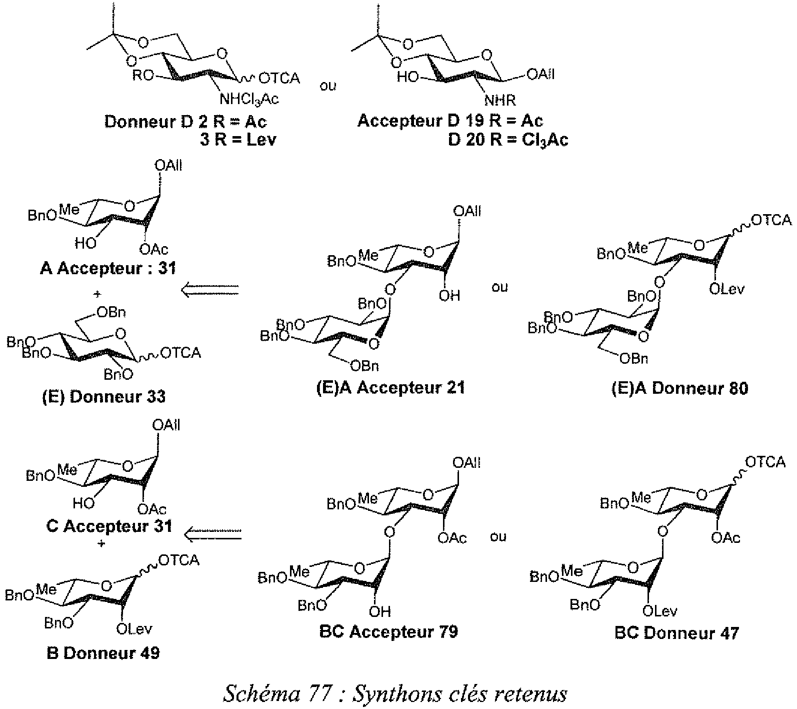

Plusieurs exemples de combinaison sont proposés ci-dessous. Les synthèses correspondantes sont en cours pour certains des procédés. Un octasaccharide complètement protégé a d'ores et déjà été synthétisé.

On remarquera également que l'emploi d'une fonction allyle anomérique ouvre la voie à des modifications ultérieures spécifiques de cette position, comme par exemple le greffage sur un support ou un carrier. Différentes méthodologies ont été proposées en ce sens dans la littérature.

### Procédés 18 et 19 : Cibles D(E)AB_{Ac}CD(E)A (XIX) et D(E)ABCD(E)A (XX)

La présente description se rapporte ainsi au procédé de préparation de l'octasaccharide D(E)AB_{Ac}CD(E)A (XIX), tel que défini dans la liste L1, caractérisé en ce qu'il comprend les étapes suivantes :
- condensation du pentasaccharide accepteur **66** et du disaccharide donneur **80** conduisant à l'heptasaccharide accepteur **126** ;
- délévulinoylation de l'heptasaccharide **126** conduisant à l'heptasaccharide **127** de préférence dans des conditions conventionnelles de déprotection sélective des lévulinates, par exemple en présence d'hydrazine monohydratée ;
- condensation de l'heptasaccharide accepteur **127** et du monosaccharide donneur **3** conduisant à l'octasaccharide **128,** de préférence dans un solvant tel que le dichlorométhane, le 1,2-dichloroéthane, le toluène, l'acétonitrile ou leur équivalents utilisés usuellement dans l'industrie en présence d'un acide de Lewis tel que le TMSOTf, l'acide triflique, le trifluorométhane sulfonate de triméthylsilyle ;
- délévulinoylation de l'octasaccharide **128** conduisant à l'octasaccharide accepteur **128a** ;
- déprotection en deux étapes, incluant l'hydrolyse acide des fonctions isopropylidène puis la débenzylation et la réduction des fonction trichloroacétamides de préférence sous atmosphère d'hydrogène en présence de palladium de l'octasaccharide **128a** conduisant à l'octasaccharide D(E)AB_{Ac}CD(E)A (schéma 78).

La description se rapporte également au procédé de préparation de l'octasaccharide D(E)ABCD(E)A) (XX), tel que défini dans la liste L1, caractérisé en ce qu'il comprend les étapes suivantes :
- condensation du pentasaccharide accepteur **66** et du disaccharide donneur **80** conduisant à l'heptasaccharide accepteur **126** ;
- délévulinoylation de l'heptasaccharide **126** conduisant à l'heptasaccharide **127** de préférence dans des conditions conventionnelles de déprotection sélective des lévulinates, par exemple en présence d'hydrazine monohydratée ;
- condensation de l'heptasaccharide accepteur **127** et du monosaccharide donneur **3** conduisant à l'octasaccharide **128,** de préférence en présence d'un acide de Lewis tel que le TMSOTf, l'acide triflique, le trifluorométhane sulfonate de triméthylsilyle ;
- transestérification de l'octasaccharide **128** puis hydrolyse acide des isopropylidènes et débenzylation et réduction des fonctions trichloroacétamides conduisant à l'octasaccharide D(E)ABCD(E)A de préférence par traitement sous pression d'hydrogène en présence de palladium sur charbon (schéma 78).

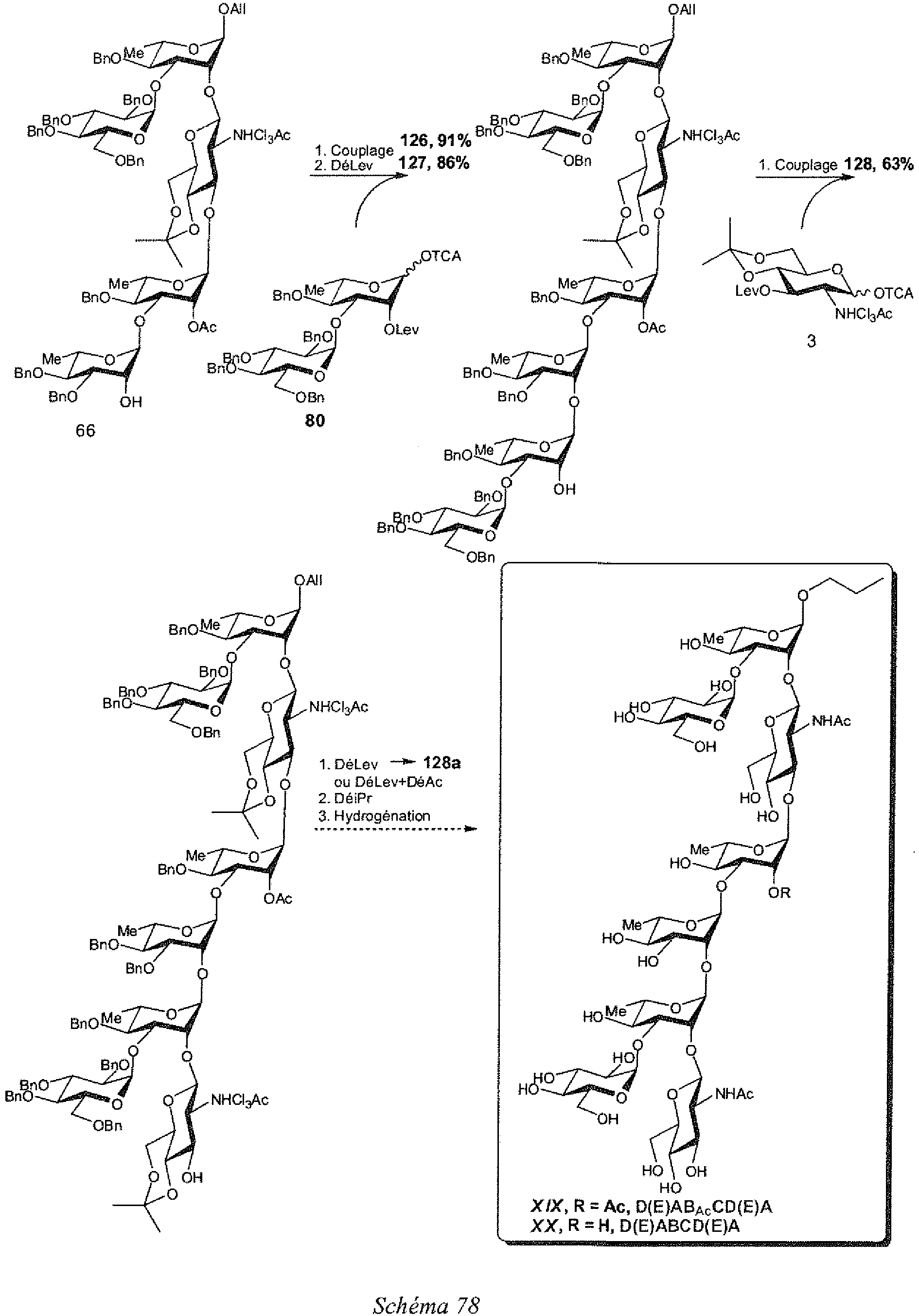

### Mise en oeuvre expérimentale des procédés 18 et 19

### Procédé 18 :

### ➢ Allyl (2,3,4,6-tétra-O-benzyl-α-D-glucopyranosyl)-(1→3)-(4-O-benzyl-2-O-évulinoyl-α-L-rhamnopyranosyl)-(1→2)-(3,4-di-O-benzyl-α-L-rhamnopyranosyl)-(1→3)-(2-O-acétyl-4-O-benzyl-α-L-rhamnopyranosyl)-(1→3)-(2-désoxy-4,6-O-isopropylidène-2-trichloroacétamido-β-D-glucopyranosyl)-(1→2)-[2,3,4,6-tétra-O-benzyl-α-D-glucopyranosyl-(1→3)]-4-O-benzyl-α-L-rhamnopyranoside 126 :

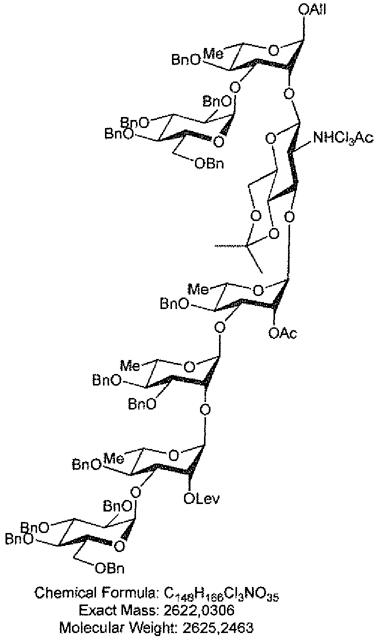

Du TMSOTf (14,0 µL, 75,0 µmol, 0,3 éq.) est additionné à une solution d'accepteur **66** (440 mg, 248 µmol) et de donneur **80** (380 mg, 370 µmol, 1,5 éq.) dans du Tol (6 mL), en présence de tamis moléculaire 4 Å (236 mg), agitée sous argon à -5°C. Après 20 min. d'agitation, le suivi par CCM (Tol/EtOAc, 8/2) indique la disparition de **66** (Rf = 0,15) et l'apparition d'un nouveau composé moins polaire (Rf = 0,4). La réaction est stoppée par addition de triéthylamine (0,5 mL) puis le milieu réactionnel est filtré et concentré à l'évaporateur rotatif. Le brut obtenu (248 µmol) est dissous dans du DMF (10 mL) contenant du 2-méthoxypropène (48,0 µL, 498 µmol, 2 éq.). Du CSA (480 mg, 2,0 mmol, 8 éq.) est ajouté par fractions au milieu réactionnel jusqu'à obtenir un pH de 2. Le milieu réactionnel est agité à TA et la réaction est suivie par CCM (Tol/EtOAc, 8/2). Après 2 h d'agitation, le MeOH formé est évaporé à l'évaporateur rotatif et du 2-méthoxypropène (24,0 µL, 250 µmol, 1 éq.) est de nouveau ajouté. Après 1 h d'agitation, le milieu réactionnel est neutralisé avec de la triéthylamine (0,2 mL) puis concentré à l'évaporateur rotatif. L'huile jaune obtenue est purifiée par chromatographie sur gel de silice (Tol/EtOAc, 9/1→7/3) pour obtenir le glycoside d'allyle **126** sous forme d'un solide blanc (596 mg, 91%).
Rf = 0,4 (Tol/ EtOAc, 8/2).
RMN ¹H (CDCl₃), *δ* Ok
RMN ¹³C (CDCl₃), *δ* Ok

| | | |
|---|---|---|
| HRMS (ESI⁺) : | [M+Na]⁺ pas visible | m/z théorique : |
| | | m/z mesurée : |

### ➢ Allyl (2,3,4,6-tétra-O-benzyl-α-D-glucopyranosyl)-(1→3)-(4-O-benzyl-α-L-rhamnopyranosyl)-(1→2)-(3,4-di-O-benzyl-α-L-rhamnopyranosyl)-(1→3)-(2-O-acétyl-4-O-benzyl-α-L-rhamnopyranosyl)-(1-+3)-(2-désoxy-4,6-O-isopropylidène-2-trichloroacétamido-β-D-glucopyranosyl)-(1→2)-[2,3,4,6-tétra-O-benzyl-α-D-glucopyranosyl-(1→3)]--4-O-benzyl-α-L-rhamnopyranoside 127 :

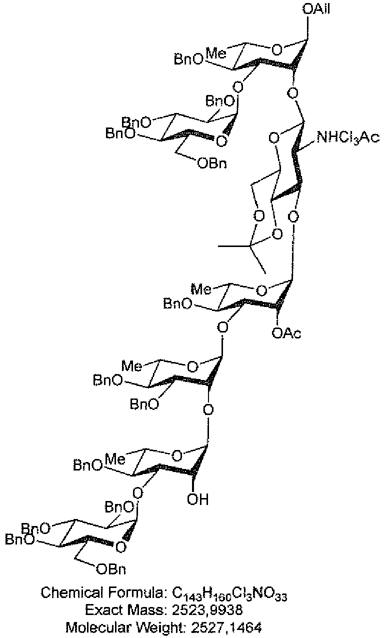

Le glycoside d'allyle **126** (1,23 g, 468 µmοl) est dissous dans un mélange pyridine/acide acétique (3/2, 10 mL). De l'hydrazine monohydraté (227 µL, 4,7 mmol, 10 éq.) est ajouté goutte à goutte au milieu réactionnel. Après 35 min d'agitation à 0°C, le suivi par CCM (Tol/EtOAc, 7/3) indique l'apparition d'un produit légèrement moins polaire (Rf = 0,6). Le milieu réactionnel est alors repris à l'eau froide (20 mL) et la phase aqueuse est extraite rapidement au DCM (3 x 80 mL). Les phases organiques sont regroupées et lavées avec une solution de NaCl sat, filtrées sur filtre séparateur de phase et concentrées à l'évaporateur rotatif. L'huile obtenue est purifiée par chromatographie sur gel de silice (Tol/EtOAc, 9/1→8/2) pour obtenir l'alcool **127** sous forme d'un solide blanc (1,05 g, 87%).
Rf = 0,6 (Tol/ EtOAc, 7/3).
RMN ¹R (CDCl₃), *δ* Ok
RMN ¹³C (CDCl₃), *δ* Ok

| | | |
|---|---|---|
| HRMS (ESI⁺) : | [M+ NH₄]⁺ C₁₄₃H₁₆₀NO₃₃³⁵Cl₃NH₄ | m/z théorique : 2542.0281 |
| | | m/z mesurée : 2542.0186 |

### ➢ Allyl (2-désoxy-4,6-O-isopropylidène-3-O-lévulinoyl-2-trichloroacétamido-β-D-glucopyranose)-(1→2)-[2,3,4,6-tétra-O-benzyl-α-D-glucopyranosyl-(1→3)]-(4-O-benzyl-α-L-rhamnopyranosyl)-(1→2)-(3,4-di-O-benzyl-α-L-rhamnopyranosyl)-(1→3)-(2-O-acétyl-4-O-benzyl-α-L-rhamnopyranosyl)-(1→3)-(2-désoxy-4,6-O-isopropylidène-2-trichloroacétamido-β-D-glucopyranosyl)-(12)-[2,3,4,6-tétra-O-benzyl-α-D-glucopyranosyl-(1→3)]-4-O-benzyl-α-L-rhamnopyranoside 128 :

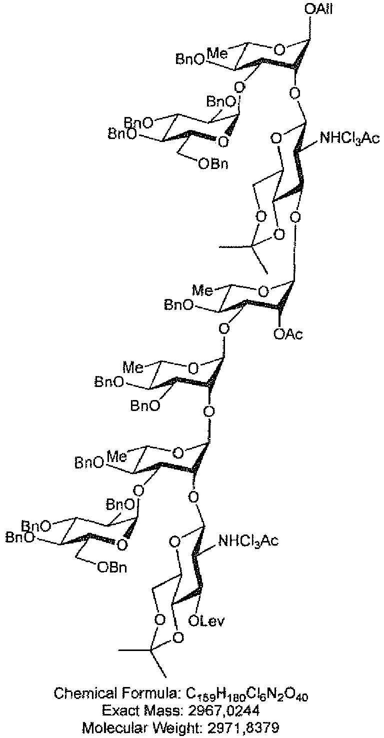

Du TMSOTf (12,5 µL, 70,0 µmol, 0,35 éq.) est additionné à une solution d'accepteur **127** (455 mg, 180 µmol) et de donneur **3** (380 mg, 314 µmol, 1,7 éq.) dans du Tol (8 mL), en présence de tamis moléculaire 4 Å (173 mg), agitée sous argon à 0°C. Après 20 min. d'agitation, le suivi par CCM (Tol/EtOAc, 7/3) indique la disparition de **127** (Rf = 0,6) et l'apparition d'un nouveau composé moins polaire (Rf = 0,5). La réaction est stoppée par addition de triéthylamine (0,5 mL) puis le milieu réactionnel est filtré et concentré à l'évaporateur rotatif. Le brut obtenu (180 µmol) est dissous dans du DMF (8 mL) contenant du 2-méthoxypropène (35,0 µL, 360 µmοl, 2 éq.). Du CSA (250 mg, 1,1 mmol, 6 éq.) est ajouté par fractions au milieu réactionnel jusqu'à obtenir un pH de 2. Le milieu réactionnel est agité à TA et la réaction est suivie par CCM (Tol/EtOAc, 7/3). Après 2 h d'agitation, le MeOH formé est évaporé à l'évaporateur rotatif et du 2-méthoxypropène (17,5 µL, 180 µmοl, 1 éq.) est de nouveau ajouté. Après 1 h d'agitation, le milieu réactionnel est neutralisé avec de la triéthylamine (0,2 mL) puis concentré à l'évaporateur rotatif. L'huile jaune obtenue est purifiée par chromatographie sur gel de silice (Tol/EtOAc, 9/1→75/25) pour obtenir le glycoside d'allyle **128** sous forme d'un solide blanc (340 mg, 63%).
Rf = 0,5 (Tol/ EtOAc, 7/3).
RMN ¹H (CDCl₃), *δ* Ok
RMN ¹³C (CDCl₃), *δ* Ok

| | | |
|---|---|---|
| HRMS (ESI⁺) : | [M+Na]⁺ pas visible | m/z théorique : |
| | | m/z mesurée : |

### Procédés 20 et 21 : Cibles B_{Ac}CD(E)AB_{Ac}CD(E)A (XXI) et BCD(E)ABCD(E)A (XXII)

La description se rapporte en outre au procédé de préparation du décasaccharide B_{Ac}CD(E)AB_{Ac}CD(E)A (XXI), tel que défini dans la liste L1, caractérisé en ce qu'il comprend les étapes suivantes :
- condensation de l'octasaccharide accepteur **128** et du disaccharide donneur **47** conduisant au décasaccharide **129** ;
- délévulinoylation du décasaccharide **129** conduisant au décasaccharide **130** ;
- clivage du groupement isopropylidène, notamment par hydrolyse acide, du décasaccharide **130** conduisant au décasaccharide **132** ;
- déprotection par hydrogénolyse du décasaccharide **132** conduisant au décasaccharide B_{Ac}CD(E)AB_{Ac}CD(E)A de préférence par traitement sous pression d'hydrogène en présence de palladium sur charbon (schéma 79).

La description se rapporte encore au procédé de préparation du décasaccharide BCD(E)ABCD(E)A (XXII), tel que défini dans la lsite L1, caractérisé en ce qu'il comprend les étapes suivantes :
- condensation de l'octasaccharide accepteur **128** et du disaccharide donneur **47** conduisant au décasaccharide **129** ;
- délévulinoylation et désacétylation simultanées par transestérification du décasaccharide **129** conduisant au décasaccharide **131** ;
- clivage du groupement isopropylène, notamment par hydrolyse acide, du décasaccharide **131** conduisant au décasaccharide **133** ;
- déprotection du décasaccharide **133** conduisant au décasaccharide BCD(E)ABCD(E)A de préférence par traitement sous pression d'hydrogène en présence de palladium sur charbon (schéma 79).

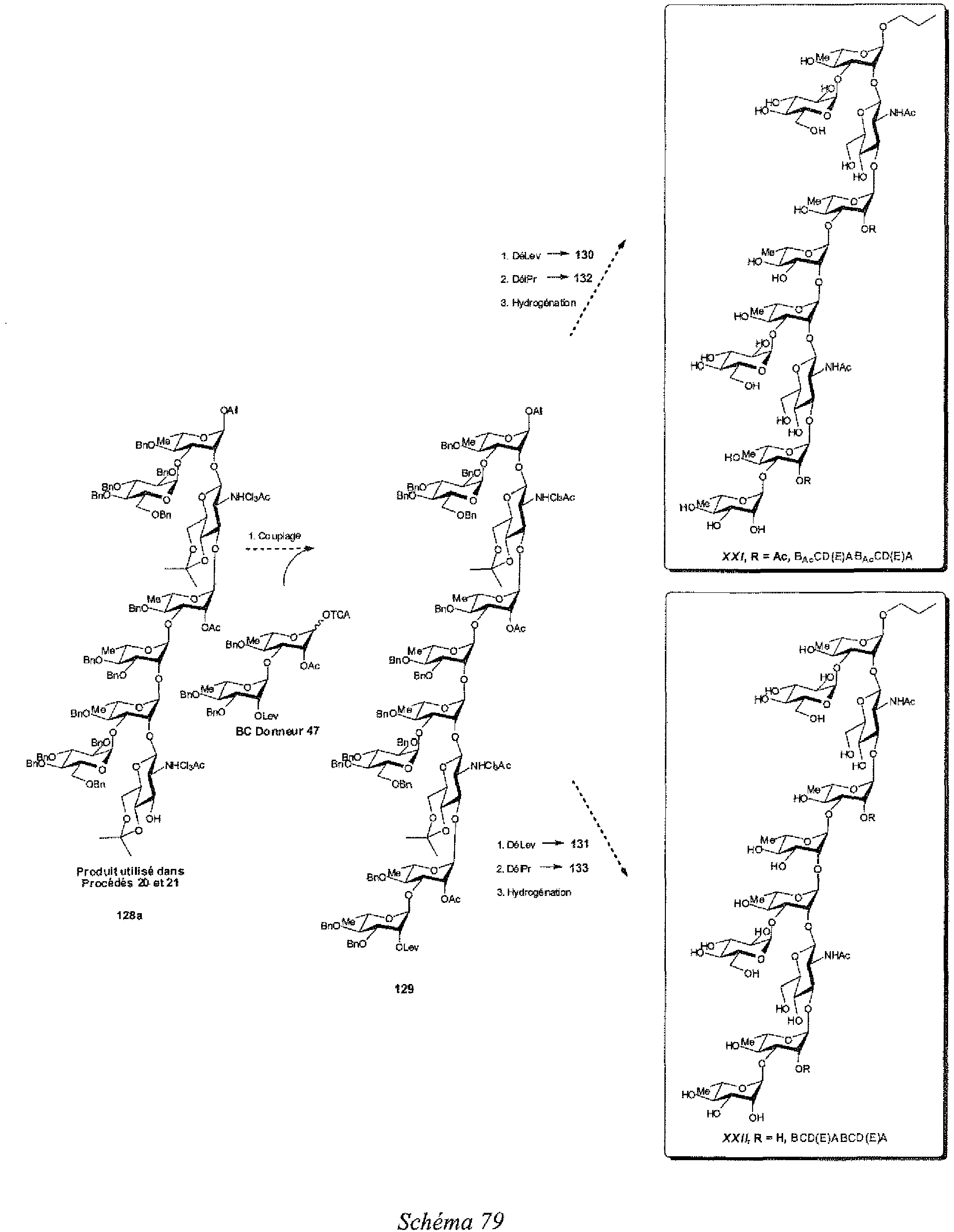

### Procédés 22 et 23 : Cibles D(E)AB_{Ac}CD (XXIII)_et D(E)ABCD (XXIV)

La description se rapporte au procédé de préparation de l'hexasaccharide D(E)AB_{Ac}CD (XXIII), tel que défini dans la liste L1, caractérisé en ce qu'il comprend les étapes suivantes :
- condensation du monosaccharide accepteur **20** et du tétrasaccharide donneur **86** conduisant au pentasaccharide accepteur **134** ;
- délévulinoylation du pentasaccharide **134** conduisant au pentasaccharide **135** ;
- condensation du pentasaccharide accepteur **135** et du monosaccharide donneur **3** conduisant à l'hexasaccharide **136** ;
- delévulinoylation de l'hexasaccharide **136** conduisant à l'hexasaccharide **136a** ;
- déprotection en deux étapes de l'hexasaccharide **136a** incluant une étape d'hydrolyse acide des fonctions isopropylidènse puis la débenzylation et la réduction concomitante des fonctions trichloroacétamides conduisant à l'hexasaccharide D(E)AB_{Ac}CD de préférence par traitement sous pression d'hydrogène en présence de palladium sur charbon (schéma 80).

La description se rapporte aussi au procédé de préparation de l'hexasaccharide D(E)ABCD (XXIV), tel que défini dans la lsite L1, caractérisé en ce qu'il comprend les étapes suivantes :
- condensation du monosaccharide accepteur **20** et du tétrasaccharide donneur **86** conduisant au pentasaccharide **134** ;
- délévulinoylation du pentasaccharide **134** conduisant au pentasaccharide accepteur **135** ;
- condensation du pentasaccharide accepteur **135** et du monosaccharide donneur **3** conduisant à l'hexasaccharide **136** ;
- transestérification de l'hexasaccharide **136** puis hydrolyse acide des fonctions isopropylidènes suivie de l'hydrogénolyse des fonctions benzyles et de la réduction concomitante des fonctions trichloroacétamides conduisant à l'hexasaccharide D(E)ABCD de préférence sous pression d'hydrogène en présence de palladium sur charbon (schéma 80).

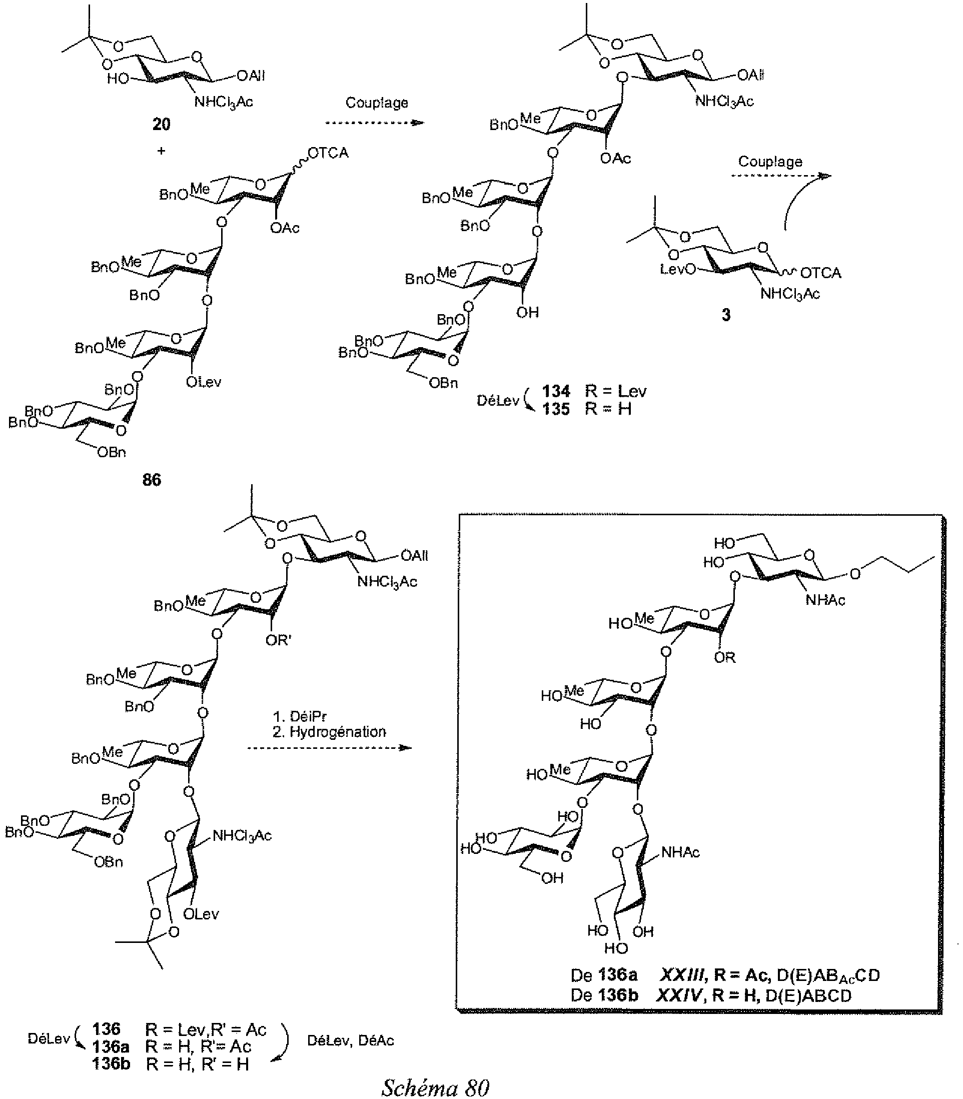

### Procédés 24 et 25 : Cibles B_{Ac}CD(E)AB_{Ac}CD (XXV) et BCD(E)ABCD (XXVI)

La description se rapporte au procédé de préparation de l'octasaccharide B_{ac}CD(E)AB_{Ac}CD (XXV), tel que défini dans la lsite L1, caractérisé en ce qu'il comprend les étapes suivantes :
- condensation de l'hexasaccharide accepteur **136** et du disaccharide donneur **47** conduisant à l'octasaccharide **137** ;
- délévulinoylation de l'octasaccharide **137** conduisant à l'octasaccharide accepteur **137a** ;
- déprotection en deux étapes de l'hexasaccharide **137a** incluant une étape d'hydrolyse acide des fonctions isopropylidènse puis la débenzylation et la réduction concomitante des fonctions trichloroacétamides conduisant à l'octasaccharide B_{ac}CD(E)AB_{Ac}CD de préférence par traitement sous pression d'hydrogène en présence de palladium sur charbon (schéma 81).

La description se rapporte au procédé de préparation de l'octasaccharide BCD(E)ABCD (XXVI), tel que défini aux revendications 1, 12 ou 13, caractérisé en ce qu'il comprend les étapes suivantes :
- condensation de l'hexasaccharide accepteur **136** et du disaccharide donneur **47** conduisant à l'octasaccharide **137** ;
- transestérification de l'octasaccharide **137** puis hydrolyse acide des fonctions isopropylidènes suivie de l'hydrogénolyse des fonctions benzyles et de la réduction concomitante des fonctions trichloroacétamides conduisant à l'octasaccharide BCD(E)ABCD de préférence sous pression d'hydrogène en présence de palladium sur charbon (schéma 81).

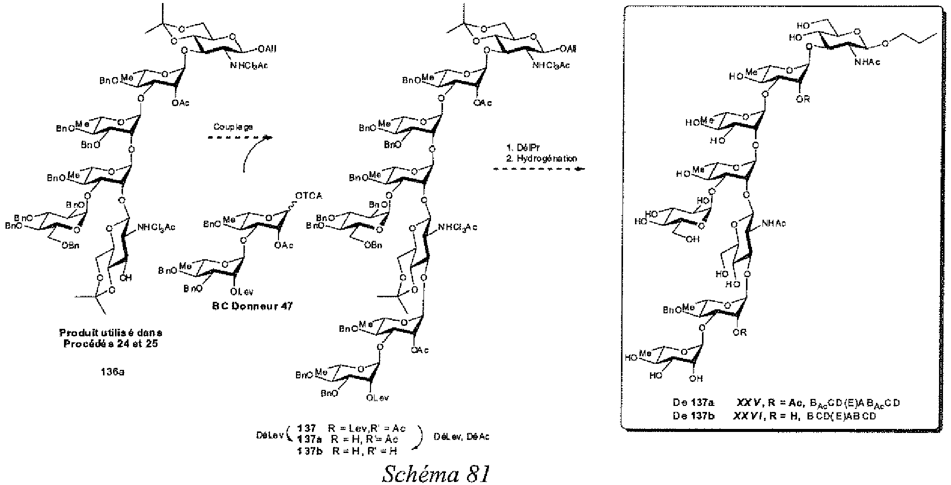

### Procédés 26 et 27 : Cibles D(E)AB_{Ac}CD(E)AB_{Ac}C (XXVII) et D(E)ABCD(E)ABC (XXVIII)

La description se rapporte au procédé de préparation du décasaccharide D(E)AB_{Ac}CD(E)AB_{Ac}C (XXVII), tel que défini dans la liste L1, caractérisé en ce qu'il comprend les étapes suivantes :
- condensation du tétrasaccharide accepteur **85** et du monosaccharide donneur 3 conduisant au pentasaccharide **138a** ;
- délévulinoylation du pentasaccharide **138a** conduisant au pentasaccharide accepteur **138b** ;
- condensation du pentasaccharide accepteur **138b** et du disaccharide donneur 47 conduisant à l'heptasaccharide **139a** ;
- délévulinoylation de l'heptasaccharide **139a** conduisant à l'heptasaccharide **139b** ;
- condensation de l'heptasaccharide accepteur **139b** et du disaccharide donneur 80 conduisant au nonasaccharide **140a** ;
- délévulinoylation du nonasaccharide **140a** conduisant au nonasaccharide accepteur **140b** ;
- condensation du nonasaccharide accepteur **140b** et du monosaccharide donneur **3** conduisant au décasaccharide **141a** ;
- délévulinoylation du décasaccharide **141a** conduisant au décasaccharide accepteur **141b** ;
- déprotection en deux étapes du décasaccharide **141b** incluant une étape d'hydrolyse acide des fonctions isopropylidènes puis la débenzylation et la réduction concomitante des fonctions trichloroacétamides conduisant au décasaccharide D(E)AB_{Ac}CD(E)AB_{Ac}C de préférence par traitement sous pression d'hydrogène en présence de palladium sur charbon (schéma 82).

La description se rapporte au procédé de préparation du décasaccharide D(E)ABCD(E)ABC (XXVIII), tel que défini dans la liste L1, caractérisé en ce qu'il comprend les étapes suivantes :
- condensation du tétrasaccharide accepteur **85** et du monosaccharide donneur **3** conduisant au pentasaccharide **138a** ;
- délévulinoylation du pentasaccharide accepteur **138a** conduisant au pentasaccharide accepteur **138b** ;
- condensation du pentasaccharide **138b** et du disaccharide donneur **47** conduisant à l'heptasaccharide **139a** ;
- délévulinoylation de l'heptasaccharide **139a** conduisant à l'heptasaccharide accepteur **139b** ;
- condensation de l'heptasaccharide accepteur **139b** et du disaccharide donneur **80** conduisant au nonasaccharide **140** ;
- délévulinoylation du nonasaccharide **140a** conduisant au nonasaccharide accepteur **140b** ;
- condensation du nonasaccharide accepteur **140b** et du monosaccharide donneur **3** conduisant au décasaccharide **141a** ;
- déprotection en deux étapes du décasaccharide **141a** incluant une étape de transestérification, une étape d'hydrolyse acide des fonctions isopropylidènes puis la débenzylation et la réduction concomitante des fonctions trichloroacétamides conduisant au décasaccharide D(E)ABCD(E)ABC de préférence par traitement sous pression d'hydrogène en présence de palladium sur charbon (schéma 82).

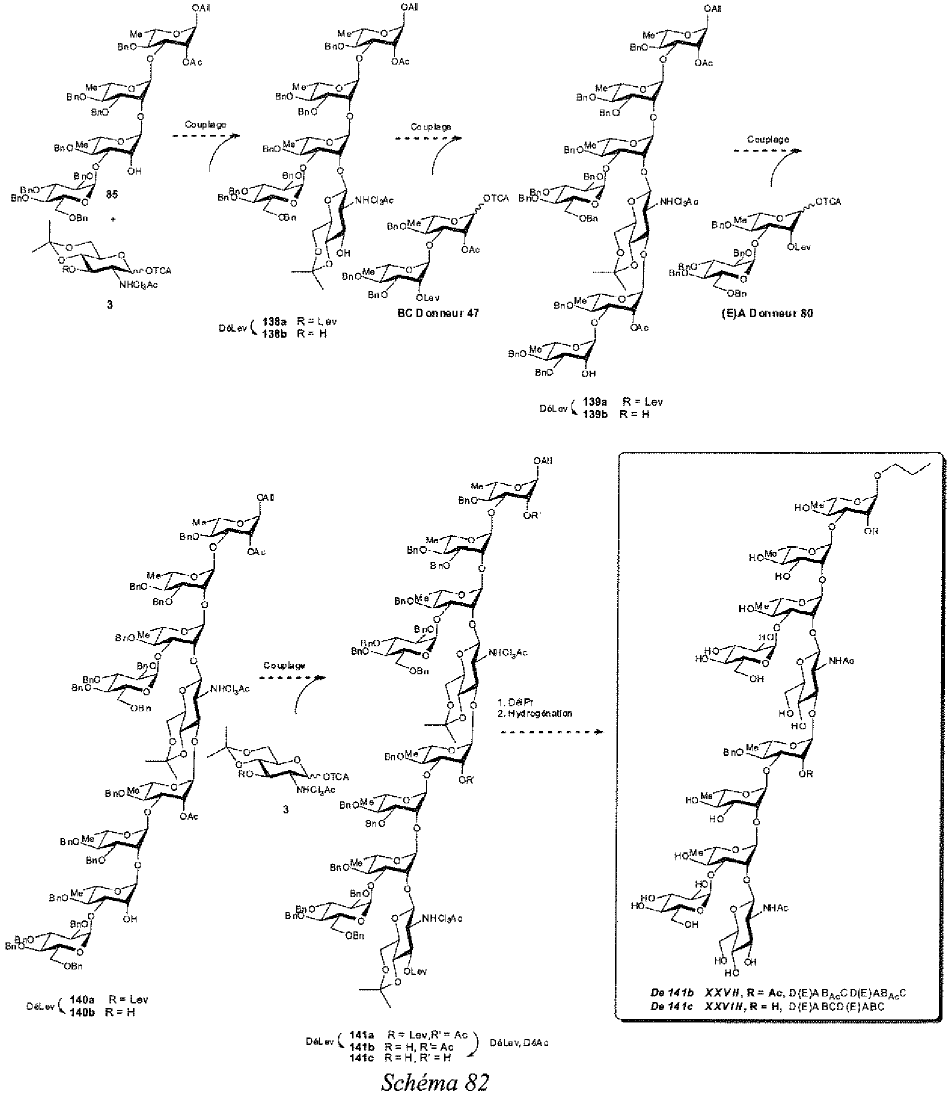

### Exemple de mise en évidence de l'activité biologique des saccharides selon l'invention

### 1. Obtention des anticorps monoclonaux (mAbs)

Protocole 1) Quatre souris BP2 ont reçu par voie intrapéritonéale (i.p.) 5x10⁶ bactéries vivantes de la souche *S. flexneri* 3a toutes les 3 semaines. Après 8 injections, la souris ayant développé la meilleure réponse anticorps contre le LPS de *S. flexneri* 3a, a reçu une injection de rappel 3 jours avant d'être sacrifiée pour réaliser la fusion cellulaire avec les splénocytes issus de la rate. Deux mAbs de type IgG (**G17-12 and G17-37**) reconnaissant spécifiquement le LPS de *S. flexneri* 3a ont été obtenus et caractérisés.

Protocole 2) Dix souris Balb/C et quatre souris BP2 ont été immunisées par injection en i.p. avec des bactéries vivantes virulentes de la souche *S. flexneri* 3a, toutes les trois semaines, en utilisant 2x10⁶ bactéries pour les 2 premières immunisations et 5x10⁶ pour les 4 suivantes. Comme la réponse anticorps anti-LPS 3a était trop faible pour réaliser une fusion cellulaire une série supplémentaire de 4 injections a été réalisée. Deux mAbs de type IgM, **G19-2 et L18-9**, ont été obtenus.

### 2. Caractérisation des déterminants oligosaccharidiques reconnus par les mAbs.

La caractérisation des déterminants oligosaccharidiques reconnus par les quatre mAbs obtenus a été réalisée en mesurant par ELISA d'inhibition l'interaction des différents oligosaccharides synthétisés avec ces mAbs.
1) Tout d'abord, une courbe standard DO = f (concentration du mAb) a été établie pour chaque anticorps.
   Pour cela, du LPS purifié à partir de *S. flexneri* 3a a été fixé dans les puits de plaques ELISA (plaque à 96 puits), à une concentration de 25 µg/ml dans un tampon carbonate pH 9.6, à 4°C, pendant la nuit. Après lavage des puits avec une solution de PBS/ Tween 20 (0.05%) 1% et une étape de saturation avec PBS/BSA 1% pendant 30 minutes à 4°C, différentes concentrations de chaque mAb ont été incubées 30 minutes à 4°C. Après une étape de lavage, un anticorps secondaire couplé à la phosphatase alcaline et spécifique des IgG ou des IgM de souris a été ajouté à une dilution de 1/5000 (Sigma- Aldrich) pendant 1h à 37°C. Après lavage, la réaction enzymatique a été effectuée avec le substrat spécifique de la phosphatase alcaline (12 mg de *p-*nitrophénylphosphate dans 1.2 ml de tampon Tris-HCl buffer 1M (pH 8.8) et 10.8 ml de NaCl 5M). Une fois la réaction colorée développée, les plaques ont été lues à 405 nm (lecteur de plaque Dynatech MR 4000). La courbe standard OD = *f*(Ab concentration) a été affinée avec l'équation *Y* = *aX*2 +*bX* +*c,* où Y correspond à la DO et *X* à la concentration en anticorps. Un facteur de corrélation (r2) de 0.99 a été généralement obtenu.
2) La concentration en oligosaccharide capable d'inhiber 50% de la fixation du mAb sur le LPS 3a a été ensuite déterminée comme suit.
   Chaque mAb a été utilisé à une concentration donnée, celle définie comme la première concentration testée donnant une DO maximum sur la courbe standard (le premier point au début du plateau de la courbe). Différentes concentrations de chacun des oligosaccharides à tester ont ensuite été incubées séparément avec le mAb à concentration constante, pendant la nuit à 4°C, dans le tampon PBS-BSA 1%. La mesure de la concentration d'anticorps libre a été réalisée comme mentionné ci-dessus, après incubation des mélanges mAb/oligosaccharides à 4°C pendant 30 minutes sur des plaques recouvertes de LPS 3a et en utilisant la courbe standard réalisée en parallèle sur la même plaque. Pour chaque mAb et chaque oligosaccharide, une valeur appelée IC₅₀ a ensuite été calculée correspondant à la concentration en oligosaccharide permettant d'inhiber 50% de la fixation du mAb sur le LPS 3a.

Les tableaux suivants résument les résultats obtenus pour les oligosaccharides disponibles à ce jour en interaction avec les deux mIgG et un des deux mAb IgM.

Par ailleurs, l'IC₅₀ caractérisant l'interaction de ces anticorps avec le LPS 3a a été également défini. Les valeurs sont 19 ng/ml pour L18-9 (mIgM) ; 39 ng/ml pour G19-2 (mlgM) ; et 450 ng/ml pour G17-12 et G17-37 (mIgG). Il est important de noter qu'ici la valeur est exprimée en ng/ml car il est très difficile d'évaluer la masse moléculaire du LPS ne connaissant pas précisément la longueur des chaînes et donc le nombre de répétitions du motif oligosaccharide de base.

**Tableau 1 : mIgG C7-37 et mIgG G7-12**

| **Saccharide** | **Nom abrégé** | **IC50 avec mIgG C7-37** | **IC50 avec mIgG G7-12** |
|---|---|---|---|
| E)A-OPr | A | >5mM | >2,5mM |
| E)AB-OPr | B | >5mM | >2,5mM |
| D(E)A-OPr | C | >5mM | >2,5mM |
| D(E)A-OMethyl | D | >5mM | >2,5mM |
| D(E)AB-OPr | I | >5mM | >2,5mM |
| C(2,3-OAc)D(E)AB-OPr | E | >5mM | >2,5mM |
| C(OH)D(E)A-OPr | F | >5mM | >2,5mM |
| C(2-OAc)D(E)A-OPr | J | >5mM | >2,5mM |
| EABC(2-OAc)-OPr | G | 0,41 mM | 0,45 mM |
| EABC-Opr | H | >5mM | >2,5mM |
| D(E)ABC(2-OAc )-OPr | K | +/- 4mM | >2,5mM |
| D(E)ABC)-OPr | L | >5mM | >2,5mM |
| (E)ABC(2-OAc)D-OPr | M | 0,28mM | 0,315mM |
| (E)ABCD-OPr | N | > 1mM | >2,5mM |
| BCD(E)A-Opr-Oac | O | 0,195mM | 0,175 mM |
| BCD(E)A-Opr | P | > 1mM | >2,5mM |
| BCD(E)AB-Opr | Q | > 5mM | >2,5mM |
| BC(2-OAc)D(E)AB-OPr | R | 0,23mM | 0,205 mM |

Il est important de noter : (i) qu'il n'y a aucune différence entre ces deux anticorps pour la reconnaissance des oligosaccharides. Comme ils sont issus d'une même dilution limite, il est fort probable qu'il s'agit en fait d'un seul et même mAb ; (ii) ces résultats mettent en évidence le rôle crucial joué par l'acétylation du résidu C dans la reconnaissance.

**Tableau 2 : IgM (G19-2)**

| | | |
|---|---|---|
| (E)A-OPr | **A** | >2,5mM |
| (E)AB-OPr | **B** | >2,5mM |
| **D(E)A-OPr** | **C** | >2,5mM |
| **D(E)A-OMethyl** | **D** | >2,5mM |
| **D(E)AB-OPr** | **I** | >2,5mM |
| **C(2,3-OAc)D(E)AB-OPr** | **E** | >2,5mM |
| **C(OH)D(E)A-OPr** | **F** | >2,5mM |
| **C(2-OAc)D(E)A-OPr** | **J** | >2,5mM |
| **EAB(2-OAc)-OPr** | **G** | **1,43 mM** |
| **EABC-Opr** | **H** | >2,5mM |
| **D(F)ABC(2-OAc)-OPr** | **K** | >2,5mM |
| **D(E)ABC)-OPr** | **L** | >2,5mM |
| **(E)ABC(2-OAc)D-OPr** | **M** | **1,28 mM** |
| **(E)ABCD-OPr** | **N** | >2,5mM |
| **BCD(E)A-Opr-Oac** | **O** | **1,98mM** |
| **BCD(E)A-Opr** | **P** | >2,5mM |
| **BCD(E)AB-Opr** | **Q** | >2,5mM |
| **BC(2-OAc)D(E)AB-OPr** | **R** | **2,2mM** |

Les mêmes oligosaccharides G, M, O et R sont reconnus par cet anticorps mIgM et par les mIgG (tableaux 1 et 2) confirmant l'importance de la présence de l'acétyl. L'apport de résidus additionnels à la séquence oligosaccharidique G n'améliore pas la reconnaissance. Les valeurs obtenues ici sont plus élevées indiquant que l'interaction est plus faible que celle observée avec les mIgG.

### Références Bibliographiques

1. Blatter, G.; Beau, J. M.; Jacquinet, J. C. Carbohydr. Res. 1994, 260, 189-202.
2. Kinzy, W.; Schmidt, R. R. Liebigs Ann Chem 1987, 407-415.
3. Shiozaki, M.; Kobayashi, Y.; Arai, M.; Watanabe, T.; Hiraoka, T.; Nishijima, M.; Kuge, S.; Otsuka, T.; Akamatsu, Y. J Med Chem 1991, 34, 2643-2646.
4. Hasegawa, A.; Ozaki, M.; Kiso, M.; Azuma, I. J. Carbohydr. Chem. 1984, 3, 331-341.
5. Myers, A. G.; Gin, D. Y.; Rogers, D. H. J Am Chem Soc 1993, 115, 2036-2038.
6. Oikawa, M.; Tanaka, T.; Fukuda, M.; Kusumoto, S. Tetrahadron Lett 2004, 45, 4039-4042.
7. Contant, C.; Jacquinet, J. C. J. Chem. Soc., Perkin Trans. 1 1995, 1573-1581.
8. Bergmann, Max ; Zervas, Leonidas Ber. Dtsch. Chem. Ges. 1931, 64, 975-980.
9. Medgyes, A.; Farkas, E.; Liptak, A.; Pozsgay, V. Tetrahedron 1997, 53, 4159-4178.
10. Myszka, H.; Bednarczyk, D.; Najder, M.; Kaca, W. Carbohydr. Res, 2003, 338, 133-141.
11. Inouye, Y.; Onodera, K.; Kitaoka, S.; Hirano, S. J. Am. Chem. Soc. 1956, 78, 4722-4724.
12. Inouye, Y.; Onodera, K.; Kitaoka, S.; Hirano, S. J. Chem. Soc. 1960, 25, 1265-1267.
13. Ponticelli, F.; Trendafilova, A.; Valoti, M.; Saponara, S.; Sgaragli, G. P. Carbohydr. Res. 2001, 330, 459-468.
14. Chittaboina, S.; Hodges, B.; Wang, Q. Lett. Org. Chem. 2006, 3, 35-38.
15. Excoffier, Gerard; Gagnaire, Didier; Utille, Jean-Pierre. Carbohydr. Res. 1975, 39, 368-373.
16. Lindhorst, T.K. Essentials of Carbohydrate Chemistry and Biochemistry; WILEY-VCH, 2000, 50.
17. Zhang, J.; Kovac, P. J. Carbohydr. Chem. 1999, 18, 461-469.
18. Furstner, A.; Muller, T. J. Am. Chem. Soc. 1999, 121, 7814-7821.
19. Larson, D. P.; Heathcock, C. H. J. Org. Chem. 1997, 62, 8406-8418.
20. Urban, F. J.; Moore, B. S.; Breitenbach, R. Tetrahedron Lett. 1990, 31, 4421-4424.
21. Yamazaki, F.; Sato, S.; Nukada, T.; Ito, Y.; Ogawa, T. Carbohydr. Res. 1990, 201, 31-50.
22. Lindhorst, T.K. Essentials of Carbohydrate Chemistry and Biochemistry; WILEY-VCH, 2000, 88.
23. Schmidt, R. R.; Jung, K-H. Oligosaccharide synthesis with Trichloroacetimidates Chapter 12. In Modern Synthetic Methods in Carbohydrate Chemistry; Hanessian, S. Ed.; Marcel Dekker Inc: New York, 1997; pp. 283-312.
24. Calinaud, P.; Gelas, J. Synthesis of Isopropylidene, Benzylidene, and Related Acetals Chapter 1. In Preparative Carbohydrate Chemistry; Hanessian, S. Ed.; Marcel Dekker Inc: New York, 1997; pp. 3-34.
25. Barili, P. L.; Berti, G.; Catelani, G.; Colonna, F.; Marra, A. Tetrahedron Lett. 1986, 27, 2307-2310.
26. Chittenden, G. J. F. Carbohydr. Res. 1980, 87, 219-226.
27. Chittenden, G. J. F. Carbohydr. Res. 1982, 108, 81-87.
28. Grindley, T. B.; Cote, C. J. P.; Wickramage, C. Carbohydr. Res. 1985, 140, 215-238.
29. Posner, G. H.; Loomis, G. L.; Sawaya, H. S. Tetrahedron Lett. 1975, 1373-1376.
30. Gelas, J.; Horton, D. Carbohydr. Res. 1978, 67, 371-387.
31. Wolfrom, M. L.; Diwadkar, A. B.; Gelas, J.; Horton, D. Carbohydr. Res. 1974, 35, 87-96.
32. Hasegawa, A.; Nakajima, M. Carbohydr. Res. 1973, 29, 239-245.
33. Hasegawa, A.; Kiso, M. Carbohydr. Res. 1978, 63, 91-98.
34. Schmidt, R. R.; Michel, J.; Roos, M. Liebigs Ann. Chem. 1984, 1343-1357.
35. Schmidt, R. R.; Stumpp, M. Liebigs Ann. Chem. 1983, 1249-1256.
36. Wegmann, B.; Schmidt, R. R. J. Carbohydr. Chem. 1987, 6, 357-375.
37. Alessi, Frederic; Doutheau, Alain; Anker, Daniel; Condemine, Guy; Robert-Baudouy, Janine. Tetrahedron 1996, 52, 4625-4636.
38. Crich, D.; Dudkin, V. JAm Chem Soc 2001, 123, 6819-6825.
39. Nashed, M. A.; Anderson, L. J. Chem. Soc.-Chem. Commun. 1982, 1274-1276.
40. Oltvoort, J. J.; Vanboeckel, C. A. A.; Dekoning, J. H.; Vanboom, J. H. Synthesis-Stuttgart 1981, 305-308.
41. Hecker, S. J.; Minich, M. L.; Lackey, K. J. Org. Chem. 1990, 55, 4904-4911.
42. Gigg, R.; Payne, S.; Conant, R. J. Carbohydr. Chem. 1983, 2, 207-223.
43. Pinto, B. M.; Morissette, D. G.; Bundle, D. R. J. Chem. Soc., Perkin Trans. 1 1987, 9-14.
44. Mulard, L. A.; Ughetto-Monfrin, J. J. Carbohydr. Chem. 2000, 19, 503-526.
45. Mulard, L. A.; Clement, M. J.; Segat-Dioury, F.; Delepierre, M. Tetrahedron 2002, 58, 2593-2604.
46. Lindhorst, T.K. Essentials of Carbohydrate Chemistry and Biochemistry; WILEY-VCH, 2000, 42.
47. Pinto, B. M.; Reimer, K. B.; Tixidre, A. Carbohydr. Res. 1991, 210, 199-219.
48. Smith, A. B.; Rivero, R. A.; Hale, K. J.; Vaccaro, H. A. J. Am. Chem. Soc. 1991, 113, 2092-2112.
49. Mulard, L. A.; Ughetto-Monfrin, J. J. Carbohydr. Chem. 1999, 18, 721-753.
50. Schmidt, R. R.; Michel, J. Tetrahedron Lett. 1984, 25, 821-824.
51. Zhang, K. Q.; Li, S. C.; Mao, J. M.; Chen, H. M.; Cai, M. S. Chemical Journal of Chinese Universities-Chinese 1997, 18, 1469-1473.
52. Schmidt, R. R.; Kinzy, W. ANOMERIC-OXYGEN ACTIVATION FOR GLYCOSIDE SYNTHESIS - THE TRICHLOROACETIMIDATE METHOD. In Advances in Carbohydrate Chemistry and Biochemistry, Vol 50, 1994; Vol. 50; pp. 21-123.
53. Hoffmann, M. G.; Schmidt, R. R. Liebigs Ann. Chem. 1985, 2403-2419.
54. Chapman, A. W.. Journal of Chemical Society 1925, 127, 1992-1998.
55. Mumm, O; Hesse, H; Volquartz, H Ber. Dtsch. Chem. Ges. 1915, 48, 379-391.
56. Wiberg, K. B.; Rowland, B. I. J. Am. Chem. Soc. 1955, 77, 2205-2209.
57. Nifantev, N. E.; Amochaeva, V. Y.; Shashkov, A. S. Bioorg. Khim 1992, 18, 562-569.
58. Mulard, L. A.; Glaudemans, C. P. Carbohydr. Res. 1998, 311, 121-33*.*
59. Heathcock, C. H.; Ratcliffe, R. J. Am. Chem. Soc. 1971, 93, 1746.
60. Zemplen, G. Ber. Dtsch. Chem. Ges. 1920, 60, 1555-1564.
61. Donohoe, T. J.; Logan, J. G.; Laffan, D. D. P. Org. Lett. 2003, 5, 4995-4998.
62. Belot, F.; Guerreiro, C.; Baleux, F.; Mulard, L. A. Chem. Eur, J. 2005, 11, 1625-1635.
63. Backinowsky, L. V.; Gomtsyan, A. R.; Byramova, N. E.; Kochetkov, N. K. Bioorg. Khim 1984, 10, 79-87.
64. Bakinovskii, L. V.; Gomtsyan, A. R.; Bairamova, N. E.; Kochetkov, N. K. Bioorg. Khim 1985, 11, 254-263.
65. Nifantev, N. E.; Shashkov, A. S.; Khatuntseva, E. A.; Tsvetkov, Y. E.; Sherman, A. A.; Kochetkov, N. K. Bioorg. Khim 1994, 20, 1001-1012.
66. Koenigs, Wilhelm; Knorr, Eduard. Chem. Ber. 1901, 34, 957-981.
67. Castro Palomino, Julio C.; Hemandez Rensoli, Marylin; Verez Bencomo, Vicente. J. Carbohydr. Chem. 1996, 15, 137-146.
68. Fekete, A.; Gyergyoi, K.; Kover, K. E.; Bajza, I.; Liptak, A. Carbohydr. Res. 2006, 341, 1312-1321.
69. Geyer, K.; Seeberger, P. H. Helv. Chim. Acta 2007, 90, 395-403.
70. Li, A. X.; Kong, F. Z. Carbohydr. Res. 2004, 339, 2499-2506.
71. Ning, J.; Zhang, W. H.; Yi, Y. T.; Yang, G. B.; Wu, Z. K.; Yi, J.; Kong, F. Z. Biorg. Med. Chem. 2003, 11, 2193-2203.
72. Yang, F.; He, H. M.; Du, Y. G.; Lu, M. J. Carbohydr. Res. 2002, 337, 1165-1169.
73. Zeng, Y.; Ning, J.; Kong, F. Z. Tetrahedron Lett. 2002, 43, 3729-3733.
74. Zeng, Y.; Ning, J.; Kong, F. Z. Carbohydr. Res. 2003, 338, 307-311.
75. Zhang, G. H.; Fu, M. K.; Ning, J. Carbohydr. Res. 2005, 340, 597-602.
76. Demchenko, A.; Stauch, T.; Boons, G. J. Synlett 1997, 818-&.
77. Demchenko, A. V.; Rousson, E.; Boons, G. J. Tetrahedron Lett. 1999, 40, 6523-6526.
78. Manabe, S.; Ito, Y.; Ogawa, T. Synlett 1998, 628-+.
79. Nishimur, D; Hasegawa, A.; Nakajima, M. Agricultural and Biological Chemistry 1972, 36, 1767-&.
80. Leblanc, Y.; Fitzsimmons, B. J.; Adams, J.; Perez, F.; Rokach, J. J. Org. Chem. 1986, 51, 789-793.
81. Nicolaou, K. C.; Reddy, K. R.; Skokotas, G.; Sato, F.; Xiao, X. Y.; Hwang, C. K. J. Am. Chem. Soc. 1993, 115, 3558-3575.
82. Kitagawa, I.; Baek, N. I.; Ohashi, K.; Sakagami, M.; Yoshikawa, M.; Shibuya, H. Chemical & Pharmaceutical Bulletin 1989, 37, 1131-1133.
83. Lubineau, A.; Basset-Carpentier, K.; Auge, C. Carbohydr. Res. 1997, 300, 161-167.
84. Zhang, S. Q.; Li, Z. J.; Wang, A. B.; Cai, M. S.; Feng, R. Carbohydr. Res. 1998, 308, 281-285.
85. Baeschlin, D. K.; Green, L. G,; Hahn, M. G.; Hinzen, B.; Ince, S. J.; Ley, S. V. Tetrahedron: Asym 2000, 11, 173-197.
86. Belot, F.; Wright, K.; Costachel, C.; Phalipon, A.; Mulard, L. A. J. Org. Chem. 2004, 69, 1060-74.
87. Pozsgay, V.; Coxon, B. Carbohydr. Res. 1994, 257, 189-215.
88. Hassner, A.; Strand, G.; Rubinstein, M.; Patchornik, A. J. Am. Chem. Soc. 1975, 97, 1614-1615.
89. Vanboom, J. H.; Burgers, P. M. J. Tetrahedron Lett. 1976, 4875-4878.
90. Kosemura, S.; Yamamura, S.; Kakuta, H.; Mizutani, J.; Hasegawa, K. Tetrahedron Lett. 1993, 34, 2653-2656.
91. Yamamura, T.; Hada, N.; Kaburaki, A.; Yamano, K.; Takeda, T. Carbohydr. Res. 2004, 339, 2749-2759.
92. Kovac, P.; Edgar, K. J. J. Org. Chem. 1992, 57, 2455-2467.
93. Mulard, L. A.; Clement, M. J.; Imberty, A.; Delepierre, M. Eur. J. Org. Chem. 2002, 2486-2498.
94. Nagashima, N.; Ohno, M. Chem. Lett. 1987, 141-144.
95. Westerduin, P.; De Haan, P. E.; Dees, M. J.; Van Boom, J. H. Carbohydr. Res. 1988, 180, 195-205.
96. Bousquet, E.; Khitri, M.; Lay, L.; Nicotra, F.; Panza, L.; Russo, G. Carbohydr. Res. 1998, 311, 171-181.
97. Jeker, N.; Tamm, C. Helv. Chim. Acta 1988, 71, 1895-1903.
98. Jeker, N.; Tamm, C. Helv. Chim. Acta 1988, 71, 1904-1913.
99. Koeners, H. J.; Verhoeven, J.; Vanboom, J. H. Tetrahedron Lett. 1980, 21, 381-382.
100. Koeners, H. J.; Verhoeven, J.; Vanboom, J. H. Recl. Trav. Chim. Pays-Bas-J. Roy. Neth. Chem. Soc. 1981, 100, 65-72.
101. Osborn, H. M. I. Carbohydrates; Academic Press, New York, 2003, 26.
102. Ravida, A.; Liu, X. Y.; Kovacs, L.; Seeberger, P. H. Org. Lett. 2006, 8, 1815-1818.
103. Kong, F. Z. Carbohydr. Res. 2007, 342, 345-373.
104. Dong, L.; Roosenberg, J. M.; Miller, M. J. J. Am. Chem. Soc. 2002, 124, 15001-15005.
105. Zhu, Y. L.; Kong, F. Z. Synlett 2000, 1783-1787.
106. Lindhorst, T. K. J. Carbohydr. Chem. 1997, 16, 237-243.
107. Franks, N. E.; Montgomery, R. Carbohydr. Res. 1968, 6, 286-298.
108. Srivastava, V. K.; Schuerch, C. J. Org. Chem. 1981, 46, 1121-1126.
109. Glushka, J. N.; Perlin, A. S. Carbohydr. Res. 1990, 205, 305-321.
110. Kordes, M.; Winsel, H.; de Meijere, A. Eur. J. Org. Chem. 2000, 3235-3245.
111. Rips, R.; Derappe, Ch; Buu-Hoi, N. P. J. Org. Chem. 1960, 25, 390-392.
112. Meldgaard, M.; Hansen, F. G.; Wengel, J. J. Org. Chem. 2004, 69, 6310-6322.
113. Elliott, W. J.; Fried, J. J. Org. Chem. 1978, 43, 2708-2710.
114. Belot, F.; Rabuka, D.; Fukuda, M.; Hindsgaul, O. Tetrahedron Lett. 2002, 43, 7743-7747.
115. Belot, F.; Costachel, C.; Wright, K.; Phalipon, A.; Mulard, L. A. Tetrahedron Lett. 2002, 43, 8215-8218.
116. Nishikawa, T.; Urabe, D.; Tomita, M.; Tsujimoto, T.; Iwabuchi, T.; Isobe, M. Org. Lett. 2006, 8, 3263-3265.

## Revendications

1. Conjugués spécifiques de *Shigella flexneri* de sérotype 3a et/ou X, comprenant un saccharide (oligosaccharide ou un polysaccharide) sélectionné dans le groupe de la liste L1 constitué par :
(X)ₓ-{D(E)A}ₙ-(Y)_{y}
(X)ₓ-{(E)AB}ₙ-(Y)_{y}
(X)ₓ-{CD(E)A}ₙ-(Y)_{y}
(X)ₓ-{D(E)AB}ₙ-(Y)_{y}
(X)ₓ-{(E)ABC}ₙ-(Y)_{y}
(X)ₓ-{BCD(E)A}ₙ-(Y)_{y}
(X)ₓ-{CD(E)AB)ₙ-(Y)_{y}
(X)ₓ-{D(E)ABC}ₙ-(Y)_{y}
(X)ₓ-{(E)ABCD}ₙ-(Y)_{y,}
et dans lesquels :
A représente un résidu α-L-Rha*p*-(1,2),
B représente un résidu α-L-Rha*p*-(1,3),
C représente un résidu α-L-Rha*p*-(1,3) ou un résidu [2-O-acétyle] α-L-Rha*p*-(1,3),
D représente un résidu β-D-GlcNAc*p*-(1,2) et
E représente un résidu α-D-Glc*p*-(1,3),
x et y représentent indépendamment 0 ou 1,
X et Y représentent un mono-, di- ou oligosaccharide et sont indépendamment sélectionnés dans le groupe constitué par A, B, C, D, E, AB, BC, CD, DA, (E)A, ABC, BCD, CDA, DAB, (E)AB, D(E)A, ABCD, BCDA, CDAB, DABC, (E)ABC, D(E)AB, CD(E)A, (E)ABCD, D(E)ABC, CD(E)AB et BCD(E)A,
X et Y sont tels que la séquence de l'enchaînement -ABCD- est toujours conservée,
n est un nombre entier compris entre 1 et 10, de préférence entre 2 et 6 lesquels oligo- ou polysaccharides étant liés à une molécule M de support.

2. Conjugué selon la revendication 1, **caractérisé en ce que** la molécule M de support est sélectionnée dans le groupe constitué par des peptides et des protéines comprenant au moins un épitope T, de préférence dans le groupe constitué par la protéine IpaD, le peptide PADRE, la toxine tétanique, la protéine KLH ou la protéine CRM₁₉₇.

3. Conjugué selon la revendication 1 ou la revendication 2, **caractérisé en ce que** ledit saccharide est directement lié à ladite molécule M de support.

4. Conjugué selon la revendication 1 ou la revendication 2, **caractérisé en ce que** ledit saccharide est lié à ladite molécule M de support via un espaceur.

5. Conjugué selon l'une quelconque des revendications 1 à 4, **caractérisé en ce que** le rapport saccharide:molécule M est compris entre 1:1 et 200:1, de préférence entre 10:1 et 30:1.

6. Conjugué selon l'une quelconque des revendications 1 à 5, **caractérisé en ce que** le saccharide est sélectionné dans le groupe constitué par les tétrasaccharides, les pentasaccharides et les multimères de ces derniers respectant la séquence de l'enchaînement - ABCD-.

7. Conjugué selon la revendication 6, **caractérisé en ce que** ledit saccharide est sélectionné dans le groupe constitué par :
[CD(E)A], [D(E)AB], [(E)ABC], [BCD(E)A], [CD(E)AB], [D(E)ABC], [(E)ABCD], [BCD(E)A]ₙ, [CD(E)AB]ₙ, [D(E)ABC]ₙ, [(E)ABCD]ₙ, [BCD(E)AB], [D(E)ABCD], [D(E)ABCD(E)A], [BCD(E)ABCD], [BCD(E)ABCD(E)A], [D(E)ABCD(E)ABC], avec n étant compris entre 2 et 5.

8. Composition immunogène comprenant un conjugué selon l'une quelconque des revendications 1 à 7 et au moins un excipient pharmaceutiquement acceptable.

9. Composition selon la revendication 8, **caractérisée en ce que** ledit saccharide est un pentasaccharide ou un multimère de ce dernier.

10. Composition immunogène selon la revendication 8 ou la revendication 9, **caractérisée en ce qu'**elle comprend en outre un immunogène capable de protéger contre un autre agent pathogène, tel que *S. flexneri* de sérotype 1b, 2a et 6 ou d'autres espèces de *Shigella,* telles que *S. dysenteriae* et *S. sonnei* ou d'autres pathogènes responsables de diarrhées chez l'homme.

11. Composition selon l'une quelconque des revendications 8 à 10, **caractérisée en ce qu'**elle est formulée pour une administration parentérale, orale, intra-nasale ou intradermique.

## Patentansprüche

1. Konjugate, die für *Shigella flexneri* vom Serotyp 3a und/oder X spezifisch sind, umfassend ein Saccharid (Oligosaccharid oder ein Polysaccharid), ausgewählt aus der Gruppe der Liste L1, bestehend aus:
(X)ₓ-{D(E)A}ₙ-(Y)_{y}
(X)ₓ-{(E)AB}ₙ-(Y)_{y}
(X)ₓ-{CD(E)A}ₙ-(Y)_{y}
(X)ₓ-{D(E)AB}ₙ-(Y)_{y}
(X)ₓ-{(E)ABC}ₙ-(Y)_{y}
(X)ₓ-{BCD(E)A}ₙ-(Y)_{y}
(X)ₓ-{CD(E)AB}ₙ-(Y)_{y}
(X)ₓ-{D(E)ABC}ₙ-(Y)_{y}
(X)ₓ-{(E)ABCD}ₙ-(Y)_{y},
und wobei:
A für einen α-L-Rha*p*-(1,2)-Rest steht,
B für einen α-L-Rha*p*-(1,3)-Rest steht,
C für einen α-L-Rha*p*-(1,3)-Rest oder einen [2-O-Acetyl] α-L-Rha*p*-(1,3)-Rest steht,
D für einen β-D-GlcNAc*p*-(1,2)-Rest steht, und
E für einen α-D-Glc*p*-(1,3)-Rest steht,
x und y unabhängig für 0 oder 1 stehen,
X und Y für ein Mono-, Di- oder Oligosaccharid stehen und unabhängig ausgewählt sind aus der Gruppe bestehend aus A, B, C, D, E, AB, BC, CD, DA, (E)A, ABC, BCD, CDA, DAB, (E)AB, D(E)A, ABCD, BCDA, CDAB,
DABC, (E)ABC, D(E)AB, CD(E)A, (E)ABCD, D(E)ABC, CD(E)AB und BCD(E)A,
X und Y so sind, dass die Sequenz der Verkettung -ABCD- immer aufrechterhalten bleibt,
n eine ganze Zahl im Bereich zwischen 1 und 10, vorzugsweise zwischen 2 und 6 ist,
wobei die Oligo- oder Polysaccharide an ein Trägermolekül M gebunden sind.

2. Konjugat nach Anspruch 1, **dadurch gekennzeichnet, dass** das Trägermolekül M ausgewählt ist aus der Gruppe bestehend aus Peptiden und Proteinen, die mindestens ein T-Epitop umfassen, vorzugsweise aus der Gruppe bestehend aus dem Protein IpaD, dem Peptid PADRE, dem Tetanustoxin, dem Protein KLH oder dem Protein CRM₁₉₇.

3. Konjugat nach Anspruch 1 oder Anspruch 2, **dadurch gekennzeichnet, dass** das Saccharid direkt an das Trägermolekül M gebunden ist.

4. Konjugat nach Anspruch 1 oder Anspruch 2, **dadurch gekennzeichnet, dass** das Saccharid über einen Spacer an das Trägermolekül M gebunden ist.

5. Konjugat nach einem der Ansprüche 1 bis 4, **dadurch gekennzeichnet, dass** das Verhältnis Saccharid : Molekül M im Bereich zwischen 1 : 1 und 200 : 1, vorzugsweise zwischen 10 : 1 und 30 : 1 beträgt.

6. Konjugat nach einem der Ansprüche 1 bis 5, **dadurch gekennzeichnet, dass** das Saccharid ausgewählt ist aus der Gruppe bestehend aus den Tetrasacchariden, den Pentasacchariden und den Multimeren dieser letzteren unter Einhaltung der Sequenz der Verkettung -ABCD-.

7. Konjugat nach Anspruch 6, **dadurch gekennzeichnet, dass** das Saccharid ausgewählt ist aus der Gruppe bestehend aus:
[CD(E)A], [D(E)AB], [(E)ABC], [BCD(E)A], [CD(E)AB], [D(E)ABC], [(E)ABCD], [BCD(E)A]ₙ, [CD(E)AB]ₙ, [D(E)ABC]ₙ, [(E)ABCD]ₙ, [BCD(E)AB], [D(E)ABCD], [D(E)ABCD(E)A], [BCD(E)ABCD], [BCD(E)ABCD(E)A], [D(E)ABCD(E)ABC], wobei n im Bereich zwischen 2 und 5 beträgt.

8. Immunogene Zusammensetzung, die ein Konjugat nach einem der Ansprüche 1 bis 7 und mindestens einen pharmazeutisch verträglichen Hilfsstoff umfasst.

9. Zusammensetzung nach Anspruch 8, **dadurch gekennzeichnet, dass** das Saccharid ein Pentasaccharid oder ein Multimer dieses letzteren ist.

10. Immunogene Zusammensetzung nach Anspruch 8 oder Anspruch 9, **dadurch gekennzeichnet, dass** sie weiter ein Immunogen umfasst, das in der Lage ist, gegen einen weiteren Erreger zu schützen, wie etwa *S*. *flexneri* vom Serotyp 1b, 2a und 6 oder weitere *Shigella*-Arten, wie etwa *S*. *dysenteriae* und *S*. *sonnei* oder weitere Erreger, die für Durchfälle beim Menschen verantwortlich sind.

11. Zusammensetzung nach einem der Ansprüche 8 bis 10, **dadurch gekennzeichnet, dass** sie für eine parenterale, orale, intranasale oder intradermale Verabreichung formuliert ist.

## Claims

1. Specific conjugates of *Shigella flexneri* of serotype 3a and/or X, comprising a saccharide (oligosaccharide or polysaccharide) selected from the group of list L1 consisting of:
(X)ₓ-{D(E)A}ₙ-(Y)_{y}
(X)ₓ-{(E)AB}ₙ-(Y)_{y}
(X)ₓ-{CD(E)A}ₙ-(Y)_{y}
(X)ₓ-{D(E)AB}ₙ-(Y)_{y}
(X)ₓ-{(E)ABC}ₙ-(Y)_{y}
(X)ₓ-{BCD(E)A)ₙ-(Y)_{y}
(X)ₓ-{CD(E)AB}ₙ-(Y)_{y}
(X)ₓ-{D(E)ABC}ₙ-(Y)_{y}
(X)ₓ-{(E)ABCD}ₙ-(Y)_{y},
and wherein:
A represents a residue α-L-Rha*p*-(1,2),
B represents a residue α-L-Rha*p*-(1,3),
C represents a residue α-L-Rha*p*-(1,3) or a residue [2-O-acetyl] α-L-Rhap-(1,3),
D represents a residue β-D-GlcNAc*p*-(1,2) and
E represents a residue α-D-Glc*p*-(1,3),
x and y represent independently 0 or 1,
X and Y represent a mono-, di- or oligosaccharide and are independently selected from the group consisting of A, B, C, D, E, AB, BC, CD, DA, (E)A, ABC, BCD, CDA, DAB, (E)AB, D(E)A, ABCD, BCDA, CDAB, DABC, (E)ABC, D(E)AB, CD(E)A, (E)ABCD, D(E)ABC, CD(E)AB and BCD(E)A,
X and Y are such that the sequence of the chain -ABCD- is always maintained,
n is a natural number between 1 and 10, preferably between 2 and 6
said oligo- or polysaccharides being bonded to a support molecule M.

2. Conjugate according to claim 1, **characterised in that** the support molecule M is selected from the group consisting of peptides and proteins comprising at least one epitope T, preferably from the group consisting of the protein IpaD, the peptide PADRE, the tetanus toxin, the protein KLH or the protein CRM₁₉₇.

3. Conjugate according to claim 1 or claim 2, **characterised in that** said saccharide is directly bonded to said support molecule M.

4. Conjugate according to claim 1 or claim 2, **characterised in that** said saccharide is directly bonded to said support molecule M through a spacer.

5. Conjugate according to any one of the claims 1 to 4, **characterised in that** the ratio of saccharide to the molecule M ranges from 1:1 to 200:1, preferably from 10:1 to 30:1.

6. Conjugate according to any one of the claims 1 to 5, **characterised in that** the saccharide is selected from the group consisting of the tetrasaccharides, the pentasaccharides and their multimers, respecting the chain sequence -ABCD-.

7. Conjugate according to claim 6, **characterised in that** said saccharide is selected from the group consisting of:
[CD(E)A], [D(E)AB], [(E)ABC], [BCD(E)A], [CD(E)AB], [D(E)ABC], [(E)ABCD], [BCD(E)A]ₙ, [CD(E)AB]ₙ, [D(E)ABC]ₙ, [(E)ABCD]ₙ, [BCD(E)AB], [D(E)ABCD], [D(E)ABCD(E)A], [BCD(E)ABCD], [BCD(E)ABCD(E)A], [D(E)ABCD(E)ABC], where n ranges from 2 to 5.

8. Immunogenic composition comprising a conjugate according to any one of the claims 1 to 7 and at least one pharmaceutically-acceptable excipient.

9. Composition according to claim 8, **characterised in that** said saccharide is a pentasaccharide or one of its multimers.

10. Immunogenic composition according to claim 8 or claim 9, **characterised in that** it further comprises an immunogen protecting against another disease causative agent, such as *S*. *flexneri* of serotype 1b, 2a and 6 or other species of *Shigella*, such as *S*. *dysenteriae* and *S*. *sonnei* or other pathogens responsible for diarrhoea in humans.

11. Composition according to any one of the claims 8 to 10, **characterised in that** it is formulated for parenteral, oral, intranasal or intradermal administration.
